(19) Europäisches Patentamt / European Patent Office / Office européen des brevets

(11) **EP 4 104 857 A1**

(12) **EUROPEAN PATENT APPLICATION**
published in accordance with Art. 153(4) EPC

(43) Date of publication:
**21.12.2022 Bulletin 2022/51**

(21) Application number: **21754475.8**

(22) Date of filing: **10.02.2021**

(51) International Patent Classification (IPC):
**A61K 39/395** (2006.01)  **A61K 45/00** (2006.01)
**A61P 35/00** (2006.01)  **A61P 43/00** (2006.01)
**C07K 16/28** (2006.01)  **C07K 16/30** (2006.01)
**C07K 16/46** (2006.01)  **C12N 15/13** (2006.01)

(52) Cooperative Patent Classification (CPC):
**A61K 39/395; A61K 45/00; A61P 35/00;
A61P 43/00; C07K 16/00; C07K 16/28;
C07K 16/30; C07K 16/46**

(86) International application number:
**PCT/JP2021/004871**

(87) International publication number:
**WO 2021/162020 (19.08.2021 Gazette 2021/33)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **12.02.2020   JP 2020021275
21.08.2020   JP 2020140489**

(71) Applicant: **CHUGAI SEIYAKU KABUSHIKI KAISHA
Tokyo 115-8543 (JP)**

(72) Inventors:
• **SAKURAI, Mika**
  **Tokyo 103-8324 (JP)**
• **NARITA, Yoshinori**
  **Synapse, 138623 (SG)**
• **TANIGUCHI, Kenji**
  **Kamakura-shi, Kanagawa 247-8530 (JP)**

• **MIKAMI, Hirofumi**
  **Gotemba-shi, Shizuoka 412-8513 (JP)**
• **HORIKAWA, Sayuri**
  **Kamakura-shi, Kanagawa 247-8530 (JP)**
• **UCHIKAWA, Ryo**
  **Kamakura-shi, Kanagawa 247-8530 (JP)**
• **ONO, Natsuki**
  **Gotemba-shi, Shizuoka 412-8513 (JP)**
• **HAMADA, Koki**
  **Kamakura-shi, Kanagawa 247-8530 (JP)**

(74) Representative: **Vossius & Partner
Patentanwälte Rechtsanwälte mbB
Siebertstraße 3
81675 München (DE)**

Remarks:
The complete document including Reference Table(s) and the Sequence Listing(s) can be downloaded from the EPO website

(54) **ANTI-CD137 ANTIGEN-BINDING MOLECULE FOR USE IN CANCER TREATMENT**

(57)  An objective of the present disclosure is to provide: anticancer agents having an immune cell-activating effect, a cytotoxic activity, or an antitumor activity, but having a low effect on non-tumor tissues such as normal tissues and having few side effects; combination therapies using those anticancer agents and other anticancer agents; and pharmaceutical combinations for use in those combination therapies.

Anticancer agents expected to be applied to various types of cancers, which have an immune cell-activating effect, a cytotoxic activity, or an antitumor activity while having a low effect on non-tumor tissues such as normal tissues and having few side effects by using as an active ingredient an anti-CD 137 antigen-binding molecule of the present disclosure, the binding activity of which to CD137 changes depending on various substances (for example, low molecular weight compounds) in target tissues, are provided. Combination therapies using those anticancer agents and other anticancer agents, and pharmaceutical compositions for use in those combination therapies are also provided.

EP 4 104 857 A1

FIG. 1

**Description**

[Technical Field]

The present disclosure relates to anticancer agents comprising an anti-CD 137 antigen-binding molecule, and therapeutic methods using such an agent and another anticancer agent in combination.

[Background Art]

Cancer is a fatal disease that is difficult to cure completely except for some cases. The outcome of treatment with chemotherapeutic agents, which is the main therapeutic method, is by no means good. It has been suggested that not only the heterogeneity of cancer cells themselves but the tumor microenvironment plays a significant role as a factor making cancer treatment difficult (NPL 1). Recently, unresectable malignant melanoma and such were shown to be potentially curable with an anti-CTLA-4 antibody, which suppresses the immunosuppressive function of CTLA-4 and thereby promotes activation of T cells (NPL 2). In the year 2011, an anti-human CTLA-4 monoclonal antibody (ipilimumab) was approved by the U.S. Food and Drug Administration (FDA) as the first immune-activating antibody drug in the world. Furthermore, inhibitory antibodies against PD-1 and PD-L1, other immune checkpoint molecules than CTLA-4, have also been reported to have therapeutic effects (NPL 3), and approved by FDA.

It is understood that T cells, which have important roles in tumor immunity, are activated by two signals: 1) binding of a T cell receptor (TCR) to an antigenic peptide presented by major histocompatibility complex (MHC) class I molecules and activation of the TCR; and 2) binding of a costimulatory molecule on the surface of T cells to its ligands on the antigen-presenting cells and activation of the costimulatory molecule. In addition, activation of costimulatory molecules belonging to the tumor necrosis factor receptor superfamily (TNFRSF), including CD137 (4-1BB), on the surface of T cells has been described as important for T cell activation (NPL 4).

TNFRSF includes CD137, CD40, OX40, RANK, GITR, and such molecules. CD137 is reportedly expressed not only on the surface of T cells but also on the surface of other immune cells such as dendritic cells (DC), B cells, NK cells, macrophages, and neutrophils (NPL 5).

CD137 agonist antibody has already been demonstrated to show antitumor effect in a mouse model, and this has been shown to result mainly from activation of CD8-positive T cells and NK cells by the mouse model experiments (NPL 6). However, side effects due to the nonspecific hepatotoxicity of CD137 agonist antibody have become clinical and non-clinical problems, hindering the desired progress of drug development (NPL 7, NPL 8). It is suggested that the side effects are caused mainly by activation of immune cells in non-tumor, non-immune tissues such as liver which involves binding of the antibody to the Fcγ receptor via the antibody constant region (NPL 9). On the other hand, it has been reported that in order for agonistic anti-TNF receptor superfamily member antibodies to exhibit agonistic activity in vivo, the antibody needs be cross-linked by Fcγ receptor-expressing cells (FcγRII-expressing cells) (NPL 10). That is, binding of CD137 agonist antibody to Fcγ receptor is involved in both the drug efficacy of the antitumor effect of the antibody and its side effects such as hepatotoxicity. Thus, increasing the binding between the antibody and the Fcγ receptor is expected to enhance the drug efficacy but may also increase hepatotoxic side effects, and reducing the binding between the antibody and the Fcγ receptor may reduce the side effects but also reduce the drug efficacy. There has been no report so far of a CD137 agonist antibody whose drug efficacy and side effects are separated. Moreover, the antitumor effect of CD137 agonist antibody itself is by no means clinically potent, and further enhancement of the drug efficacy is wanted along with avoidance of the toxicity. Accordingly, a new drug is desired to be developed that is capable of inducing antitumor immune responses while reducing those side effects.

When a therapeutic antibody is administered into a living body, it is desirable that its target antigen be expressed specifically at the site of lesion only. However, in many cases, the same antigen is also expressed in non-lesion sites, i.e. normal tissues, and this could be a cause of side effects unwanted from the viewpoint of treatment. For example, while antibodies against tumor antigens can exhibit cytotoxic activity on tumor cells by ADCC etc., they could also damage normal cells if the same antigen is expressed in normal cells. In order to solve the above-mentioned problems, a focus was placed on the phenomenon in which certain compounds are abundantly present in target tissues (e.g. tumor tissues), and a technology to search for antigen-binding molecules with varying antigen-binding activity depending on the concentration of such compounds was developed (for example, PTL 1).

In recent years, the efficacy of immunotherapeutic agents represented by inhibitors that target immune checkpoint molecules such as CTLA-4, PD-1, and PD-L1 has been clinically proven. However, these drugs are not effective for all patients, and further enhancement of drug efficacy is needed. As to the combined use of immunotherapies, it has been confirmed that the combined use of nivolumab and ipilimumab enhances the drug efficacy in melanoma as compared with ipilimumab alone (Non-Patent Document 11).

[Citation List]

[Patent Literature]

[PTL 1] WO2013/180200

[Non Patent Literature]

[NPL 1] Hanahan, Cell, 2011, 144, 646-74
[NPL 2] Prieto, Clin Cancer Res. 2012, 18, 2039-47
[NPL 3] Hamid, Expert Opin. Biol. Ther., 2013, 6, 847-61
[NPL 4] Summers, Nat Rev Immunol, 2012, 12, 339-51
[NPL 5] Vinay, Cellular & Molecular Immunology, 2011, 8, 281-284
[NPL 6] Houot, Blood, 2009, 114, 3431-8
[NPL 7] Ascierto, Semin Oncol, 2010, 37, 508-16
[NPL 8] Dubrot, Cancer Immunol Immunother, 2010, 59, 1223-33
[NPL 9] Schabowsky, Vaccine, 2009, 28, 512-22
[NPL 10] Li, Proc Natl Acad Sci USA. 2013, 110(48), 19501-6
[NPL 11] N Eng J Med (2015) vol.373, p.23-34

[Summary of Invention]

[Technical Problem]

The present disclosure relates to anticancer agents comprising an anti-CD 137 antigen-binding molecule, and combination therapies with another anticancer agent.

[Solution to Problem]

In order to provide anti-CD137 antigen-binding molecules which have immunocyte-activating effect, cytotoxic activity, or antitumor activity and meanwhile have reduced effect on non-tumor tissues such as normal tissues and have less side effects, and provide methods of using the same, the present disclosure provides anticancer agents comprising as an active ingredient an anti-CD 137 antigen-binding molecule characterized in that their binding activity to CD137 varies depending on various compounds (e.g. small molecule compounds) in target tissues (e.g. tumor tissues). The present disclosure also provides combination therapies using such an anticancer agent comprising an anti-CD 137 antigen-binding molecule and another agent.

Specifically, the present disclosure provides anticancer agents comprising an anti-CD137 antigen-binding molecule, methods of using the same, combination therapies using such an anticancer agent and another anticancer agent, kits, and such, as exemplarily described below.

[1] An anticancer agent comprising as an active ingredient an anti-CD 137 antigen-binding molecule comprising any combination of HVR-H1, HVR-H2, HVR-H3, HVR-L1, HVR-L2, and HVR-L3 selected from (a) to (m) below:

(a) HVR-H1 comprising the amino acid sequence of SEQ ID NO: 7, HVR-H2 comprising the amino acid sequence of SEQ ID NO: 8, HVR-H3 comprising the amino acid sequence of SEQ ID NO: 17, HVR-L1 comprising the amino acid sequence of SEQ ID NO: 21, HVR-L2 comprising the amino acid sequence of SEQ ID NO: 26, and HVR-L3 comprising the amino acid sequence of SEQ ID NO: 27;
(b) HVR-H1 comprising the amino acid sequence of SEQ ID NO: 7, HVR-H2 comprising the amino acid sequence of SEQ ID NO: 9, HVR-H3 comprising the amino acid sequence of SEQ ID NO: 17, HVR-L1 comprising the amino acid sequence of SEQ ID NO: 22, HVR-L2 comprising the amino acid sequence of SEQ ID NO: 26, and HVR-L3 comprising the amino acid sequence of SEQ ID NO: 27;
(c) HVR-H1 comprising the amino acid sequence of SEQ ID NO: 7, HVR-H2 comprising the amino acid sequence of SEQ ID NO: 10, HVR-H3 comprising the amino acid sequence of SEQ ID NO: 17, HVR-L1 comprising the amino acid sequence of SEQ ID NO: 22, HVR-L2 comprising the amino acid sequence of SEQ ID NO: 26, and HVR-L3 comprising the amino acid sequence of SEQ ID NO: 27;
(d) HVR-H1 comprising the amino acid sequence of SEQ ID NO: 7, HVR-H2 comprising the amino acid sequence of SEQ ID NO: 11, HVR-H3 comprising the amino acid sequence of SEQ ID NO: 18, HVR-L1 comprising the amino acid sequence of SEQ ID NO: 21, HVR-L2 comprising the amino acid sequence of SEQ ID NO: 26, and HVR-L3 comprising the amino acid sequence of SEQ ID NO: 27;

(e) HVR-H1 comprising the amino acid sequence of SEQ ID NO: 7, HVR-H2 comprising the amino acid sequence of SEQ ID NO: 8, HVR-H3 comprising the amino acid sequence of SEQ ID NO: 18, HVR-L1 comprising the amino acid sequence of SEQ ID NO: 21, HVR-L2 comprising the amino acid sequence of SEQ ID NO: 26, and HVR-L3 comprising the amino acid sequence of SEQ ID NO: 27;

(f) HVR-H1 comprising the amino acid sequence of SEQ ID NO: 7, HVR-H2 comprising the amino acid sequence of SEQ ID NO: 12, HVR-H3 comprising the amino acid sequence of SEQ ID NO: 18, HVR-L1 comprising the amino acid sequence of SEQ ID NO: 21, HVR-L2 comprising the amino acid sequence of SEQ ID NO: 26, and HVR-L3 comprising the amino acid sequence of SEQ ID NO: 28;

(g) HVR-H1 comprising the amino acid sequence of SEQ ID NO: 7, HVR-H2 comprising the amino acid sequence of SEQ ID NO: 13, HVR-H3 comprising the amino acid sequence of SEQ ID NO: 18, HVR-L1 comprising the amino acid sequence of SEQ ID NO: 21, HVR-L2 comprising the amino acid sequence of SEQ ID NO: 26, and HVR-L3 comprising the amino acid sequence of SEQ ID NO: 29;

(h) HVR-H1 comprising the amino acid sequence of SEQ ID NO: 7, HVR-H2 comprising the amino acid sequence of SEQ ID NO: 14, HVR-H3 comprising the amino acid sequence of SEQ ID NO: 19, HVR-L1 comprising the amino acid sequence of SEQ ID NO: 23, HVR-L2 comprising the amino acid sequence of SEQ ID NO: 26, and HVR-L3 comprising the amino acid sequence of SEQ ID NO: 27;

(i) HVR-H1 comprising the amino acid sequence of SEQ ID NO: 7, HVR-H2 comprising the amino acid sequence of SEQ ID NO: 15, HVR-H3 comprising the amino acid sequence of SEQ ID NO: 20, HVR-L1 comprising the amino acid sequence of SEQ ID NO: 24, HVR-L2 comprising the amino acid sequence of SEQ ID NO: 26, and HVR-L3 comprising the amino acid sequence of SEQ ID NO: 27;

(j) HVR-H1 comprising the amino acid sequence of SEQ ID NO: 7, HVR-H2 comprising the amino acid sequence of SEQ ID NO: 15, HVR-H3 comprising the amino acid sequence of SEQ ID NO: 20, HVR-L1 comprising the amino acid sequence of SEQ ID NO: 25, HVR-L2 comprising the amino acid sequence of SEQ ID NO: 26, and HVR-L3 comprising the amino acid sequence of SEQ ID NO: 27;

(k) HVR-H1 comprising the amino acid sequence of SEQ ID NO: 7, HVR-H2 comprising the amino acid sequence of SEQ ID NO: 16, HVR-H3 comprising the amino acid sequence of SEQ ID NO: 20, HVR-L1 comprising the amino acid sequence of SEQ ID NO: 25, HVR-L2 comprising the amino acid sequence of SEQ ID NO: 26, and HVR-L3 comprising the amino acid sequence of SEQ ID NO: 27;

(l) HVR-H1 comprising the amino acid sequence of SEQ ID NO: 7, HVR-H2 comprising the amino acid sequence of SEQ ID NO: 14, HVR-H3 comprising the amino acid sequence of SEQ ID NO: 19, HVR-L1 comprising the amino acid sequence of SEQ ID NO: 24, HVR-L2 comprising the amino acid sequence of SEQ ID NO: 26, and HVR-L3 comprising the amino acid sequence of SEQ ID NO: 27; and

(m) HVR-H1 comprising the amino acid sequence of SEQ ID NO: 7, HVR-H2 comprising the amino acid sequence of SEQ ID NO: 14, HVR-H3 comprising the amino acid sequence of SEQ ID NO: 17, HVR-L1 comprising the amino acid sequence of SEQ ID NO: 21, HVR-L2 comprising the amino acid sequence of SEQ ID NO: 26, and HVR-L3 comprising the amino acid sequence of SEQ ID NO: 27.

[1.1] An anticancer agent comprising as an active ingredient an anti-CD137 antigen-binding molecule comprising:

(a) a VH having at least 95% sequence identity with any one of the amino acid sequences of SEQ ID NOs: 43 to 53; or
(b) a VL having at least 95% sequence identity with any one of the amino acid sequences of SEQ ID NOs: 54 to 60.

[1.2] An anticancer agent comprising an anti-CD 137 antigen-binding molecule which comprises any combination of VH and VL selected from (a) to (m) below:

(a) a VH having at least 95% sequence identity with the amino acid sequence of SEQ ID NO: 43, and a VL having at least 95% sequence identity with the amino acid sequence of SEQ ID NO: 54;
(b) a VH having at least 95% sequence identity with the amino acid sequence of SEQ ID NO: 44, and a VL having at least 95% sequence identity with the amino acid sequence of SEQ ID NO: 55;
(c) a VH having at least 95% sequence identity with the amino acid sequence of SEQ ID NO: 45, and a VL having at least 95% sequence identity with the amino acid sequence of SEQ ID NO: 55;
(d) a VH having at least 95% sequence identity with the amino acid sequence of SEQ ID NO: 46, and a VL having at least 95% sequence identity with the amino acid sequence of SEQ ID NO: 54;
(e) a VH having at least 95% sequence identity with the amino acid sequence of SEQ ID NO: 47, and a VL having at least 95% sequence identity with the amino acid sequence of SEQ ID NO: 54;

(f) a VH having at least 95% sequence identity with the amino acid sequence of SEQ ID NO: 48, and a VL having at least 95% sequence identity with the amino acid sequence of SEQ ID NO: 56;

(g) a VH having at least 95% sequence identity with the amino acid sequence of SEQ ID NO: 49, and a VL having at least 95% sequence identity with the amino acid sequence of SEQ ID NO: 57;

(h) a VH having at least 95% sequence identity with the amino acid sequence of SEQ ID NO: 50, and a VL having at least 95% sequence identity with the amino acid sequence of SEQ ID NO: 58;

(i) a VH having at least 95% sequence identity with the amino acid sequence of SEQ ID NO: 51, and a VL having at least 95% sequence identity with the amino acid sequence of SEQ ID NO: 59;

(j) a VH having at least 95% sequence identity with the amino acid sequence of SEQ ID NO: 51, and a VL having at least 95% sequence identity with the amino acid sequence of SEQ ID NO: 60;

(k) a VH having at least 95% sequence identity with the amino acid sequence of SEQ ID NO: 52, and a VL having at least 95% sequence identity with the amino acid sequence of SEQ ID NO: 60;

(l) a VH having at least 95% sequence identity with the amino acid sequence of SEQ ID NO: 50, and a VL having at least 95% sequence identity with the amino acid sequence of SEQ ID NO: 59; and

(m) a VH having at least 95% sequence identity with the amino acid sequence of SEQ ID NO: 53, and a VL having at least 95% sequence identity with the amino acid sequence of SEQ ID NO: 54.

[2] An anticancer agent comprising an anti-CD 137 antigen-binding molecule which comprises any combination of VH and VL selected from (a) to (m) below:

(a) a VH comprising the amino acid sequence of SEQ ID NO: 43, and a VL comprising the amino acid sequence of SEQ ID NO: 54;

(b) a VH comprising the amino acid sequence of SEQ ID NO: 44, and a VL comprising the amino acid sequence of SEQ ID NO: 55;

(c) a VH comprising the amino acid sequence of SEQ ID NO: 45, and a VL comprising the amino acid sequence of SEQ ID NO: 55;

(d) a VH comprising the amino acid sequence of SEQ ID NO: 46, and a VL comprising the amino acid sequence of SEQ ID NO: 54;

(e) a VH comprising the amino acid sequence of SEQ ID NO: 47, and a VL comprising the amino acid sequence of SEQ ID NO: 54;

(f) a VH comprising the amino acid sequence of SEQ ID NO: 48, and a VL comprising the amino acid sequence of SEQ ID NO: 56;

(g) a VH comprising the amino acid sequence of SEQ ID NO: 49, and a VL comprising the amino acid sequence of SEQ ID NO: 57;

(h) a VH comprising the amino acid sequence of SEQ ID NO: 50, and a VL comprising the amino acid sequence of SEQ ID NO: 58;

(i) a VH comprising the amino acid sequence of SEQ ID NO: 51, and a VL comprising the amino acid sequence of SEQ ID NO: 59;

(j) a VH comprising the amino acid sequence of SEQ ID NO: 51, and a VL comprising the amino acid sequence of SEQ ID NO: 60;

(k) a VH comprising the amino acid sequence of SEQ ID NO: 52, and a VL comprising the amino acid sequence of SEQ ID NO: 60;

(l) a VH comprising the amino acid sequence of SEQ ID NO: 50, and a VL comprising the amino acid sequence of SEQ ID NO: 59; and

(m) a VH comprising the amino acid sequence of SEQ ID NO: 53, and a VL comprising the amino acid sequence of SEQ ID NO: 54.

[2.1] The anticancer agent of any one of [1] to [2], wherein the anti-CD137 antigen-binding molecule has CD137-binding activity dependent on a small molecule compound.

[2.2] An anticancer agent comprising an anti-CD137 antigen-binding molecule which has CD137-binding activity dependent on a small molecule compound, wherein the anti-CD137 antigen-binding molecule competes with the antigen binding molecule of any one of [1] to [2.1] for binding to CD137 in the presence of 10 μM or more, 50 μM or more, 100 μM or more, 150 μM or more, 200 μM or more, or 250 μM or more of the small molecule compound.

[2.3] An anticancer agent comprising an anti-CD137 antigen-binding molecule which has CD137-binding activity dependent on a small molecule compound, wherein the anti-CD137 antigen-binding molecule binds to the same epitope of CD137 bound by the antigen binding molecule of any one of [1] to [2.1] in the presence of 10 μM or more, 50 μM or more, 100 μM or more, 150 μM or more, 200 μM or more, or 250 μM or more of the small molecule compound.

[2.4] The anticancer agent of any one of [2.1] to [2.3], wherein the small molecule compound is an adenosine-containing compound.

[2.5] The anticancer agent of any one of [2.1] to [2.4], wherein the small molecule compound is ATP.

[2.6] The anticancer agent of any one of [1] to [3.4], wherein the anti-CD137 antigen-binding molecule is a monoclonal antibody or an antigen-binding fragment thereof.

[3] The anticancer agent of any one of [1] to [2.6], wherein the anti-CD137 antigen-binding molecule is a human antibody, humanized antibody, or chimeric antibody, or an antigen-binding fragment of any of them.

[3.1] The anticancer agent of any one of [1] to [3], wherein the anti-CD137 antigen-binding molecule is a full-length IgG1 antibody.

[3.2] The anticancer agent of any one of [1] to [3.1], wherein the anti-CD 137 antigen-binding molecule comprises an altered Fc region in which at least one amino acid is altered, wherein the altered Fc region has increased binding activity to FcγRIIb as compared to a parent Fc region which does not comprise the amino acid alteration.

[3.3] The anticancer agent of [3.2], wherein the at least one amino acid alteration is at least one amino acid substitution selected from the group consisting of G236N, H268D, and A330K according to EU numbering.

[3.4] The anticancer agent of [3.2] or [3.3], wherein the at least one amino acid alteration is a combination of amino acid substitutions G236N/H268D/A330K according to EU numbering.

[3.5] The anticancer agent of any one of [1] to [3.4], wherein the anti-CD137 antigen-binding molecule comprises an altered Fc region in which at least one amino acid is altered, wherein the anti-CD137 antigen-binding molecule has an increased isoelectric point (pI) as compared to that of a parent anti-CD137 antigen-binding molecule comprising a parent Fc region that does not comprise the amino acid alteration.

[3.6] The anticancer agent of [3.5], wherein the at least one amino acid alteration is at least one amino acid substitution selected from the group consisting of Q311R, P343R, and D413K according to EU numbering.

[3.7] The anticancer agent of [3.5] or [3.6], wherein the at least one amino acid alteration is (i) amino acid substitution P343R, (ii) a combination of amino acid substitutions Q311R/P343R, or (iii) a combination of amino acid substitutions Q311R/D413K, according to EU numbering.

[4] The anticancer agent of any one of [1] to [3.7], wherein the anti-CD137 antigen-binding molecule comprises an altered Fc region, and the altered Fc region comprises any one combination of amino acid alterations selected from the following according to EU numbering:

L235W/G236N/H268D/Q295L/K326T/A330K/P343R/D413K;
K214R/L235W/G236N/H268D/Q295L/K326T/A330K/P343R/D413K;
L234Y/P238D/T250V/V264I/T307P/A330K/P343R/D413K;
L234Y/P238D/V264I/A330K/P343R/D413K;
L234Y/G237D/P238D/T250V/T307P/A330K/P343R/D413K;
L234Y/G237D/P238D/A330K/P343R/D413K;
L235W/G236N/H268D/Q295L/K326T/A330K/Q311R/P343R;
L234Y/P238D/T250V/V264I/T307P/A330K/Q311R/P343R;
L234Y/P238D/V264I/A330K/Q311R/P343R;
L234Y/G237D/P238D/T250V/T307P/A330K/Q311R/P343R;
L234Y/G237D/P238D/A330K/Q311R/P343R;
L235W/G236N/H268D/Q295L/K326T/A330K/P343R;
K214R/L235W/G236N/H268D/Q295L/K326T/A330K/P343R;
L235W/G236N/H268D/Q295L/K326T/A330K/D413K;
K214R/G236N/H268D/A330K/P343R;
K214R/L235W/G236N/H268D/A330K/P343R;
K214R/G236N/H268D/A330K/D413K;
K214R/G236N/H268D/A330K/P343R/D413K;
K214R/L235W/G236N/H268D/A330K/P343R/D413K;
K214R/G236N/H268D/A330K/Q311R;
K214R/L235W/G236N/H268D/A330K/Q311R;
K214R/G236N/H268D/A330K/Q311R/P343R;
K214R/L235W/G236N/H268D/A330K/Q311R/P343R;
K214R/G236N/H268D/A330K/Q311R/D413K;
K214R/L235W/G236N/H268D/A330K/Q311R/D413K; and
K214R/L235W/G236N/H268D/Q295L/K326T/A330K/Q311R.

[4.1] The anticancer agent of any one of [3.2] to [4], wherein the altered Fc region is derived from a human IgG1 Fc region.

[4.2] The anticancer agent of any one of [3.2] to [4.1], wherein the altered Fc region further comprises a deletion at positions 446 and 447 according to EU numbering.

[4.3] The anticancer agent of any one of [1] to [4.2], wherein the anti-CD 137 antigen-binding molecule comprises a heavy chain constant region comprising any one of the amino acid sequences of SEQ ID NOs: 64 to 85.

[5] An anticancer agent comprising an anti-CD 137 antigen-binding molecule which comprises any combination of VH, VL, CH and CL selected from (i) to (xxxviii) below:

(i) VH comprising the amino acid sequence of SEQ ID NO: 43, CH comprising the amino acid sequence of SEQ ID NO: 64, VL comprising the amino acid sequence of SEQ ID NO: 54, and CL comprising the amino acid sequence of SEQ ID NO: 63;

(ii) VH comprising the amino acid sequence of SEQ ID NO: 43, CH comprising the amino acid sequence of SEQ ID NO: 66, VL comprising the amino acid sequence of SEQ ID NO: 54, and CL comprising the amino acid sequence of SEQ ID NO: 63;

(iii) VH comprising the amino acid sequence of SEQ ID NO: 43, CH comprising the amino acid sequence of SEQ ID NO: 67, VL comprising the amino acid sequence of SEQ ID NO: 54, and CL comprising the amino acid sequence of SEQ ID NO: 63;

(iv) VH comprising the amino acid sequence of SEQ ID NO: 43, CH comprising the amino acid sequence of SEQ ID NO: 68, VL comprising the amino acid sequence of SEQ ID NO: 54, and CL comprising the amino acid sequence of SEQ ID NO: 63;

(v) VH comprising the amino acid sequence of SEQ ID NO: 43, CH comprising the amino acid sequence of SEQ ID NO: 69, VL comprising the amino acid sequence of SEQ ID NO: 54, and CL comprising the amino acid sequence of SEQ ID NO: 63;

(vi) VH comprising the amino acid sequence of SEQ ID NO: 43, CH comprising the amino acid sequence of SEQ ID NO: 70, VL comprising the amino acid sequence of SEQ ID NO: 54, and CL comprising the amino acid sequence of SEQ ID NO: 63;

(vii) VH comprising the amino acid sequence of SEQ ID NO: 43, CH comprising the amino acid sequence of SEQ ID NO: 71, VL comprising the amino acid sequence of SEQ ID NO: 54, and CL comprising the amino acid sequence of SEQ ID NO: 63;

(viii) VH comprising the amino acid sequence of SEQ ID NO: 43, CH comprising the amino acid sequence of SEQ ID NO: 73, VL comprising the amino acid sequence of SEQ ID NO: 54, and CL comprising the amino acid sequence of SEQ ID NO: 63;

(ix) VH comprising the amino acid sequence of SEQ ID NO: 43, CH comprising the amino acid sequence of SEQ ID NO: 75, VL comprising the amino acid sequence of SEQ ID NO: 54, and CL comprising the amino acid sequence of SEQ ID NO: 63;

(x) VH comprising the amino acid sequence of SEQ ID NO: 43, CH comprising the amino acid sequence of SEQ ID NO: 78, VL comprising the amino acid sequence of SEQ ID NO: 54, and CL comprising the amino acid sequence of SEQ ID NO: 63;

(xi) VH comprising the amino acid sequence of SEQ ID NO: 43, CH comprising the amino acid sequence of SEQ ID NO: 80, VL comprising the amino acid sequence of SEQ ID NO: 54, and CL comprising the amino acid sequence of SEQ ID NO: 63;

(xii) VH comprising the amino acid sequence of SEQ ID NO: 43, CH comprising the amino acid sequence of SEQ ID NO: 82, VL comprising the amino acid sequence of SEQ ID NO: 54, and CL comprising the amino acid sequence of SEQ ID NO: 63;

(xiii) VH comprising the amino acid sequence of SEQ ID NO: 43, CH comprising the amino acid sequence of SEQ ID NO: 84, VL comprising the amino acid sequence of SEQ ID NO: 54, and CL comprising the amino acid sequence of SEQ ID NO: 63;

(xiv) VH comprising the amino acid sequence of SEQ ID NO: 43, CH comprising the amino acid sequence of SEQ ID NO: 85, VL comprising the amino acid sequence of SEQ ID NO: 54, and CL comprising the amino acid sequence of SEQ ID NO: 63;

(xv) VH comprising the amino acid sequence of SEQ ID NO: 51, CH comprising the amino acid sequence of SEQ ID NO: 65, VL comprising the amino acid sequence of SEQ ID NO: 59, and CL comprising the amino acid sequence of SEQ ID NO: 63;

(xvi) VH comprising the amino acid sequence of SEQ ID NO: 51, CH comprising the amino acid sequence of SEQ ID NO: 72, VL comprising the amino acid sequence of SEQ ID NO: 59, and CL comprising the amino acid sequence of SEQ ID NO: 63;

(xvii) VH comprising the amino acid sequence of SEQ ID NO: 51, CH comprising the amino acid sequence of SEQ ID NO: 74, VL comprising the amino acid sequence of SEQ ID NO: 59, and CL comprising the amino acid sequence of SEQ ID NO: 63;

(xviii) VH comprising the amino acid sequence of SEQ ID NO: 51, CH comprising the amino acid sequence of SEQ ID NO: 75, VL comprising the amino acid sequence of SEQ ID NO: 59, and CL comprising the amino acid sequence of SEQ ID NO: 63;

(xix) VH comprising the amino acid sequence of SEQ ID NO: 51, CH comprising the amino acid sequence of SEQ ID NO: 77, VL comprising the amino acid sequence of SEQ ID NO: 59, and CL comprising the amino acid sequence of SEQ ID NO: 63;

(xx) VH comprising the amino acid sequence of SEQ ID NO: 51, CH comprising the amino acid sequence of SEQ ID NO: 78, VL comprising the amino acid sequence of SEQ ID NO: 59, and CL comprising the amino acid sequence of SEQ ID NO: 63;

(xxi) VH comprising the amino acid sequence of SEQ ID NO: 51, CH comprising the amino acid sequence of SEQ ID NO: 79, VL comprising the amino acid sequence of SEQ ID NO: 59, and CL comprising the amino acid sequence of SEQ ID NO: 63;

(xxii) VH comprising the amino acid sequence of SEQ ID NO: 51, CH comprising the amino acid sequence of SEQ ID NO: 80, VL comprising the amino acid sequence of SEQ ID NO: 59, and CL comprising the amino acid sequence of SEQ ID NO: 63;

(xxiii) VH comprising the amino acid sequence of SEQ ID NO: 51, CH comprising the amino acid sequence of SEQ ID NO: 81, VL comprising the amino acid sequence of SEQ ID NO: 59, and CL comprising the amino acid sequence of SEQ ID NO: 63;

(xxiv) VH comprising the amino acid sequence of SEQ ID NO: 51, CH comprising the amino acid sequence of SEQ ID NO: 82, VL comprising the amino acid sequence of SEQ ID NO: 59, and CL comprising the amino acid sequence of SEQ ID NO: 63;

(xxv) VH comprising the amino acid sequence of SEQ ID NO: 51, CH comprising the amino acid sequence of SEQ ID NO: 83, VL comprising the amino acid sequence of SEQ ID NO: 59, and CL comprising the amino acid sequence of SEQ ID NO: 63;

(xxvi) VH comprising the amino acid sequence of SEQ ID NO: 51, CH comprising the amino acid sequence of SEQ ID NO: 84, VL comprising the amino acid sequence of SEQ ID NO: 59, and CL comprising the amino acid sequence of SEQ ID NO: 63;

(xxvii) VH comprising the amino acid sequence of SEQ ID NO: 51, CH comprising the amino acid sequence of SEQ ID NO: 72, VL comprising the amino acid sequence of SEQ ID NO: 60, and CL comprising the amino acid sequence of SEQ ID NO: 63;

(xxviii) VH comprising the amino acid sequence of SEQ ID NO: 51, CH comprising the amino acid sequence of SEQ ID NO: 74, VL comprising the amino acid sequence of SEQ ID NO: 60, and CL comprising the amino acid sequence of SEQ ID NO: 63;

(xxix) VH comprising the amino acid sequence of SEQ ID NO: 51, CH comprising the amino acid sequence of SEQ ID NO: 75, VL comprising the amino acid sequence of SEQ ID NO: 60, and CL comprising the amino acid sequence of SEQ ID NO: 63;

(xxx) VH comprising the amino acid sequence of SEQ ID NO: 51, CH comprising the amino acid sequence of SEQ ID NO: 77, VL comprising the amino acid sequence of SEQ ID NO: 60, and CL comprising the amino acid sequence of SEQ ID NO: 63;

(xxxi) VH comprising the amino acid sequence of SEQ ID NO: 51, CH comprising the amino acid sequence of SEQ ID NO: 78, VL comprising the amino acid sequence of SEQ ID NO: 60, and CL comprising the amino acid sequence of SEQ ID NO: 63;

(xxxii) VH comprising the amino acid sequence of SEQ ID NO: 51, CH comprising the amino acid sequence of SEQ ID NO: 79, VL comprising the amino acid sequence of SEQ ID NO: 60, and CL comprising the amino acid sequence of SEQ ID NO: 63;

(xxxiii) VH comprising the amino acid sequence of SEQ ID NO: 51, CH comprising the amino acid sequence of SEQ ID NO: 80, VL comprising the amino acid sequence of SEQ ID NO: 60, and CL comprising the amino acid sequence of SEQ ID NO: 63;

(xxxiv) VH comprising the amino acid sequence of SEQ ID NO: 51, CH comprising the amino acid sequence of SEQ ID NO: 81, VL comprising the amino acid sequence of SEQ ID NO: 60, and CL comprising the amino acid sequence of SEQ ID NO: 63;

(xxxv) VH comprising the amino acid sequence of SEQ ID NO: 51, CH comprising the amino acid sequence of SEQ ID NO: 82, VL comprising the amino acid sequence of SEQ ID NO: 60, and CL comprising the amino acid sequence of SEQ ID NO: 63;

(xxxvi) VH comprising the amino acid sequence of SEQ ID NO: 51, CH comprising the amino acid sequence of SEQ ID NO: 83, VL comprising the amino acid sequence of SEQ ID NO: 60, and CL comprising the amino acid sequence of SEQ ID NO: 63;

(xxxvii) VH comprising the amino acid sequence of SEQ ID NO: 51, CH comprising the amino acid sequence of SEQ ID NO: 84, VL comprising the amino acid sequence of SEQ ID NO: 60, and CL comprising the amino acid sequence of SEQ ID NO: 63; and

(xxxviii) VH comprising the amino acid sequence of SEQ ID NO: 51, CH comprising the amino acid sequence of SEQ ID NO: 85, VL comprising the amino acid sequence of SEQ ID NO: 60, and CL comprising the amino acid sequence of SEQ ID NO: 63.

[6] The anticancer agent of any one of [1] to [5], which is to be administered to a cancer patient having (i) a solid cancer infiltrated with one or more cells selected from a B cell, a dendritic cell, a natural killer cell, a macrophage, and a CD8$^+$ T cell, and/or (ii) a solid cancer infiltrated with a regulatory T (Treg) cell or CD4$^+$ T cell.

[6.1] The anticancer agent of any one of [1] to [6], which is to be administered to a cancer patient having a solid cancer infiltrated with a CD8$^+$ T cell.

[7] The anticancer agent of any one of [1] to [6.1], which is to be administered to a patient having a cancer refractory to treatment with an immune checkpoint inhibitor.

[8] The anticancer agent of any one of [1] to [7], which is to be administered to a cancer patient having one or more cancers selected from the group consisting of gastric cancer, head and neck cancer, esophageal cancer, colorectal cancer, lung cancer, mesothelioma, liver cancer, ovarian cancer, breast cancer, colon cancer, kidney cancer, skin cancer, muscle tumor, pancreatic cancer, prostate cancer, testicular cancer, uterine cancer, cholangiocarcinoma, Merkel cell carcinoma, bladder cancer, thyroid cancer, schwannoma, adrenal cancer, anal cancer, central nervous system tumor, neuroendocrine tissue tumor, penile cancer, pleural tumor, salivary gland tumor, vulvar cancer, thymoma, lymphoma, myeloid leukemia and pediatric cancer.

[8.1] The anticancer agent of any one of [1] to [8], which is to be administered to a cancer patient having one or more cancers selected from the group consisting of gastric cancer, lung cancer, mesothelioma, liver cancer, breast cancer, skin cancer, lymphoma, and myeloid leukemia.

[9] The anticancer agent of any one of [1] to [5] for use in combination with at least one other anticancer agent.

[9.1] The anticancer agent of [9], wherein the anticancer agent is characterized by being administered at the same time as the other anticancer agent.

[9.2] The anticancer agent of [9], wherein the anticancer agent is characterized by being administered before or after administration of the other anticancer agent.

[10] The anticancer agent of any one of [9] to [9.2], wherein the other anticancer agent is at least one anticancer agent selected from a chemotherapeutic agent, a T cell-activating agonist agent, an immune checkpoint inhibitor, a T cell-redirecting antigen-binding molecule, an antifibrotic agent, and an angiogenesis inhibitor.

[10.1] The anticancer agent of [10], wherein the other anticancer agent is a chemotherapeutic agent.

[10.2] The anticancer agent of [10] or [10.1], wherein the other anticancer agent is at least one chemotherapeutic agent selected from an antimetabolite, a plant alkaloid, and a platinum compound.

[10.3] The anticancer agent of [10], wherein the other anticancer agent is a T cell-activating agonist agent.

[10.4] The anticancer agent of [10] or [10.3], wherein the other anticancer agent is an agonistic antibody for TNFRSF.

[11] The anticancer agent of [10], wherein the other anticancer agent is an immune checkpoint inhibitor.

[12] The anticancer agent of [10] or [11], wherein the other anticancer agent is at least one immune checkpoint inhibitor selected from an anti-PDI antibody, an anti-PDLI antibody, an anti-TIGIT antibody, an anti-TIM3 antibody, and an anti-LAG3 antibody.

[12.1] The anticancer agent of any one of [10], [11] and [12], wherein the other anticancer agent is an anti-PDLI antibody and/or an anti-TIGIT antibody.

[12.2] The anticancer agent of any one of [9] to [12.1], which is to be administered to a patient having a cancer refractory to treatment with an immune checkpoint inhibitor.

[12.3] The anticancer agent of [12.2], wherein the cancer refractory to treatment with an immune checkpoint inhibitor is a cancer having a gene mutation in JAK1, JAK2, and/or B2M.

[13] The anticancer agent of [10], wherein the other anticancer agent is a T cell-redirecting antigen-binding molecule.

[13.1] The anticancer agent according to [10] or [13], wherein the other anticancer agent is a multispecific antibody having binding activity to CD3 and a cancer antigen.

[13.2] The anticancer agent of [13.1], wherein the multispecific antibody is a bispecific antibody.

[14] The anticancer agent of any one of [9] to [9.2], wherein the other anticancer agent is an agent that depletes and/or inactivates one or more cells selected from a regulatory T cell, a CD4$^+$ T cell, a B cell, a NK cell, and a macrophage.

[15] The anticancer agent of any one of [9] to [14], which is to be administered to a cancer patient having one or more cancers selected from the group consisting of gastric cancer, head and neck cancer, esophageal cancer, colorectal cancer, lung cancer, mesothelioma, liver cancer, ovarian cancer, breast cancer, colon cancer, kidney cancer, skin cancer, muscle tumor, pancreatic cancer, prostate cancer, testicular cancer, uterine cancer, cholangiocarcinoma, Merkel cell carcinoma, bladder cancer, thyroid cancer, schwannoma, adrenal cancer, anal cancer, central nervous system tumor, neuroendocrine tissue tumor, penile cancer, pleural tumor, salivary gland tumor, vulvar cancer, thymoma, lymphoma, myeloid leukemia, and pediatric cancer.

[15.1] The anticancer agent of any one of [9] to [15], which is to be administered to a cancer patient having one or more cancers selected from the group consisting of gastric cancer, colorectal cancer, lung cancer, mesothelioma, liver cancer, breast cancer, skin cancer, lymphoma, and myeloid leukemia.

[16] The anticancer agent of any one of [1] to [5], which is for use in combination therapy with at least one other anticancer agent for enhancing the activation of T cells in a tumor tissue of an individual.

[16.1] The anticancer agent of [16], wherein when the expression level of at least one gene selected from CD8b1, Gzmb, Prf1, and Ifng is increased in the tumor tissue, it is judged that the activation of T cells in the tumor tissue has been enhanced.

[17] The anticancer agent of any one of [1] to [5], which is for use in combination therapy with at least one other anticancer agent for promoting the proliferation of CD8$^+$ T cells in a tumor tissue of an individual.

[17.1] The anticancer agent of any one of [16] to [17], which is characterized by being administered at the same time as the other anticancer agent.

[17.2] The anticancer agent of any one of [16] to [17], which is characterized by being administered before or after the administration of the other anticancer agent.

[17.3] The anticancer agent of any one of [16] to [17.2], wherein the other anticancer agent is at least one anticancer agent selected from a chemotherapeutic agent, a T cell-activating agonist agent, an immune checkpoint inhibitor, a T cell-redirecting antigen-binding molecule, an antifibrotic agent, and an angiogenesis inhibitor.

[17.4] The anticancer agent of any one of [16] to [17.2], wherein the other anticancer agent is an agent that depletes and/or inactivates one or more cells selected from a regulatory T cell, a CD4$^+$ T cell, a B cell, a NK cell, and a macrophage.

[18] A pharmaceutical composition for treating cancer, which comprises a combination of the anti-CD137 antigen-binding molecule of any one of [1] to [5] and at least one other anticancer agent.

[18.1] The pharmaceutical composition of [18], wherein the anti-CD137 antigen-binding molecule is used so as to be administered to a patient at the same time as the other anticancer agent.

[18.2] The pharmaceutical composition of [18], wherein the anti-CD137 antigen-binding molecule is used so as to be administered to a patient before or after administration of the other anticancer agent.

[19] The pharmaceutical composition of any one of [18] to [18.2], wherein the other anticancer agent is at least one anticancer agent selected from a chemotherapeutic agent, a T cell-activating agonist agent, an immune checkpoint inhibitor, a T cell-redirecting antigen-binding molecule, an antifibrotic agent, and an angiogenesis inhibitor.

[19.1] The pharmaceutical composition of [19], wherein the other anticancer agent is a chemotherapeutic agent.

[19.2] The pharmaceutical composition of [19] or [19.1], wherein the other anticancer agent is at least one chemotherapeutic agent selected from an antimetabolite, a plant alkaloid, and a platinum compound.

[19.3] The pharmaceutical composition of [19], wherein the other anticancer agent is a T cell-activating agonist agent.

[19.4] The pharmaceutical composition of [19] or [19.3], wherein the other anticancer agent is an agonistic antibody for TNFRSF.

[20] The pharmaceutical composition of [19], wherein the other anticancer agent is an immune checkpoint inhibitor.

[21] The pharmaceutical composition of [19] or [20], wherein the other anticancer agent is at least one immune checkpoint inhibitor selected from an anti-PDI antibody, an anti-PDLI antibody, an anti-TIGIT antibody, an anti-TIM3 antibody, and an anti-LAG3 antibody.

[21.1] The pharmaceutical composition of any one of [19], [20], and [21], wherein the other anticancer agent is an anti-PDLI antibody and/or an anti-TIGIT antibody.

[21.2] The pharmaceutical composition of any one of [18] to [21.1], which is for the treatment of a cancer refractory to treatment with an immune checkpoint inhibitor.

[21.3] The pharmaceutical composition of [21.2], wherein the cancer refractory to treatment with an immune checkpoint inhibitor is a cancer having a gene mutation in JAK1, JAK2, and/or B2M.

[22] The pharmaceutical composition of [19], wherein the other anticancer agent is a T cell-redirecting antigen-binding molecule.

[22.1] The pharmaceutical composition of [19] or [22], wherein the other anticancer agent is a multispecific antibody having binding activity to CD3 and a cancer antigen.

[22.2] The pharmaceutical composition of [22.1], wherein the multispecific antibody is a bispecific antibody.

[23] The pharmaceutical composition of any one of [18] to [18.2], wherein the other anticancer agent is an agent that depletes and/or inactivates one or more cells selected from a regulatory T cell, a CD4$^+$ T cell, a B cell, a NK cell, and a macrophage.

[24] The pharmaceutical composition of any one of [18] to [23], wherein the cancer is one or more cancers selected from the group consisting of gastric cancer, head and neck cancer, esophageal cancer, colorectal cancer, lung cancer, mesothelioma, liver cancer, ovarian cancer, breast cancer, colon cancer, kidney cancer, skin cancer, muscle tumor, pancreatic cancer, prostate cancer, testicular cancer, uterine cancer, cholangiocarcinoma, Merkel cell carcinoma, bladder cancer, thyroid cancer, schwannoma, adrenal cancer, anal cancer, central nervous system tumor, neuroendocrine tissue tumor, penile cancer, pleural tumor, salivary gland tumor, vulvar cancer, thymoma, lymphoma, myeloid leukemia, and pediatric cancer.

[24.1] The pharmaceutical composition of any one of [18] to [24], wherein the cancer is one or more cancers selected from the group consisting of gastric cancer, colorectal cancer, lung cancer, mesothelioma, liver cancer, breast cancer, skin cancer, lymphoma, and myeloid leukemia.

[25] A combination of the anti-CD 137 antigen-binding molecule of any one of [1] to [5] and at least one other anticancer agent for treating cancer.

[25.1] The combination of [25], wherein the anti-CD 137 antigen-binding molecule is used so as to be administered to a patient at the same time as the other anticancer agent.

[25.2] The combination of [25], wherein the anti-CD 137 antigen-binding molecule is used so as to be administered to a patient before or after administration of the other anticancer agent.

[26] The combination of any one of [25] to [25.2], wherein the other anticancer agent is at least one anticancer agent selected from a chemotherapeutic agent, a T cell-activating agonist agent, an immune checkpoint inhibitor, a T cell-redirecting antigen-binding molecule, an antifibrotic agent, and an angiogenesis inhibitor.

[26.1] The combination of [26], wherein the other anticancer agent is a chemotherapeutic agent.

[26.2] The combination of [26] or [26.1], wherein the other anticancer agent is at least one chemotherapeutic agent selected from an antimetabolite, a plant alkaloid, and a platinum compound.

[26.3] The combination of [26], wherein the other anticancer agent is a T cell-activating agonist agent.

[26.4] The combination of [26] or [26.3], wherein the other anticancer agent is an agonistic antibody for TNFRSF.

[27] The combination of [26], wherein the other anticancer agent is an immune checkpoint inhibitor.

[28] The combination of [26] or [27], wherein the other anticancer agent is at least one immune checkpoint inhibitor selected from an anti-PDI antibody, an anti-PDLI antibody, an anti-TIGIT antibody, an anti-TIM3 antibody, and an anti-LAG3 antibody.

[28.1] The combination of any one of [26], [27] and [28], wherein the other anticancer agent is an anti-PDLI antibody and/or an anti-TIGIT antibody.

[28.2] The combination of any one of [25] to [28.1], which is for the treatment of a cancer refractory to treatment with an immune checkpoint inhibitor.

[28.3] The combination of [28.2], wherein the cancer refractory to treatment with an immune checkpoint inhibitor is a cancer having a gene mutation in JAK1, JAK2, and/or B2M.

[29] The combination of [26], wherein the other anticancer agent is a T cell-redirecting antigen-binding molecule.

[29.1] The combination of [26] or [29], wherein the other anticancer agent is a multispecific antibody having binding activity to CD3 and a cancer antigen.

[29.2] The combination of [29.1], wherein the multispecific antibody is a bispecific antibody.

[30] The combination of any one of [25] to [25.2], wherein the other anticancer agent is an agent that depletes and/or inactivates one or more cells selected from a regulatory T cell, a CD4$^+$ T cell, a B cell, a NK cell, and a macrophage.

[31] The combination of any one of [25] to [30], wherein the cancer is one or more cancers selected from the group consisting of gastric cancer, head and neck cancer, esophageal cancer, colorectal cancer, lung cancer, mesothelioma, liver cancer, ovarian cancer, breast cancer, colon cancer, kidney cancer, skin cancer, muscle tumor, pancreatic cancer, prostate cancer, testicular cancer, uterine cancer, cholangiocarcinoma, Merkel cell carcinoma, bladder cancer, thyroid cancer, schwannoma, adrenal cancer, anal cancer, central nervous system tumor, neuroendocrine tissue tumor, penile cancer, pleural tumor, salivary gland tumor, vulvar cancer, thymoma, lymphoma, myeloid leukemia, and pediatric cancer.

[31.1] The combination of any one of [25] to [31], wherein the cancer is one or more cancers selected from the group consisting of gastric cancer, colorectal cancer, lung cancer, mesothelioma, liver cancer, breast cancer, skin cancer, lymphoma, and myeloid leukemia.

[32] Use of a combination of the anti-CD137 antigen-binding molecule of any one of [1] to [5] and at least one other anticancer agent as an anticancer agent.

[32.1] The use of [32], wherein the anti-CD137 antigen-binding molecule is used so as to be administered to a patient at the same time as the other anticancer agent.

[32.2] The use of [32], wherein the anti-CD137 antigen-binding molecule is used so as to be administered to a patient before or after administration of the other anticancer agent.

[33] The use of any one of [32] to [32.2], wherein the other anticancer agent is at least one anticancer agent selected from a chemotherapeutic agent, a T cell-activating agonist agent, an immune checkpoint inhibitor, a T cell-redirecting antigen-binding molecule, an antifibrotic agent, and an angiogenesis inhibitor.

[33.1] The use of [33], wherein the other anticancer agent is a chemotherapeutic agent.

[33.2] The use of [33] or [33.1], wherein the other anticancer agent is at least one chemotherapeutic agent selected from an antimetabolite, a plant alkaloid, and a platinum compound.

[33.3] The use of [33], wherein the other anticancer agent is a T cell- activating agonist agent.

[33.4] The use of [33] or [33.3], wherein the other anticancer agent is an agonistic antibody for TNFRSF.

[34] The use of [33], wherein the other anticancer agent is an immune checkpoint inhibitor.

[35] The use of [33] or [34], wherein the other anticancer agent is at least one immune checkpoint inhibitor selected from an anti-PDI antibody, an anti-PDLI antibody, an anti-TIGIT antibody, an anti-TIM3 antibody, and an anti-LAG3 antibody.

[35.1] The combination of any one of [33], [34], and [35], wherein the other anticancer agent is an anti-PDLI antibody and/or an anti-TIGIT antibody.

[35.2] The use of any one of [32] to [35.1], which is for the treatment of a cancer refractory to treatment with an immune checkpoint inhibitor.

[35.3] The use of [35.2], wherein the cancer refractory to treatment with an immune checkpoint inhibitor is a cancer having a gene mutation in JAK1, JAK2, and/or B2M.

[36] The use of [33], wherein the other anticancer agent is a T cell-redirecting antigen-binding molecule.

[36.1] The use of [33] or [36], wherein the other anticancer agent is a multispecific antibody having binding activity to CD3 and a cancer antigen.

[36.2] The use of [36.1], wherein the multispecific antibody is a bispecific antibody.

[37] The use of any one of [32] to [32.2], wherein the other anticancer agent is an agent that depletes and/or inactivates one or more cells selected from a regulatory T cell, a CD4$^+$ T cell, a B cell, a NK cell, and a macrophage.

[38] The use of any one of [32] to [37], wherein the cancer is one or more cancers selected from the group consisting of gastric cancer, head and neck cancer, esophageal cancer, colorectal cancer, lung cancer, mesothelioma, liver cancer, ovarian cancer, breast cancer, colon cancer, kidney cancer, skin cancer, muscle tumor, pancreatic cancer, prostate cancer, testicular cancer, uterine cancer, cholangiocarcinoma, Merkel cell carcinoma, bladder cancer, thyroid cancer, schwannoma, adrenal cancer, anal cancer, central nervous system tumor, neuroendocrine tissue tumor, penile cancer, pleural tumor, salivary gland tumor, vulvar cancer, thymoma, lymphoma, myeloid leukemia, and pediatric cancer.

[38.1] The use of any one of [32] to [38], wherein the cancer is one or more cancers selected from the group consisting of gastric cancer, colorectal cancer, lung cancer, mesothelioma, liver cancer, breast cancer, skin cancer, lymphoma, and myeloid leukemia.

[39] A kit for the treatment of cancer, comprising:

(1) a pharmaceutical composition comprising the anti-CD 137 antigen-binding molecule of any one of [1] to [5] as an active ingredient; and
(2) a package insert or label instructing administration of at least one other anticancer agent before, at the same time as, or after the administration of the pharmaceutical composition.

[39.1] The kit of [39], wherein the pharmaceutical composition is filled in a container.

[39.2] The kit of any one of [39] to [39.2], wherein the pharmaceutical composition comprising the anti-CD137 antigen-binding molecule as an active ingredient is used so as to be administered to a patient at the same time as the other anticancer agent.

[39.3] The kit according to any one of [39] or [39.1], wherein the pharmaceutical composition comprising the anti-CD137 antigen-binding molecule as an active ingredient is used so as to be administered to a patient before or after administration of the other anticancer agent.

[40] The kit of any one of [39] to [39.2], wherein the other anticancer agent is at least one anticancer agent selected from a chemotherapeutic agent, a T cell-activating agonist agent, an immune checkpoint inhibitor, a T cell-redirecting antigen-binding molecule, an antifibrotic agent, and an angiogenesis inhibitor.

[40.1] The kit of [40], wherein the other anticancer agent is a chemotherapeutic agent.

[40.2] The kit of [40] or [40.1], wherein the other anticancer agent is at least one chemotherapeutic agent selected from an antimetabolite, a plant alkaloid, and a platinum compound.

[40.3] The kit of [40], wherein the other anticancer agent is a T cell-activating agonist agent.

[40.4] The kit of [40] or [40.3], wherein the other anticancer agent is an agonistic antibody for TNFRSF.

[41] The kit of [40], wherein the anticancer agent is an immune checkpoint inhibitor.

[42] The kit of [40] or [41], wherein the anticancer agent is at least one immune checkpoint inhibitor selected from an anti-PDI antibody, an anti-PDLI antibody, an anti-TIGIT antibody, an anti-TIM3 antibody, and an anti-LAG3 antibody.

[42.1] The kit of any one of [40], [41], and [42], wherein the anticancer agent is an anti-PDLI antibody and/or an anti-TIGIT antibody.

[42.2] The kit of any one of [39] to [42.1], which is for the treatment of a cancer refractory to treatment with an immune checkpoint inhibitor.

[42.3] The kit of [42.2], wherein the cancer refractory to treatment with an immune checkpoint inhibitor is a cancer having a gene mutation in JAK1, JAK2, and/or B2M.

[43] The kit of [40], wherein the anticancer agent is a T cell-redirecting antigen-binding molecule.

[43.1] The kit of [40] or [43], wherein the anticancer agent is a multispecific antibody having binding activity to CD3 and a cancer antigen.

[43.2] The kit of [43.1], wherein the multispecific antibody is a bispecific antibody.

[44] The kit of any one of [39] to [39.2], wherein the other anticancer agent is an agent that depletes and/or inactivates one or more cells selected from a regulatory T cell, a CD4$^+$ T cell, a B cell, a NK cell, and a macrophage.

[45] The kit of any one of [39] to [44], wherein the cancer is one or more cancers selected from the group consisting of gastric cancer, head and neck cancer, esophageal cancer, colorectal cancer, lung cancer, mesothelioma, liver cancer, ovarian cancer, breast cancer, colon cancer, kidney cancer, skin cancer, muscle tumor, pancreatic cancer, prostate cancer, testicular cancer, uterine cancer, cholangiocarcinoma, Merkel cell carcinoma, bladder cancer, thyroid cancer, schwannoma, adrenal cancer, anal cancer, central nervous system tumor, neuroendocrine tissue tumor, penile cancer, pleural tumor, salivary gland tumor, vulvar cancer, thymoma, lymphoma, myeloid leukemia, and pediatric cancer.

[45.1] The kit of any one of [39] to [44], wherein the cancer is one or more cancers selected from the group consisting of gastric cancer, colorectal cancer, lung cancer, mesothelioma, liver cancer, breast cancer, skin cancer, lymphoma, and myeloid leukemia.

[46] A method for treating a cancer, comprising administering the anti-CD 137 antigen-binding molecule of any one of [1] to [5] to a patient having (i) a solid cancer infiltrated with one or more cells selected from a B cell, a dendritic cell, a natural killer cell, a macrophage, and a CD8$^+$ T cell, and/or (ii) a solid cancer infiltrated with a regulatory T (Treg) cell or a CD4$^+$ T cell.

[46.1] The method of [46], wherein the patient is a patient having a solid cancer infiltrated with a CD8$^+$ T cell.

[47] The method of [46] or [46.1], wherein the patient has a cancer refractory to treatment with an immune checkpoint inhibitor.

[48] The method of any one of [46] to [47], wherein the patient has one or more cancers selected from the group consisting of gastric cancer, head and neck cancer, esophageal cancer, colorectal cancer, lung cancer, mesothelioma, liver cancer, ovarian cancer, breast cancer, colon cancer, kidney cancer, skin cancer, muscle tumor, pancreatic cancer, prostate cancer, testicular cancer, uterine cancer, cholangiocarcinoma, Merkel cell carcinoma, bladder cancer, thyroid cancer, schwannoma, adrenal cancer, anal cancer, central nervous system tumor, neuroendocrine tissue tumor, penile cancer, pleural tumor, salivary gland tumor, vulvar cancer, thymoma, lymphoma, myeloid leukemia and pediatric cancer.

[48.1] The method of any one of [46] to [48], wherein the patient is a patient having one or more cancers selected from the group consisting of gastric cancer, lung cancer, mesothelioma, liver cancer, breast cancer, skin cancer, lymphoma, and myeloid leukemia.

[49] A method for treating cancer, which comprises administering the anti-CD137 antigen-binding molecule of any one of [1] to [5] to a patient in combination with at least one other anticancer agent.

[49.1] The method of [49], wherein the anti-CD 137 antigen-binding molecule is administered to a patient at the same time as the other anticancer agent.

[49.2] The method according to [49], wherein the anti-CD 137 antigen-binding molecule is administered to a patient before or after administration of the other anticancer agent.

[50] The method of any one of [49] to [49.2], wherein the other anticancer agent is at least one anticancer agent selected from a chemotherapeutic agent, a T cell-activating agonist agent, an immune checkpoint inhibitor, a T cell-redirecting antigen-binding molecule, an antifibrotic agent, and an angiogenesis inhibitor.

[50.1] The method of [50], wherein the other anticancer agent is a chemotherapeutic agent.

[50.2] The method of [50] or [50.1], wherein the other anticancer agent is at least one chemotherapeutic agent selected from an antimetabolite, a plant alkaloid, and a platinum compound.

[50.3] The method of [50], wherein the other anticancer agent is a T cell-activating agonist agent.

[50.4] The method of [50] or [50.3], wherein the other anticancer agent is an agonistic antibody for TNFRSF.

[51] The method of [50], wherein the other anticancer agent is an immune checkpoint inhibitor.

[52] The method of [50] or [51], wherein the other anticancer agent is at least one immune checkpoint inhibitor selected from an anti-PDI antibody, an anti-PDLI antibody, an anti-TIGIT antibody, an anti-TIM3 antibody, and an anti-LAG3 antibody.

[52.1] The method of any one of [50], [51], and [52], wherein the other anticancer agent is an anti-PDLI antibody and/or an anti-TIGIT antibody.

[52.2] The method of any one of [49] to [52.1] for the treatment of a cancer refractory to treatment with an immune checkpoint inhibitor.

[52.3] The method of [52.2], wherein the cancer refractory to treatment with an immune checkpoint inhibitor is a cancer having a gene mutation in JAK1, JAK2, and/or B2M.

[53] The method of [50], wherein the other anticancer agent is a T cell-redirecting antigen-binding molecule.

[53.1] The method of [50] or [53], wherein the other anticancer agent is a multispecific antibody having binding activity to CD3 and a cancer antigen.

[53.2] The method of [53.1], wherein the multispecific antibody is a bispecific antibody.

[54] The method of any one of [49] to [49.2], wherein the other anticancer agent is an agent that depletes and/or inactivates one or more cells selected from a regulatory T cell, a CD4$^+$ T cell, a B cell, a NK cell, and a macrophage.

[55] The method of any one of [49] to [54], wherein the cancer is one or more cancers selected from the group consisting of gastric cancer, head and neck cancer, esophageal cancer, colorectal cancer, lung cancer, mesothelioma, liver cancer, ovarian cancer, breast cancer, colon cancer, kidney cancer, skin cancer, muscle tumor, pancreatic cancer, prostate cancer, testicular cancer, uterine cancer, cholangiocarcinoma, Merkel cell carcinoma, bladder cancer, thyroid cancer, schwannoma, adrenal cancer, anal cancer, central nervous system tumor, neuroendocrine tissue tumor, penile cancer, pleural tumor, salivary gland tumor, vulvar cancer, thymoma, lymphoma, myeloid leukemia, and pediatric cancer.

[55.1] The method of any one of [49] to [55], wherein the cancer is one or more cancers selected from the group consisting of gastric cancer, colorectal cancer, lung cancer, mesothelioma, liver cancer, breast cancer, skin cancer, lymphoma, and myeloid leukemia.

[56] The anti-CD 137 antigen-binding molecule according to any one of [1] to [5], which is for use in the treatment of:

(i) a solid cancer infiltrated with one or more cells selected from a B cell, a dendritic cell, a natural killer cell, a macrophage, and a CD8$^+$ T cell, and/or
(ii) a solid cancer infiltrated with a regulatory T (Treg) cell or a CD4$^+$ T cell.

[56.1] The anti-CD 137 antigen-binding molecule of [56], wherein the cancer is a solid cancer infiltrated with a CD8$^+$ T cell.

[57] The anti-CD 137 antigen-binding molecule of [56] or [56.1], wherein the cancer is a cancer refractory to treatment with an immune checkpoint inhibitor.

[58] The anti-CD137 antigen-binding molecule of any one of [56] to [57], wherein the cancer is one or more cancers selected from the group consisting of gastric cancer, head and neck cancer, esophageal cancer, colorectal cancer, lung cancer, mesothelioma, liver cancer, ovarian cancer, breast cancer, colon cancer, kidney cancer, skin cancer, muscle tumor, pancreatic cancer, prostate cancer, testicular cancer, uterine cancer, cholangiocarcinoma, Merkel cell carcinoma, bladder cancer, thyroid cancer, schwannoma, adrenal cancer, anal cancer, central nervous system tumor, neuroendocrine tissue tumor, penile cancer, pleural tumor, salivary gland tumor, vulvar cancer, thymoma, lymphoma, myeloid leukemia, and pediatric cancer.

[58.1] The anti-CD137 antigen-binding molecule of any one of [56] to [58], wherein the cancer is one or more cancers selected from the group consisting of gastric cancer, lung cancer, mesothelioma, liver cancer, breast cancer, skin cancer, lymphoma, and myeloid leukemia.

[59] The anti-CD 137 antigen-binding molecule of any one of [1] to [5], which is for use in the treatment of cancer in combination with at least one other anticancer agent.

[59.1] The anti-CD 137 antigen-binding molecule of [59], wherein the treatment is characterized by administering the anti-CD137 antigen-binding molecule at the same time as the other anticancer agent.

[59.2] The anti-CD 137 antigen-binding molecule of [59], wherein the treatment is characterized by administering the anti-CD 137 antigen-binding molecule before or after administration of the other anticancer agent.

[60] The anti-CD137 antigen-binding molecule of any one of [59] to [59.2], wherein the other anticancer agent is at least one anticancer agent selected from a chemotherapeutic agent, a T cell-activating agonist agent, an immune checkpoint inhibitor, a T cell-redirecting antigen-binding molecule, an antifibrotic agent, and an angiogenesis inhibitor.

[60.1] The anti-CD 137 antigen-binding molecule of [60], wherein the other anticancer agent is a chemotherapeutic agent.

[60.2] The anti-CD 137 antigen-binding molecule of [60] or [60.1], wherein the other anticancer agent is at least one chemotherapeutic agent selected from an antimetabolite, a plant alkaloid, and a platinum compound.

[60.3] The anti-CD 137 antigen-binding molecule of [60], wherein the other anticancer agent is a T cell-activating agonist agent.

[60.4] The anti-CD 137 antigen-binding molecule of [60] or [60.3], wherein the other anticancer agent is an agonistic antibody for TNFRSF.

[61] The anti-CD 137 antigen-binding molecule of [60], wherein the other anticancer agent is an immune checkpoint inhibitor.

[62] The anti-CD 137 antigen-binding molecule of [60] or [61], wherein the other anticancer agent is at least one immune checkpoint inhibitor selected from an anti-PDI antibody, an anti-PDL1 antibody, an anti-TIGIT antibody, an anti-TIM3 antibody, and an anti-LAG3 antibody.

[62.1] The anti-CD137 antigen-binding molecule of any one of [60], [61], and [62], wherein the other anticancer agent is an anti-PDLI antibody and/or an anti-TIGIT antibody.

[62.2] The anti-CD137 antigen-binding molecule of any one of [59] to [62.1], which is for use in the treatment of a cancer refractory to treatment with an immune checkpoint inhibitor.

[62.3] The anti-CD137 antigen-binding molecule of [62.2], wherein the cancer refractory to treatment with an immune checkpoint inhibitor is a cancer having a gene mutation in JAK1, JAK2, and/or B2M.

[63] The anti-CD 137 antigen-binding molecule of [60], wherein the other anticancer agent is a T cell-redirecting antigen-binding molecule.

[63.1] The anti-CD 137 antigen-binding molecule of [60] or [63], wherein the other anticancer agent is a multispecific antibody having binding activity to CD3 and a cancer antigen.

[63.2] The anti-CD137 antigen-binding molecule of [63.1], wherein the multispecific antibody is a bispecific antibody.

[64] The anti-CD137 antigen-binding molecule of any one of [59] to [59.2], wherein the other anticancer agent is an agent that depletes and/or inactivates one or more cells selected from a regulatory T cell, a CD4$^+$ T cell, a B cell, a NK cell, and a macrophage.

[65] The anti-CD137 antigen-binding molecule of any one of [59] to [64], wherein the cancer is one or more cancers selected from the group consisting of gastric cancer, head and neck cancer, esophageal cancer, colorectal cancer, lung cancer, mesothelioma, liver cancer, ovarian cancer, breast cancer, colon cancer, kidney cancer, skin cancer, muscle tumor, pancreatic cancer, prostate cancer, testicular cancer, uterine cancer, cholangiocarcinoma, Merkel cell carcinoma, bladder cancer, thyroid cancer, schwannoma, adrenal cancer, anal cancer, central nervous system tumor, neuroendocrine tissue tumor, penile cancer, pleural tumor, salivary gland tumor, vulvar cancer, thymoma, lymphoma, myeloid leukemia, and pediatric cancer.

[65.1] The anti-CD137 antigen-binding molecule of any one of [59] to [65], wherein the cancer is one or more cancers selected from the group consisting of gastric cancer, colorectal cancer, lung cancer, mesothelioma, liver cancer, breast cancer, skin cancer, lymphoma, and myeloid leukemia.

[66] Use of the anti-CD137 antigen-binding molecule of any one of [1] to [5] for the manufacture of a medicament for treating cancer, wherein the cancer is

(i) a solid cancer infiltrated with one or more cells selected from a B cell, a dendritic cell, a natural killer cell, a macrophage, and a CD8$^+$ T cell, and/or
(ii) a solid cancer infiltrated with a regulatory T (Treg) cell or a CD4$^+$ T cell.

[66.1] The use of [66], wherein the cancer is a solid cancer infiltrated with a CD8$^+$ T cell.

[67] The use of [66] or [66.1], wherein the cancer is a cancer refractory to treatment with an immune checkpoint inhibitor.

[68] The use of any one of [66] to [67], wherein the cancer is one or more cancers selected from the group consisting of gastric cancer, head and neck cancer, esophageal cancer, colorectal cancer, lung cancer, mesothelioma, liver cancer, ovarian cancer, breast cancer, colon cancer, kidney cancer, skin cancer, muscle tumor, pancreatic cancer, prostate cancer, testicular cancer, uterine cancer, cholangiocarcinoma, Merkel cell carcinoma, bladder cancer, thyroid cancer, schwannoma, adrenal cancer, anal cancer, central nervous system tumor, neuroendocrine tissue tumor, penile cancer, pleural tumor, salivary gland tumor, vulvar cancer, thymoma, lymphoma, myeloid leukemia, and pediatric cancer.

[68.1] The use of any one of [66] to [68], wherein the cancer is one or more cancers selected from the group consisting of gastric cancer, lung cancer, mesothelioma, liver cancer, breast cancer, skin cancer, lymphoma, and myeloid leukemia.

[69] Use of the anti-CD 137 antigen-binding molecule of any one of [1] to [5] in the manufacture of a medicament for treating cancer, wherein the medicament is characterized by being used in combination with at least one other anticancer agent.

[69.1] The use of [69], wherein the medicament is characterized by being administered at the same time as the other anticancer agent.

[69.2] The use of [69], wherein the medicament is characterized by being administered before or after administration of the other anticancer agent.

[70] The use of any one of [69] to [69.2], wherein the other anticancer agent is at least one anticancer agent selected from a chemotherapeutic agent, a T cell-activating agonist agent, an immune checkpoint inhibitor, a T cell-redirecting antigen-binding molecule, an antifibrotic agent, and an angiogenesis inhibitor.

[70.1] The use of [70], wherein the other anticancer agent is a chemotherapeutic agent.

[70.2] The use of [70] or [70.1], wherein the other anticancer agent is at least one chemotherapeutic agent selected from an antimetabolite, a plant alkaloid, and a platinum compound.

[70.3] The use of [70], wherein the other anticancer agent is a T cell-activating agonist agent.

[70.4] The use of [70] or [70.3], wherein the other anticancer agent is an agonistic antibody for TNFRSF.

[71] The use of [70], wherein the other anticancer agent is an immune checkpoint inhibitor.

[72] The use of [70] or [71], wherein the other anticancer agent is at least one immune checkpoint inhibitor selected from an anti-PDI antibody, an anti-PDLI antibody, an anti-TIGIT antibody, an anti-TIM3 antibody, and an anti-LAG3 antibody.

[72.1] The use of any one of [70], [71], and [72], wherein the other anticancer agent is an anti-PDL1 antibody and/or an anti-TIGIT antibody.

[72.2] The use of any one of [69] to [72.1] for use in the treatment of a cancer refractory to treatment with an immune checkpoint inhibitor.

[72.3] The use of [72.2], wherein the cancer refractory to treatment with an immune checkpoint inhibitor is a cancer having a gene mutation in JAK1, JAK2, and/or B2M.

[73] The use of [70], wherein the other anticancer agent is a T cell-redirecting antigen-binding molecule.

[73.1] The use of [70] or [73], wherein the other anticancer agent is a multispecific antibody having binding activity to CD3 and a cancer antigen.

[73.2] The use of [73.1], wherein the multispecific antibody is a bispecific antibody.

[74] The use of any one of [69] to [69.2], wherein the other anticancer agent is an agent that depletes and/or inactivates one or more cells selected from a regulatory T cell, a CD4$^+$ T cell, a B cell, a NK cell, and a macrophage.

[75] The use of any one of [69] to [74], wherein the cancer is one or more cancers selected from the group consisting of gastric cancer, head and neck cancer, esophageal cancer, colorectal cancer, lung cancer, mesothelioma, liver cancer, ovarian cancer, breast cancer, colon cancer, kidney cancer, skin cancer, muscle tumor, pancreatic cancer, prostate cancer, testicular cancer, uterine cancer, cholangiocarcinoma, Merkel cell carcinoma, bladder cancer, thyroid cancer, schwannoma, adrenal cancer, anal cancer, central nervous system tumor, neuroendocrine tissue tumor, penile cancer, pleural tumor, salivary gland tumor, vulvar cancer, thymoma, lymphoma, myeloid leukemia, and pediatric cancer.

[75.1] The use of any one of [69] to [75], wherein the cancer is one or more cancers selected from the group consisting of gastric cancer, colorectal cancer, lung cancer, mesothelioma, liver cancer, breast cancer, skin cancer, lymphoma, and myeloid leukemia.

[Brief Description of the Drawings]

Fig. 1 shows the antitumor effect of A551-MB110/B379-ml0r in a mouse model transplanted with LLC1/OVA/GPC3 clone C5 cells.
Each point shows the mean value for the tumor volume of one group (n = 7).
Fig. 2 shows the gene expression level in a tumor sample by the administration of A551-MB110/B379-ml0r in a mouse model transplanted with LLC1/OVA/GPC3 clone C5 cells.
Subfigure (A) shows the expression level of CD8b1, Subfigure (B) shows the expression level of Gzmb, Subfigure (C) shows the expression level of Prf1, and Subfigure (D) shows the expression level of Ifng.
Fig. 3 shows the degree of activation of CD8$^+$ T cells in a tumor tissue by the administration of A551-MB110/B379-ml0r in a mouse model transplanted with LLC1/OVA/GPC3 clone C5 cells.
Subfigure (A) shows the proportion of OVA tetramer$^+$ T cells among CD8$^+$ T cells. Subfigure (B) shows the proportion of Granzyme B$^+$ T cells among CD8$^+$ T cells. Subfigure (C) shows the proportion of PD-1$^+$ T cells among CD8$^+$ T cells. Subfigure (D) shows the proportion of KLRG-1$^+$ T cells among CD8$^+$ T cells. Subfigure (E) shows the proportion of ICOS$^+$ T cells among CD8$^+$ T cells.

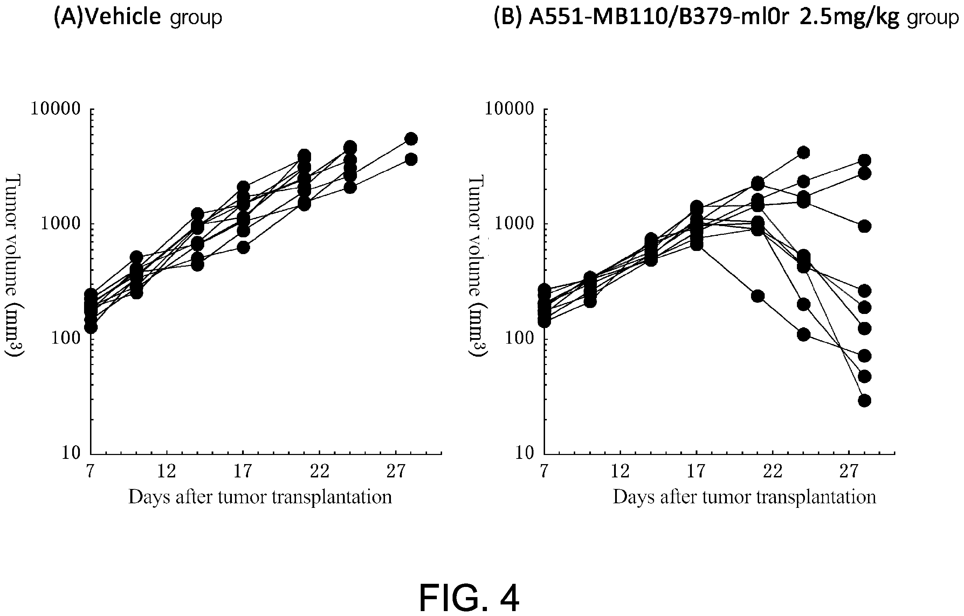

Fig. 4 shows the antitumor effect of A551-MB110/B379-ml0r in a mouse model transplanted with E.G7-OVA cells.
Subfigure (A) shows the change in tumor volume of each mouse in the vehicle-administered group. Subfigure (B) shows the change in tumor volume of each mouse in the A551-MB110/B379-ml0r (2.5 mg/kg) administration group.

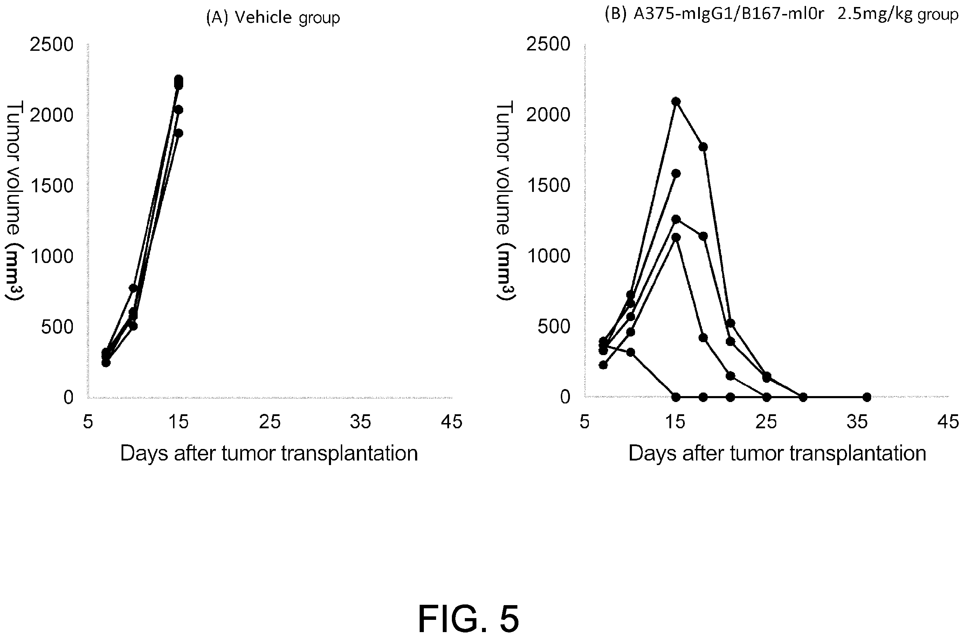

Fig. 5 shows the antitumor effect of A375-mIgG1/B167-ml0r in a mouse model transplanted with E.G7-OVA cells.
Subfigure (A) shows the change in tumor volume of each mouse in the vehicle-administered group. Subfigure (B) shows the change in tumor volume of each mouse in the A375-mIgG1/B167-ml0r (2.5 mg/kg) administration group.

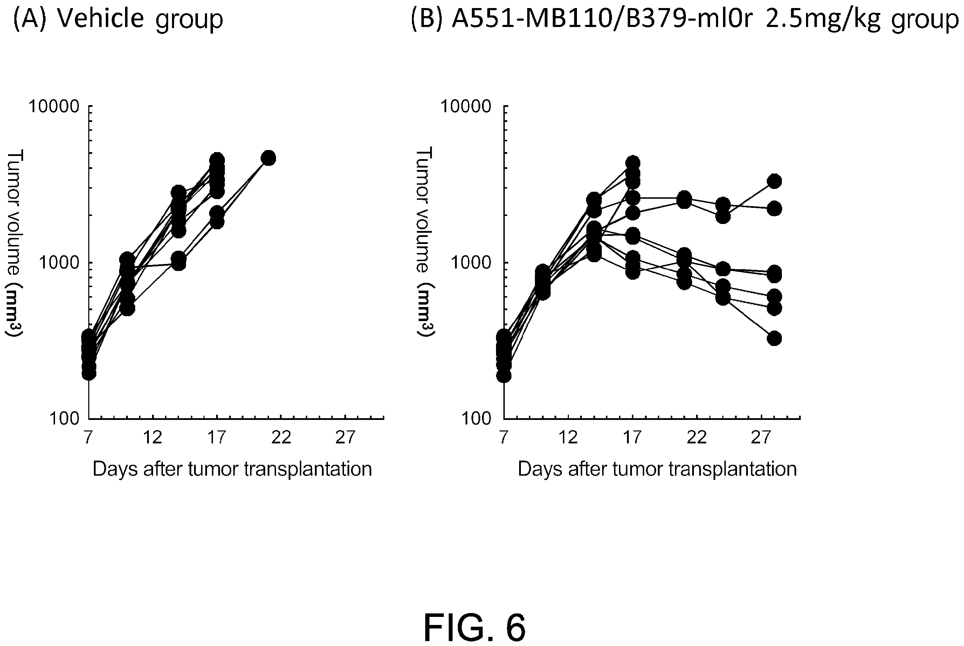

Fig. 6 shows the antitumor effect of A551-MB110/B379-ml0r in a mouse model transplanted with C1498 cells.
Subfigure (A) shows the change in tumor volume of each mouse in the vehicle-administered group. Subfigure (B) shows the change in tumor volume of each mouse in the A551-MB110/B379-ml0r (2.5 mg/kg) administration group.

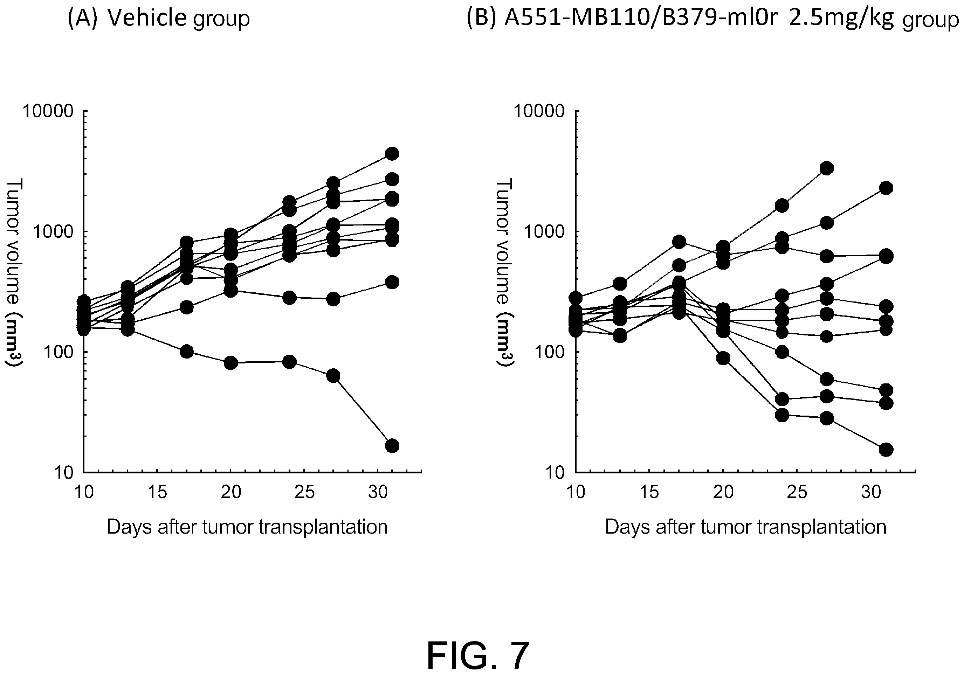

Fig. 7 shows the antitumor effect of A551-MB110/B379-ml0r in a mouse model transplanted with Hepa1-6/hGPC3 cells.

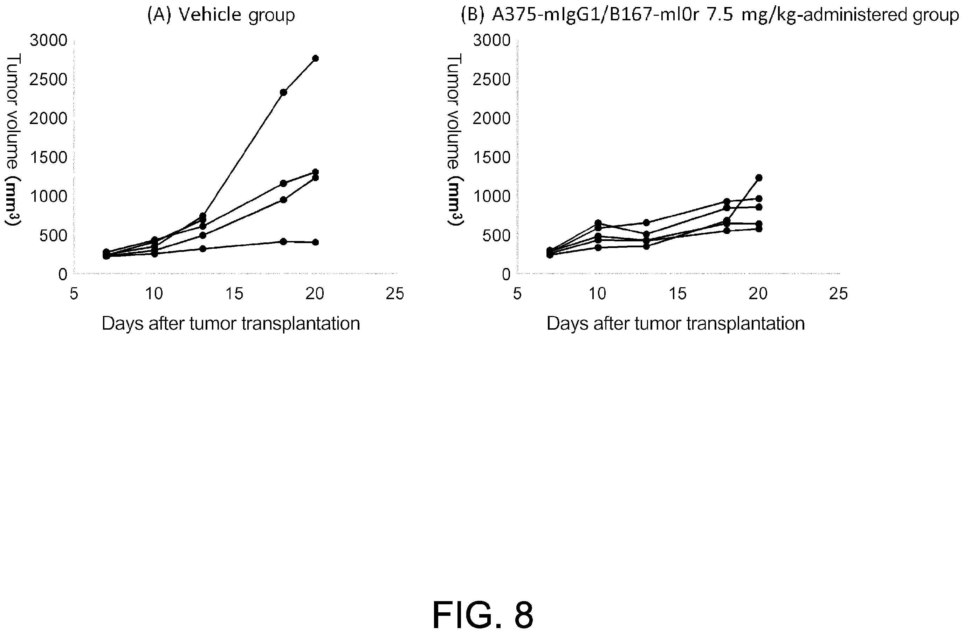

Subfigure (A) shows the change in tumor volume of each mouse in the vehicle-administered group. Subfigure (B) shows the change in tumor volume of each mouse in the A551-MB110/B379-ml0r (2.5 mg/kg) administration group. Fig. 8 shows the antitumor effect of A375-mIgG1/B167-ml0r in a mouse model transplanted with Hepa1-6/hGPC3 cells.

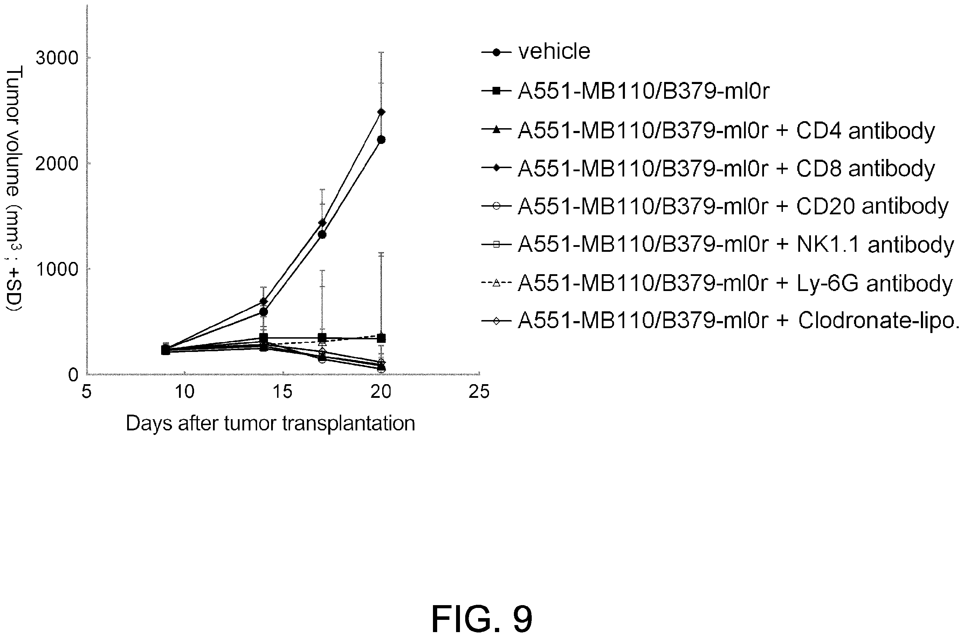

Subfigure (A) shows the change in tumor volume of each mouse in the vehicle-administered group. Subfigure (B) shows the change in tumor volume of each mouse in the A375-mIgG1/B167-ml0r (7.5 mg/kg) administration group. Fig. 9 shows the influence of the depletion (removal) of various immune cells (CD4$^+$ T cells, CD8$^+$ T cells, B cells, NK cells, granulocytes, macrophages) on the antitumor effect of A551-MB110/B379-ml0r in a mouse model transplanted with LLC1/OVA/GPC3 clone C5 cells.

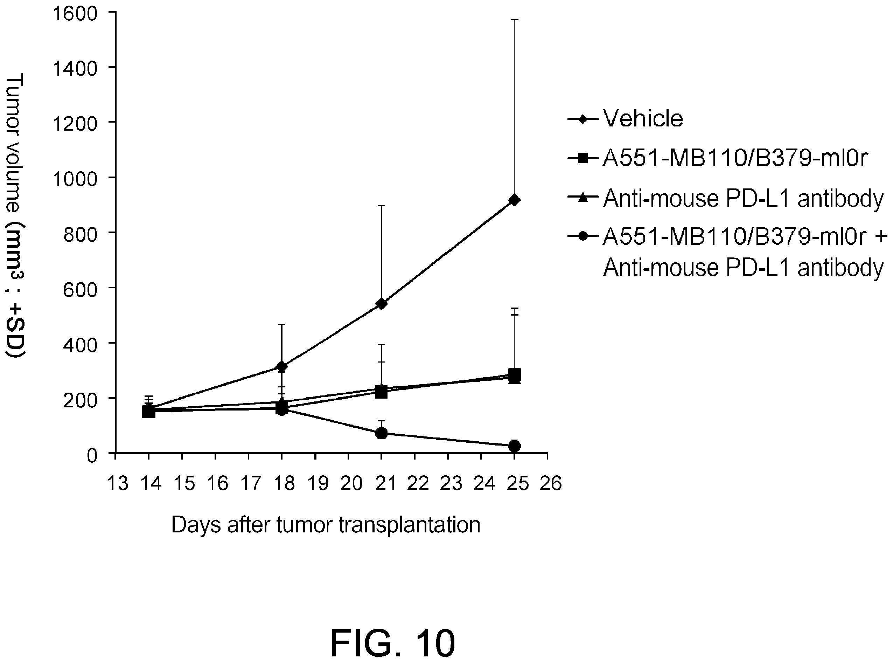

Fig. 10 shows the antitumor effect of A551-MB110/B379-ml0r, an anti-mouse PD-L1 antibody, and a combination of these in a mouse model transplanted with MC38 cells.

Each point shows the mean value for the tumor volume of one group (n = 6).

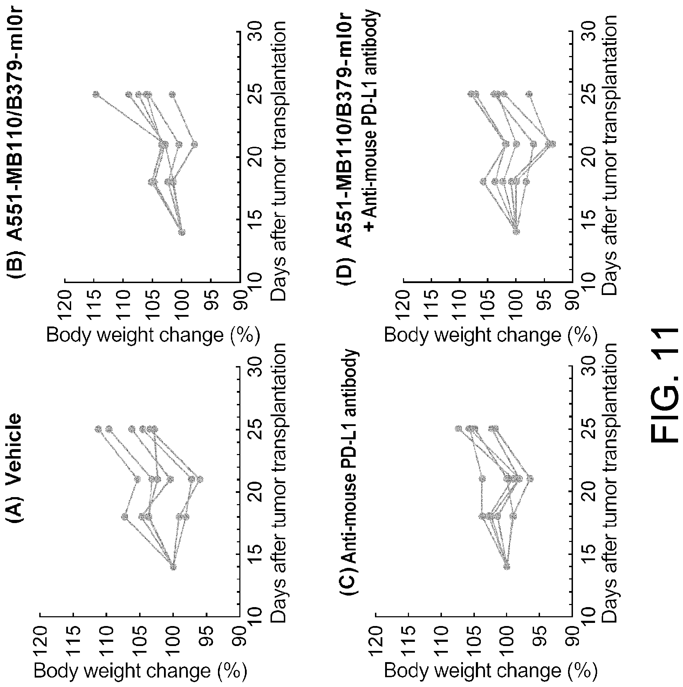

Fig. 11 shows the antitumor effect (body weight change) of A551-MB110/B379-ml0r, an anti-mouse PD-L1 antibody, or a combination thereof in a mouse model transplanted with MC38 cells.

Subfigure (A) shows the change in body weight of each mouse in the vehicle-administered group. Subfigure (B) shows the change in body weight of each mouse in the A551-MB110/B379-ml0r-administered group. Subfigure (C) shows the change in body weight of each mouse in the anti-mouse PD-L1 antibody-administered group. Subfigure (D) shows the change in body weight of each mouse in the group administered with the combination of A551-MB110/B379-ml0r and anti-mouse PD-L1 antibody.

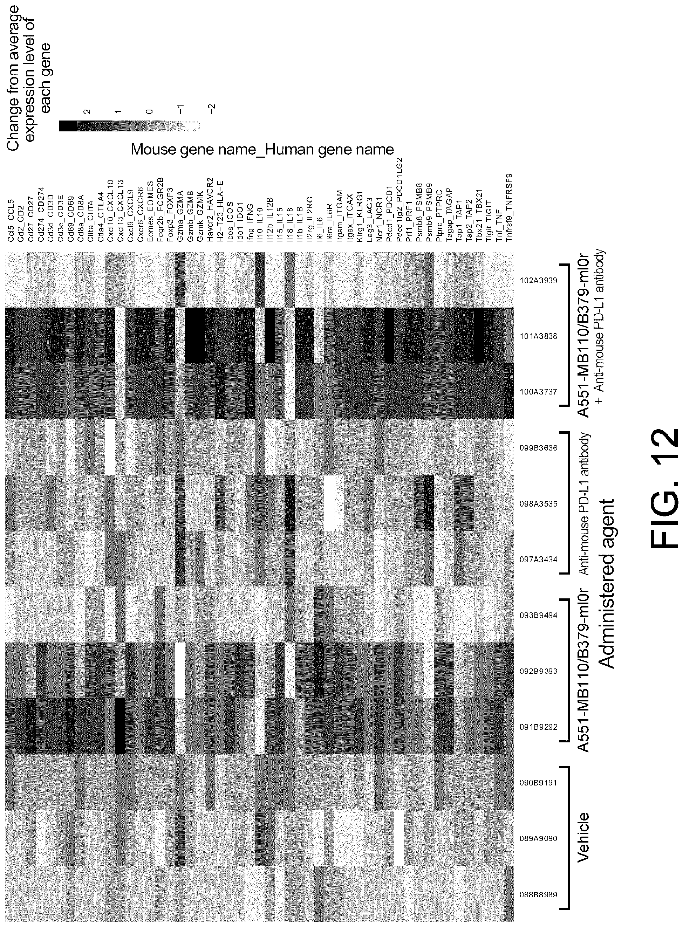

Fig. 12 shows changes in the expression of various immune-related genes by the administration of A551-MB110/B379-ml0r, an anti-mouse PD-L1 antibody, or a combination thereof to a mouse model transplanted with MC38 cells, before and after administration of each antibody or combination thereof.

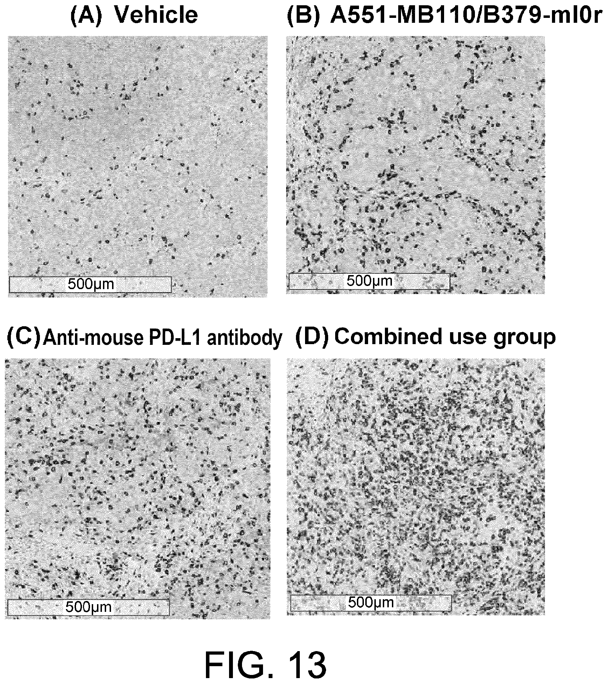

Fig. 13 shows the degree of CD8 expression in a tumor tissue of a mouse model transplanted with MC38 cells by the administration of A551-MB110/B379-ml0r, an anti-mouse PD-L1 antibody, or a combination thereof.

Subfigure (A) shows the degree of CD8 expression in tumor tissue of the vehicle-administered group, subfigure (B) shows the same of the A551-MB110/B379-ml0r-administered group, subfigure (C) shows the same of the anti-mouse PD-L1 antibody-administered group, and subfigure (D) shows the same of the A551-MB110/B379-ml0r and anti-mouse PD-L1 antibody combination-administered group.

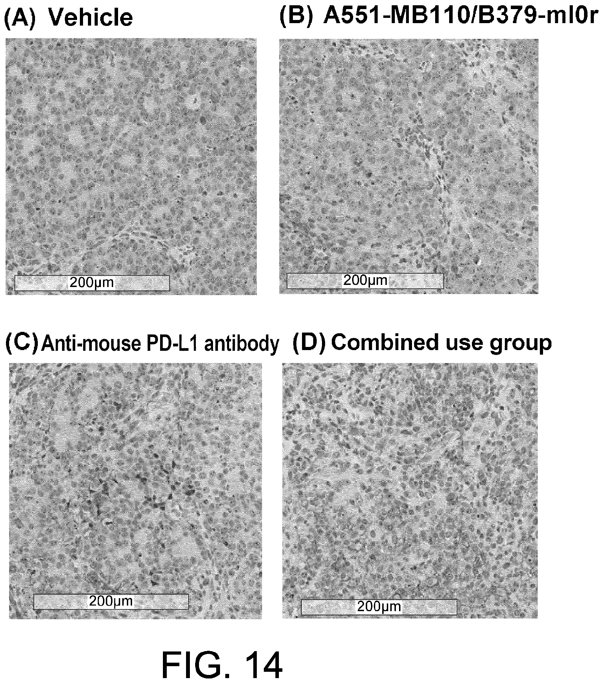

Fig. 14 shows the degree of PD-L1 expression in a tumor tissue by the administration of A551-MB110/B379-ml0r, anti-mouse PD-L1 antibody, or a combination thereof to a mouse model transplanted with MC38 cells.

Subfigure (A) shows the degree of PD-L1 expression in a tumor tissue of the vehicle-administered group, subfigure (B) shows the same of the A551-MB110/B379-ml0r-administered group, subfigure (C) shows the same of the anti-mouse PD-L1 antibody-administered group, and subfigure (D) shows the same of the A551-MB110/B379-ml0r and anti-mouse PD-L1 antibody combination-administered group.

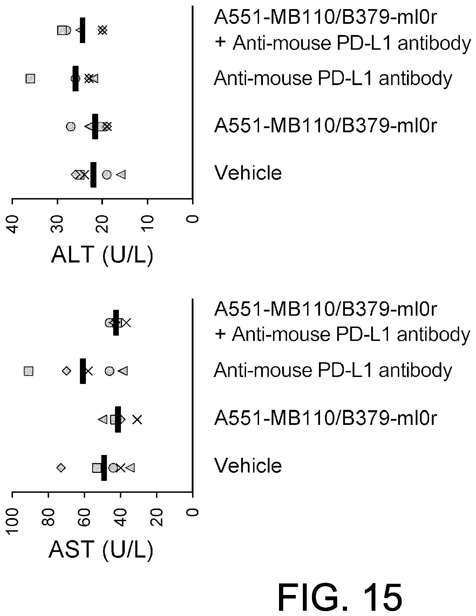

Fig. 15 shows the degree of liver function markers detected by a blood test due to the administration of A551-MB110/B379-ml0r, anti-mouse PD-L1 antibody, or a combination thereof to a mouse model transplanted with MC38 cells.

The top subfigure shows ALT (U/L) and the bottom subfigure shows AST (U/L).

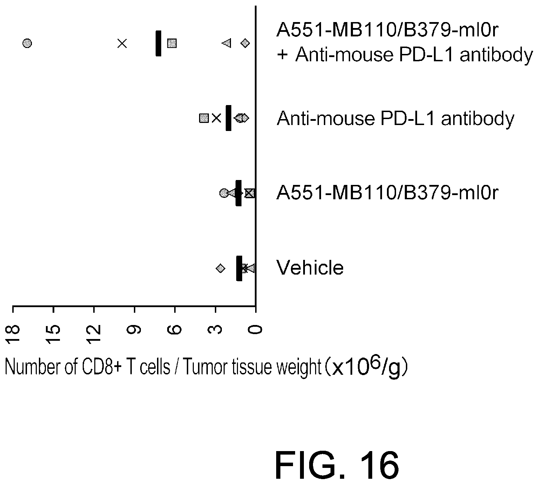

Fig. 16 shows the degree of increase in the number of CD8$^+$ T cells in a tumor tissue due to the administration of A551-MB110/B379-ml0r, anti-mouse PD-L1 antibody, or a combination thereof to a mouse model transplanted with MC38 cells.

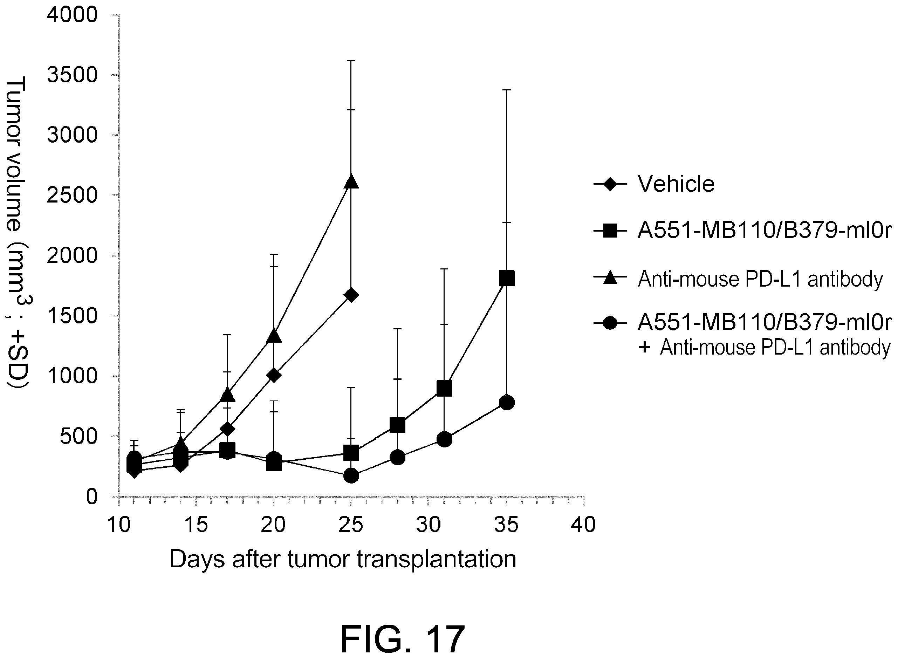

Fig. 17 shows the antitumor effect of A551-MB110/B379-ml0r, an anti-mouse PD-L1 antibody, or a combination thereof in a mouse model transplanted with LLC1/OVA/GPC3 clone C5 cells.

Each point shows the mean value for the tumor volume of one group (n = 5).

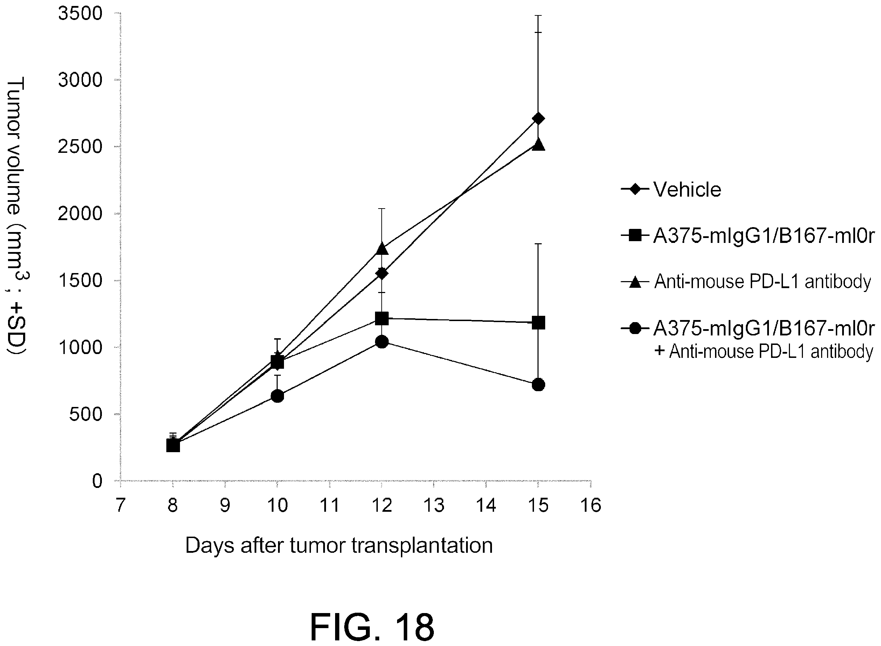

Fig. 18 shows the antitumor effect of A375-mIgG1/B167-ml0r, an anti-mouse PD-L1 antibody, or a combination thereof in a mouse model transplanted with C1498 cells.

Each point shows the mean value for the tumor volume of one group (n = 5).

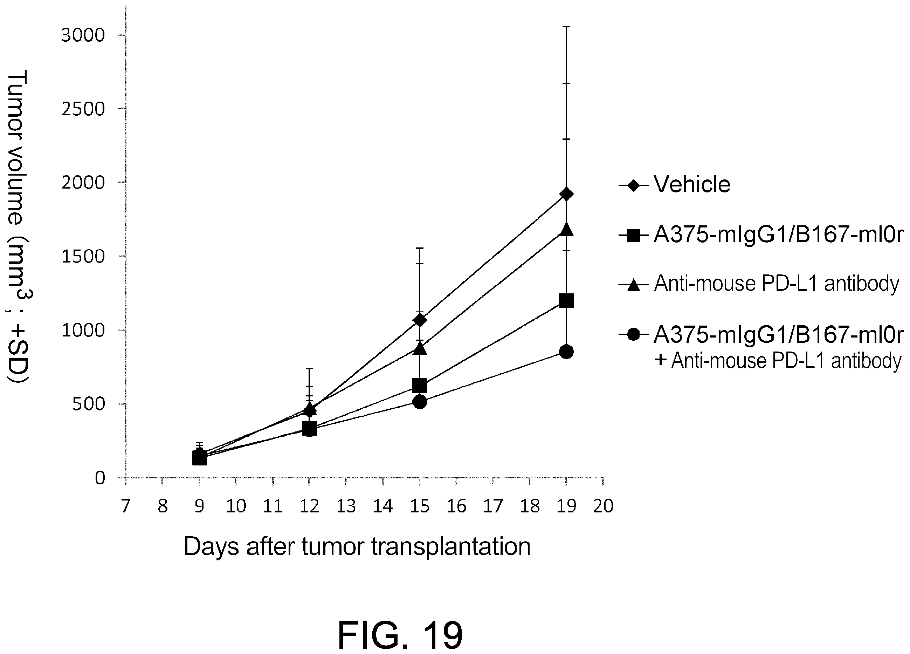

Fig. 19 shows the antitumor effect of A375-mIgGl/B167-ml0r, an anti-mouse PD-L1 antibody, or a combination thereof in a mouse model transplanted with AE17 cells.

Each point shows the mean value for the tumor volume of one group (n = 5).

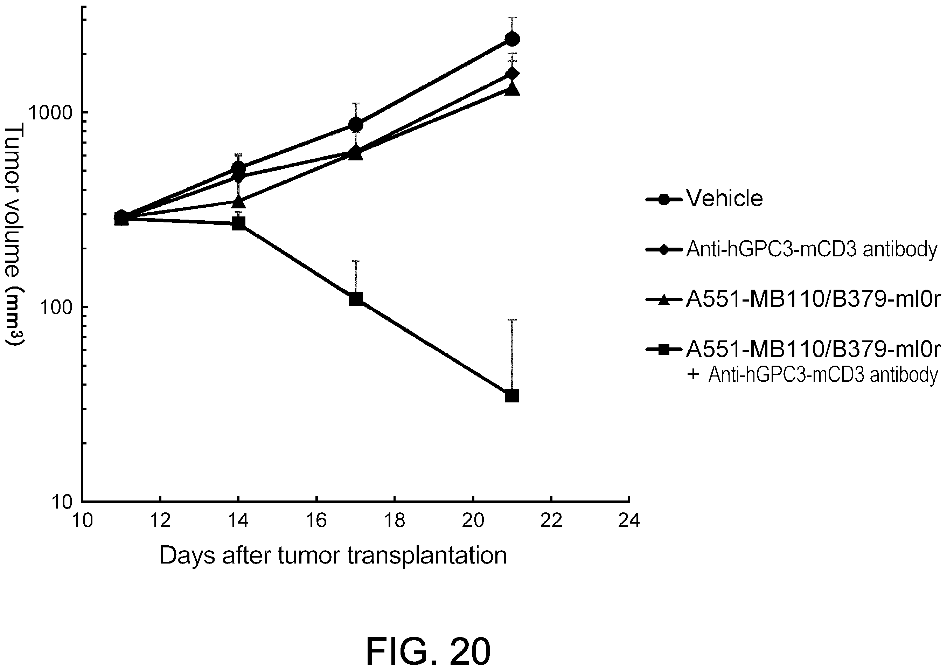

Fig. 20 shows the antitumor effect of A551-MB110/B379-ml0r, anti-hGPC3-mCD3 antibody, or a combination thereof in a mouse model transplanted with LLC1/hGPC3.

Each point shows the mean value for the tumor volume of one group (n = 5).

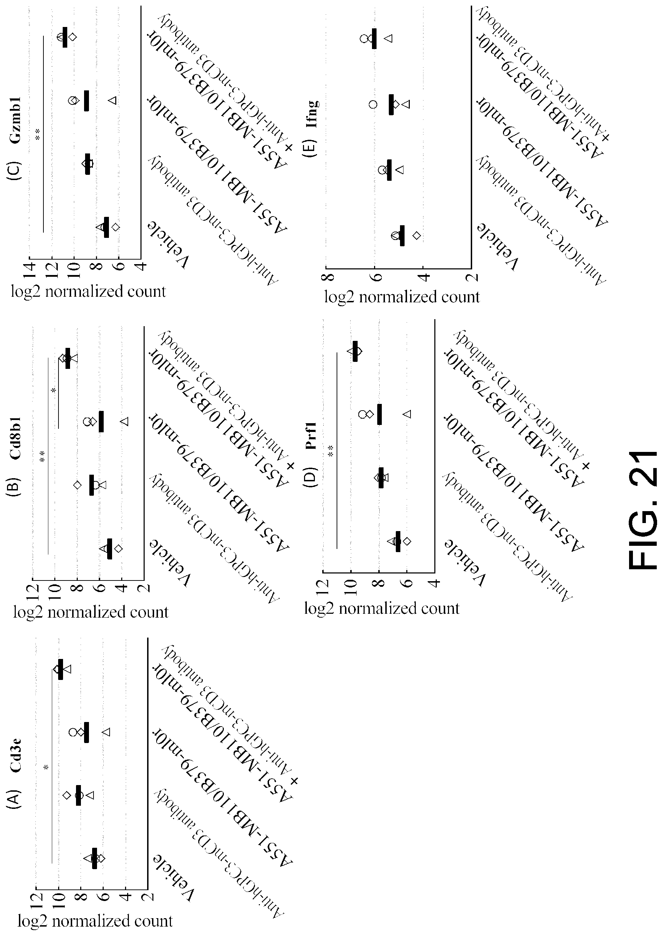

Fig. 21 shows the gene expression level in a tumor sample due to the administration of A551-MB110/B379-ml0r, anti-hGPC3-mCD3 antibody, or a combination thereof in a mouse model transplanted with LLC1/hGPC3.

Subfigure (A) shows the expression level of CD3e, subfigure (B) shows the expression level of CD8b1, subfigure (C) shows the expression level of Gzmb, and subfigure (D) shows the expression level of Prf1, and the subfigure (E) shows the expression level of Ifng, respectively.

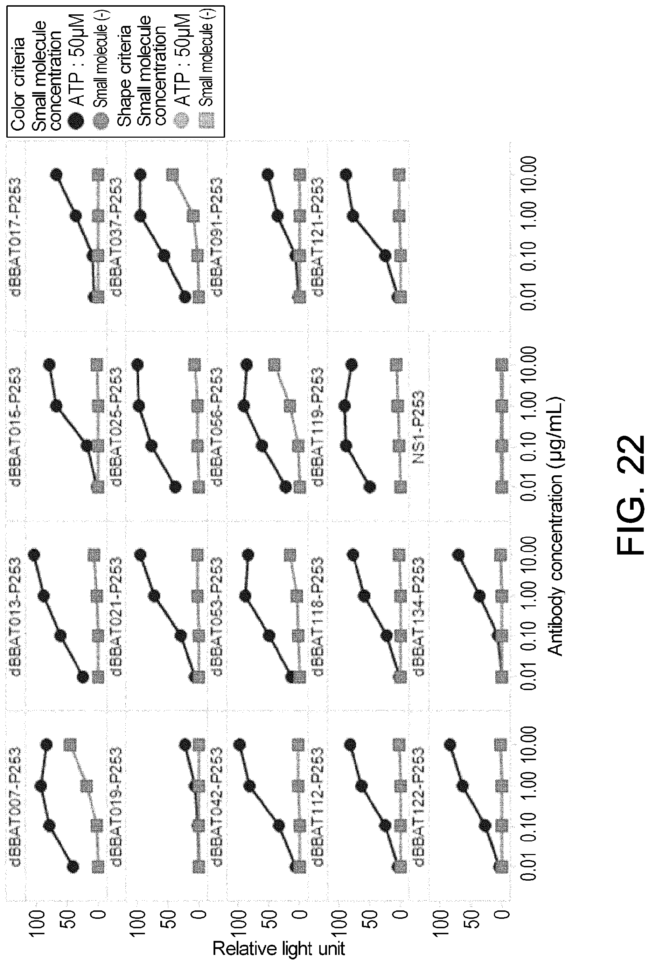

Fig. 22 is a diagram showing the agonist activity of various anti-CD137 antibodies tested using Jurkat cells in the presence or absence of ATP.

The X axis shows the antibody concentration (μg/mL) and the Y axis shows the relative light unit.

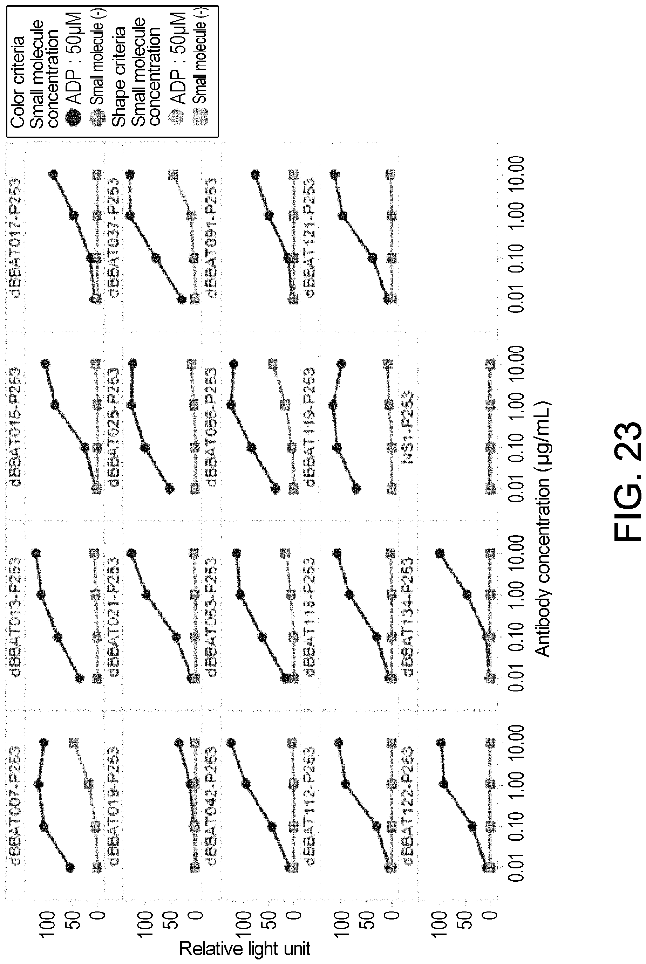

Fig. 23 is a diagram showing the agonist activity of various anti-CD137 antibodies tested using Jurkat cells in the presence or absence of ADP.

The X axis shows the antibody concentration (μg/mL) and the Y axis shows the relative light unit.

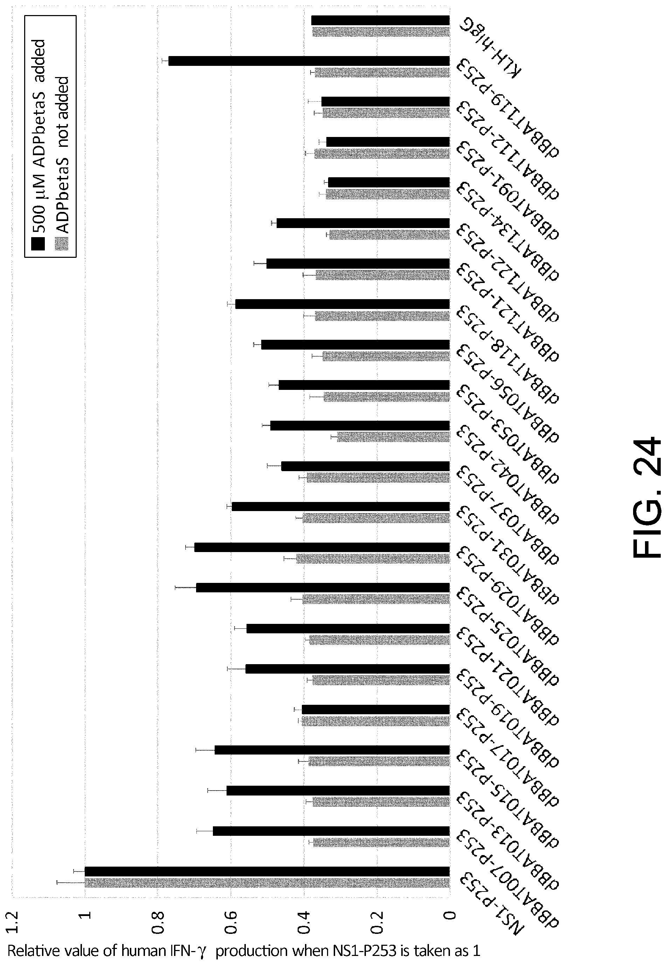

Fig. 24 is a diagram showing the agonist activity of various anti-CD137 antibodies tested using human T cells in the presence or absence of ADPbetaS.

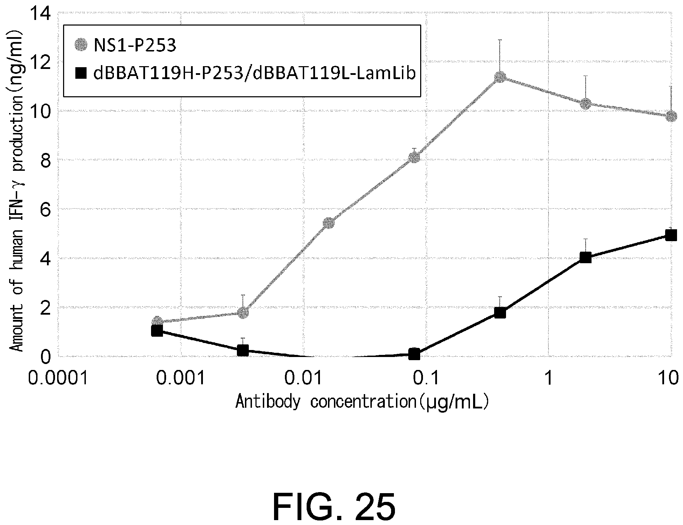

Fig. 25 is a diagram showing the agonist activity of dBBAT119-P253/dBBAT119L-LamLib (small molecule switch anti-CD137 antibody) or NS1-P253 (non-switch anti-CD137 antibody) tested using human T cells in the presence or absence of ADPbetaS.

The X axis shows the antibody concentration (μg/mL) and the Y axis shows the amount of IFN-γ production (ng/mL).

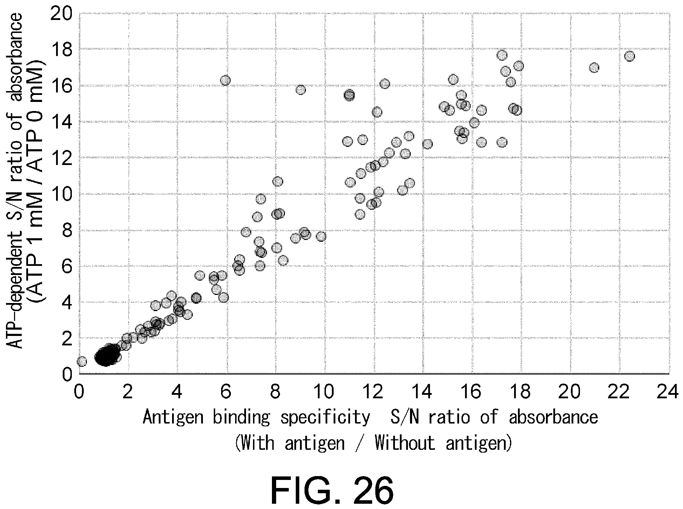

Fig. 26 is a diagram showing the ATP-dependent antigen-binding activity of various anti-CD137 antibodies (switch anti-CD 137 antibody with improved binding activity) tested with phage ELISA.

The Y axis shows the S/N ratio of absorbance in the presence/absence of ATP, and the X axis shows the S/N ratio in the presence/absence of the antigen.

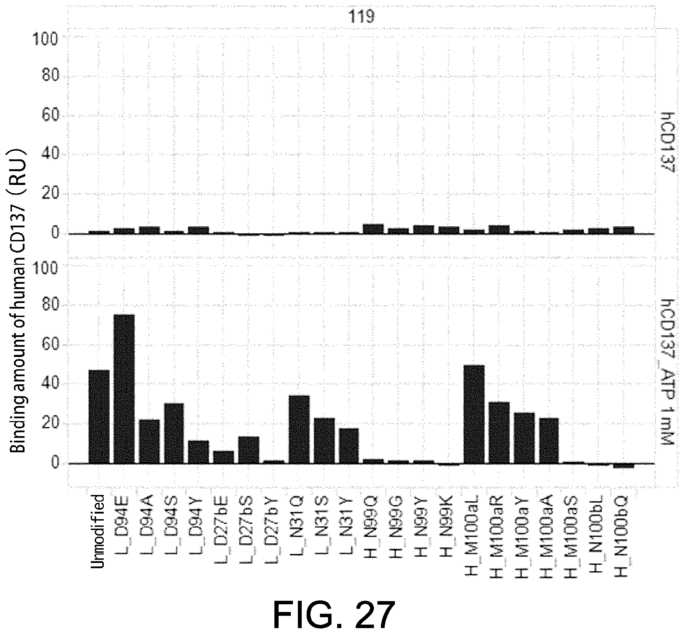

Fig. 27 is a diagram showing the binding activity of various variants of the anti-CD137 antibody (dBBAT119H-P253/dBBAT119L-LamLib) to human CD137 in the presence or absence of ATP.

The upper row shows the binding activity to human CD137 in the absence of ATP and the lower row shows the binding activity to human CD137 in the presence of ATP.

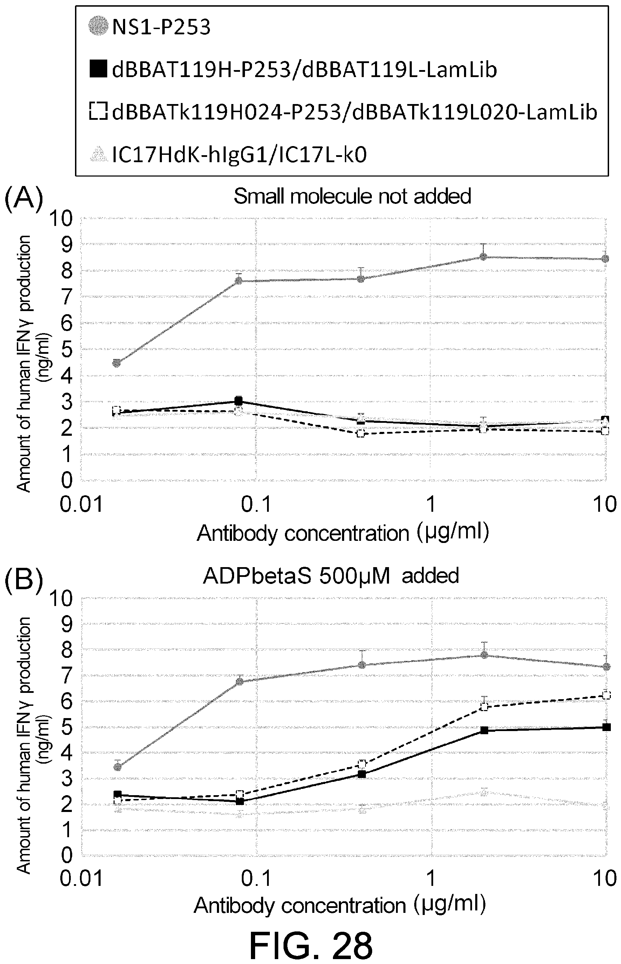

Fig. 28 is a diagram showing the agonist activity of dBBAT119H-P253/dBBAT119L-LamLib, dBBATk119H024-P253/dBBATk119L020-LamLib, IC17HdK-hIgG1/IC17L-k0 (control), or NS1-P253 (non-switch anti-CD 137 antibody) tested using human T cells in the presence or absence of ADPbetaS.

Subfigure (A) shows the test results in the absence of ADPbetaS and subfigure (B) shows the test results in the presence of ADPbetaS.

The X axis shows the antibody concentration (μg/mL) and the Y axis shows the amount of IFN-γ production (ng/mL).

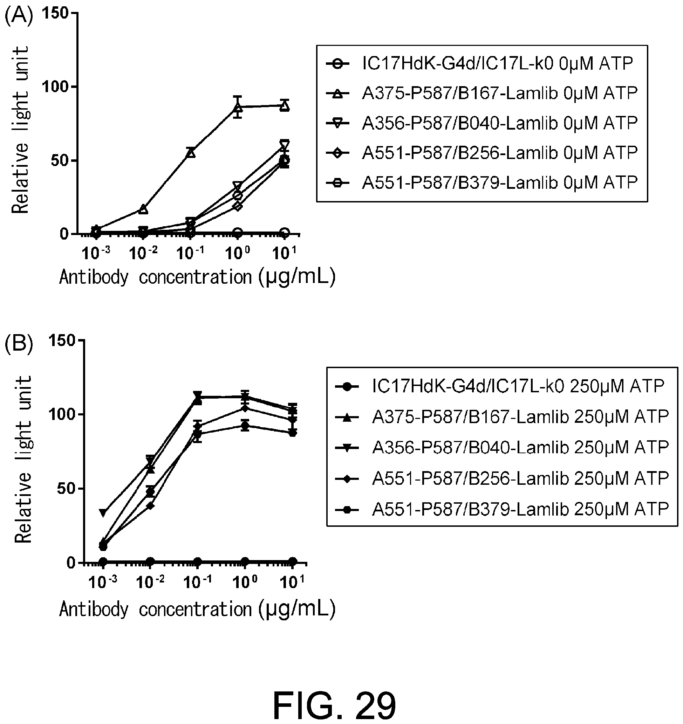

Fig. 29 is a diagram showing the agonist activity of various switch anti-CD137 antibodies tested using 4-1BB Jurkat reporter gene assay in the presence or absence of ATP.

Subfigure (A) shows the test results in the absence of ATP and subfigure (B) shows the test results in the presence of ATP.

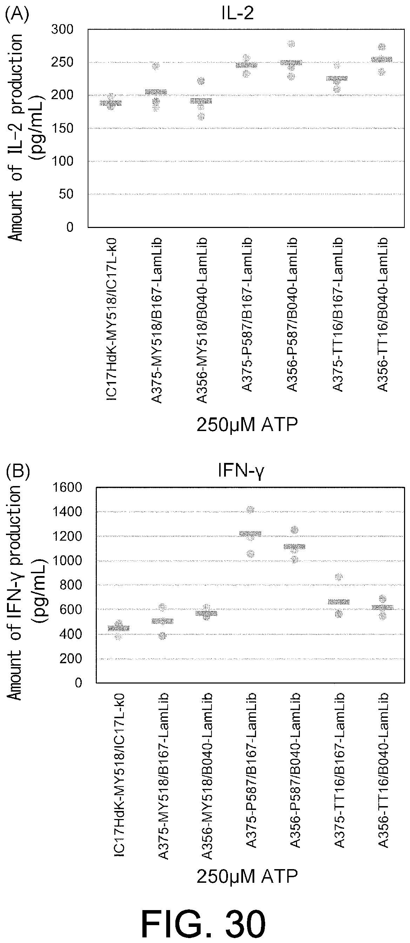

Fig. 30 is a diagram showing the enhancement in agonist activity of various switch anti-CD137 antibodies in the presence of ATP due to increase in the binding activity of heavy chain constant regions to Fcγ receptors, tested using human peripheral blood mononuclear cells.

Subfigure (A) shows the agonist activity determined using the amount of IL-2 production as an index, and subfigure (B) shows the agonist activity determined using the amount of IFN-γ production as an index.

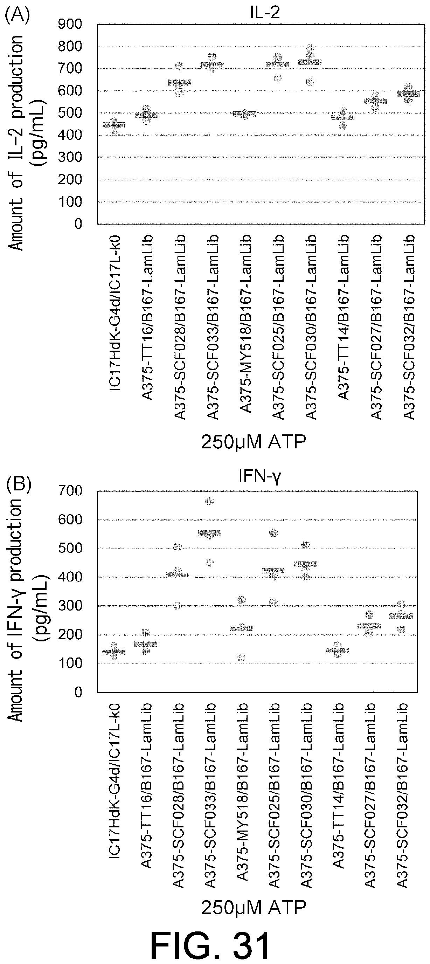

Fig. 31 is a diagram showing the enhancement in agonist activity of various switch anti-CD137 antibodies in the presence of ATP due to increase in the binding activity of heavy chain constant regions to Fcγ receptors or increase in the pI of heavy chain constant regions, tested using human peripheral blood mononuclear cells.

Subfigure (A) shows the agonist activity determined using the amount of IL-2 production as an index, and subfigure (B) shows the agonist activity determined using the amount of IFN-γ production as an index.

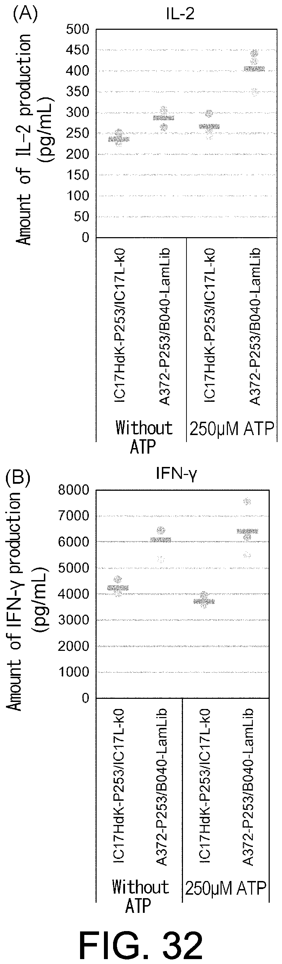

Fig. 32 is a diagram showing the enhancement in agonist activity of various switch anti-CD137 antibodies in the presence or absence of ATP due to increase in the binding activity of heavy chain constant regions to Fcγ receptors, tested using human peripheral blood mononuclear cells.

Subfigure (A) shows the agonist activity determined using the amount of IL-2 production as an index, and subfigure (B) shows the agonist activity determined using the amount of IFN-γ production as an index.

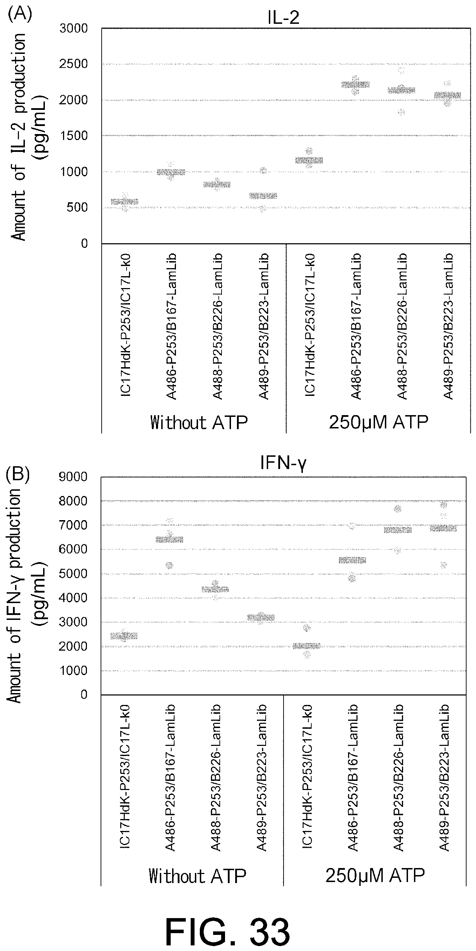

Fig. 33 is a diagram showing the enhancement in agonist activity of various switch anti-CD137 antibodies in the presence or absence of ATP due to increase in the binding activity of heavy chain constant regions to Fcγ receptors, tested using human peripheral blood mononuclear cells.

Subfigure (A) shows the agonist activity determined using the amount of IL-2 production as an index, and subfigure (B) shows the agonist activity determined using the amount of IFN-γ production as an index.

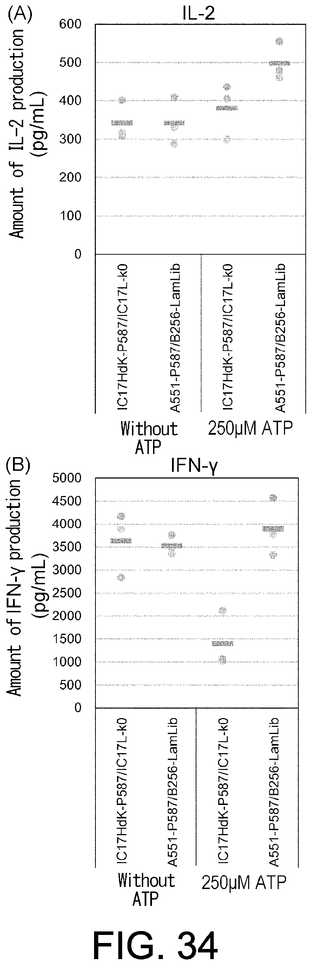

Fig. 34 is a diagram showing the enhancement in agonist activity of various switch anti-CD137 antibodies in the presence or absence of ATP due to increase in the binding activity of heavy chain constant regions to Fcγ receptors, tested using human peripheral blood mononuclear cells.

Subfigure (A) shows the agonist activity determined using the amount of IL-2 production as an index, and subfigure (B) shows the agonist activity determined using the amount of IFN-γ production as an index.

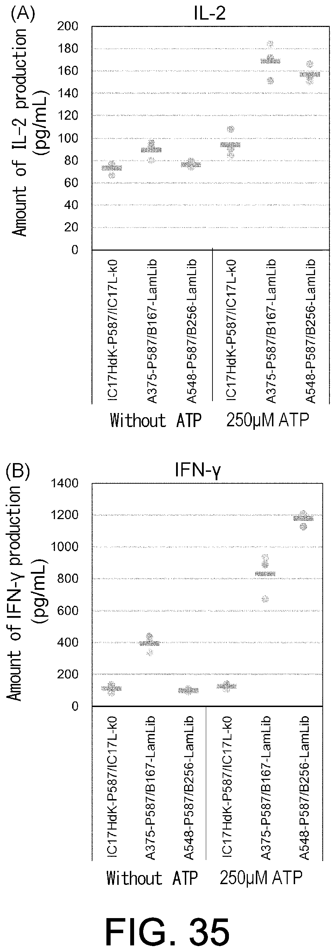

Fig. 35 is a diagram showing the enhancement in agonist activity of various switch anti-CD137 antibodies in the presence or absence of ATP due to increase in the binding activity of heavy chain constant regions to Fcγ receptors, tested using human peripheral blood mononuclear cells.

Subfigure (A) shows the agonist activity determined using the amount of IL-2 production as an index, and subfigure (B) shows the agonist activity determined using the amount of IFN-γ production as an index.

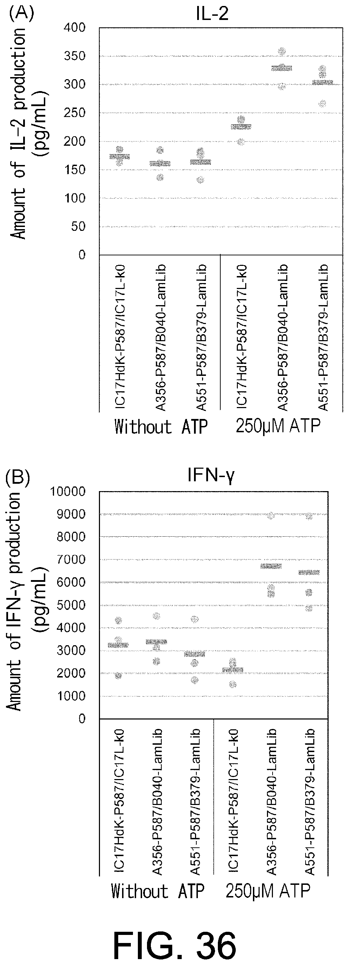

Fig. 36 is a diagram showing the enhancement in agonist activity of various switch anti-CD137 antibodies in the presence or absence of ATP due to increase in the binding activity of heavy chain constant regions to Fcγ receptors, tested using human peripheral blood mononuclear cells.

Subfigure (A) shows the agonist activity determined using the amount of IL-2 production as an index, and subfigure (B) shows the agonist activity determined using the amount of IFN-γ production as an index.

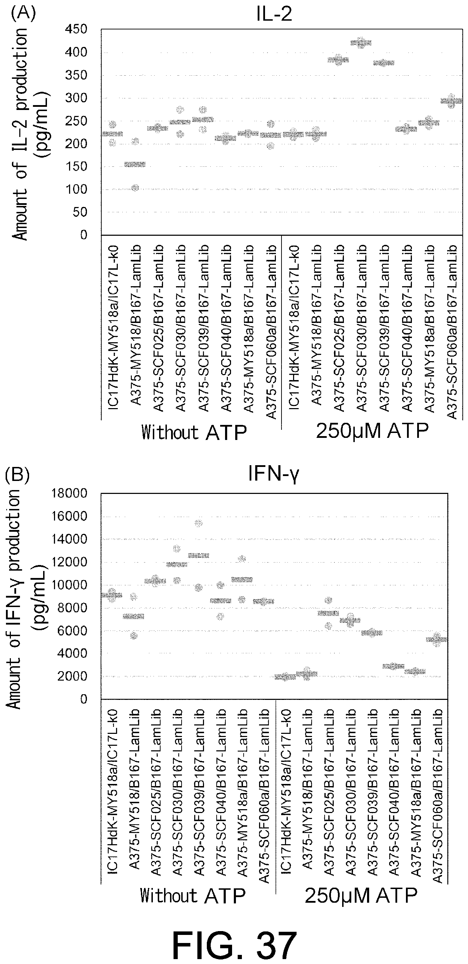

Fig. 37 is a diagram showing the enhancement in agonist activity of various switch anti-CD137 antibodies in the presence or absence of ATP due to increase in the pI of heavy chain constant regions, tested using human peripheral blood mononuclear cells.

Subfigure (A) shows the agonist activity determined using the amount of IL-2 production as an index, and subfigure (B) shows the agonist activity determined using the amount of IFN-γ production as an index.

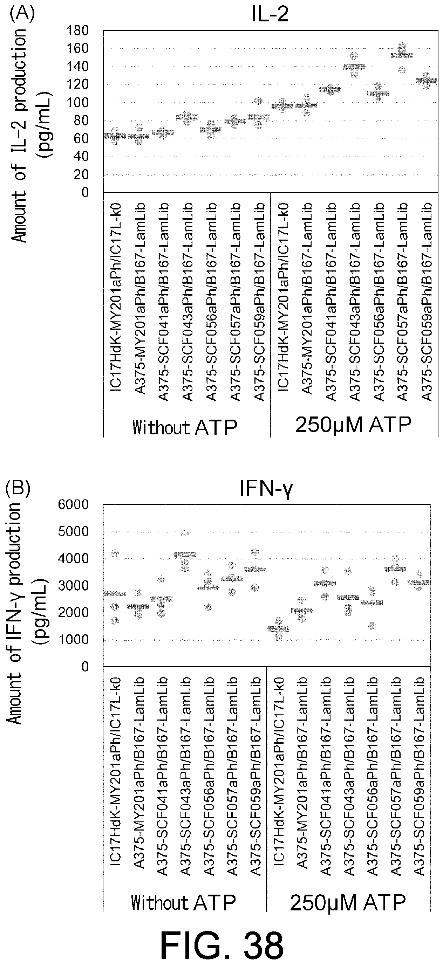

Fig. 38 is a diagram showing the enhancement in agonist activity of various switch anti-CD137 antibodies in the presence or absence of ATP due to increase in the pI of heavy chain constant regions, tested using human peripheral blood mononuclear cells.

Subfigure (A) shows the agonist activity determined using the amount of IL-2 production as an index, and subfigure (B) shows the agonist activity determined using the amount of IFN-γ production as an index.

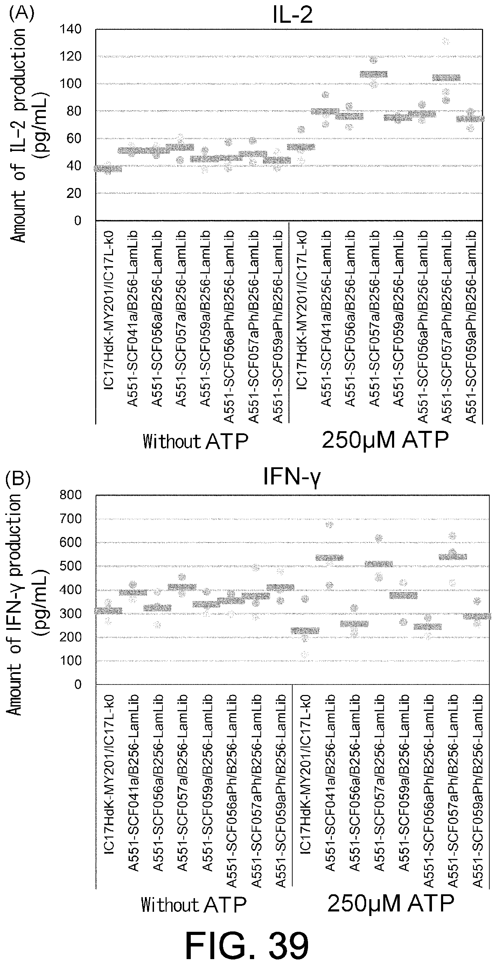

Fig. 39 is a diagram showing the enhancement in agonist activity of various switch anti-CD137 antibodies in the presence or absence of ATP due to increase in the pI of heavy chain constant regions, tested using human peripheral blood mononuclear cells.

Sub figure (A) shows the agonist activity determined using the amount of IL-2 production as an index, and subfigure (B) shows the agonist activity determined using the amount of IFN-γ production as an index.

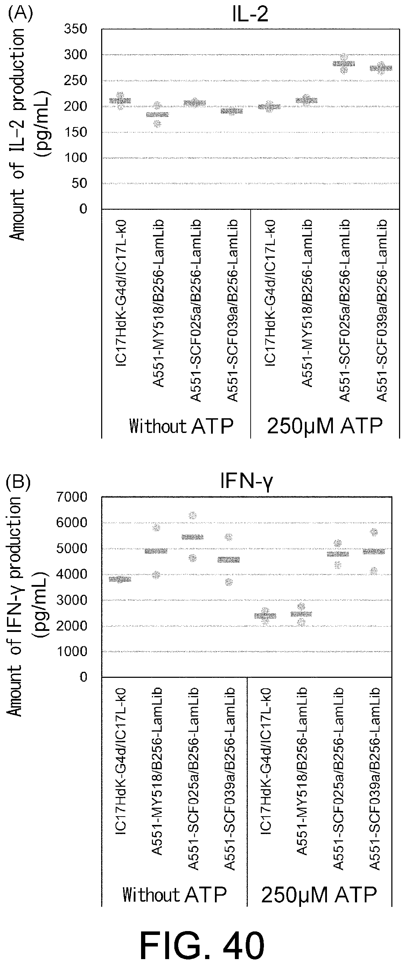

Fig. 40 is a diagram showing the enhancement in agonist activity of various switch anti-CD137 antibodies in the presence or absence of ATP due to increase in the pI of heavy chain constant regions, tested using human peripheral blood mononuclear cells.

Subfigure (A) shows the agonist activity determined using the amount of IL-2 production as an index, and subfigure (B) shows the agonist activity determined using the amount of IFN-γ production as an index.

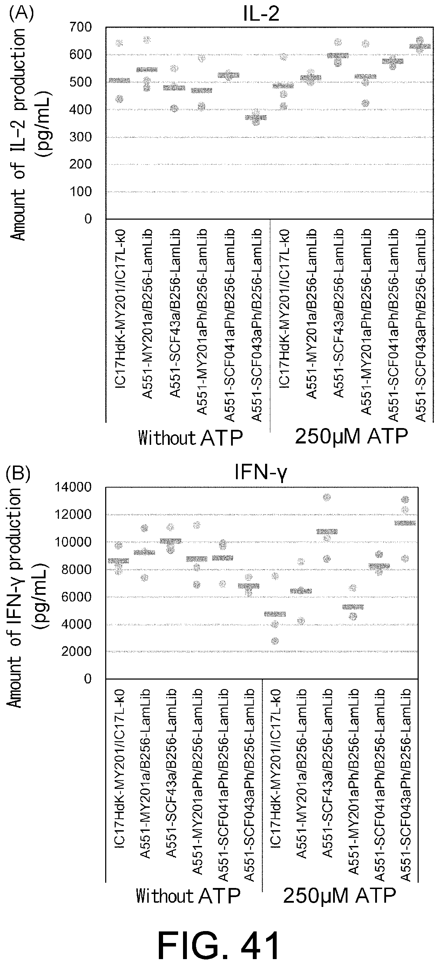

Fig. 41 is a diagram showing the enhancement in agonist activity of various switch anti-CD137 antibodies in the presence or absence of ATP due to increase in the pI of heavy chain constant regions, tested using human peripheral blood mononuclear cells.

Subfigure (A) shows the agonist activity determined using the amount of IL-2 production as an index, and subfigure (B) shows the agonist activity determined using the amount of IFN-γ production as an index.

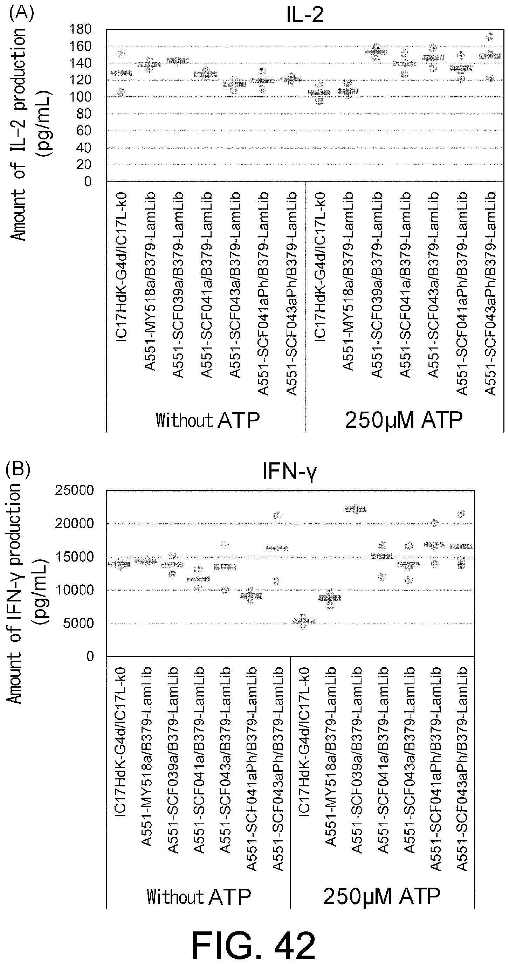

Fig. 42 is a diagram showing the enhancement in agonist activity of various switch anti-CD137 antibodies in the presence or absence of ATP due to increase in the pI of heavy chain constant regions, tested using human peripheral blood mononuclear cells.

Subfigure (A) shows the agonist activity determined using the amount of IL-2 production as an index, and subfigure (B) shows the agonist activity determined using the amount of IFN-γ production as an index.

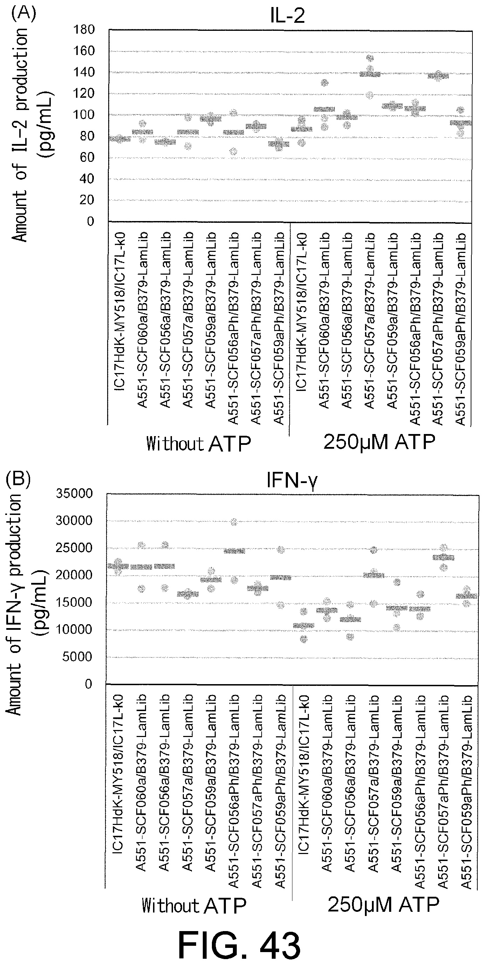

Fig. 43 is a diagram showing the enhancement in agonist activity of various switch anti-CD137 antibodies in the presence or absence of ATP due to increase in the pI of heavy chain constant regions, tested using human peripheral blood mononuclear cells.

Subfigure (A) shows the agonist activity determined using the amount of IL-2 production as an index, and subfigure (B) shows the agonist activity determined using the amount of IFN-γ production as an index.

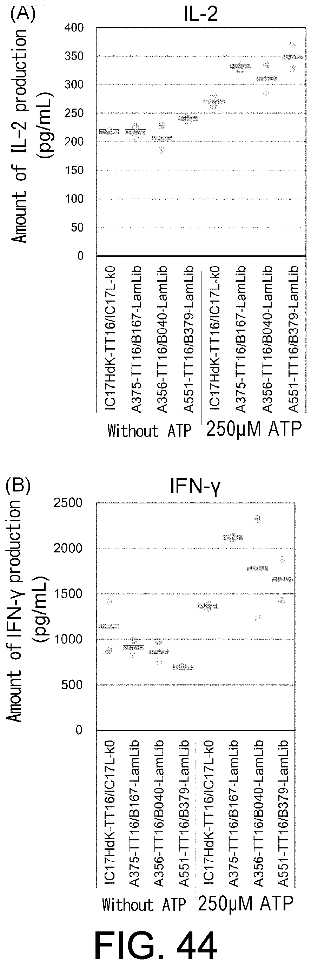

Fig. 44 is a diagram showing the enhancement in agonist activity of various switch anti-CD137 antibodies in the presence or absence of ATP due to increase in the binding activity of heavy chain constant regions to Fcγ receptors, tested using human peripheral blood mononuclear cells.

Subfigure (A) shows the agonist activity determined using the amount of IL-2 production as an index, and subfigure (B) shows the agonist activity determined using the amount of IFN-γ production as an index.

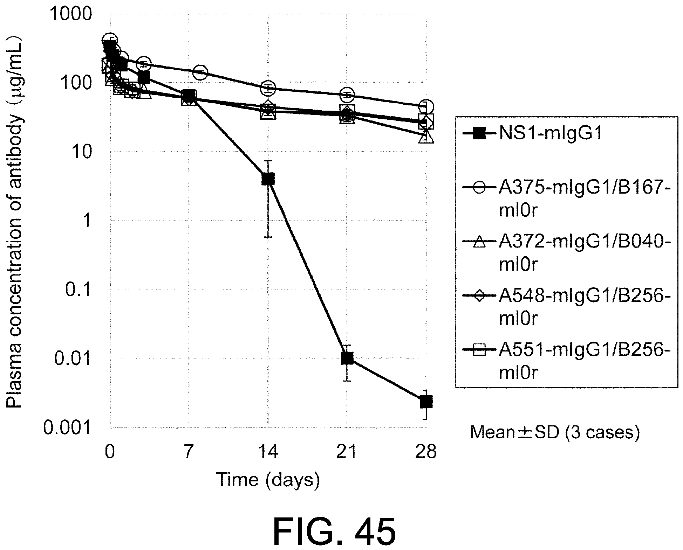

Fig. 45 is a diagram showing the plasma concentration of various switch and non-switch anti-CD 137 antibodies tested using human CD137 knock-in mouse.

The Fcs are all of mIgG1.

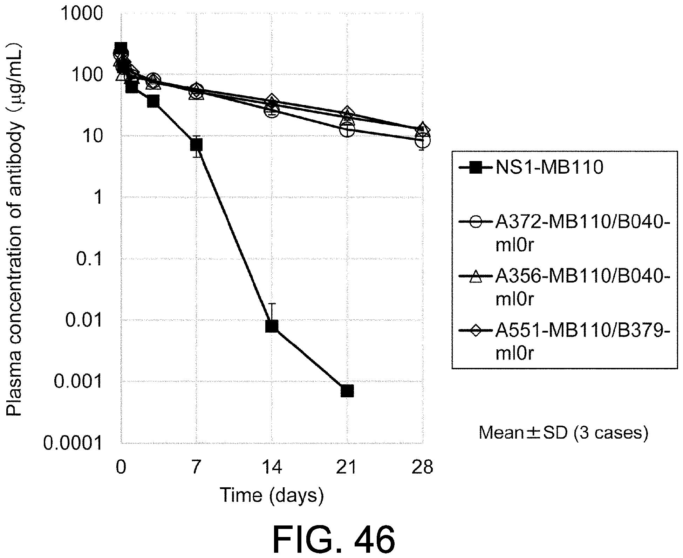

Fig. 46 is a diagram showing the plasma concentration of various switch and non-switch anti-CD 137 antibodies tested using human CD137 knock-in mouse.

The Fcs are all of MB110.

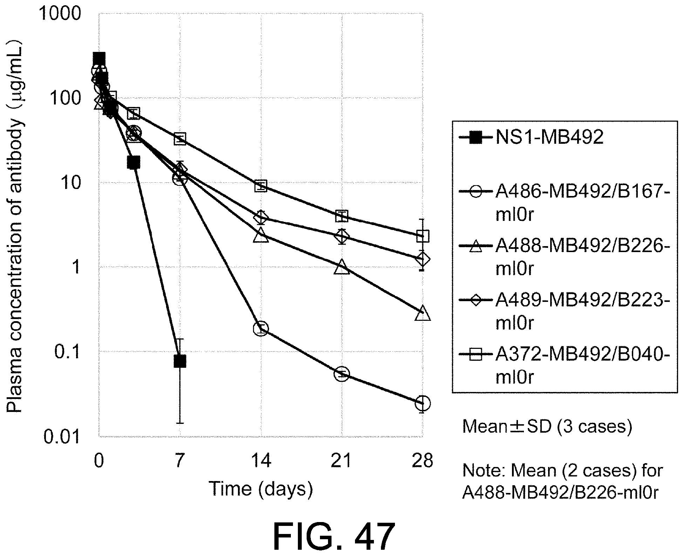

Fig. 47 is a diagram showing the plasma concentration of various switch and non-switch anti-CD 137 antibodies tested using human CD137 knock-in mouse.

The Fcs are all of MB492.

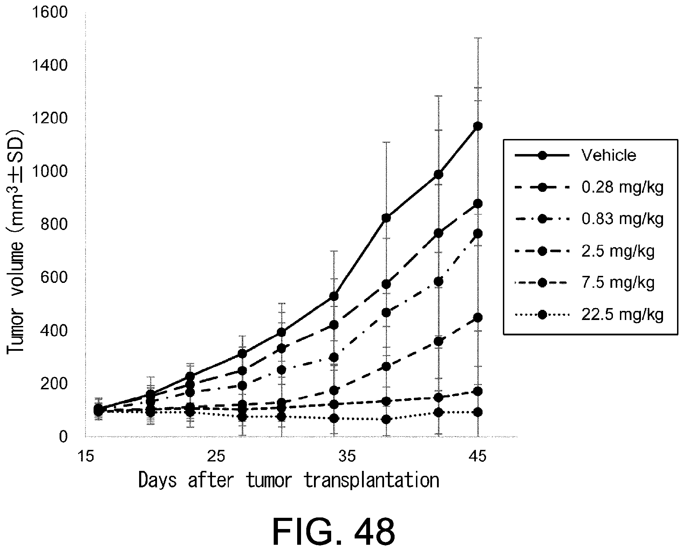

Fig. 48 is a diagram showing the anti-tumor effect of A375-mIgG1/B167-ml0r in a mouse model transplanted with MC38 cells.

Each dot shows the mean value of a group (n=5) of tumor volumes.

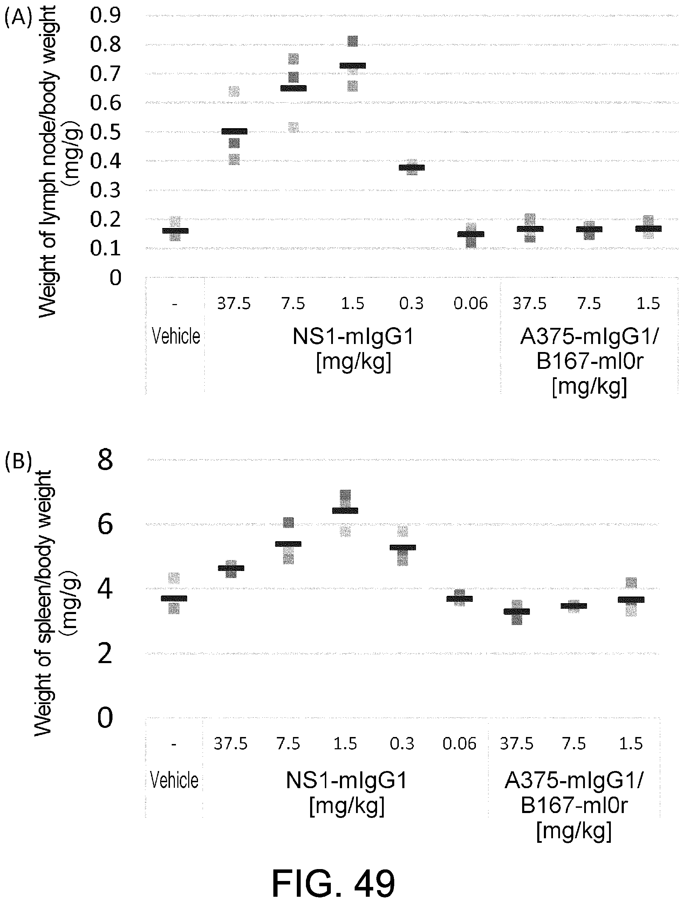

Fig. 49 is a diagram showing the weight of organs in a mouse model transplanted with MC38 cells after administration of antibodies (NO1-mIgG1 or A375-mIgG1B167-ml0r).

Subfigure (A) shows the weight of lymph node and subfigure (B) shows the weight of spleen.

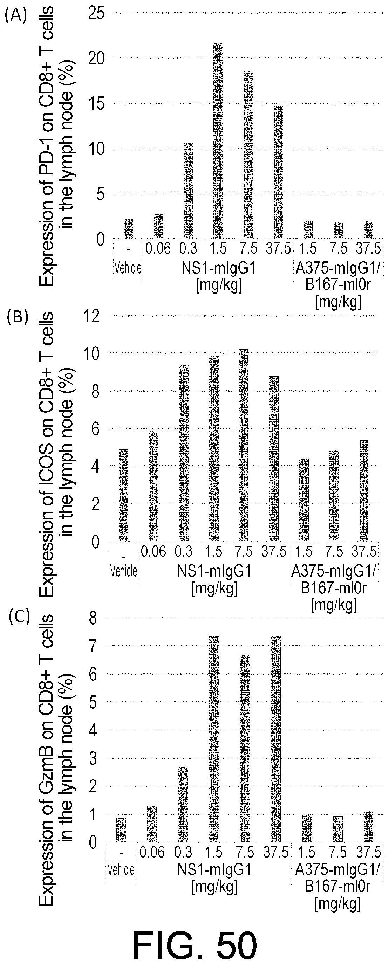

Fig. 50 is a diagram showing the degree of T cell activation in the lymph nodes of a mouse model transplanted with MC38 cells after administration of NO1-mIgG1 or A375-mIgG1/B167-ml0r.

Subfigure (A) shows the percentage of PD-1 positive T cells in CD8 positive T cells, subfigure (B) shows the percentage of ICOS positive T cells in CD8 positive T cells, and subfigure (C) shows the percentage of Granzyme B positive T cells in CD8 positive T cells.

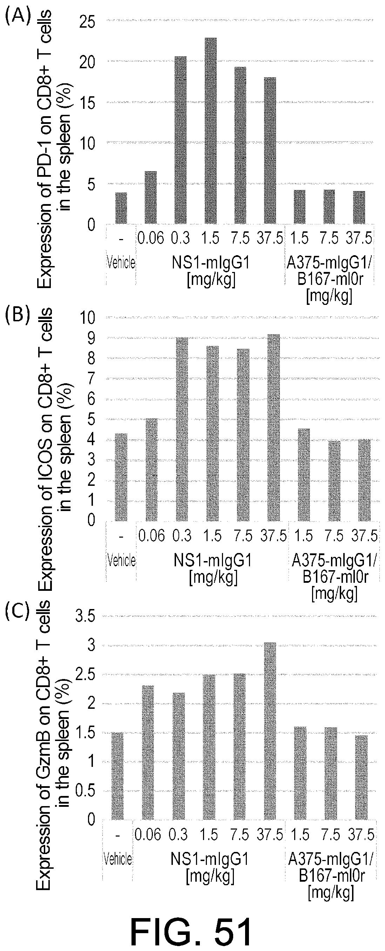

Fig. 51 is a diagram showing the degree of T cell activation in the spleen of a mouse model transplanted with the MC38 cell line after administration of NO1-mIgG1 or A375-mIgG1/B167-ml0r.

Subfigure (A) shows the percentage of PD-1 positive T cells in CD8 positive T cells, subfigure (B) shows the percentage of ICOS positive T cells in CD8 positive T cells, and subfigure (C) shows the percentage of Granzyme B positive T cells in CD8 positive T cells.

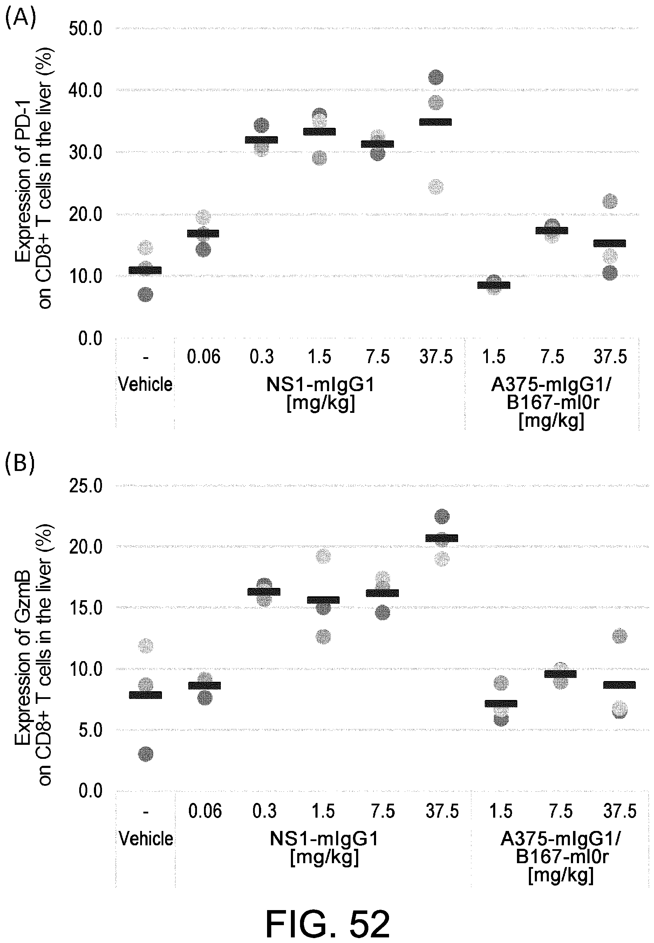

Fig. 52 is a diagram showing the degree of T cell activation in the liver of a mouse model transplanted with the MC38 cell line after administration of NO1-mIgG1 or A375-mIgG1/B167-ml0r.

Subfigure (A) shows the percentage of PD-1 positive T cells in CD8 positive T cells, and subfigure (B) shows the percentage of Granzyme B positive T cells in CD8 positive T cells.

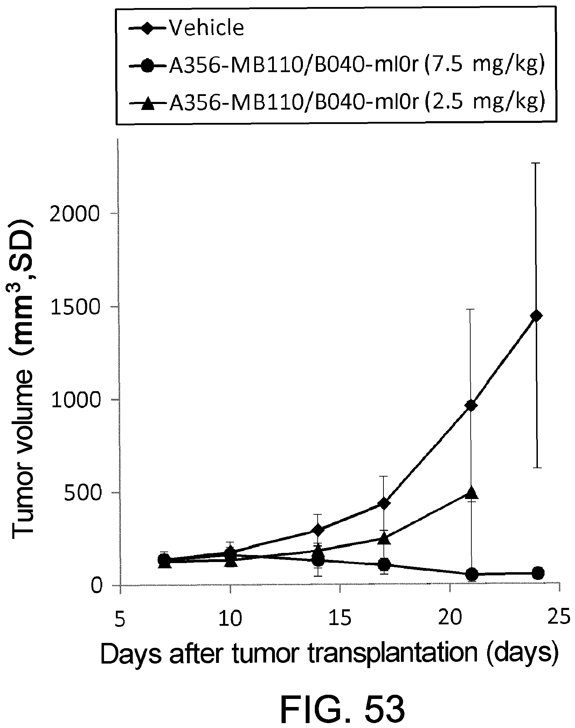

Fig. 53 is a diagram showing the anti-tumor effect of A356-MB110/B040-ml0r in a mouse model transplanted with the MC38 cell line.

Each dot shows the mean value of a group (n=5) of tumor volumes.

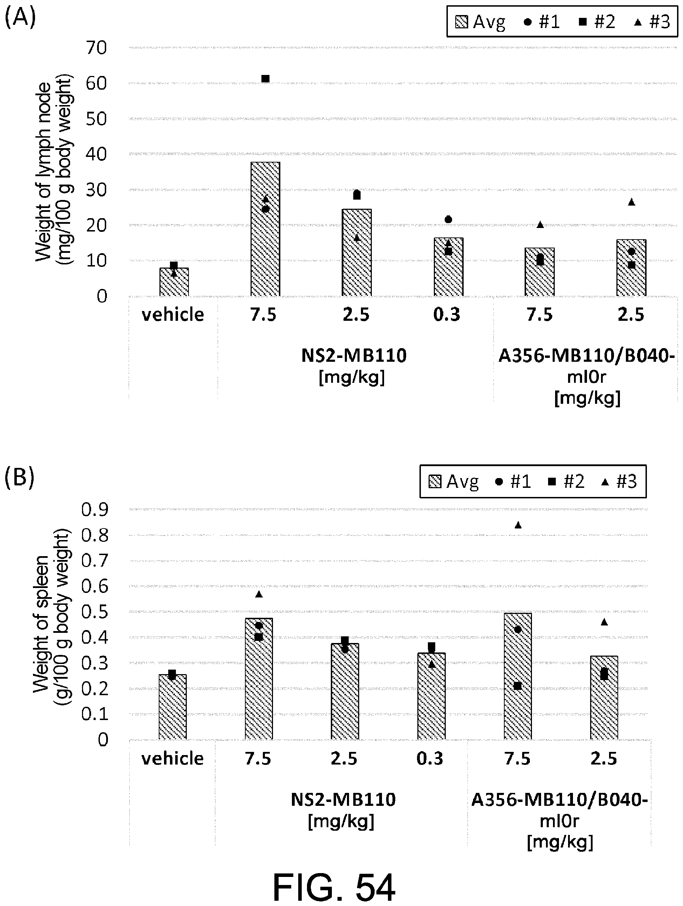

Fig. 54 is a diagram showing the weight of organs in a mouse model transplanted with the MC38 cell line after administration of NS2-MB110 or A356-MB110/B040-ml0r.

Subfigure (A) shows the weight of lymph node and subfigure (B) shows the weight of spleen.

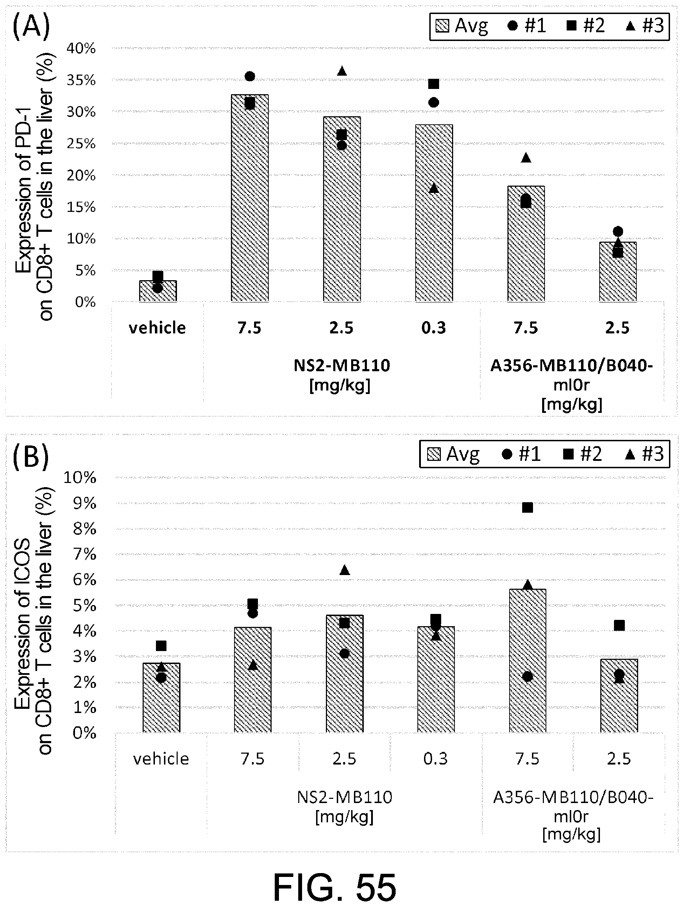

Fig. 55 is a diagram showing the degree of T cell activation in the liver of a mouse model transplanted with the MC38 cell line after administration of NS2-MB110 or A356-MB110/B040-ml0r.

Subfigure (A) shows the percentage of PD-1 positive T cells in CD8 positive T cells, and subfigure (B) shows the percentage of ICOS positive T cells in CD8 positive T cells.

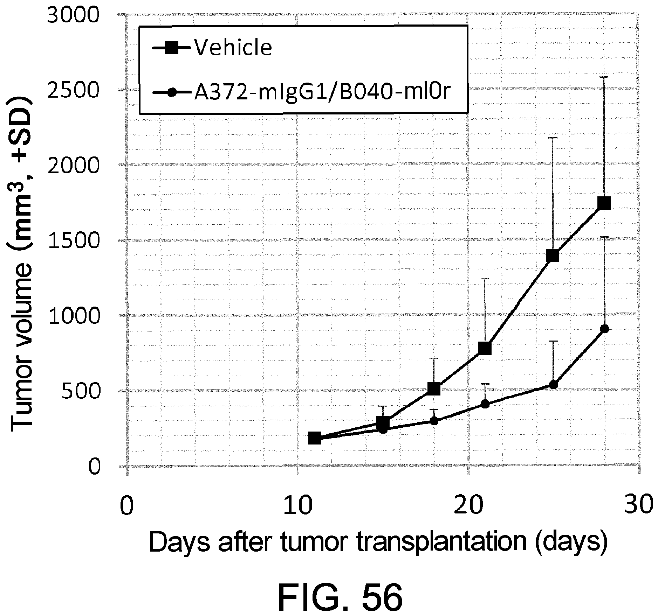

Fig. 56 is a diagram showing the anti-tumor effect of A372-mIgG1/B040-ml0r in a mouse model transplanted with the MC38 cell line.

Each dot shows the mean value of a group (n=5) of tumor volumes.

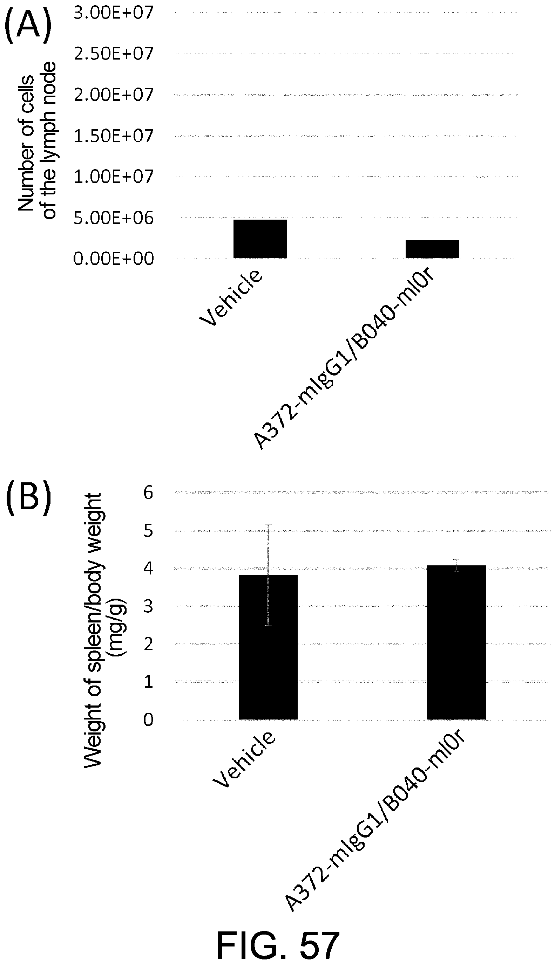

Fig. 57 shows the number of cells of the lymph node (subfigure (A)) and the weight of spleen (subfigure (B)) in a mouse model transplanted with the MC38 cell line after administration of A372-mIgG1/B040-ml0r.

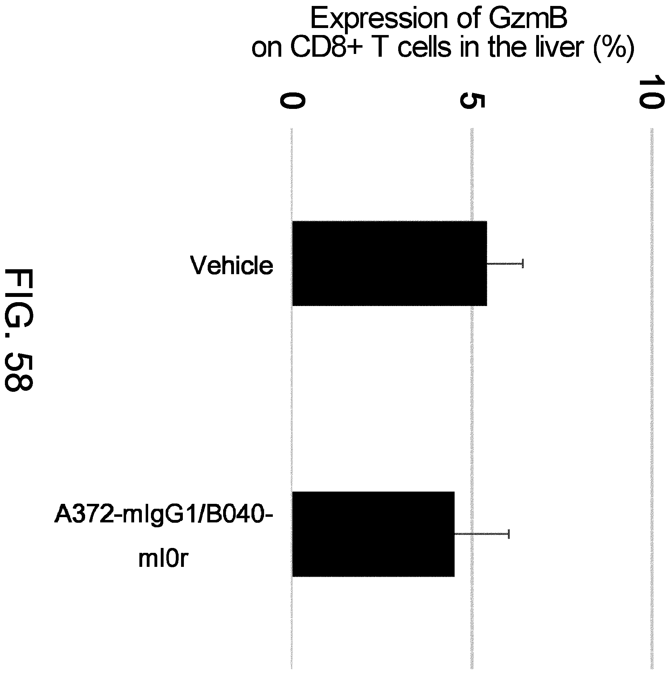

Fig. 58 is a diagram showing the degree of T cell activation in the liver of a mouse model transplanted with the MC38 cell line after administration of A372-mIgG1/B040-ml0r (percentage of Granzyme B positive T cells in CD8 positive T cells).

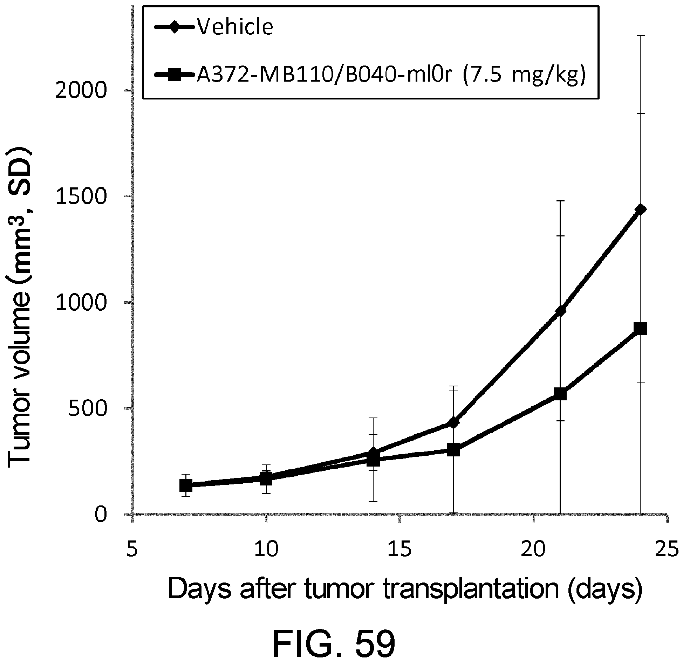

Fig. 59 is a diagram showing the anti-tumor effect of A372-MB110/B040-ml0r in a mouse model transplanted with the MC38 cell line.

Each dot shows the mean value of a group (n=5) of tumor volumes.

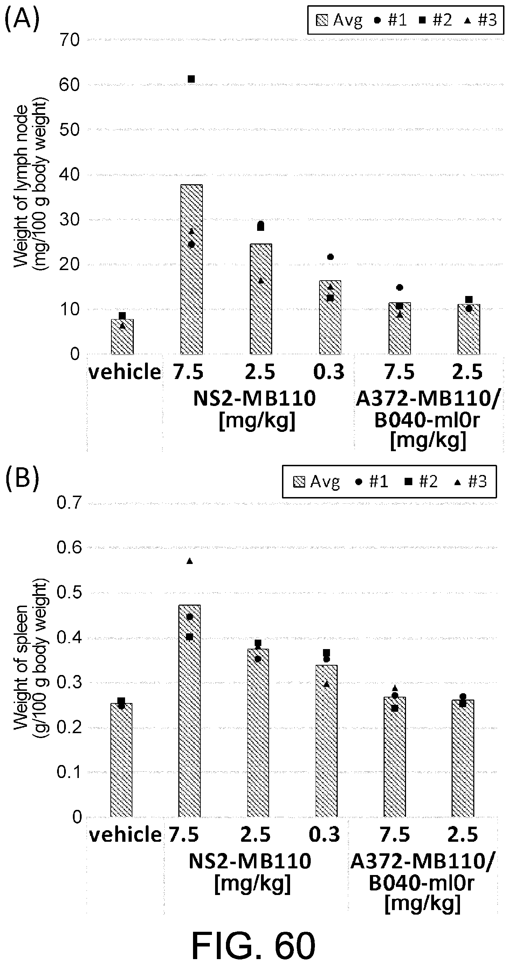

Fig. 60 is a diagram showing the weight of organs in a mouse model transplanted with the MC38 cell line after administration of NS2-MB110 or A372-MB110/B040-ml0r.

Subfigure (A) shows the weight of lymph node and subfigure (B) shows the weight of spleen.

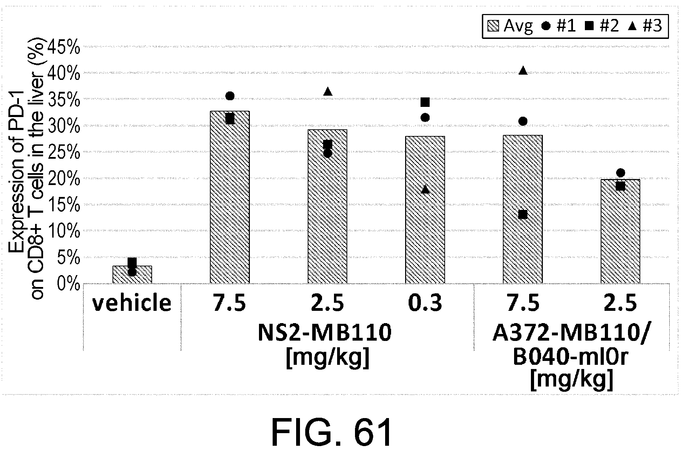

Fig. 61 is a diagram showing the degree of T cell activation in the liver of a mouse model transplanted with the MC38 cell line after administration of NS2-MB110 or A372-MB110/B040-ml0r (percentage of PD-1 positive T cells in CD8 positive T cells).

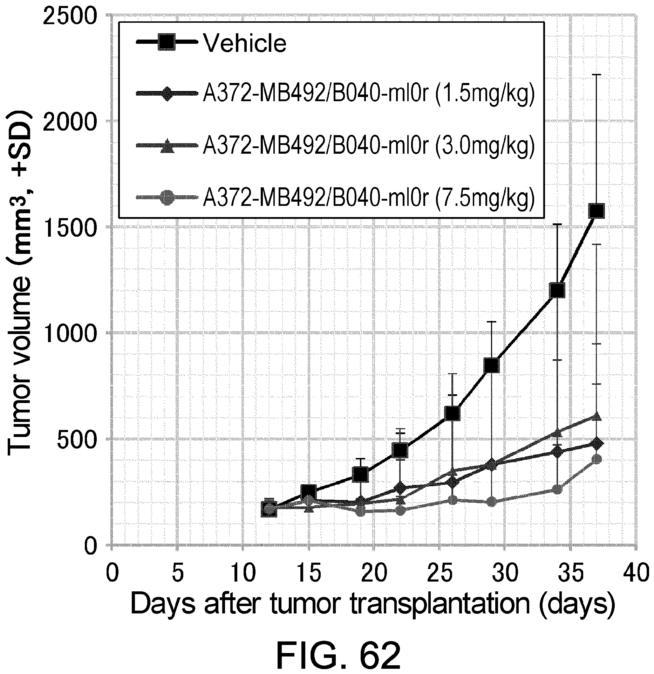

Fig. 62 is a diagram showing the anti-tumor effect of A372-MB492/B040-ml0r in a mouse model transplanted with the MC38 cell line.

Each dot shows the mean value of a group (n=5) of tumor volumes.

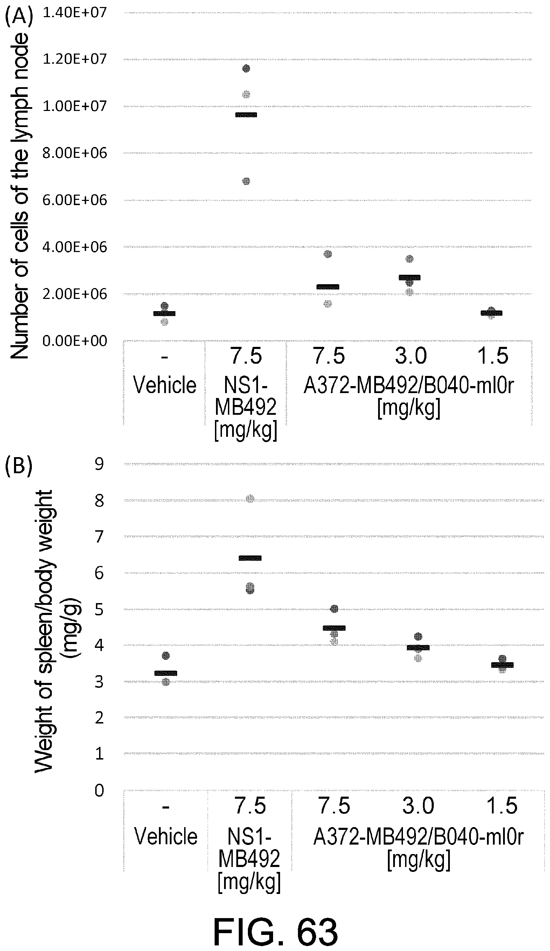

Fig. 63 is a diagram showing the number of cells of the lymph node and the organ weight of spleen in a mouse model transplanted with the MC38 cell line after administration of NS1-MB492 or A372-MB492/B040-ml0r.

Subfigure (A) shows the number of cells of the lymph node and subfigure (B) shows the organ weight of spleen.

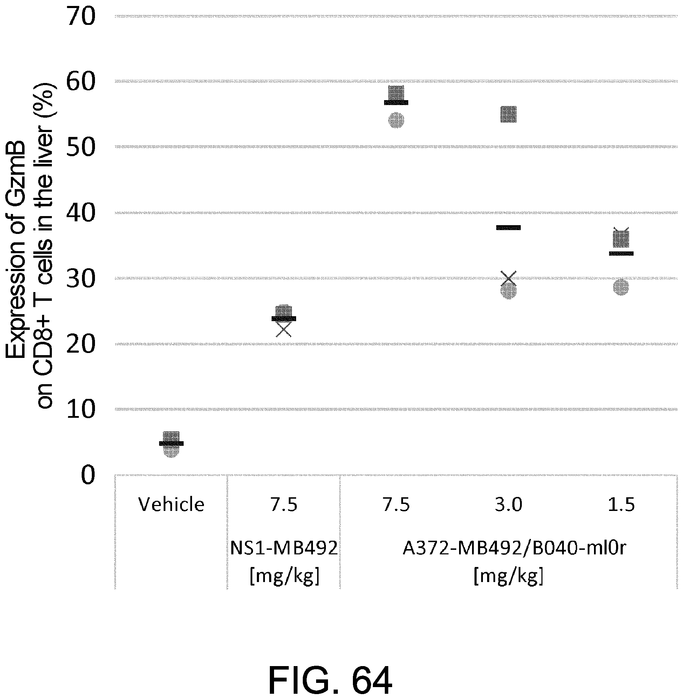

Fig. 64 is a diagram showing the degree of T cell activation in the liver of a mouse model transplanted with the MC38 cell line after administration of NS1-MB492 or A372-MB492/B040-ml0r (percentage of Granzyme B positive T cells in CD8 positive T cells).

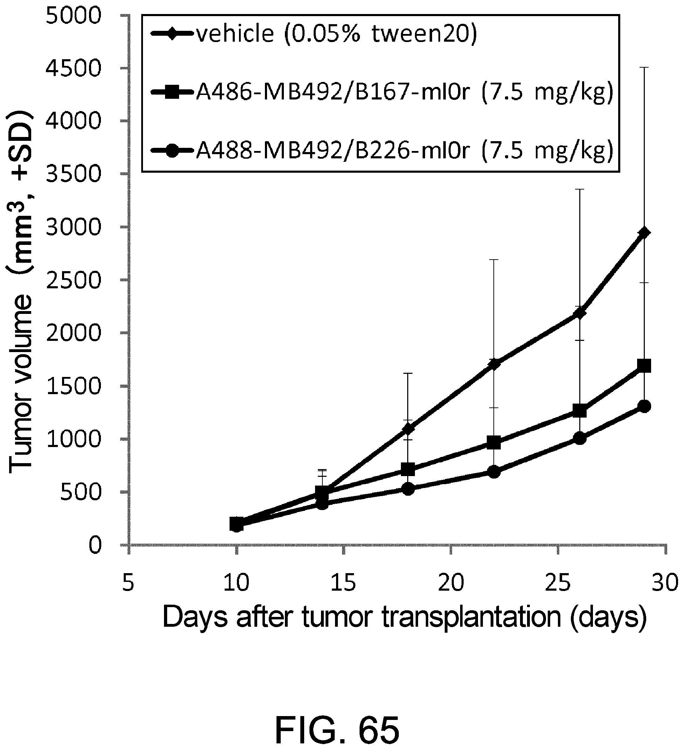

Fig. 65 is a diagram showing the anti-tumor effect of A486-MB492/B167-ml0r or A488-MB492/B226-ml0r in a mouse model transplanted with the MC38 cell line.

Each dot shows the mean value of a group (n=5) of tumor volumes.

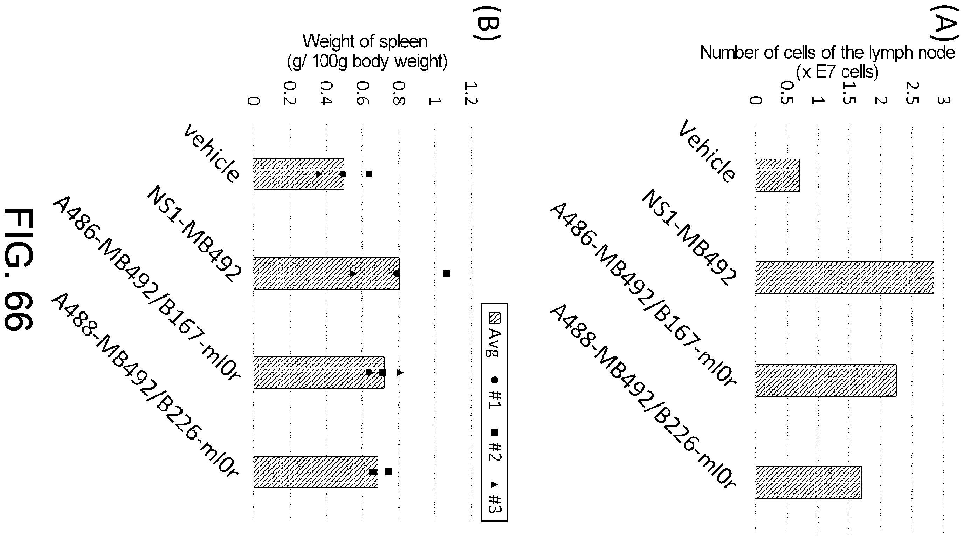

Fig. 66 is a diagram showing the number of cells per lymph node and the weight of spleen in a mouse model transplanted with the MC38 cell line after administration of NS1-MB492, A486-MB492/B167-ml0r, or A488-MB492/B226-ml0r.

Subfigure (A) shows the number of cells per lymph node and subfigure (B) shows the weight of spleen.

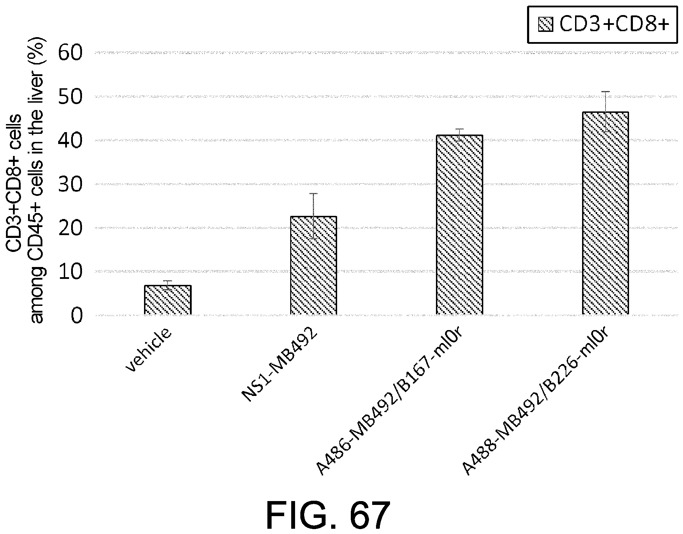

Fig. 67 is a diagram showing the level of infiltration of effector cells in the liver of a mouse model transplanted with the MC38 cell line after administration of NS1-MB492, A486-MB492/B167-ml0r, or A488-MB492/B226-ml0r (percentage of CD3 positive and CD8 positive T cells in CD45 positive T cells).

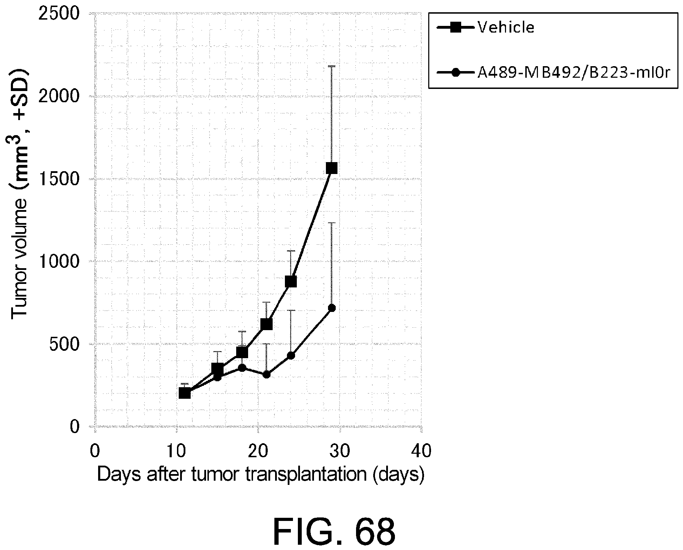

Fig. 68 is a diagram showing the anti-tumor effect of A489-MB492/B223-ml0r in a mouse model transplanted with the MC38 cell line.

Each dot shows the mean value of a group (n=5) of tumor volumes.

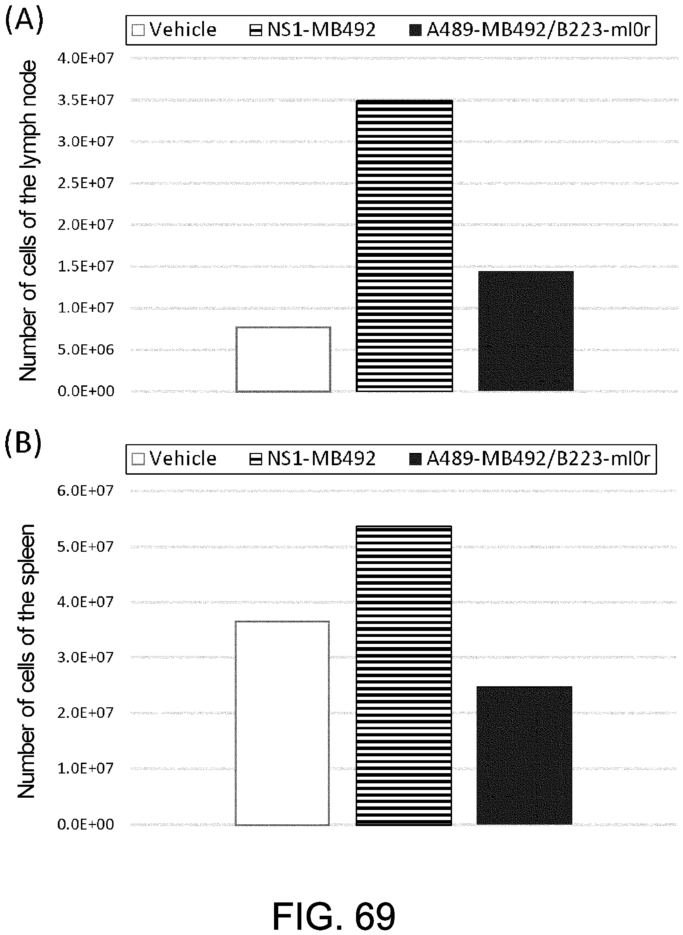

Fig. 69 is a diagram showing the number of cells of the lymph node and the number of cells in a lymphocyte fraction of spleen in a mouse model transplanted with the MC38 cell line after administration of NS1-MB492 or A489-MB492/B223-ml0r.

Subfigure (A) shows the number of cells of the lymph node and subfigure (B) shows the number of cells in a lymphocyte fraction of spleen.

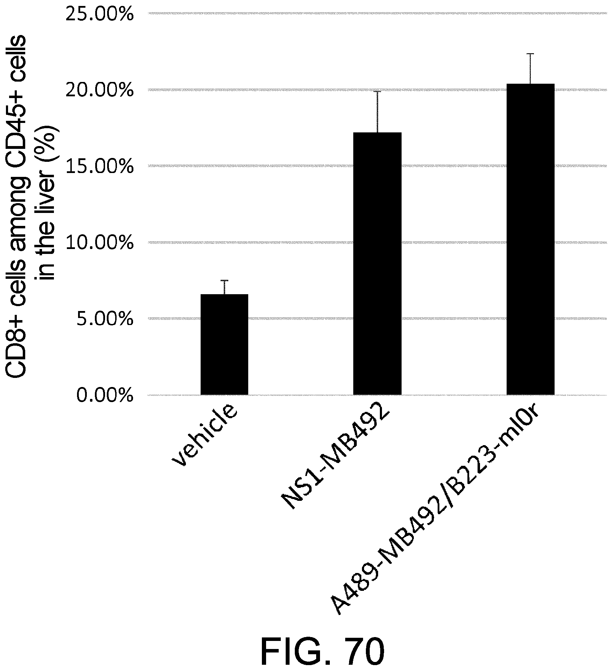

Fig. 70 is a diagram showing the degree of T cell activation in the liver of a mouse model transplanted with the MC38 cell line after administration of NS1-MB492 or A489-MB492/B223-ml0r (percentage of CD8 positive T cells in CD45 positive T cells).

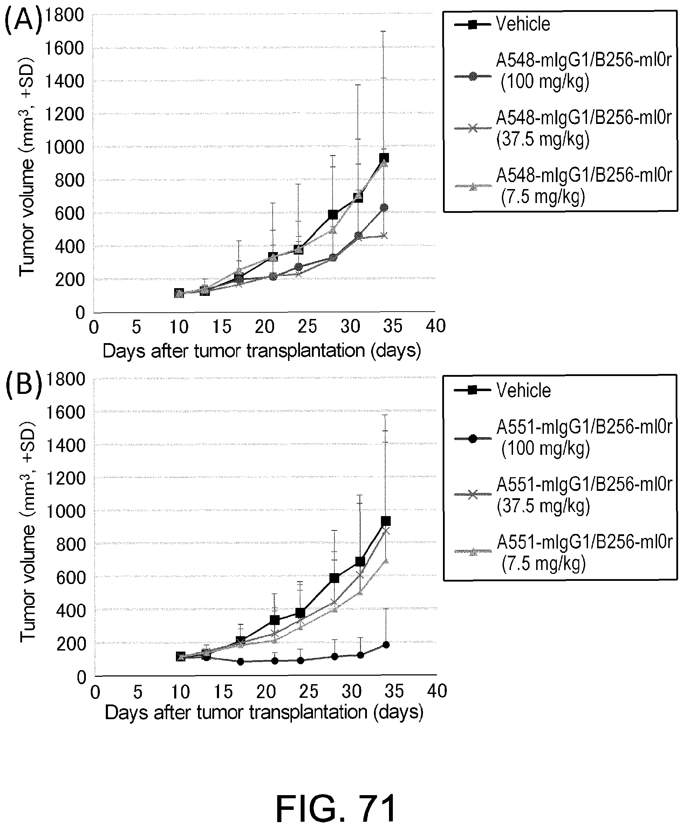

Fig. 71 is a diagram showing the anti-tumor effect of A548-mIgG1/B256-ml0r and A551-mIgG1/B256-ml0r in a mouse model transplanted with the MC38 cell line.

Subfigure (A) shows the anti-tumor effect of A548-mIgG1B256-ml0r and subfigure (B) shows the anti-tumor effect of A551-mIgG1/B256-ml0r.

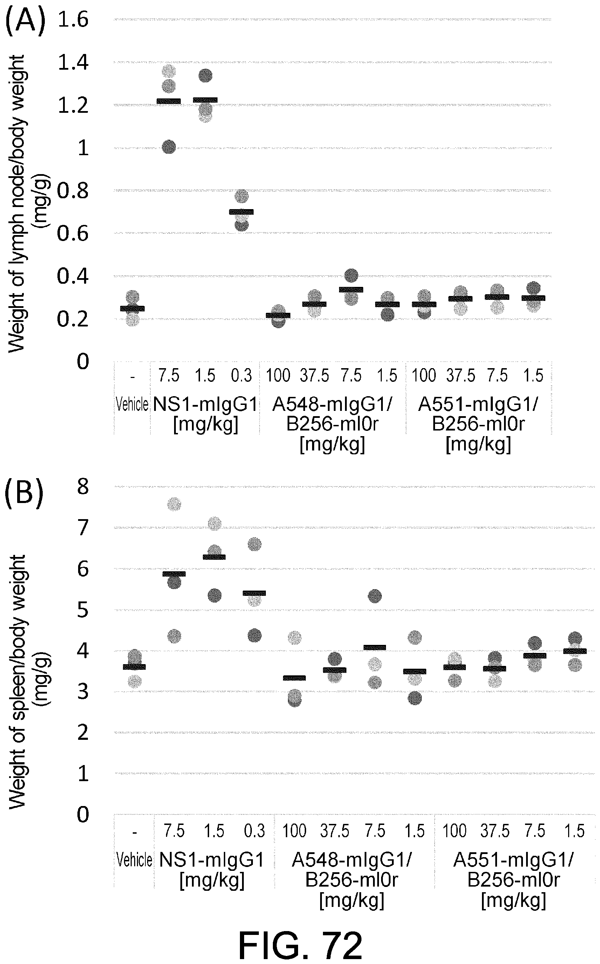

Fig. 72 is a diagram showing the weight of organs in a mouse model transplanted with the MC38 cell line after administration of NS1-mIgG1, A548-mIgG1/B256-ml0r, or A551-mIgG1/B256-ml0r.

Subfigure (A) shows the weight of lymph node and subfigure (B) shows the weight of spleen.

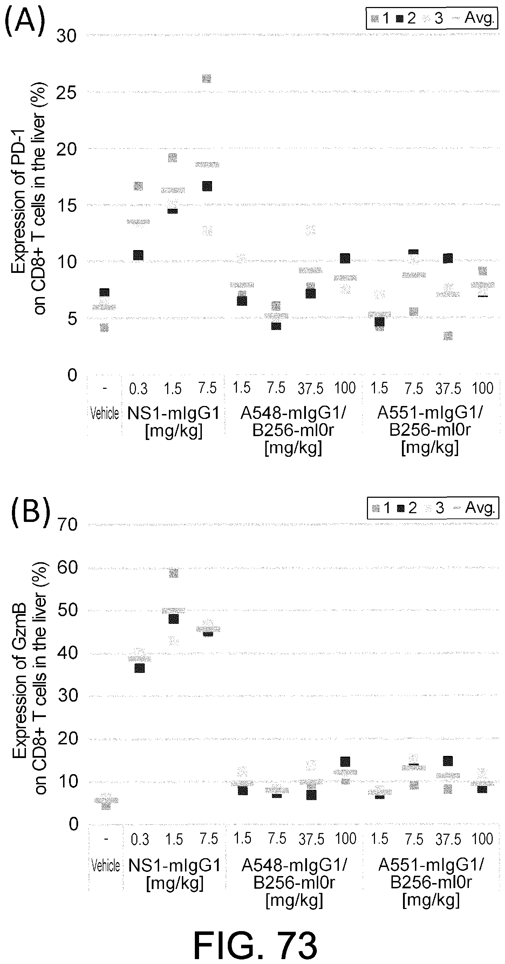

Fig. 73 is a diagram showing the degree of T cell activation in the liver of a mouse model transplanted with the MC38 cell line after administration of NS1-mIgG1, A548-mIgG1/B256-ml0r, or A551-mIgG1/B256-ml0r.

Subfigure (A) shows the percentage of PD-1 positive T cells in CD8 positive T cells, and subfigure (B) shows the percentage of Granzyme B positive T cells in CD8 positive T cells.

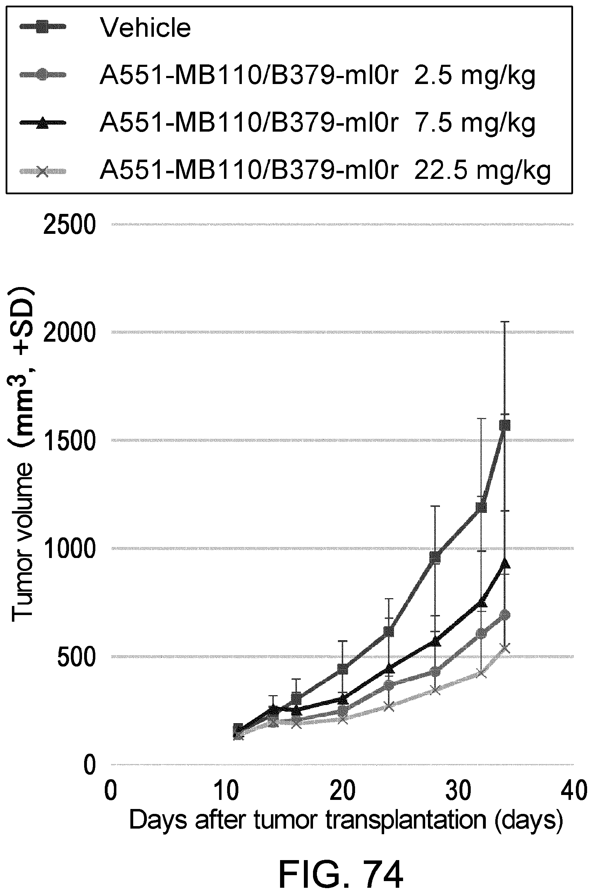

Fig. 74 is a diagram showing the anti-tumor effect of A551-MB110/B379-ml0r in a mouse model transplanted with the MC38 cell line.

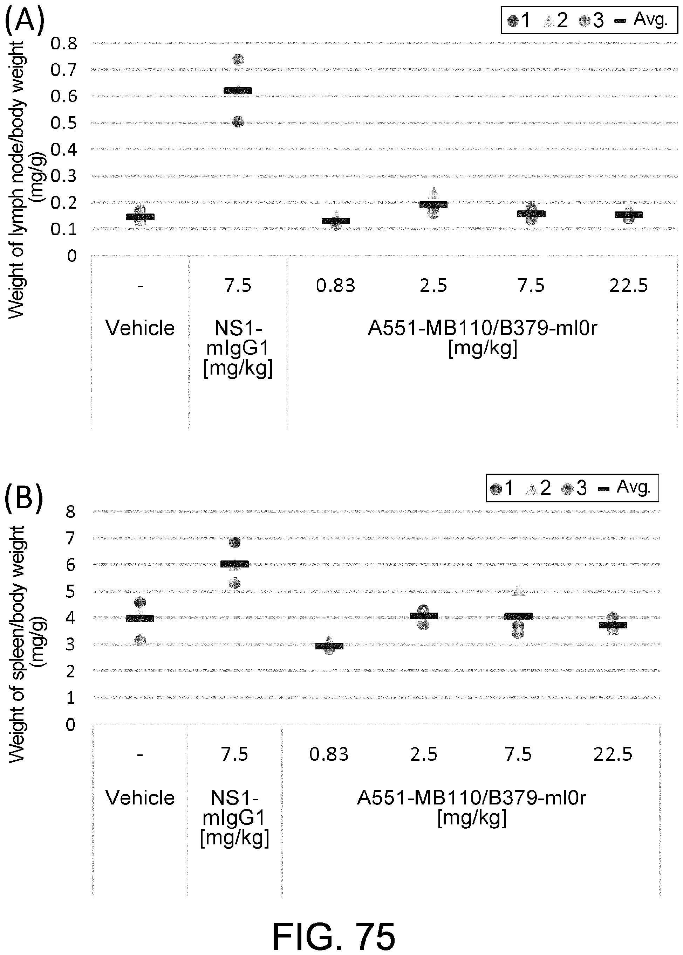

Fig. 75 is a diagram showing the weight of organs in a mouse model transplanted with the MC38 cell line after administration of NS1-mIgG1 or A551-MB110/B379-ml0r.

Subfigure (A) shows the weight of lymph node and subfigure (B) shows the weight of spleen.

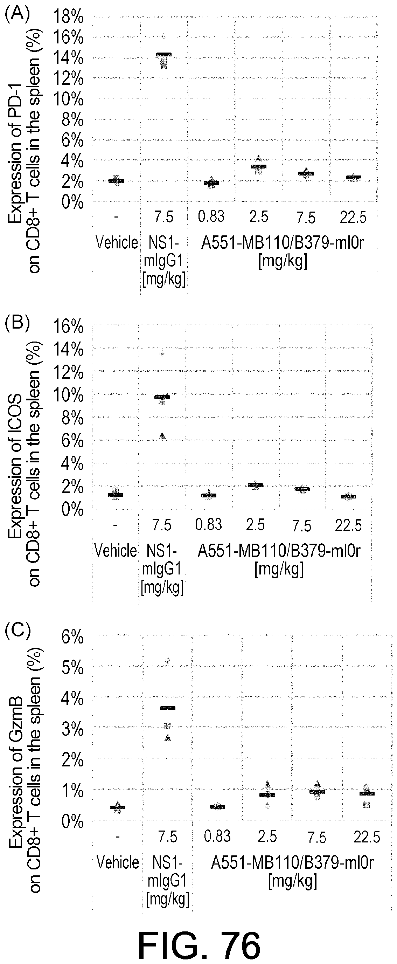

Fig. 76 is a diagram showing the degree of T cell activation in the spleen of a mouse model transplanted with the MC38 cell line after administration of NS1-mIgG1 or A551-MB110/B379-ml0r.

Subfigure (A) shows the percentage of PD-1 positive T cells in CD8 positive T cells, subfigure (B) shows the percentage of ICOS positive T cells in CD8 positive T cells, and subfigure (C) shows the percentage of Granzyme B positive T cells in CD8 positive T cells.

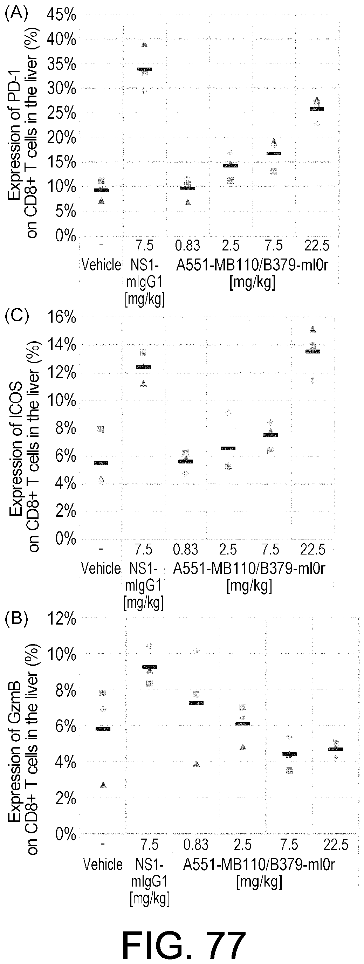

Fig. 77 is a diagram showing the degree of T cell activation in the liver of a mouse model transplanted with the MC38 cell line after administration of NS1-mIgG1 or A551-MB110/B379-ml0r.

Subfigure (A) shows the percentage of PD-1 positive T cells in CD8 positive T cells, subfigure (C) shows the percentage of ICOS positive T cells in CD8 positive T cells, and subfigure (B) shows the percentage of Granzyme B positive T cells in CD8 positive T cells.

The X axis shows the antibody concentration ($\mu$g/mL) and the Y axis shows the relative light unit.

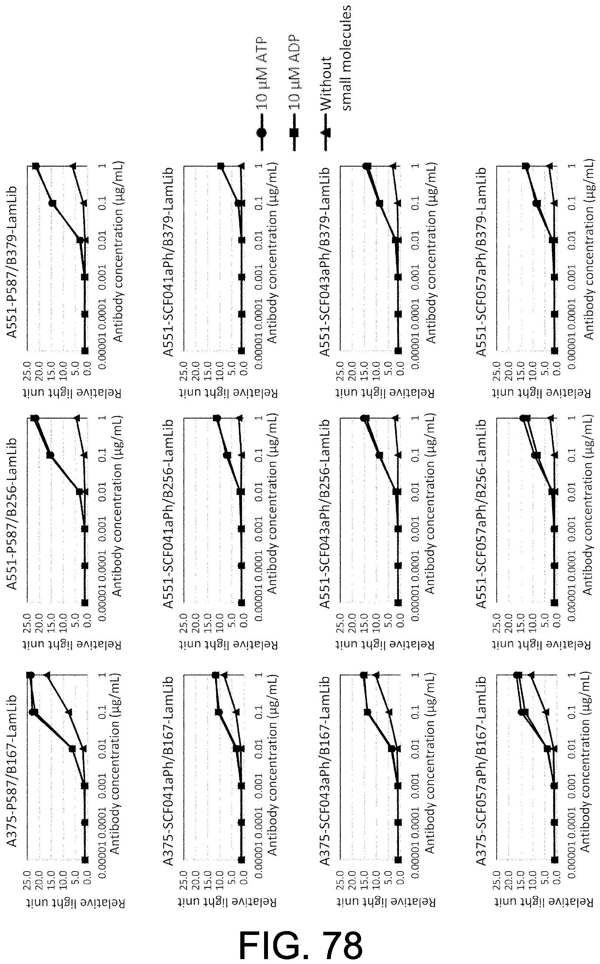

Fig. 78 is a diagram showing the agonist activity of various anti-CD137 antibodies tested using 4-1BB Jurkat cells in the presence or absence of the small molecule compound (ATP or ADP).

The X axis shows the antibody concentration ($\mu$g/mL) and the Y axis shows the relative light unit.

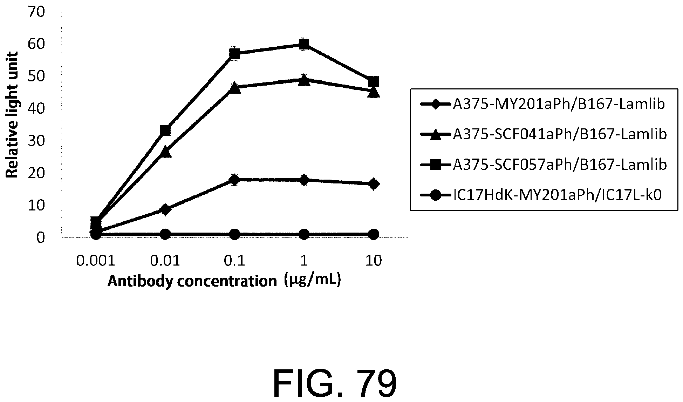

Fig. 79 is a diagram showing the agonist activity of various switch anti-CD137 antibodies tested using 4-1BB Jurkat reporter gene assay in the presence of ATP.

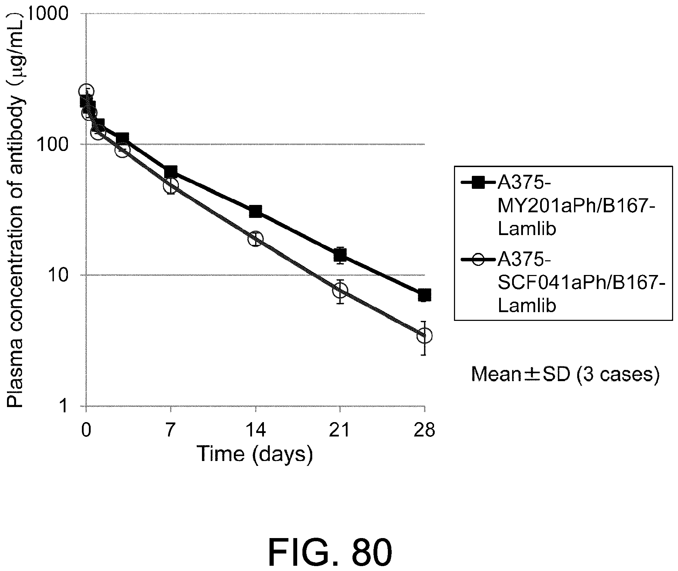

Fig. 80 is a diagram showing the comparison in plasma kinetics of each of the anti-CD137 switch antibodies A375-SCF041aPh/B167-Lamlib and A375-MY201aPh/B167-Lamlib. The vertical axis of the graph shows the plasma concentration of each antibody.

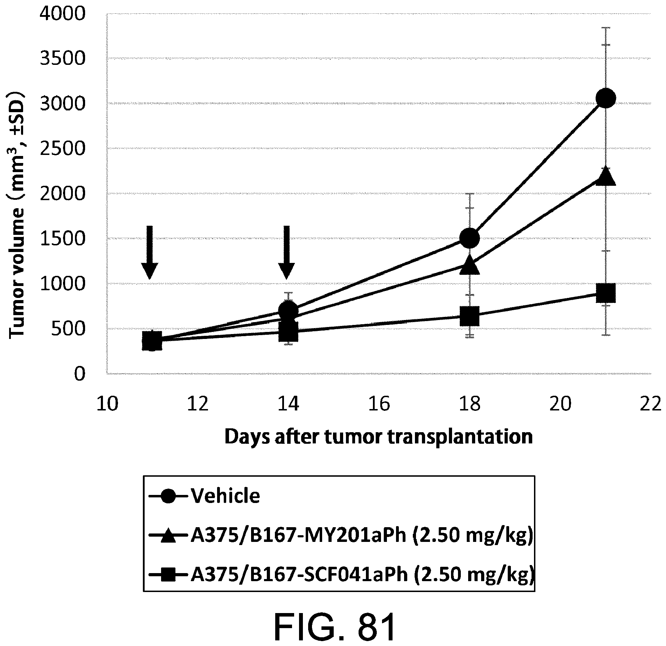

Fig. 81 is a diagram showing the anti-tumor effect of each of A375/B167-SCF041aPh and A375/B167-MY201aPh in a mouse model prepared by transplanting the LLC1/OVA/GPC3 cell line into hCD137KI/mFcγR2bKO/hFcγR2bTg#90 mice.

Each dot shows the mean value of a group (n=5) of tumor volumes.

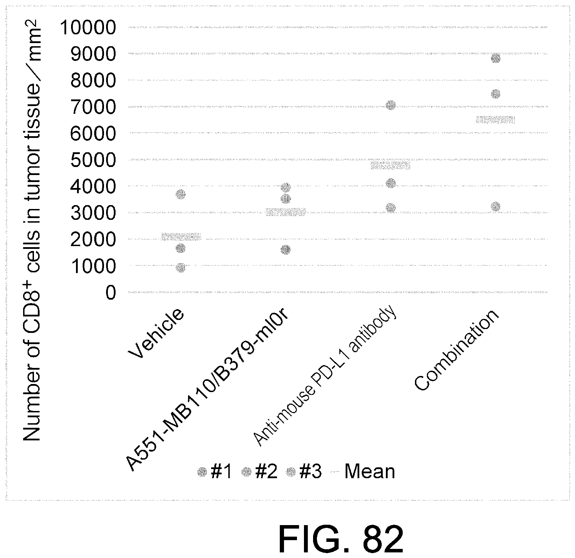

Fig. 82 shows the number of CD8⁺ cells in a tumor tissue after administration of A551-MB110/B379-ml0r, an anti-mouse PD-L1 antibody, or a combination thereof to a mouse model transplanted with MC38 cells.

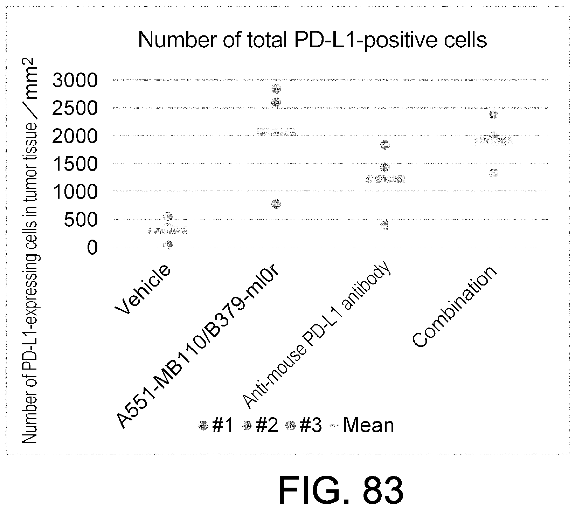

Fig. 83 shows the number of PD-L1⁺ cells in a tumor tissue after administration of A551-MB110/B379-ml0r, an anti-mouse PD-L1 antibody, or a combination thereof to a mouse model transplanted with MC38 cells.

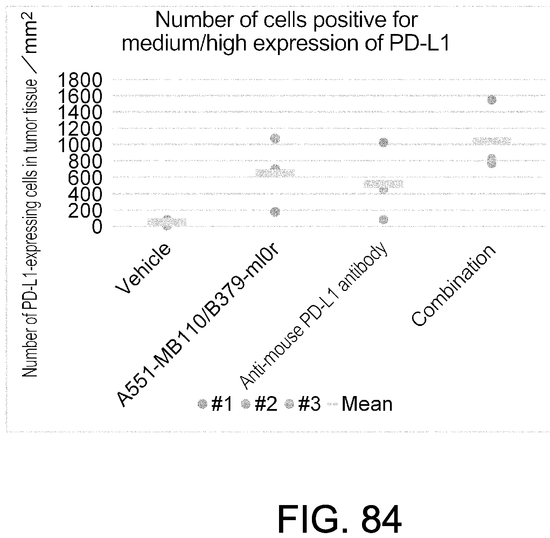

Fig. 84 shows the number of cells with medium or high expression of PD-L1 in a tumor tissue after administration of A551-MB110/B379-ml0r, an anti-mouse PD-L1 antibody, or a combination thereof to a mouse model transplanted with MC38 cells.

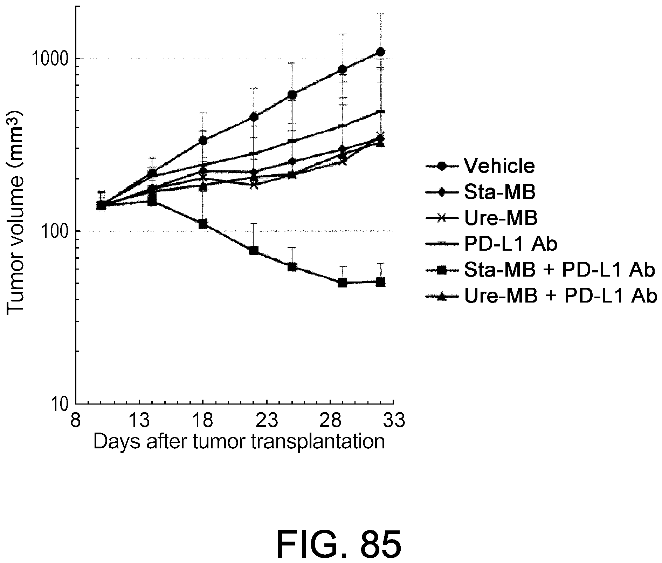

Fig. 85 shows the tumor growth suppression effect in a mouse model transplanted with MC38 cells by the administration of A551-MB110/B379-ml0r ("Sta-MB"), UreH-MB110/UreL-mk1 ("Ure-MB") or anti-mouse PD-L1 antibody ("PD-L1-Ab") alone, or combined administration of A551-MB110/B379-ml0r ("Sta-MB") and anti-mouse PD-L1 antibody ("PD-L1-Ab"), or combined administration of UreH-MB110/UreL-mk1 ("Ure-MB") and anti-mouse PD-L1 antibody ("PD-L1-Ab").

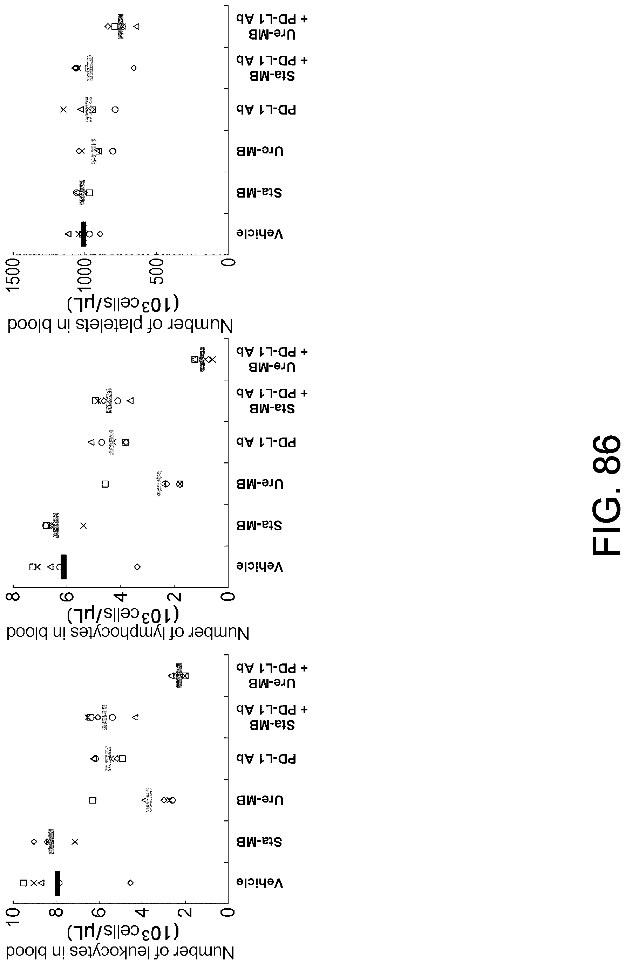

Fig. 86 shows the degree of fluctuation of blood parameters (white blood cell concentration, platelet concentration, and lymphocyte concentration) in a mouse model transplanted with MC38 cells due to the administration of A551-MB110/B379-ml0r ("Sta-MB"), UreH-MB110/UreL-mk1 ("Ure-MB") or anti-mouse PD-L1 antibody ("PD-L1-Ab") alone, or combined administration of A551-MB110/B379-ml0r ("Sta-MB") and anti-mouse PD-L1 antibody ("PD-L1-Ab"), or combined administration of UreH-MB110/UreL- mk1 ("Ure-MB") and anti-mouse PD-L1 antibody ("PD-L1-Ab").

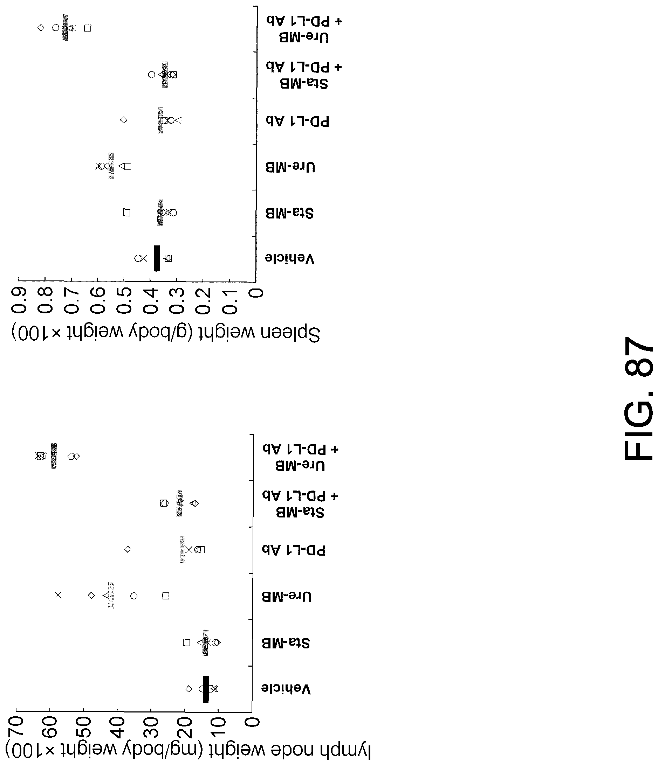

Fig. 87 shows the organ weight of the spleen and the tumor-draining lymph nodes (DLN) in a mouse model transplanted with MC38 cells following the administration of A551-MB110/B379-ml0r ("Sta-MB"), UreH-MB110/UreL-mk1 ("Ure-MB") or anti-mouse PD-L1 antibody ("PD-L1-Ab") alone, or combined administration of A551-MB110/B379-ml0r ("Sta-MB") and anti-mouse PD-L1 antibody ("PD-L1-Ab"), or combined administration of UreH-MB110/UreL-mk1 ("Ure-MB") and anti-mouse PD-L1 antibody ("PD-L1-Ab").

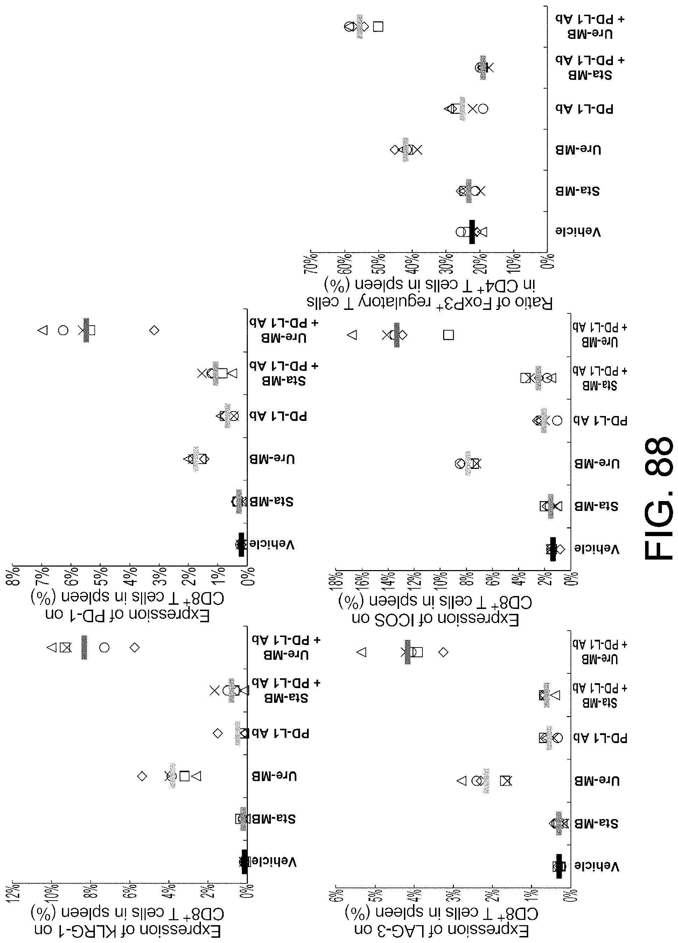

Fig. 88 shows the expression ratio of KLRG-1, ICOS, PD-1, LAG-3 in CD8⁺ T cells and the ratio of Foxp3⁺ regulatory T cells in CD4⁺ T cells in the spleen in a mouse model transplanted with MC38 cells following the administration of A551-MB110/B379-ml0r ("Sta-MB"), UreH-MB110/UreL-mk1 ("Ure-MB") or anti-mouse PD-L1 antibody ("PD-L1-Ab") alone, or combined administration of A551-MB110/B379-ml0r ("Sta-MB") and anti-mouse PD-L1 antibody ("PD-L1-Ab"), or combined administration of UreH-MB110/UreL-mk1 ("Ure-MB") and anti-mouse PD-L1 antibody ("PD-L1-Ab").

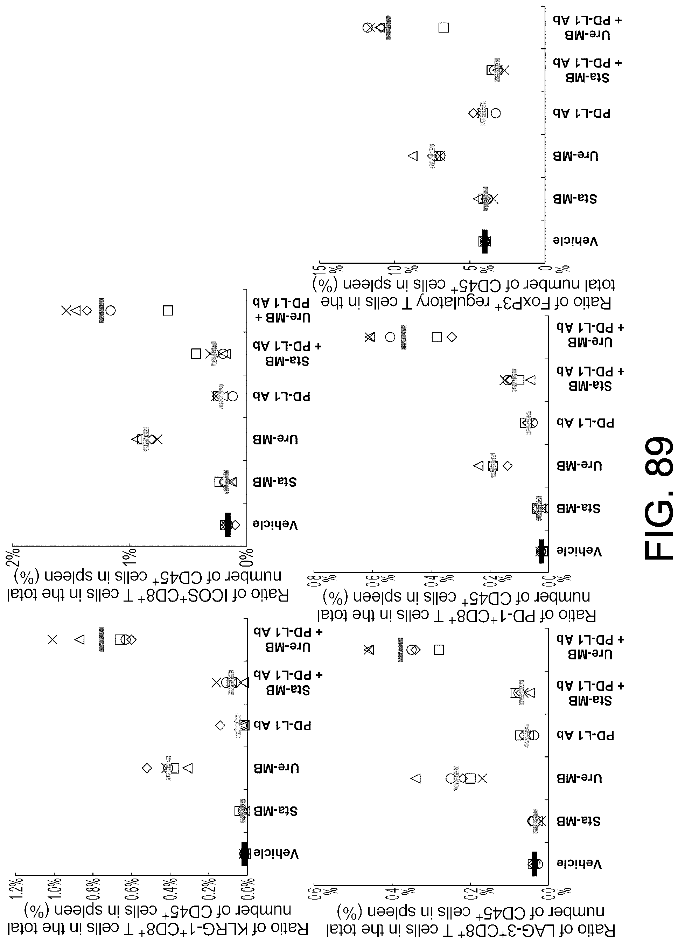

Fig. 89 shows the ratio of each activation marker-positive CD8⁺ T cells in CD45⁺ leukocytes and Foxp3⁺ regulatory T cell ratio in CD45⁺ leukocytes in the spleen in a mouse model transplanted with MC38 cells, following the administration of A551-MB110/B379-ml0r ("Sta-MB"), UreH-MB110/UreL-mk1 ("Ure-MB") or anti-mouse PD-L1 antibody ("PD-L1-Ab") alone, or combined administration of A551-MB110/B379-ml0r ("Sta-MB") and anti-mouse PD-L1 antibody ("PD-L1-Ab"), or combined administration of UreH-MB110/UreL-mk1 ("Ure-MB") and anti-mouse PD-L1 antibody ("PD-L1-Ab").

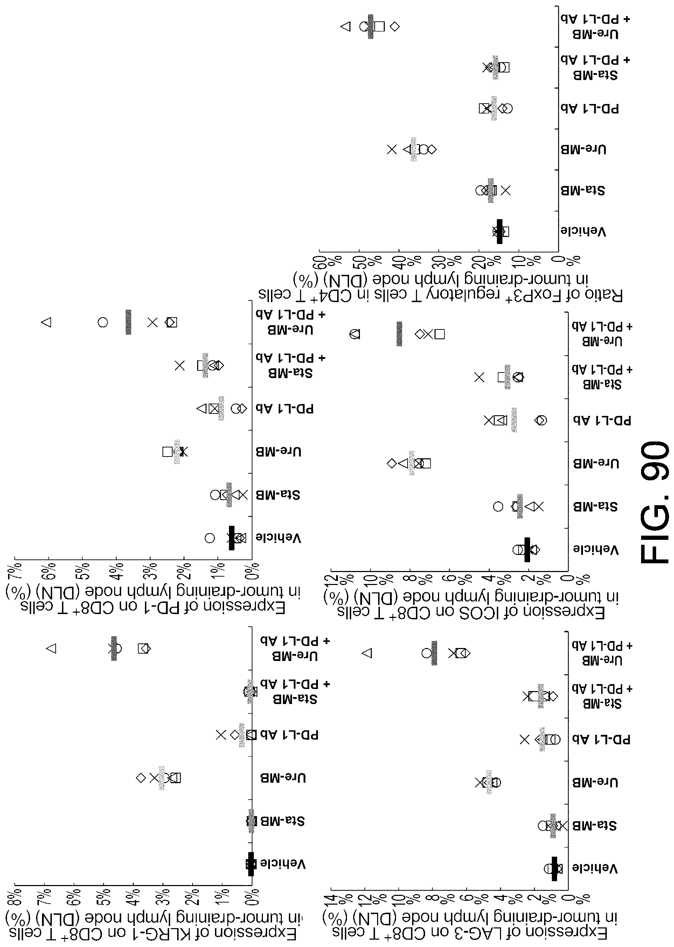

Fig. 90 shows the expression ratio of KLRG-1, ICOS, PD-1, and LAG-3 in CD8⁺ T cells and the ratio of Foxp3⁺ regulatory T cells in CD4⁺ T cells in tumor-draining lymph nodes (DLN) in a mouse model transplanted with MC38 cells, following the administration of A551-MB110/B379-ml0r ("Sta-MB"), UreH-MB110/UreL-mk1 ("Ure-MB") or anti-mouse PD-L1 antibody ("PD-L1-Ab") alone, or combined administration of A551-MB11 0/B379-ml0r ("Sta-MB") and anti-mouse PD-L1 antibody ("PD-L1-Ab"), or combined administration of UreH-MB110/UreL-mk1 ("Ure-MB") and anti-mouse PD-L1 antibody ("PD-L1-Ab").

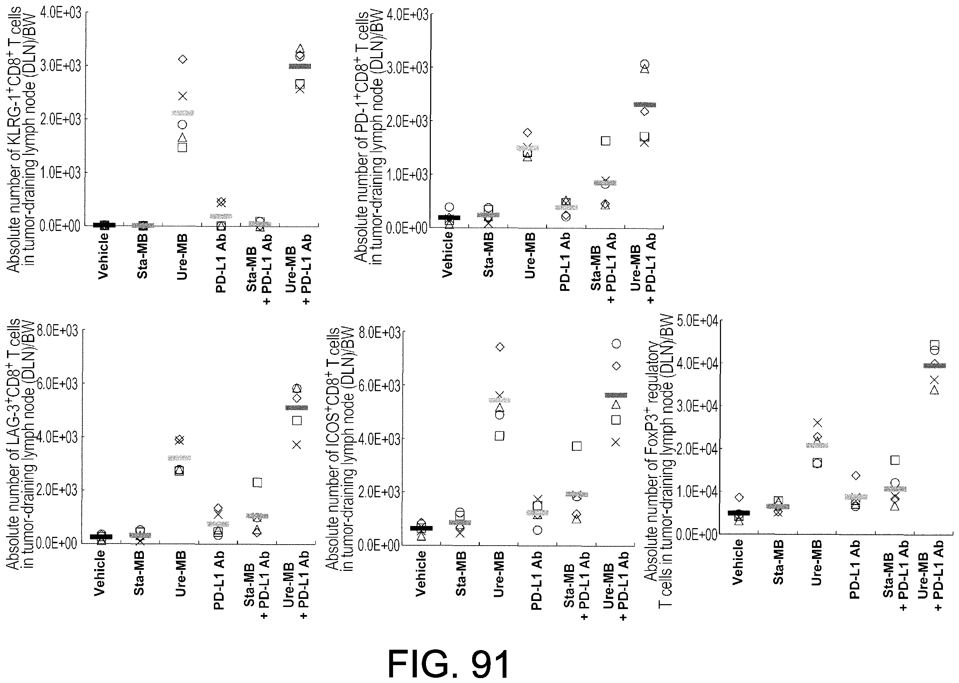

Fig. 91 shows the absolute number of each activation marker-positive CD8⁺ T cells and the absolute number of Foxp3⁺ regulatory T cells in CD4⁺ T cells in tumor-draining lymph nodes (DLN) in a mouse model transplanted with MC38 cells, following the administration of A551-MB110/B379-ml0r ("Sta-MB"), UreH-MB110/UreL-mk1 ("Ure-MB") or anti-mouse PD-L1 antibody ("PD-L1-Ab") alone, or combined administration of A551-MB110/B379-ml0r ("Sta-MB") and anti-mouse PD-L1 antibody ("PD-L1-Ab"), or combined administration of UreH-MB110/UreL-mk1 ("Ure-MB") and anti-mouse PD-L1 antibody ("PD-L1-Ab").

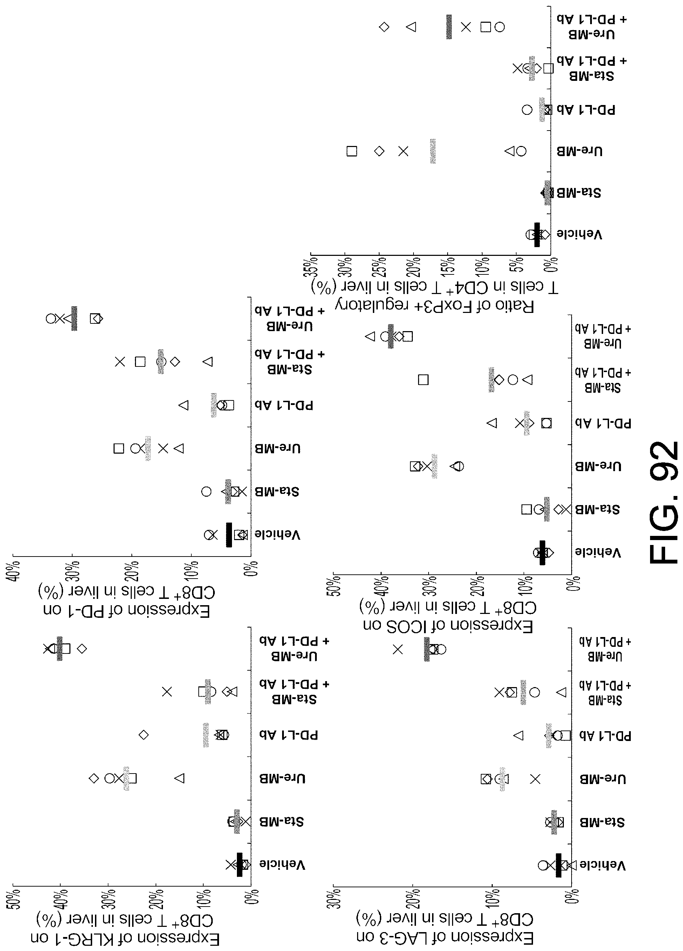

Fig. 92 shows the expression ratio of KLRG-1, ICOS, PD-1, and LAG-3 in CD8⁺ T cells and the ratio of Foxp3⁺ regulatory T cells in CD4⁺ T cells in the liver in a mouse model transplanted with MC38 cells, following the administration of A551-MB110/B379-ml0r ("Sta-MB"), UreH-MB110/UreL-mk1 ("Ure-MB") or anti-mouse PD-L1 antibody ("PD-L1-Ab") alone, or combined administration of A551-MB110/B379-ml0r ("Sta-MB") and anti-mouse PD-L1 antibody ("PD-L1-Ab"), or combined administration of UreH-MB110/UreL-mk1 ("Ure-MB") and anti-mouse PD-L1 antibody ("PD-L1-Ab").

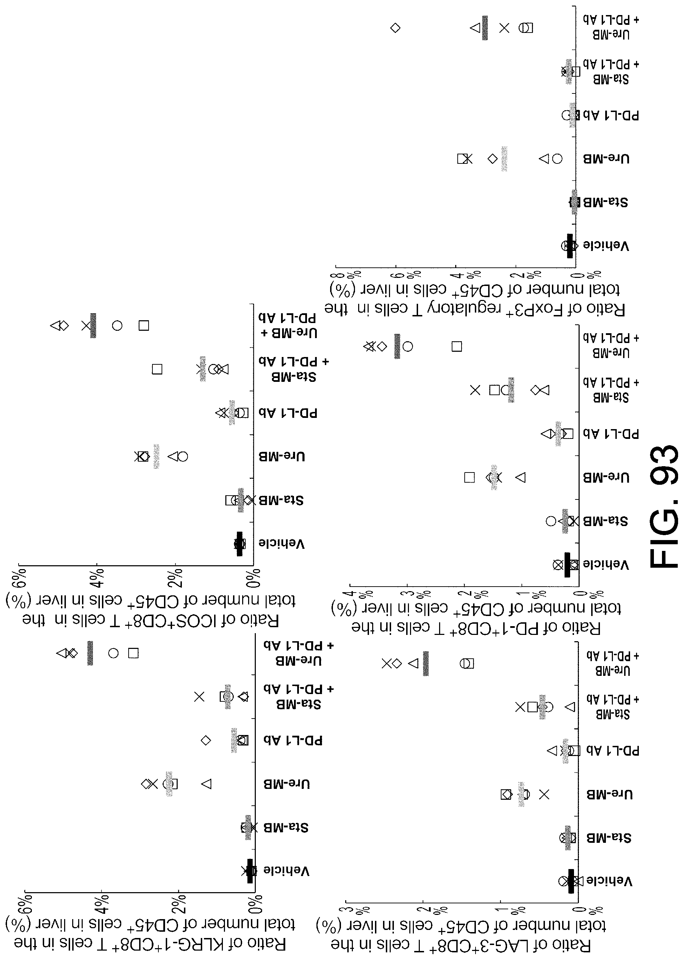

Fig. 93 shows the ratio of each activation marker-positive CD8⁺ T cells in CD45⁺ leukocytes and the ratio of Foxp3⁺ regulatory T cells in CD45⁺ leukocytes in the liver in a mouse model transplanted with MC38 cells, following the administration of A551-MB110/B379-ml0r ("Sta-MB"), UreH-MB110/UreL-mk1 ("Ure-MB") or anti-mouse PD-L1 antibody ("PD-L1-Ab") alone, or combined administration of A551-MB110/B379-ml0r ("Sta-MB") and anti-mouse PD-L1 antibody ("PD-L1-Ab"), or a combined administration of UreH-MB110/UreL-mk1 ("Ure-MB") and anti-mouse PD-L1 antibody ("PD-L1-Ab").

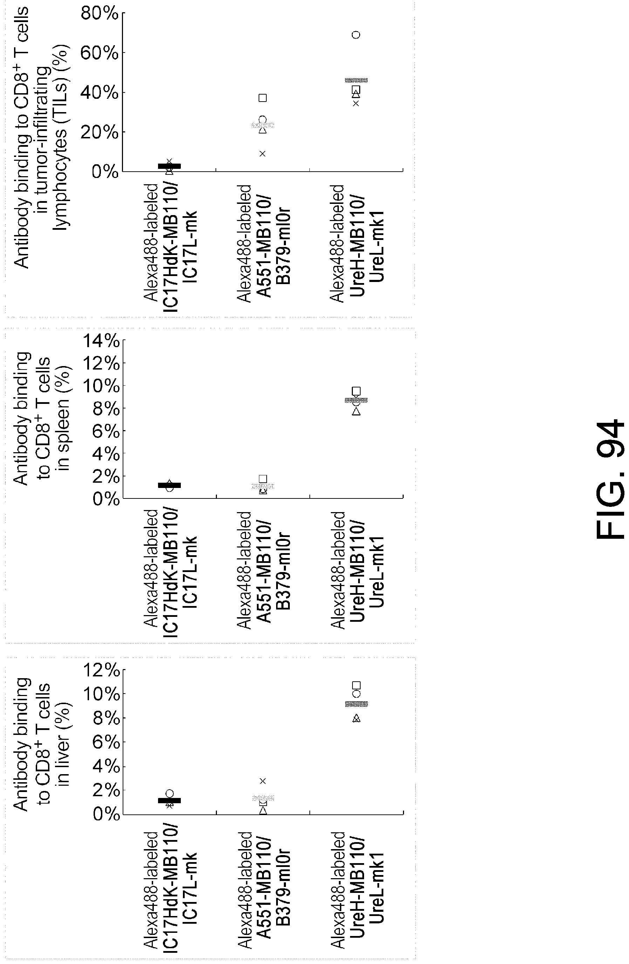

Fig. 94 shows the degree of binding of Alexa488-labeled A551-MB110/B379-ml0r, Alexa488-labeled IC17HdK-MB110/IC17L-mk (negative control substance), and the non-switch anti-CD137 antibody Alexa488-labeled UreH-MB110/UreL-mk1 (positive control substance) to the lymphocyte fraction in a tumor, spleen, and liver in a mouse model transplanted with MC38 cells as analyzed by flow cytometry (FCM).

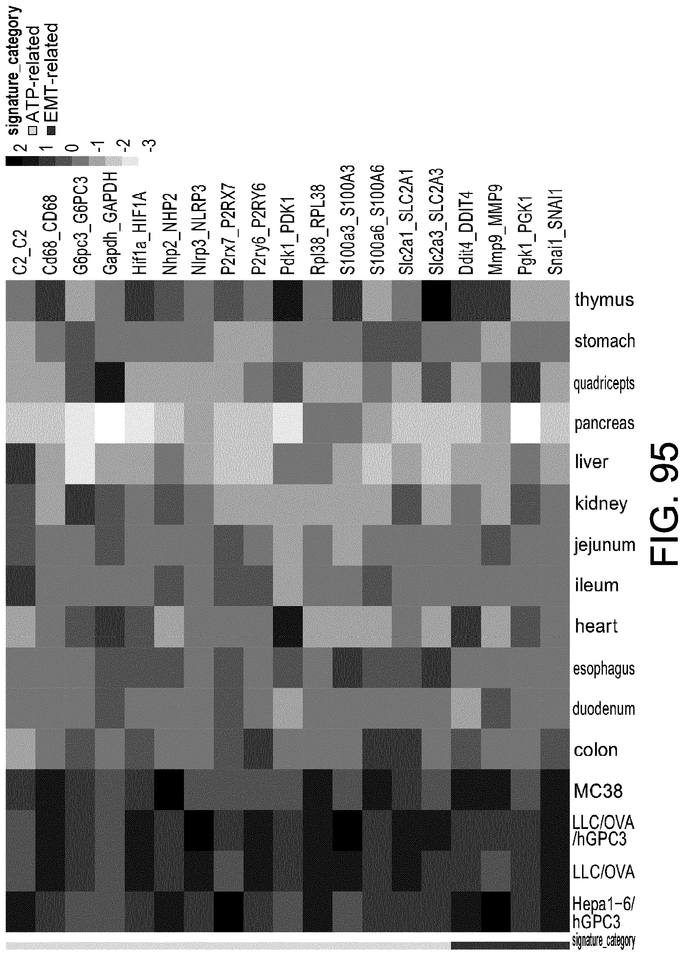

Fig. 95 shows the heat map of a group of genes which show an expression change associated with extracellular ATP in various normal organ tissues and various tumor tissues.

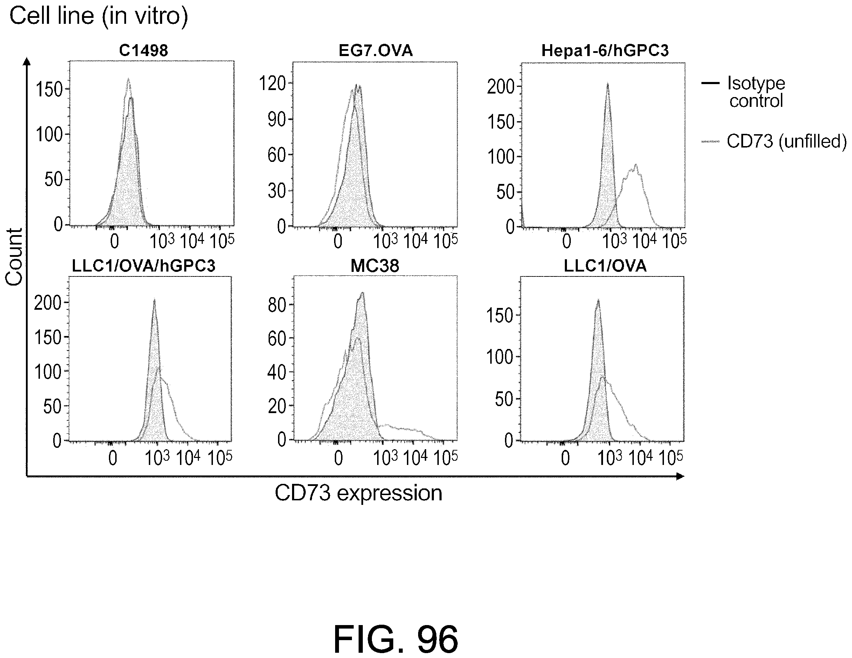

Fig. 96 shows the degree of expression of CD73 in various cancer cell lines as analyzed by flow cytometry (FCM).

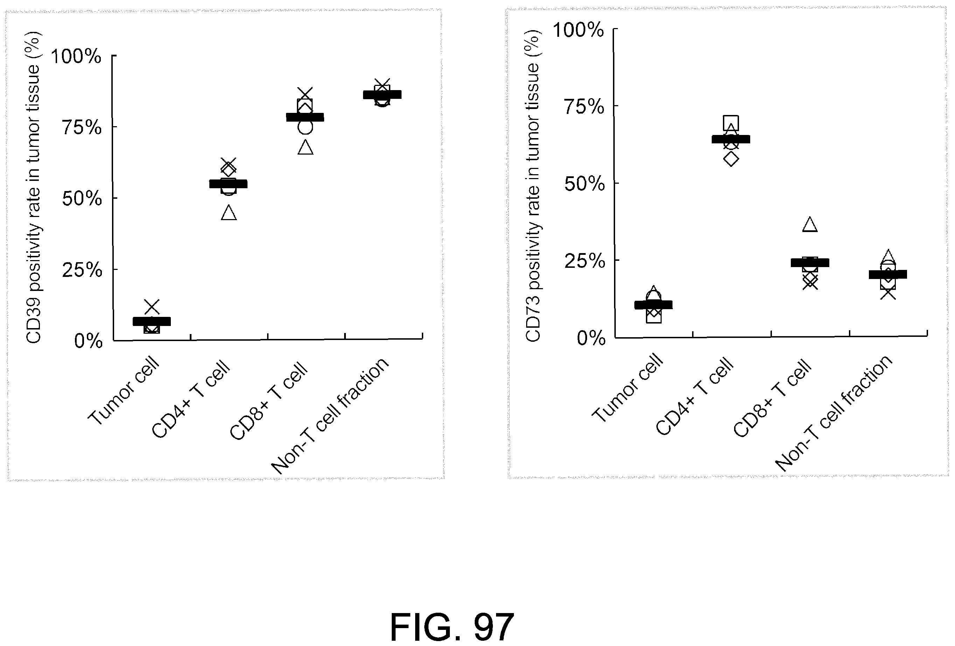

Fig. 97 shows the rate of expression of CD39 and CD73 in tumor cells, CD4$^+$ T cells, CD8$^+$ T cells, and non-T cells from mice transplanted with the LLC1/OVA/GPC3 clone C5 cell line, as analyzed by flow cytometry (FCM).

Regarding the expression of CD39 and CD73, tumor cells, CD4$^+$ T cells, CD8$^+$ T cells, and non-T cells were each tested 5 times using different samples, and the mean value was calculated.

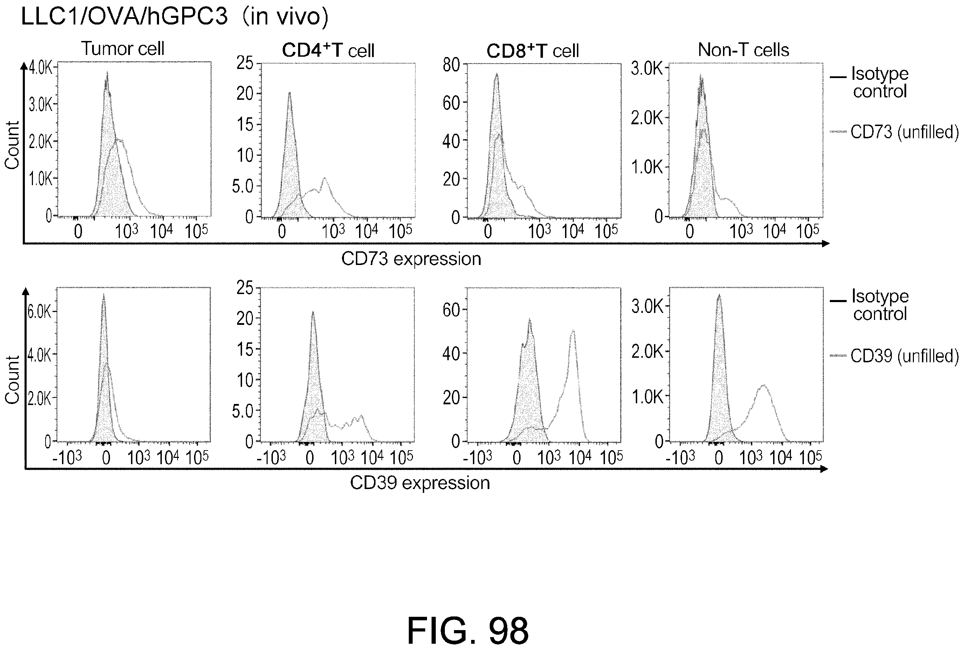

Fig. 98 is a representative figure showing the rate of expression of CD39 and CD73 in tumor cells, CD4$^+$ T cells, CD8$^+$ T cells, and non-T cells from mice transplanted with the LLC1/OVA/GPC3 clone C5 cell line, as analyzed by flow cytometry (FCM).

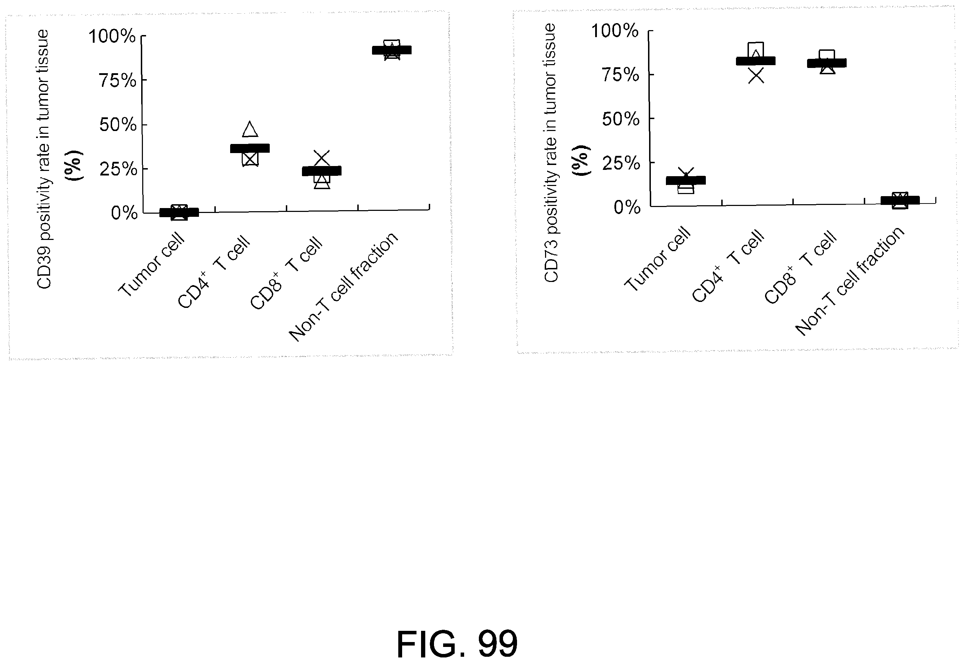

Fig. 99 shows the expression rate of CD39 and CD73 in tumor cells, CD4$^+$ T cells, CD8$^+$ T cells, and non-T cells from mice transplanted with the MC38 cell line, as analyzed by flow cytometry (FCM).

Regarding the expression of CD39 and CD73, tumor cells, CD4$^+$ T cells, CD8$^+$ T cells, and non-T cells were each tested 5 times using different samples, and the mean value was calculated.

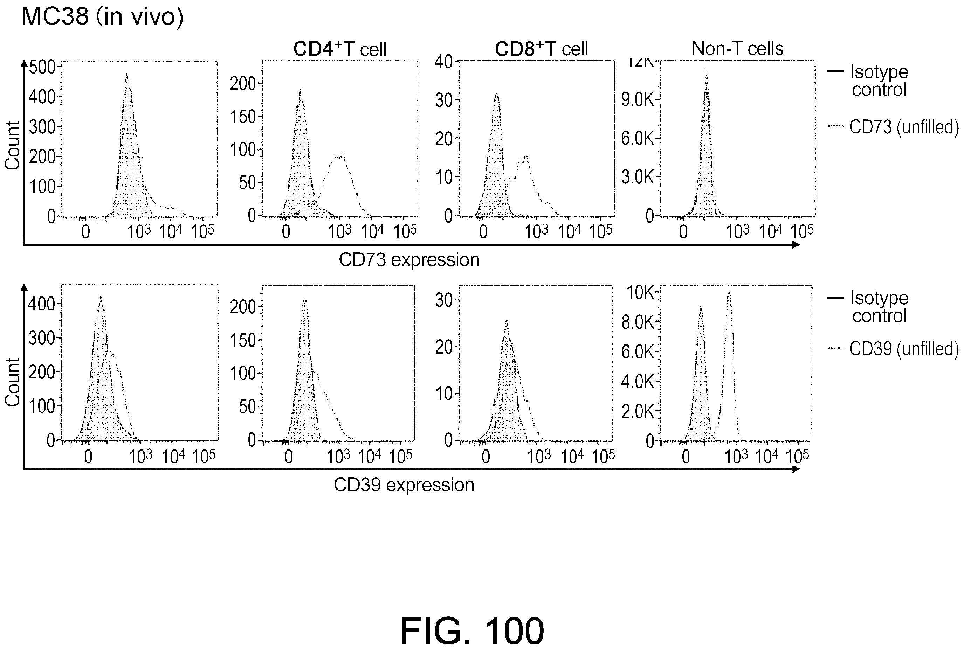

Fig. 100 is a representative figure showing the expression rates of CD39 and CD73 in tumor cells, CD4$^+$ T cells, CD8$^+$ T cells, and non-T cells from mice transplanted with the MC38 cell line, as analyzed by flow cytometry (FCM).

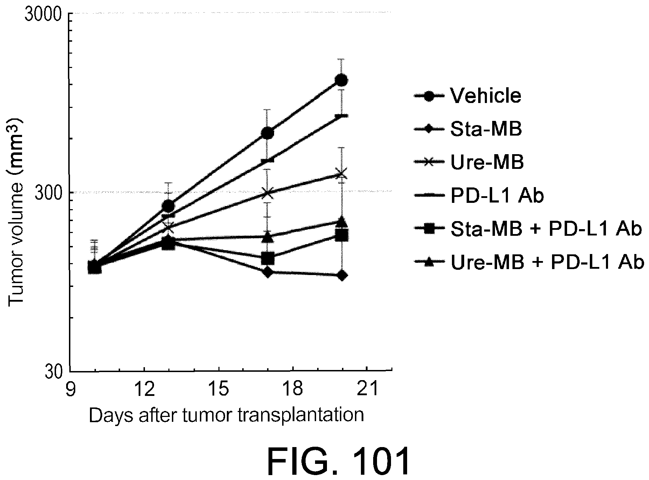

Fig. 101 shows the antitumor effect in a mouse model transplanted with LLC1/OVA cells by the administration of A551-MB110/B379-ml0r ("Sta-MB"), UreH-MB110/UreL-mk1 ("Ure-MB") or anti-mouse PD-L1 antibody ("PD-L1-Ab") alone, or combined administration of A551-MB110/B379-ml0r ("Sta-MB") and anti-mouse PD-L1 antibody ("PD-L1-Ab"), or combined administration of UreH-MB110/UreL-mk1 ("Ure-MB") and anti-mouse PD-L1 antibody ("PD-L1-Ab").

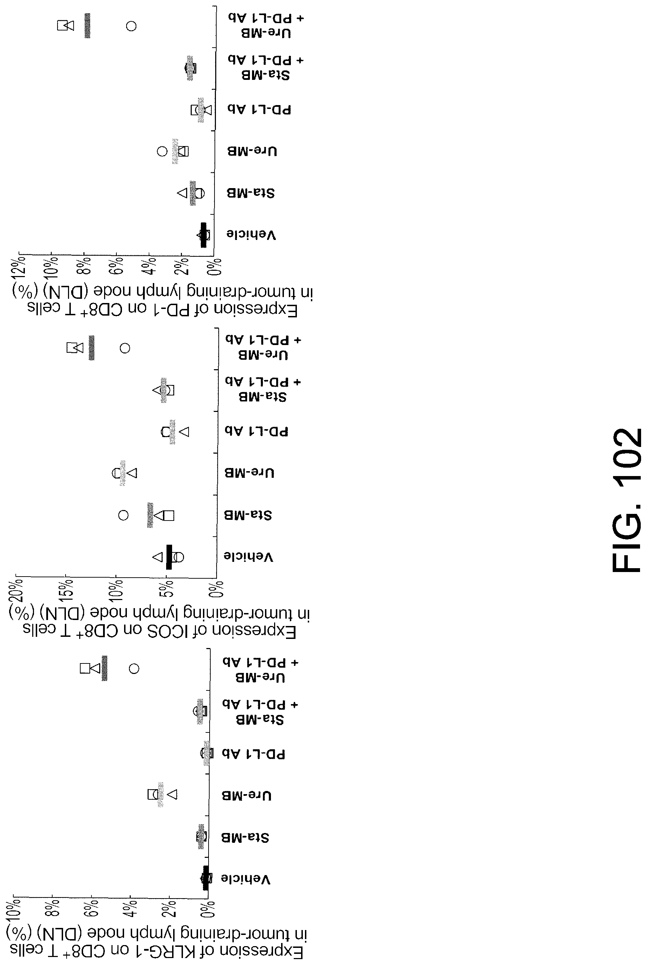

Fig. 102 shows the expression rate of KLRG-1, ICOS, and PD-1 in CD8$^+$ T cells in tumor-draining lymph nodes (DLN) in a mouse model transplanted with LLC1/OVA cells, following the administration of A551-MB110/B379-ml0r ("Sta-MB"), UreH-MB110/UreL-mk1 ("Ure-MB") or anti-mouse PD-L1 antibody ("PD-L1-Ab") alone, or combined administration of A551-MB110/B379-ml0r ("Sta-MB") and anti-mouse PD-L1 antibody ("PD-L1-Ab"), or combined administration of UreH-MB110/UreL-mk1 ("Ure-MB") and anti-mouse PD-L1 antibody ("PD-L1-Ab").

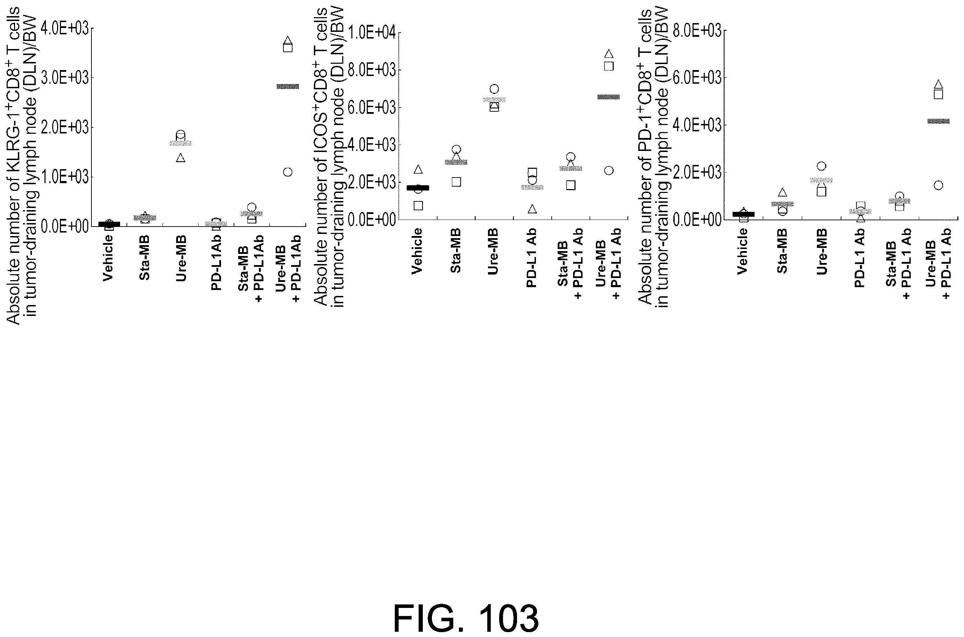

Fig. 103 shows the absolute number of each activation marker-positive CD8$^+$ T cells in tumor-draining lymph nodes (DLN) in a mouse model transplanted with LLC1/OVA cells, following the administration of A551-MB110/B379-ml0r ("Sta-MB"), UreH-MB110/UreL-mk1 ("Ure-MB") or anti-mouse PD-L1 antibody ("PD-L1-Ab") alone, or combined administration of A551-MB110/B379-ml0r ("Sta-MB") and anti-mouse PD-L1 antibody ("PD-L1-Ab"), or combined administration of UreH-MB110/UreL-mk1 ("Ure-MB") and anti-mouse PD-L1 antibody ("PD-L1-Ab").

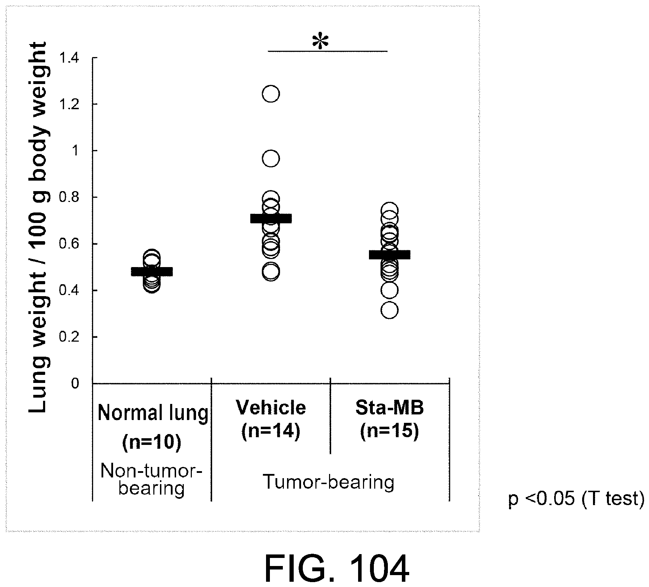

Fig. 104 shows the tumor growth suppression effect in a mouse model transplanted with LLC1/OVA cells by administration of A551-MB110/B379-ml0r ("Sta-MB") alone.

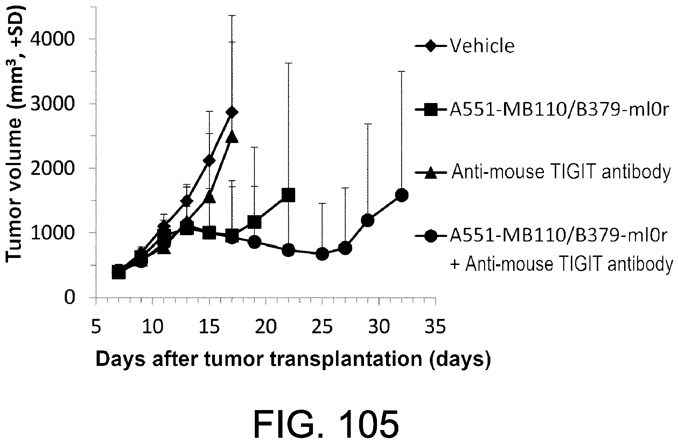

Fig. 105 shows the tumor growth suppression effect by administration of the switch anti-CD 137 antibody A551-MB110/B379-ml0r or anti-TIGIT antibody alone, or a combination of the switch anti-CD 137 antibody A551-MB110/B379-ml0r and anti-TIGIT antibody, in a mouse model transplanted with the mouse AML C1498 cell line.

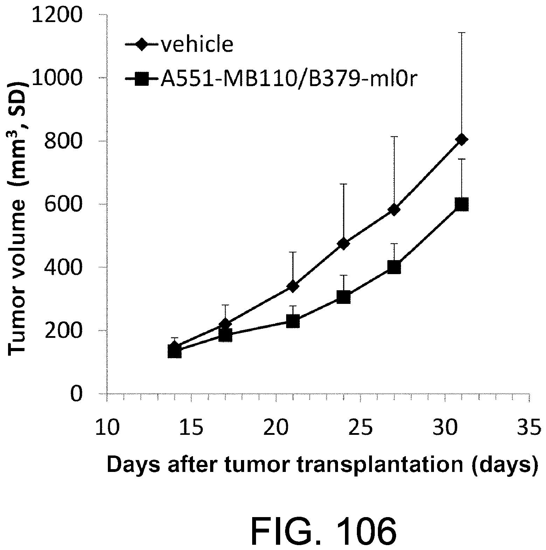

Fig. 106 shows the antitumor effect of the switch anti-CD 137 antibody A551-MB110/B379-ml0r in a mouse model transplanted with the MC38-hGPC3#G64 B2M KO clone 5 cell line.

[Description of Embodiments]

I. Definitions

The term "binding activity" refers to the strength of the sum total of noncovalent interactions between one or more binding sites of a molecule (e.g., an antibody) and its binding partner (e.g., an antigen). Herein, "binding activity" is not strictly limited to a 1:1 interaction between members of a binding pair (e.g., antibody and antigen). For example, when the members of a binding pair reflect a monovalent 1:1 interaction, the binding activity is particularly called the intrinsic binding affinity (affinity). When a member of a binding pair is capable of both monovalent binding and multivalent binding, the binding activity is the sum of each binding strength. The binding activity of a molecule X for its partner Y can generally be represented by the dissociation constant (KD) or "binding amount of analyte per unit amount of ligand" (hereinbelow, may be referred to as "binding amount"). Those skilled in the art would understand that, generally, lower value of dissociation constant (KD) means higher binding activity, and higher value of "binding amount of analyte per unit amount of ligand" or "binding amount" means higher binding activity. Binding activity can be measured by common methods known in the art, including those described herein. Specific illustrative and exemplary embodiments for measuring binding activity are described in the following.

A "binding activity-matured" antigen-binding molecule or antibody, or "binding activity-increased (enhanced)" antigen-binding molecule or antibody refers to an antibody with one or more alterations in one or more hypervariable regions (HVRs), compared to a parent antigen-binding molecule or a parent antibody which does not carry such alterations, such alterations resulting in an improvement in the binding activity of the antigen-binding molecule or antibody for antigen.

The terms "anti-CD 137 antigen-binding molecule" or "anti-CD 137 antibody" and "an antigen-binding molecule that binds to CD137" or "an antibody that binds to CD137" refer to an antigen-binding molecule or antibody that is capable of binding to CD137 with sufficient binding activity such that the antigen-binding molecule or antibody is useful as a diagnostic and/or therapeutic agent in targeting CD137. In certain embodiments, an anti-CD 137 antibody binds to an epitope of CD137 that is conserved among CD137 from different species.

The term an anti-CD 137 antigen-binding molecule or an anti-CD 137 antibody "having CD137 binding activity dependent on a small molecule compound" means an antigen-binding molecule or an antibody that shows higher binding activity to CD137 in the presence of the small molecule compound as compared to binding activity to CD137 in the absence of the small molecule compound. In one embodiment, "the presence of a small molecule compound" refers to the condition where the small molecule compound is present at a concentration of 10 micromolar or more, 50 micromolar or more, 100 micromolar or more, 150 micromolar or more, 200 micromolar or more, or 250 micromolar or more. In one embodiment, the extent of binding activity of an anti-CD 137 antigen-binding molecule or antibody to an unrelated, non-CD 137 protein in the presence of a small molecule compound is less than about 10% of the binding of the antigen-binding molecule or antibody to CD137 as measured, e.g., by a radioimmunoassay (RIA) or by surface plasmon resonance (SPR). In certain embodiments, in the presence of a low-molecular weight compound, an anti-CD 137 antigen-binding molecule or antibody has a dissociation constant (KD) of 1 micromolar or less, 100 nM or less, 10 nM or less, 1 nM or less, 0.1 nM or less, 0.01 nM or less, or 0.001 nM or less (e.g., $10^{-6}$ M or less, $10^{-7}$ M or less, $10^{-8}$ M or less, $10^{-9}$ M or less, $10^{-10}$ M or less, e.g., from $10^{-6}$ M to $10^{-10}$ M, from $10^{-7}$ M to $10^{-9}$ M, e.g., from $10^{-7}$ M to $10^{-8}$ M).

Herein, the term "antigen-binding molecule" is used in its broadest sense, and refers to a molecule that specifically binds to an antigenic determinant. In one embodiment, the antigen-binding molecule is an antibody, antibody fragment, or antibody derivative.

An "agonistic antigen-binding molecule" or "agonistic antibody", as used herein, is an antigen-binding molecule or antibody which significantly induces or potentiates a biological activity of the antigen to which it binds (e.g., CD137 and CD3).

Therefore, if the antigen is, for example, CD137, such antigen-binding molecule or antibody having agonistic action is called "CD137 agonistic antigen-binding molecule" or "CD137 agonistic antibody", respectively. In the same manner, if the antigen is, for example, CD3, such antigen-binding molecule or antibody having agonistic action is called "CD3 agonistic antigen-binding molecule" or "CD3 agonistic antibody", respectively.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, $F(ab')_2$; diabodies; linear antibodies; single-chain antibody molecules (e.g. scFv); and multispecific antibodies formed from antibody fragments.

An "antigen-binding molecule that binds to the same epitope" or "antibody that binds to the same epitope" as a reference antigen-binding molecule or reference antibody refers to an antibody or antigen-binding molecule that blocks binding of the reference antibody or reference antigen-binding molecule to its antigen in a competition assay by 50% or more, and conversely, the reference antibody blocks binding of the antibody to its antigen in a competition assay by 50% or more. An exemplary competition assay is provided herein. In one embodiment, in the case that the reference antigen-binding molecule or reference antibody shows antigen binding activity in a manner dependent on a low-molecular weight compound, the competitive assay is carried out in the presence of the low-molecular weight compound.

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., $IgG_1$, $IgG_2$, $IgG_3$, $IgG_4$, $IgA_1$, and $IgA_2$. The heavy chain constant domains that correspond to the different classes of immunoglobulins are called alpha, delta, epsilon, gamma, and mu, respectively.

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B cell receptor); and B cell activation.

"Cytotoxicity" refers to activity that inhibits or prevents cellular function, and/or causes cell death or destruction. Cytotoxicity may be, for example, antibody-dependent cell-mediated cytotoxicity (ADCC) activity, complement-dependent cytotoxicity (CDC) activity, and cytotoxicity by T cells; and may be cytotoxicity caused by cytotoxic agents (for example, radioisotopes and chemotherapeutic agents) such as immunoconjugates.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) or glycine-lysine (residues 446-447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

The term "variant Fc region" herein comprises an amino acid sequence which differs from that of a native sequence Fc region by virtue of at least one amino acid modification, preferably one or more amino acid substitution(s). Preferably, the variant Fc region has at least one amino acid substitution compared to a native sequence Fc region or to the Fc region of a parent polypeptide, e.g. from about one to about ten amino acid substitutions, and preferably from about one to about five amino acid substitutions in a native sequence Fc region or in the Fc region of the parent polypeptide. The variant Fc region herein will preferably possess at least about 80% homology with a native sequence Fc region and/or with an Fc region of a parent polypeptide, and most preferably at least about 90% homology therewith, more preferably at least about 95% homology therewith.

Herein, amino acid alterations or substitutions within an Fc region or a constant region may be represented by the combination of the EU numbering system and amino acids. For example, S424N stands for substitution at position 424 in EU numbering from serine (Ser) to asparagine (Asn). EU424N stands for substitution at position 424 in EU numbering from an amino acid (any type) to asparagine (Asn).

The term "Fc region-comprising antibody" herein refers to an antibody that comprises an Fc region. The C-terminal lysine (residue 447 according to the EU numbering system) or C-terminal glycine-lysine (residues 446-447) of the Fc region may be removed, for example, during purification of the antibody or by recombinant engineering of the nucleic acid encoding the antibody. Accordingly, a composition comprising an antibody having an Fc region according to the present disclosure can comprise an antibody with G446-K447, with G446 and without K447, with all G446-K447 removed, or a mixture of three types of antibodies described above.

The terms "full length antibody," "intact antibody," and "whole antibody" are used herein interchangeably to refer to an antibody having a structure substantially similar to a native antibody structure or having heavy chains that contain an Fc region or a variant Fc region as defined herein.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

An "acceptor human framework" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, Fifth Edition, NIH Publication 91-3242, Bethesda MD (1991), vols. 1-3. In one embodiment, for the VL, the subgroup is subgroup kappa I as in Kabat et al., *supra.* In one embodiment, for the VH, the subgroup is subgroup III as in Kabat et al., *supra.*

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

The term "hypervariable region" or "HVR" as used herein refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Generally, antibodies comprise six HVRs: three in the VH (HI, H2, H3), and three in the VL (L1, L2, L3). Exemplary HVRs herein include:

(a) hypervariable loops occurring at amino acid residues 26-32 (L1), 50-52 (L2), 91-96 (L3), 26-32 (HI), 53-55 (H2), and 96-101 (H3) (Chothia and Lesk, J. Mol. Biol. 196:901-917 (1987));

(b) CDRs occurring at amino acid residues 24-34 (L1), 50-56 (L2), 89-97 (L3), 31-35b (HI), 50-65 (H2), and 95-102 (H3) (Kabat et al., Sequences of Proteins of Immunological Interest, 5th Ed. Public Health Service, National Institutes of Health, Bethesda, MD (1991));

(c) antigen contacts occurring at amino acid residues 27c-36 (L1), 46-55 (L2), 89-96 (L3), 30-35b (HI), 47-58 (H2), and 93-101 (H3) (MacCallum et al. J. Mol. Biol. 262: 732-745 (1996)); and

(d) combinations of (a), (b), and/or (c), including HVR amino acid residues 46-56 (L2), 47-56 (L2), 48-56 (L2), 49-56 (L2), 26-35 (HI), 26-35b (HI), 49-65 (H2), 93-102 (H3), and 94-102 (H3).

Unless otherwise indicated, HVR residues and other residues in the variable domain (e.g., FR residues) are numbered herein according to Kabat et al., *supra.* Herein, HVR residues or other residues within a variable domain (e.g., FR residues) and amino acid alterations or substitutions at such residues may be represented by the combination of the Kabat numbering system and amino acids. For example, N99 stands for asparagine (Asn) at position 99 in Kabat numbering, and N99A stands for substitution at position 99 in Kabat numbering from asparagine (Asn) to alanine (Ala).

An "immunoconjugate" is an antibody conjugated to one or more heterologous molecule(s), including but not limited to a cytotoxic agent.

The term "cytotoxic agent" as used herein refers to a substance that inhibits or prevents a cellular function and/or causes cell death or destruction. Cytotoxic agents include, but are not limited to, radioactive isotopes (e.g., $^{211}$At, $^{131}$I, $^{125}$I, $^{90}$Y, $^{186}$Re, $^{188}$Re, $^{153}$Sm, $^{212}$Bi, $^{32}$P, $^{212}$Pb and radioactive isotopes of Lu); chemotherapeutic agents or drugs (e.g., methotrexate, adriamycin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

An "isolated" antibody is one which has been separated from a component of its natural environment. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman et al., J. Chromatogr. B 848:79-87 (2007).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

"Encoded nucleic acid coding for anti-CD 137 antigen-binding molecule" refers to one or more nucleic acid molecules that code for polypeptide(s) constituting the antigen-binding molecule. "Isolated nucleic acid encoding an anti-CD 137 antibody" refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

The term "monoclonal antibody" as used herein refers to an antibody obtained from a population of substantially homogeneous antibodies, i.e., the individual antibodies composing the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present disclosure may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

A "naked antibody" refers to an antibody that is not conjugated to a heterologous moiety (e.g., a cytotoxic moiety) or radiolabel. The naked antibody may be present in a pharmaceutical formulation.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CHI, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa ($\kappa$) and lambda ($\lambda$), based on the amino acid sequence of its constant domain.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, Megalign (DNASTAR) software, or GENETYX (registered trademark) (Genetyx Co., Ltd.). Those skilled in the art can determine appropriate parameters for aligning sequences, including any algorithms needed to achieve maximal alignment over the full length of the sequences being compared.

The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary. In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

An "effective amount" of an agent, e.g., a pharmaceutical formulation, refers to an amount effective, at dosages and for periods of time necessary, to achieve the desired therapeutic or prophylactic result.

An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is a human.

The term "CD137," as used herein, refers to any native CD137 from any vertebrate source, including mammals such as primates (e.g. humans) and rodents (e.g., mice and rats), unless otherwise indicated. The term encompasses "full-length" unprocessed CD137 as well as any form of CD137 that results from processing in the cell. The term also encompasses naturally occurring variants of CD137, e.g., splice variants or allelic variants.

An amino acid sequence of an exemplary human full-length CD137 is shown in SEQ ID NO: 1 (NCBI Reference Sequence: NP_001552.2) and an amino acid sequence of an exemplary human CD137 extracellular region is shown in SEQ ID NO: 2. An amino acid sequence of an exemplary mouse full-length CD137 is shown in SEQ ID NO: 3 (NCBI Reference Sequence: NP_035742.1) and an amino acid sequence of an exemplary mouse CD137 extracellular region is shown in SEQ ID NO: 4. An amino acid sequence of an exemplary monkey full-length CD137 is shown in SEQ ID NO: 5 (NCBI Reference Sequence: ABY47575.1) and an amino acid sequence of an exemplary monkey CD137 extracellular region is shown in SEQ ID NO: 6.

CD137 is a member of tumor necrosis factor (TNF) receptor family. Its alternative names are tumor necrosis factor receptor superfamily member 9 (TNFRSF9), 4-1BB, and ILA. In addition to its expression on activated CD4$^+$ and CD8$^+$ T cells, CD137 is expressed in B cells, dendritic cells, natural killer (NK) and NK-T cells, macrophages, monocytes, neutrophils, CD4$^+$ CD25$^+$ regulatory T cells, and vascular endothelial cells. Expression in cancer cells is also reported (Labiano, et al. Oncoimmunology, vol. 24: e1062967 (2015)). The natural CD137 ligand, CD137L, is presented by antigen-presenting cells such as B cells, monocytes/macrophages, and dendritic cells (Watts, et al., Annu. Rev. Immunol., vol. 23: p. 23-68 (2005)). Through its interaction with the ligand, CD137 causes increase of TCR-induced T cell proliferation, cytokine production, functional maturation, suppression of apoptosis, and long-term survival of CD8$^+$ T cells (Nam, et al., Curr. Cancer Drug Targets, vol. 5: p. 357-363 (2005); Watts, et al., Annu. Rev. Immunol., vol. 23: p. 23-68 (2005)).

The term "regulatory T cell" or "Treg cell" is a T cell responsible for the suppressive regulation (immune tolerance) of the immune response. In one embodiment, regulatory T cells are CD4$^+$ and/or CD25$^+$ T cells.

The terms "carcinoma", "cancer" and "cancerous" refer to or describe the physiological condition in mammals that is typically characterized by unregulated cell growth/proliferation.

The term "tumor" refers to all neoplastic cell growth and proliferation, whether malignant or benign, and all pre-cancerous and cancerous cells and tissues. The terms "carcinoma", "cancer," "cancerous," "cell proliferative disorder," "proliferative disorder" and "tumor" are not mutually exclusive as referred to herein.

The terms "cell proliferative disorder" and "proliferative disorder" refer to disorders that are associated with some degree of abnormal cell proliferation. In one embodiment, the cell proliferative disorder is cancer.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the present disclosure are used to delay development of a disease or to slow the progression of a disease.

## II. Compositions and methods (anti-CD 137 agonistic antigen-binding molecules)

In one aspect, the present disclosure is based, in part, on anti-CD 137 agonistic antigen-binding molecules and uses thereof. In certain embodiments, antibodies that bind to CD137 are provided. The antibodies in the present disclosure can exhibit activating action on immune cells, cytotoxicity, or anti-tumor activity and, therefore, are useful, for example, in diagnosing or treating cancer.

A. Exemplary anti-CD 137 antigen-binding molecules or antibodies

In one aspect, the present disclosure provides isolated antigen-binding molecules or antibodies that bind to CD137. In certain embodiments, the anti-CD137 antigen-binding molecules or antibodies

- have CD137 binding activity dependent on a small molecule compound;
- bind to the extracellular region of CD137;
- form a ternary complex together with a low-molecular weight compound and CD137;
- bind to human-derived CD137 and monkey-derived CD137;
- are agonistic for CD137 activity;
- show agonistic activity to CD137 in the presence of a low-molecular weight compound;
- have low agonistic activity to CD137 in the absence of the low-molecular weight compound; and/or
- substantially do not show agonistic activity to CD137 in the absence of the low-molecular weight compound.

[Binding activity of antigen-binding molecules or antibodies]

In certain embodiments, the binding activity of the antigen-binding molecules or antibodies provided herein is, in the presence of a low-molecular weight compound, with dissociation constant (KD) of 1 micromolar or less, 100 nM or less, 10 nM or less, 1 nM or less, 0.1 nM or less, 0.01 nM or less, or 0.001 nM or less (for example, $10^{-6}$ M or less, $10^{-7}$ M or less, $10^{-8}$ M or less, $10^{-9}$ M or less, $10^{-10}$ M or less, for example $10^{-6}$ M to $10^{-10}$ M, $10^{-7}$ M to $10^{-9}$ M, for example $10^{-7}$ M to $10^{-8}$ M).

In one embodiment, binding activity of an antigen-binding molecule or antibody is measured by a radiolabeled antigen binding assay (RIA) and represented by KD. In one embodiment, an RIA is performed with the Fab version of an antibody of interest and its antigen. For example, solution binding affinity of Fabs for antigen is measured by equilibrating Fab with a minimal concentration of ($^{125}$I)-labeled antigen in the presence of a titration series of unlabeled antigen, then capturing bound antigen with an anti-Fab antibody-coated plate (see, e.g., Chen et al., J. Mol. Biol. 293:865-881(1999)). To establish conditions for the assay, MICROTITER (registered trademark) multi-well plates (Thermo Scientific) are coated overnight with 5 microgram/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23 degrees C). In a non-adsorbent plate (Nunc #269620), 100 pM or 26 pM [$^{125}$I]-antigen are mixed with serial dilutions of a Fab of interest (e.g., consistent with assessment of the anti-VEGF antibody, Fab-12, in Presta et al., Cancer Res. 57:4593-4599 (1997)). The Fab of interest is then incubated overnight; however, the incubation may continue for a longer period (e.g., about 65 hours) to ensure that equilibrium is reached. Thereafter, the mixtures are transferred to the capture plate for incubation at room temperature (e.g., for one hour). The solution is then removed and the plate washed eight times with 0.1% polysorbate 20 (TWEEN-20 (registered trademark)) in PBS. When the plates have dried, 150 microliter/well of scintillant (MICROSCINT-20™; Packard) is added, and the plates are counted on a TOPCOUNT™ gamma counter (Packard) for ten minutes. Concentrations of each Fab that give less than or equal to 20% of maximal binding are chosen for use in competitive binding assays.

In one embodiment, for measuring binding activity of an antibody, ligand-capturing methods, for example, using BIACORE (registered trademark) T200 or BIACORE (registered trademark) 4000 (GE Healthcare, Uppsala, Sweden), which rely upon surface plasmon resonance analysis methods as the measurement principle, are used. BIACORE (registered trademark) Control Software is used for operation of devices. In one embodiment, amine-coupling kit (GE Healthcare, Uppsala, Sweden) is used according to the manufacturer's instructions to let a molecule for ligand capturing, for example, an anti-tag antibody, an anti-IgG antibody, protein A, etc. fixed onto a sensor chip (GE Healthcare, Uppsala, Sweden) coated with carboxymethyldextran. The ligand-capturing molecule is diluted with a 10 mM sodium acetate solution at an appropriate pH and is injected at an appropriate flow rate and for an appropriate injection time. Binding activity measurements are measured using a 0.05% polysorbate 20 (in other name Tween (registered trademark)-20)-containing buffer as a measurement buffer, at a flow rate of 10-30 microliter/minute, and at a measurement temperature of preferably at 25 degrees C or 37 degrees C. For the measurement carried out with an antibody captured by the ligand-capturing molecule as a ligand, an antibody is injected to let a target amount of the antibody captured, and then a serial dilution of an antigen and/or an Fc receptor (analyte) prepared using the measurement buffer is injected. For the measurement carried out with an antigen and/or an Fc receptor captured by the ligand-capturing molecule as a ligand, an antigen and/or an Fc receptor is injected to let a target amount thereof captured, and then a serial dilution of an antibody (analyte) prepared using the measurement buffer is injected.

In one embodiment, the measurement results are analyzed using BIACORE (registered trademark) Evaluation Software. Kinetics parameter calculation is carried out by fitting sensorgrams of association and dissociation at the same time using a 1:1 binding model, and an association rate (kon or ka), a dissociation rate (koff or kd), and an equilibrium dissociation constant (KD) may be calculated. For the case of weak binding activity, in particular, for the cases where dissociation is fast and kinetics parameters are difficult to calculate, the Steady state model may be used to calculate the equilibrium dissociation constant (KD). As additional parameters concerning binding activity, "binding amount of analyte per unit ligand amount" may be calculated by dividing a binding amount of analyte (resonance unit: RU) at a specific concentration by an amount of captured ligand.

[Small molecule compound-dependent binding activity]

In one aspect, the anti-CD137 antigen-binding molecule or antibody has small molecule compound-dependent CD137-binding activity. In one non-limiting embodiment, the anti-CD 137 antigen-binding molecule or antibody has a higher binding activity towards CD137 in the presence of a small molecule compound as compared to the binding activity towards CD137 in the absence of the small molecule compound. In a different embodiment, the anti-CD 137 antigen-binding molecule or antibody has a higher binding activity towards CD137 in the presence of a high concentration of a small molecule compound compared to the CD137-binding activity in the presence of a low concentration of the small molecule compound. In one preferred embodiment, the binding activity of the anti-CD 137 antigen-binding molecule or antibody for CD137 in the presence of a small molecule compound is 2-fold or more, 3-fold or more, 5-fold or more, 10-fold or more, 15-fold or more, 20-fold or more, 25-fold or more, 30-fold or more, 50-fold or more, 100-fold or more, 200-fold or more, 300-fold or more, 500-fold or more $1 \times 10^3$-fold or more, $2 \times 10^3$-fold or more, $3 \times 10^3$-fold or more, $5 \times 10^3$-fold or more, $1 \times \times 10^4$-fold or more, $2 \times 10^4$-fold or more, $3 \times 10^4$-fold or more, $5 \times 10^4$-fold or more, or $1 \times 10^5$-fold or more, compared to the binding activity in the absence of the small molecule compound. In a different preferred embodiment, the binding activity of the anti-CD137 antigen-binding molecule or antibody for CD137 in the presence of a small molecule compound is higher than 2-fold, higher than 3-fold, higher than 5-fold, higher than 10-fold, higher than 15-fold, higher than 20-fold, higher than 25-fold, higher than 30-fold, higher than 50-fold, higher than 100-fold, higher than 200-folds, higher than 300-fold, higher than 500-fold, higher than $1 \times 10^3$-folds, higher than $2 \times 10^3$-folds, higher than $3 \times 10^3$-fold, higher than $5 \times 10^3$-fold, higher than $1 \times 10^4$-fold, higher than $2 \times 10^4$-fold, higher than $3 \times 10^4$-fold, higher than $5 \times 10^4$-fold, or higher than $1 \times 10^5$-fold, compared to the binding activity in the absence of the small molecule compound.

The concentration of the small molecule compound can be any arbitrary concentration as long as the difference in the binding activity of anti-CD 137 antigen-binding molecule or antibody is detected. In one embodiment, the concentration of the small molecule compound "in the presence of a small molecule compound" and/or "in the presence of a high concentration of a small molecule compound" is, for example, 100 nM or more, 500 nM or more, 1 μM or more, 3 μM or more, 5 μM or more, 10 μM or more, 50 μM or more, 100 μM or more, 150 μM or more, 200 μM or more, 250 μM or more, 300 μM or more, 400 μM or more, 500 μM or more, or 1 mM or more. Alternatively, the concentration can be defined to be an amount that is sufficient for the anti-CD137 antigen-binding molecule or antibody to show maximum binding activity. Further, in one embodiment, the concentration of the small molecule compound "in the presence of a low concentration of a small molecule compound" may be, for example, 500 μM or less, 250 μM or less, 200 μM or less, 150 μM or less, 100 μM or less, 50 μM or less, 10 μM or less, 1 μM or less, 500 nM or less, 100 nM or less, 50 nM or less, or 10nM or 1nM or less. The case where the concentration of the small molecule compound is zero, or the substantial concentration is zero, can also be selected as an embodiment of the low concentration.

Here, the term "substantial concentration is zero" means, for example, a concentration that is so minute that it cannot be detected by current technology although the small molecule compound is present.

In one embodiment, the binding activity towards CD137 in the presence of a small molecule compound at a concentration of 10 μM, 50 μM, 100 μM, 150 μM, 200 μM or 250 μM is, 2-fold or more, 5-fold or more, 10-fold or more, 15-fold or more, 16-fold or more, 17-fold or more, 18-fold or more, 19-fold or more, or 20-fold or more, compared to the binding activity towards CD137 in the absence of the small molecule compound. In one embodiment, the binding activity of the anti-CD 137 antigen-binding molecule or an antibody against CD137 in the presence of a small molecule compound at 10 μM or more is, 2-fold or more, 5-fold or more, 10-fold or more, 15-fold or more, 16-fold or more, 17-fold or more, 18-fold or more, 19-fold or more, or 20-fold or more, compared to the binding activity towards CD137 in the absence of the small molecule compound. In one embodiment, the binding activity of the anti-CD137 antigen-binding molecule or an antibody against CD137 in the presence of a small molecule compound at 100 μM or more is, 2-fold or more, 5-fold or more, 10-fold or more, 15-fold or more, 16-fold or more, 17-fold or more, 18-fold or more, 19-fold or more, or 20-fold or more, compared to the binding activity towards CD137 in the absence of the small molecule compound.

In one embodiment, the binding activity (KD) of the anti-CD137 antigen-binding molecule or antibody towards CD137 in the presence of a small molecule compound at 10 μM or more is, a dissociation constant (KD) of $9 \times 10^{-7}$ M or less, $8 \times 10^{-7}$ M or less, $7 \times 10^{-7}$ M or less, $6 \times 10^{-7}$ M or less, $5 \times 10^{-7}$ M or less, or $4 \times 10^{-7}$ M or less, or preferably, a dissociation constant (KD) of $5 \times 10^{-7}$ M or less. In a further embodiment, the binding activity (KD) of the anti-CD 137 antigen-binding molecule or antibody towards CD137 in the absence of a small molecule compound is too large to be calculated by Biacore (weak binding activity), or it is a disassociation constant (KD) of $1 \times 10^{-7}$ M or more, $5 \times 10^{-7}$ M or more, $7 \times 10^{-7}$ M or more, $8 \times 10^{-7}$ M or more, $9 \times 10^{-7}$ M or more, $1 \times 10^{-6}$ M or more, $2 \times 10^{-6}$ M or more, $3 \times 10^{-6}$ M or more, or $4 \times 10^{-6}$ M or more, or preferably, is a dissociation constant (KD) of $1 \times 10^{-6}$ M or more. In another embodiment, the binding activity (KD) of the anti-CD 137 antigen-binding molecule or an antibody towards CD137 in the presence of a small molecule compound at 100 $\mu$M or more is, $9 \times 10^{-7}$ M or less, $8 \times 10^{-7}$ M or less, $7 \times 10^{-7}$ M or less, $6 \times 10^{-7}$ M or less, $5 \times 10^{-7}$ M or less, $4 \times 10^{-7}$ M or less, $3 \times 10^{-7}$ M or less, $2 \times 10^{-7}$ M or less, or $1 \times 10^{-7}$ M or less, or preferably, the dissociation constant (KD) is $2 \times 10^{-7}$ M or less. In a further embodiment, the binding activity (KD) of the anti-CD137 antigen-binding molecule or an antibody towards CD137 in the absence of the small molecule compound is too large to be calculated by Biacore (weak binding activity), or it is a disassociation constant (KD) of $1 \times 10^{-7}$ M or more, $5 \times 10^{-7}$ M or more, $7 \times 10^{-7}$ M or more, $8 \times 10^{-7}$ M or more, $9 \times 10^{-7}$ M or more, $1 \times 10^{-6}$ M or more, $2 \times 10^{-6}$ M or more, $3 \times 10^{-6}$ M or more, or $4 \times 10^{-6}$ M or more, or preferably, a dissociation constant (KD) of $1 \times 10^{-6}$ M or more.

In one embodiment, the binding activity (KD) of the anti-CD137 antigen-binding molecule or antibody towards CD137 in the presence of a small molecule compound at 10 $\mu$M or more is a dissociation constant (KD) of $8 \times 10^{-8}$ M or less, and the binding activity (KD) towards CD137 in the absence of compound is too large to be calculated by Biacore (weak binding activity). In another embodiment, the binding activity (KD) of the anti-CD137 antigen binding molecule or antibody towards CD137 in the presence of a small molecule compound at 100 $\mu$M is a dissociation constant (KD) of $2 \times 10^{-8}$ M or less, and the binding activity towards CD137 in the absence of the small molecule compound is too large to be calculated by Biacore (weak binding activity).

In one aspect, the present disclosure provides an anti-CD 137 antigen-binding molecule or antibody in which the value of, [binding activity (binding amount) towards CD137 in the presence of a low-molecular compound at 10 $\mu$M or more]/[binding activity (binding amount) towards CD137 in the absence of the small molecule compound)] is the same as or greater than the value of a reference anti-CD137 antigen-binding molecule. In a different aspect, the present disclosure provides an anti-CD137 antigen-binding molecule or antibody in which the value of, [binding activity (binding amount) towards CD137 in the presence of a low-molecular compound at 100 $\mu$M or more]/[binding activity (binding amount) towards CD137 in the absence of the small molecule compound)] is the same as or greater than the value of a reference anti-CD137 antigen-binding molecule. In any of the above aspects, the reference anti-CD137 antigen binding molecule can be selected from anti-CD137 antibodies containing HVR-H1, HVR-H2, HVR-H3 , HVR-L1, HVR-L2 and HVR-L3 having the same amino acid sequences as HVR-H1, HVR-H2, HVR-H3 , HVR-L1, HVR-L2 and HVR-L3 contained in A375/B167, A372/B040, A356/B040, A486/B167, A487/B167, A488/B226, A489/B223, A548/B376, A551/B256, A551/B379, A555/B379, A548/B256 or A549/B167 described in Table 37.

In one embodiment, the reference anti-CD137 antigen-binding molecule is an antibody comprising the amino acid sequence of A375/B167, A372/B040, A356/B040, A486/B167, A487/B167, A488/B226, A489/B223, A548/B376, A551/B256, A551/B379, A555/B379, A548/B256, or A549/B167 described in Table 37 as a heavy chain variable region/light chain variable region combination. In a different preferred embodiment, the reference antigen-binding molecule is an anti-CD 137 antibody comprising HVR-H1, HVR-H2, HVR-H3, HVR-L1, HVR-L2 and HVR-L3 having the same amino acid sequences as HVR-H1, HVR-H2, HVR-H3, HVR-L1, HVR-L2 and HVR-L3 comprised in A375/B167. In a further embodiment, the reference anti-CD137 antigen-binding molecule is an anti-CD137 antibody comprising A375/B167 as a heavy chain variable region/light chain variable region combination. In a different preferable embodiment, the reference antigen-binding molecule is an anti-CD137 antibody comprising HVR-H1, HVR-H2, HVR-H3, HVR-L1, HVR-L2 and HVR-L3 having the same amino acid sequences as HVR-H1, HVR-H2, HVR-H3, HVR-L1, HVR-L2 and HVR-L3 comprised in A551/B379. In a further embodiment, the reference anti-CD137 antigen-binding molecule is an anti-CD137 antibody comprising A551/B379 as a heavy chain variable region/light chain variable region combination. In a preferable embodiment, the reference antigen-binding molecule comprises heavy and light chain constant regions of human origin (for example, G1T3 (SEQ ID NO: 138) as the heavy chain constant region, human $\lambda$ chain Lamlib (SEQ ID NO: 63) as the light chain constant region).

In one aspect, the present disclosure provides an anti-CD 137 antigen-binding molecule or antibody in which the binding activity (binding amount) towards CD137 in the absence of a small molecule compound is the same as or lower than that of a reference anti-CD137 antigen-binding molecule, and also, the binding activity (binding amount) towards CD137 in the presence of the small molecule compound at 10 $\mu$M or more is equal to or higher than the binding activity of the reference anti-CD 137 antigen-binding molecule towards CD137 under the same conditions. In a different aspect, the present disclosure provides an anti-CD 137 antigen binding molecule or antibody in which the binding activity towards CD137 in the absence of a small molecule compound is the same as or lower than that of a reference anti-CD137 antigen-binding molecule, and also, the binding activity (binding amount) towards CD137 in the presence of the small molecule compound at 10 $\mu$M or more is equal to or higher than the binding activity (binding amount) of the reference anti-CD 137 antigen-binding molecule towards CD137 under the same conditions. In any of the above aspects, the reference anti-CD137 antigen-binding molecule can be selected from anti-CD137 antibodies containing HVR-H1, HVR-H2, HVR-H3, HVR-L1, HVR-L2 and HVR-L3 having the same amino acid sequences as HVR-H1, HVR-H2, HVR-H3, HVR-L1, HVR-L2 and HVR-L3 contained in A375/B167, A372/B040, A356/B040, A486/B167, A487/B167, A488/B226, A489/B223, A548/B376, A551/B256, A551/B379, A555/B379, A548/B256 or A549/B167 described in Table 37.

In one embodiment, the reference anti-CD137 antigen-binding molecule is an anti-CD 137 antibody comprising the amino acid sequence of A375/B167, A372/B040, A356/B040, A486/B167, A487/B167, A488/B226, A489/B223, A548/B376, A551/B256, A551/B379, A555/B379, A548/B256, or A549/B167 described in Table 37 as a heavy chain variable region/light chain variable region combination. In a preferred embodiment, the reference anti-CD137 antigen-binding molecule is an anti-CD137 antibody comprising HVR-H1, HVR-H2, HVR-H3, HVR-L1, HVR-L2 and HVR-L3 having the same amino acid sequences as HVR-H1, HVR-H2, HVR-H3, HVR-L1, HVR-L2 and HVR-L3 comprised in A375/B167. In a further embodiment, the reference anti-CD137 antigen-binding molecule is an anti-CD137 antibody comprising A375/B167 as a heavy chain variable region/light chain variable region combination. In a different preferable embodiment, the reference anti-CD 137 antigen-binding molecule is an anti-CD137 antibody comprising HVR-H1, HVR-H2, HVR-H3, HVR-L1, HVR-L2 and HVR-L3 having the same amino acid sequences as HVR-H1, HVR-H2, HVR-H3, HVR-L1, HVR-L2 and HVR-L3 comprised in A551/B379. In a further embodiment, the reference anti-CD 137 antigen-binding molecule is an anti-CD 137 antibody comprising A551/B379 as a heavy chain variable region/light chain variable region combination. In a preferable embodiment, the reference antigen-binding molecule comprises heavy and light chain constant regions of human origin (for example, G1T3 (SEQ ID NO: 138) as the heavy chain constant region, human λ chain Lamlib (SEQ ID NO: 63) as the light chain constant region).

In one aspect, the present disclosure provides an anti-CD 137 antigen-binding molecule or antibody in which the value of, [binding activity (KD) towards CD137 in the presence of a low-molecular compound at 1 μM]/[binding activity (KD) towards CD137 in the presence of the low-molecular compound at 10μM or more] is the same as or greater than the value of a reference antigen-binding molecule. In a different aspect, the present disclosure provides an anti-CD 137 antigen-binding molecule or antibody in which the value of, [binding activity (KD) towards CD137 in the presence of a low-molecular compound at 1 μM]/[binding activity (KD) towards CD137 in the presence of the low-molecular compound at 100 μM or more] is the same as or greater than the value of a reference antigen-binding molecule. In any of the above aspects, the reference antigen-binding molecule can be selected from antibodies containing HVR-H1, HVR-H2, HVR-H3 , HVR-L1, HVR-L2 and HVR-L3 having the same amino acid sequences as HVR-H1, HVR-H2, HVR-H3, HVR-L1, HVR-L2 and HVR-L3 contained in A375/B167, A372/B040, A356/B040, A486/B167, A487/B167, A488/B226, A489/B223, A548/B376, A551/B256, A551/B379, A555/B379, A548/B256 or A549/B167 described in Table 37.

In one embodiment, the reference antigen-binding molecule is an antibody comprising the amino acid sequence of A375/B167, A372/B040, A356/B040, A486/B167, A487/B167, A488/B226, A489/B223, A548/B376, A551/B256, A551/B379, A555/B379, A548/B256, or A549/B167 described in Table 37 as a heavy chain variable region/light chain variable region combination. In a preferred embodiment, the reference antigen-binding molecule is an antibody comprising HVR-H1, HVR- H2, HVR-H3, HVR-L1, HVR-L2 and HVR-L3 having the same amino acid sequences as HVR-H1, HVR-H2, HVR-H3, HVR-L1, HVR-L2 and HVR-L3 comprised in A375/B167. In a further embodiment, the reference antigen-binding molecule is an antibody comprising A375/B167 as a heavy chain variable region/light chain variable region combination. In a different embodiment, the reference antigen-binding molecule is an antibody comprising HVR-H1, HVR-H2, HVR-H3, HVR-L1, HVR-L2 and HVR-L3 having the same amino acid sequences as HVR-H1, HVR-H2, HVR-H3, HVR-L1, HVR-L2 and HVR-L3 comprised in A551/B379. In a further embodiment, the reference antigen-binding molecule is an antibody comprising A551/B379 as a heavy chain variable region/light chain variable region combination. In a preferable embodiment, the reference antigen-binding molecule comprises heavy and light chain constant regions of human origin (for example, G1T3 (SEQ ID NO: 138) as the heavy chain constant region, human λ chain Lamlib (SEQ ID NO: 63) as the light chain constant region).

In one embodiment, the binding activity of the anti-CD 137 antibody towards CD137, with the presence, absence, high concentration and/or low concentration of a small molecule compound, is measured, for example, by a ligand capture method using BIACORE (registered trademark) T200 with surface plasmon resonance spectroscopy as principle of measurement.

Details of an exemplary method for measuring the binding activity of anti-CD 137 antibody towards CD137 are described below. In one embodiment, the binding activity of anti-CD 137 antibody towards CD137 is evaluated by BIACORE (registered trademark) T200. In a preferred embodiment, this assay uses 20 mM ACES (pH 7.4), 150 mM NaCl, 2 mM MgCl$_2$, and 0.05% Tween 20 as a running buffer, and is carried out at 37°C. In one embodiment, this measurement is carried out after capturing an antibody as a ligand on the ligand capture molecule. Specifically, a suitable amount (e.g., about 100 RU, 200 RU, 300 RU, 400 RU, or 500 RU) of the antibody is captured by, interacting an antibody solution prepared using the running buffer with a chip prepared firstly by immobilizing Sure Protein A (GE Healthcare) on a Series S Sensor Chip CM3 (GE Healthcare).

In a preferred embodiment, about 100 to 500 RU, preferably about 250 to 400 RU of antibody is captured. Next, the binding activity towards CD137 in the presence and absence of a small molecule compound is evaluated by interacting a CD137 solution prepared using a running buffer added with a small molecule compound to a target concentration (for

example, 1 μM, 10 μM, 50 μM or 100 μM), or a CD137 solution prepared using a running buffer that does not contain the small molecule compound. Although the concentration of CD137 in the CD137 solution can be determined appropriately, for example, when hCD137-HisBAP (see Reference Example 1-1) is used as antigen, the measurement is carried out using an antigen concentration of 0 nM, 15.625 nM, 62.5 nM, 250 nM, and 1000 nM, respectively. In one embodiment, the dissociation constant (KD) of anti-CD 137 antibody to human CD137 is calculated using Biacore T200 Evaluation Software 2.0. Specifically, the binding rate constant ka (L/mol/s) and the dissociation rate constant kd (1/s) are calculated by global fitting a sensorgram obtained by measurement using the 1:1 Langmuir binding model. The dissociation constant KD (mol/L) is calculated from the values.

Further exemplary assay methods for measuring the binding activity of anti-CD 137 antibody to CD137 will be described in detail below. Binding of anti-CD 137 antibodies to human CD137 is assessed with Biacore T200. The binding towards human CD137 was measured using 20 mM ACES (pH 7.4), 150 mM NaCl, 2 mM $MgCl_2$, and 0.05% Tween 20 as a running buffer, and this was carried out at 37°C. First, an antibody of about 250 to 400 RU is captured by interacting an antibody solution prepared using the running buffer with a chip where Sure Protein A (GE Healthcare) is immobilized on a Series S Sensor Chip CM3 (GE Healthcare). Next, a human CD137 solution prepared using a running buffer added with ATP in a target concentration (for example, 1 μM, 10 μM, 50 μM or 100 μM), or a human CD137 solution prepared using a running buffer that does not contain ATP, is interacted to evaluate the binding activity towards CD137 in the presence and absence of ATP. hCD137-HisBAP prepared by the method of Reference Example (1-1) is used as human CD137 which is the antigen, and the measurement is carried out at antigen concentrations of 0 nM, 15.625 nM, 62.5 nM, 250 nM, and 1000 nM, respectively. The chip is regenerated using 25 mM NaOH and 10 mM Glycine-HCl (pH 1.5), and measurement is conducted by repeatedly capturing the antibodies. The dissociation constant of each antibody for human CD137 is calculated using Biacore T200 Evaluation Software 2.0. Specifically, the binding rate constant ka (L/mol/s) and the dissociation rate constant kd (1/s) are calculated by global fitting the sensorgram obtained by the measurement using the 1:1 Langmuir binding model. The dissociation constant KD (mol/L) is calculated from the values.

In one embodiment, the binding activity of the anti-CD 137 antibody towards CD137 (preferably human CD137) can also be rephrased as "the amount of CD137 binding per unit amount of antibody". Specifically, by using the sensorgrams obtained by the above assay method using BIACORE (registered trademark) T200, the binding amount of CD137 to antibody (RU) is divided by the amount of antibody captured to calculate "the amount of CD137 binding per unit amount of antibody". In one embodiment, the binding activity of the anti-CD 137 antibody towards CD137 (preferably human CD137) can also be measured by the method described in Reference Example 5-3 or 6-2.

The terms "small molecule" and "small molecule compound" refer to a naturally-occurring chemical substance other than "biopolymers" present in the living body, or a non-naturally-occurring chemical substance. Preferably, it is a target tissue-specific compound or a non-naturally-occurring compound, but is not limited thereto. In one embodiment, the "small molecule compound" in the present disclosure is a "cancer tissue-specific compound" or a "cancer tissue-specific metabolite". The term in the present disclosure "a compound specific to cancer tissue (cancer tissue-specific compound)" refers to a compound which exists differentially in tumor tissue, compared to non-tumor tissue. As used herein, the term "cancer" is generally used to describe a malignant neoplasm, and may be metastatic or non-metastatic. The term "metabolism" refers to chemical changes that occur within the tissue of an organism, including "assimilation" and "catabolism". Assimilation refers to the biosynthesis or accumulation of molecules, and catabolism refers to the degradation of molecules. A "metabolite" is an intermediate or product resulting from substance metabolism.

The term "target tissue" means any tissue in the living body to which the antigen-binding molecule of the present disclosure is intended to be delivered. The target tissue may be a histologically distinguishable tissue such as various organs or a pathologically distinguishable tissue such as normal tissues and diseased tissues. In certain embodiments, the target tissue is a tumor tissue. In contrast, "non-target tissue(s)" means tissues in the living body other than the target tissue.

The term "tumor tissue" means a tissue that comprises at least one tumor cell. Generally, a tumor tissue is made of a population of tumor cells constituting the tumor main body (parenchyma) and connective tissues and blood vessels existing in between tumor cells and supporting the tumor (stroma). In some cases, these are clearly distinguishable, but there are cases where these are mixed up. In some cases, there are cells such as immune cells that have infiltrated into the tumor tissue. In contrast, "non-tumor tissue" means a tissue in the living body other than tumor tissue(s). Non-diseased healthy tissues/normal tissues are representatives of such non-tumor tissues.

As a non-limiting embodiment of a cancer tissue-specific compound, or a cancer tissue-specific metabolite as used in the present disclosure, at least one compound selected from the compounds detailed below can be suitably exemplified. The meaning of "at least one compound" includes, in addition to the case where the binding activity against the antigen by the same antigen-binding domains described later depends on one type of cancer tissue-specific compound or cancer tissue-specific metabolite, the case where the binding activity depends on several types of cancer tissue-specific compounds or cancer tissue-specific metabolites.

As used herein, the term "target tissue-specific compound" refers to a compound that is differentially present in target tissue as compared to non-target tissue. In several embodiments, the target tissue-specific compound can be a compound defined by a qualitative target tissue specificity such as being present in target tissue but not in non-target tissues, or being present in non-target tissue but not in target tissue. In a different embodiment, the target tissue-specific compound may be a compound defined by a quantitative target tissue specificity such as being present in target tissue at a concentration which is different (for example, a higher concentration or lower concentration) compared to non-target tissue. In a specific embodiment, the target tissue-specific compound is present in target tissue at a concentration which is, for example, 1.05-fold or more, 1.1-fold or more, 1.15-fold or more, 1.2-fold or more, 1.25-fold or more, 1.3-fold or more, 1.35-fold or more, 1.4-fold or more, 1.45-fold or more , 1.5-fold or more, 1.55-fold or more, 1.6-fold or more, 1.65-fold or more, 1.7-fold or more, 1.75-fold or more, 1.8-fold or more, 1.85-fold or more, 1.9-fold or more, 1.95-fold or more, 2-fold or more, 2.1-fold or more, 2.2-fold or more, 2.3-fold or more, 2.4-fold or more, 2.5-fold or more, 3-fold or more, 5-fold or more, 10-fold or more, 50-fold or more, 100-fold or more, $10^3$-fold or more , $10^4$-fold or more, $10^5$-fold or more, $10^6$-folds or more, or higher compared to non-target tissue. In another embodiment, the target tissue specific compound is present in target tissue at a concentration which is, for example, 1.05-fold or more, 1.1-fold or more, 1.15-fold or more, 1.2-fold or more, 1.25-fold or more, 1.3-fold or more, 1.35-fold or more, 1.4-fold or more, 1.45-fold or more, 1.5-fold or more, 1.55-fold or more, 1.6-fold or more , 1.65-fold or more, 1.7-fold or more, 1.75-fold or more, 1.8-fold or more, 1.85-fold or more, 1.9-fold or more, 1.95-fold or more, 2-fold or more, 2.1-fold or more, 2.2-fold or more, 2.3-fold or more, 2.4-fold or more, 2.5-fold or more, 3-fold or more, 5-fold or more, 10-fold or more, 50-fold or more, 100-fold or more, $10^3$-folds or more, $10^4$-folds or more, $10^5$-folds or more, $10^6$-folds or more, or higher compared to non-target tissue. In a specific embodiment, a target tissue-specific compound, as compared to non-target tissue, is present in a target tissue at a concentration that is statistically significantly higher or lower (i.e., as determined using either one of Welch's t-test or rank sum test of Wilcoxon, the p value is less than 0.05 and/or the q value is less than 0.10). In a specific embodiment, the target tissue-specific compound is a tumor tissue-specific compound.

In a specific embodiment, a tumor tissue-specific compound is a metabolite produced by a metabolism specific to a tumor cell. The metabolite may be a product that is generated by metabolism essential for life activities (primary metabolite), or a product generated by a metabolism not necessarily required for life activities (secondary metabolite). Examples of primary metabolites may include sugars, proteins, lipids, nucleic acids, and the like. Examples of secondary metabolites include antibiotics and dyes. The metabolite may be a biopolymer or a small molecule. In a specific embodiment, the biopolymer is a molecule having a molecular weight of about 5000 or more which consists of one or more types of repeating units, including, for example, polysaccharides, polypeptides, and polynucleotides. In a specific embodiment, small molecules are molecules having a molecular weight of about 500 or less and are chemical substances present within the living body. In a further embodiment, the tumor tissue-specific compound is a small molecule metabolite specifically produced in tumor cells (Eva Gottfried, Katrin Peter and Marina P. Kreutz, From Molecular and Modular Tumor Therapy (2010) 3 (2), 111-132). In a further embodiment, the tumor tissue-specific compound is a metabolite that is produced specifically by a cell infiltrating into tumor tissue (e.g., an immune cell) or a stromal cell (e.g., a cancer associated fibroblast (CAF)) present in a tumor tissue. Examples of immune cells infiltrating into the tumor tissue are dendritic cells, suppressive dendritic cells, regulatory T cells, exhausted T cells, myeloma-derived suppressor cells (MDSCs), and the like. In a further embodiment, a metabolite which is produced by cells present in tumor tissue (e.g., tumor cells, immune cells, stromal cells, etc.), which is released to the outside of the cell when the cells die by apoptosis or necrosis, or the like, can also be included in the tumor tissue-specific compounds of the present disclosure.

To identify a tumor tissue-specific compound, analysis at the transcriptome level (e.g., Dhanasekaran et al. (Nature (2001) 41 2, 822-826), Lapointe et al. (Proc. Natl. Acad. Sci. USA (2004) 101, 811-816), or Perou et al. (Nature (2000) 406, 747-752)), analysis at the proteome level (e.g., Ahram et al. (Mol. Carcinog. (2002) 33, 9-15), and Hood et al. (Mol. Cell. Proteomics (2005) 4, 1741-1753)), and analysis of metabology centering on metabolomic profiling (metabolomics) can be used appropriately. That is, in order to identify a metabolite in a test sample, high performance liquid chromatography (HPLC), nuclear magnetic resonance (NMR) (Brindle et al. (J. Mol. Recognit. (1997) 10, 182-187)), mass spectrometry (GC/MS and LC/MS) (Gates and Sweeley (Clin. Chem. (1978) 24, 1663-1673)), and metabolic profiling that uses ELISA, and such, can be suitably used alone and/or in combination.

In a specific embodiment, the tumor tissue-specific compound is at least one compound selected from the group consisting of: nucleosides having a purine ring structure, amino acids and metabolites thereof, lipids and metabolites thereof, primary metabolites of the carbohydrate metabolism, as well as nicotinamide and its metabolites. In a further embodiment, the tumor tissue-specific compound is at least one compound selected from (1) to (6) below:

(1) nucleosides having a purine structure such as adenosine (ADO), adenosine triphosphate (ATP), adenosine diphosphate (ADP), adenosine monophosphate (AMP), and inosine;
(2) amino acids such as alanine, glutamic acid and aspartic acid;
(3) metabolites of amino acids such as kynurenine, anthranilic acid, 3-hydroxykynurenine, and kynurenic acid;
(4) metabolites of arachidonic acid such as prostaglandin E2;
(5) primary metabolites of the glycolytic pathway or Krebs cycle such as lactic acid, succinic acid, and citric acid; and,

(6) metabolites of nicotinamide such as 1-methyl nicotinamide.

(1) Nucleosides having a purine structure such as adenosine (ADO), adenosine triphosphate (ATP), adenosine diphosphate (ADP), adenosine monophosphate (AMP), and inosine

It is known that, when tumor cells undergo cell death, a large amount of intracellular ATP leaks out. Therefore, the ATP concentration in tumor tissue is significantly higher than that in normal tissue (PLoS One. (2008) 3, e2599). AMP is metabolized by enzymes on the cell surface such as extracellular-5'-nucleotidase (eco-5'-nucleotidase) (CD73) (Resta and Thompson (Immunol. Rev. (1998) 161, 95- 109) and Sadej et al. (Melanoma Res. (2006) 16, 21 3-222)). Adenosine is a purine nucleoside that is constitutively present in the extracellular environment at low concentrations, but a marked increase in the extracellular adenosine concentration has been reported in hypoxic tissues found in solid tumors (Blay and Hoskin (Cancer Res. (1997) 57, 260 2-2605)). CD73 is expressed on the surface of tumors and immune cells (Kobie et al. (J. Immunol. (2006) 17 7, 6780-6786)) and elevated activity has been found in breast cancer (Canbolat et al. (Breast Cancer Res. Treat. (1996) 37, 189-193)), stomach cancer (Durak et al. (Cancer Lett. (1994) 84, 199-202)), pancreatic cancer (Flocke and Mannherz (Biochim. Biophys. Acta (1991) 1076, 273-281)), and glioblastoma (Bardot et al. (Br. J. Cancer (1994) 70, 212-218)). It has been proposed that accumulation of adenosine in tumor tissue is a result of increase in dephosphorylation of AMP by cytoplasmic 5'-nucleotidase (Headrick and Willis (Biochem. J. (1989) 261, 541-550)). Furthermore, regulatory T cells infiltrating into tumor tissue also express ATPase and produce adenosine (Proc. Natl. Acad. Sci. USA (2006) 103 (35), 13132-13137; Curr. Med. Chem. (2011) 18: 5217-5223). The produced adenosine is thought to keep tumor tissues in an immunosuppressive environment via adenosine receptors such as the A2A receptor (Curr. Med. Chem. (2011) 18, 5217-5223). Thus, ATP, ADP, AMP, adenosine, and such, that are considered to be accumulated at a high concentration in tumor tissue by metabolism of purine nucleotides are examples of the tumor tissue-specific compounds used in the present disclosure. Additionally, as adenosine undergoes degradation into inosine by adenosine deaminase, inosine is accumulated at a high concentration.

In a specific embodiment, nucleosides having a purine ring structure include adenosine-containing compounds. In specific embodiments, adenosine-containing compounds include, for example, adenosine (ADO), adenosine triphosphate (ATP), adenosine diphosphate (ADP), adenosine monophosphate (AMP), cyclic adenosine monophosphate (cAMP), deoxyadenosine (dADO), deoxyadenosine triphosphate (dATP), deoxyadenosine diphosphate (dADP), deoxyadenosine monophosphate (dAMP), adenosine [y-thio]triphosphate (ATPyS), and such. In another embodiment, nucleosides having a purine ring structure include inosine which is a metabolite of adenosine.

Furthermore, in a specific embodiment, nucleosides having a purine ring structure include commercially available nucleosides having a purine ring structure such as ADPbetaS (Sigma Inc.) and such.

(2) Amino acids such as alanine, glutamic acid and aspartic acid

The rate of uptake of glutamine, which acts as a nitrogen carrier in the living body, is increased in tumor cells, and such glutamine incorporation and the resulting conversion to glutamic acid and lactic acid (glutamine degradation (glutaminolysis)) are thought to be features of tumor cells (Mazurek and Eigenbrodt (Anticancer Res. (2003) 23, 1149-1154, and Mazurek et al. (J. cell. Physiol. (1999) 181, 136-146). Glutamine levels in plasma are decreased in cancer patients, while glutamic acid concentration is increased (Droge et al. (Immunobiology (1987) 174, 473-479)), and studies of the metabolism of $^{13}$C-labelled glucose in lung cancer tissue showed a correlation among the concentrations of $^{13}$C-labeled succinic acid, $^{13}$C-labeled alanine, $^{13}$C-labeled glutamic acid, and $^{13}$C-labeled citrate. For these reasons, alanine, glutamic acid, aspartic acid and such, which are thought to be accumulated in high concentrations in tumor tissue due to, e.g., glutamine degradation, are examples of tumor tissue-specific compounds used in this disclosure.

(3) Metabolites of amino acids such as kynurenine, anthranilic acid, 3-hydroxykynurenine, and kynurenic acid

Indoleamine 2,3-dioxygenase (IDO) is a tryptophan-metabolizing enzyme highly expressed in many cancers such as melanoma, colon cancer, kidney cancer and such (Uyttenhove et al. (Nat. Med. (2003) 9, 1269-1274)). IDO catalyzes the conversion of tryptophan to kynurenine. In gliomas which do not express IDO, kynurenine is produced from tryptophan by tryptophan 2,3-dioxygenase (TDO) of the liver (Opitz et al. (Nature (2011) 478 (7368), 197-203)). IDO is also expressed on dendritic cells infiltrating into tumor tissue, and dendritic cells also produce kynurenine (J. Immunol. (2008) 181, 5396-5404). Further, IDO is also expressed in myeloid-derived suppressor cells (MDSC) of tumor tissues, and MDSC also produces kynurenine (Yu et al. (J. Immunol. (2013) 190, 3783-3797)). Kynurenine is converted into anthranilic acid by kynureninase, and to 3-hydroxykynurenine by kynurenine 3-hydroxylase. Both anthranilic acid and 3-hydroxykynurenine are converted to 3-hydroxyanthranilic acid, a precursor of NAD. Kynurenine is converted to kynurenic acid by kynurenine aminotransferase. Due to these reasons, kynurenine and its metabolite, i.e., anthranilic acid, 3-hydroxykynurenine, kynurenic acid and the like are examples of tumor tissue-specific compounds used in the present disclosure, in particular, tumor cell-specific metabolites.

## (4) Metabolites of arachidonic acid such as prostaglandin E2

Prostaglandin E2 (PGE2) promotes the growth of colon cancer cells and suppresses their apoptosis (Sheng et al. (Cancer Res. (1998) 58, 362-366)). Of PGE2 synthetases, it has been mainly found that COX-1 is constitutively expressed in almost all tissues, whereas COX-2 is induced by certain inflammatory cytokines and oncogenes in tumors (Warner and Mitchell (FASEB J. (2004) 18, 790-804)). Overexpression of COX-2 has been reported to be associated with a poor prognosis in breast cancer (Denkert et al. (Clin. Breast Cancer (2004) 4, 428-433)) and rapid disease progression in ovarian cancer (Denker et al. (Mod. Pathol. (2006) 19, 1261-1269)). In addition, regulatory T cells infiltrating into tumor tissue also produce PGE2 (Curr. Med. Chem. (2011) 18, 5217-5223). Due to these reasons, metabolites of arachidonic acid such as PGE2 are examples of tumor tissue-specific compounds, in particular tumor cell-specific metabolites or tumor tissue-infiltrating immune cell-specific metabolites. Besides PGE2, Thromboxane A2 (TXA2) production is enhanced in tumor tissues such as those in colon cancer (J. Lab. Clin. Med. (1993) 122, 518-523).

## (5) Primary metabolites of the glycolytic pathway or Krebs cycle such as lactic acid succinic acid and citric acid

The glycolytic phenotype characterized by upregulation of glycolytic (Embden-Meyerhof pathway) enzymes such as pyruvate kinase, hexokinase, and lactate dehydrogenase (LDH) has been conventionally known as the Warburg effect, a feature of solid tumors. Lactic acid which is the end product of glycolysis, and succinic acid and citric acid produced by the Krebs cycle are known to be accumulated in tumor tissues (Teresa et al. (Mol. Cancer (2009) 8, 41-59)). Due to these reasons, lactic acid, succinic acid, citric acid, and such, which are primary metabolites produced by glycolysis, are examples of tumor tissue-specific compounds, in particular tumor cell-specific metabolites, used in the present disclosure. In addition, it is known that due to cell death, succinate, which is present in a high concentration in cells, leaks out of cells (Nature Immunology, (2008) 9, 1261-1269). This is thought to be the reason for increased succinic acid concentration in tumor tissues where cell death is frequently occurring.

## (6) Metabolites of nicotinamide such as 1-methyl nicotinamide

It is known that nicotinamide N-methyltransferase is highly expressed in a plurality of human tumor tissues. It is also known that 1-methyl-nicotinamide, which is a stable metabolite of nicotinamide produced by this enzyme, is secreted to the outside of tumor cells (Yamada et al. (J. Nutr. Sci. Vitaminol. (2010) 56, 83-86)). Due to this reason, 1-methyl nicotinamide, and such, which are thought to be accumulated in tumor tissue at a high concentration as a result of the metabolism of nicotinamide are examples of tumor tissue-specific compounds used in the present disclosure.

An "antigen-binding molecule" of the present disclosure comprises an "antigen-binding domain." As the "antigen-binding domain", a domain of any structure can be used as long as it binds to the target antigen. In one embodiment, the antigen-binding domains of this disclosure include, for example, variable regions of antibody heavy chains and/or light chains, Avimers containing a module (A domain) of about 35 amino acids contained in various cell membrane proteins in the living body (International Publications WO2004/044011 and WO2005/040229), Adnectins containing the 10Fn3 domain of fibronectin which is a glycoprotein expressed on the cell membrane (International Publication WO2002/032925), Affibodies using as scaffold an IgG binding domain of 58 amino acids of Protein A (WO WO1995/001937), DARPins (Designed Ankyrin Repeat proteins) using an ankyrin repeat (AR) which is a 33-amino-acid repeating sequence as base (International Publication WO2002/020565), Anticalins containing a lipocalin such as neutrophil gelatinase-associated lipocalin (NGAL) as base (International Publication WO2003/029462), variable lymphocyte receptors (VLRs) which are proteins that function in adaptive immune systems of jawless vertebrates such as the Lampetra japonica and Eptatretus, and contain a leucine-rich-repeat module (LRR) module (International Publication WO2008/016854), and such. In a specific embodiment, the antigen binding domain of this disclosure comprises heavy and light chain variable regions of an antibody. In a further embodiment, the antigen-binding domain of the present disclosure includes, for example, scFv (single chain Fv), single chain antibodies, Fv, scFv2 (single chain Fv2), Fab, or F (ab')2.

## [HVR and variable region]

In one aspect, the present disclosure provides an anti-CD 137 antigen-binding molecule or antibody that comprises at least one, at least two, or all three VH HVR sequences selected from: (a) HVR-H1 comprising the amino acid sequence of SEQ ID NO: 7; (b) HVR-H2 comprising any one of the amino acid sequences selected from SEQ ID NOs: 8, 9, 10, 11, 12, 13, 14, 15, and 16; and (c) HVR-H3 comprising any one of the amino acid sequences selected from SEQ ID NO: 17, 18, 19, or 20. In one embodiment, the anti-CD137 antigen-binding molecule or antibody comprises: (a) HVR-H1 comprising the amino acid sequence of SEQ ID NO: 7; (b) HVR-H2 comprising any one of the amino acid sequences selected from SEQ ID NOs: 8, 9, 10, 11, 12, 13, 14, 15, and 16; and (c) HVR-H3 comprising any one of the amino acid sequences selected from SEQ ID NO: 17, 18, 19, or 20.

In one embodiment, the anti-CD137 antigen-binding molecule is an antibody comprising the amino acid sequence of A375/B167, A372/B040, A356/B040, A486/B167, A487/B167, A488/B226, A489/B223, A548/B376, A551/B256, A551/B379, A555/B379, A548/B256, or A549/B167 described in Table 37 as a heavy chain variable region/light chain variable region combination. In a preferred embodiment, the antigen-binding molecule is an anti-CD 137 antibody comprising HVR-H1, HVR-H2, HVR-H3, HVR-L1, HVR-L2 and HVR-L3 having the same amino acid sequences as HVR-H1, HVR-H2, HVR-H3, HVR-L1, HVR-L2 and HVR-L3 comprised in A375/B167. In a further embodiment, the anti-CD 137 antigen-binding molecule is an anti-CD 137 antibody comprising A375/B167 as a heavy chain variable region/light chain variable region combination. In a different preferable embodiment, the anti-CD137 antigen-binding molecule is an anti-CD137 antibody comprising HVR-H1, HVR-H2, HVR-H3, HVR-L1, HVR-L2 and HVR-L3 having the same amino acid sequences as HVR-H1, HVR-H2, HVR-H3, HVR-L1, HVR-L2 and HVR-L3 comprised in A551/B379. In a further embodiment, the anti-CD137 antigen-binding molecule is an anti-CD 137 antibody comprising A551/B379 as a heavy chain variable region/light chain variable region combination.

In one embodiment, the anti-CD137 antigen-binding molecule or antibody comprises (a) HVR-H1 comprising the amino acid sequence of SEQ ID NO: 7; (b) HVR-H2 comprising the amino acid sequence of SEQ ID NO: 8; and (c) HVR-H3 comprising the amino acid sequence of SEQ ID NO: 17.

In one embodiment, the anti-CD137 antigen-binding molecule or antibody comprises (a) HVR-H1 comprising the amino acid sequence of SEQ ID NO: 7; (b) HVR-H2 comprising the amino acid sequence of SEQ ID NO: 9; and (c) HVR-H3 comprising the amino acid sequence of SEQ ID NO: 17.

In one embodiment, the anti-CD137 antigen-binding molecule or antibody comprises (a) HVR-H1 comprising the amino acid sequence of SEQ ID NO: 7; (b) HVR-H2 comprising the amino acid sequence of SEQ ID NO: 10; and (c) HVR-H3 comprising the amino acid sequence of SEQ ID NO: 17.

In one embodiment, the anti-CD137 antigen-binding molecule or antibody comprises (a) HVR-H1 comprising the amino acid sequence of SEQ ID NO: 7; (b) HVR-H2 comprising the amino acid sequence of SEQ ID NO: 11; and (c) HVR-H3 comprising the amino acid sequence of SEQ ID NO: 18.

In one embodiment, the anti-CD137 antigen-binding molecule or antibody comprises (a) HVR-H1 comprising the amino acid sequence of SEQ ID NO: 7; (b) HVR-H2 comprising the amino acid sequence of SEQ ID NO: 8; and (c) HVR-H3 comprising the amino acid sequence of SEQ ID NO: 18.

In one embodiment, the anti-CD137 antigen-binding molecule or antibody comprises (a) HVR-H1 comprising the amino acid sequence of SEQ ID NO: 7; (b) HVR-H2 comprising the amino acid sequence of SEQ ID NO: 12; and (c) HVR-H3 comprising the amino acid sequence of SEQ ID NO: 18.

In one embodiment, the anti-CD137 antigen-binding molecule or antibody comprises (a) HVR-H1 comprising the amino acid sequence of SEQ ID NO: 7; (b) HVR-H2 comprising the amino acid sequence of SEQ ID NO: 13; and (c) HVR-H3 comprising the amino acid sequence of SEQ ID NO: 18.

In one embodiment, the anti-CD137 antigen-binding molecule or antibody comprises (a) HVR-H1 comprising the amino acid sequence of SEQ ID NO: 7; (b) HVR-H2 comprising the amino acid sequence of SEQ ID NO: 14; and (c) HVR-H3 comprising the amino acid sequence of SEQ ID NO: 19.

In one embodiment, the anti-CD137 antigen-binding molecule or antibody comprises (a) HVR-H1 comprising the amino acid sequence of SEQ ID NO: 7; (b) HVR-H2 comprising the amino acid sequence of SEQ ID NO: 15; and (c) HVR-H3 comprising the amino acid sequence of SEQ ID NO: 20.

In one embodiment, the anti-CD137 antigen-binding molecule or antibody comprises (a) HVR-H1 comprising the amino acid sequence of SEQ ID NO: 7; (b) HVR-H2 comprising the amino acid sequence of SEQ ID NO: 16; and (c) HVR-H3 comprising the amino acid sequence of SEQ ID NO: 20.

In one embodiment, the anti-CD137 antigen-binding molecule or antibody comprises (a) HVR-H1 comprising the amino acid sequence of SEQ ID NO: 7; (b) HVR-H2 comprising the amino acid sequence of SEQ ID NO: 14; and (c) HVR-H3 comprising the amino acid sequence of SEQ ID NO: 17.

In a different aspect, the present disclosure provides an anti-CD 137 antigen-binding molecule or antibody that comprises at least one, at least two, or all three VL HVR sequences selected from: (a) HVR-L1 comprising any one of the amino acid sequences selected from SEQ ID NOs: 21, 22, 23, 24, and 25; (b) HVR-L2 comprising the amino acid sequence of SEQ ID NO: 26; and (c) HVR-L3 comprising any one of the amino acid sequences selected from SEQ ID NOs: 27, 28 and 29. In one embodiment, the anti-CD137 antigen-binding molecule or antibody comprises (a) HVR-L1 comprising any one of the amino acid sequences selected from SEQ ID NOs: 21, 22, 23, 24, and 25; (b) HVR-L2 comprising the amino acid sequence of SEQ ID NO: 26; and (c) HVR-L3 comprising any one of the amino acid sequences selected from SEQ ID NOs: 27, 28 and 29.

In one embodiment, the anti-CD137 antigen-binding molecule or antibody comprises (a) HVR-L1 comprising the amino acid sequence of SEQ ID NO: 21; (b) HVR-L2 comprising the amino acid sequence of SEQ ID NO: 26; and (c) HVR-L3 comprising the amino acid sequence of SEQ ID NO: 27.

In one embodiment, the anti-CD137 antigen-binding molecule or antibody comprises (a) HVR-L1 comprising the amino acid sequence of SEQ ID NO: 22; (b) HVR-L2 comprising the amino acid sequence of SEQ ID NO: 26; and (c) HVR-L3 comprising the amino acid sequence of SEQ ID NO: 27.

In one embodiment, the anti-CD137 antigen-binding molecule or antibody comprises (a) HVR-L1 comprising the amino acid sequence of SEQ ID NO: 21; (b) HVR-L2 comprising the amino acid sequence of SEQ ID NO: 26; and (c) HVR-L3 comprising the amino acid sequence of SEQ ID NO: 28.

In one embodiment, the anti-CD137 antigen-binding molecule or antibody comprises (a) HVR-L1 comprising the amino acid sequence of SEQ ID NO: 21; (b) HVR-L2 comprising the amino acid sequence of SEQ ID NO: 26; and (c) HVR-L3 comprising the amino acid sequence of SEQ ID NO: 29.

In one embodiment, the anti-CD137 antigen-binding molecule or antibody comprises (a) HVR-L1 comprising the amino acid sequence of SEQ ID NO: 23; (b) HVR-L2 comprising the amino acid sequence of SEQ ID NO: 26; and (c) HVR-L3 comprising the amino acid sequence of SEQ ID NO: 27.

In one embodiment, the anti-CD137 antigen-binding molecule or antibody comprises (a) HVR-L1 comprising the amino acid sequence of SEQ ID NO: 24; (b) HVR-L2 comprising the amino acid sequence of SEQ ID NO: 26; and (c) HVR-L3 comprising the amino acid sequence of SEQ ID NO: 27.

In one embodiment, the anti-CD137 antigen-binding molecule or antibody comprises (a) HVR-L1 comprising the amino acid sequence of SEQ ID NO: 25; (b) HVR-L2 comprising the amino acid sequence of SEQ ID NO: 26; and (c) HVR-L3 comprising the amino acid sequence of SEQ ID NO: 27.

In another aspect, the anti-CD137 antigen-binding molecule or antibody of this disclosure comprises (a) a VH domain comprising at least one, at least two, or all three VH HVR sequences selected from (i) HVR-H1 comprising the amino acid sequence of SEQ ID NO: 7; (ii) HVR-H2 comprising any one of the amino acid sequences selected from SEQ ID NOs: 8, 9, 10, 11, 12, 13, 14, 15, and 16; and (iii) HVR-H3 comprising any one of the amino acid sequences selected from SEQ ID NOs: 17, 18, 19, or 20; and (b) a VL domain comprising at least one, at least two, or all three VL HVR sequences selected from (i) HVR-L1 comprising any one of the amino acid sequences selected from SEQ ID NOs: 21, 22, 23, 24, and 25; (ii) HVR-L2 comprising the amino acid sequence of SEQ ID NO: 26; and (iii) HVR-L3 comprising any one of the amino acid sequences selected from SEQ ID NOs: 27, 28, and 29.

In another aspect, the present disclosure provides an anti-CD 137 antigen-binding molecule or antibody comprising (a) HVR-H1 comprising the amino acid sequence of SEQ ID NO: 7; (b) HVR-H2 comprising the amino acid sequence of SEQ ID NO: 8; (c) HVR-H3 comprising the amino acid sequence of SEQ ID NO: 17; (d) HVR-L1 comprising the amino acid sequence of SEQ ID NO: 21; (e) HVR-L2 comprising the amino acid sequence of SEQ ID NO: 26; and (f) HVR-L3 comprising the amino acid sequence of SEQ ID NO: 27.

In another aspect, the present disclosure provides an anti-CD 137 antigen-binding molecule or antibody comprising (a) HVR-H1 comprising the amino acid sequence of SEQ ID NO: 7; (b) HVR-H2 comprising the amino acid sequence of SEQ ID NO: 9; (c) HVR-H3 comprising the amino acid sequence of SEQ ID NO: 17; (d) HVR-L1 comprising the amino acid sequence of SEQ ID NO: 22; (e) HVR-L2 comprising the amino acid sequence of SEQ ID NO: 26; and (f) HVR-L3 comprising the amino acid sequence of SEQ ID NO: 27.

In another aspect, the present disclosure provides an anti-CD 137 antigen-binding molecule or antibody comprising (a) HVR-H1 comprising the amino acid sequence of SEQ ID NO: 7; (b) HVR-H2 comprising the amino acid sequence of SEQ ID NO: 10; (c) HVR-H3 comprising the amino acid sequence of SEQ ID NO: 17; (d) HVR-L1 comprising the amino acid sequence of SEQ ID NO: 22; (e) HVR-L2 comprising the amino acid sequence of SEQ ID NO: 26; and (f) HVR-L3 comprising the amino acid sequence of SEQ ID NO: 27.

In another aspect, the present disclosure provides an anti-CD 137 antigen-binding molecule or antibody comprising (a) HVR-H1 comprising the amino acid sequence of SEQ ID NO: 7; (b) HVR-H2 comprising the amino acid sequence of SEQ ID NO: 11; (c) HVR-H3 comprising the amino acid sequence of SEQ ID NO: 18; (d) HVR-L1 comprising the amino acid sequence of SEQ ID NO: 21; (e) HVR-L2 comprising the amino acid sequence of SEQ ID NO: 26; and (f) HVR-L3 comprising the amino acid sequence of SEQ ID NO: 27.

In another aspect, the present disclosure provides an anti-CD 137 antigen-binding molecule or antibody comprising (a) HVR-H1 comprising the amino acid sequence of SEQ ID NO: 7; (b) HVR-H2 comprising the amino acid sequence of SEQ ID NO: 8; (c) HVR-H3 comprising the amino acid sequence of SEQ ID NO: 18; (d) HVR-L1 comprising the amino acid sequence of SEQ ID NO: 21; (e) HVR-L2 comprising the amino acid sequence of SEQ ID NO: 26; and (f) HVR-L3 comprising the amino acid sequence of SEQ ID NO: 27.

In another aspect, the present disclosure provides an anti-CD 137 antigen-binding molecule or antibody comprising (a) HVR-H1 comprising the amino acid sequence of SEQ ID NO: 7; (b) HVR-H2 comprising the amino acid sequence of SEQ ID NO: 12; (c) HVR-H3 comprising the amino acid sequence of SEQ ID NO: 18; (d) HVR-L1 comprising the amino acid sequence of SEQ ID NO: 21; (e) HVR-L2 comprising the amino acid sequence of SEQ ID NO: 26; and (f) HVR-L3 comprising the amino acid sequence of SEQ ID NO: 28.

In another aspect, the present disclosure provides an anti-CD 137 antigen-binding molecule or antibody comprising (a) HVR-H1 comprising the amino acid sequence of SEQ ID NO: 7; (b) HVR-H2 comprising the amino acid sequence of SEQ ID NO: 13; (c) HVR-H3 comprising the amino acid sequence of SEQ ID NO: 18; (d) HVR-L1 comprising the amino acid sequence of SEQ ID NO: 21; (e) HVR-L2 comprising the amino acid sequence of SEQ ID NO: 26; and (f) HVR-L3 comprising the amino acid sequence of SEQ ID NO: 29.

In another aspect, the present disclosure provides an anti-CD 137 antigen-binding molecule or antibody comprising (a) HVR-H1 comprising the amino acid sequence of SEQ ID NO: 7; (b) HVR-H2 comprising the amino acid sequence of SEQ ID NO: 14; (c) HVR-H3 comprising the amino acid sequence of SEQ ID NO: 19; (d) HVR-L1 comprising the amino acid sequence of SEQ ID NO: 23; (e) HVR-L2 comprising the amino acid sequence of SEQ ID NO: 26; and (f) HVR-L3 comprising the amino acid sequence of SEQ ID NO: 27.

In another aspect, the present disclosure provides an anti-CD 137 antigen-binding molecule or antibody comprising (a) HVR-H1 comprising the amino acid sequence of SEQ ID NO: 7; (b) HVR-H2 comprising the amino acid sequence of SEQ ID NO: 15; (c) HVR-H3 comprising the amino acid sequence of SEQ ID NO: 20; (d) HVR-L1 comprising the amino acid sequence of SEQ ID NO: 24; (e) HVR-L2 comprising the amino acid sequence of SEQ ID NO: 26; and (f) HVR-L3 comprising the amino acid sequence of SEQ ID NO: 27.

In another aspect, the present disclosure provides an anti-CD 137 antigen-binding molecule or antibody comprising (a) HVR-H1 comprising the amino acid sequence of SEQ ID NO: 7; (b) HVR-H2 comprising the amino acid sequence of SEQ ID NO: 15; (c) HVR-H3 comprising the amino acid sequence of SEQ ID NO: 20; (d) HVR-L1 comprising the amino acid sequence of SEQ ID NO: 25; (e) HVR-L2 comprising the amino acid sequence of SEQ ID NO: 26; and (f) HVR-L3 comprising the amino acid sequence of SEQ ID NO: 27.

In another aspect, the present disclosure provides an anti-CD 137 antigen-binding molecule or antibody comprising (a) HVR-H1 comprising the amino acid sequence of SEQ ID NO: 7; (b) HVR-H2 comprising the amino acid sequence of SEQ ID NO: 16; (c) HVR-H3 comprising the amino acid sequence of SEQ ID NO: 20; (d) HVR-L1 comprising the amino acid sequence of SEQ ID NO: 25; (e) HVR-L2 comprising the amino acid sequence of SEQ ID NO: 26; and (f) HVR-L3 comprising the amino acid sequence of SEQ ID NO: 27.

In another aspect, the present disclosure provides an anti-CD 137 antigen-binding molecule or antibody comprising (a) HVR-H1 comprising the amino acid sequence of SEQ ID NO: 7; (b) HVR-H2 comprising the amino acid sequence of SEQ ID NO: 14; (c) HVR-H3 comprising the amino acid sequence of SEQ ID NO: 19; (d) HVR-L1 comprising the amino acid sequence of SEQ ID NO: 24; (e) HVR-L2 comprising the amino acid sequence of SEQ ID NO: 26; and (f) HVR-L3 comprising the amino acid sequence of SEQ ID NO: 27.

In another aspect, the present disclosure provides an anti-CD 137 antigen-binding molecule or antibody comprising (a) HVR-H1 comprising the amino acid sequence of SEQ ID NO: 7; (b) HVR-H2 comprising the amino acid sequence of SEQ ID NO: 14; (c) HVR-H3 comprising the amino acid sequence of SEQ ID NO: 17; (d) HVR-L1 comprising the amino acid sequence of SEQ ID NO: 21; (e) HVR-L2 comprising the amino acid sequence of SEQ ID NO: 26; and (f) HVR-L3 comprising the amino acid sequence of SEQ ID NO: 27.

In a specific embodiment, any one or more amino acids of the above-described anti-CD 137 antibody are substituted at the following HVR positions:

> In HVR-H2 (SEQ ID NO: 30): positions 5, 6, 7, 10, 13, 14, and/or 17
> In HVR-H3 (SEQ ID NO: 31): position 3 and/or 6
> In HVR-L1 (SEQ ID NO: 32): positions 4, 5, 9, and/or 11
> In HVR -L3 (SEQ ID NO: 33): positions 6, 7, and/or 8.

In a specific embodiment, the substitutions provided by the present specification are conservative substitutions. In a specific embodiment, any one or more substitutions in the following may be performed in any combination:

> In HVR-H2 (SEQ ID NO: 8): K5H or S; S6G; T7S; E10Y; D13E; S14Q; V17G or L
> In HVR-H3 (SEQ ID NO: 17): A3P, K or I; F6E
> In HVR-L1 (SEQ ID NO: 21): R4S; Y5T; Y9F; E11N
> In HVR-L3 (SEQ ID NO: 27): E6P; H7A; Q8I

All possible combinations of the above-mentioned substitutions are encompassed in the consensus sequences of SEQ ID NOs: 30, 31, 32 and 33 for HVR-H2, HVR-H3, HVR-L1 and HVR-L3, respectively.

In any of the above embodiments, an anti-CD 137 antigen-binding molecule or antibody is humanized. In one embodiment, an anti-CD 137 antigen-binding molecule or antibody comprises HVRs as in any of the above embodiments, and further comprises an acceptor human framework, e.g. a human immunoglobulin framework or a human consensus framework. In another embodiment, an anti-CD 137 antigen-binding molecule or antibody comprises HVRs as in any of the above embodiments, and further comprises a heavy chain variable region (VH) or a light chain variable region (VL) comprising a framework (FR) sequence. In one embodiment, FR1 in the heavy chain variable region comprises the amino acid sequence of SEQ ID NO: 35, FR2 in the heavy chain variable region comprises the amino acid sequence of SEQ ID NO: 36, FR3 in the heavy chain variable region comprises the amino acid sequence of SEQ ID NO: 37, and FR4 in the heavy chain variable region comprises the amino acid sequence of SEQ ID No: 38. In one embodiment, FR1 in the light chain variable region comprises the amino acid sequence of SEQ ID NO: 39, FR2 in the light chain variable region comprises the amino acid sequence of SEQ ID NO: 40, FR3 in the light chain variable region comprises the amino acid sequence of SEQ ID NO: 41, and FR4 in the light chain variable region comprises the amino acid sequence of SEQ ID NO: 42.

In another aspect, an anti-CD 137 antigen-binding molecule or antibody comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO: 43, 44, 45, 46, 47, 48, 49, 50, 51, 52, or 53. In certain embodiments, a VH sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-CD137 antigen-binding molecule or antibody comprising that sequence retains the ability to bind to CD137. In certain embodiments, a total of 1 to 10 amino acids have been substituted, inserted and/or deleted in SEQ ID NO: 43, 44, 45, 46, 47, 48, 49, 50, 51, 52, or 53. In certain embodiments, substitutions, insertions, or deletions occur in regions outside the HVRs (i.e., in the FRs). Optionally, the anti-CD137 antibody comprises the VH sequence in SEQ ID NO: 43, 44, 45, 46, 47, 48, 49, 50, 51, 52, or 53, including post-translational modifications of that sequence. In a particular embodiment, the VH comprises one, two or three HVRs selected from: (a) HVR-H1 comprising the amino acid sequence of SEQ ID NO: 7; (b) HVR-H2 comprising any one amino acid sequence selected from SEQ ID NOs: 8, 9, 10, 11, 12, 13, 14, 15, and 16; and (c) HVR-H3 comprising any one amino acid sequence selected from SEQ ID NO: 17, 18, 19, or 20. Post-translational modifications include but are not limited to a modification of glutamine or glutamate in N-terminal of heavy chain or light chain to pyroglutamic acid by pyroglutamylation.

In another aspect, an anti-CD 137 antigen-binding molecule or antibody is provided, wherein the antibody comprises a light chain variable domain (VL) having at least 90%, 91%, 92%, 93%, 94%, 95%, 96%, 97%, 98%, 99%, or 100% sequence identity to the amino acid sequence of SEQ ID NO: 54, 55, 56, 57, 58, 59, or 60. In certain embodiments, a VL sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-CD 137 antigen-binding molecule or antibody comprising that sequence retains the ability to bind to CD137. In certain embodiments, a total of 1 to 10 amino acids have been substituted, inserted and/or deleted in SEQ ID NO: 54, 55, 56, 57, 58, 59, or 60. In certain embodiments, the substitutions, insertions, or deletions occur in regions outside the HVRs (i.e., in the FRs). Optionally, the anti-CD 137 antigen-binding molecule or antibody comprises the VL sequence in SEQ ID NO: 54, 55, 56, 57, 58, 59, or 60, including post-translational modifications of that sequence. In a particular embodiment, the VL comprises one, two or three HVRs selected from (a) HVR-L1 comprising any one amino acid sequence selected from SEQ ID NOs: 21, 22, 23, 24, and 25; (b) HVR-L2 comprising the amino acid sequence of SEQ ID NO: 26; and (c) HVR-L3 comprising any one amino acid sequence selected from SEQ ID NO: 27, 28, and 29. Post-translational modifications include but are not limited to a modification of glutamine or glutamate in N-terminal of heavy chain or light chain to pyroglutamic acid by pyroglutamylation.

In another aspect, an anti-CD 137 antigen-binding molecule or antibody is provided, wherein the antigen-binding molecule or antibody comprises a VH as in any of the embodiments provided above, and a VL as in any of the embodiments provided above.

- In one embodiment, the anti-CD137 antigen-binding molecule or antibody comprises the VH and VL sequences in SEQ ID NO: 43 and SEQ ID NO: 54, respectively, including post-translational modifications of those sequences.
- In one embodiment, the anti-CD137 antigen-binding molecule or antibody comprises the VH and VL sequences in SEQ ID NO: 44 and SEQ ID NO: 55, respectively, including post-translational modifications of those sequences.
- In one embodiment, the anti-CD137 antigen-binding molecule or antibody comprises the VH and VL sequences in SEQ ID NO: 45 and SEQ ID NO: 55, respectively, including post-translational modifications of those sequences.
- In one embodiment, the anti-CD137 antigen-binding molecule or antibody comprises the VH and VL sequences in SEQ ID NO: 46 and SEQ ID NO: 54, respectively, including post-translational modifications of those sequences.
- In one embodiment, the anti-CD137 antigen-binding molecule or antibody comprises the VH and VL sequences in SEQ ID NO: 47 and SEQ ID NO: 54, respectively, including post-translational modifications of those sequences.
- In one embodiment, the anti-CD137 antigen-binding molecule or antibody comprises the VH and VL sequences in SEQ ID NO: 48 and SEQ ID NO: 56, respectively, including post-translational modifications of those sequences.
- In one embodiment, the anti-CD137 antigen-binding molecule or antibody comprises the VH and VL sequences in SEQ ID NO: 49 and SEQ ID NO: 57, respectively, including post-translational modifications of those sequences.
- In one embodiment, the anti-CD137 antigen-binding molecule or antibody comprises the VH and VL sequences in SEQ ID NO: 50 and SEQ ID NO: 58, respectively, including post-translational modifications of those sequences.
- In one embodiment, the anti-CD137 antigen-binding molecule or antibody comprises the VH and VL sequences in SEQ ID NO: 51 and SEQ ID NO: 59, respectively, including post-translational modifications of those sequences.

- In one embodiment, the anti-CD137 antigen-binding molecule or antibody comprises the VH and VL sequences in SEQ ID NO: 51 and SEQ ID NO: 60, respectively, including post-translational modifications of those sequences.
- In one embodiment, the anti-CD137 antigen-binding molecule or antibody comprises the VH and VL sequences in SEQ ID NO: 52 and SEQ ID NO: 60, respectively, including post-translational modifications of those sequences.
- In one embodiment, the anti-CD137 antigen-binding molecule or antibody comprises the VH and VL sequences in SEQ ID NO: 50 and SEQ ID NO: 59, respectively, including post-translational modifications of those sequences.
- In one embodiment, the anti-CD137 antigen-binding molecule or antibody comprises the VH and VL sequences in SEQ ID NO: 53 and SEQ ID NO: 54, respectively, including post-translational modifications of those sequences.

The above-mentioned post-translational modifications include but are not limited to a modification of glutamine or glutamate in N-terminal of heavy chain or light chain to pyroglutamic acid by pyroglutamylation.

SEQ ID NOs corresponding to the amino acid sequences of the preferred heavy chain variable region and light chain variable region and their HVR1, 2, and 3 for each anti-CD 137 antigen-binding molecule or antibody of the present disclosure are shown in the table below.

[Table 1]

| Heavy chain/ light chain variable regions | SEQ ID NO of variable region | | SEQ ID NO of hypervariable region (HVR) | | | | | |
|---|---|---|---|---|---|---|---|---|
| | Heavy chain | Light chain | H1 | H2 | H3 | L1 | L2 | L3 |
| A375/B167 | 43 | 54 | 7 | 8 | 17 | 21 | 26 | 27 |
| A372/B040 | 44 | 55 | 7 | 9 | 17 | 22 | 26 | 27 |
| A356/B040 | 45 | 55 | 7 | 10 | 17 | 22 | 26 | 27 |
| A486/B167 | 46 | 54 | 7 | 11 | 18 | 21 | 26 | 27 |
| A487/B167 | 47 | 54 | 7 | 8 | 18 | 21 | 26 | 27 |
| A488/B226 | 48 | 56 | 7 | 12 | 18 | 21 | 26 | 28 |
| A489/B223 | 49 | 57 | 7 | 13 | 18 | 21 | 26 | 29 |
| A548/B376 | 50 | 58 | 7 | 14 | 19 | 23 | 26 | 27 |
| A551/B256 | 51 | 59 | 7 | 15 | 20 | 24 | 26 | 27 |
| A551/B379 | 51 | 60 | 7 | 15 | 20 | 25 | 26 | 27 |
| A555/B379 | 52 | 60 | 7 | 16 | 20 | 25 | 26 | 27 |
| A548/B256 | 50 | 59 | 7 | 14 | 19 | 24 | 26 | 27 |
| A549/B167 | 53 | 54 | 7 | 14 | 17 | 21 | 26 | 27 |

When an anti-CD 137 antigen-binding molecule or antibody provided herein has glutamine as the heavy chain or light chain N terminus amino acid, that amino acid may be substituted by glutamic acid. When an anti-CD 137 antibody provided herein has glutamic acid as the heavy chain or light chain N terminus amino acid, that amino acid may be substituted by glutamine.

In a preferred embodiment, the anti-CD 137 antigen-binding molecules or antibodies comprising the above-described HVRs, heavy chain variable regions, and/or light chain variable regions all have low-molecular-weight-compound-dependent binding activity toward CD137 as described above.

[Constant regions]

In another aspect, the anti-CD137 antigen-binding molecules or antibodies comprise a constant region. The constant region may be a heavy chain constant region (including an Fc region), a light chain constant region, or both. In a further aspect, the anti-CD137 antigen-binding molecules or antibodies comprise an Fc region. In some embodiments, the constant region is the one with the native sequence. Examples of heavy chain constant regions derived from native antibodies include, for example, a heavy chain constant region of human IgG1 (SEQ ID NOs: 61, 62), human IgG2, human IgG3, human IgG4, and such. Examples of light chain constant regions derived from native antibodies include, for example, human kappa chain, human lambda chain (e.g., SEQ ID NO: 63), and such.

A "parent constant region" or "parent Fc region" used herein refers to a constant region or an Fc region prior to introducing amino acid alteration(s) described herein. A "parent antigen-binding molecule" refers to an antigen-binding molecule that comprises the parent constant region or parent Fc region. In some embodiments, the parent Fc region is an Fc region having a native sequence (or an Fc region of a native antibody). Antibodies include, for example, IgA (IgA1, IgA2), IgD, IgE, IgG (IgG1, IgG2, IgG3, IgG4), IgM, etc. Antibodies may be derived from human or monkey (e.g., cynomolgus, rhesus macaque, marmoset, chimpanzee, or baboon). Native antibodies may also include naturally-occurring mutations. A plurality of allotype sequences of IgGs due to genetic polymorphism are described in "Sequences of proteins of immunological interest", NIH Publication No. 91-3242, and any of them may be used in the present disclosure. In one embodiment, the parent Fc region is an Fc region derived from a heavy chain constant region of human IgG1, shown in SEQ ID NO: 61, 62, or 182.

In one aspect, the anti-CD137 antigen-binding molecules or antibodies have an increased isoelectric point (pI), as compared to anti-CD137 antigen-binding molecules or antibodies that comprise a native sequence Fc region or a parent Fc region. In some embodiments, variant Fc regions include at least one amino acid alteration. In further embodiments, the amino acid alteration leads to the elevation of isoelectric point (pI) of the variant Fc region as compared to the parent Fc region. Without being bound by a particular theory, it is believed that the pH of biological fluids (for example, plasma) is in a neutral pH range. In biological fluids, the net positive charge of a pI-increased antigen-binding molecule or antibody is increased due to the increased pI, and as a result the antigen-binding molecule or antibody is more strongly attracted by physicochemical Coulomb interaction to the endothelial cell surface that has a net negative charge compared to an antigen-binding molecule or antibody not having an increased pI. By this, the agonistic antigen-binding molecules (or antibodies), or antigen-bound agonistic antigen-binding molecules (or antibodies) may come closer to the surface of cells which express Fc-gamma receptor, resulting in an increased binding of the antigen-binding molecules or antibodies to Fc-gamma receptor-expressing cells. For those anti-CD137 agonistic antigen-binding molecules or antibodies that show CD137 agonistic activity based on the contribution by binding activity toward Fc-gamma receptor, anti-CD137 agonistic antigen-binding molecules or antibodies having increased binding toward Fc-gamma receptor-expressing cells due to the pI-increasing amino acid alterations can exhibit stronger CD137 agonistic activity as compared to anti-CD137 agonistic antigen-binding molecules or antibodies having no pI-increasing amino acid alterations.

In the present disclosure, pI may be either a theoretical or an experimentally determined pI. The value of pI can be determined, for example, by isoelectric focusing known to those skilled in the art. The value of a theoretical pI can be calculated, for example, using gene and amino acid sequence analysis software (Genetyx, *etc.*). In calculating, properties of an antibody may be reflected in a calculation formula. For example, (i) generally, Cys conserved within an antibody forms a disulfide bond and does not carry electric charge of the side chain; therefore, such Cys may be excluded from the calculation and only the free-form Cys which does not form a disulfide bond may be included in the calculation. Alternatively, (ii) charge state or isoelectric point of antibodies can change because of post-translational modifications; therefore, a calculation formula may be modified as follows, giving consideration on such post-translational modifications: (a) when the N-terminus of the heavy chain is Q (glutamine), the N-terminal amino group is excluded from the calculation, assuming that pyroglutamylation occurs, (b) when the C-terminus of the heavy chain is K (lysine), K (one residue) is excluded from the calculation, assuming that truncation occurs; and (c) side chains of all C (cysteine) present at generally conserved positions are excluded from the calculation, assuming that all these C form disulfide bonds within the molecule. In one preferred embodiment, both above-described (i) and (ii) may be reflected in the calculation formula.

In one embodiment, the pI value may be increased, for example, at least by 0.01, 0.03, 0.05, 0.1, 0.2, 0.3, 0.4, 0.5, or more, at least by 0.6, 0.7, 0.8, 0.9, or more, at least by 1.0, 1.1, 1.2, 1.3, 1.4, 1.5, or more, or at least by 1.6, 1.7, 1.8, 1.9, 2.0, 2.1, 2.2, 2.3, 2.4, 2.5, 3.0 or more, as compared to before modification.

In one embodiment, amino acid alterations relating to pI increase and methods for increasing pI of an antigen-binding molecule or antibody are described herein in detail at "III. Compositions and methods (agonistic antigen-binding molecules comprising a variant Fc region with increased isoelectric point (pI))". Those skilled in the art would understand that any amino acid alterations and methods for increasing pI described under "III. Compositions and methods (agonistic antigen-binding molecules comprising a variant Fc region with increased isoelectric point (pI))" can be applied to the anti-CD137 antigen-binding molecules or antibodies.

In one embodiment, the anti-CD137 antigen-binding molecules or antibodies have a variant Fc region with increased pI, and the variant Fc region comprises at least one amino acid alteration of at least one position selected from the group consisting of positions 285, 311, 312, 315, 318, 333, 335, 337, 341, 342, 343, 384, 385, 388, 390, 399, 400, 401, 402, 413, 420, 422, and 431, according to EU numbering. In further embodiments, variant Fc regions with increased pI comprise Arg or Lys at each selected position.

In further embodiments, the anti-CD137 antigen-binding molecules or antibodies have a variant Fc region with increased pI, and the variant Fc region comprises at least one amino acid alteration of at least one position selected from the group consisting of positions 311, 343, and 413, according to EU numbering. In further embodiments, the variant Fc regions with increased pI comprise an amino acid alteration at position 311, 343, or 413 according to EU numbering. In a further embodiment, the variant Fc regions with increased pI comprise Arg or Lys at each selected position.

In another aspect, the present disclosure provides anti-CD137 antigen-binding molecules or antibodies comprising a variant Fc region with increased pI, which comprises amino acid alterations of any one of following (1) to (3): (1) at positions 311 and 343; (2) at positions 311 and 413; and (3) at positions 343 and 413, according to EU numbering. In further embodiments, the variant Fc regions with increased pI comprise Arg or Lys at each selected position.

In one embodiment, the anti-CD137 antigen-binding molecules or antibodies of the present disclosure comprise a variant Fc region which comprises amino acid alteration(s) identified in Table 2 below.

Amino acid alterations for increasing pI of an Fc region

[Table 2]

| Number | Amino acid substitutions (EU numbering) |
|--------|------------------------------------------|
| 1 | P343R/D413K |
| 2 | Q311R/P343R |
| 3 | P343R |
| 4 | D413K |
| 5 | Q311R |
| 6 | Q311R/D413K |

In one embodiment, the anti-CD137 antigen-binding molecules or antibodies comprise a variant Fc region prepared by making amino acid alteration(s) to an Fc region having a native sequence. In one embodiment, the variant Fc regions have an increased binding activity towards at least one Fc-gamma receptor selected from the group consisting of Fc-gamma RIa, Fc-gamma RIIa, Fc-gamma RIIb, Fc-gamma RIIIa, and Fc-gamma RIIIb, as compared to an Fc region having a native sequence or a parent Fc region. Preferably, the variant Fc regions have an increased binding activity towards Fc-gamma RIIb, as compared to an Fc region having a native sequence or a parent Fc region. It is reported that an anti-CD 137 antibody comprising a variant Fc region with increased binding activity towards Fc-gamma RIIb has increased agonistic activity, as compared to the anti-CD 137 antibody comprising an Fc region with a native sequence. In one embodiment, as amino acid alterations to increase binding activity towards Fc-gamma RIIb, amino acid alterations taught in WO2012/115241, WO2014/030728, WO2014/163101, and/or WO2017/104783 may be used, for example. In one preferred embodiment, alterations to increase binding activity towards Fc-gamma RIIb are amino acid alteration(s) of at least one position selected from the group consisting of positions 234, 235, 236, 237, 238, 264, 268, 295, 326, and 330, according to EU numbering.

"Fc gamma receptors" (herein, referred to as Fc gamma receptors, Fc gamma R or FcgR) refers to receptors that may bind to the Fc region of IgG1, IgG2, IgG3, and IgG4 monoclonal antibodies, and practically means any member of the family of proteins encoded by the Fc gamma receptor genes. In humans, this family includes Fc gamma RI (CD64) including isoforms Fc gamma RIa, Fc gamma RIb, and Fc gamma RIc; Fc gamma RII (CD32) including isoforms Fc gamma RIIa (including allotypes H131 (type H) and R131 (type R)), Fc gamma RIIb (including Fc gamma RIIb-1 and Fc gamma RIIb-2), and Fc gamma RIIc; and Fc gamma RIII (CD16) including isoforms Fc gamma RIIIa (including allotypes V158 and F158), and Fc gamma RIIIb (including allotypes Fc gamma RIIIb-NA1 and Fc gamma RIIIb-NA2), and any human Fc gamma Rs, Fc gamma R isoforms or allotypes yet to be discovered, but is not limited thereto. Fc gamma RIIb1 and Fc gamma RIIb2 have been reported as splicing variants of human Fc gamma RIIb. In addition, a splicing variant named Fc gamma RIIb3 has been reported (J Exp Med, 1989, 170: 1369-1385). In addition to these splicing variants, human Fc gamma RIIb includes AAI46679.1 registered in NCBI and all splicing variants registered in NCBI, which are NP_001002273.1, NP_001002274.1, NP_001002275.1, NP_001177757.1, and NP_003992.3. Furthermore, human Fc gamma RIIb includes every previously-reported genetic polymorphism, as well as Fc gamma RIIb (Arthritis Rheum. 48: 3242-3252 (2003); Kono et al., Hum. Mol. Genet. 14: 2881-2892 (2005); and Kyogoju et al., Arthritis Rheum. 46: 1242-1254 (2002)), and every genetic polymorphism that will be reported in the future.

In Fc gamma RIIa, there are two allotypes, one where the amino acid at position 131 of Fc gamma RIIa is histidine (type H) and the other where the amino acid at position 131 is substituted with arginine (type R) (Warrmerdam, J. Exp. Med. 172: 19-25 (1990)).

The Fc gamma R includes human, mouse, rat, rabbit, and monkey-derived Fc gamma Rs but is not limited thereto, and may be derived from any organism. Mouse Fc gamma Rs include Fc gamma RI (CD64), Fc gamma RII (CD32), Fc gamma RIII (CD16), and Fc gamma RIII-2 (CD16-2), and any mouse Fc gamma Rs, or Fc gamma R isoforms, but are not limited thereto.

In another aspect, the present disclosure provides the anti-CD137 antigen-binding molecules or antibodies comprising a variant Fc region having an increased binding activity towards Fc-gamma RIIb, which comprises amino acid alterations of any one of following (1) to (8): (1) at positions 234, 238, 264, and 330; (2) at positions 234, 238, and 330; (3) at positions 234, 237, 238, and 330; (4) at positions 236, 268, and 330; (5) at positions 235, 236, 268, 295, 326, and 330; according to EU numbering.

In one embodiment, the anti-CD137 antigen-binding molecules or antibodies of the present disclosure comprise a variant Fc region comprising amino acid alterations identified in Table 3 below. In a further embodiment, the anti-CD137 antigen-binding molecules or antibodies of the present disclosure comprises a variant Fc region which further comprises, in addition to the amino acid alteration(s) described in Table 2 (Amino acid alterations involving increasing pI of an Fc region), any one combination of amino acid alterations identified in Table 3 below.

Amino acid alterations for increasing Fc-gamma RIIb binding activity of an Fc region

[Table 3]

| Number | Amino acid substitution (EU numbering) |
| --- | --- |
| 1 | L234Y/P238D/V264I/A330K |
| 2 | L234Y/P238D/A330K |
| 3 | L234Y/G237D/P238D/A330K |
| 4 | G236N/H268D/A330K |
| 5 | L235W/G236N/H268D/Q295L/K326T/A330K |

In one embodiment, the present disclosure provides variant Fc regions, including those variant Fc regions that have alteration of at least one amino acid and have binding activity towards Fc-gamma RIIb equivalent to or higher than that of a reference Fc region. In one embodiment, the reference Fc region is an Fc region comprising any one combination of amino acid alterations identified in Table 3 above. In one preferred embodiment, the reference Fc region is an Fc region comprised in a heavy chain constant region TT14 (SEQ ID NO: 149), TT16 (SEQ ID NO: 150), MY201 (SEQ ID NO: 153), or MY518 (SEQ ID NO: 154). In one preferred embodiment, the reference Fc region is an Fc region comprised in a heavy chain constant region MY201 (SEQ ID NO: 153) or MY518 (SEQ ID NO: 154).

In another aspect, the present disclosure provides isolated agonistic antigen-binding molecules or antibodies that comprise a variant Fc region with increased binding activity towards Fc-gamma receptor (preferably, Fc-gamma RIIb) and increased pI. In a certain embodiment, the variant Fc regions described herein comprise at least two amino acid alterations in the parent Fc regions. As described above, an antigen-binding molecule or antibody with an increased pI is more strongly attracted by physicochemical Coulomb interaction to the endothelial cell surface that has a net negative charge compared to an antigen-binding molecule or antibody not having an increased pI. Therefore, for those agonistic antigen-binding molecules or antibodies that show agonistic activity based on the contribution by binding activity toward Fc-gamma receptor (preferably Fc-gamma RIIb), agonistic activity of the antigen-binding molecules or antibodies can be increased by combining amino acid alteration(s) to increase Fc-gamma receptor (preferably Fc-gamma RIIb) and amino acid alteration(s) to increase pI.

In one embodiment, the anti-CD137 antigen-binding molecules or antibodies comprise a variant Fc region that comprises both the amino acid alteration(s) to increase binding activity towards Fc-gamma receptor (e.g., Fc-gamma RIIb) and amino acid alteration(s) to increase isoelectric point (pI), described above. As described above, an antigen-binding molecule or antibody with an increased pI is more strongly attracted by physicochemical Coulomb interaction to the endothelial cell surface that has a net negative charge compared to an antigen-binding molecule or antibody not having an increased pI. Therefore, for those anti-CD137 agonistic antigen-binding molecules or antibodies that show CD137 agonistic activity based on the contribution by binding activity toward Fc-gamma receptor (preferably Fc-gamma RIIb), agonistic activity of the anti-CD137 antigen-binding molecules or antibodies can be increased by combining amino acid alteration(s) to increase Fc-gamma receptor (preferably Fc-gamma RIIb) and amino acid alteration(s) to increase pI.

In one aspect, the present disclosure provides polypeptides comprising a variant Fc region with an increased binding activity towards Fc-gamma RIIb and with an increased pI, which comprises at least three amino acid alterations including (a) at least one amino acid alteration of at least one position selected from the group consisting of positions 234, 235, 236, 237, 238, 264, 268, 295, 326, and 330, according to EU numbering, and (b) at least two amino acid alterations of at least two positions selected from the group consisting of positions 311, 343, and 413, according to EU numbering.

In another aspect, the present disclosure provides polypeptides comprising a variant Fc region with an increased binding activity towards Fc-gamma RIIb and an increased pI, which comprises amino acid alterations of any one of following (1) to (26):

(1) positions 235, 236, 268, 295, 326, 330, 343, and 413;
(2) positions 214, 235, 236, 268, 295, 326, 330, 343, and 413;
(3) positions 234, 238, 250, 264, 307, 330, 343, and 413;
(4) positions 234, 238, 264, 330, 343, and 413;
(5) positions 234, 237, 238, 250, 307, 330, 343, and 413;
(6) positions 234, 237, 238, 330, 343, and 413;
(7) positions 235, 236, 268, 295, 326, 330, 311, and 343;
(8) positions 234, 238, 250, 264, 307, 330, 311, and 343;
(9) positions 234, 238, 264, 330, 311, and 343;
(10) positions 234, 237, 238, 250, 307, 330, 311, and 343;
(11) positions 234, 237, 238, 330, 311, and 343;
(12) positions 235, 236, 268, 295, 326, 330, and 343;
(13) positions 214, 235, 236, 268, 295, 326, 330, and 343;
(14) positions 235, 236, 268, 295, 326, 330, and 413;
(15) positions 214, 236, 268, 330, and 343;
(16) positions 214, 235, 236, 268, 330, and 343;
(17) positions 214, 236, 268, 330, and 413;
(18) positions 214, 236, 268, 330, 343, and 413;
(19) positions 214, 235, 236, 268, 330, 343, and 413;
(20) positions 214, 236, 268, 330, and 311;
(21) positions 214, 235, 236, 268, 330, and 311;
(22) positions 214, 236, 268, 330, 311, and 343;
(23) positions 214, 235, 236, 268, 330, 311, and 343;
(24) positions 214, 236, 268, 330, 311, and 413;
(25) positions 214, 235, 236, 268, 330, 311, and 413;
(26) positions 214, 235, 236, 268, 295, 326, 330, and 311,

according to EU numbering.

In one embodiment, the variant Fc regions of the present disclosure comprise any one combination of amino acid alterations identified in Table 4 below.

[Table 4]

| Number | Amino acid substitutions (EU numbering) |
|---|---|
| 1 | L235W/G236N/H268D/Q295L/K326T/A330K/P343R/D413K |
| 2 | K214R/L235W/G236N/H268D/Q295L/K326T/A330K/P343R/D413K |
| 3 | L234Y/P238D/T250V/V264I/T307P/A330K/P343R/D413K |
| 4 | L234Y/P238D/V264I/A330K/P343R/D413K |
| 5 | L234Y/G237D/P238D/T250V/T307P/A330K/P343R/D413K |
| 6 | L234Y/G237D/P238D/A330K/P343R/D413K |
| 7 | L235W/G236N/H268D/Q295L/K326T/A330K/Q311R/P343R |
| 8 | L234Y/P238D/T250V/V264I/T307P/A330K/Q311R/P343R |
| 9 | L234Y/P238D/V264I/A330K/Q311R/P343R |
| 10 | L234Y/G237D/P238D/T250V/T307P/A330K/Q311R/P343R |
| 11 | L234Y/G237D/P238D/A330K/Q311R/P343R |
| 12 | L235W/G236N/H268D/Q295L/K326T/A330K/P343R |
| 13 | K214R/L235W/G236N/H268D/Q295L/K326T/A330K/P343R |

(continued)

| Number | Amino acid substitutions (EU numbering) |
|--------|------------------------------------------|
| 14 | L235W/G236N/H268D/Q295L/K326T/A330K/D413K |
| 15 | K214R/G236N/H268D/A330K/P343R |
| 16 | K214R/L235W/G236N/H268D/A330K/P343R |
| 17 | K214R/G236N/H268D/A330K/D413K |
| 18 | K214R/G236N/H268D/A330K/P343R/D413K |
| 19 | K214R/L235W/G236N/H268D/A330K/P343R/D413K |
| 20 | K214R/G236N/H268D/A330K/Q311R |
| 21 | K214R/L235W/G236N/H268D/A330K/Q311R |
| 22 | K214R/G236N/H268D/A330K/Q311R/P343R |
| 23 | K214R/L235W/G236N/H268D/A330K/Q311R/P343R |
| 24 | K214R/G236N/H268D/A330K/Q311R/D413K |
| 25 | K214R/L235W/G236N/H268D/A330K/Q311R/D413K |
| 26 | K214R/L235W/G236N/H268D/Q295L/K326T/A330K/Q311R |

In one embodiment, the variant Fc regions comprising any one combination of amino acid alterations described in Table 4 above lacks the amino acid at position 447 according to EU numbering. In a preferred embodiment, the variant Fc regions comprising any one combination of amino acid alterations described in Table 4 above lacks the amino acids at positions 446 and 447 according to EU numbering.

Those skilled in the art would understand that at least one amino acid alteration to increase binding activity towards Fc-gamma R (including Fc-gamma RIIb) as compared to the parent Fc region as described or suggested, for example, in WO2013/047752, WO2013/125667, WO2014/030728, WO2014/163101, or WO2017104783, and at least one amino acid alteration to increase pI as compared to the parent Fc region as described or suggested, for example, in WO2017/104783, WO2017/046994, and any combination of these amino acid alterations may be used, in addition to the alterations provided as illustrations above.

In addition, amino acid alterations performed for other purpose(s) can be combined in a variant Fc region described herein. For example, amino acid substitutions that increase FcRn-binding activity (Hinton et al., J. Immunol. 176(1): 346-356 (2006); Dall'Acqua et al., J. Biol. Chem. 281(33): 23514-23524 (2006); Petkova et al., Intl. Immunol. 18(12): 1759-1769 (2006); Zalevsky et al., Nat. Biotechnol. 28(2): 157-159 (2010); WO 2006/019447; WO 2006/053301; and WO 2009/086320), and amino acid substitutions for improving antibody heterogeneity or stability (WO 2009/041613) may be added. Alternatively, polypeptides with the property of promoting antigen clearance, which are described in WO 2011/122011, WO 2012/132067, WO 2013/046704 or WO 2013/180201, polypeptides with the property of specific binding to a target tissue, which are described in WO 2013/180200, polypeptides with the property for repeated binding to a plurality of antigen molecules, which are described in WO 2009/125825, WO 2012/073992 or WO 2013/047752, can be combined with a variant Fc region described herein. Alternatively, with the objective of conferring binding activity to other antigens, the amino acid alterations disclosed in EP1752471 and EP1772465 may be combined in CH3 of a variant Fc region described herein.

In one embodiment, the anti-CD137 antigen-binding molecules or antibodies of the present disclosure comprise a heavy chain constant region comprising any one amino acid sequence selected from SEQ ID NOs: 64-85. Preferably, the anti-CD137 antigen-binding molecules or antibodies of the present disclosure comprise a heavy chain constant region comprising the amino acid sequence of SEQ ID NO: 75 or 82.

In one preferred embodiment, the anti-CD 137 antigen-binding molecules or antibodies comprising the above-described variant Fc region has above-described CD137-binding activity dependent on a small molecule compound.

In one embodiment, the anti-CD137 antigen-binding molecules or antibodies of the present disclosure comprise the following variable region and constant region: a variable region comprising the above-described HVR, heavy chain variable region, and/or light chain variable region; and the above-described variant Fc region. In one preferred embodiment, the anti-CD137 antigen-binding molecules or antibodies of the present disclosure may be any one anti-CD137 antibody selected from the antibodies described in Table 72.

In a further aspect, the present disclosure provides antigen-binding molecules or antibodies binding to the same epitope on CD137 as the anti-CD137 antigen-binding molecules or antibodies provided herein, in the presence of a low-molecular weight compound (e.g., in the presence of 10 micromolar or more, 50 micromolar or more, 100 micromolar or more, 150 micromolar or more, 200 micromolar or more, or 250 micromolar or more of the low-molecular weight compound). For example, in a certain embodiment, those antigen-binding molecules or antibodies are provided that bind to the same epitope with the anti-CD137 antigen-binding molecules or antibodies comprising A375/B167, A372/B040, A356/B040, A486/B167, A487/B167, A488/B226, A489/B223, A548/B376, A551/B256, A551/B379, A555/B379, A548/B256, and/or A549/B167 described in Table 37, as a combination of heavy chain variable region/light chain variable region. In one embodiment, the anti-CD137 antigen-binding molecules or antibodies of the present disclosure having CD137 binding activity that is dependent on antigen-binding activity dependent on a small molecule compound recognize an epitope formed by a complex formed from the antigen (e.g., CD137) and the low-molecular weight compound (e.g., ATP).

In a further aspect, the present disclosure provides antigen-binding molecules or antibodies that compete for the binding to CD137 with the anti-CD 137 antigen-binding molecules or antibodies provided herein, in the presence of a low-molecular weight compound (e.g., in the presence of 10 micromolar or more, 50 micromolar or more, 100 micromolar or more, 150 micromolar or more, 200 micromolar or more, or 250 micromolar or more of the low-molecular weight compound). For example, in a certain embodiment, these compete for the site of binding to CD137 with the anti-CD 137 antigen-binding molecules or antibodies comprising A375/B167, A372/B040, A356/B040, A486/B167, A487/B167, A488/B226, A489/B223, A548/B376, A551/B256, A551/B379, A555/B379, A548/B256, and/or A549/B167 described in Table 37, as a combination of heavy chain variable region/light chain variable region.

In a further aspect of the present disclosure, an anti-CD 137 antigen-binding molecule or antibody according to any of the above embodiments is a monoclonal antibody, including a chimeric, humanized or human antibody. In one embodiment, an anti-CD 137 antibody is an antibody fragment, e.g., a Fv, Fab, Fab', scFv, diabody, or $F(ab')_2$ fragment. In another embodiment, the antibody is a full length antibody, e.g., an intact IgG1 antibody or other antibody class or isotype as defined herein.

In a further aspect, an anti-CD 137 antigen-binding molecule or antibody according to any of the above embodiments may incorporate any of the features, singly or in combination, as described in Sections 1-7 below:

## 1. Agonistic activity of anti-CD 137 antigen-binding molecule or antibody

In a specific embodiment, the anti-CD 137 antigen-binding molecule or antibody in the present disclosure has CD137 agonistic activity. CD137 signaling not only stimulates IFN-$\gamma$ secretion and proliferation of NK cells (Buechele et al., 2012; Lin et al., 2008; Melero et al., 1998), but also enhances their survival and DC activation indicated by upregulation of co-stimulatory molecules and cytokine secretion (Choi et al., 2009; Futagawa et al., 2002; Wilcox et al., 2002). However, CD137 is best characterized as a co-stimulatory molecule that regulates TCR-induced activation in both CD4+ and CD8+ subsets of T cells. In combination with TCR activation, anti-CD137 agonist antibodies enhance T cell proliferation, stimulate lymphokine secretion, and reduce the sensitivity of T lymphocytes to activation-induced cell death (reviewed in Snel et al., 2011). Of those phenomena, the physiological phenomena observed after CD137 signaling on T cells are mediated by downstream signals activated by CD137 signaling, such as TRAF2, TRAF1, in particular NF-kappaB, JNK, Erk, Akt, survivin, Bcl-XL, and/or Bcl-2 (Ward-Kavanagh et al., Immunity, 44: 1005 (2016)).

In one embodiment, the "anti-CD 137 agonistic antigen-binding molecule" or "anti-CD 137 agonistic antibody" is an antigen-binding molecule or antibody that, by binding to CD137, transduces the CD137 signal, and significantly induces or enhances IFN-gamma secretion, proliferation, and increased survival of NK cells; DC activation indicated by up-regulation of cytokine secretion and co-stimulatory molecules; TCR induction; T cell proliferation; and/or lymphokine secretion. In a different embodiment, the "anti-CD 137 agonistic antigen-binding molecule" or "anti-CD 137 agonistic antibody" is an antigen-binding molecule or antibody that transduces the CD137 signal by binding to CD137 on T cells, and significantly induces activation of NF-kappaB of the T cells. Further, the antigen-binding molecule or an antibody "shows CD137 agonistic activity" means that any of the above-mentioned physiological phenomena is observed when the antigen-binding molecule or antibody binds to CD137. The method of measuring CD137 agonistic activity is described in detail in the section of "C. Assays" below.

In a specific embodiment, the anti-CD 137 antigen-binding molecule or antibody in the present disclosure has small molecule compound-dependent CD137 agonistic activity. In one non-limiting embodiment, the CD137 agonistic activity of the anti-CD137 antigen binding molecule or antibody against CD137 in the presence of a small molecule compound is higher than the CD137 agonistic activity in the absence of the small molecule compound. In a different embodiment, the CD137 agonistic activity of the anti-CD137 antigen binding molecule or antibody in the presence of a high concentration of a small molecule compound is higher compared to the CD137 agonistic activity in the presence of a low concentration of the small molecule compound. In a further embodiment, the CD137 agonistic activity of the anti-CD137 antigen binding molecule or antibody in the presence of a small molecule compound is 2-fold or more, 3-fold or more, 5-fold or more, 10-fold or more, 20-fold or more, 30-fold or more, 50-fold or more, 100-fold or more, 200-fold or more, 300-fold or more, 500-fold or more, $1 \times 10^3$-fold or more, $2 \times 10^3$-fold or more, $3 \times 10^3$-fold or more, $5 \times$

$10^3$-fold or more, $1 \times 10^4$-fold or more, $2 \times 10^4$-fold or more, $3 \times 10^4$-fold or more, $5 \times 10^4$-fold or more, or $1 \times 10^5$-fold or more, as compared to the CD137 agonistic activity in the absence of the small molecule compound.

Any suitable concentration may be selected as the concentration of the small molecule compound as long as a difference in the binding activity of the anti-CD 137 antigen-binding molecule or antibody is detected. In one embodiment, the anti-CD 137 antigen-binding molecule or antibody transduces the CD137 signal by binding to CD137 on the cell surface. Therefore, one skilled in the art would understand that the anti-CD137 antigen-binding molecule or antibody that has small molecule compound-dependent CD137 binding activity has CD137 agonistic activity dependent on the small molecule compound. However, on the other hand, since the methods for measuring binding activity and agonistic activity are different, one skilled in the art would understand that the concentration of a small molecule compound for which a difference in binding activity is detected can be different from the concentration of the small molecule compound for which a difference in agonistic activity is detected (e.g., for an anti-CD137 antigen-binding molecule or antibody whose CD137 binding activity in the presence of a small molecule compound at 10 $\mu$M is 2-fold or more compared to the CD137 binding activity in the absence of the small molecule compound, the CD137 agonistic activity (assay value) in the presence of the small molecule compound at 10 $\mu$M can be less than 2-fold compared to the CD137 agonistic activity (assay value) in the absence of the small molecule compound). Furthermore, it would be understood by those skilled in the art that the determination of agonistic activity can vary depending on the assay of CD137 agonistic activity (refer to "C. Assays").

In one embodiment, the anti-CD137 antigen-binding molecule or antibody (i) shows agonistic activity towards CD137 in the presence of a small molecule compound at 10 $\mu$M, 50 $\mu$M, 100 $\mu$M, 150 $\mu$M, 200 $\mu$M, or 250 $\mu$M, and (ii) shows substantially no agonistic activity towards CD137 in the absence of the small molecule compound, or has low agonistic activity towards CD137 in the absence of the small molecule compound (compared to the presence of the small molecule compound).

In one embodiment, when the agonistic activity of the anti-CD137 antigen-binding molecule or antibody is evaluated by "a) Agonistic activity assay (PBMC)" explained in detail in "C. Assays", the anti-CD 137 antigen-binding molecule or antibody (i) exhibits an agonistic activity towards CD137 in the presence of a small molecule compound at 250 $\mu$M, and (ii) has a low agonistic activity towards CD137 in the absence of the small molecule compound (compared to the presence of the small molecule compound). In a further embodiment, the anti-CD137 antigen-binding molecule or antibody (i) shows an agonistic activity towards CD137 in the presence of a small molecule compound at 250$\mu$M, and (ii) shows substantially no agonistic activity towards CD137 in the absence of the small molecule compound.

In one embodiment, when the agonistic activity of the anti-CD137 antigen-binding molecule or antibody is evaluated by "b) Agonistic activity assay (reporter gene assay)" explained in detail in "C. Assays", the anti-CD 137 antigen-binding molecule or antibody (i) exhibits agonistic activity towards CD137 in the presence of 10 $\mu$M, 50 $\mu$M, 100 $\mu$M, 150 $\mu$M, 200 $\mu$M, or 250 $\mu$M of a small molecule compound, and (ii) has substantially no agonistic activity towards CD137 or has a lower agonistic activity in the absence of the small molecule compound (as compared to that in the presence of the small molecule compound). Antibody concentrations in the reporter gene assay may be arbitrarily selected, for example, the final concentration of antibody is 0, 0.001, 0.01, 0.1, 1, or 10 $\mu$g/mL. In a preferred embodiment, the final concentration of antibody is 0.1 $\mu$g/mL or 1 $\mu$g/mL.

In one embodiment, when the final concentration of antibody is 0.1 $\mu$g/mL in "b) Agonistic activity assay (reporter gene assay)" explained in detail in "C. Assays", (i) the CD137 agonistic activity (relative light unit) of the anti-CD 137 antigen-binding molecule or antibody in the presence 10 $\mu$M of a small molecule compound is 2-fold or more, 3-fold or more, 5-fold or more, 10-fold or more, 20-fold or more, 30-fold or more, 50-fold or more, 60-fold or more, 70-fold or more, 80-fold or more, or 90-fold or more higher, as compared to (ii) the CD137 agonistic activity (relative light unit) in the absence of the small molecule compound. In one embodiment, when the final concentration of antibody is 0.1 $\mu$g/mL in "b) Agonistic activity assay (reporter gene assay)" explained in detail in "C. Assays", (i) the CD137 agonistic activity (relative light unit) of the anti-CD 137 antigen-binding molecule or antibody in the presence 100 $\mu$M of a small molecule compound is 2-fold or more, 3-fold or more, 5-fold or more, 10-fold or more, 20-fold or more, 30-fold or more, 50-fold or more, 60-fold or more, 70-fold or more, 80-fold or more, or 90-fold or more higher, as compared to (ii) the CD137 agonistic activity (relative light unit) in the absence of the small molecule compound. In one embodiment, when the final concentration of antibody is 0.1 $\mu$g/mL in "b) Agonistic activity assay (reporter gene assay)" explained in detail in "C. Assays", (i) the CD137 agonistic activity (relative light unit) of the anti-CD137 antigen-binding molecule or antibody in the presence 250 $\mu$M of a small molecule compound is 2-fold or more, 3-fold or more, 5-fold or more, 10-fold or more, 20-fold or more, 30-fold or more, 50-fold or more, 60-fold or more, 70-fold or more, 80-fold or more, or 90-fold or more higher, as compared to (ii) the CD137 agonistic activity (relative light unit) in the absence of the small molecule compound. In any of the above embodiments, further, 0.1 $\mu$g/mL of an anti-CD137 antigen-binding molecule or antibody exhibits substantially no CD137 agonistic activity in the absence of the small molecule compound.

In one embodiment, when the final concentration of antibody is 1 $\mu$g/mL in "b) Agonistic activity assay (reporter gene assay)" explained in detail in "C. Assays", (i) the CD137 agonistic activity (relative light unit) of the anti-CD137 antigen-binding molecule or antibody in the presence 10 $\mu$M of a small molecule compound is 2-fold or more, 3-fold or more, 5-fold or more, 10-fold or more, 20-fold or more, 30-fold or more, 50-fold or more, 60-fold or more, 70-fold or more, 80-fold or more, or 90-fold or more higher, as compared to (ii) the CD137 agonistic activity (relative light unit) in the absence of the small molecule compound. In one embodiment, when the final concentration of antibody is 0.1 $\mu$g/mL in "b) Agonistic activity assay (reporter gene assay)" explained in detail in "Assays" (i) the CD137 agonistic activity (relative light unit) of the anti-CD 137 antigen-binding molecule or antibody in the presence 100 $\mu$M of a small molecule compound is 2-fold or more, 3-fold or more, 5-fold or more, 10-fold or more, 20-fold or more, 30-fold or more, 50-fold or more, 60-fold or more, 70-fold or more, 80-fold or more, or 90-fold or more higher, as compared to (ii) the CD137 agonistic activity (relative light unit) in the absence of the small molecule compound. In one embodiment, when the final concentration of antibody is 0.1 $\mu$g/mL in "b) Agonistic activity assay (reporter gene assay)" explained in detail in "C. Assays", (i) the CD137 agonistic activity (relative light unit) of the anti-CD137 antigen-binding molecule or antibody in the presence 250 $\mu$M of a small molecule compound is 2-fold or more, 3-fold or more, 5-fold or more, 10-fold or more, 20-fold or more, 30-fold or more, 50-fold or more, 60-fold or more, 70-fold or more, 80-fold or more, or 90-fold or more higher, as compared to (ii) the CD137 agonistic activity (relative light unit) in the absence of the small molecule compound. In any of the above embodiments, further, 1 $\mu$g/mL of an anti-CD137 antigen-binding molecule or antibody exhibits substantially no CD137 agonistic activity in the absence of the small molecule compound.

## 2. Antibody Fragments

In certain embodiments, an antibody provided herein is an antibody fragment. Antibody fragments include, but are not limited to, Fab, Fab', Fab'-SH, F(ab')$_2$, Fv, and scFv fragments, and other fragments described below. For a review of certain antibody fragments, see Hudson et al. Nat. Med. 9:129-134 (2003). For a review of scFv fragments, see, e.g., Pluckthun, in The Pharmacology of Monoclonal Antibodies, vol. 113, Rosenburg and Moore eds., (Springer-Verlag, New York), pp. 269-315 (1994); see also WO 93/16185; and U.S. Patent Nos. 5,571,894 and 5,587,458. For discussion of Fab and F(ab')$_2$ fragments comprising salvage receptor binding epitope residues and having increased in vivo half-life, see U.S. Patent No. 5,869,046.

Diabodies are antibody fragments with two antigen-binding sites that may be bivalent or bispecific. See, for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; *see,* e.g., U.S. Patent No. 6,248,516 B1).

Antibody fragments can be made by various techniques, including but not limited to proteolytic digestion of an intact antibody as well as production by recombinant host cells (e.g. *E. coli* or phage), as described herein.

## 3. Chimeric and Humanized Antibodies

In certain embodiments, an antibody provided herein is a chimeric antibody. Certain chimeric antibodies are described, e.g., in U.S. Patent No. 4,816,567; and Morrison et al., Proc. Natl. Acad. Sci. USA, 81:6851-6855 (1984)). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro and Fransson, Front. Biosci. 13:1619-1633 (2008), and are further described, e.g., in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing specificity determining region (SDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, e.g., Sims et al. J. Immunol. 151:2296 (1993)); framework regions derived from the consensus sequence of human antibodies of a particular subgroup of light or heavy chain variable regions (see, e.g., Carter et al. Proc. Natl. Acad. Sci. USA, 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

#### 4. Human Antibodies

In certain embodiments, an antibody provided herein is a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). See also, e.g., U.S. Patent Nos. 6,075,181 and 6,150,584 describing XENOMOUSE™ technology; U.S. Patent No. 5,770,429 describing HuMab (registered trademark) technology; U.S. Patent No. 7,041,870 describing K-M MOUSE (registered trademark) technology, and U.S. Patent Application Publication No. US 2007/0061900, describing VelociMouse (registered trademark) technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. (See, e.g., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

#### 5. Library-Derived Antibodies

Antibodies of the present disclosure may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom et al. in Methods in Molecular Biology 178:1-37 (O'Brien et al., ed., Human Press, Totowa, NJ, 2001) and further described, e.g., in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement in vitro, as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

The antigen-binding molecules or antibodies having antigen-binding activity dependent on a low-molecular weight compound of the present disclosure may be selected by conducting screening over a library of antigen-binding molecules. As such library, the above-described combinatorial libraries may be used. The library of antigen-binding molecules may be with unbiased repertoire of antigen-binding molecules (naive library) or may be with biased repertoire of antigen-binding molecules. Examples of the latter type of library include a library of antigen-binding molecules to which binding activity towards a specified compound is conferred in advance. In a certain embodiment, an antigen-binding molecule library is a library of antigen-binding molecules to which amino acid alteration(s) to confer binding activity towards a specified compound is introduced in advance. Examples of such type of library include libraries described, for example, in the International Publication, WO 2015/083764.

6. Multispecific Antibodies

In certain embodiments, an antibody provided herein is a multispecific antibody, e.g. a bispecific antibody. Multispecific antibodies are monoclonal antibodies that have binding specificities for at least two different sites. In certain embodiments, one of the binding specificities is for CD137 and the other is for any other antigen. In certain embodiments, bispecific antibodies may bind to two different epitopes of CD137. Bispecific antibodies may also be used to localize cytotoxic agents to cells which express CD137. Bispecific antibodies can be prepared as full length antibodies or antibody fragments.

In one embodiment, the anti-CD137 antigen-binding molecules or antibodies of the present disclosure are bi-specific antibodies, one arm of them having CD137 binding activity dependent on a small molecule compound and another arm of them binding to an antigen different from CD137. The "antigen" different from CD137 is not particularly limited in the structure. In other words, the antigen can be inorganic or organic substances. Exemplary antigens are disclosed below. In one embodiment, the antigens are preferably antigens expressed in cancer cells, immune cells, stroma cells, or such in cancer tissues or inflammatory tissues.

Herein, "antigens" are not particularly limited in their structure, as long as they comprise epitopes to which antigen-binding molecules of the present invention bind. Antigens can be inorganic or organic substances. In some embodiments, examples of antigens include: 17-IA, 4-1BB, 4Dc, 6-keto-PGFla, 8-iso-PGF2a, 8-oxo-dG, A1 adenosine receptor, A33, ACE, ACE-2, activin, activin A, activin AB, activin B, activin C, activin RIA, activin RIA ALK-2, activin RIB ALK-4, activin RIIA, activin RIIB, ADAM, ADAM10, ADAM12, ADAM15, ADAM 17/TACE, ADAM8, ADAM9, ADAMTS, ADAMTS4, ADAMTS5, addressin, aFGF, ALCAM, ALK, ALK-1, ALK-7, alpha-1-antitrypsin, alpha-V/beta-1 antagonist, ANG, Ang, APAF-1, APE, APJ, APP, APRIL, AR, ARC, ART, artemin, anti-Id, ASPARTIC, atrial natriuretic peptide, av/b3 integrin, Axl, b2M, B7-1, B7-2, B7-H, B-lymphocyte stimulating factor (BlyS), BACE, BACE-1, Bad, BAFF, BAFF-R, Bag-1, BAK, Bax, BCA-1, BCAM, Bcl, BCMA, BDNF, b-ECGF, bFGF, BID, Bik, BIM, BLC, BL-CAM, BLK, BMP, BMP-2 BMP-2a, BMP-3 Osteogenin, BMP-4 BMP-2b, BMP-5, BMP-6 Vgr-1, BMP-7 (OP-1), BMP-8 (BMP-8a, OP-2), BMPR, BMPR-IA (ALK-3), BMPR-IB (ALK-6), BRK-2, RPK-1, BMPR-II (BRK-3), BMP, b-NGF, BOK, bombesin, bone-derived neurotrophic factor, BPDE, BPDE-DNA, BTC, complement factor 3 (C3), C3a, C4, C5, C5a, C10, CA125, CAD-8, calcitonin, cAMP, carcinoembryonic antigen (CEA), cancer associated antigen, cathepsin A, cathepsin B, cathepsin C/DPPI, cathepsin D, cathepsin E, cathepsin H, cathepsin L, cathepsin O, cathepsin S, cathepsin V, cathepsin X/Z/P, CBL, CCI, CCK2, CCL, CCL1, CCL11, CCL12, CCL13, CCL14, CCL15, CCL16, CCL17, CCL18, CCL19, CCL2, CCL20, CCL21, CCL22, CCL23, CCL24, CCL25, CCL26, CCL27, CCL28, CCL3, CCL4, CCL5, CCL6, CCL7, CCL8, CCL9/10, CCR, CCR1, CCR10, CCR10, CCR2, CCR3, CCR4, CCR5, CCR6, CCR7, CCR8, CCR9, CD1, CD2, CD3, CD3E, CD4, CD5, CD6, CD7, CD8, CD10, CD11a, CD11b, CD11c, CD13, CD14, CD15, CD16, CD18, CD19, CD20, CD21, CD22, CD23, CD25, CD27L, CD28, CD29, CD30, CD30L, CD32, CD33 (p67 protein), CD34, CD38, CD40, CD40L, CD44, CD45, CD46, CD49a, CD52, CD54, CD55, CD56, CD61, CD64, CD66e, CD74, CD80 (B7-1), CD89, CD95, CD123, CD137, CD138, CD140a, CD146, CD147, CD148, CD152, CD164, CEACAM5, CFTR, cGMP, CINC, Botulinum toxin, Clostridium perfringens toxin, CKb8-1, Claudin-6, CLC, CMV, CMV UL, CNTF, CNTN-1, COX, C-Ret, CRG-2, CT-1, CTACK, CTGF, CTLA-4, PD1, PDL1, LAG3, TIM3, galectin-9, CX3CL1, CX3CR1, CXCL, CXCL1, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16, CXCR, CXCR1, CXCR2, CXCR3, CXCR4, CXCR5, CXCR6, cytokeratin tumor associated antigen, DAN, DCC, DcR3, DC-SIGN, complement regulatory factor (Decay accelerating factor), des (1-3)-IGF-I (brain IGF-1), Dhh, digoxin, DLL3, DNAM-1, Dnase, Dpp, DPPIV/CD26, Dtk, ECAD, EDA, EDA-A1, EDA-A2, EDAR, EGF, EGFR (ErbB-1), EMA, EMMPRIN, ENA, endothelin receptor, enkephalinase, eNOS, Eot, eotaxin 1, EpCAM, ephrin B2/EphB4, EPO, ERCC, E-selectin, ET-1, factor IIa, factor VII, factor VIIIc, factor IX, fibroblast activation protein (FAP), Fas, FcR1, FEN-1, ferritin, FGF, FGF-19, FGF-2, FGF-3, FGF-8, FGFR, FGFR-3, fibrin, FL, FLIP, Flt-3, Flt-4, follicle stimulating hormone, fractalkine, FZD1, FZD2, FZD3, FZD4, FZD5, FZD6, FZD7, FZD8, FZD9, FZD10, G250, Gas6, GCP-2, GCSF, GD2, GD3, GDF, GDF-1, GDF-3 (Vgr-2), GDF-5 (BMP-14, CDMP-1), GDF-6 (BMP-13, CDMP-2), GDF-7 (BMP-12, CDMP-3), GDF-8 (myostatin), GDF-9, GDF-15 (MIC-1), GDNF, GDNF, GFAP, GFRa-1, GFR-alpha1, GFR-alpha2, GFR-alpha3, GITR, glucagon, Glut4, glycoprotein IIb/IIIa (GPIIb/IIIa), GM-CSF, gp130, gp72, GRO, growth hormone releasing hormone, hapten (NP-cap or NIP-cap), HB-EGF, HCC, HCMV gB envelope glycoprotein, HCMV gH envelope glycoprotein, HCMV UL, hematopoietic growth factor (HGF), Hep B gp120, heparanase, Her2, Her2/neu (ErbB-2), Her3 (ErbB-3), Her4 (ErbB-4), herpes simplex virus (HSV) gB glycoprotein, HSV gD glycoprotein, HGFA, high molecular weight melanoma-associated antigen (HMW-MAA), HIV gp120, HIV IIIB gp 120 V3 loop, HLA, HLA-DR, HM1.24, HMFG PEM, HRG, Hrk, human cardiac myosin, human cytomegalovirus (HCMV), human growth hormone (HGH), HVEM, 1-309, IAP, ICAM, ICAM-1, ICAM-3, ICE, ICOS, IFNg, Ig, IgA receptor, IgE, IGF, IGF binding protein, IGF-1R, IGFBP, IGF-I, IGF-II, IL, IL-1, IL-1R, IL-2, IL-2R, IL-4, IL-4R, IL-5, IL-5R, IL-6, IL-6R, IL-8, IL-9, IL-10, IL-12, IL-13, IL-15, IL-18, IL-18R, IL-21, IL-23, IL-27, interferon (INF)-alpha, INF-beta, INF-gamma, inhibin, iNOS, insulin A chain, insulin B chain, insulin-like growth factor1, integrin alpha2, integrin alpha3, integrin alpha4, integrin alpha4/beta1, integrin alpha4/beta7, integrin alpha5 (alpha V), integrin alpha5/beta1, integrin alpha5/beta3, integrin alpha6, integrin beta1, integrin beta2,interferon gamma, IP-10, I-TAC, JE, kallikrein 2, kallikrein 5, kallikrein 6, kallikrein 11, kallikrein 12, kallikrein 14, kallikrein 15, kallikrein L1, kallikrein L2, kallikrein L3, kallikrein L4, KC, KDR, keratinocyte growth factor (KGF), laminin 5, LAMP, LAP, LAP (TGF-1), latent TGF-1, latent TGF-1 bp1, LBP, LDGF, LECT2, lefty, Lewis-Y antigen, Lewis-Y associated antigen, LFA-1, LFA-3, Lfo, LIF, LIGHT, lipoprotein, LIX, LKN, Lptn, L-selectin, LT-a, LT-b, LTB4, LTBP-1, lung surface, luteinizing hormone, lymphotoxin beta receptor, Mac-1, MAdCAM, MAG, MAP2, MARC, MCAM, MCAM, MCK-2, MCP, M-CSF, MDC, Mer, METALLOPROTEASES, MGDF receptor, MGMT, MHC (HLA-DR), MIF, MIG, MIP, MIP-1-alpha, MK, MMAC1, MMP, MMP-1, MMP-10, MMP-11, MMP-12, MMP-13, MMP-14, MMP-15, MMP-2, MMP-24, MMP-3, MMP-7, MMP-8, MMP-9, MPIF, Mpo, MSK, MSP, mucin (Muc1), MUC18, Mullerian-inhibiting substance, Mug, MuSK, NAIP, NAP, NCAD, N-C adherin, NCA 90, NCAM, NCAM, neprilysin, neurotrophin-3, -4, or -6, neurturin, nerve growth factor (NGF), NGFR, NGF-beta, nNOS, NO, NOS, Npn, NRG-3, NT, NTN, OB, OGG1, OPG, OPN, OSM, OX40L, OX40R, p150, p95, PADPr, parathyroid hormone, PARC, PARP, PBR, PBSF, PCAD, P-cadherin, PCNA, PDGF, PDGF, PDK-1, PECAM, PEM, PF4, PGE, PGF, PGI2, PGJ2, PIN, PLA2, placental alkaline phosphatase (PLAP), PlGF, PLP, PP14, proinsulin, prorelaxin, protein C, PS, PSA, PSCA, prostate-specific membrane antigen (PSMA), PTEN, PTHrp, Ptk, PTN, R51, RANK, RANKL, RANTES, RANTES, relaxin A chain, relaxin B chain, renin, respiratory syncytial virus (RSV) F, RSV Fgp, Ret, Rheumatoid factor, RLIP76, RPA2, RSK, S100, SCF/KL, SDF-1, SERINE, serum albumin, sFRP-3, Shh, SIGIRR, SK-1, SLAM, SLPI, SMAC, SMDF, SMOH, SOD, SPARC, Stat, STEAP, STEAP-II, TACE, TACI, TAG-72 (tumor-associated glycoprotein-72), TARC, TCA-3, T-cell receptor (for example, T-cell receptor alpha/beta), TdT, TECK, TEM1, TEM5, TEM7, TEM8, TERT, testis PLAP-like alkaline phosphatase, TfR, TGF, TGF-alpha, TGF-beta, TGF-beta Pan Specific, TGF-betaRI (ALK-5), TGF-betaRII, TGF-betaRIIb, TGF-betaRIII, TGF-beta1, TGF-beta2, TGF-beta3, TGF-beta4, TGF-beta5, thrombin, thymus Ck-1, thyroid-stimulating hormone, Tie, TIMP, TIQ, tissue factor, TMEFF2, Tmpo, TMPRSS2, TNF, TNF-alpha, TNF-alphabeta, TNF-beta2, TNFc, TNF-RI, TNF-RII, TNFRSF10A (TRAIL R1 Apo-2, DR4), TNFRSF10B (TRAIL R2 DR5, KILLER, TRICK-2A, TRICK-B), TNFRSF10C (TRAIL R3 DcR1, LIT, TRID), TNFRSF10D (TRAIL R4 DcR2, TRUNDD), TNFRSF11A (RANK ODF R, TRANCE R), TNFRSF11B (OPG OCIF, TR1), TNFRSF12 (TWEAK R FN14), TNFRSF13B (TACI), TNFRSF13C (BAFF R), TNFRSF14 (HVEM ATAR, HveA, LIGHT R, TR2), TNFRSF16 (NGFR p75NTR), TNFRSF17 (BCMA), TNFRSF18 (GITR AITR), TNFRSF19 (TROY TAJ, TRADE), TNFRSF19L (RELT), TNFRSF1A (TNF RI CD120a, p55-60), TNFRSF1B (TNF RII CD120b, p75-80), TNFRSF26 (TNFRH3), TNFRSF3 (LTbR TNF RIII, TNFC R), TNFRSF4 (OX40 ACT35, TXGP1 R), TNFRSF5 (CD40 p50), TNFRSF6 (Fas Apo-1, APT1, CD95), TNFRSF6B (DcR3 M68, TR6), TNFRSF7 (CD27), TNFRSF8 (CD30), TNFRSF9 (4-1BB CD137, ILA), TNFRSF21 (DR6), TNFRSF22 (DcTRAIL R2 TNFRH2), TNFRST23 (DcTRAIL R1 TNFRH1), TNFRSF25 (DR3 Apo-3, LARD, TR-3, TRAMP, WSL-1), TNFSF10 (TRAIL Apo-2 ligand, TL2), TNFSF11 (TRANCE/RANK ligand ODF, OPG ligand), TNFSF12 (TWEAK Apo-3 ligand, DR3 ligand), TNFSF13 (APRIL TALL2), TNFSF13B (BAFF BLYS, TALL1, THANK, TNFSF20), TNFSF14 (LIGHT HVEM ligand, LTg), TNFSF15 (TL1A/VEGI), TNFSF18 (GITR ligand AITR ligand, TL6), TNFSF1A (TNF-a Conectin, DIF, TNFSF2), TNFSF1B (TNF-b LTa, TNFSF1), TNFSF3 (LTb TNFC, p33), TNFSF4 (OX40 ligand gp34, TXGP1), TNFSF5 (CD40 ligand CD154, gp39, HIGM1, IMD3, TRAP), TNFSF6 (Fas ligand Apo-1 ligand, APT1 ligand), TNFSF7 (CD27 ligand CD70), TNFSF8 (CD30 ligand CD153), TNFSF9 (4-1BB ligand CD137 ligand), TP-1, t-PA, Tpo, TRAIL, TRAIL R, TRAIL-R1, TRAIL-R2, TRANCE, transferrin receptor, TRF, Trk, TROP-2, TLR1 (Toll-like receptor 1), TLR2, TLR3, TLR4, TLR5, TLR6, TLR7, TLR8, TLR9, TLR10, TSG, TSLP, tumor associated antigen CA125, tumor associated antigen expressing Lewis-Y associated carbohydrates, TWEAK, TXB2, Ung, uPAR, uPAR-1, urokinase, VCAM, VCAM-1, VECAD, VE-Cadherin, VE-cadherin-2, VEFGR-1 (flt-1), VEGF, VEGFR, VEGFR-3 (flt-4), VEGI, VIM, virus antigen, VLA, VLA-1, VLA-4, VNR integrin, von Willebrand factor, WIF-1, WNT1, WNT2, WNT2B/13, WNT3, WNT3A, WNT4, WNT5A, WNT5B, WNT6, WNT7A, WNT7B, WNT8A, WNT8B, WNT9A, WNT9B, WNT10A, WNT10B, WNT11, WNT16, XCL1, XCL2, XCR1, XEDAR, XIAP, XPD, HMGB1, IgA, A beta, CD81, CD97, CD98, DDR1, DKK1, EREG, Hsp90, IL-17/IL-17R, IL-20/IL-20R, oxidized LDL, PCSK9, prekallikrein, RON, TMEM16F, SOD1, Chromogranin A, Chromogranin B, tau, VAP1, high molecular weight kininogen, IL-31, IL-31R, Nav1.1, Nav1.2, Nav1.3, Nav1.4, Nav1.5, Nav1.6, Nav1.7, Nav1.8, Nav1.9, EPCR, C1, C1q, C1r, C1s, C2, C2a, C2b, C3, C3a, C3b, C4, C4a, C4b, C5, C5a, C5b, C6, C7, C8, C9, factor B, factor D, factor H, properdin, sclerostin, fibrinogen, fibrin, prothrombin, thrombin, tissue factor, factor V, factor Va, factor VII, factor VIIa, factor VIII, factor VIIIa, factor IX, factor IXa, factor X, factor Xa, factor XI, factor XIa, factor XII, factor XIIa, factor XIII, factor XIIIa, TFPI, antithrombin III, EPCR, thrombomodulin, TAPI, tPA, plasminogen, plasmin, PAI-1, PAI-2, GPC3, Syndecan-1, Syndecan-2, Syndecan-3, Syndecan-4, LPA, and SIP. In some embodiments, examples of antigens include receptors for hormone and growth factors. In certain embodiments, antigens are those that are expressed in or secreted from cells present in tumor tissues (e.g., tumor cells, immune cells, stromal cells, or such).

Techniques for making multispecific antibodies include, but are not limited to, recombinant co-expression of two immunoglobulin heavy chain-light chain pairs having different specificities (see Milstein and Cuello, *Nature* 305: 537 (1983)), WO 93/08829, and Traunecker et al., *EMBO J.* 10: 3655 (1991)), and "knob-in-hole" engineering (see, e.g., U.S. Patent No. 5,731,168). Multi-specific antibodies may also be made by engineering electrostatic steering effects for making antibody Fc-heterodimeric molecules (WO 2009/089004A1); crosslinking two or more antibodies or fragments (see, e.g., US Patent No. 4,676,980, and Brennan et al., Science, 229: 81 (1985)); using leucine zippers to produce bispecific antibodies (see, e.g., Kostelny et al., J. Immunol., 148(5):1547-1553 (1992)); using "diabody" technology for making bispecific antibody fragments (see, e.g., Hollinger et al., Proc. Natl. Acad. Sci. USA, 90:6444-6448 (1993)); and using single-chain Fv (scFv) dimers (see,e.g. Gruber et al., J. Immunol., 152:5368 (1994)); and preparing trispecific antibodies as described, e.g., in Tutt et al. J. Immunol. 147: 60 (1991).

Engineered antibodies with three or more functional antigen binding sites, including "Octopus antibodies," are also included herein (see, e.g. US 2006/0025576A1).

The antibody or fragment herein also includes a "Dual Acting Fab" or "DAF" comprising an antigen binding site that binds to CD137 as well as another, different antigen (see, US 2008/0069820, for example).

7. Antibody Variants

In certain embodiments, amino acid sequence variants of the antibodies provided herein are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding.

a) Substitution, Insertion, and Deletion Variants

In certain embodiments, antibody variants having one or more amino acid substitutions are provided. Sites of interest for substitutional mutagenesis include the HVRs and FRs. Conservative substitutions are shown in Table 5 under the heading of "preferred substitutions." More substantial changes are provided in Table 5 under the heading of "exemplary substitutions," and as further described below in reference to amino acid side chain classes. Amino acid substitutions may be introduced into an antibody of interest and the products screened for a desired activity, e.g., retained/improved antigen binding, decreased immunogenicity, or improved ADCC or CDC.

[Table 5]

| Original Residue | Exemplary Substitutions | Preferred Substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |

(continued)

| Original Residue | Exemplary Substitutions | Preferred Substitutions |
|---|---|---|
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:

(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

One type of substitutional variant involves substituting one or more hypervariable region residues of a parent antibody (e.g. a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g. binding affinity).

Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, Methods Mol. Biol. 207:179-196 (2008)), and/or residues that contact antigen, with the resulting variant VH or VL being tested for binding affinity. Affinity maturation by constructing and reselecting from secondary libraries has been described, e.g., in Hoogenboom et al. in Methods in Molecular Biology 178:1-37 (O'Brien et al., ed., Human Press, Totowa, NJ, (2001).) In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may, for example, be outside of antigen contacting residues in the HVRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as arg, asp, his, lys, and glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex may be analyzed to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion of an enzyme (e.g. for ADEPT) or a polypeptide which increases the plasma half-life of the antibody to the N- or C-terminus of the antibody.

b) Glycosylation variants

In certain embodiments, an antibody provided herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the present disclosure may be made in order to create antibody variants with certain improved properties.

In one embodiment, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (EU numbering of Fc region residues); however, Asn297 may also be located about +/- 3 amino acids upstream or downstream of position 297, i.e., between positions 294 and 300, due to minor sequence variations in antibodies. Such fucosylation variants may have improved ADCC function. See, e.g., US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams et al., especially at Example 11), and knockout cell lines, such as alpha-1,6-fucosyltransferase gene, *FUT8,* knockout CHO cells (see, e.g., Yamane-Ohnuki et al. Biotech. Bioeng. 87: 614 (2004); Kanda, Y. et al., Biotechnol. Bioeng., 94(4):680-688 (2006); and WO2003/085107).

Antibodies variants are further provided with bisected oligosaccharides, e.g., in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878 (Jean-Mairet et al.); US Patent No. 6,602,684 (Umana et al.); and US 2005/0123546 (Umana et al.). Antibody variants with at least one galactose residue in the oligosaccharide attached to the Fc region are also provided. Such antibody variants may have improved CDC function. Such antibody variants are described, e.g., in WO 1997/30087 (Patel et al.); WO 1998/58964 (Raju, S.); and WO 1999/22764 (Raju, S.).

c) Fc region variants

In certain embodiments, one or more amino acid modifications may be introduced into the Fc region of an antibody provided herein, thereby generating an Fc region variant (which may be also called "an altered Fc region").

The Fc region variant may comprise a human Fc region sequence (*e.g.*, a human IgG1, IgG2, IgG3 or IgG4 Fc region) comprising an amino acid alteration (*e.g.* a substitution) at one or more amino acid positions.

In certain embodiments, the present disclosure contemplates an antibody variant that possesses some but not all effector functions, which make it a desirable candidate for applications in which the half life of the antibody *in vivo* is important yet certain effector functions (such as complement and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks Fc gamma R binding (hence likely lacking ADCC activity), but retains FcRn binding activity. The primary cells for mediating ADCC, NK cells, express Fc gamma RIII only, whereas monocytes express Fc gamma RI, Fc gamma RII and Fc gamma RIII. FcR expression on hematopoietic cells is summarized in Table 3 on page 464 of Ravetch and Kinet, Annu. Rev. Immunol. 9:457-492 (1991). Non-limiting examples of *in vitro* assays to assess ADCC activity of a molecule of interest is described in U.S. Patent No. 5,500,362 (see, e.g. Hellstrom, I. et al. Proc. Nat'l Acad. Sci. USA 83:7059-7063 (1986)) and Hellstrom, I et al., Proc. Nat'l Acad. Sci. USA 82:1499-1502 (1985); 5,821,337 (see Bruggemann, M. et al., J. Exp. Med. 166:1351-1361 (1987)). Alternatively, non-radioactive assays methods may be employed (see, for example, ACT1™ non-radioactive cytotoxicity assay for flow cytometry (CellTechnology, Inc. Mountain View, CA; and CytoTox 96 (registered trademark) non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo, e.g.,* in a animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and *in vivo* clearance/half life determinations can also be performed using methods known in the art (see, e.g., Petkova, S.B. et al., Int'l. Immunol. 18(12):1759-1769 (2006)).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with increased or decreased binding to FcRs are described. (See, e.g., U.S. Patent No. 6,737,056; WO 2004/056312, and Shields et al., J. Biol. Chem. 9(2): 6591-6604 (2001).)

In certain embodiments, an antibody variant comprises an Fc region with one or more amino acid substitutions which improve ADCC, e.g., substitutions at positions 298, 333, and/or 334 of the Fc region (EU numbering of residues).

In some embodiments, alterations are made in the Fc region that result in altered (*i.e.,* either increased or decreased) C1q binding and/or Complement Dependent Cytotoxicity (CDC), e.g., as described in US Patent No. 6,194,551, WO 99/51642, and Idusogie et al. J. Immunol. 164: 4178-4184 (2000).

Antibodies with increased half lives and increased binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). Those antibodies comprise an Fc region with one or more substitutions therein which increase binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826).

See also Duncan & Winter, Nature 322:738-40 (1988); U.S. Patent No. 5,648,260; U.S. Patent No. 5,624,821; and WO 94/29351 concerning other examples of Fc region variants.

In one embodiment, binding activity towards each human Fc-gamma receptor (Fc-gamma R) of an antibody Fc region (including a variant Fc region (the same applies hereafter)) may be measured by a ligand-capturing method using, for example, BIACORE (registered trademark) T200, which rely upon surface plasmon resonance analysis methods as the measurement principle.

Details of an exemplary method of measuring binding activity of antibody Fc region towards various human Fc-gamma receptors (Fc-gamma Rs) are described below. In one embodiment, binding activity of an antibody Fc region towards Fc-gamma R is evaluated using BIACORE (registered trademark) T200. In a preferred embodiment, this measurement is carried out at 25 degrees C, using a measurement buffer 50 mM phosphate, 150 mM NaCl, 0.05 w/v%-P20, pH 7.4. Specifically, about 1000 RU of an antibody comprising a variant Fc region is first captured onto a sensor chip, with CaptureSelect (trademark) Human Fab-lambda Kinetics Biotin Conjugate (ThermoFisher scientific) immobilized as a ligand-capturing molecule. Human Fc-gamma Rs are diluted with the measurement buffer to 8 nM for Fc-gamma RIa and to 1000 nM for other Fc-gamma Rs, and are allowed to bind to the captured antibody. Binding activity of each antibody towards each Fc-gamma R is assessed by calculating amount of bound Fc-gamma R per unit amount of antibody (RU) using Biacore T200 Evaluation Software 2.0. In one embodiment, binding activity of an antibody Fc region towards various human Fc-gamma receptors (Fc-gamma Rs) may be measured by the method described in Reference Example 7-4.

In one preferred embodiment, the Fc-gamma Rs used for the above-described measurement method may be an extracellular domain of an Fc-gamma R prepared by the method described below. First, synthesis of a gene of an extracellular domain of an Fc-gamma R is carried out by a method known to those skilled in the art. For this synthesis, sequences of each Fc-gamma R is prepared based on information registered at the NCBI. More specifically, the sequence for Fc-gamma RI is prepared based on the sequence of NCBI accession # NM_000566.3, the sequence for Fc-gamma RIIa is prepared based on the sequence of NCBI accession # NM_001136219.1, the sequence for Fc-gamma RIIb is prepared based on the sequence of NCBI accession # NM_004001.3, and the sequence for Fc-gamma RIIIa is prepared based on the sequence of NCBI accession # NM_001127593.1, and His-tag is added to the C terminus. Polymorphic sites for Fc-gamma RIIa are prepared with reference to J. Exp. Med., 1990, 172, 19-25, and polymorphic sites for Fc-gamma RIIIa are prepared with reference to J. Clin. Invest., 1997, 100, 1059-1070. Obtained gene fragments are inserted into an expression vector for animal cells to prepare expression vectors. The prepared expression vectors are transiently introduced into FreeStyle293 cells (Invitrogen) derived from human embryonic kidney cancer cells and a protein of interest is allowed to be expressed. Culture supernatant is collected and filtered through 0.22 micro-meter filter, and then purified basically through the hereafter described four steps. As the first step, cation exchange column chromatography (SP Sepharose FF) is carried out; as the second step, affinity column chromatography to His tags (HisTrap HP); as the third step, gel filtration column chromatography (Superdex200); and as the fourth step, aseptic filtration. Note that for Fc-gamma RI, anion-exchange column chromatography using Q sepharose FF is carried out as the first step. Concentration of the purified protein is calculated based on absorptivity coefficient, calculated by measuring absorbance at 280 nm using a spectrophotometer and using PACE or such method for the measured values (Protein Science, 1995, 4, 2411-2423).

In one embodiment, binding activity of an antibody Fc region towards human FcRn may be measured by a ligand-capturing method using, for example, BIACORE (registered trademark) T200, which rely upon surface plasmon resonance analysis methods as the measurement principle.

Details of an exemplary method of measuring binding activity of antibody Fc region towards human FcRn are described below. In one embodiment, binding activity of an antibody Fc region towards human FcRn is evaluated using BIACORE (registered trademark) T200. In a preferred embodiment, this measurement is carried out at 25 degrees C, using a measurement buffer 50 mM phosphate, 150 mM NaCl, 0.05 w/v%-P20, pH 6.0. Specifically, about 400 RU of an antibody comprising an Fc region is first captured onto a sensor chip, onto which CaptureSelect (trademark) Human Fab-lambda Kinetics Biotin Conjugate (ThermoFisher scientific) is immobilized as a ligand-capturing molecule, and then human FcRn diluted using the measurement buffer is allowed to bind thereto. Binding activity of each antibody towards FcRn is assessed by calculating KD (M) using Steady state model in Biacore T200 Evaluation Software 2.0. In one preferred embodiment, the human FcRn protein used in this measurement is prepared according to the method described in Reference Example 2 of WO2010107110. In one embodiment, binding activity of an antibody Fc region towards various human FcRn may be measured by the method described in Reference Example 7-5.

d) Cysteine engineered antibody variants

In certain embodiments, it may be desirable to create cysteine engineered antibodies, e.g., "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, e.g., in U.S. Patent No. 7,521,541.

e) Antibody Derivatives

In certain embodiments, an antibody provided herein may be further modified to contain additional nonproteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, polypropylene glycol homopolymers, polypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of an antibody and nonproteinaceous moiety that may be selectively heated by exposure to radiation are provided. In one embodiment, the nonproteinaceous moiety is a carbon nanotube (Kam et al., Proc. Natl. Acad. Sci. USA 102: 11600-11605 (2005)). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

### B. Recombinant Methods and Compositions

Antibodies may be produced using recombinant methods and compositions, e.g., as described in U.S. Patent No. 4,816,567. In one embodiment, isolated nucleic acid encoding an anti-CD137 antigen-binding molecule or antibody described herein is provided. Such nucleic acid may encode an amino acid sequence comprising the VL and/or an amino acid sequence comprising the VH of the antibody (e.g., the light and/or heavy chains of the antibody). In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one embodiment, the host cell is eukaryotic, e.g. a Chinese Hamster Ovary (CHO) cell or lymphoid cell (e.g., Y0, NS0, Sp2/0 cell). In one embodiment, a method of making an anti-CD 137 antigen-binding molecule or antibody is provided, wherein the method comprises culturing a host cell comprising a nucleic acid encoding the antibody, as provided above, under conditions suitable for expression of the anti-CD 137 antigen-binding molecule or antibody, and optionally recovering the antibody from the host cell (or host cell culture medium).

For recombinant production of an anti-CD 137 antigen-binding molecule or antibody, nucleic acid encoding an antibody, e.g., as described above, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in *E. coli*.) After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, Nat. Biotech. 22:1409-1414 (2004), and Li et al., Nat. Biotech. 24:210-215 (2006).

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. *See,* e.g., US Patent Nos. 5,959,177, 6,040,498, 6,420,548, 7,125,978, and 6,417,429 (describing PLANTIBODIES™ technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham et al., J. Gen Virol. 36:59 (1977)); baby hamster kidney cells (BHK); mouse sertoli cells (TM4 cells as described, e.g., in Mather, Biol. Reprod. 23:243-251 (1980)); monkey kidney cells (CV1); African green monkey kidney cells (VERO-76); human cervical carcinoma cells (HELA); canine kidney cells (MDCK); buffalo rat liver cells (BRL 3A); human lung cells (W138); human liver cells (Hep G2); mouse mammary tumor (MMT 060562); TRI cells, as described, e.g., in Mather et al., Annals N.Y. Acad. Sci. 383:44-68 (1982); MRC 5 cells; and FS4 cells. Other useful mammalian host cell lines include Chinese hamster ovary (CHO) cells, including DHFR⁻ CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, see, e.g., Yazaki and Wu, Methods in Molecular Biology, Vol. 248 (B.K.C. Lo, ed., Humana Press, Totowa, NJ), pp. 255-268 (2003).

C. Assays

Anti-CD137 antigen-binding molecules or antibodies provided herein may be identified, screened for, or characterized for their physical/chemical properties and/or biological activities by various assays known in the art.

1. Binding assays and other assays

In one aspect, an antigen-binding molecule or antibody of the present disclosure is tested for its antigen binding activity, e.g., by known methods such as ELISA, Western blot, etc.

In another aspect, competition assay in the presence of a small molecule compound may be utilized in order to identify an antigen-binding molecule or antibody that competes for the binding to CD137 with the anti-CD137 antigen-binding molecules or antibodies comprising A375/B167, A372/B040, A356/B040, A486/B167, A487/B167, A488/B226, A489/B223, A548/B376, A551/B256, A551/B379, A555/B379, A548/B256, and/or A549/B167 described in Table 37 as a combination of heavy chain variable region/light chain variable region in the presence of a low-molecular weight compound (e.g., in the presence of 10 micromolar or more, 50 micromolar or more, 100 micromolar or more, 150 micromolar or more, 200 micromolar or more, or 250 micromolar or more of the low-molecular weight compound). In certain embodiments, such competing antigen-binding molecules or antibodies bind to the same epitope (e.g., linear epitope or conformational epitope) that is bound by the anti-CD137 antigen-binding molecules or antibodies comprising A375/B167, A372/B040, A356/B040, A486/B167, A487/B167, A488/B226, A489/B223, A548/B376, A551/B256, A551/B379, A555/B379, A548/B256, and/or A549/B167 described in Table 37 as a combination of heavy chain variable region/light chain variable region. Detailed exemplary methods for mapping an epitope to which an antigen-binding molecule or antibody binds are provided in Morris (1996) "Epitope Mapping Protocols," in Methods in Molecular Biology vol. 66 (Humana Press, Totowa, NJ). In one embodiment, the anti-CD137 antigen-binding molecules or antibodies of the present disclosure having CD137 binding activity that is dependent on antigen-binding activity dependent on a small molecule compound recognize an epitope formed by a complex formed from the antigen (e.g., CD137) and the low-molecular weight compound (e.g., ATP).

In an exemplary competition assay using an antibody, immobilized CD137 is incubated in the presence of a low-molecular weight compound (e.g., in the presence of 10 micromolar or more, 50 micromolar or more, 100 micromolar or more, 150 micromolar or more, 200 micromolar or more, or 250 micromolar or more of the low-molecular weight compound) in a solution containing a first labeled antibody binding to CD137 (e.g., the anti-CD137 antibody comprising A375/B167, A372/B040, A356/B040, A486/B167, A487/B167, A488/B226, A489/B223, A548/B376, A551/B256, A551/B379, A555/B379, A548/B256, and/or A549/B167 described in Table 37 as a combination of heavy chain variable region/light chain variable region) and a second unlabeled antibody that is tested for the ability to compete for the binding to CD137 with the first antibody. The second antibody may be present in a hybridoma supernatant. As a control, immobilized CD137 is incubated in a solution comprising the first labeled antibody but not the second unlabeled antibody. After incubation under conditions permissive for binding of the first antibody to [CD137, excess unbound antibody is removed, and the amount of label associated with immobilized CD137 is measured. If the amount of label associated with immobilized CD137 is substantially reduced in the test sample relative to the control sample, then that indicates that the second antibody is competing with the first antibody for binding to CD137. See Harlow and Lane (1988) Antibodies: A Laboratory Manual ch.14 (Cold Spring Harbor Laboratory, Cold Spring Harbor, NY). Those skilled in the art would understand that the assay can be carried out similarly to antigen-binding molecules other than antibodies.

2. Activity assays

In one aspect, assays are provided for identifying biological activity of anti-CD137 antigen-binding molecules or antibodies having the biological activity. Biological activity may include, e.g.,CD137 agonist activity; plasma half-life; anti-tumor activity; and low or suppressed systemic reaction in tissues other than tumors. Antigen-binding molecules or antibodies having such biological activity in vivo and/or in vitro are also provided.

In certain embodiments, an antigen-binding molecule (for example, an anti-CD 137 antigen-binding molecule) or antibody of the present disclosure is tested for such biological activity.

a) Agonistic activity assay (PBMC)

In one embodiment, the agonistic activity towards CD137 is measured by contacting CD137-expressing cells with an anti-CD 137 antigen-binding molecule or antibody in a solution to which a small molecule compound is added

or not added. In one embodiment, the agonistic activity towards CD137 in a solution in which the small molecule compound is added, and the agonistic activity towards CD137 in a solution in which the small molecule compound is not added, are respectively evaluated by the amount of cytokine production (e.g., amount of IL-2, IFN-$\gamma$, and/or IL- 6 production) which is measured within 18 hours, 24 hours, 36 hours, 48 hours, or 72 hours after a CD137-expressing cell and the CD137 antigen-binding molecule or antibody are contacted in the solution. In one embodiment, the solution in which the small molecule compound is added is adjusted such that the concentration of the small molecule compound after adjustment is 10 $\mu$M, 50 $\mu$M, 100 $\mu$M, 150 $\mu$M, 200 $\mu$M, or 250 $\mu$M. In a further embodiment, the CD137-expressing cells to be used are isolated human peripheral blood mononuclear cells (PBMCs), or T cells expanded from the isolated human PBMCs.

In one embodiment, human PBMCs which are isolated from blood collected from healthy individuals by centrifugation at 400 $\times$ g for 30 minutes at room temperature are used. Preferably, human PBMCs isolated in the following two steps are used. In the first step, Leucosep (Greiner Bio-One) supplemented with Ficoll-Paque PLUS (GE Healthcare) is centrifuged at 1000 $\times$ g for 1 minute at room temperature, and then blood diluted with PBS is added, and centrifuged at 400 $\times$ g for 30 minutes at room temperature. In the second step, after the buffy coat is collected from the tube after centrifugation, it is washed with 60 mL of PBS (Wako).

Details of an exemplary method of measuring CD137 agonistic activity using human PBMCs is described below. It is noted that even though the following example uses illustratively ATP as a small molecule compound, this does not exclude other small molecule compounds. In one embodiment, the isolated human PBMCs are diluted to a cell density 5 $\times$ 10$^6$/mL with culture medium (5% human serum (SIGMA), 95% AIM-V (Thermo Fischer Scientific)). Then, the isolated human PBMCs are contacted with an anti-human CD3$\varepsilon$ antibody and/or an anti-human CD28 antibody, whereby CD137 expression is induced in the human PBMCs. Preferably, 50 $\mu$L of 0.04 $\mu$g/mL anti-human CD3$\varepsilon$ antibody (BD Co., clone SP34) and 20 $\mu$g/mL anti-human CD28 antibody (BD, clone: CD28.2) diluted with culture medium is added to the isolated human PBMCs (100 $\mu$L at cell density 5 $\times$ 10$^6$/mL).

The human PBMC to which anti-human CD3$\varepsilon$ antibody and/or anti-human CD28 antibodies were added are then further added with (i) culture medium with or without ATP; and (ii) an anti-CD 137 antigen-binding molecule or an antibody. Preferably, 25 $\mu$L of the culture medium with or without ATP, is added. Preferably, 25 $\mu$L of the anti-CD137 antigen binding molecule or antibody at 40 $\mu$g/mL is added. More preferably, the above (i) and (ii) are added about 6 hours after contacting human PBMCs with the anti-human CD3$\varepsilon$ antibody and/or the anti-human CD28 antibody. In one embodiment, preferably, the amount of IL-2 production is measured prior to the amount of IFN-$\gamma$ production. In one embodiment, the amount of IL-2 production is measured within about 24 hours after contacting human PBMCs with the anti-human CD3$\varepsilon$ antibody and/or the anti-human CD28 antibody. Preferably, the amount of IL-2 production is measured about 24 hours after contacting human PBMCs with the anti-human CD3$\varepsilon$ antibody and/or anti-human CD28 antibody, and about 18 hours after adding the anti-CD137 antigen-binding molecule or antibody.

In another embodiment, the amount of IFN-$\gamma$ production is measured within about 48 hours after contacting human PBMCs with the anti-human CD3$\varepsilon$ antibody and/or anti-human CD28 antibody. Preferably, the amount of IFN-$\gamma$ production is measured about 48 hours after contacting human PBMCs with the anti-human CD3$\varepsilon$ antibody and/or the anti-human CD28 antibody, and about 42 hours after adding the anti-CD137 antigen-binding molecule or antibody. In one embodiment, the amount of IL-2 production and/or the amount of IFN-$\gamma$ production are/is determined by measuring the amount of IL-2 production and/or the amount of IFN-$\gamma$ production in the collected culture supernatant. In one embodiment, the human PBMCs added with the anti-human CD3$\varepsilon$ antibody and/or anti-human CD28 antibody are left to stand in a 5% CO$_2$ incubator at 37°C until all measurements are completed.

Details of a further exemplary method of measuring CD137 agonistic activity using human PBMCs are described below. Isolated human PBMCs are diluted to a cell density of 5 $\times$ 10$^6$/mL with culture medium (5% human serum (SIGMA), 95% AIM-V (Thermo Fischer Scientific)). Then, the human PBMCs are adjusted to a cell density of 5 $\times$ 10$^6$/mL, and 100 $\mu$L each of this is seeded into a 96-well multiwell plate (flat bottom, with lid) (Corning). After that, the human PBMCs are subjected to the operation of inducing CD137 expression. For example, CD137 expression in human PBMCs is induced by adding 50 $\mu$L of 0.04 $\mu$g/mL anti-human CD3$\varepsilon$ antibody (BD Co., clone SP34) and 20 $\mu$g/mL anti-human CD28 antibody (BD, clone: CD28.2) diluted with culture medium.

After CD137 expression is induced in human PBMCs, the plate is shaken and left to stand for 6 hours at 37°C in a 5% CO$_2$ incubator. Then, 25 $\mu$L each of 2 mM ATP (SIGMA) diluted with medium or medium only without ATP, and 25 $\mu$L of each antibody at 40 $\mu$g/mL are added to each well, and the plate is then shaken and left to stand for 18 hours at 37°C in a 5% CO$_2$ incubator. Then, a part of the culture supernatant is collected, and using this, the amount of IL-2 contained in the culture supernatant is quantified using Human IL-2 DuoSet ELISA kit (R&D systems) or Human IL-2 ELISA Set (BD Biosciences). The plate after the collection of the culture supernatant is again left to stand for 24 hours at 37°C again in a 5% CO$_2$ incubator. Then, a part of the culture supernatant is collected, and the amount of IFN-$\gamma$ contained in the culture supernatant is quantified using Human IFN-$\gamma$ DuoSet ELISA kit (R&D systems) or Human IFN-$\gamma$ ELISA Development Kit (PeproTech). ELISA is basically performed according to the protocol attached to the kit. For the Human IL-2 DuoSet ELISA kit (R&D systems) and Human IFN-$\gamma$ DuoSet ELISA kit (R&D systems), color development and color termination are carried out according to the protocol using a substrate solution (R&D systems) containing $H_2O_2$ and tetramethylbenzidine and IN $H_2SO_4$ (Wako). For the Human IL-2 ELISA Set (BD Biosciences), color termination is carried out using IN $H_2SO_4$ (Wako).

For the IFN-γ ELISA Development Kit (PeproTech), color development and color termination are carried out using TMB Chromogen Solution (Thermo Fischer Scientific) and IN $H_2SO_4$ (Wako). Then, the measurement of absorbance is carried out with EnVision (PerkinElmer), and the amounts (pg/mL) of IL-2 and IFN-γ in the culture supernatant are respectively calculated using a calibration curve prepared according to the protocol. In this PBMC assay, the CD137 agonist activity can be expressed as a fold change of the amounts of IL-2 and IFN-γ in the culture supernatant relative to those of a negative control antibody (an antibody that does not bind to CD137). In one embodiment, the CD137 agonistic activity is measured according to the methods described in Reference Examples 5-5-1 and 5-5-2.

In one embodiment, when the agonistic activity towards CD137 is evaluated by the amount of cytokine production (e.g., amount of IL-2, IFN-γ, and/or IL-6 production) in human PBMC assay, an anti-CD 137 antigen-binding molecule or antibody can be judged as exhibiting agonistic activity towards CD137 in the presence of a small molecule compound if the amount of cytokine production in the presence of 10 μM, 50 μM, 100 μM, 150 μM, 200 μM, or 250 μM of the small molecule compound when the anti-CD137 antigen-binding molecule or an antibody is added is 1.01-fold or more, 1.02-fold or more, 1.03-fold or more, 1.05-fold or more, 1.06-fold or more, 1.07-fold or more, 1.08-fold or more, 1.09-fold or more, 1.1-fold or more, 1.11-fold or more, 1.12-fold or more, 1.13-fold or more, 1.14-fold or more, or 1.15-fold or more, 1.5-fold or more, 2-fold or more, or 3-fold or more, as compared to the amount of cytokine production when the negative control antibody is added.

In one embodiment, when the agonistic activity towards CD137 is evaluated by the amount of IL-2 production in human PBMC assay, an anti-CD 137 antigen-binding molecule or antibody can be judged as exhibiting agonistic activity towards CD137 in the presence of a small molecule compound if the amount of IL-2 production in the presence of 10 μM, 50 μM, 100 μM, 150 μM, 200 μM, or 250 μM of the small molecule compound when the anti-CD 137 antigen-binding molecule or an antibody is added is 1.01-fold or more, 1.02-fold or more, 1.03-fold or more, or 1.05-fold or more, and is 1.05-fold or more in a preferred embodiment, as compared to the amount of IL-2 production when a negative control antibody is added.

In one embodiment, when the agonistic activity towards CD137 is evaluated by the amount of IFN-γ production in human PBMC assay, an anti-CD 137 antigen-binding molecule or an antibody can be judged as exhibiting agonistic activity towards CD137 in the presence of a small molecule compound if the amount of IFN-γ production in the presence of 10 μM, 50 μM, 100 μM, 150 μM, 200 μM, or 250 μM of the small molecule compound when the anti-CD137 antigen-binding molecule or an antibody is added is 1.1-fold or more, 1.11-fold or more, 1.12-fold or more, 1.13-fold or more, 1.14-fold or more, or 1.15-fold or more, and is 1.15 folds or more in a preferred embodiment, as compared to the amount of IFN-γ production when a negative control antibody is added.

In a further embodiment, from among the anti-CD137 antigen-binding molecules or antibodies judged to exhibit agonistic activity towards CD137 in the presence of the small molecule compound in the above-mentioned comparison with the negative control, antibodies to be judged as showing no CD137 agonistic activity or having lower CD137 agonistic activity in the absence of the small molecule compound (compared to that of the presence of the small molecule compound) are further determined. Specifically, when the following (ii) is greater than (i), the anti-CD 137 antigen-binding molecule or antibody is judged as showing no CD137 agonist activity or having lower CD137 agonist activity in the absence of the small molecule compound:

(i) [the amount of cytokine production in the absence of the small molecule compound when the anti-CD 137 antigen-binding molecule or antibody is added]/ [the amount of cytokine production in the absence of the small molecule compound when the negative control antibody is added]

(ii) [the amount of cytokine production in the presence of the small molecule compound when the anti-CD 137 antigen-binding molecule or antibody is added] / [the amount of cytokine production in the presence of the small molecule compound when the negative control antibody is added]

In a different embodiment, when the CD137 agonistic activities of a first anti-CD137 antigen-binding molecule or antibody and a second anti-CD 137 antigen-binding molecule or antibody are compared using the amount of cytokine production (e.g., the amount of IL-2, IFN-γ , and/or IL-6 production) in human PBMC assay, a first anti-CD 137 antigen-binding molecule or antibody can be judged as having a higher agonistic activity than a second anti-CD137 antigen-binding molecule or antibody if (i) the amount of cytokine production when the first anti-CD 137 antigen-binding molecule or antibody is added in the presence of 10 μM, 50 μM, 100 μM, 150 μM, 200 μM, or 250 μM of a small molecule compound is 1.01-fold or more, 1.02-fold or more, 1.03-fold or more, 1.04-fold or more, 1.05-fold or more, 1.06-fold or more, 1.07-fold or more, 1.08-fold or more, 1.09-fold or more, 1.1-fold or more, 1.11-fold or more, 1.12-fold or more, 1.13-fold or more, 1.14-fold or more, or 1.15-fold or more, 1.5-fold or more, 2-fold or more, or 3-fold or more, as compared to (ii) the amount of cytokine production when the second anti-CD137 antigen-binding molecule or antibody is added under the same conditions.

In one embodiment, when the agonistic activity towards CD137 is evaluated by the amount of IL-2 production in human PBMC assay, a first anti-CD137 antigen-binding molecule or antibody can be judged as having a higher agonistic activity than a second anti-CD137 antigen-binding molecule or antibody if (i) the amount of cytokine production when the first anti-CD 137 antigen-binding molecule or antibody is added in the presence of 10 μM, 50 μM, 100 μM, 150 μM, 200 μM, or 250 μM of a small molecule compound is 1.01-fold or more, 1.02-fold or more, 1.03-fold or more, or 1.04-fold or more, or is 1.04-fold or more in a preferable embodiment, as compared to (ii) the amount of IL-2 production when the second anti-CD137 antigen-binding molecule or antibody is added under the same conditions.

In one embodiment, when the agonistic activity towards CD137 is evaluated by the amount of IFN-γ production in human PBMC assay, a first anti-CD137 antigen-binding molecule or antibody can be judged as having a higher agonistic activity than a second anti-CD137 antigen-binding molecule or antibody if (i) the amount of cytokine production when the first anti-CD 137 antigen-binding molecule or antibody is added in the presence of 10 μM, 50 μM, 100 μM, 150 μM, 200 μM, or 250 μM of a small molecule compound is 1.05-fold or more, 1.06-fold or more, 1.07-fold or more, 1.08-fold or more, 1.09-fold or more, or 1.1-fold or more, or is 1.1-fold or more in a preferable embodiment, as compared to (ii) when the second anti-CD 137 antigen-binding molecule or antibody is added under the same conditions. In one embodiment, the first anti-CD137 antigen-binding molecule or antibody is an antigen-binding molecule or an antibody comprising the parent Fc region, and the second anti-CD 137 antigen-binding molecule is an antigen-binding molecule or antibody comprising a variant Fc region.

In one embodiment, the method described in Reference Examples 5-5-1 and 5-5-2 may be used to measure CD137 agonistic activity using human PBMCs, and the method described in Reference Example 2-6 may be used to measure CD137 agonistic activity using T cells expanded from the isolated human PBMCs.

In one embodiment, agonistic activity towards CD137 may be measured by contacting CD8-positive or CD4-positive T cells isolated from human PBMCs with an anti-CD 137 antigen-binding molecule or antibody, in a solution to which a small molecule compound is added or not added. At this time, FcγRIIb-expressing cells may be further added to the solution. Alternatively, agonistic activity towards CD137 may be measured by contacting B cells (which may be isolated from human PBMCs, or a known B cell line may be used) with an anti-CD 137 antigen-binding molecule or antibody. In one embodiment, the agonistic activity towards CD137 may be evaluated by the amount of cytokine production (for example, amounts of IL-2, IFN-γ and/or the amount of IL-6 production) that is measured after contacting, in a solution, CD8-positive T cells, CD4-positive T cells, or B cells with an anti-CD 137 antigen-binding molecule or antibody.

In one embodiment, agonistic activity assays using human peripheral blood mononuclear cells (PBMCs) are isolated from blood collected from healthy individuals. Thus, in any of the above embodiments, it would be understood by those skilled in the art that the assay results using the assays may differ by each blood sample donor. Considering this point, antibodies whose CD137 agonistic activity is not exhibited for a part or a majority of human PBMCs isolated from multiple donors may not be judged to exhibit CD137 agonistic activity, even though the criteria for agonistic activity are met in another part of human PBMCs. Furthermore, when an assay according to the above-described method is performed on human PBMCs isolated from a plurality of donors and criteria for CD137 agonistic activity are met in a majority of the donors, it may be judged that CD137 agonistic activity is exhibited. Alternatively, when an assay according to the above-described method is performed on human PBMCs isolated from a plurality of donors, the average or median of the assay values (e.g., amounts of IL-2, IFN-γ, and/or IL-6 production) may be used to determine the presence or absence of CD137 agonistic activity. In one embodiment, when the assay is performed according to the above method on human PBMCs isolated from a plurality of donors, the number of donors is, for example, 2 or more, 3 or more, 4 or more, 5 or more, 10 or more, 15 or more, or 20 or more.

b) Agonistic activity assay (reporter gene assay)

In one embodiment, the agonistic activity towards CD137 is evaluated by a reporter gene assay in a solution in which a small molecule compound is added, or not added. In one embodiment, the agonistic activity towards CD137 in a solution in which the small molecule compound is added, and the agonistic activity towards CD137 in a solution in which the small molecule compound is not added, are respectively evaluated by a luciferase luminescence signal measured after contacting T cells expressing the NF-kappaB-luciferase reporter construct and CD137 with an anti-CD 137 antigen-binding molecule, and left to stand a certain time, in the respective solution. In one embodiment, the solution in which the small molecule compound is added is adjusted such that the small molecule compound concentration after adjustment is 10 μM, 50 μM, 100 μM, 150 μM, 200 μM, or 250 μM. T cells expressing the NF-kappaB-luciferase reporter construct and CD137 are preferably, GloResponse™ NF-kappaB-Luc2/4-1BB Jurkat cell line (Promega, CS196004).

Details of an exemplary method of measuring CD137 agonistic activity using a reporter gene assay are described below. In the following examples, ATP is used as an example of the small molecule compound, but other small molecule compounds are not excluded. In one embodiment, first, FcγRIIB-expressing cells are adjusted to a concentration of $5 \times 10^4$/mL with medium (CHO culture medium (90% Ham's F12, 10 % FBS)), and left to stand overnight at 37°C in a

5% $CO_2$ incubator. In one embodiment, as FcgRIIB-expressing cells, not only cells forced to express FcgRIIB, but also cell lines that endogenously express FcgRIIB, such as B cell lines, B cells isolated from a living body, or the like can be used. In a preferred embodiment, the FcγRIIB-expressing cells are FcγRIIB CHO-K1 cells (Promega). Next, after the medium is removed by aspiration, GloResponse™ NF-kappaB-Luc2/4-1BB Jurkat cell line (hereinafter, "4-1BB Jurkat") adjusted to $2 \times 10^6$/mL with different medium (99% RPMI, 1% FBS) is added. In a preferred embodiment, per 200 μL of FcγRIIB-expressing cells adjusted with medium, 25 μL of GloResponse™ NF-kappaB-Luc2/4-1BB Jurkat cell line cells adjusted with medium is added. Subsequently, 25 μL each of anti-CD 137 antigen-binding molecule diluted with the above-mentioned medium (99% RPMI, 1% FBS) to become a desired concentration (e.g., final concentration of 0, 0.001, 0.01, 0.1, 1, 10 μg/mL) is added, and further, 25 μL of the ATP solution diluted with the above-mentioned medium (99% RPMI, 1% FBS) to become a desired concentration (e.g., final concentration is 0, 10, 50, 100, 150, 200, 250 μM ) is added. In one embodiment, the luciferase luminescence signal is measured after standing for 2 hours or less, 4 hours or less, 6 hours or less, 24 hours or less, following the addition of the anti-CD137 antigen-binding molecule to 4-1BB Jurkat. In a preferred embodiment, 4-1BB Jurkat is left to stand at 37°C for 6 hours in a 5% $CO_2$ incubator. After standing, 75 μL of Bio-Glo reagent is added, and the luminescence is measured with a plate reader. In one preferred embodiment, the Bio-Glo reagent is Bio-Glo Luciferase Assay System (buffer and substrate). In one embodiment, in order to keep the temperature during the reaction constant, the 4-1BB Jurkat may be left to stand at room temperature for 5 minutes, 10 minutes, 15 minutes, or 20 minutes after being removed from the incubator. In a preferable embodiment, the 4-1BB Jurkat is left to stand at room temperature for 15 minutes after being removed from the incubator. In one embodiment, the luminescence value of 4-1BB Jurkat added with an anti-CD137 antigen-binding molecule is divided by the luminescence value of 4-1BB Jurkat not added with the anti-CD137 antigen-binding molecule, and this value is taken as fold induction (relative light unit) and used as index for evaluating the CD137 agonistic activity of each antigen-binding molecule.

A further exemplary method of measuring CD137 agonistic activity using a reporter gene assay is described below. 200 μL each of FcγRIIB CHO-K1 cells (Promega) adjusted to a concentration of $5 \times 10^4$/mL with medium is added to each well of a 96-well plate, and left to stand overnight at 37°C in a 5% $CO_2$ incubator. As culture medium, CHO culture medium (90% Ham's F12, 10% FBS) is used. Then, after the medium is completely removed by aspiration, 25 μL of GloResponse™ NF-κB-Luc2/4-1BB Jurkat cell line adjusted to $2 \times 10^6$/mL with assay medium (99% RPMI, 1% FBS) is added to each well. Subsequently, 25 μL each of each antigen solution diluted with assay medium to a final concentration of 0, 0.001, 0.01, 0.1, 1, or 10 μg/mL is added, and finally, 25 μL of ATP solution diluted with assay medium to 0 or 250 μM is added. After the plate is left to stand for 6 hours at 37°C in a 5% $CO_2$ incubator, it is left to stand at room temperature for 15 minutes, and 75 μL of Bio-Glo reagent is added to each well. As Bio-Glo reagent, for example, Bio-Glo Luciferase Assay System (buffer and substrate) may be used. Thereafter, the luminescence of each well is measured with a plate reader. The value obtained by dividing the luminescence value of each well by the luminescence value of the well which is not added with any antibody is defined as fold induction. In the reporter gene assay, the CD137 agonistic activity may be evaluated by fold change (relative light unit) of the luminescence of wells added with each antibody against the luminescence amount of wells which are not added with any antibody.

A further exemplary method of measuring CD137 agonistic activity using a reporter gene assay is described below. To each well of a 384-well plate, 10 μL of FcγRIIB CHO-K1 Cells (Promega) adjusted to a concentration of $2 \times 10^6$/mL with assay medium (99 % RPMI, 1% FBS) is added. Subsequently, 10 μL of an antibody solution containing ADP, or an antibody solution containing ATP, or an antibody solution not containing ATP or ADP, is added to each well. 10 μL of GloResponse™ NF-κB-Luc2/4-1BB Jurkat cell line adjusted to $2 \times 10^6$/mL with the assay medium (99% RPMI, 1% FBS) is then added to each well. The final concentration of ADP is 50 μM and the final concentration of ATP is 50 μM. After the plate is left to stand at 37°C for 6 hours in a 5% $CO_2$ incubator, it is left to stand at room temperature for 15 minutes, and 30 μL of Bio-Glo reagent is added to each well. As Bio-Glo reagent, Bio-Glo Luciferase Assay System (buffer and substrate) was used. Thereafter, the luminescence of each well is measured with a plate reader. The CD137 agonistic activity may be evaluated by the relative light unit (also called luminescence fold or fold change) of the luminescence of wells added with each antibody against the luminescence of wells which are not added with any antibody.

A further exemplary method of measuring CD137 agonistic activity using a reporter gene assay is described below. To each well of a 384-well plate, 10 μL of FcγRIIB CHO-K1 Cells (Promega) adjusted to a concentration of $4 \times 10^5$/mL with assay medium (99 % RPMI, 1% FBS) is added. Subsequently, 10 μL of an antibody solution containing ADP, or an antibody solution containing ATP, or an antibody solution not containing ATP or ADP, is added to each well. 10 μL of GloResponse™ NF-κB-Luc2/4-1BB Jurkat cell line adjusted to $2 \times 10^6$/mL with the assay medium (99% RPMI, 1% FBS) is then added to each well. The final concentration of ADP is 10 μM and the final concentration of ATP is 10 μM. After the plate is left to stand at 37°C for 6 hours in a 5% $CO_2$ incubator, it is left to stand at room temperature for 15 minutes, and 30 μL of Bio-Glo reagent is added to each well. As Bio-Glo reagent, Bio-Glo Luciferase Assay System (buffer and substrate) was used. Thereafter, the luminescence of each well is measured with a plate reader. The CD137 agonistic activity may be evaluated by the relative light unit (also called luminescence fold or fold change) of the luminescence of wells added with each antibody against the luminescence of wells which are not added with any antibody.

In one embodiment, when the agonistic activity towards CD137 is measured using a reporter gene assay, an anti-CD 137 antigen-binding molecule or antibody can be judged as exhibiting an agonistic activity towards CD137 in the presence of a small molecule compound if (i) the agonistic activity towards CD137 (relative light unit) in the presence of 10 μM, 50 μM, 100 μM, 150 μM, 200 μM, or 250 μM of the small molecule compound is 1.1-fold or more, 1.2-fold or more, 1.3-fold or more, 1.5-fold or more, 2-fold or more, 3-fold or more, 5-fold or more, 10-fold or more, 20-fold or more, 30-fold or more, 50-fold or more, 60-fold or more, 70-fold or more, 80-fold or more, or 90-fold or more higher, as compared to (ii) the CD137 agonistic activity (relative light unit) in the absence of the small molecule compound. In one embodiment, the final concentration of the antibody in (i) and (ii) above is 0, 0.001, 0.01, 0.1, 1, or 10 μg/mL, and in a preferred embodiment, it is 0.1 μg/mL or 1 μg/mL.

In a further embodiment, when the anti-CD137 antigen-binding molecule or antibody is added at a specific concentration (for example, at a final concentration of 0.001, 0.01, 0.1, 1, or 10 μg/mL, or 0.1 μg/mL or 1 μg/mL in a preferred embodiment), the anti-CD137 antigen-binding molecule or antibody is judged as exhibiting substantially no CD137 agonistic activity in the absence of a small molecule compound if the fold induction (relative light unit) in the absence of the small molecule compound is 10 or less, 9 or less, 8 or less, or 5 or less in a preferred embodiment, and substantially 1 in a more preferred embodiment. In one embodiment, "fold induction (relative light unit) in the absence of a small molecule compound is substantially 1" means cases when the fold induction (relative light unit) in the absence of a small molecule compound is less than 2-fold, less than 1.9-fold, less than 1.8-fold, less than 1.7-fold, less than 1.6-fold, less than 1.5-fold, less than 1.4-fold, less than 1.3-fold, less than 1.2-fold, or less than 1.1-fold.

c) Plasma concentration

In one embodiment, the blood kinetics of an anti-CD 137 antigen-binding molecule or antibody of the present disclosure are measured and/or compared using human CD137 knock-in mice. Human CD137 knock-in mouse is prepared, for example, by replacing the mouse CD137 gene with the human CD137 gene by introducing a human CD137 gene replacement vector into mouse embryonic stem cells (ES cells). In one embodiment, a CD137 antigen-binding molecule or antibody of the present disclosure is administered through a single intravenous administration into human CD137 knock-in mice, and blood is collected multiple times over time from immediately after administration to around 5 days, 10 days, 15 days, 20 days, 25 days, or 30 days after administration. In a preferred embodiment, the CD137 antigen-binding molecule or antibody of the present disclosure is administered through a single intravenous administration into human CD137 knock-in mice, and blood is collected multiple times over time from 5 minutes to 28 days after administration. Plasma is rapidly separated from the collected blood, and antibody concentration in plasma is measured by electrochemiluminescence (ECL). In one embodiment, the antibody concentration in plasma can be measured by the method described in Reference Example 6-3-2.

In the measurement of plasma half-life using human CD137 knock-in mouse, if an anti-CD137 antigen-binding molecule or antibody disappears from plasma slower than a reference molecule, the anti-CD 137 antigen-binding molecule or antibody is judged as having more improved blood kinetics than the reference molecule. In addition, if an anti-CD 137 antigen-binding molecule or antibody having binding activity dependent on a small molecule compound (switch molecule or switch antibody) disappears from plasma slower than an anti-CD 137 antigen-binding molecule or antibody not having binding activity dependent on a small molecule compound (non-switch molecule or non-switch antibody), it can be judged that the switch molecule (or switch antibody) does not bind to CD137 expressed in a non-tumor tissue compared to the non-switch molecule (non-switch antibody).

d) Anti-tumor activity

In one aspect, an anti-CD 137 antigen-binding molecule or antibody thereof is tested for its ability to inhibit cell growth or proliferation in vivo. In certain embodiments, an anti-CD 137 antigen-binding molecule or antibody thereof is tested for its ability to inhibit tumor growth in vivo. In vivo model systems, such as allograft models or xenograft models, can be used for such testing. In an exemplary xenograft system, human tumor cells are introduced into a suitably immunocompromised non-human animal, e.g., an athymic "nude" mouse. An antibody of the present disclosure is administered to the animal. The ability of the antibody to inhibit or decrease tumor growth is measured. In certain embodiments of the above xenograft system, the human tumor cells are tumor cells from a human patient. Such xenograft models are commercially available from Oncotest GmbH (Frieberg, Germany). In certain embodiments, the human tumor cells are introduced into a suitably immunocompromised non-human animal by subcutaneous injection or by transplantation into a suitable site, such as a mammary fat pad.

In one embodiment, anti-tumor activity of the anti-CD137 antigen-binding molecules or antibodies of the present disclosure may be measured and/or compared using a syngeneic tumor cell graft model based on the above-described human CD137 knocked-in mice. Cancer cell line to use in the test may be suitably selected, but preferably is mouse colon cancer cell line MC38 cell line. In one embodiment, MC38 cell line is transplanted under the skin in the abdominal area of a mouse, and a model is regarded established when tumor volume reaches about 50-300 mm³. Following the establishment of the model, MC38 cell transplanted mice are grouped and then receive administration of each anti-CD 137 antigen-binding molecule or antibody. In one embodiment, tumor volume measurement is carried out at a frequency of once to twice a week, in order to evaluate anti-tumor activity. Tumor volume is calculated using the following equation: tumor volume = (major axis x minor axis x minor axis)/2. In one embodiment, the anti-tumor activity of the anti-CD 137 antigen-binding molecules or antibodies may be tested and evaluated following the method described in Reference Example 6-4.

In one embodiment, the anti-CD137 antigen-binding molecules or antibodies are recognized as exhibiting anti-tumor activity when the tumor volume in the anti-CD 137 antigen-binding molecule or antibody-administered group is smaller than that in the Vehicle group, or the increase in tumor volume in the anti-CD137 antigen-binding molecule or antibody-administered group is smaller than that in the Vehicle group.

e) Systemic reaction

In one embodiment, the systemic reaction to the anti-CD137 antigen-binding molecules or antibodies of the present disclosure is measured and/or compared using a syngeneic tumor cell graft model based on the above-described human CD137 knocked-in mice. Organs to measure the systemic reaction may be suitably selected, but preferably are liver, spleen, and/or lymph nodes. In one embodiment, the systemic reaction is evaluated by extirpating liver, spleen, and/or lymph nodes from human CD137 knocked-in mice at a suitable time following the administration of the anti-CD137 antigen-binding molecules or antibodies. When the organ to extirpate is spleen and/or lymph nodes, the organ is weighed and/or cells in the lymphocyte fraction are counted. In the counting, preferably, lymphocyte fraction after hemolysis is used for spleen and lymphocyte fraction obtained by homogenization is used for lymph nodes. When the organ to extirpate is liver, cells in the lymphocyte fraction obtained with Liver dissociation kit, mouse (Milteny Biotec) are counted. Furthermore, T cell analysis using flowcytometry (FCM) may be carried out on lymphocyte fraction from each organ (liver, spleen, and/or lymph nodes). In the FCM analysis, Grazyme B expression or PD-1 expression or ICOS expression in CD8-alpha positive T cells or a ratio of CD8-alpha positive T cells to CD45 positive cells is used, for example. In one embodiment, systemic reaction to the anti-CD 137 antigen-binding molecules or antibodies may be tested and evaluated following the method described in Reference Example 6-4.

In one embodiment, the anti-CD137 antigen-binding molecules or antibodies are recognized as causing suppressed systemic reaction and/or suppressed activation of immune cells in non-tumor tissues (e.g., liver, spleen, and/or lymph nodes) as compared to a reference molecule, when the anti-CD 137 antigen-binding molecule or antibody-administered group shows a lower value in the evaluation of the above-described various indicators than that for the group receiving administration of the same amount of the reference molecule. Furthermore, when the group receiving the administration of the anti-CD 137 antigen-binding molecule or antibody having binding activity dependent on a small molecule compound (switch molecule or switch antibody) shows a lower value in the evaluation of the above-described various indicators than that for the group receiving administration of the same amount of an anti-CD 137 antigen-binding molecule or antibody that does not have binding activity dependent on a small molecule compound (non-switch molecule or non-switch antibody), the switch molecule (or switch antibody) is recognized as causing suppressed systemic reaction and/or suppressed activation of immune cells in non-tumor tissues (e.g., liver, spleen, and/or lymph nodes) as compared to the non-switch molecule (or non-switch antibody).

It would be appreciated that any of the above-described measurement methods can be carried out using the immunoconjugates of the present disclosure in place of or in addition to the anti-CD137 antigen-binding molecules or antibodies.

D. Immunoconjugates

The present disclosure also provides immunoconjugates comprising an anti-CD 137 antigen-binding molecule or antibody herein conjugated to one or more cytotoxic agents, such as chemotherapeutic agents or drugs, growth inhibitory agents, toxins (e.g., protein toxins, enzymatically active toxins of bacterial, fungal, plant, or animal origin, or fragments thereof), or radioactive isotopes.

In one embodiment, an immunoconjugate is an antibody-drug conjugate (ADC) in which an antibody is conjugated to one or more drugs, including but not limited to a maytansinoid (see U.S. Patent Nos. 5,208,020, 5,416,064 and European Patent EP 0 425 235 B1); an auristatin such as monomethylauristatin drug moieties DE and DF (MMAE and MMAF) (see U.S. Patent Nos. 5,635,483 and 5,780,588, and 7,498,298); a dolastatin; a calicheamicin or derivative thereof (see U.S. Patent Nos. 5,712,374, 5,714,586, 5,739,116, 5,767,285, 5,770,701, 5,770,710, 5,773,001, and 5,877,296; Hinman et al., Cancer Res. 53:3336-3342 (1993); and Lode et al., Cancer Res. 58:2925-2928 (1998)); an anthracycline such as daunomycin or doxorubicin (see Kratz et al., Current Med. Chem. 13:477-523 (2006); Jeffrey et al., Bioorganic & Med. Chem. Letters 16:358-362 (2006); Torgov et al., Bioconj. Chem. 16:717-721 (2005); Nagy et al.,

Proc. Natl. Acad. Sci. USA 97:829-834 (2000); Dubowchik et al., Bioorg. & Med. Chem. Letters 12:1529-1532 (2002); King et al., J. Med. Chem. 45:4336-4343 (2002); and U.S. Patent No. 6,630,579); methotrexate; vindesine; a taxane such as docetaxel, paclitaxel, larotaxel, tesetaxel, and ortataxel; a trichothecene; and CC1065.

In another embodiment, an immunoconjugate comprises an antibody as described herein conjugated to an enzymatically active toxin or fragment thereof, including but not limited to diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolacca americana proteins (PAPI, PAPII, and PAP-S), momordica charantia inhibitor, curcin, crotin, saponaria officinalis inhibitor, gelonin, mitogellin, restrictocin, phenomycin, enomycin, and the tricothecenes.

In another embodiment, an immunoconjugate comprises an antibody as described herein conjugated to a radioactive atom to form a radioconjugate. A variety of radioactive isotopes are available for the production of radioconjugates. Examples include $^{211}$At, $^{131}$I, $^{125}$I, $^{90}$Y, $^{186}$Re, $^{188}$Re, $^{153}$Sm, $^{212}$Bi, $^{32}$P, $^{212}$Pb and radioactive isotopes of Lu. When the radioconjugate is used for detection, it may comprise a radioactive atom for scintigraphic studies, for example Tc-99m or $^{123}$I, or a spin label for nuclear magnetic resonance (NMR) imaging (also known as magnetic resonance imaging, MRI), such as iodine-123 again, iodine-131, indium-111, fluorine-19, carbon-13, nitrogen-15, oxygen-17, gadolinium, manganese or iron.

Conjugates of an antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., *Science* 238:1098 (1987). Carbon-14-labeled 1-isothiocyanatobenzyl-3-methyldiethylene triaminepentaacetic acid (MX-DTPA) is an exemplary chelating agent for conjugation of radionuclide to the antibody. See WO94/11026. The linker may be a "cleavable linker" facilitating release of a cytotoxic drug in the cell. For example, an acid-labile linker, peptidase-sensitive linker, photolabile linker, dimethyl linker or disulfide-containing linker (Chari et al., Cancer Res. 52:127-131 (1992); U.S. Patent No. 5,208,020) may be used.

The immunuoconjugates or ADCs herein expressly contemplate, but are not limited to such conjugates prepared with cross-linker reagents including, but not limited to, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (e.g., from Pierce Biotechnology, Inc., Rockford, IL., U.S.A).

E. Methods and Compositions for Diagnostics and Detection

In certain embodiments, any of the anti-CD137 antigen-binding molecules or antibodies provided herein is useful for detecting the presence of CD137 antigen-binding molecule or in a biological sample. The term "detecting" as used herein encompasses quantitative or qualitative detection. In certain embodiments, a biological sample comprises a cell or tissue.

In one embodiment, an anti-CD 137 antigen-binding molecule or antibody for use in a method of diagnosis or detection is provided. In a further aspect, a method of detecting the presence of CD137 in a biological sample is provided. In certain embodiments, the method comprises contacting the biological sample with an anti-CD 137 antigen-binding molecule or antibody as described herein under conditions permissive for binding of the anti-CD 137 antigen-binding molecule or antibody to CD137, and detecting whether a complex is formed between the anti-CD137 antigen-binding molecule or antibody and CD137. Such method may be an *in vitro* or *in vivo* method. In one embodiment, an anti-CD 137 antigen-binding molecule or antibody is used to select subjects eligible for therapy with an anti-CD 137 antigen-binding molecule or antibody, e.g. where CD137 is a biomarker for selection of patients.

Exemplary disorders that may be diagnosed using an antibody of the present disclosure include cancer.

In certain embodiments, labeled anti-CD 137 antigen-binding molecules or antibodies are provided. Labels include, but are not limited to, labels or moieties that are detected directly (such as fluorescent, chromophoric, electron-dense, chemiluminescent, and radioactive labels), as well as moieties, such as enzymes or ligands, that are detected indirectly, e.g., through an enzymatic reaction or molecular interaction. Exemplary labels include, but are not limited to, the radioisotopes $^{32}$P, $^{14}$C, $^{125}$I, $^{3}$H, and $^{131}$I, fluorophores such as rare earth chelates or fluorescein and its derivatives, rhodamine and its derivatives, dansyl, umbelliferone, luceriferases, e.g., firefly luciferase and bacterial luciferase (U.S. Patent No. 4,737,456), luciferin, 2,3-dihydrophthalazinediones, horseradish peroxidase (HRP), alkaline phosphatase, beta-galactosidase, glucoamylase, lysozyme, saccharide oxidases, e.g., glucose oxidase, galactose oxidase, and glucose-6-phosphate dehydrogenase, heterocyclic oxidases such as uricase and xanthine oxidase, those coupled with an enzyme that employs hydrogen peroxide to oxidize a dye precursor such as HRP, lactoperoxidase, or microperoxidase, biotin/avidin, spin labels, bacteriophage labels, stable free radicals, and the like.

F. Pharmaceutical Formulations

Pharmaceutical formulations of an anti-CD 137 antigen-binding molecule or antibody as described herein are prepared by mixing such antibody having the desired degree of purity with one or more optional pharmaceutically acceptable carriers (Remington's Pharmaceutical Sciences 16th edition, Osol, A. Ed. (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); small molecule (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Exemplary pharmaceutically acceptable carriers herein further include interstitial drug dispersion agents such as soluble neutral-active hyaluronidase glycoproteins (sHASEGP), for example, human soluble PH-20 hyaluronidase glycoproteins, such as rHuPH20 (HYLENEX (registered trademark), Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO2006/044908, the latter formulations including a histidine-acetate buffer.

Active ingredients may be entrapped in microcapsules prepared, for example, by coacervation techniques or by interfacial polymerization, for example, hydroxymethylcellulose or gelatin-microcapsules and poly-(methylmethacrylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antibody, which matrices are in the form of shaped articles, e.g. films, or microcapsules.

The formulations to be used for *in vivo* administration are generally sterile. Sterility may be readily accomplished, e.g., by filtration through sterile filtration membranes.

G. Therapeutic methods and therapeutic compositions

Any of the anti-CD 137 antigen-binding molecules or antibodies provided herein may be used in therapeutic methods.

In one aspect, in the present disclosure, anti-CD137 antigen-binding molecules or antibodies for use as a medicament are provided. In the present disclosure, examples of the medicament specifically include those for inducing anti-tumor action, for example, inhibition of neovascularization in tumors, inhibition of tumor cell proliferation, depletion of tumor-promoting B cells, etc., via activation of cells resulting from the binding of the anti-CD137 antigen-binding molecules or antibodies to CD137 expressed on immune cells such as T cells. In further aspects, in the present disclosure, anti-CD137 antigen-binding molecules or antibodies for use in treating tumor are provided. In certain embodiments, anti-CD137 antigen-binding molecules or antibodies for use in a method of treatment are provided. In certain embodiments, the present disclosure provides anti-CD137 antigen-binding molecules or antibodies for use in a method of treating an individual having tumor comprising administering to the individual an effective amount of an anti-CD 137 antigen-binding molecule or antibody. In further embodiments, in the present disclosure, examples of tumor include solid tumor into which B cells, dendritic cells, natural killer cells, macrophages, CD8-positive T cells, and/or regulatory T cells (Treg cells) have infiltrated.

In further aspects, the present disclosure provides anti-CD 137 antigen-binding molecules or antibodies for use in a method of activating immune cells in an individual, the method comprising administering to the individual an effective amount of an anti-CD137 antigen-binding molecule or antibody to activate immune cells such as B cells, dendritic cells, natural killer cells, macrophages, and/or CD8-positive T cells (more specifically, these immune cells having infiltrated into a tumor tissue).

In further aspects, the present disclosure provides anti-CD 137 antigen-binding molecules or antibodies for use in a method of damaging cells (e.g., tumor cells) in an individual, the method comprising administering to the individual an effective amount of an anti-CD137 antigen-binding molecule or antibody. In certain embodiments, examples of the tumor include solid tumor into which B cells, dendritic cells, natural killer cells, macrophages, CD8-positive T cells, and/or regulatory T cells (Treg cells) have infiltrated. An "individual" according to any of the above embodiments is preferably a human.

In further aspects, the present disclosure provides use of anti-CD 137 antigen-binding molecules or antibodies in the manufacture or preparation of a medicament. In one embodiment, the medicament is for treating tumor (depending on context, it may be more appropriate to call this as "cancer" (the same applies hereafter)). In further embodiments, the medicament is for use in a method of treating tumor (or cancer depending on context), the method comprising administering to an individual having the tumor (or cancer depending on context) an effective amount of the medicament. In further embodiments, the medicament is for inducing anti-tumor action, for example, inhibition of neovascularization in tumors, inhibition of tumor cell proliferation, depletion of tumor-promoting B cells, etc., via activation of cells resulting from the binding of an anti-CD 137 antigen-binding molecule or antibody to CD137 expressed on immune cells such as T cells. In further embodiments, the medicament is for use in a method for, for example, inhibiting neovascularization in tumors, inhibiting tumor cell proliferation, depleting tumor-promoting B cells, etc., via activation of cells resulting from the binding of an anti-CD 137 antigen-binding molecule or antibody to CD137 expressed on immune cells such as T cells in an individual, the method comprising administering to the individual an effective amount of the medicament therefor. An "individual" according to any of the above embodiments may be a human.

In further aspects, the present disclosure provides methods for treating tumor. In one embodiment, the methods comprise administering to an individual having such tumor an effective amount of an anti-CD 137 antigen-binding molecule or antibody. In further embodiments, in the present disclosure, examples of the tumor include solid tumor into which B cells, dendritic cells, natural killer cells, macrophages, CD8-positive T cells, and/or regulatory T cells (Treg cells) have infiltrated.

In further aspects, the present disclosure provides methods for activating an immune cell in a tumor tissue in an individual. In one embodiment, the method comprises administering to the individual an effective amount of an anti-CD 137 antigen-binding molecule or antibody. In further embodiments, in the present disclosure, the immune cell includes immune cells such as B cells, dendritic cells, natural killer cells, macrophages, and/or CD8-positive T cells (more specifically, these immune cells having infiltrated into a tumor tissue). In this method, when the expression level of at least one gene selected from CD8b1, Gzmb, Prfl and Ifng is increased in the tumor tissue, it can be judged that the activation of T cells in the tumor tissue is enhanced.

In a further aspect, the present disclosure provides a method for activating, or promoting the proliferation of, immune cells, particularly CD8$^+$ T cells, in an individual's tumor tissue. In one embodiment, such methods include a method which comprises administering to an individual an effective amount of an anti-CD 137 antigen-binding molecule or antibody. In the method of activating CD8$^+$ T cells, when there is an increase in the positive rate of at least one CD8$^+$ T cell activation marker selected from OVA tetramer, Granzyme B, PD-1, DLRG-1, and ICOS (positive rate (%) = number of CD8$^+$ T cells positive for the activation marker in tumor tissue/number of CD8$^+$ T cells in tumor tissue $\times$ 100), it can be judged that CD8$^+$ T cells in tumor tissue are activated.

In further aspects, the present disclosure provides methods for damaging cells (specifically, tumor cells) in an individual. In one embodiment, the methods include those methods that comprise administering to an individual an effective amount of an anti-CD137 antigen-binding molecule or antibody. In certain embodiments, examples of the tumor include solid tumor into which B cells, dendritic cells, natural killer cells, macrophages, CD8-positive T cells, and/or regulatory T cells (Treg cells) have infiltrated. An "individual" according to any of the above embodiments may be a human.

In some embodiments, the individual is a patient having cancer cells or tumor tissue containing cancer cells. Preferred cancer types in the present disclosure include, for example, gastric cancer, head and neck cancer, esophageal cancer, colorectal cancer, lung cancer, mesothelioma, liver cancer, ovarian cancer, breast cancer, colon cancer, kidney cancer, skin cancer, muscle tumor, pancreatic cancer, prostate cancer, testicular cancer, uterine cancer, cholangiocarcinoma, Merkel cell carcinoma, bladder cancer, thyroid cancer, schwannoma, adrenal cancer, anal cancer, central nervous system tumor, neuroendocrine tissue tumor, penile cancer, pleural tumor, salivary gland tumor, vulvar cancer, thymoma, lymphoma, myeloid leukemia, pediatric cancer (Wilms tumor, neuroblastoma, sarcoma, hepatoblastoma, and germ cell tumor), but are not limited thereto. More preferred cancer types include gastric cancer, colorectal cancer, lung cancer, mesothelioma, liver cancer, breast cancer, skin cancer, lymphoma, and myeloid leukemia, but are not limited thereto (Tumori. (2012) 98, 478-484; Tumor Biol. (2015) 36, 4671-4679; Am J Clin Pathol (2008) 130, 224-230; Adv Anat Pathol (2014) 21, 450-460; Med Oncol (2012) 29, 663-669; Clinical Cancer Research (2004) 10, 6612-6621; Appl Immunohistochem Mol Morphol (2009) 17, 40-46; Eur J Pediatr Surg (2015) 25, 138-144; J Clin Pathol (2011) 64, 587-591; Am J Surg Pathol (2006) 30, 1570-1575; Oncology (2007) 73, 389-394; Diagnostic Pathology (2010) 64, 1-6; Diagnostic Pathology (2015) 34 , 1-6; Am J Clin Pathol (2008) 129, 899-906; Virchows Arch (2015) 466, 67-76). More preferable cancer types include gastric cancer, lung cancer, mesothelioma, liver cancer, breast cancer, skin cancer, lymphoma, and myeloid leukemia.

An anticancer agent comprising an anti-CD 137 antigen-binding molecule of the present disclosure can be used to treat a patient having a cancer refractory to treatment with an immune checkpoint inhibitor. For example, a patient for whom the administration of an immune checkpoint inhibitor did not result in a desired efficacy can be treated with an anticancer agent of the present disclosure. A "cancer refractory to treatment with an immune checkpoint inhibitor" can also be expressed as "a cancer already treated with an immune checkpoint inhibitor", "a cancer that aggravated during or after treatment with an immune checkpoint inhibitor", "a cancer for which an immune checkpoint inhibitor is ineffective", "a cancer that recurred after treatment with an immune checkpoint inhibitor", "a cancer that is resistant to treatment with an immune checkpoint inhibitor", "a cancer for which treatment with an immune checkpoint inhibitor is inadequately effective", or "a cancer for which treatment with an immune checkpoint inhibitor is unsuccessful".

The above-mentioned cancers that are refractory to treatment with an immune checkpoint inhibitor include cancers with a gene mutation in JAK1, JAK2, and/or B2M (see, for example, Shin, Daniel Sanghoon et al. "Primary Resistance to PD-1 Blockade Mediated by JAK1/2 Mutations." Cancer Discovery Vol. 7,2 (2017): 188-201; or Sade-Feldman, Moshe et al. "Resistance to checkpoint blockade therapy through inactivation of antigen presentation." Nature Communications Vol. 8(1): 1136. 26 Oct. 2017), but are not limited thereto.

In further aspects, in the present disclosure, pharmaceutical preparations comprising an anti-CD137 antigen-binding molecule or antibody to be used for the above-described methods of treatment, uses in treating, and medicaments may comprise an effective amount of the anti-CD137 antigen-binding molecule or antibody which has lower level of immunological activation in a non-tumor tissue, compared to an anti-CD 137 antigen-binding molecule that does not have CD137 binding activity dependent on a small molecule compound.

In one embodiment, the non-tumor tissues include lymph nodes, spleen, and/or liver.

In further embodiments, the pharmaceutical preparations comprising an anti-CD 137 antigen-binding molecule or antibody to be used for the above-described methods of treatment, uses in treating, and medicaments may comprise an effective amount of the anti-CD137 antigen-binding molecule or antibody which substantially does not bind to CD137 expressed in a non-tumor tissue.

In further embodiments, the pharmaceutical preparations comprising an anti-CD 137 antigen-binding molecule or antibody to be used for the above-described methods of treatment, uses in treating, and medicaments may comprise an effective amount of the anti-CD137 antigen-binding molecule or antibody which has prolonged blood half-life as compared to an anti-CD 137 antigen-binding molecule that does not have CD137 binding activity dependent on a small molecule compound.

In further embodiments, the pharmaceutical preparations comprising an anti-CD 137 antigen-binding molecule or antibody to be used for the above-described methods of treatment, uses in treating, and medicaments may comprise an effective amount of the anti-CD137 antigen-binding molecule or antibody which has low level of side effects as compared to an anti-CD 137 antigen-binding molecule that does not have CD137 binding activity dependent on a small molecule compound.

In further embodiments, the side effects include elevated AST, elevated ALT, fever, nausea, acute hepatitis, liver damage, splenomegaly, enteritis, skin suppurative inflammation, neutropenia, lymphopenia, thrombopenia, expression of transaminase, and/or hyperbilirubinemia.

In one embodiment, the anti-CD137 antigen-binding molecules of the present disclosure have low side effects, and thus the dosage can be increased without concerns about side effects, and as a result, they can exhibit stronger drug efficacy (cytotoxic activity or antitumor activity).

In a further aspect, the present disclosure provides pharmaceutical formulations comprising any of the anti-CD137 antigen-binding molecules or antibodies provided herein, e.g., for use in any of the above therapeutic methods. In one embodiment, a pharmaceutical formulation comprises any of the anti-CD137 antigen-binding molecules or antibodies provided herein and a pharmaceutically acceptable carrier.

An antigen-binding molecule or antibody of the present disclosure can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Antibodies of the present disclosure would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The antibody need not be, but is optionally formulated with one or more agents currently used to prevent or treat the disorder in question. The effective amount of such other agents depends on the amount of antibody present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of an antibody of the present disclosure will depend on the type of disease to be treated, the type of antibody, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments. Depending on the type and severity of the disease, about 1 microgram/kg to 15 mg/kg (e.g. 0.1mg/kg-10mg/kg) of antibody can be an initial candidate dosage for administration to the patient, whether, for example, by one or more separate administrations, or by continuous infusion. One typical daily dosage might range from about 1 microgram/kg to 100 mg/kg or more, depending on the factors mentioned above. For repeated administrations over several days or longer, depending on the condition, the treatment would generally be sustained until a desired suppression of disease symptoms occurs. One exemplary dosage of the antibody would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (e.g. such that the patient receives from about two to about twenty, or *e.g.* about six doses of the antibody). An initial higher loading dose, followed by one or more lower doses may be administered. The progress of this therapy is easily monitored by conventional techniques and assays.

It is understood that any of the above formulations or therapeutic methods may be carried out using an immunoconjugate of the present disclosure in place of or in addition to an anti-CD137 antigen-binding molecule or antibody of the present disclosure. The anti-CD137 antigen-binding molecules used are antibodies comprising the amino acid sequences of A375/B167, A372/B040, A356/B040, A486/B167, A487/B167, A488/B226, A489/B223, A548/B376, A551/B256, A551/B379, A555/B379, A548/B256, or A549/B167 shown in Table 37 as a combination of heavy chain variable region/light chain variable region. In a preferred embodiment, the anti-CD 137 antigen-binding molecule is an anti-CD 137 antibody comprising HVR-H1, HVR-H2, HVR-H3, HVR-L1, HVR-L2 and HVR-L3 which have the same amino acid sequences as HVR-H1, HVR-H2, HVR-H3, HVR-L1, HVR-L2 and HVR-L3 contained in A375/B167. In a further embodiment, the anti-CD137 antigen-binding molecule may be an anti-CD137 antibody comprising A375/B167 as a combination of heavy chain variable region/light chain variable region. In a different preferred embodiment, the anti-CD137 antigen-binding molecule is an anti-CD137 antibody comprising HVR-H1 HVR-H2, HVR-H3, HVR-L1, HVR-L2 and HVR-L3 which have the same amino acid sequences as HVR-H1, HVR-H2, HVR-H3, HVR-L1, HVR-L2 and HVR-L3 contained in A551/B379. In a further embodiment, the anti-CD137 antigen-binding molecule may be an anti-CD137 antibody comprising A551/B379 as a combination of heavy chain variable region/light chain variable region. Moreover, the anti-CD137 antibody used may be any one anti-CD 137 antibody selected from the antibodies listed in Table 72.

## H. Articles of Manufacture

In another aspect of the present disclosure, an article of manufacture containing materials useful for the treatment, prevention and/or diagnosis of the disorders described above is provided. The article of manufacture comprises a container and a label on or a package insert associated with the container. Suitable containers include, for example, bottles, vials, syringes, IV solution bags, etc. The containers may be formed from a variety of materials such as glass or plastic. The container holds a composition which is by itself and may have a sterile access port (for example the container may be an intravenous solution bag or a vial having a stopper pierceable by a hypodermic injection needle). At least one active ingredient in the composition is an antibody of the present disclosure. The label or package insert indicates that the composition is used for treating the condition of choice. The article of manufacture in this embodiment of the present disclosure may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

The term "package insert" is used to refer to instructions customarily included in commercial packages of therapeutic products, that contain information about the indications, usage, dosage, administration, combination therapy, contraindications and/or warnings concerning the use of such therapeutic products.

It is understood that any of the above articles of manufacture may include an immunoconjugate of the present disclosure in place of or in addition to an anti-CD 137 antigen-binding molecule or antibody.

III. Combination therapy

A. Combination therapy and therapeutic compositions for combination therapy

The present disclosure relates to methods of treating and/or preventing cancer, which comprise using a combination of an above-mentioned anti-CD 137 antigen-binding molecule and another anticancer agent. In one embodiment, the disclosure relates to pharmaceutical compositions comprising an anti-CD 137 antigen-binding molecule which are for use in combination therapy with another anticancer agent. In a further embodiment, the disclosure relates to pharmaceutical compositions for use in combination therapy with an anti-CD 137 antigen-binding molecule of the present disclosure, which comprise another anticancer agent. The present disclosure relates to methods of treating or preventing a cancer in an individual, and/or activating immunity within a tumor tissue, comprising administering an anti-CD 137 antigen-binding molecule and another anticancer agent. The anti-CD137 antigen-binding molecule and/or other anticancer agent treat cancer and/or exert a synergistic or additive effect of treating cancer by being used in combination.
In some embodiments, the combination therapy of the present disclosure provides a method of enhancing the therapeutic or preventive effect of another anticancer agent in treating or preventing cancer, by using the other anticancer agent together with an anti-CD137 antigen-binding molecule described above. In addition, the combination therapy of the present disclosure provides a method of enhancing the therapeutic or preventive effect of the above-mentioned anti-CD137 antigen-binding molecule when treating cancer, by using the anti-CD137 antigen-binding molecule together with another anticancer agent. Here, the enhancement of the therapeutic or preventive effect means, for example, an increase in the response rate of treatment, a decrease in the amount of anticancer agent administered for treatment, and/or a shortening of the treatment period with the anticancer agent, but are not limited thereto. In addition, the combination therapy of the present disclosure provides a method of increasing progression-free survival in an individual, comprising administering an effective amount of an above-described anti-CD137 antigen-binding molecule and another anticancer agent.
In some embodiments, the combination therapy of the present disclosure includes administration of an anti-CD 137 antigen-binding molecule described above and another anticancer agent. The anti-CD137 antigen-binding molecule and the other anticancer agent can be administered by any suitable method known in the art. For example, the anti-CD137 antigen-binding molecule and the other anticancer agent can be administered in parallel (i.e., simultaneously) or sequentially (i.e., at different times). When the anti-CD137 antigen-binding molecule and the other anticancer agent are sequentially administered (that is, at different times), the administration interval between the anti-CD137 antigen-binding molecule and the other anticancer agent is not particularly limited, and may be set taking the administration route, dosage form, and such factors into account. For example, it is 0 to 168 hours, preferably 0 to 72 hours, preferably 0 to 24 hours, and more preferably 0 to 12 hours, but is not limited thereto.
The above-mentioned anti-CD137 antigen-binding molecule and the other anticancer agent can be co-administered. The anti-CD137 antigen-binding molecule can also be administered intermittently (i.e., at irregular intervals). The anti-CD137 antigen-binding molecule can be administered prior to administration of the other anticancer agent. Alternatively, the anti-CD137 antigen-binding molecule can be administered after the administration of the other anticancer agent.
Furthermore, the other anticancer agent can be administered intermittently (i.e., at irregular intervals). The other anticancer agent can also be administered prior to administration of the anti-CD137 antigen-binding molecule. Alternatively, the other anticancer agent can be administered after administration of the anti-CD137 antigen-binding molecule.
In some embodiments, the anti-CD137 antigen-binding molecules described herein and other known, or herein-described anticancer agents can be used in the above-mentioned combination therapy of an anti-CD137 antigen-binding molecule and another anticancer agent.
In addition to the above-mentioned combination therapy of an anti-CD137 antigen-binding molecule and another anticancer agent, additional therapies can be performed. Therapies added to the combination therapy of the present disclosure may include additional administration of the anti-CD137 antigen-binding molecule and/or other anticancer agent.
As a non-limiting aspect, the present disclosure provides an immune response activator, a cancer therapeutic agent or a cancer preventive agent (hereinafter, pharmaceutical composition and the like) against cancer cells or tumor tissues containing cancer cells, which comprises an anti-CD137 antigen-binding molecule described above, another anticancer agent, or a combination of an anti-CD 137 antigen-binding molecule and another anticancer agent. The pharmaceutical compositions and the like of the present disclosure can be used in combination therapies of the present disclosure. In some embodiments, by using an anti-CD 137 antigen-binding molecule described above in combination with another anticancer agent, the pharmaceutical compositions and the like of the present disclosure have a stronger effect in suppressing cell proliferation, activating immunity against cancer cells or tumor tissues containing cancer cells, or treating or preventing cancer, as compared to treatment using these alone. In addition, by using an above-described anti-CD 137 antigen-binding molecule in combination with another anticancer agent, the pharmaceutical compositions of the present disclosure have a synergistic or additive effect in suppressing cell growth, activating immunity against cancer cells or tumor tissues containing cancer cells, or treating or preventing cancer.

In some embodiments, a pharmaceutical composition or the like "comprising a combination of an anti-CD 137 antigen-binding molecule and another anticancer agent" in the present disclosure refers to a pharmaceutical composition or the like in which an above-mentioned anti-CD 137 antigen-binding molecule and another anticancer agent are combined for simultaneous, separate or sequential administration in treating or preventing a disease. For example, the pharmaceutical compositions and the like of the present disclosure can be provided in the form of a combination drug containing both an anti-CD 137 antigen-binding molecule and another anticancer agent. Further, for example, in the pharmaceutical compositions and the like of the present disclosure, a drug comprising an anti-CD 137 antigen-binding molecule and a drug comprising another anticancer agent may be separately provided, and these drugs may be used simultaneously or sequentially. The disease is not particularly limited, but is preferably cancer.

The present disclosure provides pharmaceutical compositions or the like for use in combination with other anticancer agents, which comprise an above-mentioned anti-CD 137 antigen-binding molecule as an active ingredient. The present disclosure also provides pharmaceutical compositions or the like for use in combination with an above-mentioned anti-CD137 antigen-binding molecule, which comprise another anticancer agent as an active ingredient.

The present disclosure provides pharmaceutical compositions or the like for enhancing the therapeutic effect of another anticancer agent in the treatment of cancer with the other anticancer agent by combining an above-mentioned anti-CD137 antigen-binding molecule with the other anticancer agent. Further, the present disclosure provides pharmaceutical compositions and such for enhancing the therapeutic effect of an anti-CD 137 antigen-binding molecule in the treatment of cancer with the anti-CD 137 antigen-binding molecule, by combining another anticancer agent with the above-mentioned anti-CD 137 antigen-binding molecule.

In some embodiments, the present disclosure provides the use of an anti-CD137 antigen-binding molecule and/or another anticancer agent for producing a pharmaceutical composition or the like mentioned above comprising the anti-CD137 antigen-binding molecule and/or other anticancer agent as an active ingredient(s).

In the present disclosure, the inclusion of an above-mentioned anti-CD 137 antigen-binding molecule and/or another anticancer agent as an active ingredient(s) means that the anti-CD137 antigen-binding molecule and/or other anticancer agent is contained as the main active ingredient(s). Therefore, the content ratio of the anti-CD137 antigen-binding molecule and/or the other anticancer agent is not limited.

In some embodiments, the anti-CD 137 antigen-binding molecules described herein and the other known, or herein-described anticancer agents can be used in the above-mentioned pharmaceutical compositions and the like.

In one non-limiting embodiment, other anticancer agents used in combination with anti-CD137 antigen-binding molecules include nitrogen mustard analogues, alkyl sulfonates, ethylene imines, nitrosoureas, epoxides, other alkylating agents, folic acid analogues, purine analogues, pyrimidine analogues, other metabolic antagonists, Vinca alkaloids or analogues, podophyllotoxin derivatives, camptothecin analogs, colchicine derivatives, taxanes, other plant alkaloids or natural substances, actinomycines, anthracyclines or related substances, other cytotoxic antibiotics, platinum compounds, methylhydrazines, kinase inhibitors, angiogenesis inhibitors, hormones, DNA modifying enzyme inhibitors, immunostimulators, proteosome inhibitors, enzymes, histone deacetylase inhibitors, DNA modifying enzyme inhibitors, cytokine preparations, retinoids, T cell-activating agonist agents, immune checkpoint inhibitors, indoleamine 2,3-dioxygenase (IDO) inhibitors, co-stimulatory molecule activators, natural killer cell activators, T cell-redirecting antigen-binding molecules, antifibrotic agents, regulatory T cell-depleting agents, CD4$^+$ T cell-depleting agents, B cell-depleting agents, natural killer cell-depleting agents, macrophage-depleting agents, monoclonal antibodies, other molecular target drugs, or other anticancer agents, but are not limited thereto.

The "immune checkpoint" in the present disclosure refers to a molecule that is expressed on an immunocompetent cell (including a T cell) and transmits to the immunocompetent cell a signal for inhibiting immune responses by binding to a ligand. Immune checkpoints and their ligands include, for example, PD-1, CTLA-4, TIM3, LAG3, PD-L1, PD-L2, BTNL2, B7-H3, B7-H4, CD48, CD80, 2B4, BTLA, CD160, CD60, CD86, VISTA, or TIGIT molecules, but are not limited to these. In some embodiments, the "immune checkpoint inhibitor" in the present disclosure refers to an agent that inhibits signal transduction by an immune checkpoint by inhibiting the binding of the immune checkpoint to its ligand. Preferably, the immune checkpoint inhibitor is an antigen-binding molecule that binds to the immune checkpoint or its ligand and inhibits signal transduction by the immune checkpoint, and preferably an antibody or antibody fragment.

In one non-limiting embodiment, provided is a pharmaceutical composition in which the above-mentioned other anticancer agent is a chemotherapeutic agent, T cell-activating agonist agent, immune checkpoint inhibitor, T cell-redirecting antigen-binding molecule, antifibrotic agent, angiogenesis inhibitor, regulatory T cell-depleting agent, CD4$^+$ T cell-depleting agent, B cell-depleting agent, natural killer cell-depleting agent, or macrophage-depleting agent.

In one non-limiting embodiment, the chemotherapeutic agent includes, but is not limited to, an antimetabolite, a plant alkaloid, or a platinum compound. Preferable examples of antimetabolites include, but are not limited to, enocitabine, capecitabine, carmofur, gemcitabine, cytarabine, tegafur, tegafur uracil, nelarabine, fluorouracil, fludarabine, pemetrexed, pentostatin, and methotrexate. A particularly preferred example of an antimetabolite is capecitabine. Preferable examples of plant alkaloids include, but are not limited to, irinotecan, etoposide, sobuzoxane docetaxel, nogitecan, paclitaxel, vinorelbine, vincristine, vindesine, and vinblastine. An example of a particularly preferred plant alkaloid includes paclitaxel. Preferable examples of platinum compounds include, but are not limited to, oxaliplatin, carboplatin, cisplatin, and nedaplatin. Examples of particularly preferred platinum compounds include cisplatin, carboplatin, or a combination thereof.

In one non-limiting embodiment, the T cell-activating agonist agent includes, but is not limited to, an agonistic antibody for the TNF receptor superfamily (TNFRSF) or an agonistic antibody for a co-stimulatory molecule. Molecules targeted by "agonistic antibodies for costimulatory molecules" include TMIGD2, HHLA2, ICOS, ICOS ligand, CD28, CD80, CD86 and the like. An example of a preferred co-stimulatory molecule agonistic antibody is an anti-CD28 antibody.

Molecules targeted by "agonistic antibodies for the TNF receptor superfamily" are not particularly limited as long they are factors that activate cells expressing the TNF receptor superfamily (for example, T cells and NK cells), but are preferably, factors belonging to the "TNF superfamily" or "TNF receptor superfamily". As factors belonging to the "TNF superfamily" or "TNF receptor superfamily", ligands with a trimer structure and receptors with a trimer structure to which the ligands bind, which contribute to the activation of various immune cells, are known (Nat. Rev. Immunol., 2012, 12, 339-51). Factors belonging to the TNF superfamily or TNF receptor superfamily include, for example, CD137, CD137L, CD40, CD40L, OX40, OX40L, CD27, CD70, HVEM, LIGHT, RANK, RANKL, CD30, CD153, GITR, GITRL, TNFRSF25, and TL1A. Examples of OX40 agonistic antibodies include MOXR0916, MEDI6469, MEDI0562, MEDI6383, PF-04518600, GSK-3174998 and various known OX40 agonist antibodies. Examples of CD40 agonist antibodies include RG-7876, ADC-1013, SEA-CD40, APX005M, Dacetuzumab and various known CD40 agonist antibodies. Examples of GITR agonistic antibodies include AMG228, AMK-1248, MK-4166, BMS-986156, TRX518 and various known GITR agonist antibodies. Examples of CD27 agonist antibodies include Varlilumab (CAS Registry Number: 1393344-72-3) and various known CD27 agonist antibodies. Examples of CD137 agonist antibodies include Urelumab (CAS Registry Number: 934823-49-1), PF-05082566 and various known CD137 agonist antibodies. The CD137 agonistic antibody may be a bispecific antigen-binding molecule that binds to CD137 (for example, a bispecific antibody that binds to CD137 and a cancer antigen, or a fusion protein or the like that binds to a CD137 ligand (4-1BBL) and a cancer antigen).

In one non-limiting embodiment, examples of immune checkpoint inhibitors include anti-PD-1 antibodies, anti-PD-Ll antibodies, anti-CTLA-4 antibodies, anti-TIM3 antibodies, anti-LAG3 antibodies, or anti-TIGIT antibodies, but are not limited thereto. For example, examples of anti-PD-1 antibodies include Pembrolizumab (CAS Registry Number: 1374853-91-4), Nivolumab (CAS Registry Number: 946414-94-4), MEDI0680, PDR001, BGB-A317, REGN2810, SHR-1210, PF-06801591 and various known anti-PDI antibodies. Examples of anti-PD-Ll antibodies include Atezolizumab (CAS Registry Number: 1380723-44-3), Avelumab (CAS Registry Number: 1537032-82-8), Durvalumab (CAS Registry Number: 1428935-60-7), MDX-1105 and various known anti-PD-Ll antibodies. Examples of anti-CTLA-4 antibodies include Ipilimumab (CAS Registry Number: 477022-00-9), Tremelimumab (CAS Registry Number: 745013-59-6) and various known anti-CTLA-4 antibodies. Examples of anti-TIM3 antibodies include MBG452 and various known anti-TIM3 antibodies. Examples of anti-LAG3 antibodies include BMS-986016, LAG525 and various known anti-LAG3 antibodies. Examples of anti-TIGIT antibodies include Tiragolumab (RG6058) and various known anti-TIGIT antibodies. The immune checkpoint inhibitor may also be a bispecific antigen-binding molecule that binds to (1) an immune checkpoint molecule, and (2) a TNF receptor superfamily (TNFRSF) member or a co-stimulatory molecule. In one embodiment, the combination therapy of the present disclosure may be use of a triple-agent combination of a CD137 antigen-binding molecule, an anti-PD-1 antibody, and an anti-TIGIT antibody, or a triple-agent combination of a CD137 antigen-binding molecule, an anti-PD-Ll antibody, and an anti-TIGIT antibody.

In one embodiment, the anti-CD137 antigen-binding molecule of the present disclosure and an immune checkpoint inhibitor (preferably an anti-PD-Ll antibody), when used in combination, treat cancer and/or exert a synergistic or additive effect in treating cancer, and have transient or minor side effects. Although this is not intended to be bound by a particular theory, the administration of an anti-CD137 antigen-binding molecule to an individual activates immune cells within the tumor tissue of the individual. PD-L1 expression is induced by such activation of immune cells, and PD-L1[+] cells have a role of suppressing the activated immune response. Thus, in the combination therapy of anti-CD 137 antigen-binding molecule and anti-PD-Ll antibody, the action of PD-L1[+] cells is inhibited by the anti-PD-Ll antibody, and a stronger antitumor effect can be exerted. Examples of side effects include, but are not limited to, weight loss and liver dysfunction (increased ASL and/or ALT levels).

In one non-limiting embodiment, the "T cell-redirecting antigen-binding molecule" is a multispecific antigen-binding molecule comprising "a domain containing an antibody variable region having T cell receptor complex-binding activity" and "a domain containing an antibody variable region having cancer antigen-binding activity". It is preferably a multispecific antibody, and more preferably a bispecific antibody. The multispecific (or bispecific) antibody may have a single chain antibody structure, for example, a structure in which antibody variable regions are bound by a linker. The T cell-redirecting antigen-binding molecule may further contain an Fc region in which the binding activity to the Fcγ receptor is reduced.

A "domain containing an antibody variable region having cancer antigen-binding activity" refers to a portion of an antibody comprising a region that specifically binds to a part of a cancer antigen, or to the whole cancer antigen, and is complementary thereto. As used herein, the term "cancer-specific antigen" means an antigen expressed by a cancer cell that makes it possible to distinguish between a cancer cell and a healthy cell, and for example, includes an antigen expressed as the cell becomes malignant, or an abnormal sugar chain that appears on the cell surface and protein molecules when cells become cancerous. Examples are various antigens disclosed herein (e.g., in "II. Compositions and methods (anti-CD 137 agonistic antigen-binding molecules), A. Exemplary anti-CD 137 antigen-binding molecules or antibodies, 6. Multispecific Antibodies"), but are not limited thereto. The cancer-specific antigen targeted by the cancer-specific antigen-binding domain of the present disclosure is particularly preferably one that is expressed on the cell surface, and such antigens include, for example, GPC3, CD19, CD20, EGFR, HER2, EpCAM, EREG, FAP, CEA, DLL3, Claudin-6, and FGFR3.

In one embodiment, the anti-T cell antigen-binding molecule is a multispecific antibody, preferably a bispecific antibody, comprising (1) a domain containing an antibody variable region having a glypican 3-binding activity, (2) a domain containing an antibody variable region having a T cell receptor complex-binding activity, and (3) a domain containing an Fc region in which the binding activity to the Fcγ receptor is reduced. In one embodiment, regarding the antibody L chain variable region contained in the antibody variable region having glypican 3-binding activity and the antibody variable region having T cell receptor complex binding activity, it is preferable to obtain a common L chain capable of imparting binding ability to both of the H chain having binding activity to glypican 3 and the H chain having binding activity to T cell receptor complex, and use this as a common L chain variable region of the above-mentioned bispecific antibody.

In one embodiment, the anti-T cell antigen-binding molecule is ERY974. ERY974 is a bispecific antibody comprising (1) a domain containing an antibody variable region having glypican 3-binding activity, (2) a domain containing an antibody variable region having T cell receptor complex-binding activity, and (3) a domain containing an Fc region in which the binding activity to the Fcγ receptor is reduced, and contains:

- TR01H113, disclosed in WO2016/047722 as a heavy chain variable region on the CD3 side,
- E2702sKsc, disclosed in WO 2016/047722 as a heavy chain constant region on the CD3 side,
- GCH065, disclosed in WO2016/047722 as a heavy chain variable region on the GPC3 side,
- E2704sEpsc, disclosed in WO2016/047722 as a heavy chain constant region on the GPC3 side,
- L0011, disclosed in WO2016/047722 as a common light chain variable region, and
- k0, disclosed in WO 2016/047722 as a common light chain constant region.

The "domain containing an antibody variable region having T cell receptor complex-binding activity" is preferably a domain containing an antibody variable region having T cell receptor-binding activity, and more preferably is a domain that contains an antibody variable region having CD3-binding activity. Further, the above-mentioned "domain containing an antibody variable region" is provided from a variable domain of one or a plurality of antibodies, and preferably, the domain containing an antibody variable region contains an antibody light chain variable region (VL) and an antibody heavy chain variable region (VH). Examples of domains containing such antibody variable regions include various antibody fragments such as "scFv (single chain Fv)", "single chain antibody", "Fv", and "scFv2 (single chain Fv 2)", "Fab" or "F(ab') 2" and the like.

In one non-limiting embodiment, an "antifibrotic drug" is a drug that inhibits or suppresses fibrosis, and examples include, but are not limited to, pirfenidone and nintedanib. Further, the antifibrotic drug may be a hyaluronidase drug such as PEGPH20.

In one non-limiting embodiment, examples of the angiogenesis inhibitor include, but are not limited to, anti-VEGF antibodies and anti-VEGFR2 antibodies. Examples of angiogenesis inhibitors include bevacizumab, ramucirumab, sorafenib, everolimus, temsirolimus, lenvatinib, aflibercept and various known angiogenesis inhibitors.

In one non-limiting embodiment, a "regulatory T cell-depleting agent" is an agent that depletes regulatory T cells, and includes anti-CD4 antibody, anti-CD25 antibody, anti-CCR4 antibody, and anti-CTLA4 antibody, but is not limited thereto.

In one non-limiting embodiment, a "CD4$^+$ cell-depleting agent" is an agent that depletes CD4$^+$ cells, and examples thereof include, but are not limited to, anti-CD4 antibodies.

In one non-limiting embodiment, a "B cell-depleting agent", a "natural killer cell-depleting agent", and a "macrophage-depleting agent" are agents that deplete B cells, natural killer cells, and macrophages, respectively. For example, B cell-depleting agents include, but are not limited to, rituximab and obinutuzumab, and macrophage-depleting agents include, but are not limited to, anti-CSFIR antibodies.

In one non-limiting embodiment, a "monoclonal antibody" includes, for example, anti-TGF beta antibody, anti-latent-TGF beta antibody, anti-IL-8 antibody, and the like, but is not limited thereto.

In some embodiments, the other anticancer agent in the present disclosure may be any anticancer agent without particular limitation as long as, when used in combination with an anti-CD137 antigen-binding molecule of the present disclosure, its therapeutic or preventive effect is enhanced or it enhances the therapeutic or preventive effect of the anti-CD 137 antigen-binding molecule.

In one non-non-limiting embodiment, a combination therapy of the present disclosure may include an anti-CD 137 antigen-binding molecule described above and at least one other therapeutic agent, immunomodulatory agent, cancer therapeutic vaccine, adopted T cell therapy, or the like, but is not limited to these. Suitable cancer therapeutic vaccines include, but are not limited to, whole tumor cell vaccines, tumor antigen vaccines, vector-based vaccines, oncolytic virus vaccines and dendritic cell vaccines. In addition to the above-mentioned therapies, multidisciplinary treatment combined with surgery, radiation therapy, and such may be performed.

In one non-limiting embodiment, a combination therapy of the present disclosure may be conducted by combining an above-mentioned anti-CD 137 antigen-binding molecule and cytokine therapy using a cytokine as an antitumor immune response enhancer. Examples of such cytokines include IL-2, IL-7, IL-12, IL-15, IL-17, IL-18, IL-21, IL-23, IL-27, GM-CSF, IFNα (interferon-a), IFNα-2b, IFNβ, and IFNγ, but are not limited thereto.

In one non-limiting aspect, the present disclosure provides a cell growth suppressor, a cell growth inhibitor, an immune response activator, a cancer therapeutic agent or a cancer preventive agent, which comprises an above-mentioned pharmaceutical composition.

The "individual" to whom an above-mentioned anti-CD 137 antigen-binding molecule and/or another anticancer agent is administered may be a human. In some embodiments, the individual is a patient with cancer cells or tumor tissue comprising cancer cells. Preferred cancer types in the present disclosure include, for example, gastric cancer, head and neck cancer, esophageal cancer, colorectal cancer, lung cancer, mesothelioma, liver cancer, ovarian cancer, breast cancer, colon cancer, kidney cancer, skin cancer, muscle tumor, pancreatic cancer, prostate cancer, testicular cancer, uterine cancer, cholangiocarcinoma, Merkel cell carcinoma, bladder cancer, thyroid cancer, schwannoma, adrenal cancer, anal cancer, central nervous system tumor, neuroendocrine tissue tumor, penile cancer, pleural tumor, salivary gland tumor, vulvar cancer, thymoma, lymphoma, myeloid leukemia, pediatric cancer (Wilms tumor, neuroblastoma, sarcoma, hepatoblastoma, and germ cell tumors), but are not limited thereto. More preferred cancer types include gastric cancer, colorectal cancer, lung cancer, mesothelioma, liver cancer, breast cancer, skin cancer, lymphoma, and myeloid leukemia, but are not limited thereto (Tumori. (2012) 98, 478-484; Tumor Biol. (2015) 36, 4671-4679; Am J Clin Pathol (2008) 130, 224-230; Adv Anat Pathol (2014) 21, 450-460; Med Oncol (2012) 29, 663-669; Clinical Cancer Research (2004) 10, 6612-6621; Appl Immunohistochem Mol Morphol (2009) 17, 40-46; Eur J Pediatr Surg (2015) 25, 138-144; J Clin Pathol (2011) 64, 587-591; Am J Surg Pathol (2006) 30, 1570-1575; Oncology (2007) 73, 389-394; Diagnostic Pathology (2010) 64, 1-6; Diagnostic Pathology (2015) 34 , 1-6; Am J Clin Pathol (2008) 129, 899-906; Virchows Arch (2015) 466, 67-76). More preferable cancer types include gastric cancer, colorectal cancer, lung cancer, mesothelioma, liver cancer, breast cancer, skin cancer, lymphoma, and myeloid leukemia.

In the present disclosure, the individual may be a patient who has received an above-mentioned anti-CD137 antigen-binding molecule and/or any anticancer agent treatment prior to the combination therapy of the anti-CD 137 antigen-binding molecule and another anticancer agent. Alternatively, the individual may be an individual who cannot receive standard therapy or for whom standard therapy is not effective. In some embodiments, the cancer possessed by the individual is in the early or late stages.

A combination therapy comprising an anti-CD 137 antigen-binding molecule of the present disclosure and another anticancer agent, or a pharmaceutical composition for use in the combination therapy, can be used to treat a patient with a cancer refractory to treatment with an immune checkpoint inhibitor. For example, the combination therapy (pharmaceutical composition) of the present disclosure can be used to treat a patient for whom the desired efficacy was not observed by administration of an immune checkpoint inhibitor. A "cancer refractory to an immune checkpoint inhibitor" can also be expressed as "a cancer already treated with an immune checkpoint inhibitor", "a cancer that aggravated during or after treatment with an immune checkpoint inhibitor", "a cancer for which an immune checkpoint inhibitor is ineffective", "a cancer that recurred after treatment with an immune checkpoint inhibitor", "a cancer that is resistant to treatment with an immune checkpoint inhibitor", "a cancer for which treatment with an immune checkpoint inhibitor is inadequately effective", or "a cancer for which treatment with an immune checkpoint inhibitor is unsuccessful". Preferable examples of other anticancer agents contained in the combination therapy (pharmaceutical composition) include, but are not limited to, immune checkpoint inhibitors or T cell-redirecting antigen-binding molecules.

The above-mentioned cancers refractory to treatment with an immune checkpoint inhibitor include cancers with a gene mutation in JAK1, JAK2, and/or B2M (Shin, Daniel Sanghoon et al. "Primary Resistance to PD-1 Blockade Mediated by" JAK1/2 Mutations". Cancer discovery vol. 7,2 (2017): 188-201; or Sade-Feldman, Moshe et al. "Resistance to checkpoint blockade therapy through inactivation of antigen presentation." Nature communications vol. 8,1 1136. 26 Oct. 2017, etc.), but are not limited thereto.

Alternatively, a combination therapy (pharmaceutical composition) of the present disclosure can be used to treat an individual having a cancer refractory to treatment with an anticancer agent of the present disclosure. For example, a combination therapy (pharmaceutical composition) of the present disclosure is used to treat an individual who becomes resistant to an anticancer agent of the present disclosure after administration of the anticancer agent, or an individual for whom the desired efficacy was not observed by the administration of an anticancer agent of the present disclosure. In other words, a cancer that has already been treated with an anticancer agent therapy of the present disclosure can be treated with a combination therapy (pharmaceutical composition) of the present disclosure. Preferable examples of another anticancer agent comprised in the pharmaceutical composition include, but are not limited to, immune checkpoint inhibitors or T cell-redirecting antigen-binding molecules.

In a further aspect, the present disclosure provides a method for activating immune cells in an individual's tumor tissue by a combination therapy described above. In one embodiment, the method comprises administering to an individual an effective amount of an anticancer agent comprising an anti-CD 137 antigen-binding molecule and another anticancer agent. In a further embodiment, in the present disclosure, such immune cells include B cells, dendritic cells, natural killer cells, macrophages, and/or CD8$^+$ T cells (more specifically, these immune cells invading tumor tissues). In this method, when the expression level of at least one gene selected from Cd3e, CD8b1, Gzmb, Prf1 and Ifng is increased in tumor tissue, it can be judged that the activation of T cells in the tumor tissue is enhanced.

In a further aspect, the present disclosure provides a method for activating immune cells in an individual's tumor tissue, particularly CD8$^+$ T cells, or promoting their proliferation by a combination therapy described above. In one embodiment, the method comprises administering to an individual an effective amount of an anticancer agent comprising an anti-CD 137 antigen-binding molecule and another anticancer agent. In the method of activating CD8$^+$ T cells, when there is an increase in the positive rate of at least one CD8$^+$ T cell activation marker selected from OVA tetramer, Granzyme B, PD-1, KLRG-1, and ICOS (positive rate (%) = number of CD8$^+$ T cells positive for the activation marker in tumor tissue/number of CD8$^+$ T cells in tumor tissue X 100), it can be judged that CD8$^+$ T cells in tumor tissue are activated.

It is understood that any of the above-mentioned combination therapies or therapeutic compositions for combination therapy may be conducted using an immuno conjugate of the present disclosure in place of, or in addition to, an anti-CD 137 antigen-binding molecule or antibody of the present disclosure. The anti-CD137 antigen-binding molecule used is an antibody comprising the amino acid sequences of A375/B167, A372/B040, A356/B040, A486/B167, A487/B167, A488/B226, A489/B223, A548/B376, A551/B256, A551/B379, A555/B379, A548/B256, or A549/B167 shown in Table 37 as a combination of heavy chain variable region/light chain variable region. In a preferred embodiment, the anti-CD137 antigen-binding molecule is an anti-CD137 antibody comprising HVR-H1, HVR-H2, HVR-H3, HVR-L1, HVR-L2 and HVR-L3 which have the same amino acid sequences as HVR-H1, HVR-H2, HVR-H3, HVR-L1, HVR-L2 and HVR-L3 contained in A375/B167. In a further embodiment, the anti-CD137 antigen-binding molecule may be an anti-CD 137 antibody comprising A375/B167 as a combination of heavy chain variable region/light chain variable region. In a different preferred embodiment, the anti-CD137 antigen-binding molecule is an anti-CD137 antibody comprising HVR-H1 HVR-H2, HVR-H3, HVR-L1, HVR-L2 and HVR-L3 which have the same amino acid sequences as HVR-H1, HVR-H2, HVR-H3, HVR-L1, HVR-L2 and HVR-L3 contained in A551/B379. In a further embodiment, the anti-CD137 antigen-binding molecule may be an anti-CD137 antibody comprising A551/B379 as a combination of heavy chain variable region/light chain variable region . Moreover, the anti-CD137 antibody used may be any one anti-CD137 antibody selected from the antibodies listed in Table 72.

<u>B. Kit</u>

In another aspect, the present disclosure provides kits for use in the treatment or prevention of a variety of diseases or disorders exemplified by tumors or cancers described in detail in "G. Therapeutic methods and therapeutic compositions" above in which treatment or prevention an anti-CD137 antigen-binding molecule (or immunoconjugate thereof) or a pharmaceutical composition comprising them in the present disclosure is combined with at least one other anticancer agent as described in detail in "A. Combination therapy and therapeutic compositions for combination therapy" above. Such a kit in the present disclosure may be provided, for example, as a kit for treating or preventing cancer (or tumor).

The kit in the present disclosure comprises an anti-CD 137 antigen-binding molecule (or immunoconjugate thereof) in the present disclosure as an active ingredient or a pharmaceutical composition (or pharmaceutical formulation) comprising them, and (2) a package insert or label instructing the administration of at least one other anticancer agent before, at the same time as, or after the administration of the pharmaceutical composition to a patient.

In the kit of the present disclosure, the active ingredient may be filled in a container.

The kit of the present disclosure may include a label on the container or a package insert attached to the container.

The kit of the present disclosure is provided as a commercial package, for example, as a therapeutic product.

The container in the present disclosure preferably includes, for example, a bottle, a vial, a syringe, an IV solution bag, and the like. The container may be made of various materials such as glass and plastic. The container may hold the active ingredient alone or may have a sterile access port (e.g., the container may be a bag or vial containing a solution for intravenous administration with a stopper that can be pierced by a hypodermic injection needle).

The label or package insert in the present disclosure is usually included in a commercial package of the above-mentioned therapeutic product, which is one example of the kit of the present disclosure, and is used to refer to instructions for use of such therapeutic products containing information on the indication, method of use, dosage, administration method, combination therapy, contraindications, and/or warnings. For the combination therapy, for example, it may contain instructions for using an anti-CD 137 antigen-binding molecule (or immunoconjugate thereof) or a pharmaceutical composition (or pharmaceutical formulation) comprising them in the present disclosure in combination with at least one other anticancer agent as described in detail in "A. Combination therapy and therapeutic compositions for combination therapy" above.

The kit of the present disclosure may further comprise a second (or third) container including a pharmaceutically-acceptable buffer such as Bacteriostatic Water For Injection (BWFI), phosphate buffered saline, Ringer's solution, and dextrose solution. The kits of the present disclosure may further include other equipment desired from a commercial point of view or from the user's point of view, such as other buffers, diluents, filters, needles, and syringes.

[Examples]

The following are examples of the methods and compositions of the present disclosure. It is understood that various other aspects may be implemented in the light of the general description above.

Example 1: Evaluation of the antitumor activity of an anti-CD 137 antibody by a syngeneic tumor cell transplantation model using hCD137KI mice (LLC1/OVA/GPC3 clone C5 cells)

(1-1) Preparation of the cell line and syngeneic tumor transplantation mouse model, and evaluation of the antitumor activity

In this study, in-house-established mouse lung cancer cell line LLC1/OVA/GPC3 clone C5 cells (described in Reference Example 9-4-1) and hCD137 KI mice (described in Reference Example 6-3) were used. The LLC1/OVA/GPC3 clone C5 cell line was transplanted subcutaneously into the abdomen of mice who were grouped when the tumor volume reached 164-465 mm$^3$. Grouping was performed 10 days after transplantation. The A551-MB110/B379-ml0r antibody was administered into the tail vein of mice 11 days and 14 days after transplantation as shown in Table 6 below. PBS containing 0.05% Tween 20 was used as the vehicle control.

[Table 6]

| Group | Number of animals | Pharmaceutical agent | Dose (mg/kg) | Day of administration | Route of administration |
|---|---|---|---|---|---|
| 1 | 7 | Vehicle control (PBS with 0.05% Tween 20) | - | 11 days and 14 days after transplantation | via tail vein |
| 2 | 7 | A551-MB110/B379-ml0r | 0.83 | | |
| 3 | 7 | A551-MB110/B379-ml0r | 2.5 | | |
| 4 | 7 | A551-MB110/B379-ml0r | 7.5 | | |

Tumor volume was measured 10 days, 14 days, 17 days, and 21 days after transplantation.

Tumor volume (TV) was calculated by the following formula.

$$\text{Tumor volume (mm}^3) = \text{length (mm) x width (mm) x width (mm)}/2$$

Tumor growth inhibition (TGI (%)) was calculated according to the following formulas.

$$\text{TV change (mm}^3) = \text{tumor volume at the time of measurement - tumor volume at the time of grouping}$$

$$TGI\ (\%) = (1 - (\text{Mean value of TV change for each group/Mean value of TV change for}$$

$$\text{vehicle control group})) \times 100$$

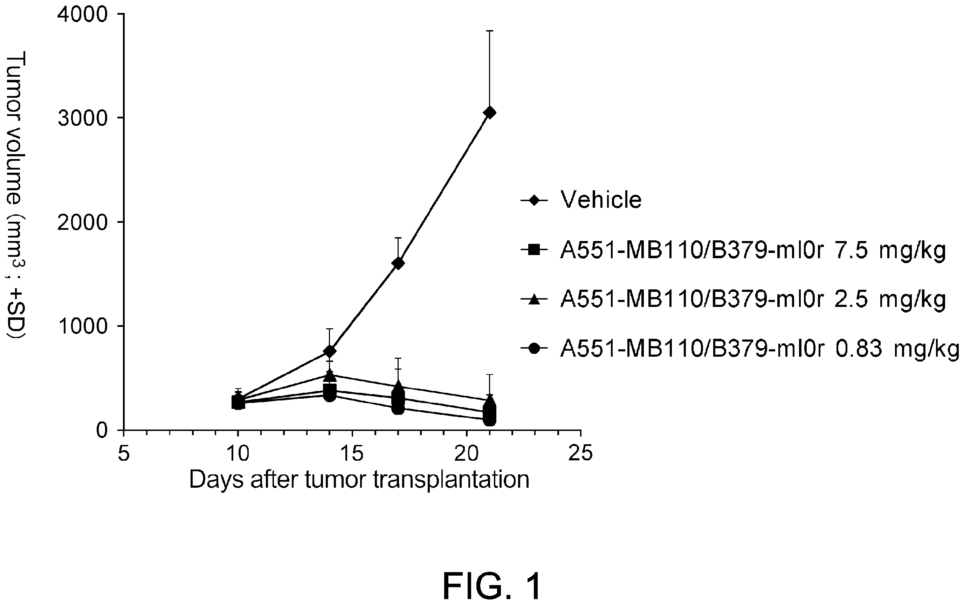

As a result, the A551-MB110/B379-ml0r antibody showed a tumor growth suppression rate of 103, 101, and 106% at 7.5, 2.5, and 0.83 mg/kg against LLC1/OVA/GPC3 clone C5 cells (Figure 1, Table 7). That is, the A551-MB110/B379-ml0r antibody showed comparable high antitumor activity at all doses of this example. One individual in the 2.5 mg/kg administration group had to be euthanized because the general condition deteriorated due to intraperitoneal dissemination of tumor cells 15 days after tumor transplantation.

[Table 7]

| Administered antibody | Dose (mg/kg) | TGI (%) at 21 days after tumor transplantation |
|---|---|---|
| A551-MB110/B379-ml0r | 0.83 | 106 |
| | 2.5 | 101 |
| | 7.5 | 103 |

(1-2) Preparation of the cell line and syngeneic tumor transplantation mouse model, and evaluation of the intratumoral response (gene expression level)

In this study, in-house-established LLC1/OVA/GPC3 clone C5 cells (described in Reference Example 9-4-1) and hCD137 KI mice (described in Reference Example 6-3) were used. The LLC1/OVA/GPC3 clone C5 cell line was transplanted subcutaneously into the abdomen of mice who were grouped when the tumor volume reached 100-180 mm$^3$. Grouping was performed 10 days after transplantation. Vehicle control and the A551-MB110/B379-ml0r antibody were administered into the tail vein of mice 11 days and 14 days after transplantation, as shown in Table 8 below. PBS containing 0.05% Tween 20 was used as the vehicle.

[Table 8]

| Group | Number of animals | Pharmaceutical agent | Dose (mg/kg) | Day of administration | Route of administration |
|---|---|---|---|---|---|
| 1 | 5 | Vehicle control (PBS with 0.05% Tween 20) | - | 11 days and 14 days after transplantation | via tail vein |
| 2 | 5 | A551-MB110/B379-ml0r | 0.83 | | |
| 3 | 5 | A551-MB110/B379-ml0r | 2.5 | | |
| 4 | 5 | A551-MB110/B379-ml0r | 7.5 | | |

The tumors were collected 15 days after transplantation. The collected tumors were immersed in RNA-later (QIAGEN) and stored. Total RNA was extracted from the tumor samples. The gene expression level of total RNA was measured using nCounter Pancancer Mouse Immune Profiling Panel (NanoString Technologies) and nCounter Digital analyzer (NanoString Technologies).

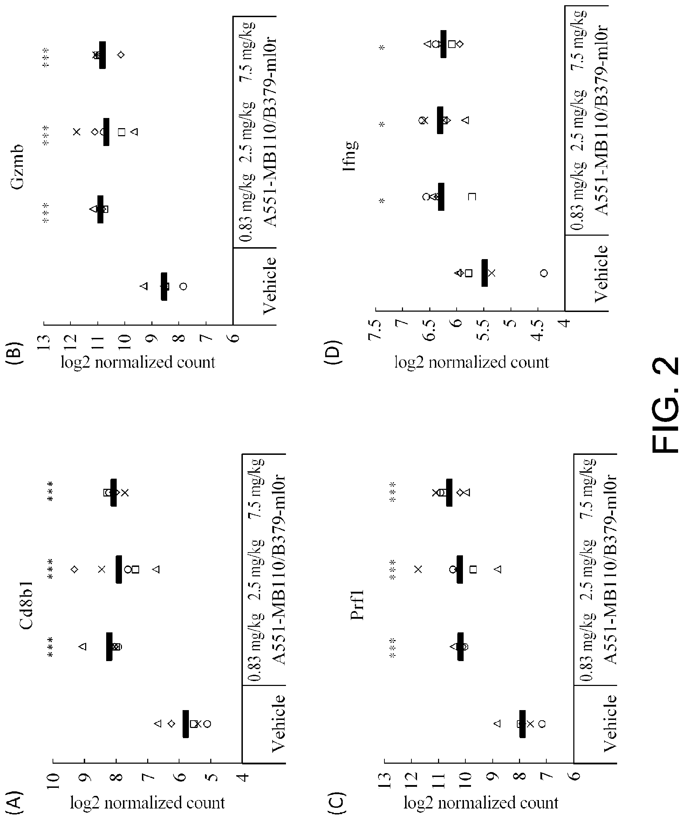

The measured gene expression level was analyzed using nSolver (NanoString Technologies) and Microsoft Excel 2013 (Microsoft corporation). Among the genes whose expression levels were measured, the expression levels of Cd8b1, Gzmb, Prf1, Ifng, which are related to the cytotoxic activity of T cells, were graphed (Figure 2). Compared with the vehicle control group, the group treated with A551-MB110/B379-ml0r alone showed an increase in the expression level of all genes (Dunnett's test, * p <0.05, ** p <0.01, *** p < 0.001)).

From the above, it was shown that under the conditions of this study, A551-MB110/B379-ml0r induces a strong intratumoral reaction to LLC1/OVA/GPC3 clone C5 cancer-bearing human CD137 knock-in mice.

(1-3) Evaluation of the intratumoral action (positive rates of various activation markers in CD8$^+$ T cells)

(1-3-1) Preparation of the cell line and syngeneic tumor transplantation mouse model, and method of evaluating the intratumoral action

In this study, in-house-established mouse lung cancer cell line LLC1/OVA/GPC3 clone C5 cell line (described in Reference Example 9-4-1) and hCD137 KI mice (described in Reference Example 6-3) were used. The LLC1/OVA/GPC3 clone C5 cell line was transplanted subcutaneously into the abdomen of mice who were grouped when the tumor volume reached 69-195 mm$^3$. Grouping was performed 12 days after transplantation. As shown in Table 9 below, A551-MB110/B379-ml0r was administered into the tail vein of mice 13 days and 16 days after transplantation. PBS containing 0.05% Tween 20 was used as the vehicle control.

Four days after the first administration, the tumor tissue was removed and the lymphocyte fraction was prepared by the method described in detail below, and the intratumor reaction was assessed by assessing the positive rate of various activation markers in CD8$^+$ T cells using flow cytometry (FCM). In the evaluation of each of these indicators, it was judged that the activation of T cells in the tumor tissue was enhanced when the indicators showing an intratumoral reaction was higher in the A551-MB110/B379-ml0r-administered group than in the vehicle-administered group.

[Table 9]

| Group | Number of animals | Pharmaceutical agent | Dose [mg/kg] | Day of administration | Day of sampling |
|---|---|---|---|---|---|
| 1 | 4 | Vehicle control (PBS with 0.05% Tween 20) | - | 13 days and 16 days after transplantation | 17 days after transplantation |
| 2 | 4 | A551-MB110/B379-ml0r | 2.5 | 13 days and 16 days after transplantation | 17 days after transplantation |

(1-3-2) Excision of tumor tissue from LLC1/OVA/GPC3 clone C5 cell line-transplanted mice and preparation of lymphocyte fraction

The number of cells in the lymphocyte fractions was measured for the excised tumor tissue. The lymphocyte fractions obtained by using the Tumor Dissociation Kit, mouse (Miltenyi Biotec) were used to measure the number of cells in the lymphocyte fractions.

(1-3-3) Evaluation of activation marker-positive rates in lymphocyte fractions derived from LLC1/OVA/GPC3 clone C5 cell line-transplanted mice by FCM

For evaluation by FCM, the positive rates of various activation markers in CD8$^+$ T cells were used. For this, in the FCM analysis, fluorescent-labeled anti-CD8 antibody (MBL), OVA-tetramer reagent (MBL), anti-Granzyme B antibody (BioLegend), anti-PD-1 antibody (BioLegend), anti-KLRG-1 antibody (BD Biosciences), and anti-ICOS antibody (Invitrogen) were used. Measurements were performed on BD LSRFortessa™ X-20 (BD Biosciences).

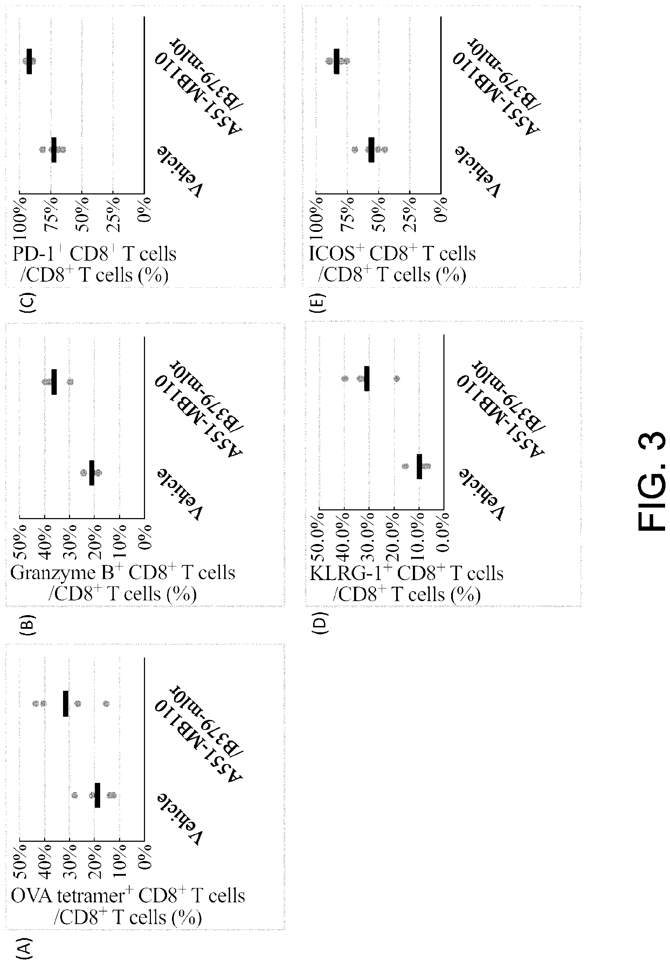

The positive rate evaluation of various activation markers in CD8$^+$ T cells using FCM showed that there was an increase in the positive rate of various activation markers in the tumor tissue in the A551-MB110/B379-ml0r-administered group compared to the vehicle-administered group (Figure 3).

From the above, A551-MB110/B379-ml0r enhanced the activation of CD8$^+$ T cells in the tumor.

Example 2: Evaluation of the antitumor activity of an anti-CD 137 antibody by a syngeneic tumor cell transplantation model using hCD137KI mice (E.G7-OVA cells)

(2-1) Preparation of the cell line and syngeneic tumor transplantation mouse model, and evaluation of the antitumor activity of the A551-MB110/B379-ml0r antibody

In this study, mouse lymphoma cell line E.G7-OVA cells (ATCC, CRL-2113) and hCD137 KI mice (described in Reference Example 6-3) were used. The E.G7-OVA cell line was transplanted subcutaneously into the abdomen of mice who were grouped when the tumor volume reached approximately 200 mm$^3$. Grouping was performed 7 days after transplantation. The A551-MB110/B379-ml0r antibody was administered into the tail vein of mice 7 days and 10 days after transplantation as shown in Table 10 below. PBS containing 0.05% Tween 20 was used as the vehicle control.

[Table 10]

| Group | Number of animals | Pharmaceutical agent | Dose (mg/kg) | Day of administration | Route of administration |
|---|---|---|---|---|---|
| 1 | 10 | Vehicle control (PBS with 0.05% Tween 20) | - | 7 days and 10 days after transplantation | via tail vein |
| 2 | 10 | A551-MB110/B379-ml0r | 0.83 | | |
| 3 | 10 | A551-MB110/B379-ml0r | 2.5 | | |

Tumor volume was measured 7 days, 10 days, 14 days, 17 days, 21 days, 24 days, and 28 days after transplantation.

Tumor volume (TV) was calculated by the following formula.

$$\text{Tumor volume (mm}^3) = \text{length (mm) x width (mm) x width (mm)/2}$$

As a result, in the E.G7-OVA cell transplantation model, the mean tumor volume of the vehicle control group was 2611.8 mm$^3$ 21 days after transplantation. The mean tumor volumes of the A551-MB110/B379-ml0r 0.83 and 2.5 mg/kg groups were 1755.7 mm$^3$ and 1322.1 mm$^3$, respectively. Compared with the vehicle control group, the A551-MB110/B379-ml0r 0.83 and 2.5 mg/kg groups showed significantly lower values 28 days after transplantation (Williams' test). The changes in tumor volume of the vehicle control group and the 2.5 mg/kg group of A551-MB110/B379-ml0r are shown in Figure 4.

(2-2) Preparation of the cell line and syngeneic tumor transplantation mouse model, and the method of evaluating the antitumor activity of the A375-mIgG1/B167-ml0r antibody

In this study, mouse lymphoma cell line E.G7-OVA cells (CRL-2113) from ATCC and hCD137 KI mice (described in Reference Example 6-3) were used. E.G7-OVA cells were transplanted subcutaneously into the abdomen of mice who were grouped when the tumor volume reached approximately 200-400 mm$^3$. Grouping was performed 7 days after transplantation. The A375-mIgG1/B167-ml0r antibody was administered into the tail vein of mice 7 days and 10 days after transplantation as shown in Table 11 below. PBS containing 0.05% Tween 20 was used as the vehicle control.

[Table 11]

| Group | Number of animals | Pharmaceutical agent | Dose (mg/kg) | Day of administration | Route of administration |
|---|---|---|---|---|---|
| 1 | 5 | Vehicle control (PBS with 0.05% Tween 20) | - | 7 days and 10 days after transplantation | via tail vein |
| 2 | 5 | A375-mIgG1/B167-ml0r | 2.5 | | |

Tumor volume was measured 7 days, 10 days, 15 days, 18 days, 21 days, 25 days, 29 days, and 36 days after transplantation.

Tumor volume (TV) was calculated by the following formula.

$$\text{Tumor volume (mm}^3) = \text{length (mm) x width (mm) x width (mm)/2}$$

As a result, the A375-mIgG1/B167-ml0r antibody showed a strong antitumor effect on E.G7-OVA cells, and complete tumor regression was observed in 4 out of 5 mice (Figure 5).

Example 3: Evaluation of the antitumor activity of an anti-CD 137 antibody by a syngeneic tumor cell transplantation model using hCD137KI mice (C1498 cells)

In this study, mouse AML (acute myeloid leukemia) cell line C1498 cells (ATCC, ITEM Number: TIB-49) from ATCC and hCD137 KI mice (described in Reference Example 6-3) were used. The C1498 cell line was transplanted subcutaneously into the abdomen of mice who were grouped when the tumor volume reached approximately 270 mm$^3$. Grouping was performed 7 days after transplantation. The A551-MB110/B379-ml0r antibody was administered into the tail vein of mice 7 days and 10 days after transplantation as shown in Table 12 below. PBS containing 0.05% Tween 20 was used as the vehicle control.

[Table 12]

| Group | Number of animals | Pharmaceutical agent | Dose (mg/kg) | Day of administration | Route of administration |
|---|---|---|---|---|---|
| 1 | 10 | Vehicle control (PBS with 0.05% Tween 20) | - | 7 days and 10 days after transplantation | via tail vein |
| 2 | 10 | A551-MB110/B379-ml0r | 2.5 | | |

Tumor volume was measured 7 days, 10 days, 14 days, 17 days, 21 days, 24 days, and 28 days after transplantation.
Tumor volume (TV) was calculated by the following formula.

$$\text{Tumor volume (mm}^3) = \text{length (mm) x width (mm) x width (mm)/2}$$

As a result, in the C1498 cell transplantation model, the mean tumor volume of the vehicle control group was 3405.3 mm$^3$ 17 days after transplantation. The mean tumor volume in the 2.5 mg/kg group of A551-MB110/B379-ml0r was 2181.3 mm$^3$. Compared with the vehicle control group, the 2.5 mg/kg group of A551-MB1 10/B379-ml0r showed a lower value 17 days after transplantation. The changes in tumor volume of the vehicle control group and the 2.5 mg/kg group of A551-MB110/B379-ml0r are shown in Figure 6.

Example 4: Evaluation of the antitumor activity of an anti-CD 137 antibody by a syngeneic tumor cell transplantation model using hCD137KI mice (Hepa1-6/hGPC3 cells)

(4-1) Preparation of the cell line and syngeneic tumor transplantation mouse model, and evaluation of the antitumor activity of A551-MB110/B379-ml0r antibody

In this study, mouse liver cancer cell line Hepa1-6/hGPC3 cells (Science Translational Medicine 04 Oct 2017, Vol. 9, Issue 410, eaal4291) prepared by introducing the expression plasmid of human Glypican-3 (GPC3) into Hepa1-6 cells (CRL-1830) obtained from ATCC, and hCD137 KI mice (described in Reference Example 6-3) were used. The Hepa1-6/hGPC3 cell line was transplanted subcutaneously into the abdomen of mice who were grouped when the tumor volume reached approximately 200 mm$^3$. Grouping was performed 10 days after transplantation. The A551-MB110/B379-ml0r antibody was administered into the tail vein of mice 10 days and 13 days after transplantation as shown in Table 13 below. PBS containing 0.05% Tween 20 was used as the vehicle control.

[Table 13]

| Group | Number of animals | Pharmaceutical agent | Dose (mg/kg) | Day of administration | Route of administration |
|---|---|---|---|---|---|
| 1 | 10 | Vehicle control (PBS with 0.05% Tween 20) | - | 10 days and 13 days after transplantation | via tail vein |
| 2 | 10 | A551-MB110/B379-ml0r | 2.5 | | |

Tumor volume was measured 10 days, 13 days, 17 days, 20 days, 24 days, 27 days, and 31 days after transplantation.

Tumor volume (TV) was calculated by the following formula.

$$\text{Tumor volume (mm}^3\text{)} = \text{length (mm)} \times \text{width (mm)} \times \text{width (mm)}/2$$

As a result, in the Hepa1-6/hGPC3 cell transplantation model, the mean tumor volume of the vehicle control group was 1525.3 mm$^3$ 31 days after transplantation. The mean tumor volume in the 2.5 mg/kg group of A551-MB110/B379-ml0r was 471.6 mm$^3$. Compared with the vehicle control group, the A551-MB110/B379-ml0r 2.5 mg/kg group showed a lower value 31 days after transplantation. The changes in tumor volume of the vehicle control group and the A551-MB110/B379-ml0r 2.5 mg/kg group are shown in Figure 7.

(4-2) Preparation of the cell line and syngeneic tumor transplantation mouse model, and evaluation of the antitumor activity of A375-mIgG1/B167-ml0r antibody

In this study, mouse liver cancer cell line Hepa1-6/GPC3 cells (Science Translational Medicine 04 Oct 2017, Vol. 9, Issue 410, eaal4291) prepared by introducing the expression plasmid of human Glypican-3 (GPC3) into Hepa1-6 cells (CRL-1830) obtained from ATCC, and hCD137 KI mice (described in Reference Example 6-3) were used. Hepa1-6/GPC3 cells were transplanted subcutaneously into the abdomen of mice who were grouped when the tumor volume reached approximately 200-300 mm$^3$. Grouping was performed 7 days after transplantation. The A375-mIgG1/B167-ml0r antibody was administered into the tail vein of mice 7 days and 10 days after transplantation as shown in Table 14 below. PBS containing 0.05% Tween 20 was used as the vehicle control.

[Table 14]

| Group | Number of animals | Pharmaceutical agent | Dose (mg/kg) | Day of administration | Route of administration |
|---|---|---|---|---|---|
| 1 | 5 | Vehicle control (PBS with 0.05% Tween 20) | - | 7 days and 10 days after transplantation | via tail vein |
| 2 | 5 | A375-mIgG1/B167-ml0r | 7.5 | | |

Tumor volume was measured 7 days, 10 days, 13 days, 18 days, and 20 days after transplantation.

Tumor volume (TV) was calculated by the following formula.

$$\text{Tumor volume (mm}^3\text{)} = \text{length (mm)} \times \text{width (mm)} \times \text{width (mm)}/2$$

As a result, the A375-mIgG1/B167-ml0r antibody showed an antitumor effect on Hepa1-6/GPC3 cells (Figure 8).

Example 5: Evaluation of the effect of various immune cells on the antitumor activity of an anti-CD137 antibody by a syngeneic tumor cell transplantation model using hCD137KI mice (LLC1/OVA/GPC3 clone C5)

In this study, in-house-established mouse lung cancer cell line LLC1/OVA/GPC3 clone C5 cells (described in Reference Example 9-4-1) and hCD137 KI mice (described in Reference Example 6-3) were used. The LLC1/OVA/GPC3 clone C5 cell line was transplanted subcutaneously into the abdomen of mice who were grouped when the tumor volume reached approximately 200-300 mm$^3$. Grouping was performed 9 days after transplantation. The A551-MB110/B379-ml0r antibody was administered into the tail vein of mice 10 days and 14 days after transplantation as shown in Table 15 below. PBS containing 0.05% Tween 20 was used as the vehicle control. Further, agents for depleting various immune cells were administered as shown in Table 16 below.

[Table 15]

| Group | Number of animals | Pharmaceutical agent | Dose (mg/kg) | Day of administration | Route of administration |
|---|---|---|---|---|---|
| 1 | 5 | Vehicle control (PBS with 0.05% Tween 20) | - | 10 days and 14 days after transplantation | via tail vein |
| 2 to 8 | 5 | A551-MB110/B379-ml0r | 2.5 | | |

[Table 16]

| Group | Number of animals | Pharmaceutical agent | Dose | Day of administration | Route of administration |
|---|---|---|---|---|---|
| 3 | 5 | Anti-mouse CD4 antibody (BioXCell, BP0003-1) | 500 μg | 9 days, 13 days, and 17 days after transplantation | |
| 4 | 5 | Anti-mouse CD8α antibody (BioXCell, BP0061) | 500 μg | 9 days, 13 days, and 17 days after transplantation | |
| 5 | 5 | Anti-mouse CD20 antibody (BioLegend, 152104) | 250 μg | 9 days after transplantation | via tail vein |
| 6 | 5 | Anti-mouse NK1.1 antibody (BioXCell, BP0036) | 500 μg | 9 days, 13 days, and 17 days after transplantation | |
| 7 | 5 | Anti-mouse Ly-6G antibody (BioXCell, BP0075-1) | 500 μg | 9 days, 13 days, and 17 days after transplantation | |
| 8 | 5 | Clodronate Liposomes (LIPOSOMA research) | 300 μL | 9 days and 13 days after transplantation | |

Tumor volume was measured 9 days, 14 days, 17 days, and 20 days after transplantation.
Tumor volume (TV) was calculated by the following formula.

$$\text{Tumor volume (mm}^3\text{)} = \text{length (mm)} \times \text{width (mm)} \times \text{width (mm)}/2$$

As a result, the efficacy of the A551-MB110/B379-ml0r antibody against LLC1/OVA/GPC3 clone C5 cells was significantly impaired by depletion of CD8 T cells (Figure 9). From this, it was inferred that the drug efficacy in this model was mainly exerted by CD8 T cells. On the other hand, depletion of CD4 T cells, B cells, NK cells, and macrophages tended to enhance drug efficacy (Figure 9). From this, it was suggested that the combined use of the A551-MB110/B379-ml0r antibody with agents that deplete these immune cells is effective in enhancing drug efficacy.

Example 6: Evaluation of an anti-CD 137 antibody single agent or combination therapy with an anti-PD-Ll antibody by a syngeneic tumor cell transplantation model using hCD137KI mice (MC38 cells)

(6-1) Preparation of the cell line and syngeneic tumor transplantation mouse model, and evaluation of the antitumor activity and body weight changing rate of A551-MB110/B379-ml0r (single agent or combination)

In this study, mouse colon cancer cell line MC38 cells licensed from the U.S. National Cancer Institute and hCD137 KI mice (described in Reference Example 6-3) were used. The MC38 cell line was transplanted subcutaneously into the abdomen of mice, and the model was considered established when the tumor volume reached approximately 100-240 mm$^3$. After the model was established, MC38 cell line-transplanted mice were grouped and then at the doses shown in Table 17, A551-MB110/B379-ml0r and/or anti-mouse PD-L1 antibody (BioXcell, clone 10F.9G2) was/were administered. Administration of A551-MB110/B379-ml0r was performed twice, 15 days and 18 days after transplantation, via the tail vein route. Anti-mouse PD-L1 antibody administration was performed twice, 15 days and 18 days after transplantation, by the intraperitoneal route. PBS containing 0.05% Tween 20 was used for vehicle control.

[Table 17]

| Group | Number of animals | Pharmaceutical agent | Dose [mg/kg] | Day of administration | Route of administration |
|---|---|---|---|---|---|
| 1 | 6 | Vehicle control (PBS with 0.05% Tween 20) | - | 15 days and 18 days after transplantation | via tail vein and intraperitoneal |
| 2 | 6 | A551-MB110/B379-ml0r | 2.5 | | via tail vein |
| | | Vehicle control (PBS with 0.05% Tween 20) | - | | intraperitoneal |
| 3 | 6 | Vehicle control (PBS with 0.05% Tween 20) | - | | via tail vein |
| | | Anti-mouse PD-L1 antibody | 10 | | intraperitoneal |
| 4 | 6 | A551-MB110/B379-ml0r | 2.5 | | via tail vein |
| | | Anti-mouse PD-L1 antibody | 10 | | intraperitoneal |

Tumor volume and body weight were measured 14 days, 18 days, 21 days, and 25 days after transplantation.

Tumor volume (TV) was calculated by the following formula.

$$\text{Tumor volume (mm}^3) = \text{length (mm)} \times \text{width (mm)} \times \text{width (mm)}/2$$

Tumor growth inhibition (TGI (%)) was calculated according to the following formulas.

$$\text{TV change (mm}^3) = \text{tumor volume 25 days after transplantation - tumor volume at the time of grouping}$$

$$\text{TGI (\%)} = (1- (\text{Mean value of TV change for each group/Mean value of TV change for vehicle control group})) \times 100$$

The weight change rate was calculated according to the following formula.

$$\text{Weight change rate (\%)} = 100 \times \text{Weight as of measurement date/Weight as of 14 days after transplantation}$$

As a result, against MC38 cells, an enhancement of the tumor growth suppression effect was observed for the A551-MB110/B379-ml0r and anti-mouse PD-L1 antibody combination group, compared with the A551-MB110/B379-ml0r single agent group or anti-mouse PD-L1 antibody single agent group. Specifically, the tumor growth suppression rate of the A551-MB110/B379-ml0r single agent group was 82%, while that of the A551-MB110/B379-ml0r combination group with the anti-mouse PD-L1 antibody was 117% (Figure 10, Table 18). In all administration groups, a transient weight loss within 6.5% was observed compared to the start of administration, but there was a tendency for recovery thereafter (Figure 11).

[Table 18]

| Tumor growth inhibition at 25 days after transplantation | | | | |
|---|---|---|---|---|
| | Vehicle control (PBS with 0.05% Tween 20) | A551-MB110/B379-ml0r | Anti-mouse PD-L1 antibody | A551-MB110/B379-ml0r + Anti-mouse PD-L1 antibody |
| Tumor growth inhibition (TGI) | - | 82% | 85% | 117% |

(6-2) Drawing a heat map showing changes in the expression of immune-related genes before and after drug administration (particularly in the anti-PD-Ll antibody combination group) based on RNA-seq data of the syngeneic mouse model

The expression value FPKM was calculated by the RSEM method (version 1.2.31) from RNA-seq data of a syngeneic tumor transplantation_mouse model using hCD137 KI mice (described in Reference Example 6-3). The reference mRNA list (gene list) was GRCm38. MC38 was used as the cell line. There were 4 groups: vehicle control, A551-MB110/B379-ml0r-administered group, anti-mouse PD-L1 antibody (BioXcell, clone 10F.9G2)-administered group, A551-MB110/B379-ml0r and anti-mouse PD-L1 antibody combination group.

The unit was converted from [FPKM] to [FPKM + 1] by adding 1 to the expression level per unit of FPKM. This is because log conversion is impossible when 0 exists as the expression value, so a minute value is added. After that, log2 conversion was performed.

Next, human orthologs were assigned to the mouse genes. In principle, they were assigned according to the NCBI HomoloGene dataset. However, although orthology assignment has not been done by NCBI HomoloGene, human CXCL9 and mouse Cxcl9, and human GZMB and mouse Gzmb, which have been orthology assigned on the NCBI gene webpage as of September 2019, were treated as orthologs separately. Next, a group of tumor immunity-related genes (all 54 genes including CD3D, CD8A, etc.) were collected from tumor immunity-related literature. Then, 51 genes excluding genes without mouse orthologs from the 54 genes were selected for examination.

Next, from the GRCm38 gene list (mRNA list) obtained from RNA-seq, only the genes in the above tumor immunology-related gene group were selected. Since RNA-seq is mouse data and the tumor immunity-related gene group is a human gene group, it was converted by ortholog to obtain a mouse gene group. Next, a heat map was drawn using the programming language R program. Here, the heat map was standardized so that the mean of all samples was 0 and the variance was 1 for each gene. The change from the average expression level of each gene was displayed by gradation (Figure 12).

(6-3) Evaluation of the intratumoral action of an anti-CD 137 antibody as single agent or in combination with an anti-PD-Ll antibody (CD8 expression and PD-L1 expression in tumor tissue)

(6-3-1) Preparation of the cell line and syngeneic tumor transplantation mouse model, and method of evaluating the intratumoral action

In this study, mouse colon cancer cell line MC38 cells licensed from the U.S. National Cancer Institute and hCD137 KI mice (described in Reference Example 6-3) were used. The MC38 cell line was transplanted subcutaneously into the abdomen of mice, and the model was considered established when the tumor volume reached approximately 100 mm$^3$. After the model was established, MC38 cell line-transplanted mice were grouped and then at the doses shown in Table 19, A551-MB110/B379-ml0r and an anti-mouse PD-L1 antibody (BioXcell, clone 10F.9G2) were administered. Administration of A551-MB110/B379-ml0r was performed once 14 days after transplantation via the tail vein route. The anti-mouse PD-L1 antibody administration was performed once 14 days after transplantation by the intraperitoneal route. PBS containing 0.05% Tween 20 was used for vehicle control.

The evaluation of intratumoral action was analyzed using the increase in CD8 expression and PD-L1 expression in the tumor tissue as an indicator. CD8 expression and PD-L1 expression were evaluated by immunohistochemical staining. In these evaluations, when the CD8 expression level or the PD-L1 expression level of tumor tissue in the A551-MB110/B379-ml0r single agent group, the anti-mouse PD-L1 antibody single agent group, or the A551-MB110/B379-ml0r and anti-mouse PD-L1 antibody combination group, was higher compared to the vehicle control group, or was higher in the combination group compared to each single agent administration group, it was judged that the number of CD8$^+$ cells or PD-L1 expressing cells have increased.

[Table 19]

| Group | Number of animals | Pharmaceutic al agent | Dose [mg/kg] | Day of administration | Route of administration | Day of sampling |
|---|---|---|---|---|---|---|
| 1 | 3 | Vehicle control | - | 14 days after transplantation | via tail vein and | 16 days after transplantation |
| | | (PBS with 0.05% Tween 20) | | | intraperitoneal | |
| 2 | 3 | A551-MB110/B379-ml0r | 2.5 | 14 days after transplantation | via tail vein | 16 days after transplantation |
| 3 | 3 | Anti-mouse PD-L1 antibody | 10 | 14 days after transplantation | intraperitoneal | 16 days after transplantation |
| 4 | 3 | A551-MB110/B379-ml0r | 2.5 | 14 days after transplantation | via tail vein | 16 days after transplantation |
| | | Anti-mouse PD-L1 antibody | 10 | | intraperitoneal | |

(6-3-2) Excision of tumor tissue from MC38 cell line-transplanted mice and preparation of tumor tissue samples

The excised tumor tissues were immersed in 10% neutral buffered formalin solution for 1 day. The tumor tissues that had undergone paraffin embedding were then sliced and then immunohistochemically stained.

(6-3-3) Immunohistochemical staining of tumor tissue samples from MC38 cell line-transplanted mice and evaluation of the infiltration of CD8+ cells and PD-L1+ cells into the tumor tissue

Anti-CD8α antibody (Cell Signaling Technology, clone D4W2Z) and OptiView DAB kit (Ventana Medical Systems) were used for immunohistochemical staining of CD8. Anti-rabbit-HQ antibody and HRP-labeled anti-HQ antibody were used as the detection secondary antibody and the detection tertiary antibody. Polyclonal anti-PD-Ll antibody (R & D) and UltraView DAB kit (Ventana Medical Systems) were used for immunohistochemical staining of PD-L1. An HRP-labeled anti-goat antibody was used as the detection secondary antibody. BenchMark XT auto-stainer (Ventana Medical Systems) was used for staining.

The results of immunohistochemical staining of CD8 are shown in Figure 13, and the results of immunohistochemical staining of PD-L1 are shown in Figure 14. In the A551-MB110/B379-ml0r and anti-mouse PD-L1 antibody combination group, increased CD8 expression and PD-L1 expression in tumor tissues were observed as compared with the vehicle control administration group and each single agent administration group. In addition, in the single agent administration group of A551-MB110/B379-ml0r and the single agent administration group of anti-mouse PD-L1 antibody, CD8 expression and PD-L1 expression in tumor tissue were increased as compared with the vehicle control administration group.

In addition, high-resolution digital images of all tissues of immunohistochemically-stained specimens were captured using a pathology scanner system for brightfield microscopes (Aperio ScanScope CS2 slide scanner, Leica Biosystems) and an 20x objective lens. For the measurement of immunohistochemical staining-positive cells of CD8 or PD-L1 in tumors, the analysis algorithm "Immune Cell v1.3" or "Membrane v1.7" in HALO image analysis software (Indica Labs, v2.3) was used respectively. Tumor areas in each tissue were manually annotated by a pathologist and evaluated. The number of each of the positive cells was indicated by the number per tumor area ($mm^2$). The quality of the positive threshold by digital image analysis was visually inspected (Figs. 82, 83, and 84).

From the above results, it was shown that the single agent administration of A551-MB110/B379-ml0r and anti-mouse PD-L1 antibody increased CD8+ cells and PD-L1+ cells in tumor tissue. In addition, it was shown that the combined administration of A551-MB110/B379-ml0r and anti-mouse PD-L1 antibody further increased the number of CD8+ cells and PD-L1+ cells in the tumor tissue as compared with the administration of each single agent. Since CD8+ cells have cytotoxic activity against cancer cells, it was thought that a stronger antitumor effect is exhibited when the number of CD8+ cells in the tumor tissue increases. Here, PD-L1 expression is induced by tumor immunity-related genes such as IFN-γ (see Figure 12), and PD-L1+ cells have a role of suppressing an abnormally-activated immune response. As mentioned above, tumor immunity-related genes were shown to be elevated in the A551-MB110/B379-ml0r administration group (Example 6-2, Figure 12). Therefore, it was thought in the combined group of A551-MB110/B379-ml0r and anti-mouse PD-L1 antibody, a stronger anti-tumor effect was obtained because the anti-mouse PD-L1 antibody inhibited the action of PD-L1. It was thought that these events contributed to the strong efficacy of the combined administration shown in Example 6-1.

(6-4) Evaluation of the hepatic and intratumoral actions of an anti-CD 137 antibody as single agent or in combination with an anti-PD-LI antibody

(6-4-1) Preparation of the cell line and syngeneic tumor transplantation mouse model, and method of evaluating the hepatic action and intratumoral action

In this study, mouse colon cancer cell line MC38 cells licensed from the U.S. National Cancer Institute and hCD137 KI mice (described in Reference Example 6-3) were used. The MC38 cell line was transplanted subcutaneously into the abdomen of mice, and the model was considered established when the tumor volume reached approximately 111-200 mm$^3$. After the model was established, MC38 cell line-transplanted mice were grouped and then at the doses shown in Table 20, A551-MB110/B379-ml0r and an anti-mouse PD-L1 antibody (BioXcell, clone 10F.9G2) were administered. Administration of A551-MB110/B379-ml0r was performed twice, 11 days and 14 days after transplantation, via the tail vein route. The anti-mouse PD-L1 antibody administration was performed twice, 11 days and 14 days after transplantation, by the intraperitoneal route. PBS containing 0.05% Tween 20 was used for vehicle control.

Hepatic action was evaluated using blood collected 4 days after the first administration. The resulting blood was centrifuged and plasma was separated. Measurements for various markers in plasma were evaluated by the methods detailed below. In the evaluation of each of these indicators, when indicators showing liver dysfunction was higher in the A551-MB110/B379-ml0r single agent group, the anti-mouse PD-L1 antibody single agent group, or the A551-MB110/B379-ml0r and anti-mouse PD-L1 antibody combination group compared to the vehicle control group, it was evaluated that liver function was damaged.

To evaluate the intratumoral reaction, tumor tissues were removed 4 days after the initial administration by the method described in detail below, and the number of cells in the lymphocyte fractions was measured. In this evaluation, when the CD8$^+$ cell number in tumor tissues was higher in the A551-MB110/B379-ml0r single agent group, the anti-mouse PD-L1 antibody single agent group, or the A551-MB110/B379-ml0r and anti-mouse PD-L1 antibody combination group compared to the vehicle control group, it was evaluated that the proliferation of CD8$^+$ T cells in the tumor tissue was promoted.

[Table 20]

| Group | Number of animals | Pharmaceutical agent | Dose [mg/kg] | Day of administration | Route of administration | Day of sampling |
|---|---|---|---|---|---|---|
| 1 | 5 | Vehicle control (PBS with 0.05% Tween 20) | - | 11 days and 14 days after transplantation | via tail vein and intraperitoneal | 15 days after transplantation |
| 2 | 5 | A551-MB110/ B379-ml0r | 2.5 | | via tail vein | 15 days after transplantation |
| | | Vehicle control (PBS with 0.05% Tween 20) | | | intraperitoneal | |
| 3 | 5 | Vehicle control | - | | via tail vein | 15 days after transplantation |
| | | (PBS with 0.05% Tween 20) | | | | |
| | | Anti-mouse PD-L1 antibody | 10 | | intraperitoneal | |
| 4 | 5 | A551-MB110/ B379-ml0r | 2.5 | | via tail vein | 15 days after transplantation |
| | | Anti-mouse PD-L1 antibody | 10 | | intraperitoneal | |

(6-4-2) Evaluation of liver function marker values by testing blood of MC38 cell line-transplanted mouse

Liver function markers in sampled blood were measured by the sample test system TBA-120FR (Canon Medical Systems).

As a result of this liver function marker measurement by the blood test, no changes in AST and ALT values were observed in any of the A551-MB110/B379-ml0r single agent group, and the anti-mouse PD-L1 antibody single agent administration group, and the A551-MB110/B379-ml0r and anti-mouse PD-L1 antibody combination group as compared with the vehicle-administered group (Figure 15).

From the above, neither A551-MB110/B379-ml0r nor the anti-mouse PD-L1 antibody impaired liver function.

(6-4-3) Excision of tumor tissue from MC38 cell line-transplanted mice and preparation of lymphocyte fractions

The number of cells in the lymphocyte fractions was measured for the excised tumor tissues. The lymphocyte fractions obtained by using the Tumor Dissociation Kit, mouse (Miltenyi Biotec) were used to measure the number of cells in the lymphocyte fractions.

(6-4-4) Evaluation of the lymphocyte fractions derived from MC38 cell line-transplanted mice

As a result of measuring the number of cells in the lymphocyte fractions using FCM, an increase in the number of CD8[+] T cells in the tumor tissue was seen for the A551-MB110/B379-ml0r and anti-mouse PD-L1 antibody combination group compared to the vehicle-administered group (Figure 16).

From the above, the combined use of A551-MB110/B379-ml0r and anti-mouse PD-L1 antibody promoted the proliferation of CD8[+] T cells in the tumor.

Example 7: Evaluation of the antitumor activity of an anti-CD 137 antibody as single agent or in combination with an anti-PD-LI antibody by a syngeneic tumor cell transplantation model using hCD137KI mice (LLC1/OVA/GPC3 clone C5 cells)

In this study, in-house-established mouse lung cancer cell line LLC1/OVA/GPC3 clone C5 cells (described in Reference Example 9-4-1) and hCD137 KI mice (described in Reference Example 6-3) were used. The LLC1/OVA/GPC3 clone C5 cell line was transplanted subcutaneously into mice who were grouped when the tumor volume reached 148-495 mm$^3$. Grouping was performed 11 days after transplantation. Vehicle control and A551-MB110/B379-ml0r were administered into the tail vein of mice 11 days and 14 days after transplantation, as shown in Table 21 below. In addition, vehicle control and anti-mouse PD-L1 antibody (BioXcell, clone: 10F.9G2) were administered intraperitoneally to mice 11 days and 14 days after transplantation as shown in Table 21 below. PBS containing 0.05% Tween 20 was used as the vehicle control.

[Table 21]

| Group | Number of animals | Pharmaceutical agent | Dose (mg/kg) | Day of administration | Route of administration |
|---|---|---|---|---|---|
| 1 | 5 | Vehicle control (PBS with 0.05% Tween 20) | - | | via tail vein and intraperitoneal |
| 2 | 5 | A551-MB110/B379-ml0r | 0.093 | | via tail vein |
| | | Vehicle control (PBS with 0.05% Tween 20) | - | | intraperitoneal |
| 3 | 5 | Vehicle control (PBS with 0.05% Tween 20) | - | 11 days and 14 days after transplantation | via tail vein |
| | | Anti-mouse PD-L1 antibody | 10 | | intraperitoneal |
| 4 | 5 | A551-MB110/B379-ml0r | 0.093 | | via tail vein |
| | | Anti-mouse PD-L1 antibody | 10 | | intraperitoneal |

Tumor volume was measured 11 days, 14 days, 17 days, 20 days, 25 days, 28 days, 31 days, and 35 days after transplantation. Tumor volume (TV) was calculated by the following formula.

$$\text{Tumor volume (mm}^3\text{)} = \text{length (mm) x width (mm) x width (mm)/2}$$

As a result, the mean tumor volume of the A551-MB110/B379-ml0r and anti-mouse PD-L1 antibody combination group 35 days after transplantation was 782 mm³, but the mean tumor volume of the A551-MB110/B379-ml0r single agent group was 1812 mm³ (Table 22). Thus, the A551-MB110/B379-ml0r and anti-mouse PD-L1 antibody combination group showed a stronger tumor growth suppression effect than the A551-MB110/B379-ml0r single agent group or the anti-mouse PD-L1 antibody single agent group. In particular, even though there was almost no tumor growth suppression effect in the anti-mouse PD-L1 antibody single agent group compared with the vehicle control, there was a high tumor growth suppression effect in the A551-MB110/B379-ml0r and anti-mouse PD-L1 antibody combination group (Figure 17). Therefore, this suggested that the combined use of A551-MB110/B379-ml0r and an immune checkpoint inhibitor is effective for cancer types for which treatment with immune checkpoint inhibitors such as PD1 and/or PD-L1 inhibitors is not sufficiently successful.

[Table 22]

| Administered antibody | Dose (mg/kg) | Tumor volume (mm³) at 35 days after tumor transplantation |
|---|---|---|
| A551-MB110/B379-ml0r | 0.093 | 1812 |
| A551-MB110/B379-ml0r<br>Anti-mouse PD-L1 antibody | 0.093<br>10 | 782 |

Example 8-1: Evaluation of the antitumor activity of an anti-CD 137 antibody as single agent or in combination with an anti-PD-LI antibody by a syngeneic tumor cell transplantation model using hCD137KI mice (C1498 cells)

In this study, mouse AML (acute myeloid leukemia) cell line C1498 cells (ATCC, ITEM Number: TIB-49) and hCD137 KI mice (described in Reference Example 6-3) were used. The C1498 cell line was transplanted subcutaneously into mice who were grouped when the tumor volume reached 173-381 mm³. Grouping was performed 8 days after transplantation. Vehicle control, the A375-mIgG1/B167-ml0r antibody, and an anti-mouse PD-L1 antibody (BioXcell, clone 10F.9G2) were administered into the tail vein of mice 8 and 12 days after transplantation, as shown in Table 23 below. PBS containing 0.05% Tween 20 was used as the vehicle control.

[Table 23]

| Group | Number of animals | Pharmaceutical agent | Dose (mg/kg) | Day of administration | Route of administration |
|---|---|---|---|---|---|
| 1 | 5 | Vehicle control (PBS with 0.05% Tween 20) | - | 8 days and 12 days after transplantation | via tail vein |
| 2 | 5 | A375-mIgG1/B167-ml0r | 7.5 | | |
| 3 | 5 | Anti-mouse PD-L1 antibody | 5 | | |
| 4 | 5 | A375-mIgG1/B167-ml0r | 7.5 | | |
| | | Anti-mouse PD-L1 antibody | 5 | | |

Tumor volume (TV) was measured 8 days, 10 days, 12 days, and 15 days after transplantation.
Tumor volume was calculated by the following formula.

$$\text{Tumor volume (mm}^3\text{)} = \text{length (mm) x width (mm) x width (mm)/2}$$

Tumor growth inhibition (TGI (%)) was calculated according to the following formulas.

$$\text{TV change (mm}^3\text{)} = \text{tumor volume at the time of measurement - tumor volume at the time of grouping}$$

$$\text{TGI (\%)} = (1 - (\text{Mean value of TV change for each group/Mean value of TV change for Vehicle control group})) \times 100$$

As a result, against C1498 cells, an enhancement of the tumor growth suppression effect was observed for the A375-mIgG1/B167-ml0r and anti-mouse PD-L1 antibody combination group, compared with the A375-mIgG1/B167-ml0r single agent group or the anti-mouse PD-L1 antibody single agent group. Specifically, the tumor growth suppression rate of the A375-mIgG1/B167-ml0r single agent group was 62%, while that of the A375-mIgG1/B167-ml0r and anti-mouse PD-L1 antibody combination group was 82% (Figure 18, Table 24).

[Table 24]

| Administered antibody | Dose (mg/kg) | TGI (%) at 15 days after tumor transplantation |
|---|---|---|
| A375-mIgG1/B167-ml0r | 7.5 | 62 |
| A375-mIgG1/B167-ml0r<br>Anti-mouse PD-L1 antibody | 7.5<br>5 | 82 |

Example 8-2: Evaluation of the antitumor activity of a single agent switch anti-CD 137 antibody or a combination of the switch anti-CD 137 antibody and an anti-TIGIT antibody by a syngeneic tumor cell transplantation model using hCD137KI mice (C1498 cells)

In this study, mouse AML (Acute Myeloid Leukemia) cell line C1498 cells (ATCC ITEM Number: TIB-49) and hCD137 KI mice (described in Reference Example 6-3) were used. The C1498 cell line was transplanted subcutaneously into the right abdomen of mice, and grouping was performed 7 days after transplantation. Individuals with a tumor volume of approximately 270 to 546 mm$^3$ were used for the grouping. Vehicle and A551-MB110/B379-ml0r antibody were administered into the tail vein of mice 7 days after transplantation. Vehicle and anti-TIGIT antibody (Clone: 1G9; Catalog No: BE0274; BioXcell) were intraperitoneally administered to mice 7 days, 9 days, 11 days, 13 days, 15 days, and 18 days after transplantation, as shown in the table below. PBS containing 0.05% Tween-20 was used as the vehicle.

[Table 86]

| Group | Number of animals | Pharmaceutical agent | Dose or administration amount per animal | Day of administration (post transplantation) | Route of administration |
|---|---|---|---|---|---|
| 1 | 5 | Vehicle control (PBS with 0.05% Tween 20) | - | 7 | via tail vein |
| | | Vehicle | - | 7, 9, 11, 13, 15, 18 | intraperitoneal |
| 2 | 5 | A551-MB110/B379-ml0r | 2.5 mg/kg | 7 | via tail vein |
| | | Vehicle | - | 7, 9, 11, 13, 15, 18 | intraperitoneal |
| 3 | 5 | Vehicle | - | 7 | via tail vein |
| | | Anti-mouse TIGIT antibody | 200 μg/mouse | 7, 9, 11, 13, 15, 18 | intraperitoneal |
| 4 | 5 | A551-MB110/B379-ml0r | 2.5 mg/kg | 7 | via tail vein |
| | | Anti-mouse TIGIT antibody | 200 μg/mouse | 7, 9, 11, 13, 15, 18 | intraperitoneal |

Tumor volume was measured 7 days, 9 days, 11 days, 13 days, 15 days, 17 days, 19 days, 22 days, 25 days, 27 days, 29 days, and 32 days after transplantation. Individuals were euthanized based on in-house euthanasia standards when the tumor volume exceeded 10% of the body weight, converting 1000 mm$^3$ tumor volume to 1 g. For each group, average tumor volume was calculated while all individuals were alive.
The tumor volume was calculated by the following formula.

$$\text{Tumor volume (mm}^3) = \text{length (mm) x width (mm) x width (mm)/2}$$

As a result, a tumor growth-suppresesing effect was observed for both cases of the single agent switch anti-CD 137 antibody A551-MB110/B379-ml0r and the combination of the switch anti-CD 137 antibody A551-MB110/B379-ml0r and the anti-TIGIT antibody.

After 22 days of transplantation, the average tumor volume of the switch anti-CD 137 antibody A551-MB110/B379-ml0r single agent-administered group was 1588 mm$^3$, and the average tumor volume of the switch anti-CD 137 antibody A551-MB110/B379-ml0r and anti-TIGIT antibody combination-administered group was 734 mm$^3$. The tumor growth suppression effect tended to be stronger in the group administered with the combination of the switch anti-CD137 antibody and the anti-TIGIT antibody compared to the switch anti-CD 137 antibody single agent-administered group (Figure 105).

Example 9: Evaluation of the antitumor activity of an anti-CD 137 antibody as single agent or in combination with an anti-PD-LI antibody by a syngeneic tumor cell transplantation model using hCD137KI mice (AE17 cells)

In this study, mouse malignant mesothelioma AE17 cells (DS Pharma Biomedical) and hCD137 KI mice (described in Reference Example 6-3) were used. AE17 tumors passaged *in vivo* were transplanted subcutaneously into mice who were grouped when the tumor volume reached 70-306 mm$^3$. Grouping was performed 9 days after transplantation. Vehicle control, A375-mIgG1/B167-ml0r, and an anti-mouse PD-L1 antibody (BioXcell, clone 10F.9G2) were administered into the tail vein of mice 9 and 12 days after transplantation as shown in Table 25 below. PBS containing 0.05% Tween 20 was used as the vehicle control.

[Table 25]

| Group | Number of animals | Pharmaceutical agent | Dose (mg/kg) | Day of administration | Route of administration |
|---|---|---|---|---|---|
| 1 | 5 | Vehicle control (PBS with 0.05% Tween 20) | - | 9 days and 12 days after transplantation | via tail vein |
| 2 | 5 | A375-mIgG1/B167-ml0r | 7.5 | | |
| 3 | 5 | Anti-mouse PD-L1 antibody | 5 | | |
| 4 | 5 | A375-mIgG1/B167-ml0r | 7.5 | | |
| | | Anti-mouse PD-L1 antibody | 5 | | |

Tumor volume was measured 9 days, 12 days, 15 days, and 19 days after transplantation.

Tumor volume (TV) was calculated by the following formula.

$$\text{Tumor volume (mm}^3) = \text{length (mm) x width (mm) x width (mm)/2}$$

Tumor growth inhibition (TGI (%)) was calculated according to the following formulas.

$$\text{TV change (mm}^3) = \text{tumor volume at the time of measurement - tumor volume at the time of grouping}$$

$$\text{TGI (\%)} = (1- (\text{Mean value of TV change for each group/Mean value of TV change for Vehicle control group})) \times 100$$

As a result, against AE17 cells, an enhancement of the tumor growth suppression effect was observed for the A375-mIgG1/B167-ml0r and anti-mouse PD-L1 antibody combination group, compared with the A375-mIgG1/B167-ml0r single agent group or the anti-mouse PD-L1 antibody single agent group. Specifically, the tumor growth suppression rate of the A375-mIgG1/B167-ml0r single agent group was 39%, while that of the A375-mIgG1/B167-ml0r and anti-mouse PD-L1 antibody combination group was 60% (Figure 19, Table 26).

[Table 26]

| Administered antibody | Dose (mg/kg) | TGI (%) at 19 days after tumor transplantation |
|---|---|---|
| A375-mIgG1/B167-ml0r | 7.5 | 39 |
| A375-mIgG1/B167-ml0r | 7.5 | |
| Anti-mouse PD-L1 antibody | 5 | 60 |

Example 10: Evaluation of an anti-CD 137 antibody as single agent or in combination with an anti-hGPC3-mCD antibody by a syngeneic tumor cell transplantation model using hCD137KI mice (LLC1/hGPC3 cells)

(10-1) Preparation of the cell line and syngeneic tumor transplantation mouse model, and evaluation of the antitumor activity of A551-MB110/B379-ml0r (single agent or combination)

In this study, in-house-established mouse lung cancer cell line LLC1/hGPC3 cells (Science Translational Medicine 04 Oct 2017, Vol. 9, Issue 410, eaal4291) and hCD137 KI mice (described in Reference Example 6-3) were used. The LLC1/hGPC3 cell line was transplanted subcutaneously into the abdomen of mice who were grouped when the tumor volume reached approximately 287 mm$^3$. Grouping was performed 11 days after transplantation. Vehicle control, the A551-MB110/B379-ml0r antibody, and an anti-hGPC3-mCD3 antibody (bispecific antibody that binds to human GPC3 and mouse CD3) were administered into the tail vein of mice 11 days after transplantation, as shown in Table 27 below. PBS containing 0.05% Tween 20 was used as the vehicle control.

[Table 27]

| Group | Number of animals | Pharmaceutical agent | Dose (mg/kg) | Day of administration | Route of administration |
|---|---|---|---|---|---|
| 1 | 5 | Vehicle control (PBS with 0.05% Tween 20) | - | 11 days after transplantation | via tail vein |
| 2 | 5 | A551-MB110/B379-ml0r antibody | 2.5 | | |
| 3 | 5 | Anti-hGPC3-mCD3 antibody | 1.0 | | |
| 4 | 5 | A551-MB110/B379-ml0r antibody | 2.5 | | |
| | | Anti-hGPC3-mCD3 antibody | 1.0 | | |

Tumor volume (TV) was measured 11 days, 14 days, 17 days, and 21 days after transplantation.
Tumor volume was calculated by the following formula.

$$\text{Tumor volume (mm}^3) = \text{length (mm) x width (mm) x width (mm)}/2$$

The results are shown in Figure 20. The mean tumor volume of the vehicle control group was 2393 mm$^3$ 21 days after transplantation. The mean tumor volumes of the anti-hGPC3-mCD3 antibody single agent group, the A551-MB110/B379-ml0r single agent group, and the anti-hGPC3-mCD3 antibody and A551-MB110/B379-ml0r combination group were 1592 mm$^3$, 1340 mm$^3$, and 35 mm$^3$, respectively. Compared with the vehicle control group, the A551-MB110/B379-ml0r single agent group and the anti-hGPC3-mCD3 antibody single agent group both showed lower mean tumor volume. Furthermore, the mean tumor volume was significantly reduced in the anti-hGPC3-mCD3 antibody and A551-MB110/B379-ml0r combination group compared with the A551-MB110/B379-ml0r single agent group and the anti-hGPC3-mCD3 antibody single agent group.
From the above, it was shown that the combined use of an anti-hGPC3-mCD3 antibody and A551-MB110/B379-ml0r synergistically enhances the antitumor effect on LLC1/hGPC3 cancer-bearing human CD137 knock-in mice.

(10-2) Preparation of the cell line and syngeneic tumor transplantation mouse model, and method of evaluating the intratumoral reaction

In this study, in-house-established mouse lung cancer cell line LLC1/hGPC3 cells (Science Translational Medicine 04 Oct 2017, Vol. 9, Issue 410, eaal4291) and hCD137 KI mice (described in Reference Example 6-3) were used. The LLC1/hGPC3 cell line was transplanted subcutaneously into the abdomen of mice who were grouped when the tumor volume reached approximately 230 mm³. Grouping was performed 10 days after transplantation. Vehicle control, the A551-MB110/B379-ml0r antibody, and an anti-hGPC3-mCD3 antibody (bispecific antibody that binds to human GPC3 and mouse CD3) were administered into the tail vein of mice 10 days after transplantation, as shown in Table 28 below. PBS containing 0.05% Tween 20 was used as the vehicle control.

[Table 28]

| Group | Number of animals | Pharmaceutical agent | Dose (mg/kg) | Day of administration | Route of administration |
|---|---|---|---|---|---|
| 1 | 3 | Vehicle control (PBS with 0.05% Tween 20) | - | 10 days after transplantation | via tail vein |
| 2 | 3 | A551-MB110/B379-ml0r antibody | 2.5 | | |
| 3 | 3 | Anti-hGPC3-mCD3 antibody | 1.0 | | |
| 4 | 3 | A551-MB110/B379-ml0r antibody | 2.5 | | |
| | | Anti-hGPC3-mCD3 antibody | 1.0 | | |

The tumor volume used for the above grouping was calculated by the following formula.

$$\text{Tumor volume (mm}^3) = \text{length (mm) x width (mm) x width (mm)/2}$$

The tumors were collected 13 days after transplantation. The collected tumors were immersed in RNA-later (QIAGEN) and stored. Total RNA was extracted from the tumor samples using the RNeasy Mini kit (QIAGEN). The gene expression level of total RNA was measured using nCounter Pancancer Mouse Immune Profiling Panel (NanoString Technologies) and nCounter Digital analyzer (NanoString Technologies).

The measured gene expression level was analyzed using nSolver (NanoString Technologies) and Microsoft Excel 2013 (Microsoft corporation). Among the genes whose expression levels were measured, the expression levels of Cd3e, Cd8b1, Gzmb, Prfl, Ifng, which are related to the cytotoxic activity of T cells, were graphed (Figure 21). Compared with the vehicle control group, the A551-MB110/B379-ml0r and anti-hGPC3-mCD3 antibody combination group showed increased expression levels in the Cd3e, Cd8b1, Gzmb, and Prfl genes (Dunnett's test, * $p <0.05$, ** $p <0.01$, *** $p <0.001$)). Compared with the vehicle group, the expression level of the A551-MB110/B379-ml0r and anti-hGPC3-mCD3 antibody combination group had an increasing tendency even for Ifng. Compared with the A551-MB110/B379-ml0r single agent group and the anti-hGPC3-mCD3 antibody single agent group, the expression levels of the A551-MB110/B379-ml0r and anti-hGPC3-mCD3 antibody combination group had an increasing tendency for all genes. In addition, the expression levels of cytotoxic activity-related genes in each single agent group tended to increase as compared with vehicle.

From the above, it was shown that the combined use of A551-MB110/B379-ml0r and an anti-hGPC3-mCD3 antibody induces a strong intratumoral reaction to LLC1/hGPC3 cancer-bearing human CD137 knock-in mice under the conditions of this study.

Example 11: Evaluation of the antitumor activity of an anti-CD137 antibody by a syngeneic tumor cell transplantation model using hCD137KI mice (breast cancer cell line)

In this study, a generally available or establishable breast cancer cell line and hCD137 KI (knock-in) mice are used. The breast cancer cell line is transplanted into mice who are grouped when the tumor volume reaches a certain level. An anti-CD 137 antibody and vehicle are then administered to the mice.

Tumor volume is measured multiple times after transplantation. When the anti-CD137 antibody-administered group shows a higher tumor growth suppression rate as compared with the vehicle-administered group, it can be evaluated that the anti-CD137 antibody has an antitumor activity against the breast cancer cell line.

Example 12: Evaluation of an anti-CD 137 antibody single agent or combination therapy with anti-PD-LI antibody by a syngeneic tumor cell transplantation model using hCD137KI mice (breast cancer cell line)

In this study, a generally available or establishable breast cancer cell line and hCD137 KI mice are used. Breast cancer cell line is transplanted into mice who are grouped when the tumor volume reaches a certain level. Then, anti-CD137 antibody single agent, anti-PD-LI antibody single agent, anti-CD137 antibody and anti-PD-LI antibody in combination, or vehicle is administered to the mice.

Tumor volume is measured multiple times after transplantation. When the anti-CD137 antibody-administered group shows a higher tumor growth suppression rate as compared with the vehicle-administered group, it can be evaluated that the anti-CD137 antibody has an antitumor activity against the breast cancer cell line. In addition, when the anti-CD137 antibody and anti-PD-L1 antibody combination-administered group shows a higher tumor growth suppression rate than any single agent administration group, it can be evaluated that there was a synergistic or additive enhancement of the tumor growth suppression effect by the combined use.

Example 13: Evaluation of an anti-CD137 antibody single agent or combination therapy with an anti-PD-LI antibody and/or anti-TIGIT antibody by a syngeneic tumor cell transplantation model using hCD137KI mice (breast cancer cell line)

In this study, a generally available or establishable breast cancer cell line and hCD137 KI mice are used. The breast cancer cell line is transplanted into mice who are grouped when the tumor volume reaches a certain level. After that, (1) anti-CD137 antibody single agent, (2) anti-PD-L1 antibody single agent, (3) anti-CD137 antibody and anti-PD-LI antibody two-agent combination, (4) anti-CD137 antibody and anti-TIGIT antibody two-agent combination, (5) anti-CD137 antibody, anti-PD-LI antibody, and anti-TIGIT antibody three-agent combination, or (6) vehicle is administered to mice.

Tumor volume is measured multiple times after transplantation. When the anti-CD137 antibody-administered group shows a higher tumor growth suppression rate as compared with the vehicle-administered group, it can be evaluated that the anti-CD137 antibody has an antitumor activity against the breast cancer cell line. In addition, when the anti-CD137 antibody and anti-PD-LI antibody two-agent combination-administered group, or the anti-CD137 antibody and anti-TIGIT antibody two-agent combination-administered group, has a higher tumor growth suppression rate than their single-agent administration groups, it can be evaluated that there was a synergistic or additive enhancement of the tumor growth suppression effect by the combined use. Furthermore, if the three-agent combination group of anti-CD137 antibody, anti-PD-LI antibody, and anti-TIGIT antibody shows a higher tumor growth suppression rate than the above-mentioned single-agent groups or two-agent combination groups, it can be evaluated that there was a synergistic or additive enhancement of the tumor growth suppression effect by the three-agent combination.

Example 14: Evaluation of an anti-CD137 antibody single agent or combination therapy with an anti-PD-LI antibody and/or anti-TIGIT antibody by a syngeneic tumor cell transplantation model using hCD137KI mice (lung cancer cell line)

In this study, a generally available or establishable lung cancer cell line and hCD137 KI mice were used. Lung cancer cell line is transplanted into mice who are grouped when the tumor volume reaches a certain level. After that, (1) anti-CD137 antibody single agent, (2) anti-PD-LI antibody single agent, (3) anti-CD137 antibody and anti-PD-LI antibody two-agent combination, (4) anti-CD 137 antibody and anti-TIGIT antibody two-agent combination, (5) anti-CD 137 antibody, anti-PD-LI antibody, and anti-TIGIT antibody three-agent combination, or (6) vehicle is administered to mice.

Tumor volume is measured multiple times after transplantation. When the anti-CD137 antibody-administered group shows a higher tumor growth suppression rate as compared with the vehicle-administered group, it can be evaluated that the anti-CD137 antibody has an antitumor activity against the lung cancer cell line. In addition, when the anti-CD137 antibody and anti-PD-LI antibody two-agent combination-administered group, or the anti-CD137 antibody and anti-TIGIT antibody two-agent combination-administered group, has a higher tumor growth suppression rate than these single-agent administration groups, it can be evaluated that there was a synergistic or additive enhancement of the tumor growth suppression effect by the combined use. Furthermore, if the three-agent combination group of anti-CD 137 antibody, anti-PD-LI antibody, and anti-TIGIT antibody shows a higher tumor growth suppression rate than the above-mentioned single-agent groups or two-agent combination groups, it can be evaluated that there was a synergistic or additive enhancement of the tumor growth suppression effect by the three-agent combination.

Example 15: Evaluation of an anti-CD 137 antibody single agent or combination therapy with an anti-PD-LI antibody and/or anti-TIGIT antibody by a syngeneic tumor cell transplantation model using hCD137KI mice (melanoma cell line)

In this study, a generally available or establishable melanoma cell line and hCD137 KI mice are used. The melanoma cell line is transplanted into mice who are grouped when the tumor volume reaches a certain level. After that, (1) anti-CD137 antibody single agent, (2) anti-PD-L1 antibody single agent, (3) anti-CD137 antibody and anti-PD-LI antibody two-agent combination, (4) anti-CD137 antibody and anti-TIGIT antibody two-agent combination, (5) anti-CD137 antibody, anti-PD-LI antibody, and anti-TIGIT antibody three-agent combination, or (6) vehicle is administered to mice.

Tumor volume is measured multiple times after transplantation. When the anti-CD137 antibody-administered group shows a higher tumor growth suppression rate as compared with the vehicle-administered group, it can be evaluated that the anti-CD137 antibody has an antitumor activity against the melanoma cell line. In addition, when the anti-CD137 antibody and anti-PD-LI antibody two-agent combination-administered group, or the anti-CD137 antibody and anti-TIGIT antibody two-agent combination-administered group, has a higher tumor growth suppression rate than these single-agent administered groups, it can be evaluated that there was a synergistic or additive enhancement of the tumor growth suppression effect by the combined use. Furthermore, if the three-agent combination group of anti-CD137 antibody, anti-PD-LI antibody, and anti-TIGIT antibody shows a higher tumor growth suppression rate than the above-mentioned single-agent group or two-agent combination group, it can be evaluated that there was a synergistic or additive enhancement of the tumor growth suppression effect by the three-agent combination.

Example 16: Evaluation of an anti-CD137 antibody single agent or combination therapy with anti-TIGIT antibody by a syngeneic tumor cell transplantation model using hCD137KI mice (colorectal cancer cell line)

In this study, a generally available or establishable colorectal cancer cell line and hCD137 KI mice are used. The colorectal cancer cell line is transplanted into mice who are grouped when the tumor volume reaches a certain level. Then, any one of anti-CD 137 antibody single agent, anti-TIGIT antibody single agent, anti-CD137 antibody and anti-TIGIT antibody in combination, and vehicle is administered to mice.

Tumor volume is measured multiple times after transplantation. When the anti-CD137 antibody-administered group shows a higher tumor growth suppression rate as compared with the vehicle-administered group, it can be evaluated that the anti-CD137 antibody has an antitumor activity against the colorectal cancer cell line. In addition, when the anti-CD137 antibody and anti-TIGIT antibody combination-administered group shows a higher tumor growth suppression rate as compared with any single agent administration group, it can be evaluated that there was a synergistic or additive enhancement of the tumor growth suppression effect by the combined use.

Example 17: Evaluation of an anti-CD 137 antibody single agent or combination therapy with an anti-PD-LI antibody and/or anti-TIGIT antibody by a syngeneic tumor cell transplantation model using hCD137KI mice (JAK1 knockout cancer cell line)

In this study, a generally available or establishable JAK1 knockout cancer cell line and hCD137 KI mice are used. The JAK1 knockout cancer cell line is transplanted into mice who are grouped when the tumor volume reaches a certain level. After that, (1) anti-CD137 antibody single agent, (2) anti-PD-LI antibody single agent, (3) anti-CD137 antibody and anti-PD-LI antibody two-agent combination, (4) anti-CD137 antibody and anti-TIGIT antibody two-agent combination, (5) anti-CD137 antibody, anti-PD-LI antibody, and anti-TIGIT antibody three-agent combination, or (6) vehicle is administered to mice.

Tumor volume is measured multiple times after transplantation. When the anti-CD137 antibody-administered group shows a higher tumor growth suppression rate as compared with the vehicle-administered group, it can be evaluated that the anti-CD137 antibody has an antitumor activity against the JAK1 knockout cancer cell line. In addition, when the anti-CD137 antibody and anti-PD-LI antibody two-agent combination-administered group, or the anti-CD137 antibody and anti-TIGIT antibody two-agent combination-administered group, shows a higher tumor growth suppression rate than these single-agent administration groups, it can be evaluated that there was a synergistic or additive enhancement of the tumor growth suppression effect by the combined use. Furthermore, if the three-agent combination group of anti-CD137 antibody, anti-PD-L1 antibody, and anti-TIGIT antibody shows a higher tumor growth suppression rate than the above-mentioned single-agent combination groups or two-agent combination groups, it can be evaluated that there was a synergistic or additive enhancement of the tumor growth suppression effect by the three-agent combination.

Example 18-1: Evaluation of an anti-CD 137 antibody single agent or combination therapy with an anti-PD-LI antibody and/or anti-TIGIT antibody by a syngeneic tumor cell transplantation model using hCD137KI mice (B2M knockout cancer cell line)

In this study, a generally available or establishable B2M knockout cancer cell line and hCD137 KI mice are used. The B2M knockout cancer cell line is transplanted into mice who are grouped when the tumor volume reaches a certain level. After that, (1) anti-CD137 antibody single agent, (2) anti-PD-LI antibody single agent, (3) anti-CD137 antibody and anti-PD-LI antibody two-agent combination, (4) anti-CD137 antibody and anti-TIGIT antibody two-agent combination, (5) anti-CD137 antibody, anti-PD-LI antibody, and anti-TIGIT antibody three-agent combination, or (6) vehicle is administered to mice.

Tumor volume is measured multiple times after transplantation. When the anti-CD137 antibody-administered group shows a higher tumor growth suppression rate as compared with the vehicle-administered group, it can be evaluated that the anti-CD137 antibody has an antitumor activity against the B2M knockout cancer cell line. In addition, when the anti-CD137 antibody and anti-PD-LI antibody two-agent combination-administered group, or the anti-CD137 antibody and anti-TIGIT antibody two-agent combination-administered group, shows a higher tumor growth suppression rate than these single-agent administration groups, it can be evaluated that there was a synergistic or additive enhancement of the tumor growth suppression effect by the combined use. Furthermore, if the three-agent combination group of anti-CD137 antibody, anti-PD-L1 antibody, and anti-TIGIT antibody shows a higher tumor growth suppression rate than the above-mentioned single-agent combination groups or two-agent combination groups, it can be evaluated that there was a synergistic or additive enhancement of the tumor growth suppression effect by the three-agent combination.

Example 18-2: Evaluation of the antitumor activity of a switch anti-CD137 antibody by a syngeneic tumor cell transplantation model using hCD137KI mice

(18-2-1) Preparation of the tumor cell line and syngeneic tumor transplantation mouse model, and method of evaluating the antitumor activity

Tumors with a mutation in the $\beta$2 microglobulin (B2M) gene (b2m mutation) are known to be resistant to treatment with anti-PD-1 antibodies, anti-PD-LI antibodies, etc. (Sharma P., et al., Cell. 168, (9), 707-23 2017; and Zaretsky JM. et al., N Engl J Med. 375 (9): 819-29. 2016).

In this study, in-house-established mouse colon cancer cell line MC38-hGPC3#G64 B2M KO clone 5 cells with the $\beta$2 microglobulin (B2M) gene knocked out and hCD137 KI mice (described in Reference Example 6-3) were used. The MC38-hGPC3#G64 B2M KO clone 5 cell line was transplanted subcutaneously into the right abdomen of mice, and grouping was performed 14 days after transplantation. Individuals with a tumor volume of 111 to 228 mm$^3$ were used for the grouping. Switch anti-CD 137 antibody A551-MB110/B379-ml0r was administered into the tail vein of mice 14 days and 21 days after transplantation. PBS containing 0.05% Tween-20 was used as the vehicle.

[Table 87]

| Group | Number of animals | Pharmaceutical agent | Dose (mg/kg) | Day of administration | Route of administration |
|---|---|---|---|---|---|
| 1 | 5 | Vehicle control (PBS with 0.05% Tween 20) | - | 14 days and 21 days after transplantation | via tail vein |
| 2 | 5 | A551-MB110/B379-ml0r | 2.5 | | |

Tumor volume was measured 14 days, 17 days, 21 days, 24 days, 27 days, and 31 days after transplantation.

The tumor volume was calculated by the following formula.

$$\text{Tumor volume (mm}^3\text{)} = \text{length (mm) x width (mm) x width (mm)/2}$$

Tumor growth inhibition (TGI (%)) was calculated according to the following formulas.

$$\text{TV change (mm}^3\text{)} = \text{tumor volume at the time of measurement - tumor volume at the time of grouping}$$

$$\text{TGI (\%)} = (1 - (\text{Mean value of TV change for each group/Mean value of TV change for Vehicle group})) \times 100$$

As a result, the switch anti-CD137 antibody A551-MB110/B379-ml0r showed a tumor growth suppression effect on MC38-hGPC3#G64 B2M KO clone 5 cells over the entire period after tumor transplantation as compared with the control, and showed a tumor growth suppression rate of 29% (Figure 106) 31 days after tumor transplantation.

The CD137 switch antibody of the present invention was shown to have an extremely significant antitumor activity against tumors having a b2m mutation, which are known to be resistant to treatment with anti-PD-1 antibodies, anti-PD-LI antibodies, and the like.

Example 19: Evaluation of the antitumor activity of an anti-CD 137 antibody as single agent or in combination with an anti-PD-LI antibody by a syngeneic tumor cell transplantation model using hCD137KI mice (MC38 cells)

(19-1) Preparation of the tumor cell line and syngeneic tumor transplantation mouse model, and evaluation of the antitumor activity of A551-MB110/B379-ml0r and UreH-MB110/UreL-mk1 (single agent or in combination with an anti-mouse PD-L1 antibody)

In this study, mouse colon cancer cell line MC38 cells licensed from the U.S. National Cancer Institute and hCD137 KI mice (described in Reference Example 6-3) were used. The MC38 cell line was transplanted subcutaneously into the abdomen of mice, and the model was considered established when the tumor volume reached 106-200 mm$^3$. After the model was established, MC38 cell line-transplanted mice were grouped and then at the doses shown in Table 77, switch antibody A551-MB110/B379-ml0r or non-switch antibody UreH-MB110/UreL-mk1 (positive control), and/or anti-mouse PD-L1 antibody (BioXcell, clone 10F.9G2) was/were administered. Administration of the switch antibody A551-MB110/B379-ml0r or the non-switch antibody UreH-MB110/UreL-mk1 was performed twice, 11 days and 14 days after transplantation, by the tail vein route. Anti-mouse PD-L1 antibody administration was performed twice, 11 days and 14 days after transplantation, by the intraperitoneal route. PBS containing 0.05% Tween 20 was used for vehicle control.

The non-switch antibody UreH-MB110/UreL-mk1 used as positive control is a non-switch CD137 antibody that has the variable region of the known anti-CD 137 antibody Urelumab (CAS Registry Number: 934823-49-1) as the variable region, and MB110 (SEQ ID NO: 145) described in WO2014030750 as the heavy chain constant region, and mouse κ chain mk1 (same amino acid sequence as mouse κ chain mk0 (SEQ ID NO: 147)) as the light chain constant region.

[Table 77]

| Group | Number of animals | Pharmaceutical agent | Dose (mg/kg) | Day of administration | Route of administration |
|---|---|---|---|---|---|
| 1 | 8 | Vehicle control (PBS with 0.05% Tween 20) | - | 11 days and 14 days after transplantation | via tail vein |
| | | Vehicle | - | | intraperitoneal |
| 2 | 8 | A551-MB110/B379-ml0r | 2.5 | | via tail vein |
| | | Vehicle | - | | intraperitoneal |
| 3 | 8 | UreH-MB110/UreL-mk1 | 7.5 | | via tail vein |
| | | Vehicle | - | | intraperitoneal |
| 4 | 8 | Vehicle | - | | via tail vein |
| | | Anti-mouse PD-L1 antibody | 10 | | intraperitoneal |

(continued)

| Group | Number of animals | Pharmaceutical agent | Dose (mg/kg) | Day of administration | Route of administration |
|---|---|---|---|---|---|
| 5 | 8 | A551-MB110/B379-ml0r | 2.5 | | via tail vein |
| | | Anti-mouse PD-L1 antibody | 10 | | intraperitoneal |
| 6 | 8 | UreH-MB110/UreL-mk1 | 7.5 | | via tail vein |
| | | Anti-mouse PD-L1 antibody | 10 | | intraperitoneal |

Tumor volume and body weight were measured 10 days, 14 days, 18 days, 22 days, 25 days, 29 days, and 32 days after transplantation.

Tumor volume (TV) was calculated by the following formula.

$$\text{Tumor volume (mm}^3) = \text{length (mm) x width (mm) x width (mm)}/2$$

Tumor growth inhibition (TGI (%)) was calculated according to the following formulas.

$$\text{TV change (mm}^3) = \text{Tumor volume 32 days after transplantation - Tumor volume at the time of grouping}$$

$$\text{TGI (\%)} = (1- (\text{Mean value of TV change for each group/Mean value of TV change for vehicle control group})) \times 100$$

As a result, against MC38 cells, the switch antibody A551-MB110/B379-ml0r single agent-administered group, the switch antibody A551-MB110/B379-ml0r and anti-mouse PD-L1 antibody combination group, and the non-switch antibody UreH-MB110/UreL-mk1 and anti-mouse PD-L1 antibody combination group showed a significant tumor growth suppression effect compared to the vehicle group, and the tumor growth suppression rates were 78%, 109%, and 81%, respectively (Figure 85, Table 78). Therefore, it was shown that the switch antibody A551-MB110/B379-ml0r and anti-mouse PD-L1 antibody combination group has the same or higher antitumor effect as the non-switch antibody UreH-MB110/UreL-mk1 and anti-mouse PD-L1 antibody combination group.

[Table 78]

| Tumor growth inhibition at 32 days after transplantation | | | | | | |
|---|---|---|---|---|---|---|
| | Vehicle control (PBS with 0.05% Tween 20) | A551-MB110/B379-ml0r | UreH-MB110/UreL-mk1 | Anti-mouse PD-L1 antibody | A551-MB110/B379-ml0r+ Anti-mouse PD-L1 antibody | UreH-MB110/UreL-mk1 + Anti-mouse PD-L1 antibody |
| Tumor growth inhibition (TGI) | - | 78 | 77 | 63 | 109 | 81 |

Example 20: Evaluation of the systemic effect of an anti-CD137 antibody as single agent or in combination with an anti-PD-Ll antibody by a syngeneic tumor cell transplantation model using hCD137KI mice

(20-1) Preparation of the tumor cell line and syngeneic tumor transplantation mouse model, and method of evaluating the intratumoral action

In this study, mouse colon cancer cell line MC38 cells licensed from the U.S. National Cancer Institute and hCD137 KI mice (described in Reference Example 6-3) were used. The MC38 cell line was transplanted subcutaneously into the abdomen of mice, and the model was considered established when the tumor volume reached approximately 200 mm$^3$. After the model was established, MC38 cell line-transplanted mice were grouped and then at the dose shown in Table 79, switch antibody A551-MB110/B379-ml0r and anti-mouse PD-L1 antibody (BioXcell, clone 10F.9G2) were administered. As a positive control substance, the non-switch anti-CD137 antibody UreH-MB110/UreL-mk1 (above) and the anti-mouse PD-L1 antibody were administered.

Administration of the switch antibody A551-MB110/B379-ml0r or the non-switch antibody UreH-MB110/UreL-mk1 was performed twice, 15 days and 18 days after transplantation, from the tail vein route. Anti-mouse PD-L1 antibody administration was performed twice, 15 days and 18 days after transplantation, by the intraperitoneal route. PBS containing 0.05% Tween 20 was used for vehicle control.

(20-2) Excision of spleen, tumor regional lymph nodes (DLN), and liver, lymphocyte fraction preparation and peripheral blood sampling from MC38 cell line-transplanted mice

Peripheral blood was collected from the inferior vena cava for blood sampling. Lymphocyte fraction isolation was performed using the resected spleen, tumor regional lymph nodes, and liver. For the resected spleen and tumor regional lymph nodes, organ weight was measured before conducting lymphocyte fraction isolation. For the spleen and tumor regional lymph nodes, a lymphocyte fraction obtained by using a cell strainer after tissue disruption was used. For the liver, the lymphocyte fraction obtained by tissue disruption using the Liver Dissociation Kit, mouse (Miltenyi Biotec) and then using a cell strainer was used. The obtained peripheral blood was analyzed by a blood cell analyzer, and the obtained spleen, tumor regional lymph nodes, and liver-derived lymphocyte fractions were analyzed by flow cytometry (FCM).

[Table 79]

| Group | Number of animals | Pharmaceutical agent | Dose [mg/kg] | Day of administration | Route of administration | Day of sampling |
|---|---|---|---|---|---|---|
| 1 | 5 | Vehicle control (PBS with 0.5% Tween 20) | - | 15 days and 18 days after transplantation | via tail vein and intraperitoneal | 19 days after transplantation |
| 2 | 5 | A551-MB110/B379-ml0r | 2.5 | | via tail vein | |
| 3 | 5 | UreH-MB110/UreL-mk1 | 7.5 | | via tail vein | |
| 4 | 5 | Anti-mouse PD-L1 antibody | 10 | | intraperitoneal | |
| 5 | 5 | A551-MB110/B379-ml0r | 2.5 | | via tail vein | |
| | | Anti-mouse PD-L1 antibody | 10 | | intraperitoneal | |
| 6 | 5 | UreH-MB110/UreL-mk1 | 7.5 | | via tail vein | |
| | | Anti-mouse PD-L1 antibody | 10 | | intraperitoneal | |

(20-3) Evaluation of blood parameter values by a blood test of MC38 cell line-transplanted mice

Blood parameters were measured with the sample test system XT-2000iV (Sysmex corp.) for the sampled blood.

Blood parameter measurement by the blood test revealed no change in leukocyte concentration, platelet concentration, and lymphocyte concentration in the A551-MB110/B379-ml0r single agent group. In the anti-mouse PD-L1 antibody single agent group, minor fluctuations in leukocyte concentration and lymphocyte concentration were observed, whereas in the A551-MB110/B379-ml0r and anti-mouse PD-L1 antibody combination group, minor fluctuations in leukocyte concentration and lymphocyte concentration similar to those in the anti-mouse PD-L1 antibody single agent group were observed (Figure 86).

On the other hand, in the UreH-MB110/UreL-mk1 single agent group, a significant decrease in leukocyte concentration and lymphocyte concentration was observed (Figure 86). Furthermore, in the UreH-MB110/UreL-mk1 and anti-mouse PD-L1 antibody combination group, a decrease in platelet concentration was observed in addition to a significant decrease in leukocyte concentration and lymphocyte concentration (Figure 86).

From the above, compared with UreH-MB110/UreL-mk1, A551-MB110/B379-ml0r did not cause fluctuations in blood parameters even when used in combination with an anti-mouse PD-L1 antibody, and its effect on peripheral blood was extremely limited.

(20-4) Evaluation of immune cell activation by organ weight measurement and flow cytometry (FCM) analysis using MC38 cell line-transplanted mice

Organ weight evaluation of spleen and tumor regional lymph nodes (DLN) showed no increase in each organ weight in the A551-MB110/B379-ml0r single agent group, anti-mouse PD-L1 antibody single agent group, and A551-MB110/B379-ml0r and anti-mouse PD-L1 combination group (Figure 87).

On the other hand, in the UreH-MB110/UreL-mk1 single agent group, an increase in organ weight of each organ was observed. In addition, in the UreH-MB110/UreL-mk1 and anti-mouse PD-L1 antibody combination group, a more remarkable increase in organ weight of each organ was observed as compared with the UreH-MB110/UreL-mk1 single agent group (Figure 87).

Next, activation of T cells derived from spleen, tumor regional lymph node, and liver was analyzed using FCM. In the evaluation of immune cell activation by FCM, the expression rate of activation markers in CD8$^+$ T cells, the increase rate of Foxp3$^+$ regulatory T cells in CD4$^+$ T cells, and the increase rate of Foxp3$^+$ regulatory T cells in CD45$^+$ cells were used. KLRG-1, ICOS, PD-1, and LAG-3 were used as activation markers in CD8$^+$ T cells. In the FCM analysis, anti-CD45 antibody (BD Biosciences), anti-CD3 antibody (BioLegend), anti-CD8 antibody (BioLegend), anti-CD4 antibody (BioLegend), anti-KLRG-1 antibody (BD Biosciences), anti-ICOS antibody (BioLegend), anti PD-1 antibody (BioLegend), anti-LAG-3 antibody (BD Biosciences), and anti-Foxp3 antibody (BD Biosciences) were used. BD LSR Fortessa X-20 (BD Biosciences) was used for the measurement.

As a result of evaluation in the spleen, there was no remarkable increase in the expression rate of KLRG-1, ICOS, PD-1, LAG-3 in CD8$^+$ T cells, the ratio of each activation marker-positive CD8$^+$ T cells in CD45$^+$ cells, as well as the ratio of Foxp3$^+$ regulatory T cells in CD4$^+$ T cells and the ratio of Foxp3$^+$ regulatory T cells in CD45$^+$ cells, in the A551-MB110/B379-ml0r and anti-mouse PD-L1 antibody combination group compared to the UreH-MB110/UreL-mk1 and anti-mouse PD-L1 antibody combination group (Figures 88 and 89).

As a result of evaluation in tumor regional lymph nodes, there was no remarkable increase in the expression rate and absolute number of KLRG-1, ICOS, PD-1, LAG-3 in CD8$^+$ T cells, and the ratio and absolute number of Foxp3$^+$ regulatory T cells in CD4$^+$ T cells, in the A551-MB110/B379-ml0r and anti-mouse PD-L1 antibody combination group compared with the UreH-MB110/UreL-mk1 and anti-mouse PD-L1 antibody combination group (Figures 90 and 91).

As a result of evaluation in the liver, there was no remarkable increase in the expression rate of KLRG-1, ICOS, PD-1, LAG-3 in CD8$^+$ T cells, the ratio of each activation marker-positive CD8$^+$ T cells in CD45$^+$ cells, and the ratio of Foxp3$^+$ regulatory T cells in CD4$^+$ T cells and the ratio of Foxp3$^+$ regulatory T cells in CD45$^+$ cells in the A551-MB110/B379-ml0r and anti-mouse PD-L1 antibody combination group compared to the UreH-MB110/UreL-mk1 and anti-mouse PD-L1 antibody combination group (Figures 92 and 93)

Based on the above, even though the switch antibody A551-MB110/B379-ml0r shows comparable tumor growth suppression effect to the non-switch antibody UreH-MB110/UreL-mk1 even when used in combination with the anti-mouse PD-L1 antibody, its effect on immune cells in normal organs such as peripheral blood, spleen, tumor regional lymph nodes, and liver was extremely limited.

Example 21: Tissue distribution evaluation of an anti-CD137 antibody

(21-1) Preparation of the tumor cell line and syngeneic tumor transplantation mouse model, and method of evaluating the antibody tissue distribution

In this study, mouse colon cancer cell line MC38 cells licensed from the U.S. National Cancer Institute and hCD137 KI mice (described in Reference Example 6-3) were used. The MC38 cell line was transplanted subcutaneously into the abdomen of mice, and the model was considered established when the tumor volume reached approximately 200 mm$^3$. After the model was established, MC38 cell line-transplanted mice were grouped and then at the doses shown in Table 80, Alexa488 labeled A551-MB110/B379-ml0r was administered. As a negative control substance, Alexa 488-labeled IC17HdK-MB110/IC17L-mk was administered. As a positive control substance, a non-switch anti-CD 137 antibody Alexa488-labeled UreH-MB110/UreL-mk1 was administered. For Alexa488 labeling to each antibody, the Alexa Fluor 488 Antibody Labeling Kit (Thermo Fisher Scientific) was used. Administration of Alexa488-labeled IC17HdK-MB110/IC17L-mk or Alexa488-labeled A551-MB110/B379-ml0r or Alexa488-labeled UreH-MB110/UreL-mk1 was performed once 14 days after transplantation by the tail vein route.

(21-2) Excision of tumor, spleen, and liver from MC38 cell line-transplanted mice and preparation of lymphocyte fractions

Lymphocyte fraction isolation was performed on the resected tumor, spleen, and liver. For the spleen, a lymphocyte fraction obtained using a cell strainer after tissue disruption was used. For tumors or liver, lymphocyte fractions obtained by tissue disruption using Tumor Dissociation Kit, mouse (Miltenyi Biotec) or Liver Dissociation Kit, mouse (Miltenyi Biotec) and then using a cell strainer were used. Flow cytometry (FCM) analysis was performed on the obtained tumor, liver, and spleen-derived lymphocyte fractions.

[Table 80]

| Group | Number of animals | Pharmaceutical agent | Dose [mg/kg] | Day of administration | Route of administration | Day of sampling |
|---|---|---|---|---|---|---|
| 1 | 4 | Alexa488-labeled IC17HdK-MB110/IC17L-mK | 2.5 | 14 days after transplantation | via tail vein | 15 days after transplantation |
| 2 | 4 | Alexa488-labeled A551-MB110/B379-ml0r | 2.5 | | via tail vein | |
| 3 | 4 | Alexa488-labeled UreH-MB110/UreL-mk1 | 2.5 | | via tail vein | |

(21-3) Evaluation of antibody tissue distribution by flow cytometry (FCM) analysis of MC38 cell line-transplanted mice

Analysis was performed using FCM for each Alexa488-labeled antibody bound to the tumor, spleen, and liver-derived lymphocyte fractions. The expression rate of Alexa488 in CD8[+] T cells was used for evaluation by FCM. Anti-CD45 antibody (BD Biosciences), anti-CD3 antibody (BioLegend), anti-CD8 antibody (BioLegend), anti-CD19 antibody (BioLegend), and anti-CD11b antibody (BioLegend) were used in the FCM analysis. BD LSR Fortessa X-20 (BD Biosciences) was used for the measurement.
As a result of evaluation of each Alexa488-labeled antibody bound to CD8[+] T cells infiltrating the tumor, a high rate of binding was observed in the Alexa488-labeled A551-MB110/B379-ml0r-administered group or the Alexa488-labeled UreH-MB110/UreL-mk1-administered group, as compared with the Alexa488-labeled IC17HdK-MB110/IC17L-mk-administered group (Figure 94). In the spleen and liver, the switch antibody Alexa488-labeled A551-MB110/B379-ml0r-administered group had a CD8[+] T cell binding rate almost equivalent to the negative control substance Alexa488-labeled IC17HdK-MB110/IC17L-mk-administered group (Figure 94).
On the other hand, in the Alexa488-labeled UreH-MB110/UreL-mk1-administered group, which is a non-switch antibody, the CD8[+] T cell binding rate was very high (Figure 94) compared to the Alexa488-labeled IC17HdK-MB110/IC17L-mk-administered group and the Alexa488-labeled A551-MB1 10/B379-ml0r-administered group.
From the above, it was shown that the switch antibody A551-MB110/B379-ml0r binds only to lymphocytes present in tumor tissue, and binding to lymphocytes present in normal organs, spleen and liver, is limited.

Example 22: Verification of a group of genes showing expression changes in association with extracellular ATP in tumor tissue

(22-1) Drawing a heat map of genes showing expression changes in association with extracellular ATP based on RNA-seq data of syngeneic tumor transplantation mouse model

From RNA-seq data obtained from each syngeneic tumor transplantation mouse model prepared by transplanting MC38 cell line, LLC/OVA/hGPC3 cell line, LLC/OVA cell line, or Hepa1-6/hGPC3 cell line into C57BL/6 mice, the expression value (unit: FPKM) was calculated by the RSEM method (version 1.2.31). The reference mRNA list (gene list) is GRCm38. In addition, the expression value was similarly calculated by the RSEM method from RNA-seq data obtained from each normal tissue of C57BL/6 mice. For RNA-seq raw data (.fastq file) of 12 kinds of normal organs, data published in literature (Sollner, Sci Data, 2017, 4: 170185) was used.
The unit was converted from [FPKM] to [FPKM + 1] by adding 1 to the gene expression level in FPKM units. This is because log conversion is impossible when 0 exists as the expression value, so a minute value is added. After that, log2 conversion was performed.

Next, human orthologs was assigned to the mouse genes. In principle, they were assigned according to the NCBI HomoloGene dataset.

Next, genes related to extracellular ATP were collected from literature (Hu, Clin Cancer Res, 2019, 25 (4), 1318-1330; Li, Cancer Discov, 2019, 9 (12), 1754-1773; Yang., Cancer Sci, 2019, 110 (8), 2456-2470; Gombault, Front Immunol, 2013, 3:414).

In addition, a group of EMT-related genes whose expression is increased by the addition of ATP was collected from literature (Yang, Cancer Sci, 2019, 110 (8), 2456-2470) (EMT: epithelial-mesenchymal transition).

Next, from the GRCm38 gene list (mRNA list) obtained from RNA-seq, only the genes in the above ATP-related gene group and EMT-related gene group were selected. Since RNA-seq is mouse data, and the ATP-related gene group and EMT-related gene group are human gene groups, they were converted by orthologs to obtain mouse gene groups. Next, genes with significantly higher expression in each cancer cell line than in the normal tissue group (p <0.05) by the t-test were selected.

Next, a heat draw map was drawn using the programming language R program. Here, the heat map was standardized so that the mean of all samples was 0 and the variance was 1 for each gene. The change from the average expression level of each gene was displayed by gradation (Figure 95).

As a result, it was shown that in tumor tissue, there are a plurality of significantly highly expressed genes in the ATP-related gene group and the EMT-related gene group as compared with the 12 kinds of normal organs. It was thought that A551-MB110/B379-ml0r binds to immune cells only in tumor tissue and does not bind to immune cells in normal tissues such as the liver and spleen (Example 21), for this. In addition, it was thought that the switch antibody that binds to the antigen in the presence of ATP induces a strong immune response only in tumor tissue (Example 20), and does not elicit an immune response in normal organs such as the liver, spleen, tumor regional lymph nodes, and peripheral blood (Example 20).

Example 23: Evaluation of the expression of CD73 and CD39 in various cancer cells by flow cytometry (FCM) analysis

(23-1) Evaluation of CD73 expression in various cancer cell lines

In this study, mouse acute myeloid leukemia cell line C1498 cells (described in Example 3) and mouse leukemia cell line E.G7-OVA cells (described in Example 2) purchased from ATCC; mouse colon cancer cell line MC38 cells (described in Example 6) licensed from the U.S. National Cancer Institute; and mouse liver cancer cell line Hepa1-6/hGPC3 cells (described in Example 4), mouse lung cancer cell line LLC1/OVA/GPC3 clone C5 cells (described in Reference Examples 9-4-1), and mouse lung cancer cell line LLC1/OVA clone 17 cells established in-house were used.

The mouse lung cancer cell line LLC1/OVA clone 17 cells were prepared by introducing chicken ovalbumin (OVA) expression plasmid to the mouse lung cancer-derived cell line LLC1 (LL/2 (also known as LLC1), distributor: ATCC, catalog number: CRL-1642). Anti-CD73 antibody (BioLegend) was used for FCM analysis in each cell line. FACS Lyric (BD Biosciences) was used for the measurement.

As a result of evaluating the membrane surface expression of CD73 in each cell line, almost no expression of CD73 was observed on the cell membrane of C1498 cells and E.G7-OVA cells, whereas it was observed on the cell membranes of MC38 cells, Hepa1-6/hGPC3 cells, LLC1/OVA/GPC3 clone C5 cells, and LLC1/OVA clone 17 cells (Figure 96). CD73 is known to be a membrane-type enzyme that breaks down AMP into adenosine. Therefore, even though MC38 cells, Hepa1-6/hGPC3 cells, LLC1/OVA/GPC3 clone C5 cells, and LLC1/OVA clone 17 cells have the property of breaking down AMP, which is required for antigen binding by switch antibody, to adenosine, the switch antibody was able to exhibit an antitumor effect (Examples 1 to 10), suggesting that the switch antibody exerts an action on tumor tissue without being affected by the expression of CD73.

(23-2) Evaluation of CD39 and CD73 expression in tumor tissues from LLC1/OVA/GPC3 clone C5 cell line-transplanted mice

(23-2-1) Preparation of the cell line and syngeneic tumor transplantation mouse model, and method for evaluating CD39 and CD73 expression of intratumoral infiltrating immune cells and tumor cells

In this study, in-house-established mouse lung cancer cell line LLC1/OVA/GPC3 clone C5 cells and hCD137 KI mice (described in Reference Example 6-3) were used. The LLC1/OVA/GPC3 clone C5 cell line was transplanted subcutaneously into the abdomen of mice, and sampling was done when the tumor volume reached approximately 500 mm$^3$ or more. Sampling was conducted 14 days after transplantation. After sampling, the tumor tissue was removed and a tumor cell fraction or lymphocyte fraction was prepared by the method described in detail below, and further, the expression of CD39 or CD73 on tumor cells, on CD4$^+$ T cells, on CD8$^+$ T cells, and on non-T cell fractions was evaluated using flow cytometry (FCM).

(23-2-2) Excision of tumor tissue from LLC1/OVA/GPC3 clone C5 cell line-transplanted mice and preparation of tumor cell fraction or lymphocyte fraction

For the excised tumor tissue, cell preparation of tumor cell fraction or lymphocyte fraction was performed. Tumor Dissociation Kit, mouse (Miltenyi Biotec) was used for the cell preparation, and the obtained tumor cell fraction or lymphocyte fraction was used for FCM analysis.

(23-2-3) Evaluation of CD39 and CD73 expression by FCM in the tumor cell fraction or lymphocyte fraction from LLC1/OVA/GPC3 clone C5 cell line-transplanted mice

For evaluation by FCM, the positive rates of CD39 and CD73 on tumor cells, on CD4$^+$ T cells, on CD8$^+$ T cells, and on non-T cell fractions were used. For this, in the FCM analysis, anti-CD45 antibody (BD Biosciences), anti-CD3 antibody (BioLegend), anti-CD8 antibody (BioLegend), anti-CD4 antibody (BioLegend), anti-CD39 antibody (BioLegend), and anti-CD73 antibody (BioLegend) were used. BD LSR Fortessa X-20 (BD Biosciences) was used for the measurement.
As a result of analyzing the expression of CD39 in each fraction, CD39 expression on tumor cells, CD4$^+$ T cells, CD8$^+$ T cells, and non-T cells was such that the expression was observed in some tumor cells, and in almost all of the CD4$^+$ T cells, CD8$^+$ T cells, and non-T cells (Figure 97). Next, as a result of analyzing the expression of CD73 in each fraction, expression was observed in some tumor cells, some CD8$^+$ T cells, and some non-T cells, whereas it was observed in almost all of the CD4$^+$ T cells (Figure 97). Representative examples of each data are shown in Figure 98.

(23-3) Evaluation of CD39 and CD73 expression in tumor tissues from MC38 cell line-transplanted mice

(23-3-1) Preparation of the cell line and syngeneic tumor transplantation mouse model, and method for evaluating CD39 and CD73 expression of intratumoral infiltrating immune cells and tumor cells

In this study, mouse colon cancer cell line MC38 cells licensed from the U.S. National Cancer Institute and hCD137 KI mice (described in Reference Example 6-3) were used. The MC38 cell line was transplanted subcutaneously into the abdomen of mice who were grouped when the tumor volume reached 163-204 mm$^3$. Grouping was performed 14 days after transplantation and sampling was performed 16 days after transplantation. Tumor tissue was removed to prepare a tumor cell fraction or lymphocyte fraction by the method described in detail below, and further, using flow cytometry (FCM), the expression of CD39 or CD73 on tumor cells, on CD4$^+$ T cells, on CD8$^+$ T cells, and on non-T cell fractions was evaluated.

(23-3-2) Excision of tumor tissue from MC38 cell line-transplanted mice and preparation of tumor cell fraction or lymphocyte fraction

For the excised tumor tissue, cell preparation of tumor cell fraction or lymphocyte fraction was performed. Tumor Dissociation Kit, mouse (Miltenyi Biotec) was used for cell preparation, and the obtained tumor cell fraction or lymphocyte fraction was used for FCM analysis.

(23-3-3) Evaluation of CD39 and CD73 expression by FCM of tumor cell fraction or lymphocyte fraction from MC38 cell line-transplanted mice

The positive ratio of CD39 and CD73 on tumor cells, on CD4$^+$ T cells, on CD8$^+$ T cells, and on non-T cell fractions were used for the evaluation by FCM. For this, in the FCM analysis, anti-CD45 antibody (BD Biosciences), anti-CD3 antibody (BioLegend), anti-CD8 antibody (BioLegend), anti-CD4 antibody (BioLegend), anti-CD39 antibody (BioLegend), and anti-CD73 antibody (BioLegend) were used. BD LSR Fortessa X-20 (BD Biosciences) was used for the measurement.
As a result of analyzing the expression of CD39 in each fraction, CD39 expression on tumor cells, CD4$^+$ T cells, CD8$^+$ T cells, and non-T cells was such that the expression was observed in some CD4$^+$ T cells and in some CD8$^+$ T cells, and in most non-T cells (Figure 99). Next, as a result of analyzing CD73 expression in each fraction, expression was observed in some tumor cells, and in almost all of the CD4$^+$ T cells and CD8$^+$ T cells (Figure 99). Almost no expression was observed in non-T cells. Representative examples of each data are shown in Figure 100.
Based on the above results, it was shown that in tumor tissues from mice transplanted with mouse colon cancer cell line MC38 cells and mouse lung cancer cell line LLC1/OVA/GPC3 clone C5 cells, CD39 and CD73 are widely expressed in intratumoral infiltrating immune cells, in addition to tumor cells. Membrane-type enzymes CD39 and CD73 are known to be enzymes that break down ATP into adenosine. Switch antibodies show antigen-binding ability in the presence of ATP, ADP, and AMP, but not in the presence of adenosine (see WO2020/032230). However, as it was demonstrated that a switch antibody bind to immune cells (Example 21) and exhibit an antitumor effect (Examples 1-10) even in tumor tissues in which CD39 and CD73 are widely expressed, such as tumor tissues from mice transplanted with mouse colon cancer cell line MC38 cells and mouse lung cancer cell line LLC1/OVA/GPC3 clone C5 cells, the switch antibody was shown to act on tumor tissues without being affected by the expression of CD39 and CD73.

Example 24: Evaluation of the antitumor activity of an anti-CD 137 antibody as single agent or in combination with anti-PD-Ll antibody by a syngeneic tumor transplantation model using hCD137KI mice (subcutaneous transplantation of LLC1/OVA clone 17 cells)

(24-1) Preparation of the tumor cell line and syngeneic tumor transplantation mouse model, and evaluation of the antitumor activity of A551-MB110/B379-ml0r (single agent or in combination with an anti-mouse PD-L1 antibody)

In this study, in-house established mouse lung cancer cell line LLC1/OVA clone 17 cells (described in Example 23, hereinafter abbreviated as LLC1/OVA cell line in this example) and hCD137 KI mice (described in Reference Example 6-3) were used. The LLC1/OVA cell line was transplanted subcutaneously into the abdomen of mice, and the model was considered established when the tumor volume reached 79-199 mm$^3$. After the model was established, LLC1/OVA cell line-transplanted mice were grouped and then at the doses shown in Table 81, switch antibody A551-MB110/B379-ml0r and anti-mouse PD-L1 antibody (BioXcell, Inc., clone 10F.9G2) were administered. As a positive control substance, non-switch antibody UreH-MB110/UreL-mk1 and anti-mouse PD-L1 antibody were administered. Administration of the switch antibody A551-MB110/B379-ml0r or the non-switch antibody UreH-MB110/UreL-mk1 was performed twice, 10 days and 13 days after transplantation, via the tail vein route. Administration of anti-mouse PD-L1 antibody was performed twice, 10 days and 13 days after transplantation, by the intraperitoneal route. PBS containing 0.05% Tween 20 was used for vehicle control.

[Table 81]

| Group | Number of animals | Pharmaceutical agent | Dose [mg/kg] | Day of administration | Route of administration |
|---|---|---|---|---|---|
| 1 | 8 | Vehicle control (PBS with 0.5% Tween 20) | - | 10 days and 13 days after transplantation | via tail vein and intraperitoneal |
| 2 | 8 | A551-MB110/B379-ml0r | 2.5 | | via tail vein |
| 3 | 8 | UreH-MB110/UreL-mk1 | 7.5 | | via tail vein |
| 4 | 8 | Anti-mouse PD-L1 antibody | 10 | | intraperitoneal |
| 5 | 8 | A551-MB110/B379-ml0r | 2.5 | | via tail vein |
| | | Anti-mouse PD-L1 antibody | 10 | | intraperitoneal |
| 6 | 8 | UreH-MB110/UreL-mk 1 | 7.5 | | via tail vein |
| | | Anti-mouse PD-L1 antibody | 10 | | intraperitoneal |

Tumor volume was measured 10 days, 13 days, 17 days, and 20 days after transplantation.
Tumor volume (TV) was calculated by the following formula.

$$\text{Tumor volume (mm}^3) = \text{length (mm) x width (mm) x width (mm)}/2$$

Tumor growth inhibition (TGI (%)) was calculated according to the following formulas.

$$\text{TV change (mm}^3) = \text{Tumor volume 20 days after transplantation - Tumor volume at the time of grouping}$$

$$TGI\ (\%) = (1- (\text{Mean value of TV change for each group/Mean value of TV change for}$$

$$\text{Vehicle control group})) \times 100$$

As a result, against LLC1/OVA tumor, the switch antibody A551-MB110/B379-ml0 and anti-mouse PD-L1 antibody combination group, the non-switch antibody UreH-MB110/UreL-mk1 and anti-mouse PD-L1 antibody combination group, and the switch antibody A551-MB110/B379-ml0r single agent group showed almost the same tumor growth suppression effect.

Specifically, the tumor growth suppression rate of the switch antibody A551-MB110/B379-ml0 and anti-mouse PD-L1 antibody combination group was 95%, whereas the non-switch antibody UreH-MB110/UreL-mk1 and anti-mouse PD-L1 antibody combination group showed a tumor growth suppression rate of 93%, and the switch antibody A551-MB110/B379-ml0r single agent group showed a tumor growth suppression rate of 101% (Figure 101, Table 82).

From the above results, it was shown that the switch antibody A551-MB110/B379-ml0 and anti-mouse PD-L1 antibody combination group had a tumor growth suppression effect similar to the non-switch antibody UreH-MB110/UreL-mk1 and anti-mouse PD-L1 antibody combination group (Figure 101, Table 82).

[Table 82]

| Tumor growth inhibition (%) at 20 days after transplantation | | | | | | |
|---|---|---|---|---|---|---|
| | Vehicle control | A551-MB110/B379-ml0r | UreH-MB110/Ure L-mk1 | Anti-mouse PD-L1 antibody | A551-MB110/B379-ml0r + Anti-mouse PD-L1 antibody | UreH-MB110/Ure L-mk1 + Anti-mouse PD-L1 antibody |
| Tumor growth inhibition (%) | - | 101 | 78 | 42 | 95 | 93 |

Example 25: Evaluation of the systemic effect of an anti-CD 137 antibody as single agent or in combination with an anti-PD-LI antibody by a syngeneic tumor cell transplantation model using hCD137KI mice (LLC1/OVA cells)

(25-1) Preparation of the tumor cell line and syngeneic tumor transplantation mouse model, and method of evaluating the intratumoral action

In this study, in-house established mouse lung cancer cell line LLC1/OVA cells (described in Example 23) and hCD137 KI mice (described in Reference Example 6-3) were used. The LLC1/OVA cell line was transplanted subcutaneously into the abdomen of mice, and the model was considered established when the tumor volume reached 91-205 mm$^3$. After the model was established, LLC1/OVA cell line-transplanted mice were grouped and then at the doses shown in Table 83, the switch antibody A551-MB110/B379-ml0r and anti-mouse PD-L1 antibody (BioXcell, Inc., clone 10F.9G2) were administered.

As a positive control substance, a non-switch anti-CD 137 antibody UreH-MB110/UreL-mk1 and anti-mouse PD-L1 antibody was administered. Administration of the switch antibody A551-MB110/B379-ml0r or the non-switch antibody UreH-MB110/UreL-mk1 was performed twice, 14 days and 17 days after transplantation, via the tail vein route. Administration of anti-mouse PD-L1 antibody was performed twice, 14 days and 17 days after transplantation, by the intraperitoneal route. PBS containing 0.05% Tween 20 was used for vehicle control.

(25-2) Excision of tumor regional lymph nodes from LLC1/OVA cell line-transplanted mice and preparation of lymphocyte fraction

For the excised tumor regional lymph nodes, a lymphocyte fraction obtained by using a cell strainer after tissue disruption was used. Flow cytometry (FCM) analysis was performed on the obtained lymphocyte fraction derived from the tumor regional lymph nodes.

[Table 83]

| Group | Number of animals | Pharmaceutical agent | Dose [mg/kg] | Day of administration | Route of administration | Day of sampling |
|---|---|---|---|---|---|---|
| 1 | 3 | Vehicle control (PBS with 0.5% Tween 20) | - | | via tail vein and intraperitoneal | |
| 2 | 3 | A551-MB110/B379-ml0r | 2.5 | | via tail vein | |
| 3 | 3 | UreH-MB110/UreL-mk1 | 7.5 | | via tail vein | |
| 4 | 3 | Anti-mouse PD-L1 antibody | 10 | 14 days and 17 days after transplantation | intraperitoneal | 18 days after transplantation |
| 5 | 3 | A551-MB110/B379-ml0r | 2.5 | | via tail vein | |
| | | Anti-mouse PD-L1 antibody | 10 | | intraperitoneal | |
| 6 | 3 | UreH-MB110/UreL-mk1 | 7.5 | | via tail vein | |
| | | Anti-mouse PD-L1 antibody | 10 | | intraperitoneal | |

(25-3) Evaluation of immune cell activation by flow cytometry (FCM) analysis in LLC1/OVA cell line-transplanted mice

Analysis was performed using FCM for T cell activation derived from tumor regional lymph nodes. The expression rate of the activation markers in CD8$^+$ T cells was used for evaluation by FCM. In the FCM analysis, anti-CD45 antibody (BD Biosciences), anti-CD3 antibody (BioLegend), anti-CD8 antibody (BioLegend), anti-KLRG-1 antibody (BD Biosciences), anti-ICOS antibody (BioLegend), and anti-PD-1 antibody (BioLegend) were used. BD LSR Fortessa X-20 (BD Biosciences) was used for the measurement.

As a result of the evaluation in tumor regional lymph nodes, there was no increase in all of the expression rates and absolute numbers of KLRG-1, ICOS, PD-1 in CD8$^+$ T cells, in the A551-MB110/B379-ml0r single agent group, anti-mouse PD-L1 antibody single agent group, and the A551-MB110/B379-ml0r and anti-mouse PD-L1 antibody combination group (Figures 102 and 103). On the other hand, a remarkable increase was observed in the UreH-MB110/UreL-mk1 single agent group and the UreH-MB110/UreL-mk1 and anti-mouse PD-L1 antibody combination group (Figures 102 and 103).

From this result, as shown in Example 24, it was revealed that, even though both the switch antibody A551-MB110/B379-ml0r and the non-switch antibody UreH-MB110/UreL-mk1 showed a similar tumor growth suppression effect even when used in combination with the anti-mouse PD-L1 antibody, the switch antibody A551-MB110/B379-ml0r had an extremely limited action on T cells in the tumor regional lymph nodes, which is a normal organ, compared to the non-switch antibody UreH-MB110/UreL-mk1, when used both as a single agent and in combination with anti-mouse PD-L1 antibody.

Example 26: Evaluation of the antitumor activity of an anti-CD 137 antibody by a syngeneic tumor transplantation model using hCD137KI mice (intravenous administration of LLC1/OVA cells)

(26-1) Preparation of the tumor cell line and syngeneic tumor transplantation mouse model, and evaluation of the antitumor activity

In this study, in-house established mouse lung cancer cell line LLC1/OVA clone17 cells (described in Example 23) and hCD137 KI mice (described in Reference Example 6-3) were used. The LLC1/OVA clone 17 cell line was transplanted into the tail vein of mice who were grouped 7 days after transplantation. The A551-MB110/B379-ml0r antibody was administered into the tail vein of mice 7 days and 14 days after transplantation as shown in Table 84 below. PBS containing 0.05% Tween 20 was used as the vehicle control. Body weight was measured 18 days after tumor transplantation, and lung weight was measured after exsanguination and euthanasia under anesthesia. Sampling was also performed on 10 normal mice without cell transplantation by the same method on the same day. Lung weight was calculated as the lung weight value per 100 g of body weight.

[Table 84]

| Group | Number of animals | Pharmaceutical agent | Dose (mg/kg) | Day of administration | Route of administration |
|---|---|---|---|---|---|
| 1 | 15 | Vehicle control (PBS with 0.05% Tween 20) | - | 7 days and 14 days after transplantation | via tail vein |
| 2 | 15 | A551-MB110/B379-ml0r | 2.5 | | |

As a result, the switch antibody A551-MB110/B379-ml0r-administered group had significantly-suppressed lung weight as compared with the vehicle-administered group (Figure 104, Table 85). Therefore, it was shown that the switch antibody A551-MB110/B379-ml0r has a drug efficacy not only on subcutaneously transplanted tumors (described in Example 24) but also on micrometastases of the lung. One mouse in the vehicle-administered group died 11 days after tumor transplantation. Small traces and bumps reminiscent of tumors were found in the lungs of the dead mouse.

[Table 85]

| Lung weight at 18 days after transplantation | | | |
|---|---|---|---|
| | Vehicle control (PBS with 0.05% Tween 20) | A551-MB110/B379-ml0r | Normal mice without cell transplantation |
| Lung weight (/100 g body weight) | 0.708 | 0.553 | 0.480 |

Reference Example 1: Antigen preparation

(1-1) Preparation of the human CD137 extracellular region

The human CD137 extracellular domain (also called hCD137) was prepared with methods known to those skilled in the art. Specifically, downstream of a gene fragment encoding an extracellular region of human CD137, a gene fragment encoding a histidine tag and a gene fragment encoding a specific sequence to which biotin is added (AviTag sequence, SEQ ID NO: 86) are linked. The gene fragment encoding a protein in which the extracellular region of human CD137, the histidine tag and Avitag are linked (human CD137 or hCD137-HisBAP, SEQ ID NO: 87), was incorporated into an animal cell expression vector. The constructed plasmid vector was transfected into FreeStyle293 cells (Invitrogen) using 293-fectin (Invitrogen). At this time, a plasmid vector comprising an EBNA1-expressing gene (SEQ ID NO: 88) was transfected at the same time. Cells transfected with the genes according to the aforementioned procedure were cultured at 37°C, 8% $CO_2$; and the protein of interest (human CD137 extracellular region) was secreted into the culture supernatant. This cell culture medium was filtered through a 0.22 $\mu$m filter to obtain a culture supernatant.
The culture supernatant was applied to the HisTrap-HP (GE Healthcare) and the human CD137 extracellular region was bound to the column. The human CD137 extracellular region was eluted using a solution of 20 mM sodium phosphate, 500 mM sodium chloride, and 500 mM imidazole, pH 7.5. Aggregates were then removed by gel-filtration chromatography using Superdex 200 26/600(GE healthcare) to yield a purified human CD137 extracellular region.
The concentration of human CD137 was calculated using the method of Pace *et al.* based on the amino acid sequence excluding the signal sequence inferred from SEQ ID NO: 87 (SEQ ID NO: 183) (Pace, C.N., et al. Protein Science 1995; 4; 2411-2423).

(1-2) Preparation of the human CD137 extracellular domain (hCD137(FXa digested))

The human CD137 extracellular domain (also called hCD137(FXa digested)) was prepared in a method known to those skilled in the art. Specifically, a gene fragment encoding the Factor Xa cleavable sequence and a gene fragment encoding the constant region of an antibody were joined downstream of a gene fragment encoding the extracellular region of human CD137. A gene fragment encoding a protein in which the extracellular region of human CD137, the FactorXa cleavable sequence and the antibody constant region are linked (hCD137-F-Fc, SEQ ID NO: 89) was incorporated into an animal expression vector. The constructed plasmid vector was transfected into FreeStyle293 cells (Invitrogen) using 293-fectin (Invitrogen). At this time, a plasmid vector containing the EBNA1-encoding gene (SEQ ID NO: 88) was transfected at the same time. Cells transfected with genes according to the aforementioned procedures were cultured at 37°C, 8% $CO_2$; and hCD137-F-Fc was secreted into the culture supernatant. The cell culture medium was filtered through a 0.22 μm filter, and the culture supernatant was collected.

Culture supernatants were applied to a column packed with Protein A (MabSelect SuRe, GE Healthcare). hCD137-F-Fc was bound to the column and eluted with 50 mM acetic acid solution. After the eluate was neutralized with 1 M Tris-HCl, pH 8.0, the solvent of hCD137-F-Fc was replaced with 20 mM Tris, 100 mM sodium chloride, 2 mM calcium chloride, pH 8.0. Next, Factor Xa Protease (NEW ENGLAND BioLabs, Cat#. P8010L) was added and hCD137-F-Fc was digested. A protease inhibitor (DNS-GGACK, Calbiochem, Cat#251700) was then added to stop the reaction. 5 M sodium chloride was added to the protease reaction solution, and the total volume of the prepared solution was applied to HiTrap Benzamidine (GE Healthcare, Cat# 17-5143-01). The column-filtered solution was further applied to a Protein A column (MabSelect SuRe, GE Healthcare), and pass fractions were collected. The pass fractions were removed of aggregates by gel-filtration chromatography using Superdex 200 Increase, 10/30 (GE Healthcare, Cat# 28990944), yielding a purified human CD137 extracellular region.

(1-3) Preparation of biotinylated Fc-fused human CD137

Biotinylated Fc-fused human CD137 (also referred to as "biotinylated hCD137-Fc" or "bio-hCD137-Fc", or hCD137-Fc-Bio) was prepared by methods known to those skilled in the art. Specifically, a gene fragment encoding the constant region of an antibody and a gene fragment encoding a specific sequence to which biotin is added (AviTag sequence, SEQ ID NO: 86) were joined downstream of a gene fragment encoding the extracellular region of human CD137. A gene fragment encoding a protein (Fc-fused human CD137, SEQ ID NO: 90) in which the human CD137 extracellular region, the antibody constant region, and Avitag have been linked was incorporated into an animal expression vector. The constructed plasmid vector was transfected into FreeStyle293 cells (Invitrogen) using 293-fectin (Invitrogen). At this time, a gene expressing EBNA1 (SEQ ID NO: 88) and a gene expressing biotin ligase (BirA, SEQ ID NO: 91) were simultaneously transfected, and biotin was added for the purpose of biotinylating Fc-fused human CD137. Cells transfected with the genes according to the aforementioned procedures were cultured at 37°C, 8% $CO_2$; and the protein of interest (biotinylated Fc-fused human CD137) was secreted into the culture supernatant. This cell culture was filtered through a 0.22 μm filter to obtain the culture supernatant.

The culture supernatant was applied to a column packed with Protein A (MabSelect SuRe, GE Healthcare), and biotinylated Fc-fused human CD137 was bound to the column. Biotinylated Fc-fused human CD137 was eluted using a 50 mM acetic acid solution. Aggregates were then removed by gel-filtration chromatography with Superdex 200, 26/600 (GE Healthcare), and purified biotinylated Fc-fused human CD137 was obtained.

(1-4) Preparation of Fc-fused human CD137

Fc-fused human CD137 (also referred to as hCD137-Fc) was prepared by methods known to those skilled in the art. Specifically, a gene fragment encoding the constant region of an antibody and a gene fragment encoding a specific sequence to which biotin is added (AviTag sequence, SEQ ID NO: 86) were joined downstream of a gene fragment encoding the extracellular region of human CD137. Gene fragments encoding a protein (Fc-fused human CD137, SEQ ID NO: 90) in which the extracellular region of human CD137, the antibody constant region, and Avitag have been linked was incorporated into an animal expression vector. The constructed plasmid vector was transfected into FreeStyle293F cells (Invitrogen) using 293-fectin (Invitrogen). At this time, a gene expressing a gene expressing EBNA1 (SEQ ID NO: 88) was simultaneously transfected. Cells transfected with the genes according to the aforementioned procedures were cultured at 37°C, 8% $CO_2$, and the protein of interest (Fc-fused human CD137) was secreted into the culture supernatant. This cell culture was filtered through a 0.22 μm filter to obtain the culture supernatant.

The culture supernatant was applied to a column packed with Protein A (MabSelect SuRe, GE Healthcare), and Fc-fused human CD137 was bound to the column. Fc-fused human CD137 was eluted using a 50 mM acetic acid solution. Next, aggregates were removed by gel-filtration chromatography with Superdex 200, 26/600 (GE Healthcare), and purified Fc-fused human CD137 was obtained.

(1-5) Preparation of biotinylated Fc-fused monkey CD137

Biotinylated Fc-fused monkey CD137 (also referred to as "cyCD137-Fc-BAP") was prepared by methods known to those skilled in the art. Specifically, a gene fragment encoding the constant region of an antibody and a gene fragment encoding a specific sequence to which biotin is added (AviTag sequence, SEQ ID NO: 86) were joined downstream of a gene fragment encoding the extracellular region of monkey CD137. A gene fragment encoding a protein in which the monkey CD137 extracellular region, the antibody constant region, and Avitag are linked (Fc-fused monkey CD137, SEQ ID NO: 92) was incorporated into an animal expression vector.

The constructed plasmid vector was transfected into FreeStyle293 cells (Invitrogen) using 293-fectin (Invitrogen). At this time, a gene expressing EBNA1 (SEQ ID NO: 88) and a gene expressing biotin ligase (BirA, SEQ ID NO: 91) were simultaneously transfected; and biotin was added for the purpose of labeling Fc-fused monkey CD137 with biotin. Cells transfected with the genes according to the aforementioned procedures were cultured at 37°C, 8% $CO_2$; and the protein of interest (biotinylated Fc-fused monkey CD137) was secreted into the culture supernatant. This cell culture was filtered through a 0.22 $\mu$m filter to obtain the culture supernatant.

The culture supernatant was applied to a column packed with Protein A (MabSelect SuRe, GE Healthcare), and biotinylated Fc-fused monkey CD137 was bound to the column. Biotinylated Fc-fused monkey CD137 was eluted using a 50 mM acetic acid solution. Aggregates were then removed by gel-filtration chromatography using Superdex 200 increase 10/300 (GE Healthcare), and purified biotinylated Fc-fused monkey CD137 was obtained.

Reference Example 2: Obtaining ATP-dependent CD137 antibodies

(2-1) Obtaining antibodies with a small molecule-dependent antigen-binding activity (small-molecule switch antibodies) from a rationally designed library utilizing ATP (1)

(2-1-1) Panning

Antibodies that exhibit binding activity towards antigens in the presence of adenosine triphosphate (adenosine 5'-triphosphate; ATP) were obtained from a rationally designed antibody phage display library constructed in a prior patent, WO2015/083764. Note that antibodies with a small molecule-dependent antigen (e.g., CD137) binding activity may be referred to as "switch antibodies" or "small-molecule switch antibodies", and antibodies with an ATP-dependent antigen (e.g., CD137) binding activity may be referred to as "switch antibodies" or "ATP switch antibodies". For acquisition, phages presenting antibodies that display binding activity in the presence of ATP towards the antigen captured on the beads were harvested. Subsequently, phages were collected from eluates eluted from the beads in the absence of ATP.

Phages were produced in a general method from *E. coli* carrying the constructed phage display phagemid. Specifically, *E. coli* carrying the constructed phagemid vector was infected with M13KO7ΔpIII (designated as "hyperphage") (PROGEN Biotechnik), and phages were harvested from supernatants cultured overnight at 30°C. A phage library solution was obtained by diluting with Tris-Buffered Saline (TBS) a population of phages precipitated by adding 2.5 M NaCl/10% PEG to the *E. coli* culture in which phage production was performed. BSA was then added to the phage library solution at a final concentration of 4%. Panning was performed using an antigen immobilized on magnetic beads. For magnetic beads, Sera-Mag NeutrAvidin beads (Thermo Fisher Scientific) or Dynabeads M-280 StreptAvidin (Life Technologies) were used. As antigen, biotinylated adenosine triphosphate (adenosine 5'-triphosphate; ATP) purchased from Jena Bioscience, or hCD137-Fc-Bio (SEQ ID NO: 90) or bio-hCD137 (SEQ ID NO: 89) which has been biotinylated from hCD137(FXa digested) using No weight Premeasured NHS-PEO4-Biotin (PIERCE) made in Reference Examples 1-2 and 1-3 was used.

Panning was performed to efficiently obtain small molecule that can play a switch role in cancer tissues dependent switch antibodies. Specifically, panning to enrich antibodies that bind to an antigen in the presence of ATP which is a small molecule, and do not bind to the antigen in the absence of ATP was performed with reference to the methods shown in the prior patent WO2015/083764. In Round 1, for all of biotinylated hCD137 (bio-hCD137), hCD137-Fc-Bio and biotinylated ATP (bio-ATP), panning was performed using a method in which solid-phase immobilization of biotinylated antigens on magnetic beads (termed "bead solid-phase method") precedes addition of a prepared phage library solution. For Bio-ATP, panning to enrich antibodies that can bind to an antigen (Bio-ATP) in the absence of ATP as a small-molecule compound was performed with reference to the methods described in the prior patent WO2015/083764 above. For hCD137-Fc-Bio, 4 nmol of an unbiotinylated human IgG1 Fc region was added to remove antibodies that bind to the Fc region. The harvested phage was added to the *E. coli* strain ER2738 to infect the phage with *E. coli,* then the harvested *E. coli* was infected with the hyperphage, and the phage was harvested from the supernatant cultured overnight at 30°C.

From Round 2 on, panning was performed only on biotinylated hCD137 (bio-hCD137) and hCD137-Fc-Bio to enrich antibodies that bind to the antigen in the presence of ATP and not to the antigen in the absence of ATP in the bead solid-phase method, by referring to the method shown in the prior patent WO2015/083764. For hCD137-Fc-Bio in both cases, 4 nmol of an unbiotinylated human IgG1 Fc region was added to remove antibodies that bind to the Fc region. Similar panning was repeated until Round 5 to enrich the antibody sequences of interest.

(2-1-2) Binding activity assessment in the presence and absence of ATP by phage ELISA

From single colonies of *E. coli* obtained by the methods described above, the phage-containing culture supernatant was harvested using a conventional method (Methods Mol. Biol. (2002) 178, 133-145). NucleoFast 96 (MACHEREY-NAGEL) was used to ultrafilter the harvested culture supernatant. Flow-through was removed by centrifuging (4,500 g, 45 min) NucleoFast 96 to which 100 μL of culture supernatant has been applied in each well. NucleoFast 96 was washed again by centrifugation (4,500 g for 30 min) by adding 100 μL of H₂O to each well. Finally, 100 μL of TBS was added, and the phage solution contained in the supernatants in the wells of NucleoFast 96, which were left to stand at room temperature for 5 minutes, was recovered.

Purified phages with added TBS or ATP/TBS were subject to ELISA by the following procedure. 384-well Streptavidin-coated Microplates (Greiner) were coated overnight with 10 μL TBS containing the biotinylated antigens (bio-hCD137, hCD137-Fc-Bio, and bio-Fc) produced in Reference Example 1. After biotinylated antigens that did not bind to the plate were removed by washing each well of the plate with Tris-Buffered Saline with Tween 20 (TBST), the wells were blocked with 80 μL of 2% Skim Milk-TBS for 1 hour or more. 2% Skim Milk-TBS was removed by TBST washes, after which the antibody-presenting phages were allowed to bind to the biotinylated antigen present in each well in the absence and presence of ATP, by leaving the plates with the prepared purified phages added to each well for 1 hour at room temperature. To each well washed with TBST or ATP/TBST, HRP-conjugated anti-M13 antibodies (GE Healthcare 27-9421-01) diluted with TBS or ATP/TBS were added, and the plate was incubated for one hour. After washing with TBST or ATP/TBST, color development of the solution in each well to which the TMB single solution (ZYMED) was added was stopped by the addition of sulfuric acid, and then color development was measured by absorbance at 450 nm.

As a result, several antibodies with altered binding activity to bio-hCD137 or hCD137-Fc-Bio in the presence and absence of ATP were identified.

The results of phage ELISA using clones after Round 4 and 5 panning are shown in Table 29.

Here, clones with an absorbance of 0.2 or more in the presence of ATP and an S/N ratio of absorbance higher than 2 in the presence/absence of the antigen were determined as positive clones. Furthermore, among the positive clones, clones with an absorbance S/N higher than 2 in the presence/absence of ATP were judged to be clones with ATP-dependent antigen-binding activity (switch clones).

[Table 29]

| Antigen used in panning | Biotinylated hCD137 | | hCD137-Fc-Bio | |
|---|---|---|---|---|
| Round | 4 | 5 | 4 | 5 |
| Number of clones subjected to ELISA | 384 | 384 | 384 | 384 |
| Number of positive clones (absorbance in ATP+ ≥ 0.2; antigen +/- ratio > 2.0) | 31 | 4 | 125 | 169 |
| Number of switch clones (ATP+/- ratio > 2) | 12 | 2 | 118 | 164 |

(2-1-3) Sequence analysis of switch antibodies whose antigen-binding activity is altered by the presence/absence of ATP

The nucleotide sequences of genes amplified using specific primers pBAD-F, G1seq-R from clones with ATP-dependent antigen-binding activity (switch clones) based on the phage ELISA results were analyzed. As a result of the analysis, the nucleotide sequences of clones judged to bind to human CD137 in the presence of ATP and not to human CD137 in the absence of ATP were obtained.

(2-2) Obtaining antibodies that bind to antigens in the presence of a small molecule from a rationally designed library utilizing ATP (2)

(2-2-1) Panning

Antibodies that exhibit binding activity to antigens in the presence of ATP were obtained from a rationally designed antibody phage display library constructed in the prior patent WO2015/083764. For acquisition, phages presenting antibodies which show binding activity to antigens in the presence of ATP were recovered, followed by phage recovery from the eluate eluted from the beads in the absence of ATP.

Phages were produced in a general method from *E. coli* carrying the constructed phage display phagemid. Specifically, *E. coli* carrying the constructed phagemid vector was infected with M13KO7TC (WO2015046554A1) or M13KO7ΔpIII (hyperphage) (PROGEN Biotechnik), and phages were recovered from supernatants cultured overnight at 30°C. A phage library solution was obtained by diluting with TBS a population of phages precipitated by adding 2.5 M NaCl /10% PEG to an *E. coli* culture in which phage production was performed. BSA was then added to the phage library solution at a final concentration of 4%. Panning was performed using antigens immobilized on magnetic beads. NeutrAvidin-coated beads (Sera-Mag SpeedBeads NeutrAvidin-coated), NeutrAvidin beads (TAMAGAWA SEIKI) or Dynabeads MyOne StreptAvidin T1 (Thermo Fisher Scientific) were used as magnetic beads. As antigen, hCD137-Fc-Bio or bio-hCD137 produced in Reference Example 1 was used.

Panning was performed to efficiently obtain small molecule switch antibodies which are dependent on the small molecule that can play a switch role in cancer tissues. Specifically, panning to enrich antibodies that bind to antigens in the presence of adenosine triphosphate (adenosine 5'-triphosphate; ATP) which is a small molecule, and which do not bind antigens in the absence of ATP was performed with reference to the methods described in the prior patent WO2015/083764. For bio-hCD137, the method which adds the prepared phage library solution after solid-phase immobilization of biotinylated antigens onto magnetic beads in advance (referred to as the "bead solid-phase method") and the method which adds magnetic beads after mixing the prepared phage library solution with biotinylated antigens in advance (referred to as the "liquid-phase method") were performed. For hCD137-Fc-Bio, 4 nmol of an unbiotinylated human IgG1 Fc region was added to remove antibodies that bind to the Fc region, and panning was performed only in the liquid-phase method. The collected phages were added to the *E. coli* strain ER2738 and allowed to infect the *E. coli*, then the collected *E. coli* was infected with M13KO7TC (WO2015046554A1) or M13KO7ΔpIII (hyperphage) (PROGEN Biotechnik), and after culturing overnight at 30°C phages were collected from the supernatant. Similar panning was repeated until Round 5.

(2-2-2) Binding activity assessment in the presence and absence of ATP by phage ELISA

From single colonies of *E. coli* obtained after each Round by the methods described above, phage-containing culture supernatants were harvested using a conventional method (Methods Mol. Biol. (2002) 178, 133-145). NucleoFast 96 (MACHEREY-NAGEL) was used to ultrafilter the harvested culture supernatants. Flow-through was removed by centrifuging (4,500 g, 45 min) the NucleoFast 96 in which 100 μL of culture supernatant was applied to each well. The NucleoFast 96, in which 100 μL of H$_2$O was added to each well, was washed again by centrifugation (4,500 g for 30 min). Finally, 100 μL of TBS was added, and the phage solution contained in the supernatants of the NucleoFast 96 wells, which were left to stand at room temperature for 5 minutes, was recovered.

Purified phages with added TBS or ATP/TBS were subject to ELISA by the procedures below. StreptaWell 96 microtiter plates (Roche) were immobilized overnight with 100 μL of TBS containing the biotinylated antigens (hCD137-Fc-Bio or bio-hCD137) produced in Reference Example 1. After removal of the biotinylated antigen that did not bind to the plates by washing each well of the plates with TBST, the wells were blocked with 250 μL of 2% SkimMilk-TBS for 1 hour or more. The 2% SkimMilk-TBS was removed, and then antibody-presenting phages were bound to the biotinylated antigen present in each well in the absence and presence of ATP by leaving the plates with the prepared purified phages added to each well at 37°C for 1 hour. To each well washed with TBST or ATP/TBST, HRP-conjugated anti-M13 antibodies (GE Healthcare 27-9421-01) diluted in TBS or ATP/TBS were added, and the plate was incubated for one hour. After TBST or ATP/TBST washes, color development of the solution in each well to which the TMB single solution (ZYMED) was added was stopped by the addition of sulfuric acid, and then color development was measured by absorbance at 450 nm.

As a result, several antibodies with altered binding activity towards bio-hCD137 or hCD137-Fc-Bio in the presence and absence of ATP were identified.

As an example, the results of phage ELISA using clones after Round 5 panning are shown in Table 30.

Here, clones with an absorbance of 0.2 or greater in the presence of ATP and an S/N ratio of absorbance greater than 2 in the presence/absence of the antigen were determined as positive clones. In addition, among the positive clones, those with an S/N ratio of absorbance higher than 2 in the presence/absence of ATP were determined as clones with ATP-dependent antigen-binding activity (switch clones).

[Table 30]

| | | | | |
|---|---|---|---|---|
| Antigen used in panning | hCD137-Fc-Bio | hCD137-Fc-Bio | Biotinylated hCD137 | Biotinylated hCD137 |
| Panning method | Liquid-phase | Liquid-phase | Liquid-phase | Bead solid-phase |
| Helper phage used in panning | M13KO7 ΔpIII | M13KO7ΔpIII and M13KO7TC | M13KO7ΔpIII | M13KO7ΔpIII |
| Number of clones subjected to ELISA | 384 | 96 | 96 | 96 |

(continued)

| Antigen used in panning | hCD137-Fc-Bio | hCD137-Fc-Bio | Biotinylated hCD137 | Biotinylated hCD137 |
|---|---|---|---|---|
| Number of positive clones (absorbance in ATP+ ≥ 0.2; antigen +/ratio > 2) | 383 | 79 | 41 | 29 |
| Number of switch clones (ATP+/- ratio > 2) | 375 | 77 | 40 | 29 |

<u>(2-2-3) Sequence analysis of switch antibodies whose antigen-binding activity is altered by the presence/absence of ATP</u>

The nucleotide sequences of genes amplified from clones with ATP-dependent antigen-binding activity (switch clones) based on the phage ELISA results were analyzed using specific primers pBAD-F, G1seq-R. As a result of the analysis, the nucleotide sequences of clones judged to bind to human CD137 in the presence of ATP and not to human CD137 in the absence of ATP were obtained.

<u>(2-3) Selection of switch antibodies</u>

Seventeen samples were selected from the clones judged to have ATP-dependent antigen binding activity obtained as a result of the analyses in Reference Examples 2-1-3 and 2-2-3, and the clone names were re-assigned as described in Table 31.

[Table 31]

| | Phage name | Clone name of antibody variable region |
|---|---|---|
| 1 | dBBATk14-4_003 | dBBAT007 |
| 2 | dBBATk14-4_089 | dBBAT013 |
| 3 | dBBATk17-4_002 | dBBAT015 |
| 4 | dBBATk17-4_009 | dBBAT017 |
| 5 | dBBATk17-4_075 | dBBAT019 |
| 6 | dBBATk05-5_052 | dBBAT021 |
| 7 | dBBATk05-5_076 | dBBAT025 |
| 8 | dBBATk12-5_038 | dBBAT029 |
| 9 | dBBATk12-5_048 | dBBAT031 |
| 10 | dBBATk14-5_012 | dBBAT037 |
| 11 | dBBATk14-5_053 | dBBAT042 |
| 12 | dBBAHk14SF-3_016 | dBBAT053 |
| 13 | dBBAHk14SF-3_032 | dBBAT056 |
| 14 | dBBAHk14FF-4_028 | dBBAT091 |
| 15 | dBBAHk14FS-5_074 | dBBAT112 |
| 16 | dBBAHk14MS-5_034 | dBBAT118 |
| 17 | dBBAHk14MS-5_093 | dBBAT119 |

<u>(2-4) Expression and purification of switch antibodies whose antigen-binding activity is altered by the presence/absence of ATP</u>

Genes encoding the variable regions of the antibodies described in Table 31, which were obtained from a rationally designed phage library, were inserted into human IgG1/Lambda plasmids for expression in animals. Antibodies were expressed using the methods below. The prepared plasmids were introduced by the lipofection method into the human fetal kidney cell-derived line FreeStyle 293-F (Invitrogen), which was suspended in the FreeStyle 293 Expression Medium (Invitrogen) at a cell density of $1.33 \times 10^6$ cells/mL, of which 3 mL was seeded in each well of 6-well plates. Antibodies were purified using rProtein A Sepharose™ Fast Flow (Amersham Biosciences) from culture supernatants cultured for four days in a $CO_2$ incubator (37°C, 8% $CO_2$, 90 rpm) in a method known to those skilled in the art. Spectrophotometers were used to measure the absorbance at 280 nm of the purified antibody solutions. From the measured values obtained, the concentrations of the purified antibodies were calculated using the extinction coefficients calculated by the PACE method (Protein Science (1995) 4, 2411-2423).

(2-5) Assessment of the anti-CD 137 antibodies identified by the phage display method

(2-5-1) Expression and purification of switch antibodies which bind to antigens depending on the presence or absence of ATP and its metabolites

Genes encoding the variable regions of antibodies obtained from a human rationally designed phage library were inserted into animal expression plasmids having a heavy chain constant region fused with a gene encoding Gly and Lys downstream of a modified human IgG1 (P253) (SEQ ID NO: 93) and a light-chain constant region, Lambda chain Lamlib (SEQ ID NO: 63). Clone names and sequence numbers were listed in Table 32.

Evaluated clones

[Table 32]

|  | Clone name | SEQ ID NO of heavy chain (full length) | SEQ ID NO of light chain (full length) |
|---|---|---|---|
| 1 | dBBAT007-P253 | 94 | 113 |
| 2 | dBBAT013-P253 | 95 | 114 |
| 3 | dBBAT015-P253 | 96 | 115 |
| 4 | dBBAT017-P253 | 97 | 116 |
| 5 | dBBAT019-P253 | 98 | 117 |
| 6 | dBBAT025-P253 | 99 | 118 |
| 7 | dBBAT029-P253 | 100 | 119 |
| 8 | dBBAT031-P253 | 100 | 119 |
| 9 | dBBAT037-P253 | 101 | 120 |
| 10 | dBBAT042-P253 | 102 | 121 |
| 11 | dBBAT053-P253 | 103 | 122 |
| 12 | dBBAT056-P253 | 104 | 123 |
| 13 | dBBAT021-P253 | 105 | 124 |
| 14 | dBBAT091-P253 | 106 | 125 |
| 15 | dBBAT112-P253 | 107 | 126 |
| 16 | dBBAT118-P253 | 108 | 127 |
| 17 | dBBAT119-P253 | 109 | 128 |
| 18 | dBBAT121-P253 | 110 | 129 |
| 19 | dBBAT122-P253 | 111 | 130 |
| 20 | dBBAT134-P253 | 112 | 131 |

Antibodies were expressed and purified using methods known to those skilled in the art. Spectrophotometers were used to measure the absorbance at 280 nm of the purified antibody solutions. From the measured values obtained, the concentrations of the purified antibodies were calculated using the extinction coefficients calculated by the PACE method (Protein Science (1995) 4, 2411-2423).

(2-5-2) Assessment of the effects of ATP, ADP, and AMP on human CD137 binding by surface plasmon resonance

Biacore T200 (GE Healthcare) was used to analyze the interaction of antigen-antibody reaction between the anti-CD 137 antibodies and hCD137 (FXa digest). The anti-CD 137 antibodies were captured on Sensor chip CM3 (GE Healthcare) to which Protein G (CALBIOCHEM) was immobilized in appropriate amounts by an amine-coupling method, and hCD137 (FXa digest) prepared in Reference Example 1-2 was allowed to interact. For the running buffer, 20 mM ACES, 150 mM NaCl, 2 mM MgCl$_2$, 0.05% Tween20 (pH 7.4) was used, and 10 mM Glycine-HCl (pH 1.5) was used as the regeneration solution.

After capturing of the anti-CD137 antibodies suspended in TBS, 500 nM hCD137 (FXa digest) was injected into each flow cell at a flow rate of 10 μL/min for three minutes. This three-minute period served as the binding phase for hCD137 (FXa digest), and after the binding phase ended, the two-minute period of switching to the running buffer served as the dissociation phase for hCD137 (FXa digest). After completion of the dissociation phase, the regenerative solution was injected at a flow rate of 30 μl/min for 30 seconds. The above was the cycle for measuring the binding activity of anti-CD 137 antibodies. The binding amount of hCD137 (FXa digest) that interacted with the anti-CD137 antibody in the binding phase was adjusted by the amount of antibody captured. Biacore T200 Evaluation Software Version 2.0 was used to display the binding amount (RU) per capture ligand 1RU, and values of the antibody capture amount (amount of antibody captured) and antigen-binding amount were obtained. The binding amount of antigen is shown in Table 33. Since the binding amount of antigen reflects the binding activity, it can be said that dependence on a small molecule is recognized when the value in the presence of the small molecule (ATP, ADP or AMP) is higher than the value without the small molecule. In particular, the larger the difference is, the higher the dependence is on a small molecule.

[Table 33]

| Clone name | Small molecule species, concentration condition | | | | | | |
|---|---|---|---|---|---|---|---|
| | 1mM ATP | 100 μM ATP | 100 μM ADP | 10 μM ADP | 1mM AMP | 100 μM AMP | Without small molecule |
| dBBAT007-P253 | 49.2 | 29.5 | 89.0 | 70.0 | 28.5 | 16.5 | 3.6 |
| dBBAT013-P253 | 26.2 | 8.1 | 77.3 | 48.5 | 13.8 | 6.6 | 2.7 |
| dBBAT015-P253 | 8.3 | 3.1 | 41.8 | 17.9 | 11.9 | 5.5 | 1.5 |
| dBBAT017-P253 | 9.8 | 5.0 | 39.7 | 18.8 | 6.8 | 4.8 | 4.4 |
| dBBAT019-P253 | 3.8 | 3.0 | 25.7 | 7.4 | 11.1 | 4.6 | 3.7 |
| dBBAT021-P253 | 9.2 | 2.4 | 56.6 | 19.6 | 5.7 | 3.0 | 2.9 |
| dBBAT025-P253 | 28.5 | 10.0 | 89.1 | 55.7 | 16.4 | 6.1 | 4.4 |
| dBBAT029-P253 | 10.9 | 6.9 | 53.3 | 38.5 | 17.4 | 11.4 | 3.7 |
| dBBAT031-P253 | 8.4 | 5.0 | 47.8 | 33.3 | 14.8 | 8.3 | 2.8 |
| dBBAT037-P253 | 27.5 | 15.9 | 72.1 | 62.9 | 21.7 | 13.8 | 3.8 |
| dBBAT042-P253 | 11.7 | 4.3 | 58.7 | 19.1 | 7.8 | 4.7 | 2.9 |

(continued)

| Clone name | Small molecule species, concentration condition | | | | | | |
|---|---|---|---|---|---|---|---|
| | 1mM ATP | 100 μM ATP | 100 μM ADP | 10 μM ADP | 1mM AMP | 100 μM AMP | Without small molecule |
| dBBAT053-P253 | 15.0 | 8.4 | 46.9 | 40.4 | 9.6 | 6.9 | 1.9 |
| dBBAT056-P253 | 20.3 | 13.4 | 59.4 | 50.7 | 12.8 | 9.7 | 3.7 |
| dBBAT118-P253 | 27.7 | 14.1 | 76.9 | 62.9 | 14.9 | 8.7 | 3.3 |
| dBBAT121-P253 | 11.5 | 4.8 | 42.3 | 24.7 | 6.1 | 2.6 | 1.9 |
| dBBAT122-P253 | 11.5 | 4.7 | 55.5 | 19.2 | 5.7 | 4.1 | 3.3 |
| dBBAT134-P253 | 5.2 | 4.4 | 29.3 | 16.2 | 3.7 | 4.5 | 3.0 |
| dBBAT091-P253 | 6.2 | 3.0 | 33.0 | 20.0 | 2.9 | 3.3 | 1.5 |
| dBBAT112-P253 | 12.6 | 6.3 | 43.0 | 27.4 | 6.5 | 4.8 | 3.7 |
| dBBAT119-P253 | 45.7 | 12.9 | 112.5 | 67.2 | 58.6 | 26.9 | 3.3 |

(2-5-3) Assessment of binding activity to a monkey CD137 antigen

The obtained antibodies were evaluated for binding to monkey CD137 by ELISA. The cyCD137-Fc-BAP prepared in Reference Example 1 was immobilized on Streptawell microtiter plates. After the unbound antigen was removed from the plates by washing each well of the plates with the wash buffer, the wells were blocked with 150 μL of a blocking buffer (TBS with 2% BSA) for 1 hour or more. The blocking buffer was removed from each well, and 100 μL of the purified antibodies diluted in TBS with a final concentration of 1 mM ADP, or TBS with a final concentration 1 mM ADP was added to each well. The plate to which the antibody was added was shaken at 600 rpm for 1 hour. The AP-conjugated anti-human lambda antibodies (BETHYL) diluted in TBS with a final concentration of 1 mM ADP were added to each well after washing with a wash buffer (TBS with 0.1% Tween20) containing a final concentration of 1 mM ADP. After incubation for one hour and washing with a wash buffer containing a final concentration of 1 mM ATP, BluePhos phosphate substrate (KPL) was added. Color development was measured by absorbance at 600 nm. The increase rate in absorbance at an antibody concentration of 0 μg/mL is shown in Table 34. Samples with a concentration-dependent increase in the absorbance ratio can be said to be bound to monkey CD137.

Absorbance ratio

[Table 34]

| Monkey CD137 | | dBBAT007 | dBBAT013 | dBBAT015 | dBBAT017 | dBBAT019 | dBBAT021 | dBBAT025 | dBBAT029 | dBBAT037 | dBBAT042 |
|---|---|---|---|---|---|---|---|---|---|---|---|
| Antibody concentration [μg/mL] | 10 | 30.5 | 43.2 | 42.9 | 25.6 | 40.5 | 40.5 | 72.0 | 50.3 | 32.5 | 41.8 |
| | 2.5 | 30.2 | 42.4 | 32.4 | 8.7 | 40.3 | 14.0 | 67.6 | 42.7 | 12.7 | 40.0 |
| | 0.625 | 29.2 | 33.3 | 14.4 | 3.1 | 39.1 | 4.6 | 49.7 | 26.7 | 4.6 | 31.3 |
| | 0.156 | 22.0 | 15.3 | 3.6 | 1.4 | 23.7 | 1.7 | 20.7 | 9.7 | 1.9 | 12.2 |
| | 0.039 | 0.7 | 4.2 | 1.5 | 1.2 | 8.2 | 1.2 | 4.5 | 2.0 | 1.3 | 2.9 |
| | 0.010 | 3.8 | 1.5 | 1.1 | 1.2 | 2.5 | 0.9 | 1.6 | 1.1 | 1.1 | 1.3 |
| | 0 | 1.0 | 1.0 | 1.0 | 1.0 | 1.0 | 1.0 | 1.0 | 1.0 | 1.0 | 1.0 |

| | | dBBAT053 | dBBAT056 | dBBAT091 | dBBAT112 | dBBAT118 | dBBAT119 | dBBAT121 | dBBAT122 | dBBAT134 |
|---|---|---|---|---|---|---|---|---|---|---|
| Antibody concentration [μg/mL] | 10 | 17.9 | 23.1 | 15.1 | 19.8 | 35.4 | 55.3 | 26.0 | 31.9 | 33.9 |
| | 2.5 | 6.9 | 10.7 | 5.5 | 8.1 | 30.6 | 56.6 | 16.4 | 16.7 | 16.8 |
| | 0.625 | 2.3 | 3.6 | 1.7 | 2.8 | 18.1 | 57.2 | 5.9 | 5.7 | 5.8 |
| | 0.156 | 1.0 | 1.3 | 0.7 | 1.0 | 7.1 | 47.7 | 1.3 | 1.9 | 2.2 |
| | 0.039 | 0.7 | 0.8 | 0.5 | 0.6 | 1.8 | 26.6 | 0.6 | 1.0 | 1.2 |
| | 0.010 | 0.8 | 1.2 | 0.6 | 0.6 | 1.0 | 10.2 | 0.6 | 0.9 | 1.0 |
| | 0 | 1.0 | 1.0 | 1.0 | 1.0 | 1.0 | 1.0 | 1.0 | 1.0 | 1.0 |

(2-5-4) Assessment of CD137 agonist activity using Jurkat cells.

The GloResponse™ NF-kappa B-Luc2/4-1BB Jurkat cell line (Promega) was used for measuring the *in vitro* antibody activity. To each well of 384-well plates, 10μL of FcγRIIB CHO-K1 cells (Promega) prepared at a concentration of 2 × 10⁶/mL with an assay medium (99% RPMI, 1% FBS) was added. Subsequently, 10 μL of an antibody solution containing ADP, an antibody solution containing ATP, or an antibody solution containing no ATP or ADP was added to each well. Then, 10 μL of the GloResponse™ NF-κB-Luc2/4-1BB Jurkat cell line prepared to 2 × 10⁶/mL with an assay medium (99% RPMI, 1% FBS) was added to each well. The final concentration of ADP was 50 μM; and the final concentration of ATP was 50 μM. Plates were left to stand for six hours at 37°C in a 5% $CO_2$ incubator followed by 15 minutes at room temperature, and 30 μL of the Bio-Glo reagent was added to each well. The Bio-Glo Luciferase Assay System (Buffer and Substrate) was used for the Bio-Glo reagent. Subsequently, the luminescence of each well was measured with a plate reader. The value of the luminescence of each well divided by the value of the luminescence of the well without antibody added was the luminescence fold and served as an indicator for evaluating the activity of each antibody.

The results obtained are shown in Figures 22 and 23. From Figures 22 and 23, it was confirmed that all antibodies except NS1-P253, which is a non-switch antibody, exhibited human CD137 agonist activity in an ATP- and ADP-dependent manner.

(2-6) Assessment *of in vitro* CD137 agonist activity of switch antibodies acquired from panning using human T cells

(2-6-1) Expansion of human T cell cultures

Human peripheral blood mononuclear cells isolated from blood samples of healthy volunteers were used. 50 mL of blood was mixed with 0.5 mL of heparin and further diluted with 50 mL of PBS. Human peripheral blood mononuclear cells were isolated in the two steps below. In the first step, Leucosep (greiner bio-one) added with Ficoll-Paque PLUS (GE Healthcare) was centrifuged at 1000 × g for one minute at room temperature, followed by addition of PBS-diluted blood, and centrifugation at room temperature was performed at 400 × g for 30 minutes. In the second step, the buffy coat was collected from the tubes after centrifugation and then washed with 60 ml of PBS (Wako). Then, a T cell activation/expansion kit/human (MACS Miltenyi biotec) was used to expand cultures of the T cells.

(2-6-2) Assessment of CD137 agonist activity *in vitro* using human T cells

2 × 10⁴ human peripheral blood mononuclear cell-derived T cells and 2 × 10⁴ REC-1 cells were suspended in 100 μL of RPMI1640 medium containing 30 U/mL human IL-2 (SIGMA), 10 ng/mL PMA (SIGMA), 0.5 μg/mL Ionomycin, 500 μM ADPbetaS (Sigma), and 10% FBS (Sigma), and seeded in 96-well multiple-well flat-bottom plates (Corning) with the control antibody IC17HdK-hIgG1/IC17L-k0, NS1-P253 which is an antibody with ATP-independent human CD137-binding activity (referred to as "non-switch antibody" or "non-switch CD137 antibody" throughout the Examples and Reference Examples), or any of the clones described in Table 32. NS1-P253, IC17HdK-hIgG1/IC17L-k0 and the clones described in Table 32 were evaluated at 10 $\mu$g/mL. IFN-$\gamma$ production in the ADPbetaS-minus medium was also evaluated. The plates were shaken and then left at 37°C for 72 hours in a 5% $CO_2$ incubator. Subsequently, the culture supernatants were harvested and the amount of IFN-$\gamma$ contained in the culture supernatants was quantified using the Human IFN-$\gamma$ ELISA Development Kit (PeproTech). ELISA was performed according to the instructions of the kit manufacturer (PeproTech). Absorbance measurements were made with EnVision (PerkinElmer).

The results are shown in Figure 24.

It was confirmed that dBBAT007-P253, dBBAT013-P253, dBBAT015-P253, dBBAT019-P253, dBBAT021-P253, dBBAT025-P253, dBBAT031-P253, dBBAT042-P253, dBBAT056-P253, dBBAT118-P253, dBBAT121-P253, dBBAT122-P253, and dBBAT119-P253 exhibit the human CD137 agonist activity in an ADPbetaS-dependent manner. ADPbetaS is an analogue of ADP that is less susceptible to hydrolysis than ADP. This indicates a possibility that these human CD137 switch antibodies exhibit human CD137 agonist activity in a manner dependent on a small molecule such as ATP, ADP or AMP.

(2-6-3) Assessment of CD137 agonist activity *in vitro* using human T cells (2)

$2 \times 10^4$ human peripheral blood mononuclear cell-derived T cells and $2 \times 10^4$ REC-1 cells were suspended in 100 $\mu$L of RPMI1640 medium containing 30 U/mL human IL-2 (SIGMA), 10 ng/mL PMA (SIGMA), 0.5 $\mu$g/mL Ionomycin, 500 $\mu$M ADPbetaS (Sigma), and 10% FBS (Sigma), and seeded in 96-well multiple-well flat-bottom plates (Corning), with either NS1-P253 (non-switch antibody) or dBBAT119-P253. NS1-P253 and dBBAT119-P253 were evaluated at 10, 2, 0.4, 0.08, 0.016, 0.0032, and 0.00064 $\mu$g/mL. The plates were shaken and then left at 37°C for 72 hours in a 5% $CO_2$ incubator. Subsequently, the culture supernatants were harvested and the amount of IFN-$\gamma$ contained in the culture supernatants was quantified using the Human IFN-$\gamma$ ELISA Development Kit (PeproTech). ELISA was performed according to the instructions of the kit manufacturer (PeproTech). Absorbance measurements were conducted with EnVision (PerkinElmer).

The results are shown in Figure 25.

It was confirmed that dBBAT119-P253 exhibits human CD137 agonist activity in the presence of ADP$\beta$S. This indicates a possibility that dBBAT119-P253 exhibits human CD137 agonist activity in a manner dependent on a small molecule such as ATP, ADP or AMP.

Reference Example 3: Enhancement of binding activity of antibodies that bind antigens in the presence of small molecules using rationally designed light-chain and heavy-chain libraries

(3-1) Construction of a library for enhancing binding activity using a rationally designed light chain library

For the antibody library containing a large number of antibodies harvested in Reference Example 2-2-1 with ATP-dependent antigen binding activity, enhancement of binding activity was performed by re-librarization of the antibody light chains.

The light-chain and heavy-chain regions of a rationally designed antibody phage display library constructed in the prior patent WO2015/083764 were used to construct an antibody light chain library and an antibody heavy chain library for enhancing binding activity. They were introduced into the above light chain library or the light-chain or heavy-chain regions of the phagemid vector library harvested in Reference Example 2-2-1, and introduced into the *E. coli* strain ER2738 by electroporation.

(3-2) Enhancement of binding activity of antibodies with ATP-dependent antigen-binding activity using a rationally designed library

Phages were produced in a general method from *E. coli* carrying the constructed phage display phagemid. Specifically, *E. coli* carrying the constructed phagemid vector was infected with M13KO7TC (WO2015/046554) or M13KO7$\Delta$pIII (hyperphage) (PROGEN Biotechnik), and phages were harvested from supernatants cultured overnight at 30°C. A phage library solution was obtained by diluting with TBS a population of phages precipitated by adding 2.5 M NaCl /10% PEG to the *E. coli* culture in which phage production was performed. BSA was then added to the phage library solution at a final concentration of 4%. Panning was performed using the antigen immobilized on magnetic beads. NeutrAvidin coated beads (Sera-Mag SpeedBeads NeutrAvidin-coated), NeutrAvidin beads (TAMAGAWA SEIKI) or Dynabeads MyOne StreptAvidin T1 (Thermo Fisher Scientific) were used as magnetic beads. As antigen, biotinylated hCD137-Fc was used.

Panning was performed to efficiently obtain small molecule switch antibodies which are dependent on the small molecule that can play a switch role in cancer tissues. Specifically, panning to enrich antibodies that bind to antigens in the presence of adenosine triphosphate (adenosine 5'-triphosphate; ATP) which is a small molecule, and which do not bind antigens in the absence of ATP was performed with reference to the methods described in the prior patent WO2015/083764.

(3-3) Assessment of binding activity in the presence and absence of ATP by phage ELISA

From single colonies of *E. coli* obtained after each round by the methods described above, phage-containing culture supernatants were harvested using a conventional method (Methods Mol. Biol. (2002) 178, 133-145). Binding activity to human CD137 in the presence and absence of ATP was then confirmed by phage ELISA with the methods described in Reference Example 2-2-2.

The results are shown in Figure 26.

A number of clones were obtained that were judged to be clones having ATP-dependent antigen binding activity, with an S/N ratio of absorbance greater than 2 in the presence/absence of the antigen and an S/N ratio of absorbance greater than 2 in the presence/absence of ATP (switch clones).

Reference Example 4: Production of modified CD137 antibodies and assessment of their activities

(4-1) Increase of binding activity due to alteration of dBBAT119H-P253/dBBAT119L-LamLib

Variants of the heavy chain variable region, dBBAT119H, and the light-chain variable region, dBBAT119L, of the anti-CD 137 antibody (clonal name: dBBAT119H-P253/dBBAT119L-LamLib) obtained in Reference Example 2-4, were produced by methods known to those skilled in the art, such as PCR. For the heavy-chain variable region, variants were made in which D10 (referring to aspartic acid (Asp) at position 10 (Kabat numbering)) and G17 (referring to glycine (Gly) at position 17 (Kabat numbering)) of dBBAT119H were replaced with glycine (Gly) and serine (Ser), respectively, to produce dBBAT119H010; and N99 (referring to asparagine (Asn) at position 99 (Kabat numbering)), M100a (referring to methionine (Met) at position 100a (Kabat numbering)), and N100b (referring to asparagine (Asn) at position 100b (Kabat numbering)) of dBBAT119H010 were replaced with other amino acids. For the light-chain variable region, variants were created in which F87 (referring to phenylalanine (Phe) at position 87 (Kabat numbering)) of dBBAT119L was replaced with tyrosine (Tyr) to produce dBBAT119L010; and D27b (referring to aspartate (Asp) at position 27b (Kabat numbering)), N31 (referring to asparagine (Asn) at position 31 (Kabat numbering)), and D94 (referring to aspartate (Asp) at position 94 (Kabat numbering)) of dBBAT119L010 were replaced with other amino acids.

For variants of the heavy-chain variable region, binding activity to human CD137 was measured by BiacoreT200 (GE Healthcare), a surface plasmon resonance analyzer. Antibodies were captured by interacting purified variants with the Protein G (CALBIOCHEM)-immobilized Series S Sensor Chip CM3 (GE Healthcare). An ATP-supplemented human CD137 (FXa digested) solution or a human CD137 (FXa digested) solution without ATP was then interacted in the presence of ATP and in the absence of ATP to assess the binding activity of the variants towards human CD137 (FXa digested). Measurements were taken at 25 °C using 20 mM ACES, 150 mM NaCl, 0.05% Tween20, and 2 mM $MgCl_2$, pH 7.4 as running buffer. The results of measurements showed that the L100a variant (the variant in which methionine (Met) at position 100a (Kabat numbering) was replaced with leucine (Leu)) had enhanced binding activity to human CD137 only in the presence of ATP (Figure 27). The heavy-chain variable region of the L100a variant is dBBATk1 19H024 (SEQ ID NO: 132). The binding amount of human CD137 was adjusted by the capture amount (1000 RU) of each variant.

For variants of the light-chain variable region, the binding activity to human CD137 (FXa digested) was determined by BiacoreT200 under similar conditions as described above. The results of measurements showed that the E94 variant (a variant in which aspartate (Asp) at position 94 (Kabat numbering) was replaced by glutamate (Glu)) had enhanced binding activity to human CD137 only in the presence of ATP (Figure 27). The light-chain variable region of the E94 variant is dBBATk1 19L020 (SEQ ID NO: 133).

Such variants combining these heavy-chain and light-chain variants are abbreviated as dBBATk119H024-P253/dBBATk119L020-LamLib (heavy-chain variable region SEQ ID NO: 132; light-chain variable region SEQ ID NO: 133; heavy-chain constant region SEQ ID NO: 93; and light-chain constant region SEQ ID NO: 63).

The antibodies in the present specification are designated according to the following rule: (heavy chain variable region)-(heavy chain constant region)/(light chain variable region)-(light chain constant region).

For example, by the antibody name of dBBAT119H-P253/dBBAT119L-LamLib, it means that the heavy chain variable region of the antibody is dBBAT119H, the heavy chain constant region is P253, the light chain variable region is dBBAT119L, and the light chain constant region is LamLib.

(4-2) Assessment of CD137 agonist activity of modified anti-human CD137 antibodies *in vitro* using human T cells

(4-2-1) Expansion of human T cell cultures

Human T cells were expanded and cultured as described in Reference Example 2-6-1.

(4-2-2) Assessment of CD137 agonist activity *in vitro* using human T cells

The methods described in Reference Example 2-6-2 evaluated IFN-γ production in the presence of NS1-P253 (non-switch antibody), dBBAT119H-P253/dBBAT119L-LamLib, dBBATk119H024-P253/dBBATk119L020-LamLib, or the control antibody, IC17HdK-hIgG1/IC17L-k0 at 10, 2, 0.4, 0.08, and 0.016 μg/ml, respectively. IFN-γ production in the ADPbetaS-minus medium was also evaluated.
The both results are shown in Figure 28.
The modified dBBATk119H024-P253/dBBATk119L020-LamLib exhibited a stronger ADPbetaS-dependent agonist activity than dBBAT119H-P253/dBBAT119L-LamLib. This indicates a possibility that dBBATk119H024-P253/dBBATk119L020-LamLib exhibits stronger ATP-, ADP-, and AMP-dependent human CD137 agonist activities than dBBAT119H-P253/dBBAT119L-LamLib.

Reference Example 5: Further modifications of the CD137 antibodies

(5-1) Search for modifications that increase the binding activity by introducing comprehensive modifications

To produce superior anti-CD 137 antibodies, amino acid modifications were introduced comprehensively into the heavy chain variable region, dBBATk119H024, and the light chain variable region, dBBATk119L020, of the anti-CD 137 antibodies produced in Reference Example 4-1. By methods known to those skilled in the art, such as PCR, variants were each made in which each of all the amino acid residues constituting the CDRs of dBBATk119H024 and dBBATk119L020 were replaced by all 18 amino acids except cysteine. Measurements of the approximately 1200 variants produced to bind human CD137 were performed using Biacore4000. Antibodies were captured by interacting culture supernatants of the variants with the Protein G (CALBIOCHEM)-immobilized Series S Sensor Chip CM3 (GE Healthcare). A small molecule (ATP)-supplemented human CD137 solution or a human CD137 solution without addition of the small molecule was then interacted in the presence of the small molecule or in the absence of the small molecule to assess the binding activity of the antibodies towards human CD137. Measurements were taken at 25°C using CaCl$_2$-supplemended running buffer of 20 mM ACES, 150 mM NaCl, 0.02% Tween20, and 2 mM MgCl$_2$, pH 7.4.

(5-2) Increase of ATP binding

The antibody heavy-chain gene A002-P253 (SEQ ID NO: 134) was created by combining the modifications found in Reference Example 5-1 that increase the binding activity to human CD137 in the presence of the small molecule, in the dBBATk119H024-P253 gene having dBBATk119H024 (SEQ ID NO: 132) as the heavy-chain variable region, and P253 (SEQ ID NO: 93) as the heavy-chain constant region which was generated by introducing S267E/L328F modification into and deleting C-terminal Gly and Lys from human IgG1. The antibody light-chain gene B040-Lamlib (SEQ ID NO: 135) was also created by combining the modifications found in Reference Example 5-1 that increased the binding activity to human CD137 in the presence of the small molecule, in the antibody light chain dBBATk119L020-Lamlib having dBBATk119L020 (SEQ ID NO: 133) as the light-chain variable region and human λ chain Lamlib (SEQ ID NO: 63) as the light-chain constant region. These genes were combined to express and purify antibodies by methods known to those skilled in the art to produce the anti-CD137 antibody of interest, A002-P253/B040-Lamlib. The heavy-chain variable region of A002-P253/B040-Lamlib is A002 (SEQ ID NO: 136), the light-chain variable region is B040 (SEQ ID NO: 137), the heavy-chain constant region is P253 (SEQ ID NO: 93), and the light-chain constant region is human λ chain Lamlib (SEQ ID NO: 63).
For the heavy-chain variable region of A002-P253/B040-Lamlib produced in this section, various variants were made in which the 53rd, 54th or 55th amino acids in Kabat numbering were replaced by other amino acids with the purpose of increasing ATP-binding activity. Table 35 shows amino acid modifications (Kabat numbering) from A002 in the heavy-chain variable regions of the generated antibodies.

[Table 35]

| Heavy-chain variable region | Amino acid alteration from A002 (Kabat numbering) |
| --- | --- |
| A002 | - |

(continued)

| Heavy-chain variable region | Amino acid alteration from A002 (Kabat numbering) |
|---|---|
| A146 | S54A / N55S |
| A159 | R53S / N55S |
| A160 | R53T / N55S |
| A161 | R53Q / N55S |
| A162 | R53K / N55S |
| A163 | R53H / N55S |
| A164 | R53S / N55T |
| A165 | R53T / N55T |
| A166 | R53Q / N55T |
| A167 | R53K / N55T |
| A168 | R53H / N55T |
| A169 | R53S / N55H |
| A170 | R53T / N55H |
| A171 | R53Q / N55H |

The binding activities of the produced variants towards ATP and human CD137 were evaluated by Biacore T200.

Measurement of the ATP-binding activity was performed at 37°C using 20 mM ACES (pH 7.4), 150 mM NaCl, 2 mM MgCl$_2$, and 0.05% Tween20 as running buffer. At first, antibodies were captured by interacting antibody solutions prepared in running buffer with the Sure Protein A (GE Healthcare)-immobilized Series S Sensor Chip CM3 (GE Healthcare). Binding activity of the antibodies was then assessed by interaction with an ATP solution prepared in running buffer. The chip was regenerated using 25 mM NaOH and 10 mM Glycine-HCl (pH 1.5), and measurements were performed by repeatedly capturing antibodies. The binding amount of ATP for each antibody was calculated by adjusting the binding amount of ATP when injected at the concentration of 100 nM by the amount of antibody captured on the chip surface as the amount of ATP per unit amount of antibody.

Measurement of the binding activity towards human CD137 was performed at 37°C using 20 mM ACES (pH 7.4), 150 mM NaCl, 2 mM MgCl$_2$, and 0.05% Tween20 as running buffer. At first, antibodies were captured by interacting antibody solutions prepared in running buffer with the Sure Protein A (GE Healthcare)-immobilized Series S Sensor Chip CM3 (GE Healthcare). Then, the binding activity to human CD137 was assessed by interaction with a human CD137 solution supplemented with 100 μM ATP as the small molecule. For the human CD137 antigen, hCD137-HisBAP prepared in Reference Example (1-1) was used, and measurements were performed at the antigen concentration of 0, 15.625, 62.5, 250 or 1000 nM. The chip was regenerated using 25 mM NaOH and 10 mM Glycine-HCl (pH 1.5), and measurements were performed by repeatedly capturing antibodies. Dissociation constant (KD) of the respective antibodies for human CD137 was calculated using Biacore T200 Evaluation Software 2.0. Specifically, the association rate constant ka (L/mol/s) and the dissociation rate constant kd (1/s) were calculated by global fitting of the sensorgrams obtained by measurement with the 1:1 Langmuir binding model, and the dissociation constant KD (mol/L) was calculated from these values.

Table 36 shows the results of these measurements.

Analysis to binding to ATP and human CD137

[Table 36]

| Antibody name | Binding to ATP | Parameter for binding to human CD137 (ATP = 100 μM) | | |
|---|---|---|---|---|
| | | ka | kd | KD |
| A002-P253/B040-Lamlib | -0.0001 | 3.94E+04 | 2.89E-03 | 7.35E-08 |

(continued)

| Antibody name | Binding to ATP | Parameter for binding to human CD137 (ATP = 100 μM) | | |
| --- | --- | --- | --- | --- |
| | | ka | kd | KD |
| A146-P253/B040-Lamlib | -0.0001 | 7.80E+04 | 2.32E-02 | 2.97E-07 |
| A159-P253/B040-Lamlib | 0.0013 | 8.21E+04 | 3.83E-02 | 4.66E-07 |
| A160-P253/B040-Lamlib | -0.0001 | 9.59E+03 | 2.46E-02 | 2.56E-06 |
| A161-P253/B040-Lamlib | 0.0010 | 1.87E+04 | 2.35E-02 | 1.25E-06 |
| A162-P253/B040-Lamlib | 0.0002 | 1.47E+05 | 3.17E-02 | 2.16E-07 |
| A163-P253/B040-Lamlib | 0.0016 | 5.60E+04 | 1.36E-02 | 2.43E-07 |
| A164-P253/B040-Lamlib | 0.0017 | 9.81E+04 | 4.38E-02 | 4.46E-07 |
| A165-P253/B040-Lamlib | 0.0002 | 1.14E+04 | 1.96E-02 | 1.72E-06 |
| A166-P253/B040-Lamlib | 0.0011 | 1.21E+04 | 1.92E-02 | 1.59E-06 |
| A167-P253/B040-Lamlib | 0.0001 | 8.45E+04 | 3.96E-02 | 4.69E-07 |
| A168-P253/B040-Lamlib | 0.0017 | 7.01E+04 | 1.81E-02 | 2.58E-07 |
| A169-P253/B040-Lamlib | 0.0011 | 1.15E+04 | 5.88E-03 | 5.11E-07 |
| A170-P253/B040-Lamlib | 0.0000 | 5.13E+03 | 5.36E-03 | 1.04E-06 |
| A171-P253/B040-Lamlib | 0.0012 | 4.93E+03 | 5.24E-03 | 1.06E-06 |

As shown in Table 36, variants in which the 53rd, 54th or 55th amino acids (Kabat numbering) of the heavy-chain variable region were replaced by other amino acids had increased ATP-binding activity compared to the A002-P253/B040-Lamlib antibody before modification, except for A146-P253/B040-Lamlib and A160-P253/B040-Lamlib. Comparison of the association rate constant ka (L/mol/s) also shows that the association rate constant for human CD137 was increased for A146-P253/B040-Lamlib, A159-P253/B040-Lamlib, A162-P253/B040-Lamlib, A163-P253/B040-Lamlib, A164-P253/B040-Lamlib, A167-P253/B040-Lamlib and A168-P253/B040-Lamlib among the antibodies evaluated above.

(5-3) Increase of the binding activity by introduction of comprehensive modifications

To generate better anti-CD 137 antibodies, modifications found in Reference Example 5-1 that increase the human CD137-binding activity in the presence of a small molecule and that decrease binding to human CD137 under conditions where the small molecule is absent, and modifications found in Reference Example 5-2 that increase ATP-binding activity and increase the association rate constant for human CD137 were combined to generate anti-human CD137 antibodies that exhibit a better profile. Antibody heavy-chain genes were generated that combine the modifications found in Reference Examples 5-1 and 5-2 in the antibody heavy-chain gene A002-G1T3 having A002 (SEQ ID NO: 136) as the heavy-chain variable region, and G1T3 (SEQ ID NO: 138) as the heavy-chain constant region, which was generated by introducing K214R/Q419E modification into and deleting C-terminal Gly and Lys from human IgG1. An antibody light-chain genes were generated by combining the modifications found in Reference Example 5-1 in the antibody light chain B040-Lamlib having B040 (SEQ ID NO: 137) as the light-chain variable region and human λ chain Lamlib as the light-chain constant region.

As subject of comparison, the antibody heavy-chain gene 20H4.9-P253 having the heavy-chain variable region 20H4.9 (SEQ ID NO: 139) of the existing anti-CD 137 antibody described in US8137667 and P253 (SEQ ID NO: 93) as the heavy-chain constant region; and an antibody light-chain gene that combines the light-chain variable region 20H4.9LC (SEQ ID NO: 140) with human κ chain k0 (SEQ ID NO: 141) as the light-chain constant region were generated. As another subject of comparison, the antibody heavy-chain gene MOR-7480.1H-P253 having the heavy-chain variable region MOR-7480.1H (SEQ ID NO: 142) which constitutes the existing anti-CD137 antibody MOR-7480.1 described in US8337850, and P253 (SEQ ID NO: 93) as the heavy-chain constant region; and the antibody light chain MOR-7480.1L-lam combining the light chain variable region MOR-7480.1L (SEQ ID NO: 143) with human λ chain lam (SEQ ID NO: 63) as the light-chain constant region, were produced (note: both human λ chain Lamlib and lam have the same amino acid sequence (SEQ ID NO: 63)).

These genes were combined to express and purify antibodies using methods known to those skilled in the art to produce the anti-CD 137 antibodies of interest. Table 37 is a list of sequence numbers of the heavy-chain variable region, light-chain variable region, heavy-chain constant region, light-chain constant region, and hypervariable region (Hyper Variable Region; also referred to as HVR or CDR) of the antibodies generated.

Amino acid sequences of the heavy chains and light chains, and their hypervariable regions (indicated by sequence ID numbers)

[Table 37]

| Antibody name | Variable region | | Constant region | | Hypervariable region (HVR) | | | | | |
|---|---|---|---|---|---|---|---|---|---|---|
| | Heavy chain | Light chain | Heavy chain | Light chain | H1 | H2 | H3 | L1 | L2 | L3 |
| A375-G1T3/ B167-Lamlib | 43 | 54 | 138 | 63 | 7 | 8 | 17 | 21 | 26 | 27 |
| A372-G1T3/ B040-Lamlib | 44 | 55 | 138 | 63 | 7 | 9 | 17 | 22 | 26 | 27 |
| A356-G1T3/ B040-Lamlib | 45 | 55 | 138 | 63 | 7 | 10 | 17 | 22 | 26 | 27 |
| A486-G1T3/ B167-Lamlib | 46 | 54 | 138 | 63 | 7 | 11 | 18 | 21 | 26 | 27 |
| A487-G1T3/ B167-Lamlib | 47 | 54 | 138 | 63 | 7 | 8 | 18 | 21 | 26 | 27 |
| A488-G1T3/ B226-Lamlib | 48 | 56 | 138 | 63 | 7 | 12 | 18 | 21 | 26 | 28 |
| A489-G1T3/ B223-Lamlib | 49 | 57 | 138 | 63 | 7 | 13 | 18 | 21 | 26 | 29 |
| A548-G1T3/ B376-Lamlib | 50 | 58 | 138 | 63 | 7 | 14 | 19 | 23 | 26 | 27 |
| A551-G1T3/ B256-Lamlib | 51 | 59 | 138 | 63 | 7 | 15 | 20 | 24 | 26 | 27 |
| A551-G1T3/ B379-Lamlib | 51 | 60 | 138 | 63 | 7 | 15 | 20 | 25 | 26 | 27 |
| A555-G1T3/ B379-Lamlib | 52 | 60 | 138 | 63 | 7 | 16 | 20 | 25 | 26 | 27 |
| A548-G1T3/ B256-Lamlib | 50 | 59 | 138 | 63 | 7 | 14 | 19 | 24 | 26 | 27 |
| A549-G1T3/ B167-Lamlib | 53 | 54 | 138 | 63 | 7 | 14 | 17 | 21 | 26 | 27 |

Binding of the generated variants to ATP and to human CD137 was evaluated in Biacore T200. Measurements of human CD137 binding were performed at 37°C using 20 mM ACES (pH 7.4), 150 mM NaCl, 2 mM $MgCl_2$, and 0.05% Tween20 as running buffer. At first, 250-400 RU of antibodies were captured by interacting the antibody solutions prepared in running buffer with the Sure Protein A (GE Healthcare)-immobilized Series S Sensor Chip CM3 (GE Healthcare). The human CD137-binding activity in the presence of ATP or in the absence of ATP was then assessed by interacting with a human CD137 solution prepared in running buffer supplemented with a desired concentration of ATP, or a human CD137 solution prepared in running buffer without ATP. For the human CD137 antigen, hCD137-HisBAP prepared in Reference Example (1-1) was used, and the KD value measurements were performed at an antigen concentration of 0, 15.625, 62.5, 250, and 1000 nM. For evaluation of the binding amount, measurements were performed at an antigen concentration of 0 and 1000 nM. The chip was regenerated using 25 mM NaOH and 10 mM Glycine-HCl (pH 1.5), and measurements were performed by repeatedly capturing antibodies. Dissociation constant of the respective

123

antibodies for human CD137 was calculated using Biacore T200 Evaluation Software 2.0. Specifically, the association rate constant ka (L/mol/s) and the dissociation rate constant kd (1/s) were calculated by global fitting of the sensorgrams obtained by measurement with the 1:1 Langmuir binding model, and the dissociation constant KD (mol/L) was calculated from these values.

Table 38 shows the results of these measurements.

Binding analysis of the modified antibodies to human CD137

[Table 38]

| Antibody name | Binding to human CD137 | | | | | | $K_D$ (M) for human CD137 | | | | | |
|---|---|---|---|---|---|---|---|---|---|---|---|---|
| | Without ATP | ATP = 1 $\mu$M | ATP = 10 $\mu$M | ATP = 100 $\mu$M | ADP = 10 $\mu$M | AMP = 10 $\mu$M | Without ATP | ATP = 1 $\mu$M | ATP = 10 $\mu$M | ATP = 100 $\mu$M | ADP = 10 $\mu$M | AMP = 10 $\mu$M |
| 20H4.9-P253/20H4.9LC-k0 | 0.280 | 0.283 | 0.283 | 0.283 | 0.287 | 0.287 | 2.68E-08 | 2.17E-08 | 2.03E-08 | 2.04E-08 | 1.90E-08 | 2.17E-08 |
| MOR-7480.1H-P253/ MOR-7480.1L-lam | 0.260 | 0.261 | 0.261 | 0.261 | 0.266 | 0.266 | 8.22E-08 | 8.40E-08 | 7.50E-08 | 6.31E-08 E-08 | 7.30E-08 | 8.46E-08 |
| A375-G1T3/B167-Lamlib | 0.053 | 0.179 | 0.255 | 0.275 | 0.280 | 0.276 | N.A. | 4.43E-07 | 6.17E-08 | 1.35E-08 | 5.43E-09 | 8.91E-09 E-09 |
| A372-G1T3/B040-Lamlib | 0.017 | 0.135 | 0.242 | 0.269 | 0.284 | 0.245 | N.A. | 1.14E-06 | 1.58E-07 | 5.25E-08 | 9.36E-09 | 1.47E-07 |
| A356-G1T3/B040-Lamlib | 0.008 | 0.083 | 0.211 | 0.262 | 0.278 | 0.255 | N.A. | N.A. | 4.90E-07 | 1.19E-07 | 3.31E-08 E-08 | 1.34E-07 |
| A486-G1T3/B167-Lamlib | 0.128 | 0.260 | 0.282 | 0.292 | 0.294 | 0.289 | 1.04E-06 * | 7.30E-08 | 1.08E-08 | 3.22E-09 | 2.48E-09 | 4.33E-09 |
| A487-G1T3/B167-Lamlib | 0.110 | 0.229 | 0.270 | 0.282 | 0.283 | 0.279 | 1.43E-06 * | 1.63E-07 | 2.70E-08 | 7.33E-09 | 3.01E-09 E-09 | 3.86E-09 |
| A488-G1T3/B226-Lamlib | 0.042 | 0.135 | 0.232 | 0.268 | 0.281 | 0.261 | N.A. | 4.24E-07 | 1.33E-07 | 5.77E-08 | 1.60E-08 | 6.80E-08 |
| A489-G1T3/B223-Lamlib | 0.004 | 0.095 | 0.214 | 0.257 | 0.276 | 0.261 | N.A. | 4.42E-06 | 3.45E-07 | 1.59E-07 | 5.45E-08 | 1.05E-07 |
| A548-G1T3/B376-Lamlib | 0.012 | 0.149 | 0.245 | 0.270 | 0.270 | 0.262 | NA | 6.75E-07 | 7.69E-08 | 1.53E-08 | 1.55E-08 | 3.71E-08 E-08 |
| A551-G1T3/B256-Lamlib | 0.006 | 0.133 | 0.254 | 0.285 | 0.285 | 0.275 | N.A. | 1.23E-06 | 1.44E-07 | 2.91E-08 E-08 | 2.81E-08 E-08 | 6.12E-08 |
| A551-G1T3/B379-Lamlib | 0.014 | 0.173 | 0.271 | 0.290 | 0.291 | 0.283 | N.A. | 5.59E-07 | 7.17E-08 | 1.58E-08 | 1.51E-08 E-08 | 3.25E-08 |
| A555-G1T3/B379-Lamlib | 0.017 | 0.180 | 0.267 | 0.282 | 0.282 | 0.274 | N.A. | 4.77E-07 | 5.84E-08 | 1.35E-08 | 1.31E-08 E-08 | 2.75E-08 |

125

(continued)

| Antibody name | Binding to human CD137 | | | | | | $K_D$ (M) for human CD137 | | | | | |
|---|---|---|---|---|---|---|---|---|---|---|---|---|
| | Without ATP | ATP = 1 $\mu$M | ATP = 10 $\mu$M | ATP = 100 $\mu$M | ADP = 10 $\mu$M | AMP = 10 $\mu$M | Without ATP | ATP = 1 $\mu$M | ATP = 10 $\mu$M | ATP = 100 $\mu$M | ADP = 10 $\mu$M | AMP = 10 $\mu$M |
| A548-G1T3/B256-Lamlib | 0.009 | 0.156 | 0.257 | 0.284 | 0.287 | 0.276 | N.A. | 9.31E-07 | 1.08E-07 | 2.63E-08 | 2.14E-08 | 4.61E-08 E-08 |
| A549-G1T3/B167-Lamlib | 0.048 | 0.209 | 0.261 | 0.277 | 0.279 | 0.272 | N.A. | 2.04E-07 | 2.83E-08 | 6.12E-09 | 5.53E-09 | 1.35E-08 |

N.A. : It is too weak to determine a KD value.
*The KD value was determined with a steady state model.

The values of "Binding to human CD137" in Table 38 above indicate the binding amount of human CD137 per unit amount of antibody when human CD137 was allowed to interact at 1000 nM under each ATP concentration condition described above, and "$K_D$ (M) for human CD137" indicates the dissociation constant for human CD137 under each ATP concentration condition. The KD values marked with * in the table were calculated with a steady state model. The binding amount of all the generated variants in the presence of 10 $\mu$M ATP was more than that under the condition where ATP was present at 1 $\mu$M, and even more in the presence of 100 $\mu$M, suggesting that they bind to human CD137 in an ATP concentration-dependent manner. On the other hand, the subjects of comparison, 20H4.9-P253/20H4.9LC-k0 and MOR-7480.1 H-P253/MOR-7480.1L-lam, did not show binding to human CD137 in an ATP concentration-dependent manner.

(5-4) Assessment of the *in vitro* ATP-dependent CD137 agonist activity of the modified anti-human CD137 antibodies using the 4-1BB Jurkat reporter gene assay

The GloResponse™ NF-κ B-Luc2/4-1BB Jurkat cell line (Promega, CS196004) was used for measurement of the *in vitro* activity of the generated variants. To each well of 96-well plates, 200 $\mu$L each of FcγRIIB CHO-K1 Cells (Promega) prepared at the concentration of 5 $\times$ 10$^4$/mL with the medium was added and left to stand overnight at 37°C in a 5% $CO_2$ incubator. A CHO culture medium (90% Ham's F12, 10% FBS) was used for the culture medium. Then, after all the medium was aspirated away, 25 $\mu$L of the GloResponse™ NF-κB-Luc2/4-1BB Jurkat cell line prepared to 2 $\times$ 10$^6$/mL with an assay medium (99% RPMI, 1% FBS) was added to each well. Subsequently, 25 $\mu$L of each antibody solution diluted with the assay medium was added so that the final concentration was 0, 0.001, 0.01, 0.1, 1, and 10 $\mu$g/mL; and finally 25 $\mu$L of an ATP solution diluted with the assay medium was added so that the final concentration became 0 and 250 $\mu$M. The plates were left to stand for 6 hours at 37°C in a 5% $CO_2$ incubator and 15 minutes at room temperature; and 75 $\mu$L of the Bio-Glo reagent was added to each well. The Bio-Glo Luciferase Assay System (Buffer and Substrate) was used for the Bio-Glo reagent. Subsequently, the luminescence of each well was measured with a plate reader. The value of the luminescence of each well divided by the value of the luminescence of the well without antibody addition was the Relative Light Unit (fold induction) and served as an indicator for evaluating the CD137 agonist activity of each antibody.
The results are shown in Figure 29.

(5-5) Assessment *of in vitro* ATP-dependent CD137 agonist activity of modified anti-human CD137 antibodies using human peripheral blood mononuclear cells

(5-5-1) Isolation of human peripheral blood mononuclear cells

Human peripheral blood mononuclear cells (PBMC) were isolated from blood samples of healthy volunteers in the Applicant's company isolated as described in Reference Example 2-6-1. Subsequently, the cells were diluted in a medium (5% human serum (SIGMA), 95% AIM-V (Thermo Fischer Scientific) at a cell density of 5 $\times$ 10$^6$/mL.

(5-5-2) Assessment of CD137 agonist activity using human peripheral blood mononuclear cells

Human peripheral blood mononuclear cells were adjusted to a cell density 5 $\times$ 10$^6$/mL and seeded into 96-well multiple-well flat-bottom plates (Corning) at 100 $\mu$L each. Subsequently, 50 $\mu$L of 0.04 $\mu$g/mL anti-human CD3ε antibody (BD, clone SP34) and 20 $\mu$g/mL anti-human CD28 antibody (BD, clone: CD28. 2) diluted in a medium were added. The plates were shaken and then left to stand in a 5% $CO_2$ incubator at 37°C for 6 hours. Each well was then supplemented with 25 $\mu$L of 2 mM ATP (SIGMA)-medium or an ATP-free medium and 25 $\mu$L of each antibody at 40 $\mu$g/mL, and the plates were shaken before being left in a 5% $CO_2$ incubator at 37°C for 18 hours. Subsequently, part of the culture supernatant was harvested, and the culture supernatant was used to quantify the IL-2 content in the culture supernatant using the Human IL-2 DuoSet ELISA kit (R&D systems) or the Human IL-2 ELISA Set (BD Biosciences). After harvesting the culture supernatant, the plates were left again in a 5% $CO_2$ incubator at 37°C for 24 hours. Subsequently, part of the culture supernatant was harvested, and the amount of IFN-γ contained in the culture supernatant was quantified using the Human IFN-γ DuoSet ELISA kit (R&D systems) or the Human IFN-γ ELISA Development Kit (PeproTech). ELISA was performed basically according to the attached protocol of the kit. For the Human IL-2 DuoSet ELISA kit (R&D systems) and the Human IFN-γ DuoSet ELISA kit (R&D systems), color development and termination of the color development were performed according to protocols using a substrate solution (R&D systems) containing $H_2O_2$ and tetramethylbenzidine, and IN $H_2SO_4$ (Wako). For the Human IL-2 ELISA Set (BD Biosciences), color development was terminated using 1 N $H_2SO_4$ (Wako). For the IFN-γ ELISA Development Kit (PeproTech), the TMB Chromogen Solution (Thermo Fischer Scientific) and IN $H_2SO_4$ (Wako) were used for color development and termination of the color development. Absorbance measurements were then made with EnVision (PerkinElmer).
The results are detailed in Reference Example 5-5-3 and later.

(5-5-3) Evaluation of the enhancement of agonist activity of the heavy chain constant region by increasing Fcγ receptor-binding activity

By *in vitro* assessment using human peripheral blood monocytes as described in Reference Examples 5-5-1 and 5-5-2, the effect of using P587, MY518, TT14, and TT16 which variously increased the FcγRIIb-binding activity of the heavy chain constant region on CD137 agonist activity was evaluated.

The antibodies evaluated are as shown in Table 39.

Production of the antibodies recited in Table 39 is described in Reference Example 7-1. Compared to the amounts of IL-2 and IFN-γ in the culture supernatant when a negative control antibody (IC17HdK-MY518/IC17L-k0, IC17HdK-G4d/IC17L-k0) was added in the presence of 250 μM ATP, when the addition of an antibody increased the amount of IL-2 in the culture supernatant by 1.05 fold or more and the amount of IFN-γ by 1.15 fold or more, it was judged that the antibody demonstrates CD137 agonist activity. Measurement of the agonist activity using human peripheral blood mononuclear cells (PBMC) may differ from donor to donor of the blood sample. In view of this point, it was determined that antibodies that did not show CD137 agonist activity for some or more than half of the human PBMC isolated from multiple donors can be judged not to show CD137 agonist activity, even if they met the standards for agonist activity for the other portion of human PBMC.

Antibodies which are judged to exhibit CD137 agonist activity in the presence of 250 μM ATP are shown in Table 39. This indicates that the CD137 agonist activity was enhanced by combining TT16 with P587 which increases the FcγRIIb-binding activity of the heavy chain constant region (Figures 30 and 31). It is thought that the agonistic activity was enhanced by increasing the binding activity towards FcγRIIb-expressing cells, which are cross-linking scaffolds required for the antibody to exhibit agonistic activity towards CD137-expressing cells (see Protein Eng Des Sel. 2013 Oct; 26(10): 589-598. Published online 2013 Jun 5. doi:10.1093/protein).

[Table 39]

| Name of antibody evaluated | Name of antibody judged to have activity in the presence of ATP |
|---|---|
| A375-MY518/B167-LamLib | A375-P587/B167-LamLib |
| A356-MY518/B040-LamLib | A356-P587/B040-LamLib |
| A375-P587/B167-LamLib | A375-TT16/B167-LamLib |
| A356-P587/B040-LamLib | A356-TT16/B040-LamLib |
| A375-TT16/B167-LamLib | |
| A356-TT16/B040-LamLib | |
| A375-TT14/B 167-LamLib | |

(5-5-4) Assessment of ATP-dependent CD137 agonist activity of variable regions

The ATP-dependent CD137 agonist activity of each variable region by combining the heavy chain constant regions P587 and P253 with various variable regions was evaluated by *in vitro* assessment using human peripheral blood monocytes as described in Reference Examples 5-5-1 and 5-5-2.

The antibodies evaluated are as shown in Table 40. Production of the antibodies recited in Table 40 is described in Reference Example 7-1. Compared to the amounts of IL-2 and IFN-γ in the culture supernatant when a negative control antibody (IC17HdK-P253/IC17L-k0, IC17HdK-P587/IC17L-k0) was added in the presence of 250 μM ATP, when the addition of an antibody increased the amount of IL-2 in the culture supernatant by 1.05 fold or more and the amount of IFN-γ by 1.15 fold or more, it was judged that the antibody demonstrates CD137 agonist activity. Measurement of the agonist activity using human peripheral blood mononuclear cells (PBMC) may differ from donor to donor of the blood sample. In view of this point, it was determined that antibodies that did not show CD137 agonist activity for some or more than half of the human PBMC isolated from multiple donors can be judged not to show CD137 agonist activity, even if they met the standards for agonist activity for the other portion of human PBMC. The antibodies judged to exhibit CD137 agonist activity in the presence of 250 μM ATP are as shown in Table 40 (Figure 32, Figure 33, Figure 34, Figure 35 and Figure 36).

In addition, among the antibodies judged to show CD137 agonist activity in the presence of 250 μM ATP, antibodies whose fold change relative to the group added with a negative control antibody (IC17HdK-P253/IC17L-k0, IC17HdK-P587/IC17L-k0) with respect to both the amounts of IL-2 and IFN-γ in the culture supernatant under the ATP-free condition was smaller than the fold change relative to the negative control under conditions in the presence of 250 μM ATP were judged to have a lower CD137 agonist activity under the ATP-free condition. The antibodies that showed CD137 agonist activity in the presence of 250 μM ATP and were judged to have lower CD137 agonist activity in the absence of ATP are as shown in Table 40 (Figure 32, Figure 33, Figure 34, Figure 35 and Figure 36). These antibodies were judged to exhibit ATP-dependent CD137 agonist activity.

From the above, it was confirmed that for the variable regions, the combinations of the heavy chain variable region/light chain variable region of (A375/B167), (A356/B040), (A372/B040), (A486/B167), (A488/B226), (A489/B223), (A551/B256), (A548/B256), and (A551/B379) exhibit CD137 agonist activity in an ATP-dependent manner.

[Table 40]

| Name of antibody evaluated | Name of antibody judged to have activity in the presence of ATP | Name of antibody judged to have lower activity in the absence of ATP |
|---|---|---|
| A372-P253/B040-LamLib | A372-P253/B040-LamLib | A372-P253/B040-LamLib |
| A486-P253/B167-LamLib | A486-P253/B167-LamLib | A486-P253/B167-LamLib |
| A488-P253/B226-Lam Lib | A488-P253/B226-LamLib | A488-P253/B226-Lam Lib |
| A489-P253/B223-LamLib | A489-P253/B223-LamLib | A489-P253/B223-LamLib |
| A551-P587/B256-LamLib | A551-P587/B256-LamLib | A551-P587/B256-LamLib |
| A375-P587/B167-LamLib | A375-P587/B167-LamLib | A375-P587/B167-LamLib |
| A548-P587/B256-Lam Lib | A548-P587/B256-LamLib | A548-P587/B256-Lam Lib |
| A356-P587/B040-Lam Lib | A356-P587/B040-LamLib | A356-P587/B040-LamLib |
| A551-P587/B379-LamLib | A551-P5871B379-LamLib | A551-P587/B379-Lam Lib |

(5-5-5) Assessment of the potentiating effect on CD137 agonist activity of combining alterations that increase the pI of the heavy-chain constant region with the heavy-chain constant region with increased Fcγ receptor-binding activity

The effect on CD137 agonist activity of introducing various amino acid modifications that increase the pI of the heavy-chain constant region into the heavy-chain constant region with variously increased FcγRIIb-binding activity was assessed by *in vitro* evaluation using human peripheral blood monocytes described in Reference Examples 5-5-1 and 5-5-2. The amino acid alterations that increase pI were introduced into the heavy-chain constant regions TT16, MY518, and TT14, and thereby heavy-chain constant regions TT16+P343R/D413K (SCF028), TT16+Q311R/P343R (SCF033), MY518+P343R/D413K (SCF025), MY518+Q311R/P343R (SCF030), TT14+P343R/D413K (SCF027), and TT14+Q311R/P343R (SCF032) were generated. The names of the evaluated antibodies into which the amino acid alterations that increase pI have been introduced, and the names of the corresponding antibodies before the amino acid alterations that increase the pI were introduced are as shown in Table 41. Production of the antibodies recited in Table 41 is described in Reference Example 7-2.

Regarding assessment of the enhancement of agonist activity by introducing pI-increasing amino acid modifications, when addition of an antibody increased the amount of IL-2 by 1.04 fold or more and the amount of IFN-γ by 1.1 fold or more in the culture supernatant relative to the group added with antibodies containing a heavy chain constant region that does not contain these amino acid modifications, A375-TT16/B167-LamLib, A375-MY518/B167-LamLib, and A375-TT14/B167-LamLib, it was judged that the CD137 agonist activity was enhanced. Compared to antibodies comprising a heavy-chain constant region without the pI-increasing amino acid modifications, antibodies comprising a heavy-chain constant region into which the amino acid modifications shown to have enhanced CD137 agonist activity in the presence of 250 μM ATP are shown in Table 42 (Figure 31).

This indicates that independent of the degree of increase in the FcγRIIb-binding activity of the heavy-chain constant region, introduction of the modifications that increase the pI of the heavy-chain constant region enhances the CD137 agonist activity of the anti-human CD137 antibody. It was suggested that: combining modifications that increase the pI of the heavy-chain constant region with modifications that increase the FcγRIIb binding of the heavy chain constant region into a heavy-chain constant region enhanced the interaction with the negatively charged FcγRIIb-expressing cell surface; and immune complexes of antibodies or antigen-bound antibodies approached the FcγRIIb-expressing cell surface more closely; and this further increased the binding to FcγRIIb-expressing cells which are cross-linking scaffolds required for antibodies to exhibit agonistic activity towards CD137-expressing cells; and thus the agonistic activity was further enhanced.

[Table 41]

| Name of evaluated heavy-chain constant region into which amino acid alterations that increase pI have been introduced | Name of corresponding heavy-chain constant region before introduction of amino acid alterations that increase pI | Introduced pI alterations |
|---|---|---|
| A375-SCF028/B167-LamLib | A375-TT16/B167-LamLib | P343R/D413K |
| A375-SCF033/B167-LamLib | A375-TT16/B167-LamLib | Q311R/P343R |
| A375-SCF025/B167-LamLib | A375-MY518/B167-LamLib | P343R/D413K |
| A375-SCF030/B167-LamLib | A375-MY518/B 167-LamLib | Q311R/P343R |
| A375-SCF027/B167-LamLib | A375-TT14/B167-LamLib | P343R/D413K |
| A375-SCF032/B167-LamLib | A375-TT14/B167-LamLib | Q311R/P343R |

[Table 42]

| Name of antibody judged to exhibit enhanced agonistic activity |
|---|
| A375-SCF028/B167-LamLib |
| A375-SCF033/B167-LamLib |
| A375-SCF025/B167-LamLib |
| A375-SCF030/B167-LamLib |
| A375-SCF027/B167-LamLib |
| A375-SCF032/B167-LamLib |

(5-5-6) Evaluation of the effect on agonist activity by introducing modifications that increase the pI of the heavy-chain constant region

The effect on CD137 agonist activity of introducing various amino acid modifications that increase the pI of the heavy-chain constant regions MY201aPh and MY518 was assessed by *in vitro* evaluation using human peripheral blood monocytes described in Reference Examples 5-5-1 and 5-5-2. The heavy-chain constant regions MY518, MY518a and MY201aPh were introduced with amino acid modifications that increase pI; and MY518+P343R/D413K (SCF025), MY518+Q311R/P343R(SCF030), MY518+P343R(SCF039), MY518+D413K (SCF040), MY518a+Q311R (SCF060a), MY201aPh+P343R(SCF041aPh), MY201aPh+P343R/D413K (SCF043aPh), MY201aPh+Q311R (SCF056aPh), MY201aPh+Q311R/P343Rb(SCF057aPh), and MY201aPh+Q311R/D413K (SCF059aPh) were thereby generated. The names of the evaluated antibodies into which the amino acid alterations that increase pI have been introduced, and the names of the antibodies before the amino acid alterations that increase the corresponding pI were introduced are shown in Table 43. Production of the antibodies recited in Table 43 is described in Reference Example 7-2.

Regarding assessment of the enhancement of agonist activity by introducing pI-increasing amino acid modifications, when addition of an antibody increased the amount of IL-2 by 1.04 fold or more and the amount of IFN-γ by 1.1 fold or more in the culture supernatant relative to the group added with antibodies comprising a heavy chain constant region that does not contain these amino acid modifications, A375-MY518a/B167-LamLib and A375-MY201aPh/B167-LamLib, it was judged that the CD137 agonist activity was enhanced. Compared to antibodies comprising a heavy-chain constant region without the pI-increasing amino acid modifications, antibodies comprising a heavy-chain constant region into which the amino acid alterations shown to have enhanced CD137 agonist activity in the presence of 250 μM ATP are shown in Table 44 (Figures 37 and 38).

[Table 43]

| Name of evaluated heavy-chain constant region into which amino acid alterations that increase pI have been introduced | Name of corresponding heavy-chain constant region before introduction of amino acid alterations that increase pI | Introduced pI alterations |
|---|---|---|
| A375-SCF025/B167-LamLib | A375-MY518/B 167-LamLib | P343R/D413K |
| A375-SCF0301B167-LamLib | A375-MY518/B167-LamLib | Q311R/P343R |
| A375-SCF039/B167-LamLib | A375-MY518/B 167-LamLib | P343R |
| A375-SCF040/B167-LamLib | A375-MY5181B167-LamLib | D413K |
| A375-SCF060a/B167-LamLib | A375-MY518a/B167-LamLib | Q311R |
| A375-SCF041aPh/B167-LamLib | A375-MY201aPh/B167-LamLib | P343R |
| A375-SCF043aPh/B167-LamLib | A375-MY201aPh/B167-LamLib | P343R/D413K |
| A375-SCF056aPh/B167-LamLib | A375-MY201aPh/B167-LamLib | Q311R |
| A375-SCF057aPh/B167-LamLib | A375-MY201aPh/B167-LamLib | Q311R/P343R |
| A375-SCF059aPh/B167-LamLib | A375-MY201aPh/B167-LamLib | Q311R/D413K |

[Table 44]

| Name of antibody judged to exhibit enhanced agonistic activity |
|---|
| A375-SCF025/B167-LamLib |
| A375-SCF030/B167-LamLib |
| A375-SCF039/B167-LamLib |
| A375-SCF040/B167-LamLib |
| A375-SCF060a/B167-LamLib |
| A375-SCF041aPh/B167-LamLib |
| A375-SCF043aPh/B167-LamLib |
| A375-SCF056aPh/B 167-LamLib |
| A375-SCF057aPh/B 167-LamLib |
| A375-SCF059aPh/B 167-LamLib |

(5-5-7) Assessment of ATP-dependent CD137 agonist activity of anti-human CD137 antibodies which combine the generated variable regions and constant regions

The ATP-dependent CD137 agonist activity of anti-human CD137 antibodies made by combining the above-described variable regions with the pI-increasing heavy chain constant regions was assessed by *in vitro* evaluation using human peripheral blood monocytes described in Reference Examples 5-5-1 and 5-5-2. The evaluated antibodies are shown in Table 45.

Compared to the amounts of IL-2 and IFN-$\gamma$ in the culture supernatant when a negative control antibody (IC17HdK-MY518a/IC17L-k0, IC17HdK-MY201aPh/IC17L-k0, IC17HdK-MY201/IC17L-k0, IC17HdK-G4d/IC17L-k0, IC17HdK-MY518/IC17L-k0, or IC17HdK-TT16/IC17L-k0) was added in the presence of 250 $\mu$M ATP, when the addition of an antibody increased the amount of IL-2 in the culture supernatant by 1.05 fold or more and the amount of IFN-$\gamma$ by 1.15 fold or more, it was judged that the antibody demonstrates CD 137 agonist activity. Measurement of the agonist activity using human peripheral blood mononuclear cells (PBMC) may differ from donor to donor of the blood sample. In view of this point, it was determined that antibodies that did not show CD137 agonist activity for some or more than half of the human PBMC isolated from multiple donors can be judged not to show CD137 agonist activity, even if they met the standards for agonist activity for the other portion of human PBMC. The antibodies judged to exhibit CD137 agonist activity in the presence of 250 $\mu$M ATP are shown in Table 45 (Figure 37, Figure 38, Figure 39, Figure 40, Figure 41, Figure 42, Figure 43, and Figure 44).

In addition, among the antibodies judged to show CD137 agonist activity in the presence of 250 μM ATP, antibodies whose fold change relative to the group added with a negative control antibody (IC17HdK-MY518a/IC17L-k0, IC17HdK-MY201aPh/IC17L-k0, IC17HdK-MY201/IC17L-k0, IC17HdK-G4d/IC17L-k0, IC17HdK-MY518/IC17L-k0, or IC17HdK-TT16/IC17L-k0) with respect to both the amounts of IL-2 and IFN-γ in the culture supernatant under the ATP-free condition was smaller than the fold change relative to the negative control under conditions in the presence of 250 μM ATP, were judged to have a lower CD137 agonist activity under the ATP-free condition. The antibodies judged to have a lower CD137 agonist activity in the absence of ATP are shown in Table 45 (Figure 37, Figure 38, Figure 39, Figure 40, Figure 41, Figure 42, Figure 43, and Figure 44). These antibodies were shown to exhibit ATP-dependent CD137 agonist activity.

[Table 45]

| Name of antibody evaluated | Name of antibody judged to have activity in the presence of ATP | Name of antibody judged to have lower activity in the absence of ATP |
|---|---|---|
| A375-MY518/B167-LamLib | A375-SCF025/B167-LamLib | A375-SCF025/B 1 67-Lam Lib |
| A375-SCF025/B 1 67-Lam Lib | A375-SCF030/B 167-Lam Lib | A375-SCF030/B167-LamLib |
| A375-SCF030/B167-Lamib | A375-SCF039/B167-LamLib | A375-SCF039/B167-LamLib |
| A375-SCF039/B167-LamLib | A375-SCF040/B 167-Lam Lib | A375-SCF040/B167-LamLib |
| A375-SCF040/B167-LamLib | A375-MY518a/B167-LamLib | A375-MY518a/B167-LamLib |
| A375-MY518a/B167-LamLib | A375-SCF060a/B167-LamLib | A375-SCF060a/B167-LamLib |
| A375-SCF060a/B167-LamLib | A375-SCF041aPh/B167-LamLib | A375-SCF041aPh/B167-LamLib |
| A375-MY201aPh/B167-LamLib | A375-SCF043aPh/B167-LamLib | A375-SCF043aPh/B167-LamLib |
| A375-SCF041aPh/B167-LamLib | A375-SCF056aPh/B167-LamLib | A375-SCF056aPh/B167-LamLib |
| A375-SCF043aPh/B167-LamLib | A375-SCF057aPh/B167-LamLib | A375-SCF057aPh/B167-LamLib |
| A375-SCF056aPh/B167-LamLib | A375-SCF059aPh/B167-LamLib | A551-SCF041a/B256-LamLib |
| A375-SCF057aPh/B167-LamLib | A551-SCF041a/B256-LamLib | A551-SCF057a/B256-LamLib |

(continued)

| Name of antibody evaluated | Name of antibody judged to have activity in the presence of ATP | Name of antibody judged to have lower activity in the absence of ATP |
|---|---|---|
| A375-SCF059aPh/B167-LamLib | A551-SCF057a/B256-LamLib | A551-SCF059a/B256-LamLib |
| A551-SCF041a/B256-LamLib | A551-SCF059a/B256-LamLib | A551-SCF057aPh/B256-LamLib |
| A551-SCF056a/B256-LamLib | A551-SCF057aPh/B256-LamLib | A551-SCF025a/B256-LamLib |
| A551-SCF057a/B256-LamLib | A551-SCF059aPh/B256-LamLib | A551-SCF039a/B256-LamLib |
| A551-SCF059a/B256-LamLib | A551-SCF025a/B256-LamLib | A551-SCF043a/B256-LamLib |
| A551-SCF056aPh/B256-LamLib | A551-SCF039a/B256-LamLib | A551-SCF041aPh/B256-LamLib |
| A551-SCF057aPh/B256-LamLib | A551-MY201a/B256-LamLib | A551-SCF043aPh/B256-LamLib |
| A551-SCF059aPh/B256-LamLib | A551-SCF043a/B256-LamLib | A551-SCF039a/B379-LamLib |
| A551-MY518/B256-LamLib | A551-SCF041aPh/B256-LamLib | A551-SCF041a/B379-LamLib |
| A551-SCF025a/B256-LamLib | A551-SCF043aPh/B256-LamLib | A551-SCF043a/B379-LamLib |
| A551-SCF039a/B256-LamLib | A551-SCF039a/B379-LamLib | A551-SCF041aPh/B379-LamLib |
| A551-MY201a/B256-LamLib | A551-SCF041a/B379-LamLib | A551-SCF043aPh/B379-LamLib |
| A551-SCF043a/B256-LamLib | A551-SCF043a/B379-LamLib | A551-SCF060a/B379-LamLib |
| A551-MY201aPh/B256-LamLib | A551-SCF041aPh/B379-LamLib | A551-SCF057a/B379-LamLib |
| A551-SCF041aPh/B256-LamLib | A551-SCF043aPh/B379-LamLib | A551-SCF059a/B379-LamLib |

(continued)

| Name of antibody evaluated | Name of antibody judged to have activity in the presence of ATP | Name of antibody judged to have lower activity in the absence of ATP |
|---|---|---|
| A551-SCF043aPh/B256-LamLib | A551-SCF060a/B379-LamLib | A551-SCF056aPh/B379-LamLib |
| A551-MY518a/B379-LamLib | A551-SCF057a/B379-LamLib | A551-SCF057aPh/B379-LamLib |
| A551-SCF039a/B379-LamLib | A551-SCF059a/B379-LamLib | A551-SCF059aPh/B379-LamLib |
| A551-SCF041a/B379-LamLib | A551-SCF056aPh/B379-LamLib | A375-TT16/B167-LamLib |
| A551-SCF043a/B379-LamLib | A551-SCF057aPh/B379-LamLib | A356-TT16/B040-LamLib |
| A551-SCF041aPh/B379-LamLib | A551-SCF059aPh/B379-LamLib | A551-TT16/B379-LamLib |
| A551-SCF043aPh/B379-LamLib | A375-TT16/B167-LamLib | |
| A551-SCF060a/B379-LamLib | A356- TT16/B040-Lam Lib | |
| A551-SCF056a/B379-LamLib | A551-TT16/B379-LamLib | |
| A551-SCF057a/B379-LamLib | | |
| A551-SCF059a/B379-LamLib | | |
| A551-SCF056aPh/B379-LamLib | | |
| A551-SCF057aPh/B379-LamLib | | |
| A551-SCF059aPh/B379-LamLib | | |
| A375-TT16/B167-LamLib | | |
| A356-TT 16/B040-Lam Lib | | |

(continued)

| Name of antibody evaluated | Name of antibody judged to have activity in the presence of ATP | Name of antibody judged to have lower activity in the absence of ATP |
|---|---|---|
| A551-TT16/B379-LamLib | | |

Reference Example 6: Human CD137 knock-in mouse administration test of anti-human CD137 switch antibodies

(6-1) Generation of antibodies for human CD137 knock-in mouse administration study

Anti-human CD137 switch and non-switch antibodies having a mouse constant region were generated for the human CD137 knock-in mouse administration study. Specifically, anti-human CD137 non-switch antibodies (20H4.9-mIgG1/20H4.9LC-mk0 abbreviation: NS1-mIgG1, 20H4.9-MB110/20H4.9LC-mk0 abbreviation: NS1-MB110, 20H4.9-MB492/20H4.9LC-mk0 abbreviation: NS1-MB492, MOR-7480.1 H-MB110/MOR-7480.1 L-ml0r abbreviation: NS2-MB110, MOR-7480.1 H-MB492/MOR-7480.1 L-ml0r abbreviation: NS2-MB492), and anti-human CD137 switch antibodies (A375-mIgG1/B167-ml0r, A372-mIgG1/B040-ml0r, A372-MB110/B040-ml0r, A372-MB492/B040-ml0r, A356-MB110/B040-ml0r, A486-MB492/B167-ml0r, A488-MB492/B226-ml0r, A489-MB492/B223-ml0r, A551-mIgG1/B256-ml0r, A551-MB110/B379-ml0r, and A548-mIgG1/B256-ml0r) were produced.
For the heavy chains of the NS1-mIgG1, NS1-MB110 and NS1-MB492 antibodies, genes of the antibody heavy chains were generated by combining the heavy-chain variable region 20H4.9 (SEQ ID NO: 139) with any of:

    (i) mIgG1, heavy-chain constant region of murine IgG1 (SEQ ID NO: 144),
    (ii) MB110 as described in WO2014030750 (SEQ ID NO: 145), and
    (iii) MB492 as described in WO2014030750 (SEQ ID NO: 146),

as the heavy-chain constant region. That is, the genes of 20H4.9-mIgG1, 20H4.9-MB110, and 20H4.9-MB492 were generated.
For the light chain of the NS1-mIgG1, NS1-MB110, and NS1-MB492 antibodies, the antibody light chain gene 20H4.9LC-mk0 was generated by combining the light-chain variable region 20H4.9LC (SEQ ID NO: 140) with the murine κ chain mk0 (SEQ ID NO: 147) as the light-chain constant region. By combining these heavy-chain and light-chain genes, each antibody was expressed and purified by methods known to those skilled in the art.
For the heavy chains of the NS2-MB110 and NS2-MB492 antibodies, genes of the antibody heavy chains were generated by combining the heavy-chain variable region MOR-7480.1 H (SEQ ID NO: 142) with either:

    (i) MB110 (SEQ ID NO: 145), or
    (ii) MB492 (SEQ ID NO: 146),

as heavy-chain constant region. That is, the MOR-7480.1H-MB110 and MOR-7480.1H-MB492 genes were generated.
For the light chains of the NS2-MB110 and NS2-MB492 antibodies, the antibody light chain gene MOR-7480.1L-ml0r was generated by combining the light-chain variable region MOR-7480.1L (SEQ ID NO: 143) with the murine λ chain ml0r (SEQ ID NO: 148) as the light-chain constant region. By combining these heavy-chain and light-chain genes, each antibody was expressed and purified by methods known to those skilled in the art.
For anti-CD 137 switch antibodies, genes of the antibody heavy chains and antibody light chains in Tables 46 and 47 below were generated; and each antibody was expressed and purified by combining these genes by methods known to those skilled in the art.

Antibody heavy chains of anti-CD 137 switch antibodies having a murine constant region

[Table 46]

| Full-length heavy chain | A375-mIgG1 | Variable region | A375 (SEQ ID NO: 43) |
|---|---|---|---|
| | | Constant region | mIgG1 (SEQ ID NO: 144) |
| Full-length heavy chain | A372-mIgG1 | Variable region | A372 (SEQ ID NO: 44) |

(continued)

| | | Constant region | mIgG1 (SEQ ID NO: 144) |
|---|---|---|---|
| Full-length heavy chain | A372-MB110 | Variable region | A372 (SEQ ID NO: 44) |
| | | Constant region | MB110 (SEQ ID NO: 145) |
| Full-length heavy chain | A372-MB492 | Variable region | A372 (SEQ ID NO: 44) |
| | | Constant region | MB492 (SEQ ID NO: 146) |
| Full-length heavy chain | A356-MB110 | Variable region | A356 (SEQ ID NO: 45) |
| | | Constant region | MB110 (SEQ ID NO: 145) |
| Full-length heavy chain | A486-MB492 | Variable region | A486 (SEQ ID NO: 46) |
| | | Constant region | MB492 (SEQ ID NO: 146) |
| Full-length heavy chain | A488-MB492 | Variable region | A488 (SEQ ID NO: 48) |
| | | Constant region | MB492 (SEQ ID NO: 146) |
| Full-length heavy chain | A489-MB492 | Variable region | A489 (SEQ ID NO: 49) |
| | | Constant region | MB492 (SEQ ID NO: 146) |
| Full-length heavy chain | A548-mIgG1 | Variable region | A548 (SEQ ID NO: 50) |
| | | Constant region | mIgG1 (SEQ ID NO: 144) |
| Full-length heavy chain | A551-mIgG1 | Variable region | A551 (SEQ ID NO: 51) |
| | | Constant region | mIgG1 (SEQ ID NO: 144) |
| Full-length heavy chain | A551-MB110 | Variable region | A551 (SEQ ID NO: 51) |
| | | Constant region | MB110 (SEQ ID NO: 145) |

Antibody light chains of anti-CD 137 switch antibodies having a murine constant region

[Table 47]

| Full-length light chain | B040-ml0r | Variable region | B040 (SEQ ID NO: 55) |
|---|---|---|---|
| | | Constant region | ml0r (SEQ ID NO: 148) |
| Full-length light chain | B167-ml0r | Variable region | B167 (SEQ ID NO: 54) |
| | | Constant region | ml0r (SEQ ID NO: 148) |
| Full-length light chain | B226-ml0r | Variable region | B226 (SEQ ID NO: 56) |
| | | Constant region | ml0r (SEQ ID NO: 148) |
| Full-length light chain | B223-ml0r | Variable region | B223 (SEQ ID NO: 57) |
| | | Constant region | ml0r (SEQ ID NO: 148) |
| Full-length light chain | B256-ml0r | Variable region | B256 (SEQ ID NO: 59) |
| | | Constant region | ml0r (SEQ ID NO: 148) |
| Full-length light chain | B379-ml0r | Variable region | B379 (SEQ ID NO: 60) |
| | | Constant region | ml0r (SEQ ID NO: 148) |

(6-2) Assessment of the human CD137-binding activity of antibodies prepared for the human CD137 knock-in mouse administration study

The binding activity of the anti-human CD137 non-switch antibody and the anti-CD137 switch antibody produced in Reference Example 6-1 towards human CD137 was evaluated. Measurements of human CD137 binding were taken at 37 °C using 20 mM ACES (pH 7.4), 150 mM NaCl, 2 mM $MgCl_2$, and 0.05% Tween20 as running buffer. At first, antibodies were captured by interacting antibody solutions prepared in running buffer with the rabbit anti-mouse IgG (Thermo Scientific)-immobilized Series S Sensor Chip CM5 (GE Healthcare). The human CD137-binding activity in the presence of ATP and in the absence of ATP was then assessed by interacting with a human CD137 solution prepared in running buffer supplemented with a desired concentration of ATP, or a human CD137 solution prepared in running buffer without ATP. For the human CD137 antigen, hCD137 (FXa digested) prepared in Reference Example (1-2) was used, and measurements were performed at an antigen concentration of 0, 15.625, 62.5, 250, and 1000 nM. The chip was regenerated using 25 mM NaOH and 10 mM Glycine-HCl (pH 1.5), and measurements were performed by repeatedly capturing antibodies. Dissociation constant (KD) of the respective antibodies for human CD137 was calculated using Biacore T200 Evaluation Software 2.0. Specifically, the association rate constant ka (L/mol/s) and the dissociation rate constant kd (1/s) were calculated by global fitting of the sensorgrams obtained by measurement with the 1:1 Langmuir binding model, and the dissociation constant KD (mol/L) was calculated from these values.

Table 48 shows the results of these measurements.

Analysis of human CD137 binding of anti-CD 137 antibodies having a murine constant region

[Table 48]

| Antibody name | $K_D$ (M) for human CD137 | | | |
|---|---|---|---|---|
| | Without ATP | ATP=1 $\mu$M | ATP=10 $\mu$M | ATP=100 $\mu$M |
| NS1-mIgG1 | 6.17E-08 | 5.62E-08 | 5.44E-08 | 5.36E-08 |
| NS1-MB110 | 6.10E-08 | 5.65E-08 | 5.39E-08 | 5.19E-08 |
| NS1-MB492 | 6.16E-08 | 5.49E-08 | 5.35E-08 | 5.04E-08 |
| NS2-MB110 | 1.88E-07 | 1.44E-07 | 1.02E-07 | 1.04E-07 |
| NS2-MB492 | 2.16E-07 | 1.70E-07 | 1.12E-07 | 1.12E-07 |
| A375-mIgG1/ B167-ml0r | N.A. | 3.43E-07 | 1.14E-07 | 2.92E-08 |
| A372-mIgG1/ B040-ml0r | N.A. | 4.78E-07 | 2.59E-07 | 1.08E-07 |
| A372-MB110/ B040-ml0r | N.A. | 6.11E-07 | 2.64E-07 | 1.12E-07 |
| A372-MB492/ B040-ml0r | N.A. | 7.82E-07 | 2.51E-07 | 1.04E-07 |
| A356-MB110/ B040-ml0r | N.A. | 1.01E-06 | 4.10E-07 | 1.68E-07 |
| A486-MB492/ B167-ml0r | N.A. | 1.31E-07 | 2.70E-08 | 7.67E-09 |
| A488-MB492/ B226-ml0r | N.A. | N.D. | 1.36E-07 | 8.25E-08 |
| A489-MB492/ B223-ml0r | N.A. | 1.62E-06 | 3.58E-07 | 1.54E-07 |
| A551-mIgG1/ B256-ml0r | N.A. | 7.55E-07 | 2.31E-07 | 6.22E-08 |
| A551-MB110/ B379-ml0r | N.A. | 5.12E-07 | 1.43E-07 | 3.51E-08 |
| A548-mIgG1/ B256-ml0r | N.A. | 8.61E-07 | 2.19E-07 | 5.58E-08 |
| N.A. : It is too weak to determine a KD value. N.D. : Not determined. | | | | |

Both the anti-human CD137 non-switch antibody and the anti-human CD137 switch antibody generated above, which comprise a murine constant region, were confirmed to bind to human CD137. All anti-human CD137 switch antibodies were also shown to bind to human CD137 in an ATP concentration-dependent manner. On the other hand, for the non-switch antibodies, no such ATP concentration-dependent binding to human CD137 was observed, and the binding was almost the same at any ATP concentration.

(6-3) Plasma kinetic assessment of modified antibodies in human CD137 knock-in mouse

(6-3-1) Generation of human CD137 knock-in mouse

By introducing a human CD137 gene substitution vector into mouse embryonic stem cells (ES cells), a human CD137 knock-in mouse was generated in which the mouse CD137 gene was replaced by a human CD137 gene. This mouse is described as an hCD137 KI mouse.

(6-3-2) Measurement of anti-human CD137 antibody concentration in plasma in the hCD137KI mouse model

Following generation of the hCD137KI mouse in Reference Example 6-3-1, the respective antibodies were administered intravenously at a single dose to the hCD137KI mouse as shown in Table 49. In Table 49, NS1-mIgG1, NS1-MB110 and NS1-MB492 are all non-switch antibodies, and the other is a switch antibody. Blood was collected multiple times over time from 5 minutes to 28 days after administration. The obtained blood was centrifuged to separate plasma. Plasma was stored in a freezer set below -20°C until measurement.

Group details for the plasma kinetic evaluation study

[Table 49]

| Group | Number of animals | Pharmaceutical agent | Dose [mg/kg] |
|---|---|---|---|
| 1 | 3 | NS1-mIgG1 | 7.5 single administration |
| 2 | 3 | NS1-MB110 | 7.5 single administration |
| 3 | 3 | NS1-MB492 | 7.5 single administration |
| 4 | 3 | A375-mIgG1/B167-ml0r | 7.5 single administration |
| 5 | 3 | A356-MB110/B040-ml0r | 7.5 single administration |
| 6 | 3 | A372-mIgG1/B040-ml0r | 7.5 single administration |
| 7 | 3 | A372-MB110/B040-ml0r | 7.5 single administration |
| 8 | 3 | A372-MB492/B040-ml0r | 7.5 single administration |
| 9 | 3 | A486-MB492/B167-ml0r | 7.5 single administration |
| 10 | 3 | A488-MB492/B226-ml0r | 7.5 single administration |
| 11 | 3 | A489-MB492/B223-ml0r | 7.5 single administration |
| 12 | 3 | A548-mIgG1/B256-ml0r | 7.5 single administration |
| 13 | 3 | A551-mIgG1/B256-ml0r | 7.5 single administration |
| 14 | 3 | A551-MB110/B379-ml0r | 7.5 single administration |

The concentration of each switch antibody in plasma was measured by the electrochemiluminescence (ECL) method. Specifically, hCD137 (Sino Biological Inc.) was diluted in PBS (-) and added to MULTI-ARRAY 96-well Plate (Meso Scale Diagnostics, LLC). hCD137 was added to the plate and shaken at room temperature for one hour, and hCD137 was immobilized to the plate. Next, for blocking, a PBS solution containing 1% BSA and 0.05% Tween 20 was added, and shaken at room temperature for one hour. Calibration curves for individual switch antibodies were prepared at plasma concentrations of 64, 32, 16, 8, 4, 2, and 1 ng/mL. After addition of a PBS solution containing 3 mM ADP, 1% BSA, and 0.05% Tween 20 to the hCD137-immobilized plate, two-fold volume of plasma samples diluted in a PBS solution containing 1% BSA and 0.05% Tween20, as well as calibration curve samples were added thereto. After the plate was shaken at room temperature for one hour, a biotinylated anti-mouse IgG antibody (Jackson ImmunoResearch Laboratories, Inc.) was added. In addition, after the plate was shaken at room temperature for one hour, SULFO-TAG Labeled Streptavidin (Meso Scale Diagnostics, LLCs) was added. In addition, after the plate was shaken at room temperature for one hour, Read buffer T (Meso Scale Diagnostics, LLCs) diluted two-fold with a PBS solution containing 2 mM ADP, 1% BSA, and 0.05% Tween 20 was added. Measurement of antibody concentrations in the hCD137KI mouse plasma was performed by detection using SULFO-TAG on SECTOR Imager (Meso Scale Diagnostics, LLC). Calculation of individual antibody concentrations in the mouse plasma was performed using SOFTmax PRO (Molecular Devices).

The concentration of non-switch antibodies in plasma was measured by the electrochemiluminescence (ECL) method. Specifically, hCD137 (Sino Biological Inc.) was diluted in PBS(-) and added to a MULTI-ARRAY 96-well Plate (Meso Scale Diagnostics, LLC). hCD137 was added to the plate and shaken at room temperature for one hour, and hCD137 was immobilized to the plate. Next, for blocking, a PBS solution containing 1% BSA and 0.05% Tween 20 was added, and shaken at room temperature for one hour. Calibration curves for individual non-switch antibodies were prepared at plasma concentrations of 32, 16, 8, 4, 2, 1, and 0.5 ng/mL. Plasma samples diluted in a PBS solution containing 1% BSA, 0.05% Tween20 as well as calibration curve samples were added to the hCD137 immobilized plate. After the plate was shaken at room temperature for one hour, a biotinylated anti-mouse IgG antibody (Jackson ImmunoResearch Laboratories, Inc.) was added. In addition, after the plate was shaken at room temperature for one hour, SULFO-TAG Labeled Streptavidin (Meso Scale Diagnostics, LLCs) was added. In addition, after the plate was shaken at room temperature for one hour, Read buffer T (Meso Scale Diagnostics, LLCs) diluted two-fold was added. Measurement of antibody concentrations in the hCD137KI mouse plasma was performed similarly to that for the switch antibody described above.

The results are shown in Figures 45, 46, and 47.

Figure 45 shows a time course of the mean plasma concentrations in the hCD137 KI mouse of antibodies having mIgG1 as Fc (NS1-mIgG1 as a non-switch antibody, and A375-mIgG1/B167-ml0r, A372-mIgG1/B040-ml0r, A548-mIgG1/B256-ml0r or A551-mIgG1/B256-ml0r as a switch antibody) among the antibodies shown in Table 49. Figure 46 shows a time course of the mean plasma concentrations in the hCD137 KI mouse of antibodies having MB110 as Fc (NS1-MB110 as a non-switch antibody, and A356-MB110/B040-ml0r, A372-MB110/B040-ml0r, and A551-MB110/B379-ml0r as switch antibodies). Figure 47 shows a time course of the mean plasma concentrations in the CD137 KI mouse of antibodies having MB492 as Fc (NS1-MB492 as a non-switch antibody, and A372-MB492/B040-ml0r, A486-MB492/B167-ml0r, A488-MB492/B226-ml0r, or A489-MB492/B223-ml0r as a switch antibody). In Figure 47, as for A488-MB492/B226-ml0r, a sharp decline in the plasma-concentration time course suspected to be caused by ADA was confirmed in one subject, and the average value was taken from two subjects.

In the hCD137 KI mouse, with any Fc, the switch antibody showed a slower elimination from plasma than the non-switch antibody. This is thought to be because compared to the switch antibody, the non-switch antibody bound to CD137 expressed in the body and eliminated from the plasma faster. Further, it was suggested that the extracellular ATP concentration in a normal tissue was sufficiently low to the extent that the switch antibodies used in this study did not bind to human CD137. These results suggest that the binding to an antigen expressed systemically can be reduced by using the switch antibodies, and thus an antibody with enhanced blood kinetics can be prepared.

(6-4) Movement of drug efficacy and systemic response markers of anti-CD137 antibodies by a syngeneic tumor-cell transplantation model using the hCD137KI mouse

(6-4-1) Generation of cell lines and a syngeneic tumor transplantation mouse model, as well as method of evaluating anti-tumor effect and systemic response

Cells of the murine colorectal cancer cell line MC38 licensed from the National Cancer Institute and the hCD137 KI mouse described in Reference Example 6-3-1 were used for various studies. The model was formed when the tumor volume reached approximately 50-300 mm$^3$ by implanting the MC38 cell line subcutaneously in the abdomen of mice. After establishment of the model, mice transplanted with MC38 cell line were grouped and then various anti-CD137 antibodies were administered.

For evaluation of the anti-tumor effect, measurement of the tumor volume was performed at a frequency of 1-2 times per week. The tumor volume was calculated according to the formula below:

$$\text{Tumor volume (mm}^3\text{)} = \text{the length (mm) x the width (mm) x the width (mm)/2}$$

By removing the liver and spleens or lymph nodes at the appropriate time after antibody administration, systemic responses were assessed by organ weight, cell count of lymphocyte fractions, or T cell analysis using flow cytometry (FCM). In the assessment of these indicators, the subject switch antibody was assessed to have suppressed systemic responses and/or T cell activation in non-tumor tissues (e.g., lymph nodes, spleens, and liver) when the indicator of systemic response was low compared to the same amount of non-switch antibody (control anti-human CD137 antibody with no ATP-dependent human CD137 binding activity).

The antibodies produced in Reference Example 6-1 (production of antibodies for the human CD137 knock-in mouse administration test) were used in various tests.

(6-4-2) Collection of various organs from the MC38 cell-line transplanted mouse and preparation of lymphocyte fractions

Weight measurement of the collected spleens and lymph nodes, and cell counting of the lymphocyte fractions were performed. Lymphocyte fractions obtained using the Liver dissociation kit, mouse (Milteny Biotec) were also used for assessment of liver activation. The lymphocyte fractions after red blood cell lysis were used for evaluation of activation in the spleens, and the lymphocyte fractions obtained by grinding were used for evaluation of activation in the lymph nodes.

(6-4-3) FCM analysis using lymphocyte fractions from various organs

For assessment of activation markers by FCM, the Granzyme B or PD-1 expression or ICOS expression on CD8$\alpha$-positive T cells or the percentage of CD8$\alpha$-positive T cells in CD45 positive cells were used. For this reason, a fluorescently labeled anti-Granzyme B antibody, an anti-PD-1 antibody, an anti-ICOS antibody, an anti-CD8$\alpha$ antibody and an anti-CD45 antibody were used in FCM analysis. Measurements were made with BD LSRFortessa™ X-20 (BD Biosciences).

(6-4-4) Drug efficacy (anti-tumor effect) studies on the A375-mIgG1/B167-ml0r antibody

Following establishment of the MC38 transplantation mouse model in Reference Example 6-4-1, Vehicle and A375-mIgG1/B167-ml0r were administered at the doses shown in Table 50. Antibody administration was performed twice via the tail vein route on the day of grouping and four days after grouping. For Vehicle, PBS containing 0.05% Tween 20 was used.

Group details for the A375-mIgG1/B167-ml0r efficacy study

[Table 50]

| Group | Number of animals | Pharmaceutical agent | Dose [mg/kg] | Day of administration |
|---|---|---|---|---|
| 1 | 5 | Vehicle | - | On the day of grouping and four days after grouping |
| 2 | 5 | A375-mIgG1/ B167-ml0r | 22.5 | On the day of grouping and four days after grouping |
| 3 | 5 | A375-mIgG1/ B167-ml0r | 7.5 | On the day of grouping and four days after grouping |
| 4 | 5 | A375-mIgG1/ B167-ml0r | 2.5 | On the day of grouping and four days after grouping |
| 5 | 5 | A375-mIgG1/ B167-ml0r | 0.83 | On the day of grouping and four days after grouping |
| 6 | 5 | A375-mIgG1/ B167-ml0r | 0.28 | On the day of grouping and four days after grouping |

The anti-tumor effect in each group was assessed by the tumor volume calculated as described in Reference Example 6-4-1. As a result, the dose-dependent anti-tumor effect of the A375-mIgG1/B167-ml0r antibody was confirmed (Figure 48).

(6-4-5) Antibody administration and sampling as well as FCM analysis for the assessment of systemic effects of A375-mIgG1/B167-ml0r

Following establishment of the MC38 transplantation mouse model in Reference Example 6-4-1, Vehicle, NS1-mIgG1 (non-switch antibody) and A375-mIgG1/B167-ml0r were administered as shown in Table 51. Antibody administration was performed twice via the tail vein route on the day of grouping and three days after grouping. For Vehicle, PBS containing 0.05% Tween 20 was used.

Five days after the initial administration, the liver, lymph nodes, and spleens were collected as shown in Reference Example 6-4-2 (collection of various organs from the MC38 cell line-transplanted mouse and preparation of lymphocyte fractions). Further, for the spleens and lymph nodes, the organs from three mice were pooled, and FCM analysis was performed as shown in Reference Example 6-4-3 (FCM analysis using lymphocyte fractions from various organs).

Group details for assessment of systemic responses with the A375-mIgG1/B167-ml0r antibody

[Table 51]

| Group | Number of animals | Pharmaceutical agent | Dose [mg/kg] | Day of administration | Day of sampling |
|---|---|---|---|---|---|
| 1 | 3 | Vehicle | - | On the day of grouping and three days after grouping | Five days after grouping |
| 2 | 3 | NS1-mIgG1 | 0.06 | On the day of grouping and three days after grouping | Five days after grouping |
| 3 | 3 | NS1-mIgG1 | 0.3 | On the day of grouping and three days after grouping | Five days after grouping |
| 4 | 3 | NS1-mIgG1 | 1.5 | On the day of grouping and three days after grouping | Five days after grouping |
| 5 | 3 | NS1-mIgG1 | 7.5 | On the day of grouping and three days after grouping | Five days after grouping |
| 6 | 3 | NS1-mIgG1 | 37.5 | On the day of grouping and three days after grouping | Five days after grouping |
| 7 | 3 | A375-mIgG1/ B167-ml0r | 1.5 | On the day of grouping and three days after grouping | Five days after grouping |
| 8 | 3 | A375-mIgG1/ B167-ml0r | 7.5 | On the day of grouping and three days after grouping | Five days after grouping |
| 9 | 3 | A375-mIgG1/ B167-ml0r | 37.5 | On the day of grouping and three days after grouping | Five days after grouping |

The systemic effects by the administered antibodies were evaluated. As a result of evaluating the organ weight of the lymph nodes and spleens, an increase in the organ weight was observed when NS1-mIgG1 was administered at 0.3 mg/kg, and more intense organ hypertrophy was observed at 1.5 mg/kg and 7.5 mg/kg. On the other hand, organ enlargement was not observed at any dose of 1.5 mg/kg, 7.5 mg/kg, or 37.5 mg/kg when A375-mIgG1/B167-ml0r was administered (Figure 49).

In addition, as a result of assessing T cell activation by FCM evaluation, an increase in the expression of all the markers PD-1, ICOS and Granzyme B was observed at 0.3 mg/kg after NS1-mIgG1 administration. On the other hand, when A375-mIgG1/B167-ml0r was administered, there was a remarkable inhibition of expression of all the markers PD-1, ICOS and Granzyme B at the doses of 1.5 mg/kg, 7.5 mg/kg, and 37.5 mg/kg (Figure 50 and Figure 51). Since the A375-mIgG1/B167-ml0r results showed that organ weight was correlated with T cell activation markers in the lymph nodes and spleens, organ weight was used mainly as an indicator of lymph node- and spleen-derived immune cell activation in the subsequent evaluations of antibodies.

As a result of evaluating the activation of T cells in the liver, an increase in the expressions of PD-1 and Granzyme B was observed when NS1-mIgG1 was administered at 0.3 mg/kg. On the other hand, when A375-mIgG1/B167-ml0r was administered, the expressions were suppressed at all doses of 1.5 mg/kg, 7.5 mg/kg, and 37.5 mg/kg (Figure 52).

Thus, the switch antibody inhibited the activation of T cells in the lymph nodes, spleens, and liver compared with the same amount of non-switch antibody.

(6-4-6) Drug efficacy (anti-tumor effect) study on the A356-MB110/B040-ml0r antibody

Following establishment of the MC38 transplantation mouse model in Reference Example 6-4-1, Vehicle and A356-MB110/B040-ml0r were administered at the doses shown in Table 52. Antibody administration was performed twice via the tail vein route on the day of grouping and three days after grouping. For Vehicle, PBS containing 0.05% Tween 20 was used.

Group details for the A356-MB110/B040-ml0r efficacy study

[Table 52]

| Group | Number of animals | Pharmaceutical agent | Dose [mg/kg] | Day of administration |
|---|---|---|---|---|
| 1 | 5 | Vehicle | - | On the day of grouping and three days after grouping |
| 2 | 5 | A356-MB110/B040-ml0r | 7.5 | On the day of grouping and three days after grouping |
| 3 | 5 | A356-MB110/B040-ml0r | 2.5 | On the day of grouping and three days after grouping |

The anti-tumor effect in each group was assessed by tumor volume calculated as described in Reference Example 6-4-1. The results confirmed the dose-dependent anti-tumor effect of A356-MB110/B040-ml0r (Figure 53).

(6-4-7) Antibody administration and sampling as well as FCM-analysis for the assessment of systemic effects of A356-MB110/B040-ml0r

Following establishment of the MC38 transplantation mouse model in Reference Example 6-4-1, Vehicle, NS2-MB110 (non-switch antibody) and A356-MB110/B040-ml0r were administered as shown in Table 53. Antibody administration was performed twice via the tail vein route on the day of grouping and three days after grouping. For Vehicle, PBS containing 0.05% Tween 20 was used.

Seven days after the first administration, the liver, lymph nodes, and spleens were collected in the manner shown in Reference Example 6-4-2 (collection of various organs from mice transplanted with the MC38 cell line and preparation of lymphocyte fractions). For the spleens and lymph nodes, only organ weight measurement was performed.

Group details for the assessment of systemic responses with the A356-MB110/B040-ml0r antibody

[Table 53]

| Group | Number of animals | Pharmaceutical agent | Dose [mg/kg] | Day of administration | Day of sampling |
|---|---|---|---|---|---|
| 1 | 3 | Vehicle | - | On the day of grouping and three days after grouping | Seven days after grouping |
| 2 | 3 | NS2-MB110 | 7.5 | On the day of grouping and three days after grouping | Seven days after grouping |
| 3 | 3 | NS2-MB110 | 2.5 | On the day of grouping and three days after grouping | Seven days after grouping |
| 4 | 3 | NS2-MB110 | 0.3 | On the day of grouping and three days after grouping | Seven days after grouping |
| 5 | 3 | A356-MB110/ B040-ml0r | 7.5 | On the day of grouping and three days after grouping | Seven days after grouping |

(continued)

| Group | Number of animals | Pharmaceutical agent | Dose [mg/kg] | Day of administration | Day of sampling |
|---|---|---|---|---|---|
| 6 | 3 | A356-MB110/ B040-ml0r | 2.5 | On the day of grouping and three days after grouping | Seven days after grouping |

Systemic effects of the administered antibodies were evaluated. The result of evaluating the organ weight of the lymph nodes and spleens when NS2-MB110 and A356-MB110/B040-ml0r were administered showed that hypertrophy was observed also when NS2-MB110 was administered at 2.5 mg/kg. On the other hand, at the time of A356-MB110/B040-ml0r administration, lymphadenopathy was suppressed even at 7.5 mg/kg, and splenomegaly was suppressed at 2.5 mg/kg (Figure 54).

In addition, the result of evaluating T cell activation in the liver showed that an increase in the PD-1 expression in CD8$\alpha$-positive cells was also observed when NS2-MB110 was administered at 0.3 mg/kg. In the meantime, when A356-MB110/B040-ml0r was administered, the PD-1 expression was suppressed at 7.5 mg/kg, and remarkably suppressed at 2.5 mg/kg. A slight increase in the ICOS expression in CD8$\alpha$-positive cells was also observed upon administration of the NS2-MB110 antibody at 0.3 mg/kg. On the other hand, it was observed that the expression of Vehicle was suppressed at 2.5 mg/kg when A356-MB110/B040-ml0r was administered (Figure 55).

Thus, the switch antibody inhibited the activation of T cells in the lymph nodes, spleens, and liver compared with the non-switch antibody.

(6-4-8) Drug efficacy (anti-tumor effect) study on the A372-mIgG1/B040-ml0r antibody

Following establishment of the MC38 transplantation mouse model in Reference Example 6-4-1, Vehicle and A372-mIgG1/B040-ml0r were administered at the doses shown in Table 54. Antibody administration was performed four times via the tail vein route on the day of grouping, and 4 days, 7 days, and 10 days after grouping. For Vehicle, PBS containing 0.05% Tween 20 was used.

Group details for the A372-mIgGl/B040-ml0r efficacy study

[Table 54]

| Group | Number of animals | Pharmaceutical agent | Dose [mg/kg] | Day of administration |
|---|---|---|---|---|
| 1 | 5 | Vehicle | - | On the day of grouping, and four, seven, and ten days after grouping |
| 2 | 5 | A372-mIgG1/ B040-ml0r | 7.5 | On the day of grouping, and four, seven, and ten days after grouping |

The anti-tumor effect in each group was assessed by tumor volume calculated as described in Reference Example 6-4-1. As a result, an anti-tumor effect of the A372-mIgG1/B040-ml0r antibody was observed at 7.5 mg/kg administration (Figure 56).

(6-4-9) Antibody administration and sampling as well as FCM analysis for the assessment of systemic effects of A372-mIgG1/B040-ml0r

Following establishment of the MC38 transplantation mouse model in Reference Example 6-4-1, Vehicle and A372-mIgG1/B040-ml0r were administered as shown in Table 55. Antibody administration was performed three times via the tail vein route on the day of grouping, and 3 days and 6 days after grouping. For Vehicle, PBS containing 0.05% Tween 20 was used.

Seven days after the initial administration, the liver, lymph nodes, and spleens were collected in the manner shown in Reference Example 6-4-2 (collection of various organs from mice transplanted with the MC38 cell line and preparation of lymphocyte fractions). For the spleens, only measurement of the organ weight was conducted; and for the lymph nodes, only cell counting of the lymphocyte fractions obtained from lymphocytes pooled from three mice was done.

Group details for the assessment of systemic responses with the A372-mIgG1/B040-ml0r antibody

[Table 55]

| Group | Number of animals | Pharmaceutical agent | Dose [mg/kg] | Day of administration | Day of sampling |
|---|---|---|---|---|---|
| 1 | 3 | Vehicle | - | On the day of grouping, and three and six days after grouping | Seven days after grouping |
| 2 | 3 | A372-mIgG1/ B040-ml0r | 7.5 | On the day of grouping, and three and six days after grouping | Seven days after grouping |

Systemic effects of the administered antibodies were evaluated. As a result of measuring the number of cells in the lymph nodes and evaluating the organ weight in the spleens, it was confirmed that there was no increase in the number of cells in the lymph nodes and an increase in the weight of the spleens at the time of A372-mIgG1/B040-ml0r administration, and that the organ weight was equivalent to that in the Vehicle group (Figure 57).
As a result of evaluating T cell activation in the liver, the granzyme B expression levels were similar to those in the Vehicle group when A372-mIgG1B040-ml0r was administered (Figure 58).
Thus, it was confirmed that the switch antibody inhibited the activation of T cells in the lymph nodes, spleens, and liver.

(6-4-10) Drug efficacy (anti-tumor effect) study on the A372-MB110/B040-ml0r antibody

Following establishment of the MC38 transplantation mouse model in Reference Example 6-4-1, Vehicle and A372-MB110/B040-ml0r were administered at the doses shown in Table 56. Antibody administration was performed twice via the tail vein route on the day of grouping and four days after grouping. For Vehicle, PBS containing 0.05% Tween 20 was used.

Group details for the A372-MB110/B040-ml0r efficacy study

[Table 56]

| Group | Number of animals | Pharmaceutical agent | Dose [mg/kg] | Day of administration |
|---|---|---|---|---|
| 1 | 5 | Vehicle | - | On the day of grouping and three days after grouping |
| 2 | 5 | A372-MB110/ B040-ml0r | 7.5 | On the day of grouping and three days after grouping |

The anti-tumor effect in each group was assessed by tumor volume calculated as described in Reference Example 6-4-1. As a result, the anti-tumor effect of A372-MB110/B040-ml0r was confirmed (Figure 59).

(6-4-11) Antibody administration and sampling as well as FCM analysis for assessment of the systemic effects of A372-MB110/B040-ml0r

Following establishment of the MC38 transplantation mouse model in Reference Example 6-4-1, Vehicle, NS2-MB110 (non-switch antibody) and A372-MB110/B040-ml0r were administered as shown in Table 57. Antibody administration was performed twice via the tail vein route on the day of grouping and 3 days after grouping. For Vehicle, PBS containing 0.05% Tween 20 was used.

Seven days after the initial administration, the liver, lymph nodes, and spleens were collected as shown in Reference Example 6-4-2 (collection of various organs from mice transplanted with the MC38 cell line and preparation of lymphocyte fractions). For the spleens and lymph nodes, only organ weight measurement was performed.

Group details for the assessment of systemic responses with the A372-MB110/B040-ml0r antibody

[Table 57]

| Group | Number of animals | Pharmaceutical agent | Dose [mg/kg] | Day of administration | Day of sampling |
|---|---|---|---|---|---|
| 1 | 3 | Vehicle | - | On the day of grouping and three days after grouping | Seven days after grouping |
| 2 | 3 | NS2-MB110 | 7.5 | On the day of grouping and three days after grouping | Seven days after grouping |
| 3 | 3 | NS2-MB110 | 2.5 | On the day of grouping and three days after grouping | Seven days after grouping |
| 4 | 3 | NS2-MB110 | 0.3 | On the day of grouping and three days after grouping | Seven days after grouping |
| 5 | 3 | A372-MB110/ B040-ml0r | 7.5 | On the day of grouping and three days after grouping | Seven days after grouping |
| 6 | 3 | A372-MB110/ B040-ml0r | 2.5 | On the day of grouping and three days after grouping | Seven days after grouping |

Systemic effects of the administered antibodies were evaluated. As a result of evaluating the organ weight of the lymph nodes and spleens, increases in the organ weight were observed when NS2-MB110 was administered at 2.5 mg/kg and 7.5 mg/kg. On the other hand, inhibition of organomegaly was observed at both doses of 2.5 mg/kg and 7.5 mg/kg when A372-MB110/B040-ml0r was administered (Figure 60).

As a result of evaluating the activation of T cells in the liver, an increase in the expression of PD-1 was observed when NS2-MB110 was administered. This was also seen when A372-MB110/B040-ml0r was administered (Figure 61).

In conclusion, the switch antibody inhibited the activation of T cells in the lymph nodes and spleens compared with the non-switch antibody.

(6-4-12) Drug efficacy (anti-tumor effect) study on the A372-MB492/B040-ml0r antibody

Following establishment of the MC38 transplantation mouse model in Reference Example 6-4-1, Vehicle and A372-MB492/B040-ml0r were administered at the doses shown in Table 58. Antibody administration was performed twice via the tail vein route on the day of grouping and three days after grouping. For Vehicle, PBS containing 0.05% Tween 20 was used.

Group details for the A372-MB492/B040-ml0r efficacy study

[Table 58]

| Group | Number of animals | Pharmaceutical agent | Dose [mg/kg] | Day of administration |
|---|---|---|---|---|
| 1 | 5 | Vehicle | - | On the day of grouping and three days after grouping |
| 2 | 5 | A372-MB492/ B040-ml0r | 1.5 | On the day of grouping and three days after grouping |

(continued)

| Group | Number of animals | Pharmaceutical agent | Dose [mg/kg] | Day of administration |
|---|---|---|---|---|
| 3 | 5 | A372-MB492/ B040-ml0r | 3.0 | On the day of grouping and three days after grouping |
| 4 | 5 | A372-MB492/ B040-ml0r | 7.5 | On the day of grouping and three days after grouping |

The anti-tumor effect in each group was assessed by tumor volume calculated as described in Reference Example 6-4-1. As a result, an anti-tumor effect of the A372-MB492/B040-ml0r antibody was observed at all doses (Figure 62).

(6-4-13) Antibody administration and sampling as well as FCM analysis for assessment of the systemic effects of the A372-MB492/B040-ml0r antibody

Following establishment of the MC38 transplantation mouse model in Reference Example 6-4-1, Vehicle, NS1-MB492 (non-switch antibody) and A372-MB492/B040-ml0r were administered as shown in Table 59. Antibody administration was performed twice via the tail vein route on the day of grouping and 3 days after grouping. For Vehicle, PBS containing 0.05% Tween 20 was used.

Eight days after the initial administration, the liver, lymph nodes, and spleens were collected in the manner shown in Reference Example 6-4-2 (collection of various organs from mice transplanted with the MC38 cell line and preparation of lymphocyte fractions). For the spleens, only measurement of the organ weight was conducted; and for the lymph nodes, only cell counting of the prepared lymphocyte fractions was performed.

Group details for the assessment of systemic responses of the A372-MB492/B040-ml0r antibody

[Table 59]

| Group | Number of animals | Pharmaceutical agent | Dose [mg/kg] | Day of administration | Day of sampling |
|---|---|---|---|---|---|
| 1 | 3 | Vehicle | - | On the day of grouping and three days after grouping | Eight days after grouping |
| 2 | 3 | NS1-MB492 | 7.5 | On the day of grouping and three days after grouping | Eight days after grouping |
| 3 | 3 | A372-MB492/ B040-ml0r | 1.5 | On the day of grouping and three days after grouping | Eight days after grouping |
| 4 | 3 | A372-MB492/ B040-ml0r | 3.0 | On the day of grouping and three days after grouping | Eight days after grouping |
| 5 | 3 | A372-MB492/ B040-ml0r | 7.5 | On the day of grouping and three days after grouping | Eight days after grouping |

Systemic effects by the administered antibodies were evaluated. Increase in the organ weight from the organ weight of the spleens when NS1-MB492 was administered, and increase in the lymphocyte fractions from cell count measurement when NS1-MB492 was administered were observed. On the other hand, when A372-MB492/B040-ml0r was administered at the dose of 1.5 mg/kg, 3.0 mg/kg, and 7.5 mg/kg, inhibition of organomegaly was observed compared with NS1-MB492 (Figure 63).

As a result of evaluating the activation of T cells in the liver, an increase in the expression of Granzyme B was observed when NS1-MB492 and A372-MB492/B040-ml0r were administered compared with Vehicle (Figure 64).

In conclusion, the switch antibody inhibited the activation of T cells in the lymph nodes and spleens compared with the non-switch antibody.

(6-4-14) Drug efficacy (anti-tumor effect) study on the A486-MB492/B167-ml0r and A488-MB492/B226-ml0r antibodies

Following establishment of the MC38 transplantation mouse model in Reference Example 6-4-1, Vehicle, A486-MB492/B167-ml0r and A488-MB492/B226-ml0r were administered at the doses shown in Table 60. Grouping was performed 10 days after cell transplantation, and antibody administration was performed via the tail vein route 11, 15, 18, and 22 days after cell transplantation for a total of four times. For Vehicle, PBS containing 0.05% Tween 20 was used.

Group details for the drug efficacy study on the A486-MB492/B167-ml0r and A488-MB492/B226-ml0r antibodies

[Table 60]

| Group | Number of animals | Pharmaceutical agent | Dose [mg/kg] | Day of administration |
|---|---|---|---|---|
| 1 | 5 | Vehicle | - | 11, 15, 18, and 22 days after transplantation |
| 2 | 5 | A486-MB492/B 167-ml0r | 7.5 | 11, 15, 18, and 22 days after transplantation |
| 3 | 5 | A488-MB492/B226-ml0r | 7.5 | 11, 15, 18, and 22 days after transplantation |

The anti-tumor effect in each group was assessed by tumor volume calculated as described in Reference Example 6-4-1. The results confirmed anti-tumor effects of A486-MB492/B167-ml0r and A488-MB492/B226-ml0r (Figure 65).

(6-4-15) Antibody administration and sampling as well as FCM analysis for assessment of the systemic effects of A486-MB492/B167-ml0r and A488-MB492/B226-ml0r

Following establishment of the MC38 transplantation mouse model in Reference Example 6-4-1, Vehicle, NS1-MB492 (non-switch antibody), A486-MB492/B167-ml0r and A488-MB492/B226-ml0r were administered as shown in Table 61. Grouping was performed 10 days after cell transplantation, and antibody administration was performed via the tail vein route 11, 15, 18, and 22 days after cell transplantation for a total of four times. For Vehicle, PBS containing 0.05% Tween 20 was used.
The liver, lymph nodes, and spleens were collected 13 days after the initial administration in the manner as shown in Reference Example 6-4-2 (collection of various organs from mice transplanted with the MC38 cell line and preparation of lymphocyte fractions). For the spleens, only measurement of the organ weight was conducted; and for the lymph nodes, only cell counting of the lymphocyte fractions was performed.

Group details for the assessment of systemic responses with the A486-MB492/B167-ml0r and A488-MB492/B226-ml0r antibodies

[Table 61]

| Group | Number of animals | Pharmaceutical agent | Dose [mg/kg] | Day of administration | Day of sampling |
|---|---|---|---|---|---|
| 1 | 3 | Vehicle | - | 11, 15, 18, and 22 days after transplantation | 24 days after transplantation (13 days after initial administration) |
| 2 | 3 | NS1-MB492 | 7.5 | 11, 15, 18, and 22 days after transplantation | 24 days after transplantation (13 days after initial administration) |

(continued)

| Group | Number of animals | Pharmaceutical agent | Dose [mg/kg] | Day of administration | Day of sampling |
|---|---|---|---|---|---|
| 3 | 3 | A486-MB492/ B167-ml0r | 7.5 | 11, 15, 18, and 22 days after transplantation | 24 days after transplantation (13 days after initial administration) |
| 4 | 3 | A488-MB492/ B226-ml0r | 7.5 | 11, 15, 18, and 22 days after transplantation | 24 days after transplantation (13 days after initial administration) |

As a result of cell count measurement of the lymphocyte fractions for the lymph nodes and measurement of spleen weight, increases in the lymphocyte cell count and spleen weight were observed when NS1-MB492 was administered. On the other hand, it was observed that increases in the lymphocyte count and spleen weight were suppressed when A486-MB492/B167-ml0r and A488-MB492/B226-ml0r were administered compared with those when NS1-MB492 was administered (Figure 66).

As a result of evaluating the activation of T cells in the liver, an increase in the percentage of CD8$\alpha$-positive cells was observed when NS1-MB492, A486-MB492/B167-ml0r and A488-MB492/B226-ml0r were administered, as compared with Vehicle (Figure 67).

From the above, it was confirmed that compared with the non-switch antibody, the switch antibody inhibited the activation of T cells in the lymph nodes and spleens.

(6-4-16) Drug efficacy (anti-tumor effect) study on the A489-MB492/B223-ml0r antibody

Following establishment of the MC38 transplantation mouse model in Reference Example 6-4-1, Vehicle and A489-MB492/B223-ml0r were administered at the doses shown in Table 62. Antibody administration was performed four times via the tail vein route on the following day of grouping and four, seven, and ten days after grouping. For Vehicle, PBS containing 0.05% Tween 20 was used.

Group details for the drug efficacy study on A489-MB492/B223-ml0r

[Table 62]

| Group | Number of animals | Pharmaceutical agent | Dose [mg/kg] | Day of administration |
|---|---|---|---|---|
| 1 | 5 | Vehicle | - | On the following day of grouping, and four, seven, and ten days after grouping |
| 2 | 5 | A489-MB492/ B223-ml0r | 7.5 | On the following day of grouping, and four, seven, and ten days after grouping |

The anti-tumor effect in each group was assessed by tumor volume calculated as described in Reference Example 6-4-1. As a result, an anti-tumor effect was confirmed at 7.5 mg/kg administration (Figure 68).

(6-4-17) Antibody administration and sampling as well as FCM analysis for the assessment of systemic effects of the A489-MB492/B223-ml0r antibody

Following establishment of the MC38 transplantation mouse model in Reference Example 6-4-1, Vehicle, NS1-MB492 (non-switch antibody) and A489-MB492/B223-ml0r were administered as shown in Table 63. Antibody administration was performed via the tail vein route on the day of grouping, and 3 days and 6 days after grouping for a total of three times. For Vehicle, PBS containing 0.05% Tween 20 was used.

Seven days after the initial administration, the liver, lymph nodes, and spleens were collected in the manner as shown in Reference Example 6-4-2 (collection of various organs from mice transplanted with the MC38 cell line and preparation of lymphocyte fractions). For the spleens and lymph nodes, only cell count measurement of the prepared lymphocyte fractions was performed.

Group details for the assessment of systemic responses of the A489-MB492/B223-ml0r antibody

[Table 63]

| Group | Number of animals | Pharmaceutical agent | Dose [mg/kg] | Day of administration | Day of sampling |
|---|---|---|---|---|---|
| 1 | 3 | Vehicle | - | On the day of grouping, and three and six days after grouping | Seven days after grouping |
| 2 | 3 | NS1-MB492 | 7.5 | On the day of grouping, and three and six days after grouping | Seven days after grouping |
| 3 | 3 | A489-MB492/ B223-ml0r | 7.5 | On the day of grouping, and three and six days after grouping | Seven days after grouping |

As a result of cell count measurement of the lymphocyte fractions for the lymph nodes and spleens, increases in the cell count were observed when NS1-MB492 was administered. On the other hand, suppression of increase in the lymphocyte count was observed when A489-MB492/B223-ml0r was administered compared with that when NS1-MB492 was administered (Figure 69).

As a result of evaluating the activation of T cells in the liver, an increase in the percentage of CD8$\alpha$-positive cells was observed when NS1-MB492 and A489-MB492/B223-ml0r were administered, as compared with Vehicle (Figure 70).

From the above, it was confirmed that compared with the non-switch antibody, the switch antibody inhibited the activation of T cells in the lymph nodes and spleens.

(6-4-18) Drug efficacy (anti-tumor effect) study on the A548-mIgG1/B256-ml0r and A551-mIgG1/B256-ml0r antibodies

Following establishment of the MC38 transplantation mouse model in Reference Example 6-4-1, Vehicle, A548-mIgG1/B256-ml0r and A551-mIgG1/B256-ml0r were administered at the doses shown in Table 64. Antibody administration was performed twice via the tail vein route on the day of grouping and three days after grouping. For Vehicle, PBS containing 0.05% Tween 20 was used.

Group details for the drug efficacy study on the A548-mIgG1/B256-ml0r and A551-mIgG1/B256-ml0r antibodies

[Table 64]

| Group | Number of animals | Pharmaceutical agent | Dose [mg/kg] | Day of administration |
|---|---|---|---|---|
| 1 | 5 | Vehicle | - | On the day of grouping and three days after grouping |
| 2 | 5 | A548-mIgG1/ B256-ml0r | 7.5 | On the day of grouping and three days after grouping |
| 3 | 5 | A548-mIgG1/ B256-ml0r | 37.5 | On the day of grouping and three days after grouping |
| 4 | 4 | A548-mIgG1/ B256-ml0r | 100 | On the day of grouping and three days after grouping |
| 5 | 5 | A551-mIgG1/ B256-ml0r | 7.5 | On the day of grouping and three days after grouping |

(continued)

| Group | Number of animals | Pharmaceutical agent | Dose [mg/kg] | Day of administration |
|---|---|---|---|---|
| 6 | 5 | A551-mIgG1/ B256-ml0r | 37.5 | On the day of grouping and three days after grouping |
| 7 | 4 | A551-mIgG1/ B256-ml0r | 100 | On the day of grouping and three days after grouping |

The anti-tumor effect in each group was assessed by tumor volume calculated as described in Reference Example 6-4-1. The results showed an anti-tumor effect by administration of A548-mIgG1/B256-ml0r at 37.5 mg/kg and 100 mg/kg. A551-mIgG1/B256-ml0r also showed a significant anti-tumor effect at 100 mg/kg (Figure 71).

(6-4-19) Antibody administration and sampling as well as FCM analysis for the assessment of systemic effects of the A548-mIgG1/B256-and A551-mIgG1/B256-antibodies

Following establishment of the MC38 transplantation mouse model in Reference Example 6-4-1, Vehicle, NS1-mIgG1 (non-switch antibody), A548-mIgG1B256-ml0r and A551-mIgG1/B256-ml0r were administered as shown in Table 65. Antibody administration was performed twice via the tail vein route on the day of grouping and 3 days after grouping. For Vehicle, PBS containing 0.05% Tween 20 was used.

Five days after the initial administration, the liver, lymph nodes, and spleens were collected in the manner as shown in Reference Example 6-4-2 (collection of various organs from mice transplanted with the MC38 cell line and preparation of lymphocyte fractions). For the spleens and lymph nodes, only organ weight measurement was performed.

Group details for assessment of systemic responses with the A548-mIgG1/B256-ml0r and A551-mIgG1/B256-ml0r antibodies

[Table 65]

| Group | Number of animals | Pharmaceutical agent | Dose [mg/kg] | Day of administration | Day of sampling |
|---|---|---|---|---|---|
| 1 | 3 | Vehicle | - | On the day of grouping and three days after grouping | Five days after grouping |
| 2 | 3 | NS1-mIgG1 | 0.3 | On the day of grouping and three days after grouping | Five days after grouping |
| 3 | 3 | NS1-mIgG1 | 1.5 | On the day of grouping and three days after grouping | Five days after grouping |
| 4 | 3 | NS1-mIgG1 | 7.5 | On the day of grouping and three days after grouping | Five days after grouping |
| 5 | 3 | A548-mIgG1/ B256-ml0r | 1.5 | On the day of grouping and three days after grouping | Five days after grouping |
| 6 | 3 | A548-mIgG1/ B256-ml0r | 7.5 | On the day of grouping and three days after grouping | Five days after grouping |
| 7 | 3 | A548-mIgG1/ B256-ml0r | 37.5 | On the day of grouping and three days after grouping | Five days after grouping |
| 8 | 3 | A548-mIgG1/ B256-ml0r | 100 | On the day of grouping and three days after grouping | Five days after grouping |
| 9 | 3 | A551-mIgG1/ B256-ml0r | 1.5 | On the day of grouping and three days after grouping | Five days after grouping |

(continued)

| Group | Number of animals | Pharmaceutical agent | Dose [mg/kg] | Day of administration | Day of sampling |
|---|---|---|---|---|---|
| 10 | 3 | A551-mIgG1/ B256-ml0r | 7.5 | On the day of grouping and three days after grouping | Five days after grouping |
| 11 | 3 | A551-mIgG1/ B256-ml0r | 37.5 | On the day of grouping and three days after grouping | Five days after grouping |
| 12 | 3 | A551-mIgG1/ B256-ml0r | 100 | On the day of grouping and three days after grouping | Five days after grouping |

As a result of evaluating the organ weight of the lymph nodes and spleens, increases in the organ weight were observed when NS1-mIgG1 was administered at 0.3 mg/kg, 1.5 mg/kg, and 7.5 mg/kg. On the other hand, organ hypertrophy was not observed when A548-mIgG1B256-ml0r or A551-mIgG1/B256-ml0r was administered at any dose of 1.5 mg/kg, 7.5 mg/kg, 37.5 mg/kg, and 100 mg/kg (Figure 72).

As a result of evaluating the activation of T cells in the liver, increases in the expressions of both Granzyme B and PD-1 were observed when NS1-mIgG1 was administered at 0.3 mg/kg. On the other hand, the expressions were suppressed when A548-mIgG1/B256-ml0r and A551-mIgG1/B256-ml0r were administered at any dose of 1.5 mg/kg, 7.5 mg/kg, 37.5 mg/kg, and 100 mg/kg (Figure 73).

Thus, compared with the non-switch antibody, the switch antibody inhibited the activation of T cells in the lymph nodes, spleens, and liver.

(6-4-20) Drug efficacy (anti-tumor effect) study on the A551-MB110/B379-ml0r antibody

Following establishment of the MC38 transplantation mouse model in Reference Example 6-4-1, Vehicle and A551-MB110/B379-ml0r were administered at the doses shown in Table 66. Antibody administration was performed twice via the tail vein route on the day of grouping and three days after grouping. For Vehicle, PBS containing 0.05% Tween 20 was used.

Group details for the drug efficacy study of the A551-MB110/B379-ml0r antibody

[Table 66]

| Group | Number of animals | Pharmaceutical agent | Dose [mg/kg] | Day of administration |
|---|---|---|---|---|
| 1 | 6 | Vehicle | - | On the day of grouping and three days after grouping |
| 2 | 5 | A551-MB110/ B379-ml0r | 2.5 | On the day of grouping and three days after grouping |
| 3 | 6 | A551-MB110/ B379-ml0r | 7.5 | On the day of grouping and three days after grouping |
| 4 | 6 | A551-MB110/ B379-ml0r | 22.5 | On the day of grouping and three days after grouping |

The anti-tumor effect in each group was assessed by tumor volume calculated as described in Reference Example 6-4-1. As a result, an anti-tumor effect was confirmed at any dosages (Figure 74).

(6-4-21) Antibody administration and sampling as well as FCM analysis for the assessment of systemic effects

Following establishment of the MC38 transplantation mouse model in Reference Example 6-4-1, Vehicle, NS1-mIgG1 (non-switch antibody) and A551-MB110/B379-ml0r were administered as shown in Table 67. Antibody administration was performed twice via the tail vein route on the day of grouping and 3 days after grouping. For Vehicle, PBS containing 0.05% Tween 20 was used.

The liver, lymph nodes, and spleens were collected 5 days after the initial administration in the manner as shown in Reference Example 6-4-2 (collection of various organs from the MC38 cell line transplanted mice and preparation of lymphocyte fractions), and FCM analysis was performed in the manner as shown in Reference Example 6-4-3 (FCM analysis using lymphocyte fractions of various organs).

Group details for the assessment of systemic responses with the A551-MB110/B379-ml0r antibody

[Table 67]

| Group | Number of animals | Pharmaceutical agent | Dose [mg/kg] | Day of administration | Day of sampling |
|---|---|---|---|---|---|
| 1 | 3 | Vehicle | - | On the day of grouping and three days after grouping | Five days after grouping |
| 2 | 3 | NS1-mIgG1 | 7.5 | On the day of grouping and three days after grouping | Five days after grouping |
| 3 | 3 | A551-MB110/ B379-ml0r | 0.83 | On the day of grouping and three days after grouping | Five days after grouping |
| 4 | 3 | A551-MB110/ B379-ml0r | 2.5 | On the day of grouping and three days after grouping | Five days after grouping |
| 5 | 3 | A551-MB110/ B379-ml0r | 7.5 | On the day of grouping and three days after grouping | Five days after grouping |
| 6 | 3 | A551-MB110/ B379-ml0r | 22.5 | On the day of grouping and three days after grouping | Five days after grouping |

As a result of evaluating the organ weight of the lymph nodes and spleens, an increase in the organ weight was observed when NS1-mIgG1 was administered at 7.5 mg/kg. On the other hand, no organ enlargement was observed when A551-MB110/B379-ml0r was administered at any dose of 0.83 mg/kg, 2.5 mg/kg, 7.5 mg/kg and 22.5 mg/kg (Figure 75). In addition, in the assessment of T cell activation in the spleens by FCM evaluation, the increase in activation markers (PD-1, ICOS, Granzyme B) observed upon NS1-mIgG1 administration was not confirmed when A551-MB110/B379-ml0r was administered at any dose (Figure 76).

In the assessment of T cell activation in the liver, increases in the expressions of both PD-1 and ICOS were observed when NS1-mIgG1 was administered at 7.5 mg/kg. On the other hand, suppression of the expression to the Vehicle level when A551-MB110/B379-ml0r was administered at 0.83 mg/kg and 2.5 mg/kg, and the expressions were suppressed even more than that of NS1-mIgG1 when it was administered at 7.5 mg/kg (Figure 77).

Thus, compared with the non-switch antibody, the switch antibody inhibited the activation of T cells in the lymph nodes, spleens, and liver.

Reference Example 7: Generation of modified Fc capable of enhancing agonist activity

(7-1) Generation of variants with increased FcγR-binding activity

Heavy chain constant region TT14 (SEQ ID NO: 149) which increases the FcγRIIb-binding activity as described in WO2017104783 (heavy chain constant region containing T250V/T307P as amino acid modifications and L234Y/P238D/V264I/A330K as amino acid modifications to increase FcγRIIb-binding activity in the Fc region comprising SEQ ID NO: 182 (modified positions are both in EU numbering)), TT16 (SEQ ID NO: 150) comprising the G237D amino acid mutation introduced into heavy chain constant region TT11 which increases FcγRIIb-binding activity as described in WO2017104783 (heavy chain constant region containing amino acid modification of T250V/T307P and L234Y/P238D/A330K as modifications to increase FcγRIIb-binding activity in the Fc region comprising SEQ ID NO: 182 (modified positions are both in EU numbering)), heavy chain constant region P587 (SEQ ID NO: 151) which increases FcγRIIb-binding activity as described in WO2014163101; and P253 (SEQ ID NO: 93) which was pre-existing FcγRIIb increased binding heavy chain constant region, were combined with the heavy chain variable region of the switch antibody (A375; A372; A356; A486; A488; A489; A548; and A551) or the heavy chain variable region of the negative control antibody (IC17HdK (SEQ ID NO: 152)). This created genes with combination of each constant region and heavy chain variable region as follows.

[Table 68]

| Full-length heavy chain | Heavy-chain variable region (SEQ ID NO) | Heavy-chain constant region (SEQ ID NO) |
|---|---|---|
| A375-TT14 | A375 (SEQ ID NO: 43) | TT14 (SEQ ID NO: 149) |
| A375-TT16 | A375 (SEQ ID NO: 43) | TT16 (SEQ ID NO: 150) |
| A375-P587 | A375 (SEQ ID NO: 43) | P587 (SEQ ID NO: 151) |
| A372-P253 | A372 (SEQ ID NO: 44) | P253 (SEQ ID NO: 93) |
| A356-TT16 | A356 (SEQ ID NO: 45) | TT16 (SEQ ID NO: 150) |
| A356-P587 | A356 (SEQ ID NO: 45) | P587 (SEQ ID NO: 151) |
| A486-P253 | A486 (SEQ ID NO: 46) | P253 (SEQ ID NO: 93) |
| A488-P253 | A488 (SEQ ID NO: 48) | P253 (SEQ ID NO: 93) |
| A489-P253 | A489 (SEQ ID NO: 49) | P253 (SEQ ID NO: 93) |
| A548-P587 | A548 (SEQ ID NO: 50) | P587 (SEQ ID NO: 151) |
| A551-TT16 | A551 (SEQ ID NO: 51) | TT16 (SEQ ID NO: 150) |
| A551-P587 | A551 (SEQ ID NO: 51) | P587 (SEQ ID NO: 151) |
| IC17HdK-TT16 | IC17HdK (SEQ ID NO: 152) | TT16 (SEQ ID NO: 150) |
| IC17HdK-P587 | IC17HdK (SEQ ID NO: 152) | P587 (SEQ ID NO: 151) |
| IC17HdK-P253 | IC17HdK (SEQ ID NO: 152) | P253 (SEQ ID NO: 93) |

The heavy chain variable region of a switch antibody (A375, A356, and A551) or the heavy chain variable region of a negative control antibody (IC17HdK (SEQ ID NO: 152)) was combined with the heavy chain constant region MY201 (SEQ ID NO: 153) (a heavy chain constant region containing G236N/H268D/A330K as amino acid modifications that increase the activity to bind FcγRIIb in the Fc region comprising the amino acid sequence of SEQ ID NO: 182 (the modification positions are all in EU numbering)) or MY518 (SEQ ID NO: 154) (a heavy chain constant region containing L235W/G236N/H268D/Q295L/K326T/A330K as amino acid modifications that increase the activity to bind FcγRIIb in the Fc region comprising the amino acid sequence of SEQ ID NO: 182 (the modification positions are all in EU numbering), which are described in WO 2017104783 and have increased binding activity to FcγR, the heavy chain constant region MY201a (SEQ ID NO: 155) or MY518a (SEQ ID NO: 156) in which the K214R modification has been introduced into MY201 or MY518, or the heavy chain constant region MY201aPh (SEQ ID NO: 157) in which the L235W modification has been introduced into MY201a. This created genes with combinations of each constant region and heavy chain variable region as follows.

[Table 69]

| Full-length heavy chain | Heavy-chain variable region (SEQ ID NO) | Heavy-chain constant region (SEQ ID NO) |
|---|---|---|
| A375-MY201aPh | A375 (SEQ ID NO: 43) | MY201aPh (SEQ ID NO: 157) |
| A375-MY518 | A375 (SEQ ID NO: 43) | MY518 (SEQ ID NO: 154) |
| A375-MY518a | A375 (SEQ ID NO: 43) | MY518a (SEQ ID NO: 156) |
| A356-MY518 | A356 (SEQ ID NO: 45) | MY518 (SEQ ID NO: 154) |
| A551-MY201a | A551 (SEQ ID NO: 51) | MY201a SEQ ID NO: 155) |
| A551-MY201aPh | A551 (SEQ ID NO: 51) | MY201aPh (SEQ ID NO: 157) |
| A551-MY518 | A551 (SEQ ID NO: 51) | MY518 (SEQ ID NO: 154) |
| A551-MY518a | A551 (SEQ ID NO: 51) | MY518a (SEQ ID NO: 156) |

(continued)

| Full-length heavy chain | Heavy-chain variable region (SEQ ID NO) | Heavy-chain constant region (SEQ ID NO) |
|---|---|---|
| IC17HdK-MY201 | IC17HdK (SEQ ID NO: 152) | MY201 (SEQ ID NO: 153) |
| IC17HdK-MY201aPh | IC17HdK (SEQ ID NO: 152) | MY201aPh (SEQ ID NO: 157) |
| IC17HdK-MY518 | IC17HdK (SEQ ID NO: 152) | MY518 (SEQ ID NO: 154) |
| IC17HdK-MY518a | IC17HdK (SEQ ID NO: 152) | MY518a (SEQ ID NO: 156) |

As shown in Table 70, the antibodies of interest were expressed and purified using methods known to those skilled in the art, by combining the above antibody heavy chain genes with the antibody light chain gene of a switch antibody (B040-Lamlib, B167-Lamlib, B226-Lamlib, B223-Lamlib, B256-Lamlib, and B379-Lamlib made in Reference Examples 5-2 and 5-3) or the antibody light chain gene of the negative control antibody (IC17L-k0, generated by combining the light chain variable region IC17L (SEQ ID NO: 158) with human κ chain k0 (SEQ ID NO: 141) as the light chain constant region).

Variants with increased FcγR-binding activity

[Table 70]

| Variable region (heavy chain / light chain) | Heavy-chain constant region | Light-chain constant region | Antibody name |
|---|---|---|---|
| A375 / B167 | MY201aPh | Lamlib | A375-MY201aPh/B167-Lamlib |
| | MY518 | Lamlib | A375-MY518/B167-Lamlib |
| | MY518a | Lamlib | A375-MY518a/B167-Lamlib |
| | P587 | Lamlib | A375-P587/B167-Lamlib |
| | TT14 | Lamlib | A375-TT14/B167-Lamlib |
| | TT16 | Lamlib | A375-TT16/B167-Lamlib |
| A372 / B040 | P253 | Lamlib | A372-P253/B040-Lamlib |
| A356 / B040 | MY518 | Lamlib | A356-MY518/B040-Lamlib |
| | P587 | Lamlib | A356-P587/B040-Lamlib |
| | TT16 | Lamlib | A356-TT16/B040-Lamlib |
| A486 / B167 | P253 | Lamlib | A486-P253/B167-Lamlib |
| A488 / B226 | P253 | Lamlib | A488-P253/B226-Lamlib |
| A489 / B223 | P253 | Lamlib | A489-P253/B223-Lamlib |
| A548 / B256 | P587 | Lamlib | A548-P587/B256-Lamlib |
| A551 / B256 | MY201a | Lamlib | A551-MY201a/B256-Lamlib |
| | MY201aPh | Lamlib | A551-MY201aPh/B256-Lamlib |
| | MY518 | Lamlib | A551-MY518/B256-Lamlib |
| | P587 | Lamlib | A551-P587/B256-Lamlib |

(continued)

| Variable region (heavy chain / light chain) | Heavy-chain constant region | Light-chain constant region | Antibody name |
|---|---|---|---|
| A551 / B379 | MY518a | Lamlib | A551-MY518a/B379-Lamlib |
| | P587 | Lamlib | A551-P587/B379-Lamlib |
| | TT16 | Lamlib | A551-TT16/B379-Lamlib |
| IC17HdK / IC17L | MY201 | k0 | IC17HdK-MY201/IC17L-k0 |
| | MY201aPh | k0 | IC17HdK-MY201aPh/IC17L-k0 |
| | MY518 | k0 | IC17HdK-MY518/IC17L-k0 |
| | MY518a | k0 | IC17HdK-MY518a/IC17L-k0 |
| | P253 | k0 | IC17HdK-P253/IC17L-k0 |
| | P587 | k0 | IC17HdK-P587/IC17L-k0 |
| | TT16 | k0 | IC17HdK-TT16/IC17L-k0 |

(7-2) Generation of variants with increased pI by amino acid modification of the constant region

A heavy chain constant region was generated by combining a heavy chain constant region with increased FcγR-binding activity made in Reference Example 7-1 with Q311R, P343R and D413K, which are amino acid mutations that increase pI without greatly altering the FcγR-binding activity as described in WO2017046994.

Specifically, genes of the heavy chain constant region with amino acid mutations introduced into each of the antibody heavy chain constant region genes were generated as shown below.

- Introduction of P343R/D413K into MY518 (SEQ ID NO: 154): SCF025 (SEQ ID NO: 64)
- Introduction of P343R/D413K into MY518a (SEQ ID NO: 156): SCF025a (SEQ ID NO: 65)
- Introduction of P343R/D413K into TT14 (SEQ ID NO: 149): SCF027 (SEQ ID NO: 66)
- Introduction of P343R/D413K into TT16 (SEQ ID NO: 150): SCF028 (SEQ ID NO: 67)
- Introduction of Q311R/P343R into MY518 (SEQ ID NO: 154): SCF030 (SEQ ID NO: 68)
- Introduction of Q311R/P343R into TT14 (SEQ ID NO: 149): SCF032 (SEQ ID NO: 69)
- Introduction of Q311R/P343R into TT16 (SEQ ID NO: 150): SCF033 (SEQ ID NO: 70)
- Introduction of P343R into MY518 (SEQ ID NO: 154): SCF039 (SEQ ID NO: 71)
- Introduction of P343R into MY518a (SEQ ID NO: 156): SCF039a (SEQ ID NO: 72)
- Introduction of D413K into MY518 (SEQ ID NO: 154): SCF040 (SEQ ID NO: 73)
- Introduction of P343R into MY201a (SEQ ID NO: 155): SCF041a (SEQ ID NO: 74)
- Introduction of P343R into MY201aPh (SEQ ID NO: 157): SCF041aPh (SEQ ID NO: 75)
- Introduction of D413K into MY201a (SEQ ID NO: 155): SCF042a (SEQ ID NO: 76)
- Introduction of P343R/D413K into MY201a (SEQ ID NO: 155): SCF043a (SEQ ID NO: 77)
- Introduction of P343R/D413K into MY201aPh (SEQ ID NO: 157): SCF043aPh (SEQ ID NO: 78)
- Introduction of Q311R into MY201a (SEQ ID NO: 155): SCF056a (SEQ ID NO: 79)
- Introduction of Q311R into MY201aPh (SEQ ID NO: 157): SCF056aPh (SEQ ID NO: 80)
- Introduction of Q311R/P343R into MY201a (SEQ ID NO: 155): SCF057a (SEQ ID NO: 81)
- Introduction of Q311R/P343R into MY201aPh (SEQ ID NO: 157): SCF057aPh (SEQ ID NO: 82)
- Introduction of Q311R/D413K into MY201a (SEQ ID NO: 155): SCF059a (SEQ ID NO: 83)
- Introduction of Q311R/D413K into MY201aPh (SEQ ID NO: 157): SCF059aPh (SEQ ID NO: 84)
- Introduction of Q311R into MY518a (SEQ ID NO: 156): SCF060a (SEQ ID NO: 85)

Antibody heavy chain genes were produced as shown in Table 71 below by combining a heavy chain constant region gene produced here with a gene of the heavy chain variable region A375 (SEQ ID NO: 43), or A551 (SEQ ID NO: 51).

[Table 71]

| Full-length heavy chain | Heavy-chain variable region (SEQ ID NO) | Heavy-chain constant region (SEQ ID NO) |
|---|---|---|
| A375-SCF025 | A375 (SEQ ID NO: 43) | SCF025 (SEQ ID NO: 64) |
| A375-SCF027 | A375 (SEQ ID NO: 43) | SCF027 (SEQ ID NO: 66) |
| A375-SCF028 | A375 (SEQ ID NO: 43) | SCF028 (SEQ ID NO: 67) |
| A375-SCF030 | A375 (SEQ ID NO: 43) | SCF030 (SEQ ID NO: 68) |
| A375-SCF032 | A375 (SEQ ID NO: 43) | SCF032 (SEQ ID NO: 69) |
| A375-SCF033 | A375 (SEQ ID NO: 43) | SCF033 (SEQ ID NO: 70) |
| A375-SCF039 | A375 (SEQ ID NO: 43) | SCF039 (SEQ ID NO: 71) |
| A375-SCF040 | A375 (SEQ ID NO: 43) | SCF040 (SEQ ID NO: 73) |
| A375-SCF041aPh | A375 (SEQ ID NO: 43) | SCF041aPh (SEQ ID NO: 75) |
| A375-SCF043aPh | A375 (SEQ ID NO: 43) | SCF043aPh (SEQ ID NO: 78) |
| A375-SCF056aPh | A375 (SEQ ID NO: 43) | SCF056aPh (SEQ ID NO: 80) |
| A375-SCF057aPh | A375 (SEQ ID NO: 43) | SCF057aPh (SEQ ID NO: 82) |
| A375-SCF059aPh | A375 (SEQ ID NO: 43) | SCF059aPh (SEQ ID NO: 84) |
| A375-SCF060a | A375 (SEQ ID NO: 43) | SCF060a (SEQ ID NO: 85) |
| A551-SCF025a | A551 (SEQ ID NO: 51) | SCF025a (SEQ ID NO: 65) |
| A551-SCF039a | A551 (SEQ ID NO: 51) | SCF039a (SEQ ID NO: 72) |
| A551-SCF041a | A551 (SEQ ID NO: 51) | SCF041a (SEQ ID NO: 74) |
| A551-SCF041aPh | A551 (SEQ ID NO: 51) | SCF041aPh (SEQ ID NO: 75) |
| A551-SCF043a | A551 (SEQ ID NO: 51) | SCF043a (SEQ ID NO: 77) |
| A551-SCF043aPh | A551 (SEQ ID NO: 51) | SCF043aPh (SEQ ID NO: 78) |
| A551-SCF056a | A551 (SEQ ID NO: 51) | SCF056a (SEQ ID NO: 79) |
| A551-SCF056aPh | A551 (SEQ ID NO: 51) | SCF056aPh (SEQ ID NO: 80) |
| A551-SCF057a | A551 (SEQ ID NO: 51) | SCF057a (SEQ ID NO: 81) |
| A551-SCF057aPh | A551 (SEQ ID NO: 51) | SCF057aPh (SEQ ID NO: 82) |
| A551-SCF059a | A551 (SEQ ID NO: 51) | SCF059a (SEQ ID NO: 83) |
| A551-SCF059aPh | A551 (SEQ ID NO: 51) | SCF059aPh (SEQ ID NO: 84) |
| A551-SCF060a | A551 (SEQ ID NO: 51) | SCF060a (SEQ ID NO: 85) |

In addition, by combining these antibody heavy chain genes with the B167-Lamlib, the B256-Lamlib, and the B379-Lamlib genes produced in Reference Example 5-3 as antibody light chain genes, the desired antibody was expressed and purified as shown in Table 72 by methods known to those skilled in the art.

[Table 72]

| Variable region (heavy chain / light chain) | Heavy-chain constant region | Light-chain constant region | Antibody name |
|---|---|---|---|
| A375 / B167 (A375 SEQ ID NO: 43) (B167 SEQ ID NO: 54) | SCF025 (SEQ ID NO: 64) | **Lamlib** (SEQ ID NO: 63) | A375-SCF025/B167- LamLib |
| | SCF027 (SEQ ID NO: 66) | **Lamlib** (SEQ ID NO: 63) | A376-SCF027/B167-LamLib |
| | SCF028 (SEQ ID NO : 67) | **Lamlib** (SEQ ID NO: 63) | A375-SCF0281B167-LamLib |
| | SCF030 (SEQ ID NO : 68) | **Lamlib** (SEQ ID NO: 63) | A375-SCF030/B167-LamLib |
| | SCF032 (SEQ ID NO : 69) | **Lamlib** (SEQ ID NO: 63) | A375-SCF032/B167-LamLib |
| | SCF033 (SEQ ID NO : 70) | **Lamlib** (SEQ ID NO: 63) | A376-SCF033/B167-LamLib |
| | SCF039 (SEQ ID NO: 71) | **Lamlib** (SEQ ID NO : 63) | A376-SCF039/B167-LamLib |
| | SCF040 (SEQ ID NO: 73) | **Lamlib** (SEQ ID NO : 63) | A376-SCF040/B167-Lam Lib |
| | SCF041aPh (SEQ ID NO: 75) | **Lamlib** (SEQ ID NO : 63) | A375-SCF041aPh/B167-LamLib |
| | SCF043aPh (SEQ ID NO : 78) | **Lamlib** (SEQ ID NO : 63) | A376-SCF043aPh/B167LamLib |
| | SCF056aPh (SEQ ID NO : 80) | **Lamlib** (SEQ ID NO : 63) | A375-SCF056aPh/B167LamLib |
| | SCF057aPh (SEQ ID NO: 82) | **Lamlib** (SEQ ID NO : 63) | A376-SCF067aPh/B167-LamLib |
| | **SCF059aPh** (SEQ ID NO: 84) | **Lamlib** (SEQ ID NO : 63) | A375-SCF059aPh/B167-LamLib |
| | SCF060a (SEQ ID NO: 85) | **Lamlib** (SEQ ID NO : 63) | A376-SCF060a/B167-LamLib |

(continued)

| Variable region (heavy chain / light chain) | Heavy-chain constant region | Light-chain constant region | Antibody name |
|---|---|---|---|
| A551 / B256 (A551 SEQ ID NO: 51) (B256 SEQ ID NO : 59) | SCF025a (SEQ ID NO: 65) | **Lamlib** (SEQ ID NO : 63) | A551-SCF025aB256-LamLib |
| | SCF039a (SEQ ID NO : 72) | **Lamlib** (SEQ ID NO : 63) | A551-SCF039aJB256-LamLib |
| | SCF041a (SEQ ID NO: 74) | **Lamlib** (SEQ ID NO : 63) | A551-SCF041a/B256-LamLib |
| | SCF041aPh (SEQ ID NO: 75) | **Lamlib** (SEQ ID NO : 63) | A551SCF041aPh/B256-LamLib |
| | SCF043a (SEQ ID NO: 77) | **Lamlib** (SEQ ID NO : 63) | A551-SCF043aB256-LamLib |
| | **SCF043aPh** (SEQ ID NO: 78) | **Lamlib** (SEQ ID NO : 63) | A551-SCF043aPh/B256-LamLib |
| | SCF056a (SEQ ID NO: 79) | **Lamlib** (SEQ ID NO: 63) | A551-SCF056a/B266-LamLib |
| | **SCF066aPh** (SEQ ID NO: 80) | **Lamlib** (SEQ ID NO: 63) | A551-SCF066aPh/B266-LamLib |
| | SCF057a (SEQ ID NO : 81) | **Lamlib** (SEQ ID NO : 63) | A551-SCF057a/B256-LamLib |
| | SCF057aPh (SEQ ID NO: 82) | **Lamlib** (SEQ ID NO : 63) | A551-SCF057aPh/B256-LamLib |
| | SCF059a (SEQ ID NO: 83) | **Lamlib** (SEQ ID NO : 63) | A551-SCF059a/B256-LamLib |
| | SCF059aPh (SEQ ID NO : 84) | **Lamlib** (SEQ ID NO : 63) | A551-SCF059aPh/B256-LamLib |

(continued)

| Variable region (heavy chain / light chain) | Heavy-chain constant region | Light-chain constant region | Antibody name |
|---|---|---|---|
| A551 / B379 (A551 SEQ ID NO : 51) (B379 SEQ ID NO : 60) | SCF039a (SEQ ID NO : 72) | **Lamlib** (SEQ ID NO : 63) | A551SCF039a/B379LamLib |
| | SCF041a (SEQ ID NO : 74) | **Lamlib** (SEQ ID NO : 63) | A551SCF041a/B379LamLib |
| | **SCF041aPh** (SEQ ID NO : 75) | **Lamlib** (SEQ ID NO : 63) | A551-SCF041aPh/B379-LamLib |
| | **SCF043a** (SEQ ID NO : 77) | **Lamlib** (SEQ ID NO : 63) | A551-SCF043a/B379-LamLib |
| | SCF043aPh (SEQ ID NO: 78) | **Lamlib** (SEQ ID NO : 63) | A551-SCF043aPh/B379-LamLib |
| | SCF056a (SEQ ID NO : 79) | **Lamlib** (SEQ ID NO : 63) | A551-SCF056a/B379-LamLib |
| | SCF056aPh (SEQ ID NO: 80) | **Lamlib** (SEQ ID NO: 63) | A551-SCF056aPh/B379-LamLib |
| | SCF057a (SEQ ID NO : 81) | **Lamlib** (SEQ ID NO: 63) | A551-SCF057a/B379-LamLib |
| | **SCF057aPh** (SEQ ID NO: 82) | **Lamlib** (SEQ ID NO: 63) | A551SCF057aPh/B379LamLib |
| | SCF059a (SEQ ID NO : 83) | **Lamlib** (SEQ ID NO: 63) | A551-SCF059a/B379-LamLib |
| | SCF069aPh (SEQ ID NO : 84) | **Lamlib** (SEQ ID NO: 63) | A551-SCF059aPh/B379LamLib |
| | SCF060a (SEQ ID NO: 85) | Lamlib (SEQ ID NO : 63) | A551-SCF060a/B379-LamLib |

(7-3) Generation of control antibodies for comparison of agonist activity

Antibodies (IC17HdK-MY201/IC17L-k0; IC17HdK-MY201aPh/IC17L-k0; IC17HdK-MY518/IC17L-k0; IC17HdK-MY518a/IC17L-k0; IC17HdK-P253/IC17L-k0; IC17HdK-P587/IC17L-k0; IC17HdK-TT16/IC17L-k0) with the heavy chain variable region IC17HdK (SEQ ID NO: 152) produced in Reference Example 7-1 as a control antibody for comparison of agonist activity were produced. In addition, the antibody of interest (IC17HdK-G4d/IC17L-k0) was expressed and purified by methods known in the art by combining the gene of the antibody light chain IC17L-k0 (SEQ ID NO: 158, variable region; SEQ ID NO: 141, constant region) with the gene of the antibody heavy chain gene IC17HdK-G4d which was produced by combining the gene of the heavy chain variable region IC17HdK (SEQ ID NO: 152) with the G4d gene (SEQ ID NO: 159) from which Gly and Lys of the C-terminus of the heavy chain constant region of human IgG4 had been removed. These control antibodies were used in Reference Example 5.

(7-4) Evaluation of human Fcγ receptor binding of variants with increased pI by amino acid modification of the constant region

Biacore T200 (GE Healthcare) was used to evaluate the binding activity to the respective human Fcγ receptor (hereafter referred to as FcγR). Measurement was performed at 25 °C using 50 mM phosphate, 150 mM NaCl, 0.05 w/v%-P20, pH 7.4 as buffer. About 1000 RU of antibody was captured using a sensor chip immobilized with Capture-Select™ Human Fab-lambda Kinetics Biotin Conjugate (Thermo Fisher Scientific) as a molecule for ligand capturing. Human FcγR was diluted to 8 nM for FcγRIa and 1000 nM for other FcγR in the measurement buffer, and it was interacted to the captured antibody. The binding activity of each antibody to FcγR was assessed by calculating the binding amount of FcγR (RU) per unit amount of antibody using Biacore T200 Evaluation Software 2.0.
The extracellular domain of FcγR was prepared by the methods below. First, gene synthesis of the extracellular domain of FcγR was carried out by a method known to those skilled in the art. At that time, the FcγR sequences were prepared based on the NCBI-registered data. Specifically, the FcγRI sequence was generated based on NCBI Accession No. NM_000566.3, the FcγRIIa sequence on NCBI Accession No. NM_001136219.1, the FcγRIIb sequence on NCBI Accession No. NM_004001.3, and the FcγRIIIa sequence on NCBI Accession No. NM_001127593.1; and a His tag was added to their C termini. The polymorphic site of FcγRIIa was produced by referring to J. Exp. Med., 1990, 172, 19-25; and the polymorphic site of FcγRIIIa was produced with reference to J. Clin. Invest., 1997, 100, 1059-1070. The resulting gene fragments were inserted into an animal cell expression vector to produce expression vectors. The produced expression vectors were transiently introduced into human embryonic kidney cell-derived FreeStyle293 cells (Invitrogen), and the proteins of interest were expressed. The culture supernatants were collected and then passed through a 0.22 μm filter, and they were purified in principle by the four steps below. The first step was performed by cation-exchange column chromatography (SP Sepharose FF), the second step by affinity column chromatography (HisTrap HP) for His tag, the third step by gel-filtration column chromatography (Superdex200), and the fourth step by sterile filtration. However, for FcγRI, anion-exchange column chromatography with Q Sepharose FF was performed in the first step. Concentrations of the purified proteins were calculated by measuring the absorbance at 280 nm using a spectrophotometer and the absorbance coefficients calculated from the obtained values by the methods of PACE et al. (Protein Science, 1995, 4, 2411-2423).

Table 73 shows the binding amount per unit amount of antibody, as well as the amount relative to the binding amount of A375-G1T3/B167-Lamlib. All the antibodies in Table 73 showed greater binding amount for human FcγRIIb per unit amount of antibody than that of A375-G1T3/B167-Lamlib, and the relative value was 2.85-14.26 when the binding amount of A375-G1T3/B167-Lamlib to human FcγRIIb was set to 1.0. The amount of human FcγRIIb binding per unit amount of antibody and the relative values of binding amount were similar in the presence or absence of amino acid modifications for increasing pI of the constant region. Also, the binding amount of antibodies containing the L235W modification to hFcgRIa was reduced compared to that of A375-TT14/B167-Lamlib.

Binding activity of each human FcγR

[Table 73]

| Abbreviated name | Amount of binding per 1RU of antibody (RU) | | | | | | Relative value of binding amount | | | | | |
|---|---|---|---|---|---|---|---|---|---|---|---|---|
| | hFcqRIa | hFcgRIIaH | hFcgRIIaR | hFcgRIIb | hFcgRIIIaF | hFcgRIIIaV | hFcgRIa | hFcgRIIaH | hFcgRIIaR | hFcqRIIb | hFcgRIIIaF | hFcgRIIIaV |
| A375-G1T3/B167-Lamlib | 0.0813 | 0.0567 | 0.0463 | 0.0110 | 0.0213 | 0.0700 | 1.00 | 1.00 | 1.00 | 1.00 | 1.00 | 1.00 |
| A375-MY201a/B167-Lamlib | 0.0396 | 0.0534 | 0.0338 | 0.0401 | 0.0007 | 0.0024 | 0.49 | 0.94 | 0.73 | 3.66 | 0.04 | 0.03 |
| A375-MY201aPh/B167-Lamlib | 0.0040 | 0.0328 | 0.0335 | 0.0313 | 0.0007 | 0.0011 | 0.05 | 0.58 | 0.72 | 2.85 | 0.03 | 0.02 |
| A375-MY518/B167-Lamlib | 0.0020 | 0.0466 | 0.0460 | 0.0482 | 0.0009 | 0.0015 | 0.02 | 0.82 | 0.99 | 4.40 | 0.04 | 0.02 |
| A375-MY518a/B167-Lamlib | 0.0033 | 0.0479 | 0.0470 | 0.0489 | 0.0006 | 0.0009 | 0.04 | 0.84 | 1.01 | 4.46 | 0.03 | 0.01 |
| A375-TT11/B167-Lamlib | 0.0075 | 0.0020 | 0.0041 | 0.0477 | -0.0009 | -0.0010 | 0.09 | 0.03 | 0.09 | 4.35 | -0.04 | -0.01 |
| A375-TT14/B167-Lamlib | 0.0045 | 0.0042 | 0.0067 | 0.0805 | 0.0000 | 0.0003 | 0.05 | 0.07 | 0.14 | 7.34 | 0.00 | 0.00 |
| A375-TT16/B167-Lamlib | 0.0034 | 0.0126 | 0.0359 | 0.1090 | 0.0001 | -0.0002 | 0.04 | 0.22 | 0.77 | 9.94 | 0.00 | 0.00 |
| A375-P587/B167-Lamlib | 0.0962 | 0.0029 | 0.0371 | 0.1427 | 0.0001 | 0.0001 | 1.18 | 0.05 | 0.80 | 13.02 | 0.00 | 0.00 |
| A375-P253/B167-Lamlib | 0.0937 | 0.0497 | 0.1678 | 0.1563 | 0.0056 | 0.0019 | 1.15 | 0.88 | 3.62 | 14.26 | 0.26 | 0.03 |
| A375-SCF025/B167-Lamlib | 0.0022 | 0.0514 | 0.0519 | 0.0512 | 0.0012 | 0.0014 | 0.03 | 0.91 | 1.12 | 4.67 | 0.06 | 0.02 |

| Abbreviated name | Amount of binding per 1RU of antibody (RU) | | | | | | Relative value of binding amount | | | | | |
|---|---|---|---|---|---|---|---|---|---|---|---|---|
| | hFcqRIa | hFcgRIIaH | hFcgRIIaR | hFcgRIIb | hFcgRIIIaF | hFcgRIIIaV | hFcgRIa | hFcgRIIaH | hFcgRIIaR | hFcqRIIb | hFcgRIIIaF | hFcgRIIIaV |
| A375-SCF025a/B167-Lamlib | 0.0021 | 0.0514 | 0.0518 | 0.0511 | 0.0009 | 0.0016 | 0.03 | 0.91 | 1.12 | 4.67 | 0.04 | 0.02 |
| A375-SCF027/B167-Lamlib | 0.0044 | 0.0031 | 0.0058 | 0.0766 | -0.0005 | -0.0006 | 0.05 | 0.06 | 0.12 | 6.99 | -0.02 | -0.01 |
| A375-SCF028/B167-Lamlib | 0.0027 | 0.0101 | 0.0299 | 0.0995 | -0.0009 | -0.0010 | 0.03 | 0.18 | 0.64 | 9.08 | -0.04 | -0.01 |
| A375-SCF030/B167-Lamlib | 0.0020 | 0.0533 | 0.0532 | 0.0532 | 0.0017 | 0.0020 | 0.02 | 0.94 | 1.15 | 4.85 | 0.08 | 0.03 |
| A375-SCF032/B167-Lamlib | 0.0049 | 0.0030 | -0.0004 | 0.0782 | -0.0007 | -0.0007 | 0.06 | 0.05 | -0.01 | 7.14 | -0.03 | -0.01 |
| A375-SCF033/B167-Lamlib | 0.0033 | 0.0103 | 0.0255 | 0.0979 | 0.0000 | 0.0002 | 0.04 | 0.18 | 0.55 | 8.94 | 0.00 | 0.00 |
| A375-SCF039/B167-Lamlib | 0.0018 | 0.0535 | 0.0542 | 0.0531 | 0.0008 | 0.0013 | 0.02 | 0.94 | 1.17 | 4.85 | 0.04 | 0.02 |
| A375-SCF039a/B167-Lamlib | 0.0018 | 0.0542 | 0.0554 | 0.0540 | 0.0012 | 0.0013 | 0.02 | 0.96 | 1.20 | 4.93 | 0.06 | 0.02 |
| A375-SCF040/B167-Lamlib | 0.0016 | 0.0454 | 0.0446 | 0.0465 | 0.0010 | 0.0013 | 0.02 | 0.80 | 0.96 | 4.24 | 0.05 | 0.02 |
| A375-SCF041a/B167-Lamlib | 0.0446 | 0.0571 | 0.0376 | 0.0433 | 0.0009 | 0.0022 | 0.55 | 1.01 | 0.81 | 3.95 | 0.04 | 0.03 |

(continued)

| Abbreviated name | Amount of binding per 1RU of antibody (RU) | | | | | | Relative value of binding amount | | | | | |
|---|---|---|---|---|---|---|---|---|---|---|---|---|
| | hFcqRIa | hFcgRIIaH | hFcgRIIaR | hFcgRIIb | hFcgRIIIaF | hFcgRIIIaV | hFcgRIa | hFcgRIIaH | hFcgRIIaR | hFcqRIIb | hFcgRIIIaF | hFcgRIIIaV |
| A375-SCF042a/B167-Lamli b | 0.0396 | 0.0522 | 0.0327 | 0.0393 | 0.0003 | 0.0014 | 0.49 | 0.92 | 0.71 | 3.59 | 0.01 | 0.02 |
| A375-SCF043a/B167-Lamlib | 0.0429 | 0.0552 | 0.0361 | 0.0419 | 0.0011 | 0.0025 | 0.53 | 0.97 | 0.78 | 3.82 | 0.05 | 0.04 |
| A375-SCF056a/B167-Lamlib | 0.0395 | 0.0529 | 0.0331 | 0.0399 | 0.0011 | 0.0023 | 0.49 | 0.93 | 0.71 | 3.64 | 0.05 | 0.03 |
| A375-SCF057a/B167-Lamlib | 0.0405 | 0.0525 | 0.0367 | 0.0412 | 0.0013 | 0.0025 | 0.50 | 0.93 | 0.79 | 3.76 | 0.06 | 0.04 |
| A375-SCF059a/B167-Lamlib | 0.0380 | 0.0502 | 0.0319 | 0.0358 | 0.0010 | 0.0021 | 0.47 | 0.88 | 0.69 | 3.27 | 0.05 | 0.03 |
| A375-SCF060a/B167-Lamlib | 0.0021 | 0.0463 | 0.0459 | 0.0472 | 0.0011 | 0.0008 | 0.03 | 0.82 | 0.99 | 4.31 | 0.05 | 0.01 |
| A375-SCF041aPh/B167-Lamlib | 0.0013 | 0.0371 | 0.0371 | 0.0350 | 0.0007 | 0.0005 | 0.02 | 0.65 | 0.80 | 3.20 | 0.03 | 0.01 |
| A375-SCF043aPh/B167-Lamlib | 0.0011 | 0.0349 | 0.0349 | 0.0339 | 0.0001 | 0.0001 | 0.01 | 0.62 | 0.75 | 3.10 | 0.00 | 0.00 |
| A375-SCF056aPh/B167-Lamlib | 0.0019 | 0.0314 | 0.0299 | 0.0292 | 0.0010 | 0.0010 | 0.02 | 0.55 | 0.64 | 2.67 | 0.05 | 0.01 |
| A375-SCF057aPh/B167-Lamlib | 0.0021 | 0.0365 | 0.0364 | 0.0345 | -0.0003 | 0.0005 | 0.03 | 0.64 | 0.79 | 3.15 | -0.02 | 0.01 |

163

(continued)

| Abbreviated name | Amount of binding per 1RU of antibody (RU) | | | | | | Relative value of binding amount | | | | | |
|---|---|---|---|---|---|---|---|---|---|---|---|---|
| | hFcqRIa | hFcgRIIaH | hFcgRIIaR | hFcgRIIb | hFcgRIIIaF | hFcgRIIIaV | hFcgRIa | hFcgRIIaH | hFcgRIIaR | hFcqRIIb | hFcgRIIIaF | hFcgRIIIaV |
| A375-SCF059aPh/B167-Lamlib | 0.0089 | 0.0332 | 0.0319 | 0.0291 | 0.0003 | 0.0028 | 0.11 | 0.59 | 0.69 | 2.66 | 0.01 | 0.04 |

(7-5) Assessment of human FcRn binding of variants with increased pI by amino acid modification of the constant region

Biacore T200 (GE Healthcare) was used to assess the binding activity to human FcRn. Measurement was performed at 25 °C using 50 mM phosphate, 150 mM NaCl, 0.05 w/v%-P20, pH 6.0 as buffer. Human FcRn proteins used for this determination were prepared by the procedure described in WO2010107110. About 400 RU of antibody was captured using a sensor chip immobilized with CaptureSelect™ Human Fab-lambda Kinetics Biotin Conjugate (Thermo Fisher Scientific) as a molecule for ligand capturing, followed by the binding of human FcRn diluted in measuring buffer. The activity of the respective antibodies to bind FcRn was assessed by calculating KD(mol/L) by the steady-state model using Biacore T200 Evaluation Software 2.0. The KD values were similar in the presence or absence of amino acid modifications for increasing pI of the constant region. Table 74 shows the KD (mol/L) of the respective antibodies for human FcRn.

[Table 74]

| Abbreviated name | $K_D$ (mol/L) |
| --- | --- |
| A375-G1T3/B167-Lamlib | 1.27E-06 |
| A375-MY201a/B167-Lamlib | 1.19E-06 |
| A375-MY201aPh/B167-Lamlib | 1.18E-06 |
| A375-MY518/B167-Lamlib | 1.27E-06 |
| A375-MY518a/B167-Lamlib | 1.32E-06 |
| A375-TT11/B167-Lamlib | 1.56E-06 |
| A375-TT14/B167-Lamlib | 1.77E-06 |
| A375-TT16/B167-Lamlib | 1.51E-06 |
| A375-P587/B167-Lamlib | 1.70E-06 |
| A375-P253/B167-Lamlib | 1.42E-06 |
| A375-SCF025/B167-Lamlib | 1.10E-06 |
| A375-SCF025a/B167-Lamlib | 1.13E-06 |
| A375-SCF027/B167-Lamlib | 1.33E-06 |
| A375-SCF028/B167-Lamlib | 1.27E-06 |
| A375-SCF030/B167-Lamlib | 8.63E-07 |
| A375-SCF032/B167-Lamlib | 9.66E-07 |
| A375-SCF033/B167-Lamlib | 8.74E-07 |
| A375-SCF039/B167-Lamlib | 1.09E-06 |
| A375-SCF039a/B167-Lamlib | 1.11E-06 |
| A375-SCF040/B167-Lamlib | 1.46E-06 |
| A375-SCF041a/B167-Lamlib | 1.10E-06 |
| A375-SCF042a/B167-Lamlib | 1.49E-06 |
| A375-SCF043a/B167-Lamlib | 1.18E-06 |
| A375-SCF056a/B167-Lamlib | 9.56E-07 |
| A375-SCF057a/B167-Lamlib | 9.28E-07 |
| A375-SCF059a/B167-Lamlib | 1.03E-06 |
| A375-SCF060a/B167-Lamlib | 1.01E-06 |
| A375-SCF041aPh/B167-Lamlib | 1.13E-06 |
| A375-SCF043aPh/B167-Lamlib | 1.21E-06 |
| A375-SCF056aPh/B167-Lamlib | 1.00E-06 |

(continued)

| Abbreviated name | $K_D$ (mol/L) |
|---|---|
| A375-SCF057aPh/B167-Lamlib | 9.09E-07 |
| A375-SCF059aPh/B167-Lamlib | 1.05E-06 |

Reference Example 8: Assessment of the *in vitro* ATP- and ADP-dependent CD137 agonist activity of the modified anti-human CD137 antibodies using the 4-1BB Jurkat reporter gene assay

The GloResponse™ NF-κB Luc2/4-1BB Jurkat cell line (Promega, CS196004) was used for measurement of the *in vitro* activity of the variants generated in Reference Example 7-2 (Table 72). To each well of 384-well plates, 10 μL each of FcγRIIB CHO-K1 Cells (Promega) prepared at the concentration of $4 \times 10^5$/mL with an assay medium (99% RPMI, 1% FBS) was added. Subsequently, 10 μL of an antibody solution containing ADP, or an antibody solution containing ATP, or an antibody solution without ATP or ADP was added each well. Then, 10 μL of the GloResponseTM NF-κB-Luc2/4-1BB Jurkat cell line prepared to $2 \times 10^6$/mL with the assay medium (99% RPMI, 1% FBS) was added to each well. The final concentration of ADP was 10 μM, and the final concentration of ATP was 10 μM. The plates were left to stand for 6 hours at 37°C in a 5% $CO_2$ incubator and 15 minutes at room temperature; and 30 μL of the Bio-Glo reagent was added to each well. The Bio-Glo Luciferase Assay System (Buffer and Substrate) used for the Bio-Glo reagent. Subsequently, the amount of luminescence of each well was measured with a plate reader. The value of the luminescence of each well divided by the value of the luminescence of the well without antibody addition was defined as "relative light unit" (fold induction), and it served as an indicator for evaluating the CD137 agonist activity of each antibody.

The results are shown in Figure 78. From Figure 78, it was confirmed that A375-P587/B167-LamLib, A551-P587/B256-LamLib, A551-P587/B379-LamLib, A375-SCF041aPh/B167-LamLib, A551-SCF041aPh/B256-LamLib, A551-SCF041aPh/B379-LamLib, A375-SCF043aPh/B167-LamLib, A551-SCF043aPh/B256-LamLib, A551-SCF043aPh/B379-LamLib, A375-SCF057aPh/B167-LamLib, A551-SCF057aPh/B256-LamLib, and A551-SCF057aPh/B379-LamLib show human CD137 agonist activity in a ATP- and ADP-dependent manner.

Reference Example 9: Enhancement of agonist activity by modified anti-CD137 antibodies (9-1) Preparation of antibodies for evaluation

Regarding the case where various amino acid modifications for increasing pI were introduced to the heavy-chain constant region with increased binding activity to FcγRIIb, the effect of such amino acid modifications on CD137 agonist activity and on the plasma kinetics and drug efficacy in hCD137KI/mFcγR2bKO/hFcγR2bTg#90 mice was evaluated. Firstly, as described in Reference Example 7-1, Reference Example 7-2, and Reference Example 7-3, A375-MY201aPh/B167-Lamlib, A375-SCF041aPh/B167-Lamlib, A375-SCF057aPh/B167-Lamlib, and IC17HdK-MY201aPh/IC17L-k0 were prepared.

(9-2) Assessment of the *in vitro* ATP-dependent CD137 agonist activity of the modified anti-human CD137 antibodies using the 4-1BB Jurkat reporter gene assay

Regarding the case where various amino acid modifications for increasing pI were introduced to the heavy-chain constant region with increased binding activity to FcγRIIb, in order to assess the effect of such amino acid modifications on CD137 agonist activity, the CD137 agonist activity of A375-MY201aPh/B167-Lamlib, A375-SCF041aPh/B167-Lamlib, A375-SCF057aPh/B167-Lamlib, and IC17HdK-MY201aPh/IC17L-k0 were assessed.

The GloResponse™ NF-κB-Luc2/4-1BB Jurkat cell line (Promega, CS196004) was used for measuring the *in vitro* activity of the produced antibodies. To each well of a 96-well plate, 200 μL of FcγRIIB CHO-K1 Cells (Promega) prepared at a concentration of $5 \times 10^4$/mL with a culture medium was added, and the plate was allowed to stand overnight in a 5% $CO_2$ incubator at 37°C. CHO culture medium (90% Ham's F12, 10% FBS) was used for the culture medium. Next, all of the culture medium was removed by suction, and then 25 μL of a GloResponse™ NF-κB-Luc2/4-1BB Jurkat cell line prepared at $2 \times 10^6$/mL with an assay medium (99% RPMI, 1% FBS) was added to each well. Next, 25μL of each antibody solution diluted with an assay medium was added at a final concentration of 0, 0.001, 0.01, 0.1, 1, and 10 μg/mL. Lastly, 25μL of an ATP solution diluted with an assay medium to make a final concentration of 250 μM was added. The plate was allowed to stand for 6 hours in a 5% $CO_2$ incubator at 37°C, and then allowed to stand for 15 minutes at room temperature, and 75 μL of a Bio-Glo reagent was added to each well. The Bio-Glo Luciferase Assay System (Buffer and Substrate) was used for the Bio-Glo reagent. Thereafter, the relative light unit of each well was determined using a plate reader. The value of the luminescence of each well divided by the value of the luminescence of the well without antibody addition was defined as "relative light unit", and it served as an indicator for evaluating the CD137 agonist activity of each antibody.

The results are shown in Figure 79. The results showed that CD137 agonist activity is increased by introducing various amino acid modifications for increasing pI.

(9-3) Pharmacokinetic study of modified anti-human CD137 antibodies in mouse

(9-3-1) Generation of hCD137KI/mFcγR2bKO/hFcγR2bTg#90 mouse

First, a human CD137 knock-in mouse was generated in which the mouse CD137 gene was replaced with a human CD137 gene, by introducing a human CD137 gene substitution vector into mouse embryonic stem cells (ES cells) together with a Zinc Finger Nuclease (ZFN) that targets mouse CD137. Next, ZFN mRNA that targets the mouse Fcgr2b gene was microinjected to mouse fertilized eggs, and mouse Fcgr2b knock-out mice were generated by selecting those introduced with the mutation at the target site. Further, a BAC vector in which the human FCGR2B gene was cloned was microinjected to mouse fertilized eggs, and by selecting from the mice obtained therefrom those introduced with the genome region of the human FCGR2B gene, a human FCGR2B transgenic mouse was generated (Iwayanagi. et al., J Immunol, 2015, 195, 3198-3205).

By crossing mice of the above three lines, a "human CD137 knock-in Fcgr2b knock-out human FCGR2B transgenic mouse" was established. This mouse is referred to as hCD137KI/mFcγR2bKO/hFcγR2bTg#90 mouse.

(9-3-2) Measurement of anti-human CD137 antibody concentration in plasma in the hCD137KJ/mFcγR2bKO/hFcγR2bTg#90 mouse model

The respective anti-human CD137 antibodies were administered intravenously at a single dose to the CD137KI/mFcγR2bKO/hFcγR2bTg#90 mice as shown in Table 75. Blood was collected multiple times over time from 5 minutes to 28 days after administration. The obtained blood was centrifuged to separate plasma. Plasma was stored in a freezer set below - 20°C until measurement.

[Table 75]

| Group | Number of animals | Pharmaceutical agent | Dose [mg/kg] |
|---|---|---|---|
| 1 | 3 | A375-MY201aPh/ B167-Lamlib | 7.5 Single administration |
| 2 | 3 | A375-SCF041aPh/ B167-Lamlib | 7.5 Single administration |

The concentration of each anti-human CD137 antibody in plasma was measured by the electrochemiluminescence (ECL) method. hCD137 (Sino Biological Inc.) was diluted with PBS(-) and added to a MULTI-ARRAY 96-well Plate (Meso Scale Diagnostics, LLC). The plate added with hCD137 was shaken for 1 hour at room temperature and hCD137 was immobilized to the plate. For blocking, a PBS solution containing 1 mM ADP, 1% BSA, and 0.05% Tween-20 was then added and the plate was shaken for 1 hour at room temperature. Calibration curves for the respective anti-human CD137 antibodies were prepared at plasma concentrations of 640, 320, 160, 80, 40, 20, and 10 ng/mL. Plasma samples diluted with a PBS solution containing 1mM ADP, 1%BSA, and 0.05% Tween-20 and calibration curve samples were added to the hCD137-immobilized plate. The plate was then shaken for 1 hour at room temperature, and then an antibody described in WO 2019112027 which specifically recognizes the constant regions of the anti-human CD137 antibodies was added as a secondary antibody. The plate was further shaken for 1 hour at room temperature and SULFO-TAG Labeled Goat Anti-Rabbit Antibody (Meso Scale Diagnostics, LLC) was then added thereto. The plate was further shaken for 1 hour at room temperature and Read buffer T (Meso Scale Diagnostics, LLC) diluted two-fold and containing 1mM ADP was added thereto. Concentration of each antibody in mouse plasma was measured by detecting SULFO-TAG with SECTOR Imager (Meso Scale Diagnostics, LLC). Concentration of each antibody in mouse plasma was calculated using SOFTmax PRO (Molecular Devices).

The results are shown in Figure 80. A375-SCF041aPh/B167-Lamlib showed a faster elimination than A375-MY201aPh/B167-Lamlib. This is thought to be because the heavy-chain constant region of A375-SCF041aPh/B167-Lamlib is introduced with amino acid modifications that increase pI.

(9-4) Drug efficacy assessment of modified anti-human CD137 antibodies in mouse

(9-4-1) Generation of cell lines and a syngeneic tumor line transplanted mouse model

As the cells, the LLC1/OVA/GPC3 clone C5 (LLC1/OVA/GPC3) cell line, produced by introducing the expression plasmids for chicken ovalbumin (OVA) and human Glypican-3 (GPC3) into the mouse lung cancer cell-derived cell line LLC1 [LL/2 (alias: LLC1), distributor: ATCC, catalog number: CRL-1642], was used. The hCD137KI/mFcγR2bKO/hFcγR2bTg#90 mouse (11 weeks old, female) described above in (19-3-1) was used as the mouse. The LLC1/OVA/GPC3 cell line was maintained and passaged in an RPMI1640 medium (Sigma-Aldrich, Co. LLC.) containing 9.8% Fetal Bovine Serum (Sigma-Aldrich, Co. LLC.), 0.44 mg/mL G418 (Nacalai Tesque, Inc.), and 0.88 mg/mL Zeocin (Thermo Fisher Scientific, Inc.). The LLC1/OVA/GPC3 cell line was transplanted subcutaneously to the abdomen of a mouse, and the model was deemed as being formed when the tumor volume reached about 250-500 mm$^3$. After the model was formed, the LLC1/OVA/GPC3 cell line-transplanted mice were grouped and then administered with the vehicle and respective anti-human CD137 antibodies.

(9-4-2) Preparation and administration of the pharmaceutical agents to be administered and tumor measurement

A375-MY201aPh/B167-Lamlib or A375-SCF041aPh /B167-Lamlib, prepared to the dosages shown in Table 76 with PBS containing 0.05% Tween-20, was administered to the LLC1/OVA/GPC3 cell line-transplanted model on day 11 and day 14 after tumor transplantation. PBS containing 0.05% Tween 20 was administered to the vehicle group. The prepared administration liquid was administered at a dose of 10 mL/kg through the tail vein.

Measurement of antitumor effect in LLC1/OVA/GPC3 cell line-transplanted model

[Table 76]

| Group | Number of animals | Pharmaceutical agent | Dose | Method of administration | Day of administration |
|---|---|---|---|---|---|
| 1 | 5 | PBS containing 0.05% Tween-20 | - | Tail vain | Day 11 and Day 14 after transplantation |
| 2 | 5 | A375-MY201aPh/ B167-Lamlib | 2.50 mg/kg | Tail vain | Day 11 and Day 14 after transplantation |
| 3 | 5 | A375-SCF041aPh/ B167-Lamlib | 2.50 mg/kg | Tail vain | Day 11 and Day 14 after transplantation |

For evaluating antitumor effect, tumor volume was measured at a frequency of 1 to 2 times per week. Tumor volume was calculated by the following equation.

$$\text{Tumor volume (mm}^3) = \text{the length (mm) x the width (mm) x the width (mm)}/2$$

As a result, the results of Reference Example (9-3-2) revealed that the change in blood concentration in A375-SCF041aPh/B167-Lamlib administered mice was smaller compared to that for A375-MY201aPh/B167-Lamlib; however, A375-SCF041aPh/B167-Lamlib exhibited stronger antitumor effect than A375-MY201aPh/B167-Lamlib (Figure 81).
Based on the above results in the present mouse model, the antitumor effect of an anti-human CD137 antibody was increased by introducing amino acid modifications that increase pI to the heavy-chain constant region.
The above-mentioned invention has been described in detail with examples and illustrations for the purposes of assisting a clear understanding, and the description and illustrations herein should not be construed as limiting the scope of the invention.
All disclosures of patents and scientific literatures cited herein throughout are explicitly incorporated by reference.

[Industrial Applicability]

Anticancer agents comprising an anti-CD 137 antigen-binding molecule of the present disclosure as an active ingredient, combination therapies using such an anticancer agent comprising an anti-CD 137 antigen-binding domain as an active ingredient and another anticancer agent in combination, pharmaceutical compositions for use in such a combination therapy, and the like are applicable to the development, manufacturing, provision, and use of pharmaceuticals that possess an immune cell-activating effect, a cytotoxic effect, or an antitumor effect, but have low effects on non-tumor tissues such as normal tissues and few side effects.

**Claims**

1. An anticancer agent comprising as an active ingredient an anti-CD137 antigen-binding molecule comprising any combination of HVR-H1, HVR-H2, HVR-H3, HVR-L1, HVR-L2, and HVR-L3 selected from (a) to (m) below:

(a) HVR-H1 comprising the amino acid sequence of SEQ ID NO: 7, HVR-H2 comprising the amino acid sequence of SEQ ID NO: 8, HVR-H3 comprising the amino acid sequence of SEQ ID NO: 17, HVR-L1 comprising the amino acid sequence of SEQ ID NO: 21, HVR-L2 comprising the amino acid sequence of SEQ ID NO: 26, and HVR-L3 comprising the amino acid sequence of SEQ ID NO: 27;
(b) HVR-H1 comprising the amino acid sequence of SEQ ID NO: 7, HVR-H2 comprising the amino acid sequence of SEQ ID NO: 9, HVR-H3 comprising the amino acid sequence of SEQ ID NO: 17, HVR-L1 comprising the amino acid sequence of SEQ ID NO: 22, HVR-L2 comprising the amino acid sequence of SEQ ID NO: 26, and HVR-L3 comprising the amino acid sequence of SEQ ID NO: 27;
(c) HVR-H1 comprising the amino acid sequence of SEQ ID NO: 7, HVR-H2 comprising the amino acid sequence of SEQ ID NO: 10, HVR-H3 comprising the amino acid sequence of SEQ ID NO: 17, HVR-L1 comprising the amino acid sequence of SEQ ID NO: 22, HVR-L2 comprising the amino acid sequence of SEQ ID NO: 26, and HVR-L3 comprising the amino acid sequence of SEQ ID NO: 27;
(d) HVR-H1 comprising the amino acid sequence of SEQ ID NO: 7, HVR-H2 comprising the amino acid sequence of SEQ ID NO: 11, HVR-H3 comprising the amino acid sequence of SEQ ID NO: 18, HVR-L1 comprising the amino acid sequence of SEQ ID NO: 21, HVR-L2 comprising the amino acid sequence of SEQ ID NO: 26, and HVR-L3 comprising the amino acid sequence of SEQ ID NO: 27;
(e) HVR-H1 comprising the amino acid sequence of SEQ ID NO: 7, HVR-H2 comprising the amino acid sequence of SEQ ID NO: 8, HVR-H3 comprising the amino acid sequence of SEQ ID NO: 18, HVR-L1 comprising the amino acid sequence of SEQ ID NO: 21, HVR-L2 comprising the amino acid sequence of SEQ ID NO: 26, and HVR-L3 comprising the amino acid sequence of SEQ ID NO: 27;
(f) HVR-H1 comprising the amino acid sequence of SEQ ID NO: 7, HVR-H2 comprising the amino acid sequence of SEQ ID NO: 12, HVR-H3 comprising the amino acid sequence of SEQ ID NO: 18, HVR-L1 comprising the amino acid sequence of SEQ ID NO: 21, HVR-L2 comprising the amino acid sequence of SEQ ID NO: 26, and HVR-L3 comprising the amino acid sequence of SEQ ID NO: 28;
(g) HVR-H1 comprising the amino acid sequence of SEQ ID NO: 7, HVR-H2 comprising the amino acid sequence of SEQ ID NO: 13, HVR-H3 comprising the amino acid sequence of SEQ ID NO: 18, HVR-L1 comprising the amino acid sequence of SEQ ID NO: 21, HVR-L2 comprising the amino acid sequence of SEQ ID NO: 26, and HVR-L3 comprising the amino acid sequence of SEQ ID NO: 29;
(h) HVR-H1 comprising the amino acid sequence of SEQ ID NO: 7, HVR-H2 comprising the amino acid sequence of SEQ ID NO: 14, HVR-H3 comprising the amino acid sequence of SEQ ID NO: 19, HVR-L1 comprising the amino acid sequence of SEQ ID NO: 23, HVR-L2 comprising the amino acid sequence of SEQ ID NO: 26, and HVR-L3 comprising the amino acid sequence of SEQ ID NO: 27;
(i) HVR-H1 comprising the amino acid sequence of SEQ ID NO: 7, HVR-H2 comprising the amino acid sequence of SEQ ID NO: 15, HVR-H3 comprising the amino acid sequence of SEQ ID NO: 20, HVR-L1 comprising the amino acid sequence of SEQ ID NO: 24, HVR-L2 comprising the amino acid sequence of SEQ ID NO: 26, and HVR-L3 comprising the amino acid sequence of SEQ ID NO: 27;
(j) HVR-H1 comprising the amino acid sequence of SEQ ID NO: 7, HVR-H2 comprising the amino acid sequence of SEQ ID NO: 15, HVR-H3 comprising the amino acid sequence of SEQ ID NO: 20, HVR-L1 comprising the amino acid sequence of SEQ ID NO: 25, HVR-L2 comprising the amino acid sequence of SEQ ID NO: 26, and HVR-L3 comprising the amino acid sequence of SEQ ID NO: 27;
(k) HVR-H1 comprising the amino acid sequence of SEQ ID NO: 7, HVR-H2 comprising the amino acid sequence of SEQ ID NO: 16, HVR-H3 comprising the amino acid sequence of SEQ ID NO: 20, HVR-L1 comprising the amino acid sequence of SEQ ID NO: 25, HVR-L2 comprising the amino acid sequence of SEQ ID NO: 26, and HVR-L3 comprising the amino acid sequence of SEQ ID NO: 27;
(l) HVR-H1 comprising the amino acid sequence of SEQ ID NO: 7, HVR-H2 comprising the amino acid sequence of SEQ ID NO: 14, HVR-H3 comprising the amino acid sequence of SEQ ID NO: 19, HVR-L1 comprising the amino acid sequence of SEQ ID NO: 24, HVR-L2 comprising the amino acid sequence of SEQ ID NO: 26, and HVR-L3 comprising the amino acid sequence of SEQ ID NO: 27; and

(m) HVR-H1 comprising the amino acid sequence of SEQ ID NO: 7, HVR-H2 comprising the amino acid sequence of SEQ ID NO: 14, HVR-H3 comprising the amino acid sequence of SEQ ID NO: 17, HVR-L1 comprising the amino acid sequence of SEQ ID NO: 21, HVR-L2 comprising the amino acid sequence of SEQ ID NO: 26, and HVR-L3 comprising the amino acid sequence of SEQ ID NO: 27.

2. An anticancer agent comprising an anti-CD137 antigen-binding molecule which comprises any combination of VH and VL selected from (a) to (m) below:

(a) a VH comprising the amino acid sequence of SEQ ID NO: 43, and a VL comprising the amino acid sequence of SEQ ID NO: 54;

(b) a VH comprising the amino acid sequence of SEQ ID NO: 44, and a VL comprising the amino acid sequence of SEQ ID NO: 55;

(c) a VH comprising the amino acid sequence of SEQ ID NO: 45, and a VL comprising the amino acid sequence of SEQ ID NO: 55;

(d) a VH comprising the amino acid sequence of SEQ ID NO: 46, and a VL comprising the amino acid sequence of SEQ ID NO: 54;

(e) a VH comprising the amino acid sequence of SEQ ID NO: 47, and a VL comprising the amino acid sequence of SEQ ID NO: 54;

(f) a VH comprising the amino acid sequence of SEQ ID NO: 48, and a VL comprising the amino acid sequence of SEQ ID NO: 56;

(g) a VH comprising the amino acid sequence of SEQ ID NO: 49, and a VL comprising the amino acid sequence of SEQ ID NO: 57;

(h) a VH comprising the amino acid sequence of SEQ ID NO: 50, and a VL comprising the amino acid sequence of SEQ ID NO: 58;

(i) a VH comprising the amino acid sequence of SEQ ID NO: 51, and a VL comprising the amino acid sequence of SEQ ID NO: 59;

(j) a VH comprising the amino acid sequence of SEQ ID NO: 51, and a VL comprising the amino acid sequence of SEQ ID NO: 60;

(k) a VH comprising the amino acid sequence of SEQ ID NO: 52, and a VL comprising the amino acid sequence of SEQ ID NO: 60;

(l) a VH comprising the amino acid sequence of SEQ ID NO: 50, and a VL comprising the amino acid sequence of SEQ ID NO: 59; and

(m) a VH comprising the amino acid sequence of SEQ ID NO: 53, and a VL comprising the amino acid sequence of SEQ ID NO: 54.

3. The anticancer agent of claim 1 or 2, wherein the anti-CD137 antigen-binding molecule is a human antibody, humanized antibody, or chimeric antibody, or an antigen-binding fragment of any of them.

4. The anticancer agent of any one of claim 1 to 3, wherein the anti-CD137 antigen-binding molecule comprises an altered Fc region, and the altered Fc region comprises any one combination of amino acid alterations selected from the following according to EU numbering:

L235W/G236N/H268D/Q295L/K326T/A330K/P343R/D413K;
K214R/L235W/G236N/H268D/Q295L/K326T/A330K/P343R/D413K;
L234Y/P238D/T250V/V264I/T307P/A330K/P343R/D413K;
L234Y/P238D/V264I/A330K/P343R/D413K;
L234Y/G237D/P238D/T250V/T307P/A330K/P343R/D413K;
L234Y/G237D/P238D/A330K/P343R/D413K;
L235W/G236N/H268D/Q295L/K326T/A330K/Q311R/P343R;
L234Y/P238D/T250V/V264I/T307P/A330K/Q311R/P343R;
L234Y/P238D/V264I/A330K/Q311R/P343R;
L234Y/G237D/P238D/T250V/T307P/A330K/Q311R/P343R;
L234Y/G237D/P238D/A330K/Q311R/P343R;
L235W/G236N/H268D/Q295L/K326T/A330K/P343R;
K214R/L235W/G236N/H268D/Q295L/K326T/A330K/P343R;
L235W/G236N/H268D/Q295L/K326T/A330K/D413K;

K214R/G236N/H268D/A330K/P343R;
K214R/L235W/G236N/H268D/A330K/P343R;
K214R/G236N/H268D/A330K/D413K;
K214R/G236N/H268D/A330K/P343R/D413K;
K214R/L235W/G236N/H268D/A330K/P343R/D413K;
K214R/G236N/H268D/A330K/Q311R;
K214R/L235W/G236N/H268D/A330K/Q311R;
K214R/G236N/H268D/A330K/Q311R/P343R;
K214R/L235W/G236N/H268D/A330K/Q311R/P343R;
K214R/G236N/H268D/A330K/Q311R/D413K;
K214R/L235W/G236N/H268D/A330K/Q311R/D413K; and
K214R/L235W/G236N/H268D/Q295L/K326T/A330K/Q311R.

5. An anticancer agent comprising an anti-CD137 antigen-binding molecule which comprises any combination of VH, VL, CH and CL selected from (i) to (xxxviii) below:

(i) VH comprising the amino acid sequence of SEQ ID NO: 43, CH comprising the amino acid sequence of SEQ ID NO: 64, VL comprising the amino acid sequence of SEQ ID NO: 54, and CL comprising the amino acid sequence of SEQ ID NO: 63;

(ii) VH comprising the amino acid sequence of SEQ ID NO: 43, CH comprising the amino acid sequence of SEQ ID NO: 66, VL comprising the amino acid sequence of SEQ ID NO: 54, and CL comprising the amino acid sequence of SEQ ID NO: 63;

(iii) VH comprising the amino acid sequence of SEQ ID NO: 43, CH comprising the amino acid sequence of SEQ ID NO: 67, VL comprising the amino acid sequence of SEQ ID NO: 54, and CL comprising the amino acid sequence of SEQ ID NO: 63;

(iv) VH comprising the amino acid sequence of SEQ ID NO: 43, CH comprising the amino acid sequence of SEQ ID NO: 68, VL comprising the amino acid sequence of SEQ ID NO: 54, and CL comprising the amino acid sequence of SEQ ID NO: 63;

(v) VH comprising the amino acid sequence of SEQ ID NO: 43, CH comprising the amino acid sequence of SEQ ID NO: 69, VL comprising the amino acid sequence of SEQ ID NO: 54, and CL comprising the amino acid sequence of SEQ ID NO: 63;

(vi) VH comprising the amino acid sequence of SEQ ID NO: 43, CH comprising the amino acid sequence of SEQ ID NO: 70, VL comprising the amino acid sequence of SEQ ID NO: 54, and CL comprising the amino acid sequence of SEQ ID NO: 63;

(vii) VH comprising the amino acid sequence of SEQ ID NO: 43, CH comprising the amino acid sequence of SEQ ID NO: 71, VL comprising the amino acid sequence of SEQ ID NO: 54, and CL comprising the amino acid sequence of SEQ ID NO: 63;

(viii) VH comprising the amino acid sequence of SEQ ID NO: 43, CH comprising the amino acid sequence of SEQ ID NO: 73, VL comprising the amino acid sequence of SEQ ID NO: 54, and CL comprising the amino acid sequence of SEQ ID NO: 63;

(ix) VH comprising the amino acid sequence of SEQ ID NO: 43, CH comprising the amino acid sequence of SEQ ID NO: 75, VL comprising the amino acid sequence of SEQ ID NO: 54, and CL comprising the amino acid sequence of SEQ ID NO: 63;

(x) VH comprising the amino acid sequence of SEQ ID NO: 43, CH comprising the amino acid sequence of SEQ ID NO: 78, VL comprising the amino acid sequence of SEQ ID NO: 54, and CL comprising the amino acid sequence of SEQ ID NO: 63;

(xi) VH comprising the amino acid sequence of SEQ ID NO: 43, CH comprising the amino acid sequence of SEQ ID NO: 80, VL comprising the amino acid sequence of SEQ ID NO: 54, and CL comprising the amino acid sequence of SEQ ID NO: 63;

(xii) VH comprising the amino acid sequence of SEQ ID NO: 43, CH comprising the amino acid sequence of SEQ ID NO: 82, VL comprising the amino acid sequence of SEQ ID NO: 54, and CL comprising the amino acid sequence of SEQ ID NO: 63;

(xiii) VH comprising the amino acid sequence of SEQ ID NO: 43, CH comprising the amino acid sequence of SEQ ID NO: 84, VL comprising the amino acid sequence of SEQ ID NO: 54, and CL comprising the amino acid sequence of SEQ ID NO: 63;

(xiv) VH comprising the amino acid sequence of SEQ ID NO: 43, CH comprising the amino acid sequence of SEQ ID NO: 85, VL comprising the amino acid sequence of SEQ ID NO: 54, and CL comprising the amino acid sequence of SEQ ID NO: 63;

(xv) VH comprising the amino acid sequence of SEQ ID NO: 51, CH comprising the amino acid sequence of SEQ ID NO: 65, VL comprising the amino acid sequence of SEQ ID NO: 59, and CL comprising the amino acid sequence of SEQ ID NO: 63;

(xvi) VH comprising the amino acid sequence of SEQ ID NO: 51, CH comprising the amino acid sequence of SEQ ID NO: 72, VL comprising the amino acid sequence of SEQ ID NO: 59, and CL comprising the amino acid sequence of SEQ ID NO: 63;

(xvii) VH comprising the amino acid sequence of SEQ ID NO: 51, CH comprising the amino acid sequence of SEQ ID NO: 74, VL comprising the amino acid sequence of SEQ ID NO: 59, and CL comprising the amino acid sequence of SEQ ID NO: 63;

(xviii) VH comprising the amino acid sequence of SEQ ID NO: 51, CH comprising the amino acid sequence of SEQ ID NO: 75, VL comprising the amino acid sequence of SEQ ID NO: 59, and CL comprising the amino acid sequence of SEQ ID NO: 63;

(xix) VH comprising the amino acid sequence of SEQ ID NO: 51, CH comprising the amino acid sequence of SEQ ID NO: 77, VL comprising the amino acid sequence of SEQ ID NO: 59, and CL comprising the amino acid sequence of SEQ ID NO: 63;

(xx) VH comprising the amino acid sequence of SEQ ID NO: 51, CH comprising the amino acid sequence of SEQ ID NO: 78, VL comprising the amino acid sequence of SEQ ID NO: 59, and CL comprising the amino acid sequence of SEQ ID NO: 63;

(xxi) VH comprising the amino acid sequence of SEQ ID NO: 51, CH comprising the amino acid sequence of SEQ ID NO: 79, VL comprising the amino acid sequence of SEQ ID NO: 59, and CL comprising the amino acid sequence of SEQ ID NO: 63;

(xxii) VH comprising the amino acid sequence of SEQ ID NO: 51, CH comprising the amino acid sequence of SEQ ID NO: 80, VL comprising the amino acid sequence of SEQ ID NO: 59, and CL comprising the amino acid sequence of SEQ ID NO: 63;

(xxiii) VH comprising the amino acid sequence of SEQ ID NO: 51, CH comprising the amino acid sequence of SEQ ID NO: 81, VL comprising the amino acid sequence of SEQ ID NO: 59, and CL comprising the amino acid sequence of SEQ ID NO: 63;

(xxiv) VH comprising the amino acid sequence of SEQ ID NO: 51, CH comprising the amino acid sequence of SEQ ID NO: 82, VL comprising the amino acid sequence of SEQ ID NO: 59, and CL comprising the amino acid sequence of SEQ ID NO: 63;

(xxv) VH comprising the amino acid sequence of SEQ ID NO: 51, CH comprising the amino acid sequence of SEQ ID NO: 83, VL comprising the amino acid sequence of SEQ ID NO: 59, and CL comprising the amino acid sequence of SEQ ID NO: 63;

(xxvi) VH comprising the amino acid sequence of SEQ ID NO: 51, CH comprising the amino acid sequence of SEQ ID NO: 84, VL comprising the amino acid sequence of SEQ ID NO: 59, and CL comprising the amino acid sequence of SEQ ID NO: 63;

(xxvii) VH comprising the amino acid sequence of SEQ ID NO: 51, CH comprising the amino acid sequence of SEQ ID NO: 72, VL comprising the amino acid sequence of SEQ ID NO: 60, and CL comprising the amino acid sequence of SEQ ID NO: 63;

(xxviii) VH comprising the amino acid sequence of SEQ ID NO: 51, CH comprising the amino acid sequence of SEQ ID NO: 74, VL comprising the amino acid sequence of SEQ ID NO: 60, and CL comprising the amino acid sequence of SEQ ID NO: 63;

(xxix) VH comprising the amino acid sequence of SEQ ID NO: 51, CH comprising the amino acid sequence of SEQ ID NO: 75, VL comprising the amino acid sequence of SEQ ID NO: 60, and CL comprising the amino acid sequence of SEQ ID NO: 63;

(xxx) VH comprising the amino acid sequence of SEQ ID NO: 51, CH comprising the amino acid sequence of SEQ ID NO: 77, VL comprising the amino acid sequence of SEQ ID NO: 60, and CL comprising the amino acid sequence of SEQ ID NO: 63;

(xxxi) VH comprising the amino acid sequence of SEQ ID NO: 51, CH comprising the amino acid sequence of SEQ ID NO: 78, VL comprising the amino acid sequence of SEQ ID NO: 60, and CL comprising the amino acid sequence of SEQ ID NO: 63;

(xxxii) VH comprising the amino acid sequence of SEQ ID NO: 51, CH comprising the amino acid sequence of SEQ ID NO: 79, VL comprising the amino acid sequence of SEQ ID NO: 60, and CL comprising the amino acid sequence of SEQ ID NO: 63;

(xxxiii) VH comprising the amino acid sequence of SEQ ID NO: 51, CH comprising the amino acid sequence of SEQ ID NO: 80, VL comprising the amino acid sequence of SEQ ID NO: 60, and CL comprising the amino acid sequence of SEQ ID NO: 63;

(xxxiv) VH comprising the amino acid sequence of SEQ ID NO: 51, CH comprising the amino acid sequence of SEQ ID NO: 81, VL comprising the amino acid sequence of SEQ ID NO: 60, and CL comprising the amino acid sequence of SEQ ID NO: 63;

(xxxv) VH comprising the amino acid sequence of SEQ ID NO: 51, CH comprising the amino acid sequence of SEQ ID NO: 82, VL comprising the amino acid sequence of SEQ ID NO: 60, and CL comprising the amino acid sequence of SEQ ID NO: 63;

(xxxvi) VH comprising the amino acid sequence of SEQ ID NO: 51, CH comprising the amino acid sequence of SEQ ID NO: 83, VL comprising the amino acid sequence of SEQ ID NO: 60, and CL comprising the amino acid sequence of SEQ ID NO: 63;

(xxxvii) VH comprising the amino acid sequence of SEQ ID NO: 51, CH comprising the amino acid sequence of SEQ ID NO: 84, VL comprising the amino acid sequence of SEQ ID NO: 60, and CL comprising the amino acid sequence of SEQ ID NO: 63; and

(xxxviii) VH comprising the amino acid sequence of SEQ ID NO: 51, CH comprising the amino acid sequence of SEQ ID NO: 85, VL comprising the amino acid sequence of SEQ ID NO: 60, and CL comprising the amino acid sequence of SEQ ID NO: 63.

6. The anticancer agent of any one of claims 1 to 5, which is to be administered to a cancer patient having (i) a solid cancer infiltrated with one or more cells selected from a B cell, a dendritic cell, a natural killer cell, a macrophage, and a CD8$^+$ T cell, and/or (ii) a solid cancer infiltrated with a regulatory T (Treg) cell or CD4$^+$ T cell.

7. The anticancer agent of any one of claims 1 to 6, which is to be administered to a patient having a cancer refractory to treatment with an immune checkpoint inhibitor.

8. The anticancer agent of any one of claims 1 to 7, which is to be administered to a cancer patient having one or more cancers selected from the group consisting of gastric cancer, head and neck cancer, esophageal cancer, colorectal cancer, lung cancer, mesothelioma, liver cancer, ovarian cancer, breast cancer, colon cancer, kidney cancer, skin cancer, muscle tumor, pancreatic cancer, prostate cancer, testicular cancer, uterine cancer, cholangiocarcinoma, Merkel cell carcinoma, bladder cancer, thyroid cancer, schwannoma, adrenal cancer, anal cancer, central nervous system tumor, neuroendocrine tissue tumor, penile cancer, pleural tumor, salivary gland tumor, vulvar cancer, thymoma, lymphoma, myeloid leukemia and pediatric cancer.

9. The anticancer agent of any one of claims 1 to 5 for use in combination with at least one other anticancer agent.

10. The anticancer agent of claim 9, wherein the other anticancer agent is at least one anticancer agent selected from a chemotherapeutic agent, a T cell-activating agonist agent, an immune checkpoint inhibitor, a T cell-redirecting antigen-binding molecule, an antifibrotic agent, and an angiogenesis inhibitor.

11. The anticancer agent of claim 10, wherein the other anticancer agent is an immune checkpoint inhibitor.

12. The anticancer agent of claim 10 or 11, wherein the other anticancer agent is at least one immune checkpoint inhibitor selected from an anti-PDI antibody, an anti-PDLI antibody, an anti-TIGIT antibody, an anti-TIM3 antibody, and an anti-LAG3 antibody.

13. The anticancer agent of claim 10, wherein the other anticancer agent is a T cell-redirecting antigen-binding molecule.

14. The anticancer agent of claim 9, wherein the other anticancer agent is an agent that depletes and/or inactivates one or more cells selected from a regulatory T cell, a CD4$^+$ T cell, a B cell, a NK cell, and a macrophage.

15. The anticancer agent of any one of claims 9 to 14, which is to be administered to a cancer patient having one or more cancers selected from the group consisting of gastric cancer, head and neck cancer, esophageal cancer, colorectal cancer, lung cancer, mesothelioma, liver cancer, ovarian cancer, breast cancer, colon cancer, kidney cancer, skin cancer, muscle tumor, pancreatic cancer, prostate cancer, testicular cancer, uterine cancer, cholangiocarcinoma, Merkel cell carcinoma, bladder cancer, thyroid cancer, schwannoma, adrenal cancer, anal cancer, central nervous system tumor, neuroendocrine tissue tumor, penile cancer, pleural tumor, salivary gland tumor, vulvar cancer, thymoma, and pediatric cancer.

**FIG. 1**

FIG. 2

(A)

OVA tetramer$^+$ CD8$^+$ T cells
/CD8$^+$ T cells (%)

50% 40% 30% 20% 10% 0%

Vehicle    A551-MB110/B379-mIor

(B)

Granzyme B$^+$ CD8$^+$ T cells
/CD8$^+$ T cells (%)

50% 40% 30% 20% 10% 0%

Vehicle    A551-MB110/B379-mIor

(C)

PD-1$^+$ CD8$^+$ T cells
/CD8$^+$ T cells (%)

100% 75% 50% 25% 0%

Vehicle    A551-MB110/B379-mIor

(D)

KLRG-1$^+$ CD8$^+$ T cells
/CD8$^+$ T cells (%)

50.0% 40.0% 30.0% 20.0% 10.0% 0.0%

Vehicle    A551-MB110/B379-mIor

(E)

ICOS$^+$ CD8$^+$ T cells
/CD8$^+$ T cells (%)

100% 75% 50% 25% 0%

Vehicle    A551-MB110/B379-mIor

FIG. 3

(A)Vehicle group

(B) A551-MB110/B379-ml0r 2.5mg/kg group

FIG. 4

EP 4 104 857 A1

FIG. 5

(A) Vehicle group

(B) A551-MB110/B379-ml0r 2.5mg/kg group

FIG. 6

EP 4 104 857 A1

FIG. 7

(A) Vehicle group

(B) A551-MB110/B379-ml0r 2.5mg/kg group

**(A) Vehicle group**

**(B) A375-mIgG1/B167-mI0r 7.5 mg/kg-administered group**

FIG. 8

FIG. 9

EP 4 104 857 A1

FIG. 10

FIG. 11

(A) Vehicle

(B) A551-MB110/B379-mI0r

(C) Anti-mouse PD-L1 antibody

(D) A551-MB110/B379-mI0r + Anti-mouse PD-L1 antibody

FIG. 12

**(A) Vehicle**　　**(B) A551-MB110/B379-ml0r**

**(C) Anti-mouse PD-L1 antibody**　**(D) Combined use group**

FIG. 13

**(A) Vehicle**

**(B) A551-MB110/B379-ml0r**

**(C) Anti-mouse PD-L1 antibody**

**(D) Combined use group**

FIG. 14

FIG. 15

FIG. 16

FIG. 17

EP 4 104 857 A1

FIG. 18

EP 4 104 857 A1

FIG. 19

FIG. 20

FIG. 21

FIG. 22

FIG. 23

FIG. 24

EP 4 104 857 A1

FIG. 25

FIG. 26

FIG. 27

FIG. 28

FIG. 29

FIG. 30

FIG. 31

FIG. 32

FIG. 33

FIG. 34

FIG. 35

FIG. 36

FIG. 37

FIG. 38

FIG. 39

FIG. 40

FIG. 41

FIG. 42

FIG. 43

FIG. 44

FIG. 45

FIG. 46

FIG. 47

FIG. 48

FIG. 49

FIG. 50

FIG. 51

FIG. 52

FIG. 53

FIG. 54

FIG. 55

FIG. 56

FIG. 57

FIG. 58

FIG. 59

FIG. 60

FIG. 61

FIG. 62

FIG. 63

FIG. 64

FIG. 65

FIG. 66

FIG. 67

FIG. 68

FIG. 69

FIG. 70

FIG. 71

FIG. 72

FIG. 73

FIG. 74

FIG. 75

FIG. 76

FIG. 77

FIG. 78

FIG. 79

FIG. 80

FIG. 81

FIG. 82

FIG. 83

FIG. 84

FIG. 85

FIG. 86

FIG. 87

FIG. 88

FIG. 89

Ratio of LAG-3+CD8+ T cells in the total number of CD45+ cells in spleen (%)

Ratio of KLRG-1+CD8+ T cells in the total number of CD45+ cells in spleen (%)

Ratio of PD-1+CD8+ T cells in the total number of CD45+ cells in spleen (%)

Ratio of ICOS+CD8+ T cells in the total number of CD45+ cells in spleen (%)

Ratio of FoxP3+ regulatory T cells in the total number of CD45+ cells in spleen (%)

**FIG. 90**

FIG. 91

FIG. 92

# FIG. 93

Ratio of LAG-3⁺CD8⁺ T cells in the total number of CD45⁺ cells in liver (%)

Ratio of KLRG-1⁺CD8⁺ T cells in the total number of CD45⁺ cells in liver (%)

Ratio of PD-1⁺CD8⁺ T cells in the total number of CD45⁺ cells in liver (%)

Ratio of ICOS⁺CD8⁺ T cells in the total number of CD45⁺ cells in liver (%)

Ratio of FoxP3⁺ regulatory T cells in the total number of CD45⁺ cells in liver (%)

FIG. 94

EP 4 104 857 A1

FIG. 95

Cell line (in vitro)

Isotype control
CD73 (unfilled)

C1498

EG7.OVA

Hepa1-6/hGPC3

LLC1/OVA/hGPC3

MC38

LLC1/OVA

Count

CD73 expression

**FIG. 96**

FIG. 97

EP 4 104 857 A1

FIG. 98

FIG. 99

FIG. 100

FIG. 101

FIG. 102

Expression of KLRG-1 on CD8⁺ T cells
in tumor-draining lymph node (DLN) (%)

Expression of ICOS on CD8⁺ T cells
in tumor-draining lymph node (DLN) (%)

Expression of PD-1 on CD8⁺ T cells
in tumor-draining lymph node (DLN) (%)

# FIG. 103

Absolute number of KLRG-1$^+$CD8$^+$ T cells in tumor-draining lymph node (DLN)/BW

Absolute number of ICOS$^+$CD8$^+$ T cells in tumor-draining lymph node (DLN)/BW

Absolute number of PD-1$^+$CD8$^+$ T cells in tumor-draining lymph node (DLN)/BW

FIG. 104

FIG. 105

FIG. 106

## INTERNATIONAL SEARCH REPORT

| International application No. |
| --- |
| PCT/JP2021/004871 |

### A. CLASSIFICATION OF SUBJECT MATTER

Int.Cl. A61K39/395(2006.01)i, A61K45/00(2006.01)i, A61P35/00(2006.01)i,
A61P43/00(2006.01)i, C07K16/28(2006.01)i, C07K16/30(2006.01)i,
C07K16/46(2006.01)i, C12N15/13(2006.01)i
FI: A61K39/395TZNA, A61P35/00, A61K45/00, A61P43/00111, A61P43/00121,
A61K39/395U, C07K16/46, C07K16/30, C12N15/13, C07K16/28

According to International Patent Classification (IPC) or to both national classification and IPC

### B. FIELDS SEARCHED

Minimum documentation searched (classification system followed by classification symbols)
Int.Cl. A61K39/395, A61K45/00, A61P35/00, A61P43/00, C07K16/28, C07K16/30,
C07K16/46, C12N15/13

| Documentation searched other than minimum documentation to the extent that such documents are included in the fields searched | |
| --- | --- |
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)
CAplus/REGISTRY/MEDLINE/EMBASE/BIOSIS/WPIDS(STN)

### C. DOCUMENTS CONSIDERED TO BE RELEVANT

| Category* | Citation of document, with indication, where appropriate, of the relevant passages | Relevant to claim No. |
| --- | --- | --- |
| X | WO 2013/180200 A1 (CHUGAI PHARMACEUTICAL CO., LTD.) 05 December 2013 (2013-12-05), claims, background art, paragraphs [0020], [0051]-[0075], examples | 1–15 |
| X | WO 2015/083764 A1 (CHUGAI PHARMACEUTICAL CO., LTD.) 11 June 2015 (2015-06-11), claims, background art, paragraphs [0016], [0022], [0056]-[0081], [0320], examples, reference examples | 1–15 |
| P, X | WO 2020/032230 A1 (CHUGAI PHARMACEUTICAL CO., LTD.) 13 February 2020 (2020-02-13), claims, examples | 1–15 |

☐ Further documents are listed in the continuation of Box C.  ☒ See patent family annex.

| * | Special categories of cited documents: | | "T" | later document published after the international filing date or priority date and not in conflict with the application but cited to understand the principle or theory underlying the invention |
| --- | --- | --- | --- | --- |
| "A" | document defining the general state of the art which is not considered to be of particular relevance | | | |
| "E" | earlier application or patent but published on or after the international filing date | | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | | |
| "P" | document published prior to the international filing date but later than the priority date claimed | | "&" | document member of the same patent family |

| Date of the actual completion of the international search | Date of mailing of the international search report |
| --- | --- |
| 22 February 2021 | 09 March 2021 |

| Name and mailing address of the ISA/ | Authorized officer |
| --- | --- |
| Japan Patent Office 3-4-3, Kasumigaseki, Chiyoda-ku, Tokyo 100-8915, Japan | Telephone No. |

Form PCT/ISA/210 (second sheet) (January 2015)

| INTERNATIONAL SEARCH REPORT<br>Information on patent family members | International application No.<br>PCT/JP2021/004871 |
|---|---|

| WO 2013/180200 A1 | 05 December 2013 | US 2015/0166654 A1<br>claims, background art,<br>paragraphs [0091], [0123]-[0142],<br>examples<br>EP 2857420 A1 |
|---|---|---|
| WO 2015/083764 A1 | 11 June 2015 | US 2016/0304862 A1<br>claims, background art,<br>paragraphs [0147], [0186]-[0202],<br>[0561], examples,<br>reference examples<br>EP 3078744 A1 |
| WO 2020/032230 A1 | 13 February 2020 | (Family: none) |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013180200 A
- WO 2004044011 A
- WO 2005040229 A
- WO 2002032925 A
- WO 1995001937 A
- WO 2002020565 A
- WO 2003029462 A
- WO 2008016854 A
- WO 2012115241 A
- WO 2014030728 A
- WO 2014163101 A
- WO 2017104783 A
- WO 2013047752 A
- WO 2013125667 A
- WO 2017046994 A
- WO 2006019447 A
- WO 2006053301 A
- WO 2009086320 A
- WO 2009041613 A
- WO 2011122011 A
- WO 2012132067 A
- WO 2013046704 A
- WO 2013180201 A
- WO 2009125825 A
- WO 2012073992 A
- EP 1752471 A
- EP 1772465 A
- WO 9316185 A
- US 5571894 A
- US 5587458 A
- US 5869046 A
- EP 404097 A
- WO 199301161 A
- US 6248516 B1
- US 4816567 A
- US 5821337 A
- US 7527791 B
- US 6982321 B
- US 7087409 B
- US 6075181 A
- US 6150584 A
- US 5770429 A
- US 7041870 B
- US 20070061900 A
- US 7189826 B
- US 5750373 A
- US 20050079574 A
- US 20050119455 A
- US 20050266000 A
- US 20070117126 A
- US 20070160598 A
- US 20070237764 A
- US 20070292936 A
- US 20090002360 A
- WO 2015083764 A
- WO 9308829 A
- US 5731168 A
- WO 2009089004 A1
- US 4676980 A
- US 20060025576 A1
- US 20080069820 A
- WO 2008077546 A
- US 20030157108 A, Presta, L.
- US 20040093621 A
- WO 200061739 A
- WO 200129246 A
- US 20030115614 A
- US 20020164328 A
- US 20040132140 A
- US 20040110704 A
- US 20040110282 A
- US 20040109865 A
- WO 2003085119 A
- WO 2003084570 A
- WO 2005035586 A
- WO 2005035778 A
- WO 2005053742 A
- WO 2002031140 A
- US 20030157108 A1, Presta, L
- WO 2004056312 A1, Adams
- WO 2003085107 A
- WO 2003011878 A, Jean-Mairet
- US 6602684 B, Umana
- US 20050123546 A, Umana
- WO 199730087 A, Patel
- WO 199858964 A, Raju, S
- WO 199922764 A, Raju, S.
- US 5500362 A
- WO 2006029879 A
- WO 2005100402 A
- US 6737056 B
- US 7332581 B
- WO 2004056312 A
- US 6194551 B
- WO 9951642 A
- US 20050014934 A1, Hinton
- US 7371826 B

- US 5648260 A
- US 5624821 A
- WO 9429351 A
- WO 2010107110 A
- US 7521541 B
- US 5648237 A
- US 5789199 A
- US 5840523 A
- US 5959177 A
- US 6040498 A
- US 6420548 B
- US 7125978 B
- US 6417429 B
- US 5208020 A
- US 5416064 A
- EP 0425235 B1
- US 5635483 A
- US 5780588 A
- US 7498298 B
- US 5712374 A
- US 5714586 A
- US 5739116 A

- US 5767285 A
- US 5770701 A
- US 5770710 A
- US 5773001 A
- US 5877296 A
- US 6630579 B
- WO 9411026 A
- US 4737456 A
- US 20050260186 A
- US 20060104968 A
- US 6267958 B
- US 6171586 B
- WO 2006044908 A
- WO 2016047722 A
- WO 2020032230 A
- WO 2015046554 A1
- WO 2015046554 A
- US 8137667 B
- US 8337850 B
- WO 2014030750 A
- WO 2019112027 A

**Non-patent literature cited in the description**

- **HANAHAN.** *Cell,* 2011, vol. 144, 646-74
- **PRIETO.** *Clin Cancer Res.,* 2012, vol. 18, 2039-47
- **HAMID.** *Expert Opin. Biol. Ther.,* 2013, vol. 6, 847-61
- **SUMMERS.** *Nat Rev Immunol,* 2012, vol. 12, 339-51
- **VINAY.** *Cellular & Molecular Immunology,* 2011, vol. 8, 281-284
- **HOUOT.** *Blood,* 2009, vol. 114, 3431-8
- **ASCIERTO.** *Semin Oncol,* 2010, vol. 37, 508-16
- **DUBROT.** *Cancer Immunol Immunother,* 2010, vol. 59, 1223-33
- **SCHABOWSKY.** *Vaccine,* 2009, vol. 28, 512-22
- **LI.** *Proc Natl Acad Sci USA.,* 2013, vol. 110 (48), 19501-6
- *N Eng J Med,* 2015, vol. 373, 23-34
- **KABAT et al.** Sequences of Proteins of Immunological Interest. Health Service, National Institutes of Health, 1991
- **KABAT et al.** Sequences of Proteins of Immunological Interest,. 1991, vol. 1-3
- **KINDT et al.** Kuby Immunology. W.H. Freeman and Co, 2007, 91
- **PORTOLANO et al.** *J. Immunol.,* 1993, vol. 150, 880-887
- **CLARKSON et al.** *Nature,* 1991, vol. 352, 624-628
- **CHOTHIA ; LESK.** *J. Mol. Biol.,* 1987, vol. 196, 901-917

- **KABAT et al.** Sequences of Proteins of Immunological Interest. Public Health Service, National Institutes of Health, 1991
- **MACCALLUM et al.** *J. Mol. Biol.,* 1996, vol. 262, 732-745
- **FLATMAN et al.** *J. Chromatogr. B,* 2007, vol. 848, 79-87
- **LABIANO et al.** *Oncoimmunology,* 2015, vol. 24, e1062967
- **WATTS et al.** *Annu. Rev. Immunol.,* 2005, vol. 23, 23-68
- **NAM et al.** *Curr. Cancer Drug Targets,* 2005, vol. 5, 357-363
- **CHEN et al.** *J. Mol. Biol.,* 1999, vol. 293, 865-881
- **PRESTA et al.** *Cancer Res.,* 1997, vol. 57, 4593-4599
- **EVA GOTTFRIED ; KATRIN PETER ; MARINA P. KREUTZ.** *From Molecular and Modular Tumor Therapy,* 2010, vol. 3 (2), 111-132
- **DHANASEKARAN et al.** *Nature,* 2001, vol. 41 (2), 822-826
- **LAPOINTE et al.** *Proc. Natl. Acad. Sci. USA,* 2004, vol. 101, 811-816
- **PEROU et al.** *Nature,* 2000, vol. 406, 747-752
- **AHRAM et al.** *Mol. Carcinog.,* 2002, vol. 33, 9-15
- **HOOD et al.** *Mol. Cell. Proteomics,* 2005, vol. 4, 1741-1753
- **BRINDLE et al.** *J. Mol. Recognit.,* 1997, vol. 10, 182-187

- **GATES ; SWEELEY.** *Clin. Chem.,* 1978, vol. 24, 1663-1673
- **RESTA ; THOMPSON.** *Immunol. Rev.,* 1998, vol. 161, 95-109
- **SADEJ et al.** *Melanoma Res.,* 2006, vol. 16 (21), 3-222
- **BLAY ; HOSKIN.** *Cancer Res.,* 1997, vol. 57 (260), 2-2605
- **KOBIE et al.** *J. Immunol.,* 2006, vol. 17 (7), 6780-6786
- **CANBOLAT et al.** *Breast Cancer Res. Treat.,* 1996, vol. 37, 189-193
- **DURAK et al.** *Cancer Lett.,* 1994, vol. 84, 199-202
- **FLOCKE ; MANNHERZ.** *Biochim. Biophys. Acta,* 1991, vol. 1076, 273-281
- **BARDOT et al.** *Br. J. Cancer,* 1994, vol. 70, 212-218
- **HEADRICK ; WILLIS.** *Biochem. J.,* 1989, vol. 261, 541-550
- *Proc. Natl. Acad. Sci. USA,* 2006, vol. 103 (35), 13132-13137
- *Curr. Med. Chem.,* 2011, vol. 18, 5217-5223
- **MAZUREK ; EIGENBRODT.** *Anticancer Res.,* 2003, vol. 23, 1149-1154
- **MAZUREK et al.** *J. cell. Physiol.,* 1999, vol. 181, 136-146
- **DROGE et al.** *Immunobiology,* 1987, vol. 174, 473-479
- **UYTTENHOVE et al.** *Nat. Med.,* 2003, vol. 9, 1269-1274
- **OPITZ et al.** *Nature,* 2011, vol. 478 (7368), 197-203
- *J. Immunol.,* 2008, vol. 181, 5396-5404
- **YU et al.** *J. Immunol.,* 2013, vol. 190, 3783-3797
- **SHENG et al.** *Cancer Res.,* 1998, vol. 58, 362-366
- *FASEB J.,* 2004, vol. 18, 790-804
- **DENKERT et al.** *Clin. Breast Cancer,* 2004, vol. 4, 428-433
- **DENKER et al.** *Mod. Pathol.,* 2006, vol. 19, 1261-1269
- *J. Lab. Clin. Med.,* 1993, vol. 122, 518-523
- **TERESA et al.** *Mol. Cancer,* 2009, vol. 8, 41-59
- *Nature Immunology,* 2008, vol. 9, 1261-1269
- **YAMADA et al.** *J. Nutr. Sci. Vitaminol.,* 2010, vol. 56, 83-86
- *J Exp Med,* 1989, vol. 170, 1369-1385
- *Arthritis Rheum.,* 2003, vol. 48, 3242-3252
- **KONO et al.** *Hum. Mol. Genet.,* 2005, vol. 14, 2881-2892
- **KYOGOJU et al.** *Arthritis Rheum,* 2002, vol. 46, 1242-1254
- **WARRMERDAM.** *J. Exp. Med.,* 1990, vol. 172, 19-25
- **HINTON et al.** *J. Immunol.,* 2006, vol. 176 (1), 346-356
- **DALL'ACQUA et al.** *J. Biol. Chem.,* 2006, vol. 281 (33), 23514-23524
- **PETKOVA et al.** *Intl. Immunol.,* 2006, vol. 18 (12), 1759-1769
- **ZALEVSKY et al.** *Nat. Biotechnol.,* 2010, vol. 28 (2), 157-159
- **WARD-KAVANAGH et al.** *Immunity,* 2016, vol. 44, 1005
- **HUDSON et al.** *Nat. Med.,* 2003, vol. 9, 129-134
- **PLUCKTHUN.** The Pharmacology of Monoclonal Antibodies. Springer-Verlag, 1994, vol. 113, 269-315
- **HOLLINGER et al.** *Proc. Natl. Acad. Sci. USA,* 1993, vol. 90, 6444-6448
- **MORRISON et al.** *Proc. Natl. Acad. Sci. USA,* 1984, vol. 81, 6851-6855
- **ALMAGRO ; FRANSSON.** *Front. Biosci.,* 2008, vol. 13, 1619-1633
- **RIECHMANN et al.** *Nature,* 1988, vol. 332, 323-329
- **QUEEN et al.** *Proc. Nat'l Acad. Sci. USA,* 1989, vol. 86, 10029-10033
- **KASHMIRI et al.** *Methods,* 2005, vol. 36, 25-34
- **PADLAN.** *Mol. Immunol.,* 1991, vol. 28, 489-498
- **DALL'ACQUA et al.** *Methods,* 2005, vol. 36, 43-60
- **OSBOURN et al.** *Methods,* 2005, vol. 36, 61-68
- **KLIMKA et al.** *Br. J. Cancer,* 2000, vol. 83, 252-260
- **SIMS et al.** *J. Immunol.,* 1993, vol. 151, 2296
- **CARTER et al.** *Proc. Natl. Acad. Sci. USA,* 1992, vol. 89, 4285
- **PRESTA et al.** *J. Immunol.,* 1993, vol. 151, 2623
- **BACA et al.** *J. Biol. Chem.,* 1997, vol. 272, 10678-10684
- **ROSOK et al.** *J. Biol. Chem.,* 1996, vol. 271, 22611-22618
- **VAN DIJK ; VAN DE WINKEL.** *Curr. Opin. Pharmacol.,* 2001, vol. 5, 368-74
- **LONBERG.** *Curr. Opin. Immunol.,* 2008, vol. 20, 450-459
- **LONBERG.** *Nat. Biotech.,* 2005, vol. 23, 1117-1125
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **BOERNER et al.** *J. Immunol.,* 1991, vol. 147, 86
- **LI et al.** *Proc. Natl. Acad. Sci. USA,* 2006, vol. 103, 3557-3562

- **NI.** *Xiandai Mianyixue,* 2006, vol. 26 (4), 265-268
- **VOLLMERS ; BRANDLEIN.** *Histology and Histopathology,* 2005, vol. 20 (3), 927-937
- **VOLLMERS ; BRANDLEIN.** *Methods and Findings in Experimental and Clinical Pharmacology,* 2005, vol. 27 (3), 185-91
- **HOOGENBOOM et al.** Methods in Molecular Biology. Human Press, 2001, vol. 178, 1-37
- **MCCAFFERTY et al.** *Nature,* vol. 348, 552-554
- **CLACKSON et al.** *Nature,* 1991, vol. 352, 624-628
- **MARKS et al.** *J. Mol. Biol.,* 1992, vol. 222, 581-597
- **MARKS ; BRADBURY.** Methods in Molecular Biology. Human Press, 2003, vol. 248, 161-175
- **SIDHU et al.** *J. Mol. Biol.,* 2004, vol. 338 (2), 299-310
- **LEE et al.** *J. Mol. Biol.,* 2004, vol. 340 (5), 1073-1093
- **FELLOUSE.** *Proc. Natl. Acad. Sci. USA,* 2004, vol. 101 (34), 12467-12472
- **LEE et al.** *J. Immunol. Methods,* 2004, vol. 284 (1-2), 119-132
- **WINTER et al.** *Ann. Rev. Immunol.,* 1994, vol. 12, 433-455
- **GRIFFITHS et al.** *EMBO J,* 1993, vol. 12, 725-734
- **HOOGENBOOM ; WINTER.** *J. Mol. Biol.,* 1992, vol. 227, 381-388
- **BRENNAN et al.** *Science,* 1985, vol. 229, 81
- **KOSTELNY et al.** *J. Immunol.,* 1992, vol. 148 (5), 1547-1553
- **GRUBER et al.** *J. Immunol.,* 1994, vol. 152, 5368
- **TUTT et al.** *J. Immunol.,* 1991, vol. 147, 60
- **CHOWDHURY.** *Methods Mol. Biol.,* 2008, vol. 207, 179-196
- **CUNNINGHAM ; WELLS.** *Science,* 1989, vol. 244, 1081-1085
- **WRIGHT et al.** *TIBTECH,* 1997, vol. 15, 26-32
- **OKAZAKI et al.** *J. Mol. Biol.,* 2004, vol. 336, 1239-1249
- **YAMANE-OHNUKI et al.** *Biotech. Bioeng.,* 2004, vol. 87, 614
- **RIPKA et al.** *Arch. Biochem. Biophys.,* 1986, vol. 249, 533-545
- **KANDA, Y. et al.** *Biotechnol. Bioeng.,* 2006, vol. 94 (4), 680-688
- **RAVETCH ; KINET.** *Annu. Rev. Immunol.,* 1991, vol. 9, 457-492
- **HELLSTROM, I. et al.** *Proc. Nat'l Acad. Sci. USA,* 1986, vol. 83, 7059-7063
- **HELLSTROM, I et al.** *Proc. Nat'l Acad. Sci. USA,* 1985, vol. 82, 1499-1502
- **BRUGGEMANN, M. et al.** *J. Exp. Med.,* 1987, vol. 166, 1351-1361
- **CLYNES et al.** *Proc. Nat'l Acad. Sci. USA,* 1998, vol. 95, 652-656
- **GAZZANO-SANTORO et al.** *J. Immunol. Methods,* 1996, vol. 202, 163
- **CRAGG, M.S. et al.** *Blood,* 2003, vol. 101, 1045-1052
- **CRAGG, M.S. ; M.J. GLENNIE.** *Blood,* 2004, vol. 103, 2738-2743
- **PETKOVA, S.B. et al.** *Int'l. Immunol.,* 2006, vol. 18 (12), 1759-1769
- **SHIELDS et al.** *J. Biol. Chem.,* 2001, vol. 9 (2), 6591-6604
- **IDUSOGIE et al.** *J. Immunol.,* 2000, vol. 164, 4178-4184
- **GUYER et al.** *J. Immunol.,* 1976, vol. 117, 587
- **KIM et al.** *J. Immunol.,* 1994, vol. 24, 249
- **DUNCAN ; WINTER.** *Nature,* 1988, vol. 322, 738-40
- *J. Exp. Med.,* 1990, vol. 172, 19-25
- *J. Clin. Invest.,* 1997, vol. 100, 1059-1070
- *Protein Science,* 1995, vol. 4, 2411-2423
- **KAM et al.** *Proc. Natl. Acad. Sci. USA,* 2005, vol. 102, 11600-11605
- **CHARLTON.** Methods in Molecular Biology. Humana Press, 2003, vol. 248, 245-254
- **GERNGROSS.** *Nat. Biotech.,* 2004, vol. 22, 1409-1414
- **LI et al.** *Nat. Biotech.,* 2006, vol. 24, 210-215
- **GRAHAM et al.** *J. Gen Virol.,* 1977, vol. 36, 59
- **MATHER.** *Biol. Reprod.,* 1980, vol. 23, 243-251
- **MATHER et al.** *Annals N.Y. Acad. Sci.,* 1982, vol. 383, 44-68
- **URLAUB et al.** *Proc. Natl. Acad. Sci. USA,* 1980, vol. 77, 4216
- **YAZAKI ; WU.** Methods in Molecular Biology. Humana Press, 2003, vol. 248, 255-268
- Epitope Mapping Protocols. **MORRIS.** Methods in Molecular Biology. Humana Pres, 1996, vol. 66
- **HARLOW ; LANE.** Antibodies: A Laboratory Manual. Cold Spring Harbor Laboratory, 1988
- **HINMAN et al.** *Cancer Res.,* 1993, vol. 53, 3336-3342
- **LODE et al.** *Cancer Res.,* 1998, vol. 58, 2925-2928
- **KRATZ et al.** *Current Med. Chem.,* 2006, vol. 13, 477-523
- **JEFFREY et al.** *Bioorganic & Med. Chem. Letters,* 2006, vol. 16, 358-362
- **TORGOV et al.** *Bioconj. Chem.,* 2005, vol. 16, 717-721
- **NAGY et al.** *Proc. Natl. Acad. Sci. USA,* 2000, vol. 97, 829-834
- **DUBOWCHIK et al.** *Bioorg. & Med. Chem. Letters,* 2002, vol. 12, 1529-1532

- **KING et al.** *J. Med. Chem.,* 2002, vol. 45, 4336-4343
- **CHARI et al.** *Cancer Res.,* 1992, vol. 52, 127-131
- Remington's Pharmaceutical Sciences. 1980
- *Tumori,* 2012, vol. 98, 478-484
- *Tumor Biol.,* 2015, vol. 36, 4671-4679
- *Am J Clin Pathol,* 2008, vol. 130, 224-230
- *Adv Anat Pathol,* 2014, vol. 21, 450-460
- *Med Oncol,* 2012, vol. 29, 663-669
- *Clinical Cancer Research,* 2004, vol. 10, 6612-6621
- *Appl Immunohistochem Mol Morphol,* 2009, vol. 17, 40-46
- *Eur J Pediatr Surg,* 2015, vol. 25, 138-144
- *J Clin Pathol,* 2011, vol. 64, 587-591
- *Am J Surg Pathol,* 2006, vol. 30, 1570-1575
- *Oncology,* 2007, vol. 73, 389-394
- *Diagnostic Pathology,* 2010, vol. 64, 1-6
- *Diagnostic Pathology,* 2015, vol. 34, 1-6
- *Am J Clin Pathol,* 2008, vol. 129, 899-906
- *Virchows Arch,* 2015, vol. 466, 67-76
- **SHIN, DANIEL SANGHOON et al.** Primary Resistance to PD-1 Blockade Mediated by JAK1/2 Mutations. *Cancer Discovery,* 2017, vol. 7 (2), 188-201
- **SADE-FELDMAN, MOSHE et al.** Resistance to checkpoint blockade therapy through inactivation of antigen presentation. *Nature Communications,* 26 October 2017, vol. 8 (1), 1136
- *Nat. Rev. Immunol.,* 2012, vol. 12, 339-51
- *CHEMICAL ABSTRACTS,* 1393344-72-3
- *CHEMICAL ABSTRACTS,* 934823-49-1
- *CHEMICAL ABSTRACTS,* 1374853-91-4
- *CHEMICAL ABSTRACTS,* 946414-94-4
- *CHEMICAL ABSTRACTS,* 1380723-44-3
- *CHEMICAL ABSTRACTS,* 537032-82-8
- *CHEMICAL ABSTRACTS,* 1428935-60-7
- *CHEMICAL ABSTRACTS,* 477022-00-9
- *CHEMICAL ABSTRACTS,* 745013-59-6
- **SHIN, DANIEL SANGHOON et al.** Primary Resistance to PD-1 Blockade Mediated by'' JAK1/2 Mutations. *Cancer discovery,* 2017, vol. 7 (2), 188-201
- **SADE-FELDMAN, MOSHE et al.** Resistance to checkpoint blockade therapy through inactivation of antigen presentation. *Nature communications,* 26 October 2017, vol. 8 (1), 1136
- *Science Translational Medicine,* 04 October 2017, vol. 9 (410), eaal4291
- **SHARMA P. et al.** *Cell,* 2017, vol. 168 (9), 707-23
- **ZARETSKY JM. et al.** *N Engl J Med.,* 2016, vol. 375 (9), 819-29
- **HU.** *Clin Cancer Res,* 2019, vol. 25 (4), 1318-1330
- **LI.** *Cancer Discov,* 2019, vol. 9 (12), 1754-1773
- **YANG.** *Cancer Sci,* 2019, vol. 110 (8), 2456-2470
- **GOMBAULT.** *Front Immunol,* 2013, vol. 3, 414
- **YANG.** *Cancer Sci,* 2019, vol. 110 (8), 2456-2470
- **PACE, C.N. et al.** *Protein Science,* 1995, vol. 4, 2411-2423
- *Methods Mol. Biol,* 2002, vol. 178, 133-145
- *Methods Mol. Biol.,* 2002, vol. 178, 133-145
- *Protein Eng Des Sel.,* 05 June 2013, vol. 26 (10), 589-598
- **PACE et al.** *Protein Science,* 1995, vol. 4, 2411-2423
- **IWAYANAGI. et al.** *J Immunol,* 2015, vol. 195, 3198-3205